# EUROPÄISCHE PATENTANMELDUNG

(11) **EP 2 141 163 A1**
(43) Veröffentlichungstag der Anmeldung: **06.01.2010**
(21) Anmeldenummer: 08075602.6
(22) Anmeldetag: 02.07.2008
(51) Int. Cl.: C07D 417/00, C07D 417/12, C07D 417/14, C07D 451/04, A61K 31/454, A61P 35/00

(54) **Substituierte Thiazolidinone, deren Herstellung und Verwendung als Arzneimittel**

(71) Anmelder: Bayer Schering Pharma Aktiengesellschaft, 13353 Berlin (DE)
(72) Erfinder: Schulze, Volker, 16562 Bergfelde (DE); Cleve, Arwed, 10707 Berlin (DE); Kosemund, Dirk, 07749 Jena (DE); Siemeister, Gerhard, 13503 Berlin (DE); Suelzle, Detlev, 13467 Berlin (DE); Hillig, Roman, 10825 Berlin (DE); Piechowiak, Guido, 14641 Priort (DE); Eberspächer, Uwe, 13465 Berlin (DE); Husemann, Manfred, 16540 Hohen-Neuendorf (DE); Fanghänel, Jörg, 10119 Berlin (DE)
(74) Vertreter: Williams, Paul Howard

(57) **Zusammenfassung**

Die Erfindung betrifft Thiazolidinone der allgemeinen Formel ( I ) 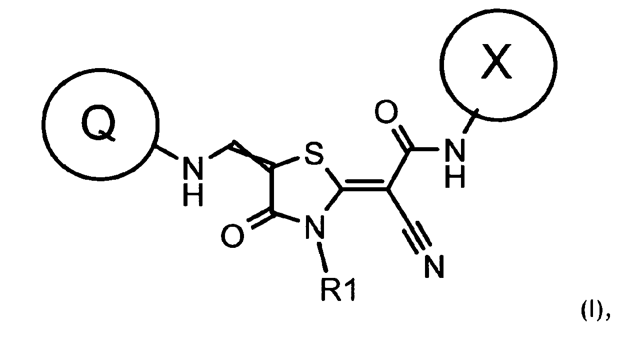 wobei R1, Q und X in den Ansprüchen definiert sind, deren Herstellung und Verwendung als Inhibitoren der Polo Like Kinase (Plk) zur Behandlung verschiedener Erkrankungen, sowie Zwischenprodukte zu deren Herstellung.

## Beschreibung

Die Erfindung betrifft substituierte Thiazolidone, deren Herstellung und Verwendung als Inhibitoren der Polo Like Kinase (Plk) zur Behandlung verschiedener Erkrankungen, sowie Zwischenprodukte zu deren Herstellung.

Tumorzellen zeichnen sich durch einen ungehemmten Zell-Zyklus-Prozess aus. Dies beruht einerseits auf dem Verlust von Kontroll-Proteinen wie RB, p16, p21, p53 etc. sowie der Aktivierung von sog. Beschleunigern des Zell-Zyklus-Prozesses, den cyclinabhängigen Kinasen (CDKs). Die CDKs sind ein in der Pharmazie anerkanntes Anti-Tumor Ziel-Protein. Neben den CDKs wurden neue Zell-Zyklus regulierende Serin/Threonin-Kinasen, sogenannte 'Polo-like Kinasen' beschrieben, die nicht nur bei der Regulation des Zell-Zyklus sondern auch an der Koordination mit anderen Vorgängen während der Mitose und Zytokinese (Ausbildung des Spindelapparates, Chromosomentrennung) beteiligt sind. Daher stellt diese Klasse von Proteinen einen interessanten Angriffspunkt zur therapeutischen Intervention proliferativer Krankheiten wie Krebs dar (Descombes und Nigg. Embo J, 17; 1328ff, 1998; Glover et al. Genes Dev 12, 3777ff, 1998).

Eine hohe Expressionsrate von Plk-1 wurde im 'non-small cell lung'-Krebs (Wolf et al. Oncogene, 14, 543ff, 1997), in Melanomen (Strebhardt et al. JAMA, 283, 479ff, 2000), in 'squamous cell carcinomas' (Knecht et al. Cancer Res, 59, 2794ff, 1999) und in 'esophageal carcinomas '(Tokumitsu et al. Int J Oncol 15, 687ff, 1999) gefunden. Eine Korrelation von hoher Expressionsrate in Tumorpatienten mit schlechter Prognose wurde für verschiedenste Tumore gezeigt (Strebhardt et al. JAMA, 283, 479ff, 2000, Knecht et al. Cancer Res, 59, 2794ff, 1999 und Tokumitsu et al. Int J Oncol 15, 687ff, 1999).

Konstitutive Expression von Plk-1 in NIH-3T3-Zellen führte zu einer malignen Transformation (erhöhte Proliferation, Wachstum in Soft-Agar, Kolonie-Bildung und Tumor-Entwicklung in Nacktmäusen (Smith et al. Biochem Biophys Res Comm,234, 397ff., 1997).

Mikroinjektionen von Plk-1-Antikörpern in HeLa-Zellen führte zu fehlerhafter Mitose (Lane et al.; Journal Cell Biol, 135, 1701ff, 1996).

Mit einem '20-mer' Antisense Oligo konnte die Expression von Plk-1 in A549-Zellen inhibiert und deren Überlebensfähigkeit gestoppt werden. Ebenso konnte eine deutliche Anti-Tumor-Wirkung in Nacktmäusen gezeigt werden (Mundt et al., Biochem Biophys Res Comm, 269, 377ff., 2000).

Die Mikroinjektion von Anti-Plk-1-Antikörpern in nichtimmortalisierte humane Hs68-Zellen, zeigte im Vergleich zu HeLa-Zellen eine deutlich höhere Fraktion von Zellen, welche in einer Wachstumsarretierung an G2 verblieben waren und weit weniger Anzeichen von fehlerhafter Mitose zeigten (Lane et al.; Journal Cell Biol, 135, 1701ff, 1996).

Im Gegensatz zu dem Wachstum von Tumor-Zellen inhibierten Antisense-Oligo-Moleküle das Wachstum und die Viabilität von primären humanen mesangialen Zellen nicht (Mundt et al., Biochem Biophys Res Comm, 269, 377ff., 2000).

In Säugern wurden bislang neben der Plk-1 drei weitere Polo-Kinasen beschrieben, die als mitogene Antwort induziert werden und ihre Funktion in der G1-Phase des Zell-Zykluses ausüben. Dies sind zum einen die sog Prk/Plk-3 (das humane Homolog der Maus-Fnk= Fibroblast growth factor induced kinase; Wiest et al., Genes, Chromosomes & Cancer, 32: 384ff, 2001), Snk/Plk-2 (Serum induced kinase, Liby et al., DNA Sequence, 11, 527-33, 2001) und Sak/Plk4 (Fode et al., Proc.Natl.Acad.Sci. U.S.A, 91, 6388ff; 1994).

Die Inhibition von Plk-1 und der anderen Kinasen der Polo-Familie, wie Plk-2, Plk-3 und Plk-4 stellt somit einen vielversprechenden Ansatz zur Behandlung verschiedener Erkrankungen dar.

Es wurde bereits publiziert, dass Thiazolidone geeignete Inhibitoren der Kinasen der Polo-Familie sind. (Schulze *et al. PCT Internationale Patentanmeldung* WO2006/063806A1 ; Santamaria et al., Molecular Biology of the Cell, 18, 4024ff, 2007).

Die Sequenzidentität innerhalb der Plk-Domänen der Polo-Familie liegt zwischen 40 und 60 %, sodass zum Teil Wechselwirkung von Inhibitoren einer Kinase mit einer oder mehrerer anderen Kinasen dieser Familie auftreten.

Die erfindungsgemäßen Verbindungen inhibieren im wesentlichen die Polo Like Kinasen, worauf auch deren Wirkung zum Beispiel gegen Krebs, wie solide Tumoren und Leukämie, Autoimmunerkrankungen wie Psoriasis, Alopezie, und Multiple Sklerose, Chemotherapeutika-induzierte Alopezie und Mukositis, kardiovaskulare Erkrankungen, wie Stenosen, Arteriosklerosen und Restenosen, infektiöse Erkrankungen, wie z. B. durch unizellulare Parasiten, wie Trypanosoma, Toxoplasma oder Plasmodium, oder durch Pilze hervorgerufen, nephrologische Erkrankungen, wie z. B. Glomerulonephritis, chronische neurodegenerative Erkrankungen, wie Huntington's Erkrankung, amyotropisch laterale Sklerose, Parkinsonsche Erkrankung, AIDS Dementia und Alzheimersche Erkrankung, akute neurodegenerative Erkrankungen, wie Ischämien des Gehirns und Neurotraumata, virale Infektionen, wie z. B. Cytomegalus-Infektionen, Herpes, Hepatitis B und C, und HIV Erkrankungen basiert.

In der PCT Internationalen Patentanmeldung WO 03/093249 werden Thiazolidinonverbindungen offenbart, die Kinasen der Polo-Familie inhibieren. Der in jener Anmeldung zur Charakterisierung der erfindungsgemäßen Verbindungen beschriebene Plk-1 Assay wird bei einer ATP-Konzentration von 10 µM durchgeführt.

Da der K_{M} von Plk-1 für ATP bei 1-2 µM und damit deutlich unterhalb der zellulären ATP-Konzentration von ca. 5 mM liegt, ist es für die Vorhersage der zellulären Aktivität notwendig, einen Plk-1 Assay bei etwa zellulärer ATP-Konzentration durchzuführen. (Z.A. Knight et all; Chemistry & Biology 2005, Vol.12, 621-637).

Die Aufgabe der vorliegenden Verbindungen besteht darin, gegenüber dem Stand der Technik, verbesserte Verbindungen, insbesondere verbessert in der Inhibition der Polo-like Kinase-1 bei millimolarer ATP-Konzentration, bereitzustellen und/oder alternative Verbindungen zur Verfügung zu stellen, die Kinasen, insbesondere Polo-like Kinase-1 bei millimolarer ATP-Konzentration inhibieren und/oder alternative Verbindungen zur Verfügung zu stellen die verbesserte antiproliferative Eigenschaften besitzen und/oder alternative Verbindungen zur Verfügung zu stellen die bessere physikochemische Eigenschaften gegenüber den im Stand der Technik offenbarten Verbindungen haben.

Die vorliegende Erfindung betrifft ein Thiazolidinonderivat mit der allgemeinen Formel ( I ) : 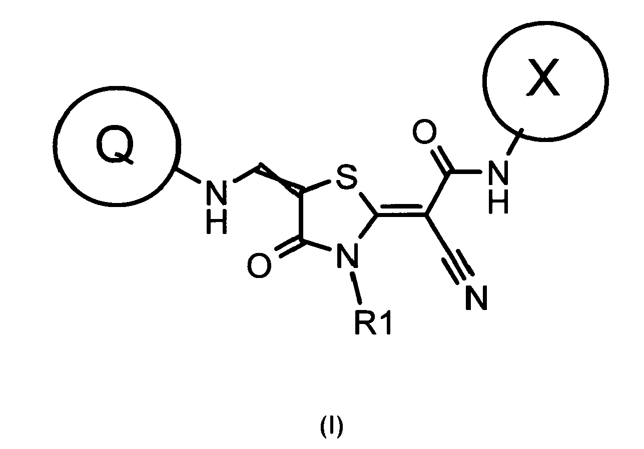 in der :
R1 für gegebenenfalls ein- oder mehrfach, gleich oder verschieden mit einem Halogenatom substituiertes C₁-C₃-Alkyl oder Cyclopropyl steht ;
X für gegebenenfalls ein oder mehrfach, gleich oder verschieden mit Cyan, Methyl, Ethyl, Isopropyl, Ethinyl, Hydroxy, Aryl, Furanyl oder einem Halogenatom substituiertes C₁-C₃-Alkyl steht ;
Q für einen Zyklus : 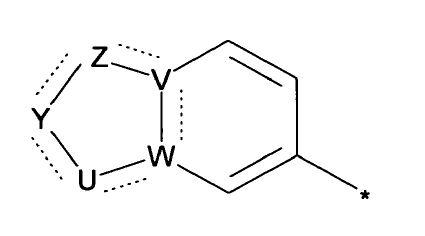
steht,
in dem :
zwischen V und W, W und U, U und Y, Y und Z sowie Z und V unabhängig voneinander jeweils eine Einfachbindung oder eine Doppelbindung besteht, wobei von diesen Bindungen nicht mehr als 2 Doppelbindungen sind,
und
V und W unabhängig voneinander für 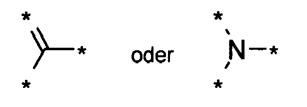 stehen,
U für : 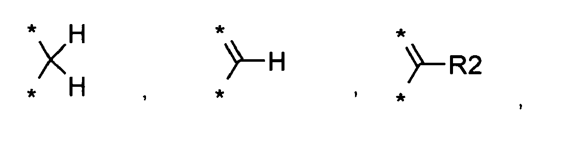 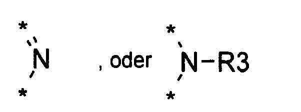 steht,
Y für : 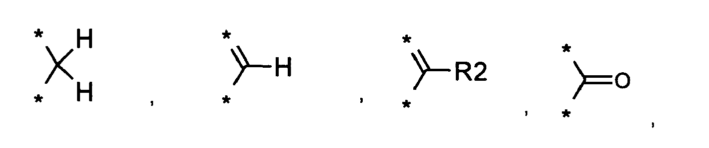 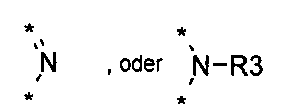 steht und
Z für : 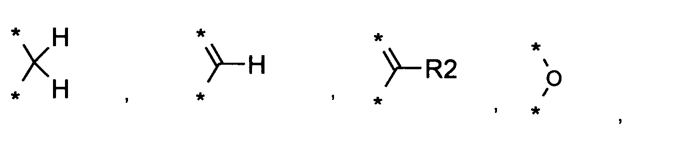 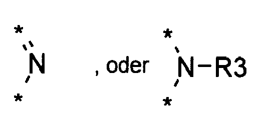 steht,
in dem :
R2 für ein Wasserstoffatom, ein Halogenatom, Cyan, Nitro, C₁-C₆-Alkyl, C₁-C₆-Haloalkyl, C₁-C₆-Alkoxy, C₁-C₆-Haloalkyloxy, C₁-C₆-Alkoxy-C₁-C₆-alkyl, Halo-C₁-C₆-alkoxy-C₁-C₆-alkyl, -(CH₂)ₙC(O)NH₂, -(CH₂)ₙC(O)-R8, -(CH₂)ₙC(O)NR4R5, -(CH₂)ₙC(O)₂R6, C₂-C₆-Alkenyl, C₂-C₆-Alkynyl, C₃-C₁₀-Cycloalkyl, C₃-C₁₀-Heterocycloalkyl, C₆-C₁₀-Aryl, -(CH₂)ₘ-C₆-C₁₀-Aryl, -(CH₂)ₙHeteroaryl, -O(CH2)ₚ-C6-C10-Aryl, -O(CH2)_{q}-Heteroaryl, -C(O)₂R6, -NR4R5, -C(O)NH₂, -C(O)-R8, -S(O)R7, -S(O)₂R7, -S(O)₂R8, -S(O)₂NR4R5, -C(O)H oder -C(O)R7, steht,
   wobei das besagte C₁-C₆-Alkyl, C₁-C₆-Haloalkyl, C₁-C₆-Alkoxy, C₁-C₆-Haloalkyloxy, C₁-C₆-Alkoxy-C₁-C₆-alkyl, Halo-C₁-C₆-alkoxy-C₁-C₆-alkyl, -(CH₂)ₙC(O)R8, -(CH₂)ₙC(O)NR4R5, -(CH₂)ₙC(O)₂R6, C₂-C₆-Alkenyl, C₂-C₆-Alkynyl, C₃-C₁₀-Cycloalkyl, C₃-C₁₀-Heterocycloalkyl, C₆-C₁₀-Aryl, -(CH₂)ₘ-C₆-C₁₀-Aryl, -(CH₂)ₙHeteroaryl, -O(CH₂)ₚ-C₆-C₁₀-aryl, -O(CH₂)_{q}-Heteroaryl, -C(O)₂R6, -NR4R5, -C(O)R8, -S(O)R7, -S(O)₂R7, -S(O)₂R8, -S(O)₂NR4R5, oder -C(O)R7 gegebenenfalls ein- oder mehrfach, gleich oder verschieden mit C₁-C₆-Alkyl, C₁-C₆-Haloalkyl, C₁-C₆-Alkoxy-C₁-C₆-alkyl, Halo-C₁-C₆-alkoxy-C₁-C₆-alkyl, C₂-C₆-Alkenyl, C₂-C₆-Alkynyl, C₃-C₁₀-Cycloalkyl, C₃-C₁₀-Heterocycloalkyl, C₆-C₁₀-Aryl, -(CH₂)ₘ-C₆-C₁₀-Aryl, -(CH₂)ₙHeteroaryl, -C(O)R7, -C(O)₂R6, -NR4R5, -C(0)NR4R5, -S(O)R7, -S(O)₂R7, -S(O)₂NR4R5, R8, R9, -(CH₂)ₙC(O)NR4R5 oder -(CH₂)ₙC(O)₂R6, substituiert ist ;
R3 für Cyan, C₁-C₆-Alkyl, C₁-C₆-Haloalkyl, C₁-C₆-Alkoxy-C₁-C₆-alkyl, Halo-C₁-C₆-alkoxy-C₁-C₆-alkyl, -(CH₂)ₙC(O)NH2, -(CH₂)ₙC(O)R8, -(CH₂)ₙC(O)NR4R5, -(CH₂)ₙC(O)₂R6, C₂-C₆-Alkenyl, C₂-C₆-Alkynyl, C₃-C₁₀-Cycloalkyl, C₃-C₁₀-Heterocycloalkyl, C₆-C₁₀-Aryl, -(CH₂)ₘ-C₆-C₁₀-Aryl, -(CH₂)ₙHeteroaryl, -C(O)₂R6, -C(O)NH₂, -C(O)R8, , -C(O)NR4R5, -S(O)R7, -S(O)₂R7, -S(O)₂R8, -S(O)₂NR4R5, -C(O)H oder -C(O)R7 steht,
   wobei das besagte C₁-C₆-Alkyl, C₁-C₆-Haloalkyl, C₁-C₆-Alkoxy-C₁-C₆-alkyl, Halo-C₁-C₆-alkoxy-C₁-C₆-alkyl, -(CH₂)ₙC(O)R8, -(CH₂)ₙC(O)NR4R5, -(CH₂)ₙC(O)₂R6, C₂-C₆-Alkenyl, C₂-C₆-Alkynyl, C₃-C₁₀-Cycloalkyl, C₃-C₁₀-Heterocycloalkyl, C₆-C₁₀-Aryl, -(CH₂)ₘ-C₆-C₁₀-Aryl, -(CH₂)ₙHeteroaryl, -C(O)₂R6, -C(O)R8, -C(O)NR4R5, -S(O)R7, -S(O)₂R7, -S(O)₂R8, -S(O)₂NR4R5, oder -C(O)R7,
   gegebenenfalls ein- oder mehrfach, gleich oder verschieden mit C₁-C₆-Alkyl, C₁-C₆-Haloalkyl, C₁-C₆-Alkoxy-C₁-C₆-alkyl, Halo-C₁-C₆-alkoxy-C₁-C₆-alkyl, C₂-C₆-Alkenyl, C₂-C₆-Alkynyl, C₃-C₁₀-Cycloalkyl, C₃-C₁₀-Heterocycloalkyl, C₆-C₁₀-Aryl, -(CH₂)ₘ-C₆-C₁₀-Aryl, -(CH₂)ₙHeteroaryl, -C(O)R7, -C(O)₂R6, -NR4R5, -C(O)NR4R5, -S(O)R7, -S(O)₂R7, -S(O)₂NR4R5, R8, R9, -(CH₂)ₙC(O)NR4R5 oder -(CH₂)ₙC(O)₂R6, substituiert ist ;
R4 und R5 unabhängig voneinander für ein Wasserstoffatom, C₁-C₆-Alkyl, C₁-C₆-Haloalkyl, C₁-C₆-Alkoxy-C₁-C₆-alkyl, Halo-C₁-C₆-alkoxy-C₁-C₆-alkyl, C₂-C₆-Alkenyl, C₂-C₆-Alkynyl, C₃-C₁₀-Cycloalkyl, C₆-C₁₀-Aryl, Heteroaryl, -(CH₂)ₘ-C₆-C₁₀-Aryl, oder -(CH₂)ₙHeteroaryl stehen ;
R6 für ein Wasserstoffatom, C₁-C₆-Alkyl, C₁-C₆-Haloalkyl, C₁-C₆-Alkoxy-C₁-C₆-alkyl, Halo-C₁-C₆-alkoxy-C₁-C₆-alkyl, C₂-C₆-Alkenyl, C₂-C₆-Alkynyl, C₃-C₁₀-Cycloalkyl, C₆-C₁₀-Aryl, Heteroaryl -(CH₂)ₘ-C₆-C₁₀-Aryl, oder -(CH₂)ₙHeteroaryl steht ;
R7 für C₁-C₆-Alkyl, C₁-C₆-Haloalkyl, C₁-C₆-Alkoxy-C₁-C₆-alkyl, Halo-C₁-C₆-alkoxy-C₁-C₆-alkyl, C₂-C₆-Alkenyl, C₂-C₆-Alkynyl, C₃-C₁₀-Cycloalkyl, C₆-C₁₀-Aryl, Heteroaryl, -(CH₂)ₘ-C₆-C₁₀-Aryl, oder -(CH₂)ₙHeteroaryl steht ;
R8 für C₃-C₁₀-Heterocycloalkyl steht, wobei das besagte C₃-C₁₀-Heterocycloalkyl gegebenenfalls ein- oder mehrfach, gleich oder verschieden mit C₁-C₆-Alkyl, C₁-C₆-Haloalkyl, C₁-C₆-Alkoxy-C₁-C₆-alkyl, Halo-C₁-C₆-alkoxy-C₁-C₆-alkyl, C₂-C₆-Alkenyl, C₂-C₆-Alkynyl, C₃-C₁₀-Cycloalkyl, C₃-C₁₀-Heterocycloalkyl, C₆-C₁₀-Aryl, -(CH₂)ₘ-C₆-C₁₀-Aryl, -(CH₂)ₙHeteroaryl, -C(O)R7, -C(O)₂R6, -NR4R5, -C(O)NR4R5 -S(O)R7, -S(O)₂R7, -S(O)₂NR4R5, -(CH₂)ₙC(O)NR4R5 oder -(CH₂)ₙC(O)₂R6, substituiert ist ;
R9 für C₃-C₁₀-Cycloalkyl steht, wobei das besagte C₃-C₁₀-Cycloalkyl gegebenenfalls ein-oder mehrfach, gleich oder verschieden mit C₁-C₆-Alkyl, C₁-C₆-Haloalkyl, C₁-C₆-Alkoxy-C₁-C₆-alkyl, Halo-C₁-C₆-alkoxy-C₁-C₆-alkyl, C₂-C₆-Alkenyl, C₂-C₆-Alkynyl, C₃-C₁₀-Cycloalkyl, C₃-C₁₀-Heterocycloalkyl, C₆-C₁₀-Aryl, -(CH₂)ₘ-C₆-C₁₀-Aryl, -(CH₂)ₙHeteroaryl, -C(O)R7, -C(O)₂R6, -NR4R5, -C(O)NR4R5, -S(O)R7, -S(O)₂R7, -S(O)₂NR4R5, -(CH₂)ₙC(O)NR4R5 oder -(CH₂)ₙC(O)₂R6, substituiert ist ; und
n, m, p und q unabhängig voneinander eine Ganzzahl von 1, 2, 3, oder 4, sind.

Eine bevorzugte Untergruppe sind Verbindungen der in diesem Dokument genannten allgemeinen Formel ( I ), in denen :
R1 für gegebenenfalls ein- oder mehrfach, gleich oder verschieden mit einem Halogenatom substituiertes Ethyl, Isopropyl, oder Cyclopropyl steht ;
X für gegebenenfalls ein oder mehrfach, gleich oder verschieden mit Cyan, Methyl, Ethyl, Ethinyl, Hydroxy, Furanyl oder einen Halogenatom substituiertes C₁-C₃-Alkyl steht ;
Q für einen Zyklus : 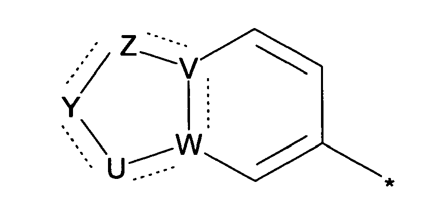
steht,
in dem :
zwischen V und W, W und U, U und Y, Y und Z sowie Z und V unabhängig voneinander jeweils eine Einfachbindung oder eine Doppelbindung besteht, wobei von diesen Bindungen nicht mehr als 2 Doppelbindungen sind,
und
V und W unabhängig voneinander für 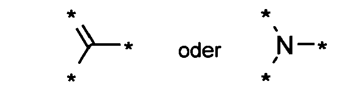 stehen,
U für : 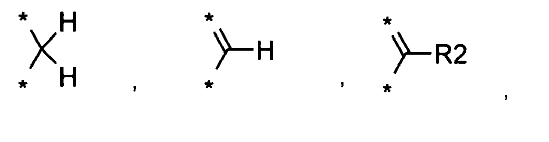 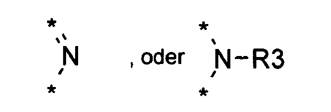 steht,
Y für : 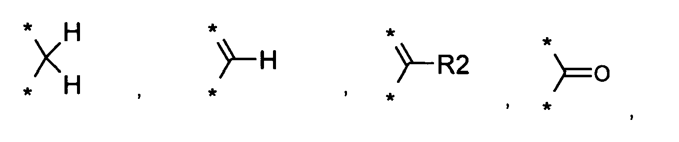 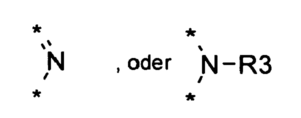 steht,
Z für : 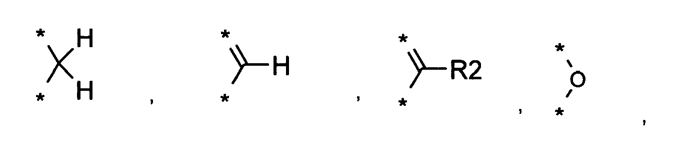 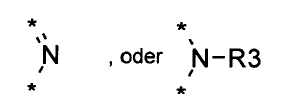 steht,
in dem :
R2 für ein Wasserstoffatom, ein Halogenatom, Cyan, C₁-C₆-Alkyl, C₁-C₆-Haloalkyl, C₁-C₆-Alkoxy-C₁-C₆-alkyl, Halo-C₁-C₆-alkoxy-C₁-C₆-alkyl, -(CH₂)ₙC(O)NH₂, -(CH₂)ₙC(O)-R8, -(CH₂)ₙC(O)NR4R5, -(CH₂)ₙC(O)₂R6, C₂-C₆-Alkenyl, C₃-C₁₀-Cycloalkyl, C₃-C₁₀-Heterocycloalkyl, C₆-C₁₀-Aryl, -(CH₂)ₘ-C₆-C₁₀-Aryl, -(CH₂)ₙHeteroaryl, -C(O)₂R6, -NR4R5, -C(O)NH₂, -C(O)-R8, -C(O)H oder -C(O)R7 steht,
   wobei das besagte C₁-C₆-Alkyl, C₁-C₆-Haloalkyl, C₁-C₆-Alkoxy-C₁-C₆-alkyl, Halo-C₁-C₆-alkoxy-C₁-C₆-alkyl, -(CH₂)ₙC(O)R8, -(CH₂)ₙC(O)NR4R5, -(CH₂)ₙC(O)₂R6, C₂-C₆-Alkenyl, C₃-C₁₀-Cycloalkyl, C₃-C₁₀-Heterocycloalkyl, C₆-C₁₀-Aryl, -(CH₂)ₘ-C₆-C₁₀-Aryl, -(CH₂)ₙHeteroaryl, -C(O)₂R6, -NR4R5, -C(O)R8, oder -C(O)R7 gegebenenfalls ein- oder mehrfach, gleich oder verschieden mit C₁-C₆-Alkyl, C₁-C₆-Haloalkyl, C₁-C₆-Alkoxy-C₁-C₆-alkyl, Halo-C₁-C₆-alkoxy-C₁-C₆-alkyl, C₂-C₆-Alkenyl, C₃-C₁₀-Cycloalkyl, C₃-C₁₀-Heterocycloalkyl, C₆-C₁₀-Aryl, -(CH₂)ₘ-C₆-C₁₀-Aryl, -(CH₂)ₙHeteroaryl, -C(O)R7, -C(O)₂R6, -NR4R5, -C(0)NR4R5, -S(O)R7, -S(O)₂R7, -S(O)₂NR4R5, R8 oder R9 substituiert ist ;
R3 für Cyan, C₁-C₆-Alkyl, C₁-C₆-Haloalkyl, C₁-C₆-Alkoxy-C₁-C₆-alkyl, Halo-C₁-C₆-alkoxy-C₁-C₆-alkyl, -(CH₂)ₙC(O)NH2, -(CH₂)ₙC(O)R8, -(CH₂)ₙC(O)NR4R5, -(CH₂)ₙC(O)₂R6, C₂-C₆-Alkenyl, C₃-C₁₀-Cycloalkyl, C₃-C₁₀-Heterocycloalkyl, C₆-C₁₀-Aryl, -(CH₂)ₘ-C₆-C₁₀-Aryl, -(CH₂)ₙHeteroaryl, -C(O)₂R6, -C(O)NH₂, -C(O)R8, , -C(O)NR4R5, -C(O)H oder -C(O)R7 steht,
   wobei der besagte C₁-C₆-Alkyl, C₁-C₆-Haloalkyl, C₁-C₆-Alkoxy-C₁-C₆-alkyl, Halo-C₁-C₆-alkoxy-C₁-C₆-alkyl, -(CH₂)ₙC(O)R8, -(CH₂)ₙC(O)NR4R5, -(CH₂)ₙC(O)₂R6, C₂-C₆-Alkenyl, C₃-C₁₀-Cycloalkyl, C₃-C₁₀-Heterocycloalkyl, C₆-C₁₀-Aryl, -(CH₂)ₘ-C₆-C₁₀-Aryl, -(CH₂)ₙHeteroaryl, -C(O)₂R6, -C(O)R8, -C(0)NR4R5, oder -C(O)R7
   gegebenenfalls ein- oder mehrfach, gleich oder verschieden mit C₁-C₆-Alkyl, C₁-C₆-Haloalkyl, C₁-C₆-Alkoxy-C₁-C₆-alkyl, Halo-C₁-C₆-alkoxy-C₁-C₆-alkyl, C₂-C₆-Alkenyl, C₃-C₁₀-Cycloalkyl, C₃-C₁₀-Heterocycloalkyl, C₆-C₁₀-Aryl, -(CH₂)ₘ-C₆-C₁₀-Aryl, -(CH₂)ₙHeteroaryl, -C(O)R7, -C(O)₂R6, -NR4R5, -C(0)NR4R5, -S(O)R7, -S(O)₂R7, -S(O)₂NR4R5, R8, R9, -(CH₂)ₙC(O)NR4R5 oder -(CH₂)ₙC(O)₂R6, substituiert ist ; und
n und m unabhängig voneinander eine Ganzzahl von 1, 2, oder 3, sind.

Eine besonders bevorzugte Untergruppe sind Verbindungen der in diesem Dokument genannten allgemeinen Formel ( I ), in denen :
R1 für gegebenenfalls ein- oder mehrfach, gleich oder verschieden mit einem Halogenatom substituiertes Ethyl steht ;
X für gegebenenfalls ein oder mehrfach, gleich oder verschieden mit Cyan, Methyl, Hydroxy oder einen Halogenatom substituiertes C₁-C₃-Alkyl steht ;
Q für : 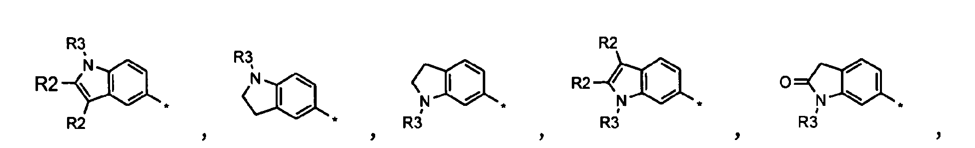 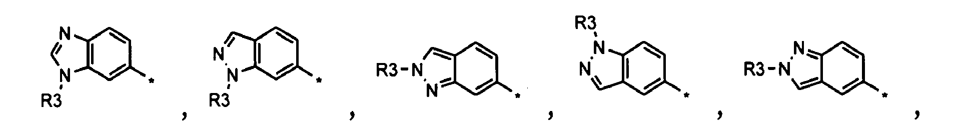 oder 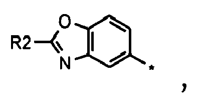 steht, in dem R2 und R3 wie oben beschrieben definiert sind ;
   und in dem n, m, p und q unabhängig voneinander 1 oder 2 sind.

Eine ganz besonders bevorzugte Untergruppe sind Verbindungen der in diesem Dokument genannten allgemeinen Formel ( I ), in denen :
Q für : 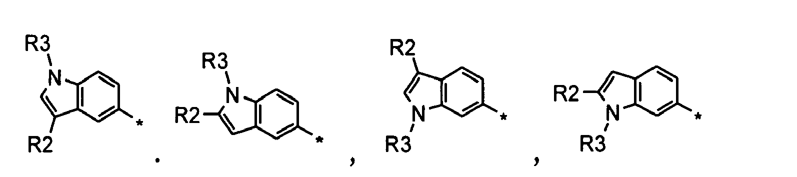 oder 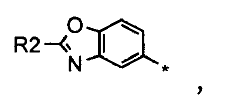
steht, in dem :
R2 für Cyan, C₁-C₆-Alkoxy-C₁-C₆-alkyl, Halo-C₁-C₆-alkoxy-C₁-C₆-alkyl, -(CH₂)ₙC(O)NH₂, - (CH₂)ₙC(O)-R8, -(CH₂)ₙC(O)₂R6, C₂-C₆-Alkenyl, C₃-C₁₀-Cycloalkyl, oder C₃-C₁₀-Heterocycloalkyl steht,
   wobei das besagte C₁-C₆-Alkoxy-C₁-C₆-alkyl, Halo-C₁-C₆-alkoxy-C₁-C₆-alkyl, -(CH₂)ₙC(O)R8, -(CH₂)ₙC(O)₂R6, C₂-C₆-Alkenyl, C₃-C₁₀-Cycloalkyl oder C₃-C₁₀-Heterocycloalkyl
   gegebenenfalls ein- oder mehrfach, gleich oder verschieden mit C₁-C₆-Alkyl, C₁-C₆-Haloalkyl, C₁-C₆-Alkoxy-C₁-C₆-alkyl, Halo-C₁-C₆-alkoxy-C₁-C₆-alkyl, C₂-C₆-Alkenyl, C₃-C₁₀-Cycloalkyl, C₃-C₁₀-Heterocycloalkyl, C₆-C₁₀-Aryl, -(CH₂)ₘ-C₆-C₁₀-Aryl, -(CH₂)ₙHeteroaryl, -C(O)R7, -C(O)₂R6, -NR4R5, -C(O)NR4R5, -S(O)R7, -S(O)₂R7, -S(O)₂NR4R5, R8 oder R9 substituiert ist.

In einer anderen ganz besonders bevorzugten Untergruppe sind Verbindungen der in diesem Dokument genannten allgemeinen Formel ( I ), in denen :
Q für : 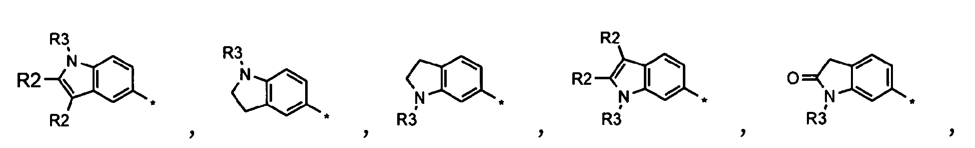 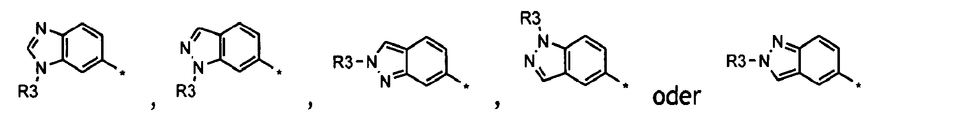 steht, in dem :

R3 für Cyan, C₁-C₆-Alkoxy-C₁-C₆-alkyl, Halo-C₁-C₆-alkoxy-C₁-C₆-alkyl, -(CH₂)ₙC(O)NH₂, - (CH₂)ₙC(O)-R8, -(CH₂)ₙC(O)₂R6, C₂-C₆-Alkenyl, C₃-C₁₀-Cycloalkyl oder C₃-C₁₀-heterocycloalkyl steht,
   wobei das besagte C₁-C₆-alkoxy-C₁-C₆-alkyl, halo-C₁-C₆-alkoxy-C₁-C₆-alkyl, -(CH₂)ₙC(O)R8, -(CH₂)ₙC(O)₂R6, C₂-C₆-alkenyl, C₃-C₁₀-cycloalkyl oder C₃-C₁₀-heterocycloalkyl,
   gegebenenfalls ein- oder mehrfach, gleich oder verschieden mit C₁-C₆-alkyl, C₁-C₆-haloalkyl, C₁-C₆-alkoxy-C₁-C₆-alkyl, halo-C₁-C₆-alkoxy-C₁-C₆-alkyl, C₂-C₆-alkenyl, C₃-C₁₀-cycloalkyl, C₃-C₁₀-heterocycloalkyl, C₆-C₁₀-aryl, -(CH₂)ₘ-C₆-C₁₀-aryl, -(CH₂)ₙheteroaryl, -C(O)R7, -C(O)₂R6, -NR4R5, -C(O)NR4R5, -S(O)R7, -S(O)₂R7, -S(O)₂NR4R5, R8, R9, -(CH₂)ₙC(O)NR4R5 oder -(CH₂)ₙC(O)₂R6 substituiert ist.

In einer anderen ganz besonders bevorzugten Untergruppe sind Verbindungen der in diesem Dokument genannten allgemeinen Formel ( I ), in denen :
R3 für C₁-C₆-Alkoxy-C₁-C₆-alkyl, Halo-C₁-C₆-alkoxy-C₁-C₆-alkyl, C₃-C₁₀-Cycloalkyl oder C₃-C₁₀-heterocycloalkyl steht,
   wobei das besagte C₁-C₆-alkoxy-C₁-C₆-alkyl, halo-C₁-C₆-alkoxy-C₁-C₆-alkyl, C₃-C₁₀-cycloalkyl oder C₃-C₁₀-heterocycloalkyl,
   gegebenenfalls ein- oder mehrfach, gleich oder verschieden mit C₁-C₆-alkyl, C₁-C₆-haloalkyl, C₁-C₆-alkoxy-C₁-C₆-alkyl, halo-C₁-C₆-alkoxy-C₁-C₆-alkyl, C₂-C₆-alkenyl, C₃-C₁₀-cycloalkyl, C₃-C₁₀-heterocycloalkyl, C₆-C₁₀-aryl, -(CH₂)ₘ-C₆-C₁₀-aryl, -(CH₂)ₙheteroaryl, -C(O)R7, -C(O)₂R6, -NR4R5, -C(O)NR4R5, -S(O)R7, -S(O)₂R7, -S(O)₂NR4R5 , R8, R9, -(CH₂)ₙC(O)NR4R5 oder -(CH₂)ₙC(O)₂R6 substituiert ist.

In einer anderen ganz besonders bevorzugten Untergruppe sind Verbindungen der in diesem Dokument genannten allgemeinen Formel ( I ), in denen :
Q für : 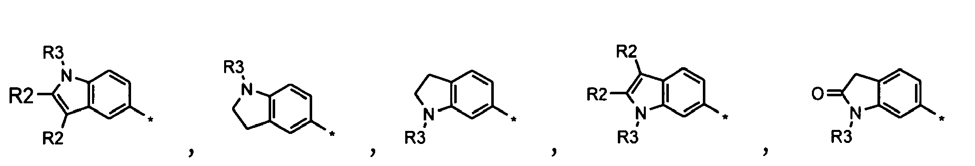 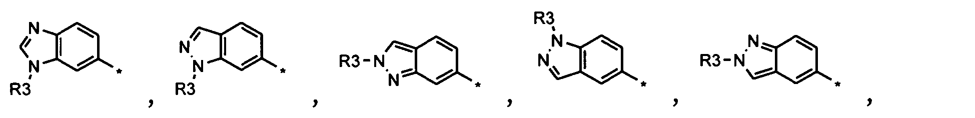 oder 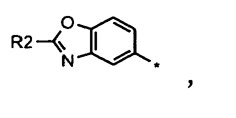
steht, in dem :
R2 für ein Wasserstoffatom, ein Halogenatom, -C₆-C₁₀-aryl, -C(O)₂R6, -NR4R5, -C(O)NH2, -C(O)-R8, -C(O)H oder -C(O)R7 steht,
   wobei das besagte C₆-C₁₀-aryl, -C(O)₂R6, -NR4R5, -C(O)R8, oder -C(O)R7, gegebenenfalls ein- oder mehrfach, gleich oder verschieden mit C₁-C₆-Alkyl, C₁-C₆-Haloalkyl, C₁-C₆-Alkoxy-C₁-C₆-alkyl, Halo-C₁-C₆-alkoxy-C₁-C₆-alkyl, C₂-C₆-Alkenyl, C₃-C₁₀-Cycloalkyl, C₃-C₁₀-Heterocycloalkyl, C₆-C₁₀-Aryl, -(CH₂)ₘ-C₆-C₁₀-Aryl, -(CH₂)ₙHeteroaryl, -C(O)R7, -C(O)₂R6, -NR4R5, -C(O)NR4R5, -S(O)R7, -S(O)₂R7, -S(O)₂NR4R5, R8 oder R9 substituiert ist ;
   oder für
   C₁-C₆-Alkyl, C₁-C₆-Haloalkyl, -(CH₂)ₙC(O)NR4R7, -(CH₂)ₘ-C₆-C₁₀-aryl oder -(CH₂)ₙheteroaryl steht,
   wobei das besagte C₁-C₆-Alkyl, C₁-C₆-Haloalkyl, -(CH₂)ₙC(O)NR4R7, -(CH₂)ₘ-C₆-C₁₀-aryl oder -(CH₂)ₙheteroaryl
   gegebenenfalls ein- oder mehrfach, gleich oder verschieden mit C₁-C₆-Alkyl, C₁-C₆-Haloalkyl, C₃-C₁₀-Cycloalkyl, C₃-C₁₀-Heterocycloalkyl, C₆-C₁₀-Aryl, -C(O)R7, -NR4R5, -S(O)R7, -S(O)₂R7, -S(O)₂NR4R5, R8 oder R9 substituiert ist ;
R3 für C₆-C₁₀-aryl, -C(O)₂R6, -C(O)NH2, -C(O)R8, -C(O)NR4R5, -C(O)H oder -C(O)R7 steht,
   wobei das besagte C₆-C₁₀-aryl, -C(O)₂R6, -C(O)R8, -C(0)NR4R5, oder -C(O)R7, gegebenenfalls ein- oder mehrfach, gleich oder verschieden mit C₁-C₆-Alkyl, C₁-C₆-Haloalkyl, C₁-C₆-Alkoxy-C₁-C₆-alkyl, Halo-C₁-C₆-alkoxy-C₁-C₆-alkyl, C₂-C₆-Alkenyl, C₃-C₁₀-Cycloalkyl, C₃-C₁₀-Heterocycloalkyl, C₆-C₁₀-Aryl, -(CH₂)ₘ-C₆-C₁₀-Aryl, -(CH₂)ₙHeteroaryl, -C(O)R7, -C(O)₂R6, -NR4R5, -C(O)NR4R5, -S(O)R7, -S(O)₂R7, -S(O)₂NR4R5, R8, R9, -(CH₂)ₙC(O)NR4R5 oder -(CH₂)ₙC(O)₂R6, substituiert ist,
oder für
C₁-C₆-Alkyl, C₁-C₆-Haloalkyl, -(CH₂)ₙC(O)NR4R7, -(CH₂)ₘ-C₆-C₁₀-aryl oder -(CH₂)ₙheteroaryl steht,
wobei das besagte C₁-C₆-Alkyl, C₁-C₆-Haloalkyl, -(CH₂)ₙC(O)NR4R7, -(CH₂)ₘ-C₆-C₁₀-aryl oder -(CH₂)ₙheteroaryl
gegebenenfalls ein- oder mehrfach, gleich oder verschieden mit C₁-C₆-Alkyl, C₁-C₆-Haloalkyl, C₃-C₁₀-Cycloalkyl, C₃-C₁₀-Heterocycloalkyl, C₆-C₁₀-Aryl, -C(O)R7, -NR4R5, -S(O)R7, -S(O)₂R7, -S(O)₂NR4R5, R8 oder R9 substituiert ist.

### Folgende Ausführungen betreffen alle Ausführungsformen der Erfindung gleichermaßen

### Alkyl:

Alkyl steht für einen linearen oder verzweigten, gesättigten, monovalenten Kohlenwasserstoffrest mit in der Regel 1 bis 6, bevorzugt 1 bis 4, und besonders bevorzugt 1 bis 3 Kohlenstoffatomen. Beispielhaft seien genannt : Methyl, Ethyl, Propyl, Butyl, Pentyl, Hexyl, *iso*-Propyl, *iso*-Butyl, *sec*-Butyl, *tert*-Butyl, *iso*-Pentyl, 2-Methylbutyl, 1-Methylbutyl, 1-Ethylpropyl, 1,2-Dimethylpropyl, *neo*-Pentyl, 1,1-Dimethylpropyl, 4-Methylpentyl, 3-Methylpentyl, 2-Methylpentyl, 1-Methylpentyl, 2-Ethylbutyl, 1-Ethylbutyl, 3,3-Dimethylbutyl, 2,2-Dimethylbutyl, 1,1-Dimethylbutyl, 2,3-Dimethylbutyl, 1,3-Dimethylbutyl und 1,2-Dimethylbutyl. Besonders bevorzugt ist ein Methyl-, Ethyl-, Propyl- oder Isopropyl-Rest.

### Alkylen:

Alkylen steht für einen linearen oder verzweigten, gesättigten, divalenten Kohlenwasserstoffrest mit in der Regel 1 bis 6, bevorzugt 1 bis 4, und besonders bevorzugt 1 bis 3 Kohlenstoffatomen. Beispielhaft seien genannt : Methylen, Ethylen, Propylen, Butylen, Pentylen, Hexylen, *iso*-Propylen, *iso*-Butylen, *sec*-Butylen, *tert-*Butylen, *iso*-Pentylen, 2-Methylbutylen, 1-Methylbutylen, 1-Ethylpropylen, 1,2-Dimethylpropylen, *neo*-Pentylen, 1,1-Dimethylpropylen, 4-Methylpentylen, 3-Methylpentylen, 2-Methylpentylen, 1-Methylpentylen, 2-Ethylbutylen, 1-Ethylbutylen, 3,3-Dimethylbutylen, 2,2-Dimethylbutylen, 1,1-Dimethylbutylen, 2,3-Dimethylbutylen, 1,3-Dimethylbutylen und 1,2-Dimethylbutylen. Besonders bevorzugt ist ein Methylen-, Ethylen- oder Propylen-Rest.

### Alkenyl:

Alkenyl steht für einen linearen oder verzweigten, monovalenten Kohlenwasserstoffrest mit mindestens einer Doppelbindung und in der Regel 2 bis 6, bevorzugt 2 bis 4, und besonders bevorzugt 2 oder 3 Kohlenstoffatomen. Im Falle von mehreren Doppelbindungen können diese isoliert oder konjugiert vorliegen, wobei isolierte Doppelbindungen bevorzugt sind. Beispielhaft seien genannt : Vinyl-, Allyl-, (*E*)-Prop-1-enyl-, (*Z*)-Prop-1-enyl-, Homoallyl-, (*E*)-But-2-enyl-, (*Z*)-But-2-enyl-, (*E*)-But-1-enyl-, (*Z*)-But-1-enyl-, Pent-4-enyl-, (*E*)-Pent-3-enyl-, (*Z*)-Pent-3-enyl-, (*E*)-Pent-2-enyl-, (*Z*)-Pent-2-enyl-, (*E*)-Pent-1-enyl-, (*Z*)-Pent-1-enyl-, Hex-5-enyl-, (*E*)-Hex-4-enyl-, (*Z*)-Hex-4-enyl-, (*E*)-Hex-3-enyl-, (*Z*)-Hex-3-enyl-, (*E*)-Hex-2-enyl-, (*Z*)-Hex-2-enyl-, (*E*)-Hex-1-enyl-, (*Z*)-Hex-1-enyl-, Isopropenyl-, 2-Methylprop-2-enyl-, 1-Methylprop-2-enyl-, 2-Methylprop-1-enyl-, (*E*)-1-Methylprop-1-enyl-, (*Z*)-1-Methylprop-1-enyl-, 3-Methylbut-3-enyl-, 2-Methylbut-3-enyl-, 1-Methylbut-3-enyl-, 3-Methylbut-2-enyl-, (*E*)-2-Methylbut-2-enyl-, (*Z*)-2-Methylbut-2-enyl-, (*E*)-1-Methylbut-2-enyl-, (Z)-1-Methylbut-2-enyl-, (*E*)-3-Methylbut-1-enyl-, (*Z*)-3-Methylbut-1-enyl-, (*E*)-2-Methylbut-1-enyl-, (*Z*)-2-Methylbut-1-enyl-, (*E*)-1-Methylbut-1-enyl-, (*Z*)-1-Methylbut-1-enyl-, 1,1-Dimethylprop-2-enyl-, 1-Ethylprop-1-enyl-, 1-Propylvinyl-, 1-Isopropylvinyl-, 4-Methylpent-4-enyl-, 3-Methylpent-4-enyl-, 2-Methylpent-4-enyl-, 1-Methylpent-4-enyl-, 4-Methylpent-3-enyl-, (*E*)-3-Methylpent-3-enyl-, (*Z*)-3-Methylpent-3-enyl-, (*E*)-2-Methylpent-3-enyl-, (*Z*)-2-Methylpent-3-enyl-, (*E*)-1-Methylpent-3-enyl-, (*Z*)-1-Methylpent-3-enyl-, (*E*)-4-Methylpent-2-enyl-, (*Z*)-4-Methylpent-2-enyl-, (*E*)-3-Methylpent-2-enyl-, (*Z*)-3-Methylpent-2-enyl-, (*E*)-2-Methylpent-2-enyl-, (*Z*)-2-Methylpent-2-enyl-, (*E*)-1-Methylpent-2-enyl-, (*Z*)-1-Methylpent-2-enyl-, (*E*)-4-Methylpent-1-enyl-, (*Z*)-4-Methylpent-1-enyl-, (*E*)-3-Methylpent-1-enyl-, (*Z*)-3-Methylpent-1-enyl-, (*E*)-2-Methylpent-1-enyl-, (*Z*)-2-Methylpent-1-enyl-, (*E*)-1-Methylpent-1-enyl-, (*Z*)-1-Methylpent-1-enyl-, 3-Ethylbut-3-enyl-, 2-Ethylbut-3-enyl-, 1-Ethylbut-3-enyl-, (*E*)-3-Ethylbut-2-enyl-, (*Z*)-3-Ethylbut-2-enyl-, (*E*)-2-Ethylbut-2-enyl-, (*Z*)-2-Ethylbut-2-enyl-, (*E*)-1-Ethylbut-2-enyl-, (*Z*)-1-Ethylbut-2-enyl-, (*E*)-3-Ethylbut-1-enyl-, (*Z*)-3-Ethylbut-1-enyl-, 2-Ethylbut-1-enyl-, (*E*)-1-Ethylbut-1-enyl-, (*Z*)-1-Ethylbut-1-enyl-, 2-Propylprop-2-enyl-, 1-Propylprop-2-enyl-, 2-Isopropylprop-2-enyl-, 1-Isopropylprop-2-enyl-, (*E*)-2-Propylprop-1-enyl-, (*Z*)-2-Propylprop-1-enyl-, (*E*)-1-Propylprop-1-enyl-, (*Z*)-1-Propylprop-1-enyl-, (*E*)-2-Isopropylprop-1-enyl-, (*Z*)-2-Isopropylprop-1-enyl-, (*E*)-1-Isopropylprop-1-enyl-, (*Z*)-1-Isopropylprop-1-enyl-, 1-(1,1-Dimethylethyl)ethenyl, Buta-1,3-dienyl-, Penta-1,4-dienyl-, Penta-1,3-dienyl-, Hexa-1,5-dienyl-, Hexa-1,4-dienyl- oder eine Hexa-1,3-dienyl-Gruppee. Besonders bevorzugt ist Vinyl oder Allyl.

### Alkinyl:

Alkinyl steht für einen linearen oder verzweigten, monovalenten Kohlenwasserstoffrest mit mindestens einer Dreifachbindung und in der Regel 2 bis 6, bevorzugt 2 bis 4, und besonders bevorzugt 2 oder 3 Kohlenstoffatomen. Beispielhaft seien genannt : Ethinyl-, Prop-1-inyl-, Prop-2-inyl-, But-1-inyl-, But-2-inyl-, But-3-inyl-, Pent-1-inyl-, Pent-2-inyl-, Pent-3-inyl-, Pent-4-inyl-, Hex-1-inyl-, Hex-2-inyl-, Hex-3-inyl-, Hex-4-inyl-, Hex-5-inyl-, 1-Methylprop-2-inyl-, 2-Methylbut-3-inyl-, 1-Methylbut-3-inyl-, 1-Methylbut-2-inyl-, 3-Methylbut-1-inyl-, 1-Ethylprop-2-inyl-, 3-Methylpent-4-inyl-, 2-Methylpent-4-inyl-, 1-Methylpent-4-inyl-, 2-Methylpent-3-inyl-, 1-Methylpent-3-inyl-, 4-Methylpent-2-inyl-, 1-Methylpent-2-inyl-, 4-Methylpent-1-inyl-, 3-Methylpent-1-inyl-, 2-Ethylbut-3-inyl-, 1-Ethylbut-3-inyl-, 1-Ethylbut-2-inyl-, 1-Propylprop-2-inyl-, 1-Isopropylprop-2-inyl-, 2,2-Dimethylbut-3-inyl-, 1,1-Dimethylbut-3-inyl-, 1,1-Dimethylbut-2-inyl- oder eine 3,3-Dimethylbut-1-inyl-Gruppe. Besonders bevorzugt ist Ethinyl, Prop-1-inyl oder Prop-2-inyl.

### Cycloalkyl:

Cycloalkyl steht für einen mono- oder bicyclischen, gesättigten, monovalenten Kohlenwasserstoffrest mit in der Regel 3 bis 10, bevorzugt 3 bis 8, und besonders bevorzugt 3 bis 7 Kohlenstoffatomen. Beispielhaft für monocyclische Cycloalkylreste seien genannt: Cyclopropyl, Cyclobutyl, Cyclopentyl, Cyclohexyl und Cycloheptyl. Besonders bevorzugt ist ein Cyclopropyl-, Cylopentyl- oder ein Cyclohexyl-Rest. Beispielhaft für bicyclische Cycloalkyl-Reste seien genannt: Perhydropentalenyl, Decalinyl.

### Cycloalkylen:

Cycloalkylen steht für einen mono- oder bicyclischen, gesättigten, divalenten Kohlenwasserstoffrest mit in der Regel 3 bis 10, bevorzugt 3 bis 8, und besonders bevorzugt 3 bis 7 Kohlenstoffatomen. Beispielhaft für monocyclische Cycloalkylenreste seien genannt : Cyclopropylen-, Cyclobutylen-, Cyclopentylen-, Cyclohexylen- und Cycloheptylen-. Besonders bevorzugt ist ein Cyclopropylen-, Cylopentylen- oder ein Cyclohexylen-Rest. Beispielhaft für bicyclische Cycloalkylen-Reste seien genannt: Perhydropentalenylen, Decalinylen.

### Cycloalkenyl:

Cycloalkenyl steht für einen mono- oder bicyclischen, monovalenten Kohlenwasserstoffrest mit mindestens einer Doppelbindung und in der Regel 4 bis 10, bevorzugt 5 bis 7, und besonders bevorzugt 5 oder 6 Kohlenstoffatomen. Beispielhaft für monocyclische Cycloalkenyl-Reste seien genannt : Cyclobut-2-enyl-, Cyclobut-1-enyl-, Cyclopent-3-enyl-, Cyclopent-2-enyl-, Cyclopent-1-enyl-, Cyclohex-3-enyl-, Cyclohex-2-enyl-, Cyclohex-1-enyl-, Cyclohept-4-enyl-, Cyclohept-3-enyl-, Cyclohept-2-enyl- und ein Cyclohept-1-enyl-Rest. Besonders bevorzugt ist ein Cyclopent-1-enyl-oder Cyclohex-1-enyl-Rest.

### Alkoxy:

Alkoxy steht für einen linearen oder verzweigten, gesättigten Alkyletherrest der Formel -*O*-Alkyl mit in der Regel 1 bis 6, bevorzugt 1 bis 4, und besonders bevorzugt 1 bis 3 Kohlenstoffatomen. Beispielhaft seien genannt : Methoxy, Ethoxy, Propoxy, Isopropoxy, tert-Butoxy, Pentyloxy und Hexyloxy.

### Alkylcarbonyl

Alkylcarbonyl steht für die Gruppe Alkyl-C(O)- mit in der Regel 1 bis 6, bevorzugt 1 bis 4, und besonders bevorzugt 1 bis 3 Kohlenstoffatomen im Alkylteil. Beispielhaft seien genannt : Acetyl und Propanoyl.

### Alkylcarbonyloxy:

Alkylcarbonyloxy steht für einen Rest der Formel Alkyl-C(O)-O-, mit in der Regel 1 bis 6, bevorzugt 1 bis 4, und besonders bevorzugt 1 bis 3 Kohlenstoffatomen im Alkylteil. Beispielhaft seien genannt : Acetoxy und Propanoyloxy.

### Alkylamino

Alkylamino steht für einen Aminorest mit einem oder zwei (unabhängig voneinander gewählten) Alkylsubstituenten mit in der Regel 1 bis 6, bevorzugt 1 bis 3 Kohlenstoffatomen. (C₁-C₃)-Alkylamino steht beispielsweise für einen Monoalkylaminorest mit 1 bis 3 Kohlenstoffatomen oder für einen Dialkylaminorest mit jeweils 1 bis 3 Kohlenstoffatomen pro Alkylsubstituent. Beispielhaft seien genannt : Methylamino, Ethylamino, Propylamino, Isopropylamino, tert-Butylamino, Pentylamino, Hexylamino, *N,N*-Dimethylamino, *N,N*-Diethylamino, *N*-Ethyl-*N-*methylamino, *N*-Methyl-*N*-propylamino, *N*-Isopropyl-*N*-propylamino, *N*-*tert*-Butyl-*N-*methylamino, *N*-Ethyl-*N*-pentylamino und *N*-Hexyl-*N*-methylamino.

### Alkylaminocarbonyl

Alkylaminocarbonyl steht für einen Aminocarbonylrest mit einem oder zwei (unabhängig voneinander gewählten) Alkylsubstituenten, mit in der Regel 1 bis 6, bevorzugt 1 bis 3 Kohlenstoffatomen. (C₁-C₃)-Alkylaminocarbonyl steht beispielsweise für einen Monoalkylaminocarbonylrest mit 1 bis 3 Kohlenstoffatomen oder für einen Dialkylaminocarbonylrest mit jeweils 1 bis 3 Kohlenstoffatomen pro Alkylsubstituent. Beispielhaft seien genannt: Methylaminocarbonyl, Ethylaminocarbonyl, Propylaminocarbonyl, Isopropylaminocarbonyl, tert-Butylaminocarbonyl, Pentylaminocarbonyl, Hexylaminocarbonyl, N,N-Dimethylaminocarbonyl, *N,N-*Diethylaminocarbonyl, *N*-Ethyl-*N*-methylaminocarbonyl, *N*-Methyl-*N-*propylaminocarbonyl, *N*-Isopropyl-*N*-propylaminocarbonyl, *N*-tert-Butyl-*N-*methylaminocarbonyl, *N*-Ethyl-*N*-pentylaminocarbonyl und *N*-Hexyl-*N-*methylaminocarbonyl.

### Alkylcarbonylamino

Alkylcarbonylamino steht für einen Rest der Formel Alkyl-C(O)-NR- mit in der Regel 1 bis 6, bevorzugt 1 bis 4, und besonders bevorzugt 1 bis 3 Kohlenstoffatomen im Alkylteil und in der Regel R gleich Wasserstoff oder Alkyl. Beispielhaft genannt : Acetylamino, Propanoylamino, Butanoylamino, Isobutanoylamino, *tert*-Butanoylamino, Pentanoylamino, Hexanoylamino.

### Alkoxycarbonyl

Alkoxycarbonyl steht für die Gruppe Alkyl-O-C(O)- mit in der Regel 1 bis 6, bevorzugt 1 bis 4, und besonders bevorzugt 1 bis 3 Kohlenstoffatomen im Alkylteil. Beispielhaft seien genannt : Methoxycarbonyl, Ethoxycarbonyl, Propoxycarbonyl, Isopropoxycarbonyl, *tert-*Butoxycarbonyl, (Pentyloxy)carbonyl und (Hexyloxy)carbonyl.

### Alkoxycarbonylamino

Alkoxycarbonylamino steht für die Gruppe Alkyl-O-C(O)-NR- mit in der Regel 1 bis 6, bevorzugt 1 bis 4, und besonders bevorzugt 1 bis 3 Kohlenstoffatomen im Alkylteil und in der Regel R gleich Wasserstoff oder Alkyl. Beispielhaft seien genannt: (Methoxycarbonyl)amino, (Ethoxycarbonyl)amino, (Propoxycarbonyl)amino, (Isopropoxycarbonyl)amino, *tert*-(Butoxycarbonyl)amino, [(Pentyloxy)carbonyl]amino und [(Hexyloxy)carbonyl]amino.

### Aryl

Aryl ist ein monovalentes, aromatisches mono- bis tricyclisches, carbocyclisches Ringsystem. Beispielhaft seien genannt : Phenyl, Naphthyl und Phenanthryl. Bevorzugt ist Phenyl.

### Arylen

Arylen ist ein divalentes, aromatisches mono- bis tricyclisches, carbocyclisches Ringsystem. Beispielhaft seien genannt : Phenylen, Naphthylen und Phenanthrylen. Bevorzugt ist Phenylen.

### Heteroaryl

Heteroaryl ist ein monovalentes, mono- bis tricyclisches Ringsystem mit mindestens einem Heteroatom. Als Heteroatome können Stickstoffatome, Sauerstoffatome und/oder Schwefelatome vorkommen. Die Bindungsvalenz kann an einem beliebigen Kohlenstoffatom oder an einem Stickstoffatom sein. Ein monocyclischer Heteroarylrest gemäß der vorliegenden Erfindung hat 5 oder 6 Ringatome. Heteroarylreste mit 5 Ringatomen umfassen beispielsweise die Ringe : Thienyl, Thiazolyl, Furyl, Pyrrolyl, Oxazolyl, Imidazolyl, Pyrazolyl, Isoxazolyl, Isothiazolyl, Oxadiazolyl, Triazolyl, Tetrazolyl und Thiadiazolyl. Heteroarylreste mit 6 Ringatomen umfassen beispielsweise die Ringe : Pyridyl, Pyridazinyl, Pyrimidinyl, Pyrazinyl und Triazinyl.

Ein bicyclischer Heteroarylrest gemäß der vorliegenden Erfindung hat 9 oder 10 Ringatome. Heteroarylreste mit 9 Ringatomen umfassen beispielsweise die Ringe : Phthalidyl, Thiophthalidyl, Indolyl, Isoindolyl, Indazolyl, Benzothiazolyl, Benzofuryl, Benzothienyl, Benzimidazolyl, Benzoxazolyl, Indolizinyl, Purinyl.
Heteroarylreste mit 10 Ringatomen umfassen beispielsweise die Ringe : Isochinotinyl, Chinolinyl, Chinolizinyl, Chinazolinyl, Chinoxalinyl, Cinnolinyl, Phthalazinyl, 1,7- or 1,8-Naphthyridinyl, Pteridinyl. Ein tricyclischer Heteroarylrest gemäß der vorliegenden Erfindung hat 13 oder 14 Ringatome. Heteroarylreste mit 14 Ringatomen umfassen beispielsweise die Ringe : Carbazolyl, Phenazinyl, Phenoxazinyl, Acridinyl.

Monocyclische Heteroarylringe mit 5 oder 6 Ringatomen sind bevorzugt.

### Heterocycloalkyl

Heterocycloalkyl im Sinne der Erfindung ist ein nicht aromatisches mono- oder bicyclisches Ringsystem mit mindestens einem Heteroatom oder einer Heterogruppe. Als Heteroatome können Stickstoffatome, Sauerstoffatome und/oder Schwefelatome vorkommen. Als Heterogruppen können -S(O)-, -S(O)₂- oder -N⁺(O⁻)- (N-oxid) vorkommen.
Ein monocyclischer Heterocycloalkylring gemäß der vorliegenden Erfindung kann 3 bis 8, bevorzugt 5 bis 8, besonders bevorzugt 5 oder 6 Ringatome aufweisen. Beispielhaft für monocyclische Heterocycloalkylreste mit 5 Ringatomen seien genannt: Pyrrolidinyl, Imidazolidinyl, Pyrazolidinyl, Pyrrolinyl und Tetrahydrofuranyl. Beispielhaft für monocyclische Heterocycloalkylreste mit 6 Ringatomen seien genannt: Piperidinyl, Piperazinyl, Morpholinyl, Tetrahydropyranyl und Thiomorpholinyl.

Ein bicyclischer Heterocycloalkylrest gemäß der vorliegenden Erfindung kann 5 bis 12, bevorzugt 8 bis 10 Ringatome aufweisen.

Bevorzugt sind 5- bis 8-gliedrige, monocyclische gesättigte Heterocycloalkylreste mit bis zu zwei Heteroatomen aus der Reihe O, N und S.

Besonders bevorzugt sind Morpholinyl, Piperidinyl und Pyrrolidinyl.

### Halogen

Die Bezeichnung Halogen umfasst Fluor, Chlor, Brom oder lod.

### Haloalkyl:

Haloalkyl steht für einen Alkylrest mit mindestens einem Halogensubstituenten.
Beispielhaft seien genannt : Trifluormethyl, 2,2,2-Trifluorethyl-, Pentafluorethyl-, 4,4,5,5,5-Pentafluorpentyl- oder 3,3,4,4,5,5,5-Heptafluorpentylgruppe. Bevorzugt sind perfluorierte Alkylreste wie Trifluormethyl oder Pentafluorethyl.

So wie in dieser Anmeldung verwendet bezeichnet "C₁-C₅", zum Beispiel im Zusammenhang mit der Definition von "C₁-C₅-Alkyl" eine Alkylgruppe mit einer endlichen Anzahl von 1 bis 5 Kohlenstoffatomen, d.h. 1, 2, 3, 4 oder 5 Kohlenstoffatome. Weiter wird die Definition "C₁-C₅" so interpretiert, dass jeder mögliche Teilbereich, wie beispielsweise C₁-C₅, C₂-C₅, C₃-C₅ , C₄-C₅, C₁-C₂, C₁-C₃, C₁-C₄ , C₁-C₅ mitbeinhaltet ist.

Die hier nicht explizit aufgeführten Bereichsangaben der Anmeldung sind analog wie die oben exemplarisch genannten Bereiche "C₁-C₅" definiert.

Unter Isomeren sind chemische Verbindungen der gleichen Summenformel aber unterschiedlicher chemischer Struktur zu verstehen. Man unterscheidet im allgemeinen Konstitutionsisomere und Stereoisomere.

Konstitutionsisomere besitzen die gleiche Summenformel, unterscheiden sich jedoch durch die Verknüpfungsweise ihrer Atome oder Atomgruppen. Hierzu zählen Funktionelle Isomere, Stellungsisomere, Tautomere oder Valenzisomere.

Stereoisomere haben grundsätzlich die gleiche Struktur (Konstitution) - und damit auch die gleiche Summenformel - unterscheiden sich aber durch die räumliche Anordnung der Atome.

Man unterscheidet im allgemeinen Konfigurationsisomere und Konformationsisomere. Konfigurationsisomere sind Stereoisomere, die sich nur durch Bindungsbruch ineinander überführen lassen. Hierzu zählen Enantiomere, Diastereomere und *E* / *Z* (*cis* / *trans*) Isomere.

Enantiomere sind Stereoisomere, die sich wie Bild und Spiegelbild zueinander verhalten und keine Symmetrieebene aufweisen. Alle Stereoisomere, die keine Enantiomere sind, bezeichnet man als Diastereomere. Ein Spezialfall sind *E* / *Z* (*cis* / *trans*) Isomere an Doppelbindungen.

Konformationsisomere sind Stereoisomere, die sich durch die Drehung von Einfachbindungen ineinander überführen lassen.

Zur Abgrenzung der Isomerie-Arten voneinander siehe auch die IUPAC Regeln Sektion E (Pure Appl. Chem. 45, 11-30, 1976).

Die erfindungsgemäßen Verbindungen der allgemeinen Formel I beinhalten auch die möglichen tautomeren Formen und umfassen die *E*- oder *Z*-Isomeren oder, falls ein chirales Zentrum vorhanden ist, auch die Racemate und Enantiomere. Hierunter sind auch Doppelbindungsisomere zu verstehen.

Die erfindungsgemäßen Verdbindungen können auch in Form von Solvaten, insbesondere von Hydraten vorliegen, wobei die erfindungsgemäßen Verbindungen demgemäß polare Lösungsmittel, insbesondere von Wasser, als Strukturelement des Kristallgitters der erfindungsgemäßen Verbindungen enthalten. Der Anteil an polarem Lösungsmittel, insbesondere Wasser kann in einem stöchiometrischen oder auch unstöchiometrischen Verhältnis vorliegen. Bei stöchiometrischen Solvaten, Hydraten spricht man auch von Hemi-, (Semi-), Mono-, Sesqui-, Di-, Tri-, Tetra-, Penta-, usw. Solvaten oder Hydraten.

Ist eine saure Funktion enthalten, sind als Salze die physiologisch verträglichen Salze organischer und anorganischer Basen geeignet, wie beispielsweise die gut löslichen Alkali- und Erdalkalisalze sowie Salze mit N-Methyl-glukamin, *N,N*-Dimethyl-glukamin, N-Ethyl-glukamin, Lysin, 1,6-Hexandiamin, Ethanolamin, Glukosamin, Sarkosin, Serinol, Tris[(hydroxymethyl)amino]methan, Aminopropandiol, Sovak-Base, 1-Amino-2,3,4-butantriol.

Ist eine basische Funktion enthalten, sind die physiologisch verträglichen Salze organischer und anorganischer Säuren geeignet wie Salzsäure, Schwefelsäure, Phosphorsäure, Zitronensäure, Weinsäure, Maleinsäure, Fumarsäure u.a.

Die erfindungsgemäßen Verbindungen der allgemeinen Formel I inhibieren im wesentlichen die Polo Like Kinasen, worauf auch deren Wirkung zum Beispiel gegen Krebs, wie solide Tumoren und Leukämie, Autoimmunerkrankungen wie Psoriasis, Alopezie, und Multiple Sklerose, Chemotherapeutika-induzierte Alopezie und Mukositis, kardiovaskulare Erkrankungen, wie Stenosen, Arteriosklerosen und Restenosen, infektiöse Erkrankungen, wie z.B. durch unizellulare Parasiten, wie Trypanosoma, Toxoplasma oder Plasmodium, oder durch Pilze hervorgerufen, nephrologische Erkrankungen, wie z.B. Glomerulonephritis, chronische neurodegenerative Erkrankungen, wie Huntington's Erkrankung, amyotropisch laterale Sklerose, Parkinsonsche Erkrankung, AIDS induzierte Dementia und Alzheimersche Erkrankung, akute neurodegenerative Erkrankungen, wie Ischämien des Gehirns und Neurotraumata, virale Infektionen, wie z.B. Cytomegalus-Infektionen, Herpes, Hepatitis B und C, und HIV Erkrankungen basiert.

Desweiteren ist Gegenstand der vorliegenden Erfindung die Verwendung der Verbindungen der allgemeinen Formel II sowie deren Solvate, Hydrate, Diastereomere, Enantiomere und Salze als Zwischenprodukte.

Zur Verwendung der erfindungsgemäßen Verbindungen der allgemeinen Formel I als Arzneimittel werden diese in die Form eines pharmazeutischen Präparats gebracht, das neben dem Wirkstoff für die enterale oder parenterale Applikation geeignete pharmazeutische, organische oder anorganische inerte Trägermaterialien, wie zum Beispiel, Wasser, Gelatine, Gummi arabicum, Milchzucker, Stärke, Magnesiumstearat, Talk, pflanzliche Öle, Polyalkylenglycole usw. enthält. Die pharmazeutischen Präparate können in fester Form, zum Beispiel als Tabletten, Dragees, Suppositorien, Kapseln oder in flüssiger Form, zum Beispiel als Lösungen, Suspensionen oder Emulsionen vorliegen. Gegebenenfalls enthalten sie darüber hinaus Hilfsstoffe, wie Konservierungs-, Stabilisierungs-, Netzmittel oder Emulgatoren; Salze zur Veränderung des osmotischen Drucks oder Puffer.
Diese pharmazeutischen Präparate sind ebenfalls Gegenstand der vorliegenden Erfindung.

Für die parenterale Anwendung sind insbesondere Injektionslösungen oder Suspensionen, insbesondere wässrige Lösungen der aktiven Verbindungen in polyhydroxyethoxyliertem Rizinusöl, geeignet.

Als Trägersysteme können auch grenzflächenaktive Hilfsstoffe wie Salze der Gallensäuren oder tierische oder pflanzliche Phospholipide, aber auch Mischungen davon sowie Liposomen oder deren Bestandteile verwendet werden.

Für die orale Anwendung sind insbesondere Tabletten, Dragees oder Kapseln mit Talkum und/oder Kohlenwasserstoffträger oder -binder, wie zum Beispiel Lactose, Mais- oder Kartoffelstärke, geeignet. Die Anwendung kann auch in flüssiger Form erfolgen, wie zum Beispiel als Saft, dem gegebenenfalls ein Süßstoff beigefügt ist.

Die enteralen, parenteralen und oralen Applikationen sind ebenfalls Gegenstand der vorliegenden Erfindung.

Die Dosierung der Wirkstoffe kann je nach Verabfolgungsweg, Alter und Gewicht des Patienten, Art und Schwere der zu behandelnden Erkrankung und ähnlichen Faktoren variieren. Die tägliche Dosis beträgt 0,5-1000 mg, vorzugsweise 50-200 mg, wobei die Dosis als einmal zu verabreichende Einzeldosis oder unterteilt in 2 oder mehreren Tagesdosen gegeben werden kann.

Ebenfalls Gegenstand der vorliegenden Erfindung ist die Verwendung der Verbindungen der allgemeinen Formel I, zur Herstellung eines Arzneimittels zur Behandlung von Krebs, Autoimmunerkrankungen, kardiovaskulären Erkrankungen, Chemotherapeutika-induzierter Alopezie und Mukositis, infektiösen Erkrankungen, nephrologischen Erkrankungen, chronischen und akuten neurodegenerativen Erkrankungen und viralen Infektionen, wobei unter Krebs solide Tumoren und Leukämie, unter Autoimmunerkrankungen Psoriasis, Alopezie und Multiple Sklerose, unter kardiovaskulären Erkrankungen Stenosen, Arteriosklerosen und Restenosen, unter infektiösen Erkrankungen durch unizelluläre Parasiten hervorgerufene Erkrankungen, unter nephrologischen Erkrankungen Glomerulonephritis, unter chronisch neurodegenerativen Erkrankungen Huntington's Erkrankung, amyotrophe Lateralsklerose, Parkinsonsche Erkrankung, AIDS induzierte Dementia und Alzheimersche Erkrankung, unter akut neurodegenerativen Erkrankungen Ischämien des Gehirns und Neurotraumata, und unter viralen Infektionen Cytomegalus-Infektionen, Herpes, Hepatitis B oder C, und HIV Erkrankungen zu verstehen sind.

Ebenfalls Gegenstand der vorliegenden Erfindung sind Arzneimittel zur Behandlung der oben aufgeführten Erkrankungen, die mindestens eine Verbindung gemäß der allgemeinen Formel I enthalten, sowie Arzneimittel mit geeigneten Formulierungs-und Trägerstoffen.

Die erfindungsgemäßen Verbindungen der allgemeinen Formel I sind unter anderem hervorragende Inhibitoren der Polo-like Kinasen, wie Plk1, Plk2, Plk3 und Plk4.

Soweit die Herstellung der Ausgangsverbindungen nicht beschrieben wird, sind diese bekannt oder analog zu bekannten Verbindungen oder hier beschriebenen Verfahren herstellbar. Es ist ebenfalls möglich, alle hier beschriebenen Umsetzungen in Parallel-Reaktoren oder mittels kombinatorischer Arbeitstechniken durchzuführen.
Die Isomerengemische können nach üblichen Methoden wie beispielsweise Kristallisation, Chromatographie oder Salzbildung in die Isomeren, wie z. B. in die Enantiomere, Diastereomere oder *E*/*Z*-Isomere aufgetrennt werden, sofern die Isomere nicht miteinander im Gleichgewicht stehen.

Die Herstellung der Salze erfolgt in üblicher Weise, indem man eine Lösung der Verbindung der Formel I mit der äquivalenten Menge oder einem Überschuss einer Base oder Säure, die gegebenenfalls in Lösung ist, versetzt und den Niederschlag abtrennt oder in üblicher Weise die Lösung aufarbeitet.

### Experimentelle Beschreibung

Die folgende Tabelle enthält die verwendeten Abkürzungen soweit sie nicht schon im Text erklärt wurden.

| **Abkürzung** | **Bedeutung** |
|---|---|
| Ac | Acetyl |
| Boc | *tert*-butoxycarbonyl |
| BOP | [(Benzotriazol-1-yl)oxy]tris(dimethylamino)phosphonium hexafluorophosphat |
| br oder b | Breit |
| Cl | Chemische Ionisierung |
| D | Dublet |
| Dd | Dublet von Dublets |
| Ddd | Dublet von Dublets von Dublets |
| Dt | Dublet von Triplets |
| Dq | Dublet von Quartets |
| DCC | *N,N'*-Dicyclohexylcarbodiimid |
| DCM | Dichlormethan |
| DMF | *N,N*-Dimethylformamid |
| DMSO | Dimethylsulfoxid |
| eq. | Äquivalent |
| ESI | Electrospray Ionisierung |
| GP | Allgemeine Vorschrift |
| HATU | 2-(7-Azabenzotriazol-1-yl)-1,1,3,3-tetramethyl uronium hexafluorophosphat |
| HPLC | high performance liquid chromatography |
| Hünigbase | Diisopropylethylamin |
| LC-MS | Flüssigkeitschromatographie-Massenspektrometrie |
| M | Multiplett |
| MS | Massenspektrometrie |
| NMR | Kernresonanzspektrometrie : Chemische Verschiebungen (δ) in ppm. |
| Pg | Schutzgruppe |
| PyBOP | [(Benzotriazol-1-yl)oxy]tris(pyrrolidin-1-yl)phosphonium hexafluorophosphat |
| Q | Quartet |
| Rac-BINAP | (±)-2,2'-Bis(diphenylphosphino)[1,1'-binaphthyl] |
| Rf | Bei Rückfluss |
| r.t. oder rt | Raumtemperatur |
| S | Singlet |
| sept. | Septet |
| T | Triplet |
| TBAF | Tetrabutylammoniumfluorid |
| TBTU | *O*-(Benzotriazol-1-yl)-*N,N,N',N'*-tetramethyluronium tetrafluoroborat |
| TEA | Triethylamin |
| TLC | Dünnschichtchromatographie |
| TFA | Trifluoressigsäure |
| THF | Tetrahydrofuran |
| TMS | Trimethylsilyl |

Die folgenden Schemata illustrieren allgemeine synthetische Zugänge zu den Verbindungen der Formel (I) der Erfindung begrenzen sie aber in keiner Weise.
Für einen Fachmann ist es offensichtlich, dass die Abfolge der beschriebenen Schritte, die in den Schemata 1-35 aufgeführt sind in verschiedenster Weise modifiziert werden können. Die Abfolge der beschriebenen Schritte stellt daher keine Begrenzung dar. Zusätzlich sind Umwandlungen von Substituenten, zum Beispiel der Reste R1 bis R9 vor oder auch nach den jeweilig beschriebenen Reaktionen möglich.
Diese Umwandungen betreffen zum Beispiel die Einführung von Schutzgruppen, die Abspaltung von Schutzgruppen, Reduktionen oder Oxidationen von funktionellen Gruppen, Halogenierungen, Metallierungen, Substitutionen oder andere dem Fachmann bekannte Reaktionen. Geeignete Schutzgruppen, ihre Einführung und Abspaltung sind bekannt (siehe zum Beispiel: T.W. Greene and P.G.M. Wuts in Protective Groups in Organic Synthesis, 3rd edition, Wiley 1999).
Die Namen der aufgeführten Verbindungen orientieren sich an der IUPAC-Nomenklatur. Es werden zusätzlich auch dem Fachmann bekannte Trivialnahmen verwendet.

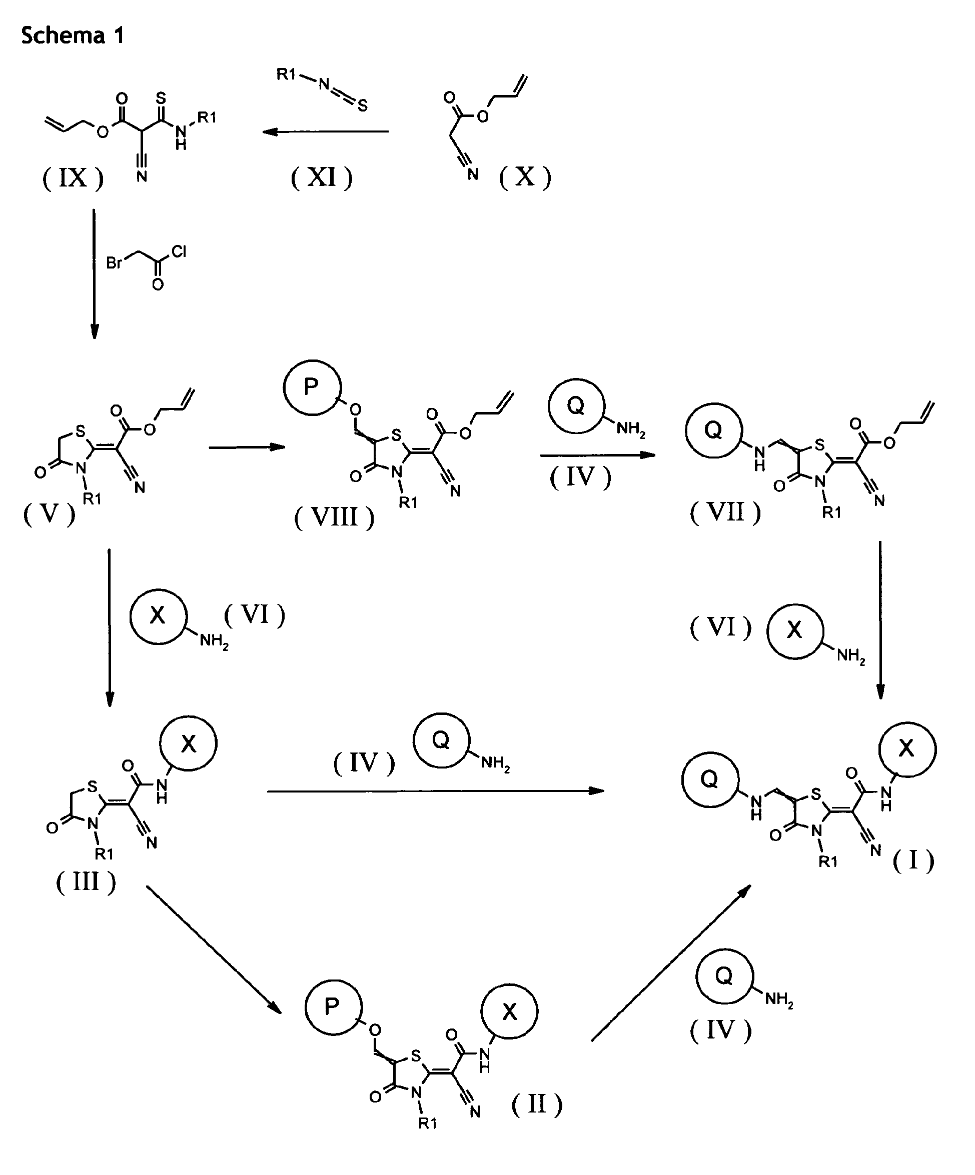

### Schema 1. Allgemeine Übersicht zur Synthese der erfindungsgemäßen Verbindungen, wobei R1, X, Q und P den Definitionen in der Beschreibung und den Ansprüchen entsprechen.

Verbindungen der allgemeinen Formel ( I ) können durch Reaktion von Verbindungen der allgemeinen Formel ( II ) mit Aminoverbindungen der allgemeinen Formel ( IV ) erhalten werden. Die Reaktion kann in verschiedenen polaren Lösungsmitteln wie zum Beispiel Alkoholen, N,N-Dimethylformamid, NMP oder Acetonitril, vorzugsweise Alkohlen, wie Ethanol, 1-Propanol oder 2-Propanol bei Temperaturen zwischen 50°C und 140°C , vorzugsweise bei 78°C (Siedepunkt von Ethanol), oder 97 - 98°C (Siedepunkt von 1-Propanol) durchgeführt werden. Eine alternative bevorzugte Durchführung besteht in der Reaktion in Ethanol oder 1-Propanol als Lösungsmittel unter Druck bei Temperaturen die 10°C bis 30°C oberhalb des Siedepunktes des verwendeten Lösungsmittels liegen.

Verbindungen der allgemeinen Formel ( II ) können durch Reaktion von Verbindungen der allgemeinen Formel ( III ) mit Orthoestern der Ameisensäure wie beispielsweise Triethylorthoformiat oder Tripropylorthoformiat in Gegenwart von Acetanhydrid bei erhöhten Temperaturen zwischen 80°C und 180°C vorzugsweise bei 150°C erhalten werden.

Eine alternative Möglichkeit zur Herstellung von Verbindungen der allgemeinen Formel ( I ) besteht in der Reaktion von Verbindungen der allgemeinen Formel ( III ) mit Aminoverbindungen der allgemeinen Formel ( IV ) in Gegenwart eines Orthoesters der Ameisensäure wie beispielsweise Triethylorthoformiat oder Tripropylorthoformiat, vorzugsweise Triethylorthoformiat in einem polaren Lösungsmittel wie zum Beispiel einem Alkohol, Acetonitril oder DMF bei Temperaturen zwischen 50°C und 160°C. Vorzugsweise wird die Reaktion in Ethanol oder 1-Propanol unter Rückfluss durchgeführt oder aber unter Druck bei Temperaturen die 10°C bis 30°C oberhalb des Siedepunktes des verwendeten Lösungsmittels liegen.

Eine weitere alternative Möglichkeit zur Herstellung von Verbindungen der allgemeinen Formel ( I ) besteht in der Esterspaltung von Verbindungen der allgemeinen Formel ( VII ) gefolgt von der Knüpfung einer Amidbindung durch Reaktion mit einem Amin der allgemeinen Formel ( VI ). Vorzugsweise können diese beiden Reaktionen auch ohne Isolierung der intermediär gebildeten Säure direkt nacheinander in demselben Reaktionsgefäß durchgeführt werden. Polare und inerte Lösungsmittel wie Tetrahydrofuran, Dichlormethan oder N,N-Dimethylformamid vorzugsweise Gemische aus Tetrahydrofuran und N,N-Dimethylformamid können dabei eingesetzt werden. Vorzugsweise kann die Spaltung des Allylesters mit einer katalytischen Menge eines Palladiumkatalysators, besonders bevorzugt Tetrakis(triphenylphosphin)palladium(0) und mit *N,N*-Dimethylbarbitursäure bei Raumtemperatur durchgeführt werden. Die Knüpfung der Amidbindung kann mit einem der üblichen Reagenzien wie zum Beispiel HATU, DCC, TBTU, BOP oder PyBOP, bevorzugterweise mit TBTU, in Gegenwart einer geeigneten Base wie zum Beispiel Triethylamin, Hünigbase, Kaliumcarbonat oder Natriumhydrogencarbonat, vorzugsweise Natriumhydrogencarbonat durchgeführt werden.

Eine weitere Möglichkeit zur Herstellung von neuen Verbindungen der allgemeinen Formel ( I ) besteht in der Abspaltung von Schutzgruppen, die in dem Rest Q in Verbindungen der allgemeinen Formel ( I ) vorhanden sind oder in der Durchführung von Transformationen wie zum Beispiel der Hydrolyse einer Esterfunktionalität, die in dem Rest Q enthalten ist. Derartige Reaktionen sind dem Fachmann wohlbekannt (siehe zum Beispiel: T.W. Greene und P.G.M. Wuts, in Protective groups in Organic Synthesis, 3rd edition, Wiley 1999) .

Verbindungen der allgemeinen Formel ( III ) können aus Verbindungen der allgemeinen Formel ( V ) durch Spaltung des Allylesters gefolgt von der Knüpfung einer Amidbindung durch Reaktion mit einem Amin der allgemeinen Formel ( VI ) hergestellt werden. Vorzugsweise können diese beiden Reaktionen auch ohne Isolierung der intermediär gebildeten Säure direkt nacheinander in demselben Reaktionsgefäß durchgeführt werden. Polare und inerte Lösungsmittel wie Tetrahydrofuran, Dichlormethan oder N,N-Dimethylformamid, vorzugsweise Gemische aus Tetrahydrofuran und N,N-Dimethylformamid können dabei eingesetzt werden. Vorzugsweise kann die Spaltung des Allylesters mit einer katalytischen Menge eines Palladiumkatalysators, besonders bevorzugt Tetrakis(triphenylphosphin)palladium(0) und mit N,N-Dimethylbarbitursäure bei Raumtemperatur durchgeführt werden. Die Knüpfung der Amidbindung kann mit einem der üblichen Reagenzien wie zum Beispiel HATU, DCC, TBTU, BOP oder PyBOP, bevorzugterweise mit TBTU, in Gegenwart einer geeigneten Base wie zum Beispiel Triethylamin, Hünigbase, Kaliumcarbonat oder Natriumhydrogencarbonat, vorzugsweise Natriumhydrogencarbonat durchgeführt werden.

Verbindungen der allgemeinen Formel ( VII ) können durch Reaktion von Verbindungen der allgemeinen Formel ( VIII ) mit Aminoverbindungen der allgemeinen Formel ( IV ) erhalten werden. Die Reaktion kann in verschiedenen polaren Lösungsmitteln wie zum Beispiel Alkoholen, N,N-Dimethylformamid, NMP oder Acetonitril, vorzugsweise Alkohlen, wie Ethanol, 1-Propanol oder 2-Propanol bei Temperaturen zwischen 50°C und 140°C , vorzugsweise bei 78°C (Siedepunkt von Ethanol), oder 97 - 98°C (Siedepunkt von 1-Propanol) durchgeführt werden. Eine alternative bevorzugte Durchführung besteht in der Reaktion in Ethanol oder 1-Propanol als Lösungsmittel unter Druck bei Temperaturen die 10°C bis 30°C oberhalb des Siedepunktes des verwendeten Lösungsmittels liegen. Verbindungen der allgemeinen Formel ( VIII ) können durch Reaktion von Verbindungen der allgemeinen Formel (V) mit Orthoestern der Ameisensäure wie beispielsweise Triethylorthoformiat oder Tripropylorthoformiat in Gegenwart von Acetanhydrid bei Temperaturen zwischen 70°C und 120°C vorzugsweise bei 100°C erhalten werden.

Verbindungen der allgemeinen Formel ( V ) können durch Reaktion von Verbindungen der allgemeinen Formel ( IX ) mit Bromessigsäurechlorid bei Temperaturen zwischen 0°C und 70°C vorzugsweise bei Raumtemperatur in inerten Lösungsmitteln wie zum Beispiel Tetrahydrofuran, Diethylether oder Acetonitril, vorzugsweise Tetrahydrofuran erhalten werden.

Verbindungen der allgemeinen Formel ( IX ) können durch Reaktion von Verbindungen der allgemeinen Formel ( X ) mit Isothiocyanaten der allgemeinen Formel ( XI ) in Gegenwart einer geeigneten Base wie zum Beispiel Triethylamin oder Hünigbase, bei Temperaturen zwischen 0°C und 90°C vorzugsweise bei 60°C erhalten werden. Die Reaktion wird vorzugsweise ohne zusätzliches Lösungsmittel durchgeführt kann aber auch in Gegenwart von inerten Lösungsmitteln wie zum Beispiel THF oder Tetrachlormethan erfolgen.

Eine Vielzahl geeigneter Isothiocyanate für die oben beschriebene Herstellung von Verbindungen der allgemeinen Formel ( IX ) sind in der Literatur beschrieben oder sind kommerziell erhältlich. Geeignete Methoden zur Synthese von Isothiocyanaten sind beispielsweise in Tetrahedron Letters 1985, 26, 1661 oder in Synthesis 1991, 1001 beschrieben.

Amine der allgemeinen Struktur ( VI ) sind entweder kommerziell erhältlich oder in der Literatur beschrieben.

Aminoverbindungen der allgemeinen Struktur ( IV ) sind entweder kommerziell erhältlich oder in wenigen Stufen herstellbar. Einige synthetische Routen zur Herstellung verschiedener Aminoverbindungen der allgemeinen Struktur ( IV ) sind im Folgenden skizziert (Schemata 2 - 35). Diese Syntheserouten beinhalten eine Vielzahl synthetischer Standardmethoden, die dem Fachmann wohlbekannt sind. Zum Teil ist die Synthese ähnlicher Aminoverbindungen auch in der Literatur beschrieben.

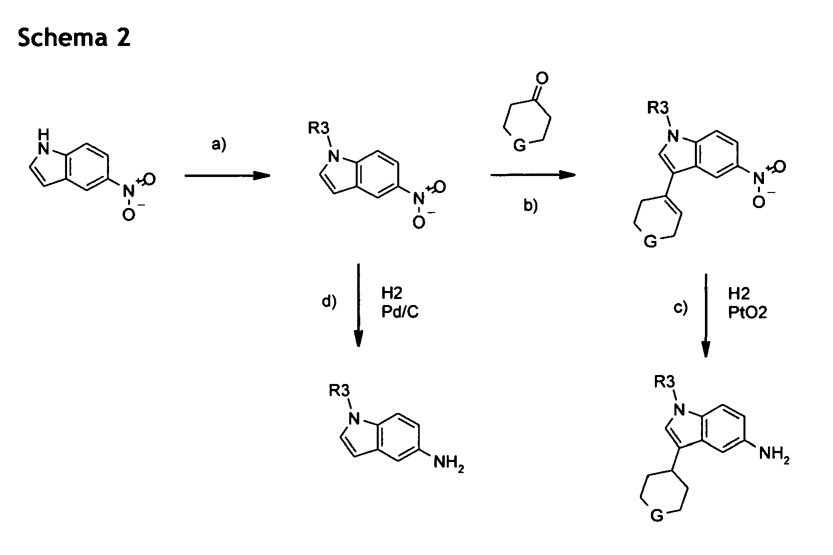

***Schema 2*.** Übersicht zur Synthese von Aminoverbindungen der allgemeinen Formel ( IV ), wobei R3 der Definition in der Beschreibung und den Ansprüchen entspricht. G entspricht einem *N*-Atom, das mit C₁-C₆-alkyl, C₁-C₆-haloalkyl, C₁-C₆-alkoxy-C₁-C₆-alkyl, C₃-C₁₀-cycloalkyl, -C(O)R7 oder -C(O)₂R7 substituiert ist.
a) Alkylierung; b) Säure katalysierte Kondensation; c), d) Hydrierung.

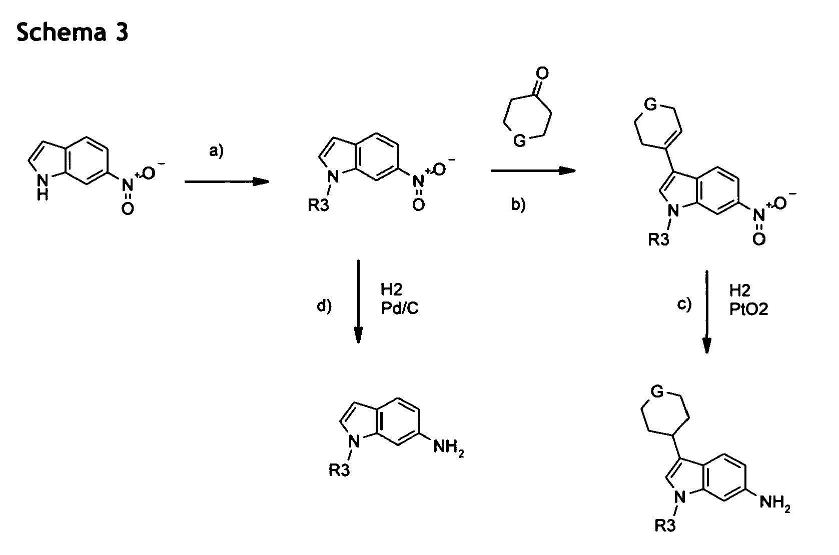

***Schema 3***. Übersicht zur Synthese von Aminoverbindungen der allgemeinen Formel ( IV ), wobei R3 der Definition in der Beschreibung und den Ansprüchen entspricht. G entspricht einem *N*-Atom, das mit C₁-C₆-alkyl, C₁-C₆-haloalkyl, C₁-C₆-alkoxy-C₁-C₆-alkyl, C₃-C₁₀-cycloalkyl, -C(O)R7 oder -C(O)₂R7 substituiert ist.
a) Alkylierung; b) Säure katalysierte Kondensation; c), d) Hydrierung.

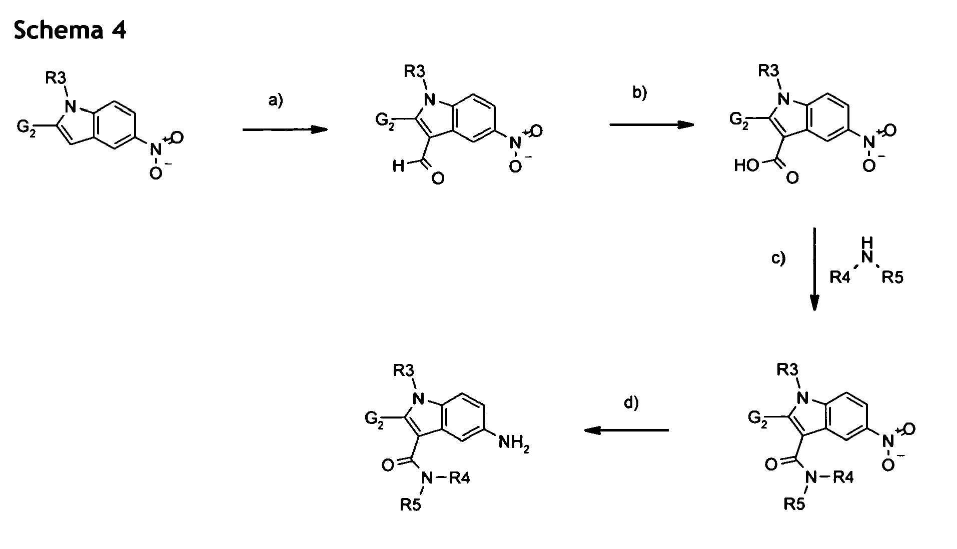

***Schema 4.*** Übersicht zur Synthese von Aminoverbindungen der allgemeinen Formel ( IV ), wobei R3, R4 und R5 den Definitionen in der Beschreibung und den Ansprüchen entsprechen. G2 entspricht H oder C₁-C₆-alkyl.
a) Formylierung; b) Oxidation; c) Peptidkupplung; d) Hydrierung.

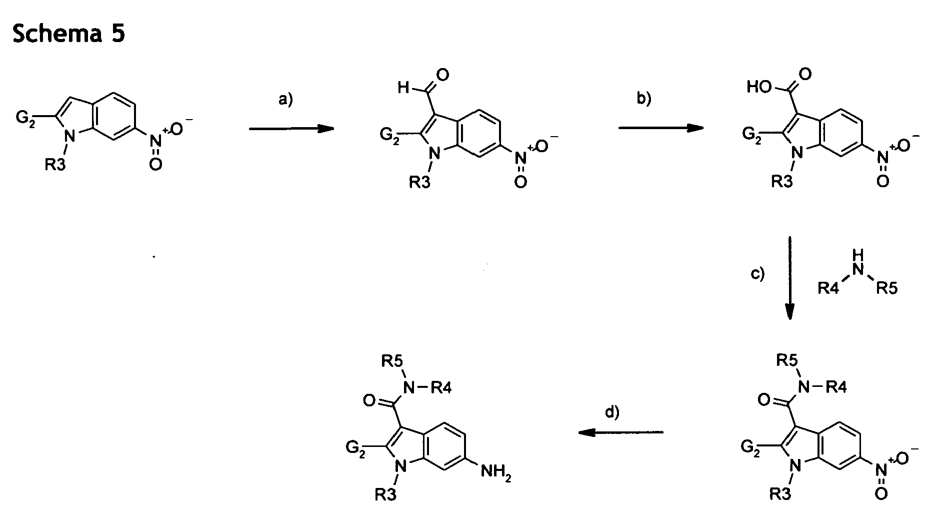

***Schema 5**.* Übersicht zur Synthese von Aminoverbindungen der allgemeinen Formel ( IV ), wobei R3, R4 und R5 den Definitionen in der Beschreibung und den Ansprüchen entsprechen. G2 entspricht H oder C₁-C₆-alkyl.
a) Formylierung; b) Oxidation; c) Peptidkupplung; d) Hydrierung.

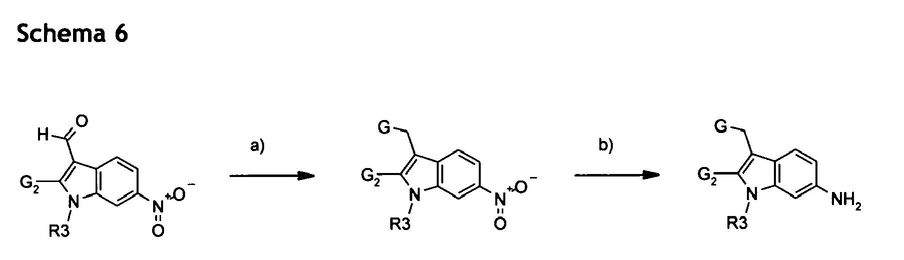

***Schema 6**.* Übersicht zur Synthese von Aminoverbindungen der allgemeinen Formel ( IV ), wobei R3 der Definition in der Beschreibung und den Ansprüchen entspricht. G entspricht R8 oder -NR4R5. G2 entspricht H oder C₁-C₆-alkyl.
a) Reduktive Aminierung; b) Hydrierung.

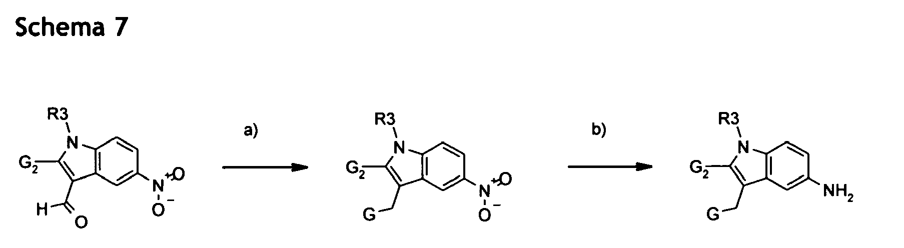

***Schema 7.*** Übersicht zur Synthese von Aminoverbindungen der allgemeinen Formel ( IV ), wobei R3 der Definition in der Beschreibung und den Ansprüchen entspricht. G entspricht R8 oder -NR4R5. G2 entspricht H oder C₁-C₆-alkyl.
a) Reduktive Aminierung; b) Hydrierung.

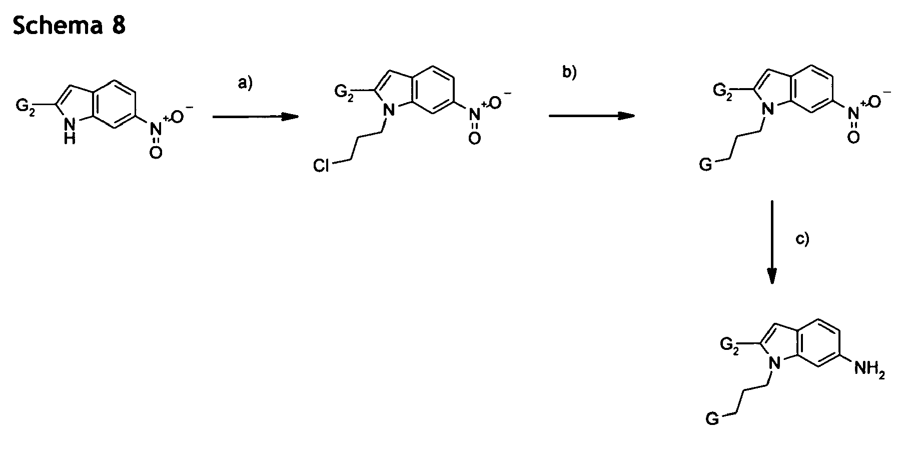

***Schema 8.*** Übersicht zur Synthese von Aminoverbindungen der allgemeinen Formel ( IV ), wobei G R8 oder -NR4R5 entspricht. G2 entspricht H oder C₁-C₆-alkyl.
a) Alkylierung; b) Reduktive Aminierung; b) Hydrierung.

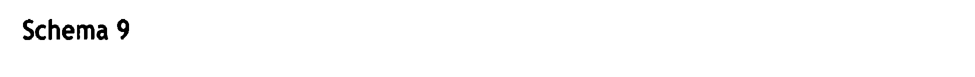

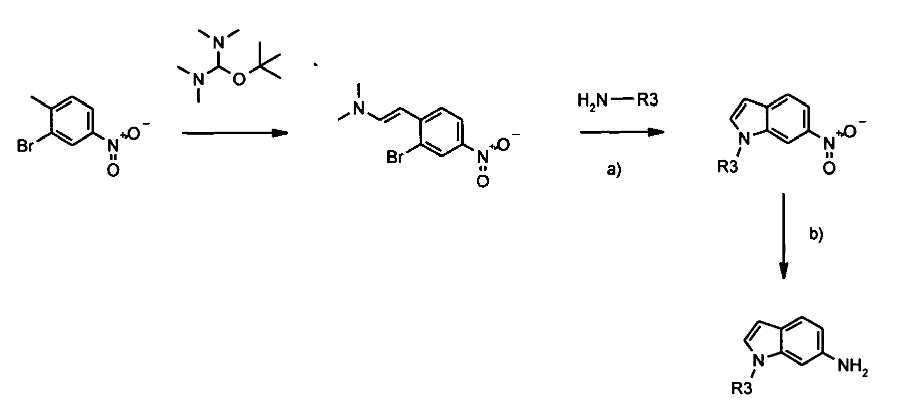

***Schema 9.*** Übersicht zur Synthese von Aminoverbindungen der allgemeinen Formel ( IV ), wobei R3 der Definition in der Beschreibung und den Ansprüchen entspricht.
a) Palladium katalysierte Aminierung und Cyclisierung; b) Hydrierung.

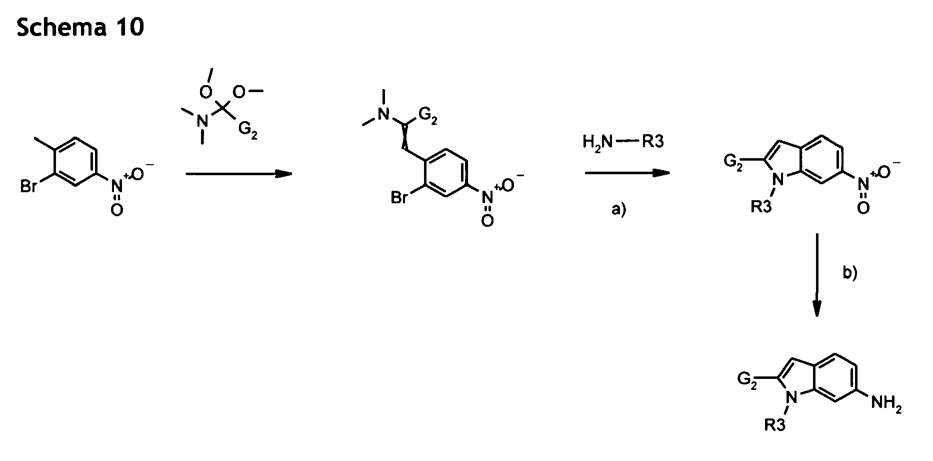

***Schema 10.*** Übersicht zur Synthese von Aminoverbindungen der allgemeinen Formel ( IV ), wobei R3 der Definition in der Beschreibung und den Ansprüchen entspricht. G2 entspricht H oder C₁-C₆-alkyl.
a) Palladium katalysierte Aminierung und Cyclisierung; b) Hydrierung.

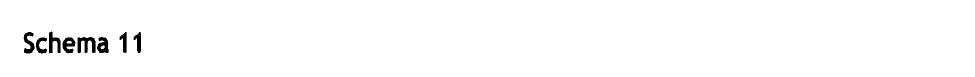

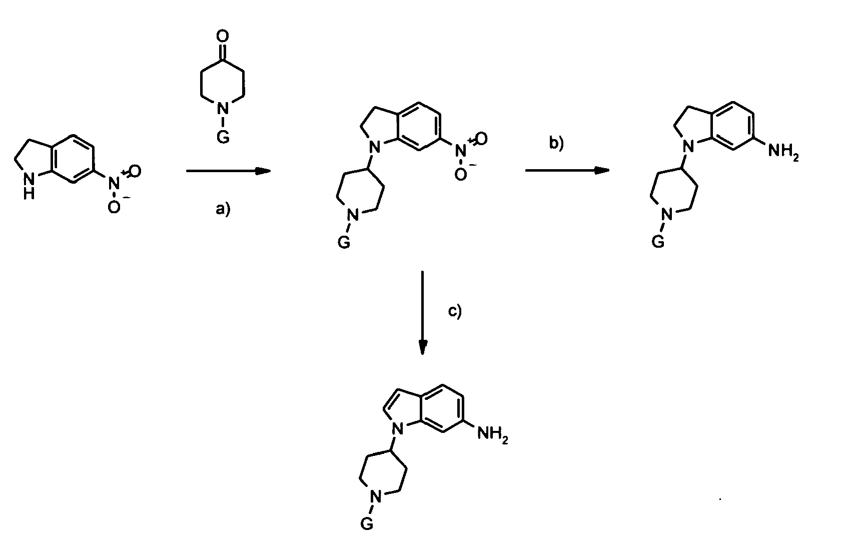

***Schema 11.*** Übersicht zur Synthese von Aminoverbindungen der allgemeinen Formel ( IV ), wobei G C₁-C₆-alkyl, C₁-C₆-haloalkyl, C₁-C₆-alkoxy-C₁-C₆-alkyl, halo-C₁-C₆-alkoxy-C₁-C₆-alkyl, C₃-C₁₀-cycloalkyl, C₃-C₁₀-heterocycloalkyl, R8, -(CH₂)ₘ-C₆-C₁₀-aryl, oder - (CH₂)ₙheteroaryl entspricht wobei das C₁-C₆-alkyl mit -C(O)NH2, -COOCH₃, -C(O)R8, oder -C(O)NR4R5 substituiert sein kann.
a) Reduktive Aminierung; b) Reduktion; c) Hydrierung/Dehydrierung.

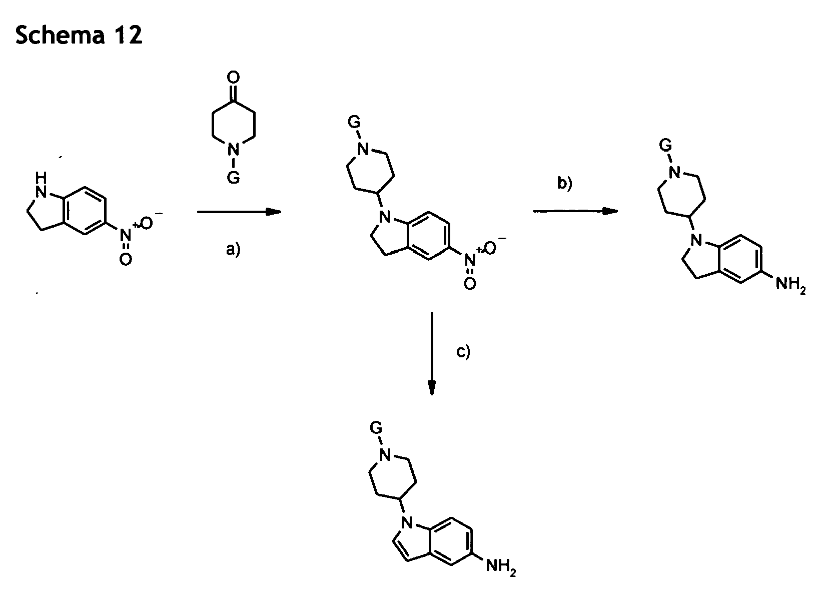

***Schema 12.*** Übersicht zur Synthese von Aminoverbindungen der allgemeinen Formel ( IV ), wobei G C₁-C₆-alkyl, C₁-C₆-haloalkyl, C₁-C₆-alkoxy-C₁-C₆-alkyl, halo-C₁-C₆-alkoxy-C₁-C₆-alkyl, C₃-C₁₀-cycloalkyl, C₃-C₁₀-heterocycloalkyl, R8, -(CH₂)ₘ-C₆-C₁₀-aryl, oder -(CH₂)ₙheteroaryt entspricht, wobei das C₁-C₆-alkyl mit -C(O)NH2, -COOCH₃, -C(O)R8, oder -C(O)NR4R5 substituiert sein kann.
a) Reduktive Aminierung; b) Reduktion; c) Hydrierung/Dehydrierung.

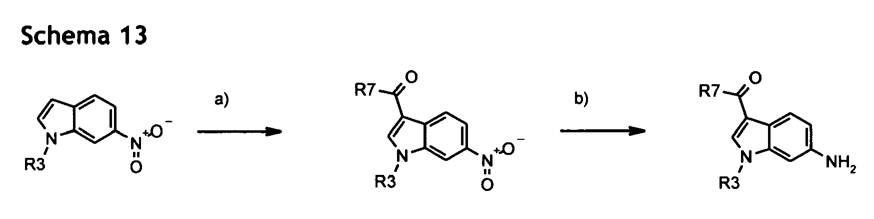

***Schema 13.*** Übersicht zur Synthese von Aminoverbindungen der allgemeinen Formel ( IV ), wobei R3 und R7 den Definitionen in der Beschreibung und den Ansprüchen entsprechen.
a) Acylierung; b) Hydrierung.

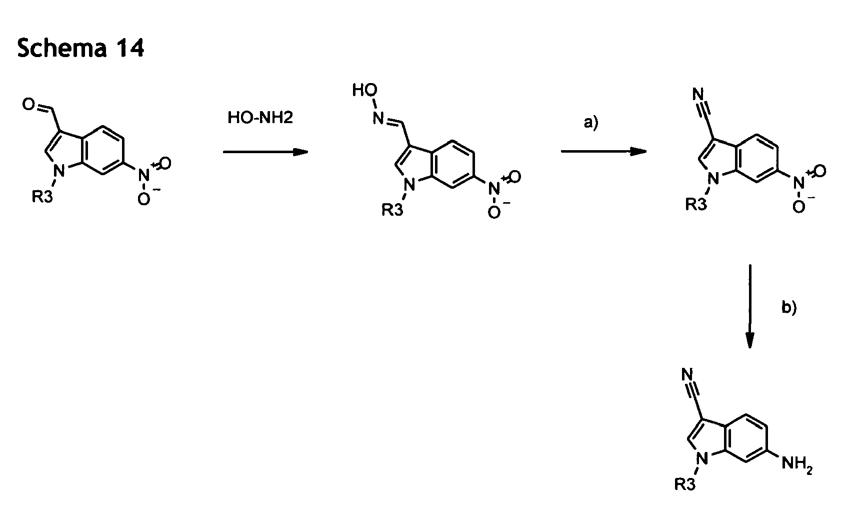

***Schema 14**.* Übersicht zur Synthese von Aminoverbindungen der allgemeinen Formel ( IV ), wobei R3 der Definition in der Beschreibung und den Ansprüchen entspricht.
a) Dehydratisierung durch Behandlung mit einem Wasser entziehenden Mittel; b) Hydrierung.

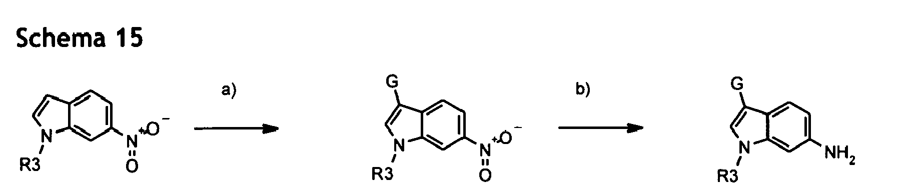

***Schema 15.*** Übersicht zur Synthese von Aminoverbindungen der allgemeinen Formel ( IV ), wobei R3 der Definition in der Beschreibung und den Ansprüchen entspricht. G entspricht einem Halogenatom.
a) Behandlung mit einem Halogenierungsmittel; b) Reduktion.

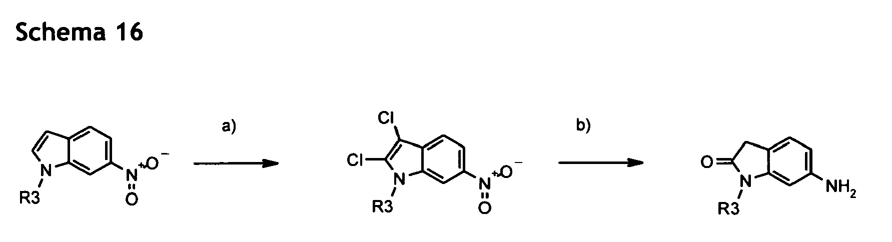

***Schema 16*.** Übersicht zur Synthese von Aminoverbindungen der allgemeinen Formel ( IV ), wobei R3 der Definition in der Beschreibung und den Ansprüchen entspricht.
a) Behandlung mit Sulfurylchlorid; b) Reduktion.

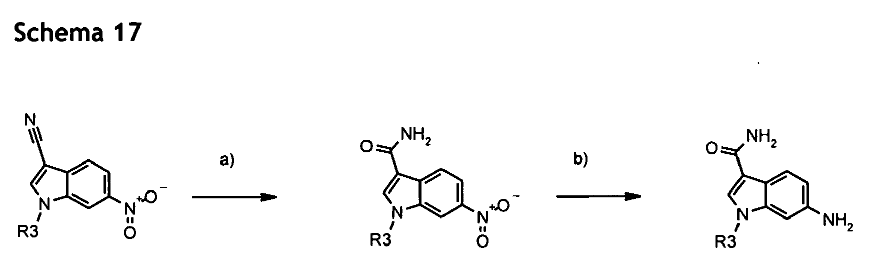

***Schema 17.*** Übersicht zur Synthese von Aminoverbindungen der allgemeinen Formel ( IV ), wobei R3 der Definition in der Beschreibung und den Ansprüchen entspricht.
a) Säure katalysierte Hydrolyse; b) Hydrierung.

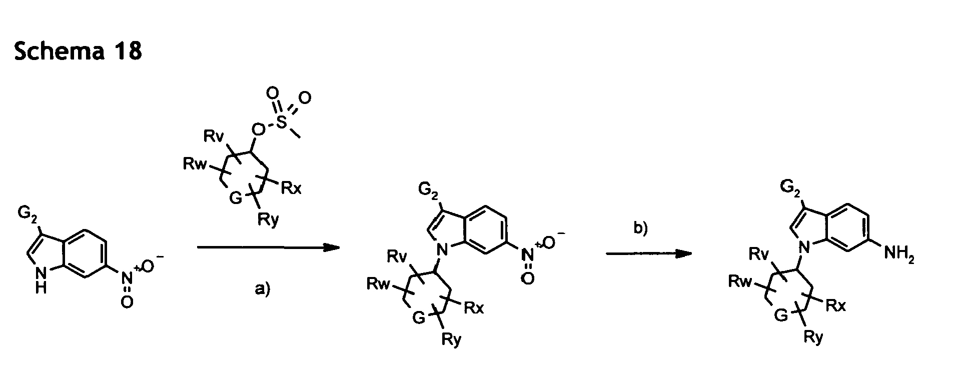

***Schema 18.*** Übersicht zur Synthese von Aminoverbindungen der allgemeinen Formel ( IV ), wobei G für -O-, -(CH₂)- oder für ein mit *t*BuOCO- substituiertes *N*-Atom steht. G2 entspricht H oder C₁-C₆-alkyl. Rv, Rw, Rx und Ry stehen unabhängig voneinander für H oder C₁-C₆-alkyl oder zwei von diesen Substituenten gemeinsam stehen für -(CH₂)ₙ- (mit n= 1-3).
a) Substitution; b) Hydrierung.

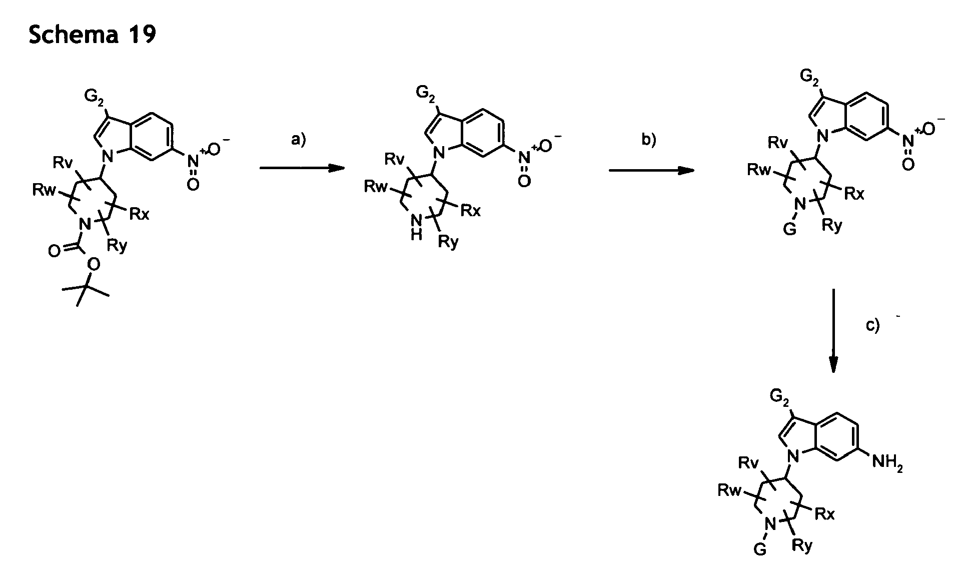

***Schema 19.*** Übersicht zur Synthese von Aminoverbindungen der allgemeinen Formel ( IV ), wobei G C₁-C₆-alkyl, C₁-C₆-haloalkyl, C₁-C₆-alkoxy-C₁-C₆-alkyl, ha[O-C₁-C₆-alkoxy-C₁-C₆-alkyl, C₂-C₆-alkenyl, C₂-C₆-alkinyl, C₃-C₁₀-cycloalkyl, C₃-C₁₀-heterocycloalkyl, R8, -(CH₂)ₘ-C₆-C₁₀-aryl, oder -(CH₂)ₙheteroaryl entspricht, wobei das C₁-C₆-alkyl mit -C(O)NH2, -COOCH₃, -C(O)R8 oder -C(O)NR4R5 substituiert sein kann. G2 entspricht H oder C₁-C₆-alkyl. Rv, Rw, Rx und Ry stehen unabhängig voneinander für H oder C₁-C₆-alkyl oder zwei von diesen Substituenten gemeinsam stehen für -(CH₂)ₙ- (mit n= 1-3).
a) Entschützung; b) Reduktive Aminierung oder Alkylierung; c) Hydrierung oder Reduktion.

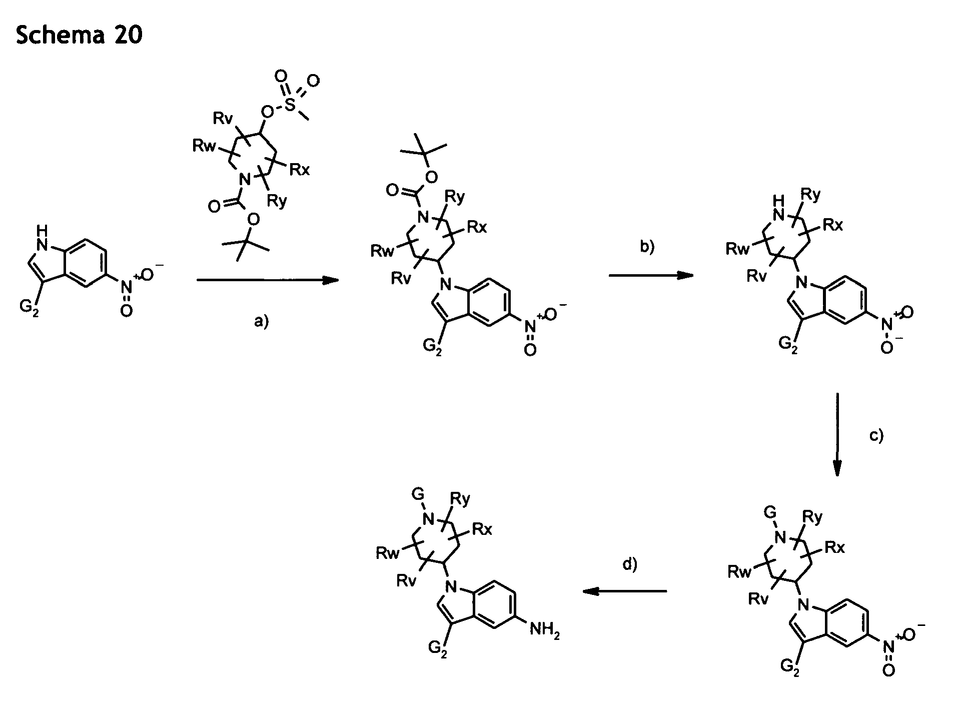

***Schema 20**.* Übersicht zur Synthese von Aminoverbindungen der allgemeinen Formel ( IV ), wobei G C₁-C₆-alkyl, C₁-C₆-haloalkyl, C₁-C₆-alkoxy-C₁-C₆-alkyl, halo-C₁-C₆-alkoxy-C₁-C₆-alkyl, C₂-C₆-alkenyl, C₂-C₆-alkinyl, C₃-C₁₀-cycloalkyl, C₃-C₁₀-heterocycloalkyl, -(CH₂)ₘ-C₆-C₁₀-aryl, oder -(CH₂)ₙheteroaryl entspricht, wobei das C₁-C₆-alkyl mit -C(O)NH2, -COOCH₃, oder -C(O)NR4R5 substituiert sein kann. G2 entspricht H oder C₁-C₆-alkyl. Rv, Rw, Rx und Ry stehen unabhängig voneinander für H oder C₁-C₆-alkyl, oder zwei von diesen Substituenten gemeinsam stehen für -(CH₂)ₙ- (mit n= 1-3).
a) Substitution; b) Entschützung; c) Reduktive Aminierung oder Alkylierung; d) Hydrierung oder Reduktion.

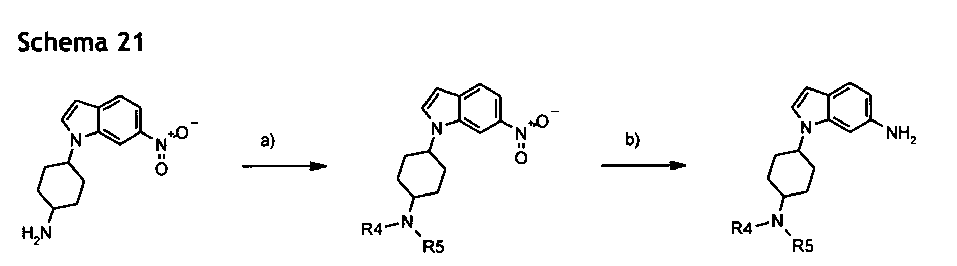

***Schema 21**.* Übersicht zur Synthese von Aminoverbindungen der allgemeinen Formel ( IV ), wobei R4 und R5 den Definitionen in der Beschreibung und den Ansprüchen entsprechen.
a) Zweimalige Reduktive Aminierung; b) Hydrierung.

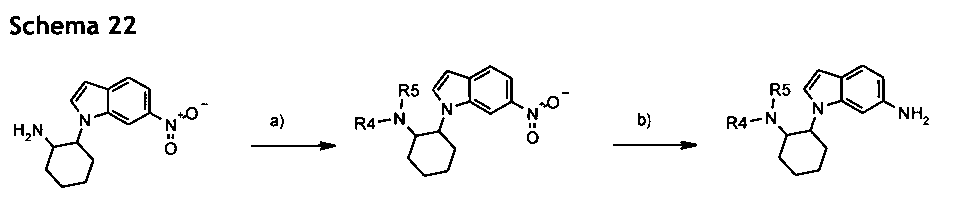

***Schema 22.*** Übersicht zur Synthese von Aminoverbindungen der allgemeinen Formel ( IV ), wobei R4 und R5 den Definitionen in der Beschreibung und den Ansprüchen entsprechen.
a) Zweimalige Reduktive Aminierung; b) Hydrierung.

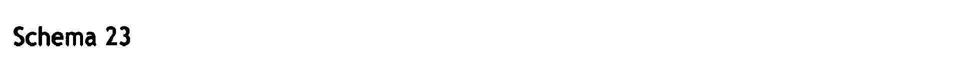

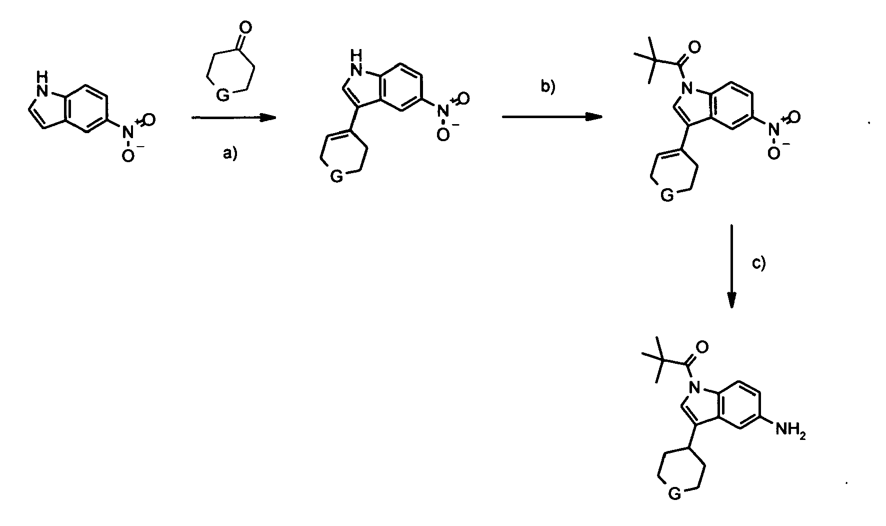

***Schema 23.*** Übersicht zur Synthese von Aminoverbindungen der allgemeinen Formel ( IV ), wobei G einem *N*-Atom, das mit C₁-C₆-alkyl, C₁-C₆-haloalkyl, C₁-C₆-alkoxy-C₁-C₆-alkyl, C₃-C₁₀-cycloalkyl, -C(O)R7 oder -C(O)₂R7 substituiert ist, entspricht.
a) Säure katalysierte Kondensation; b) Acylierung; c) Hydrierung.

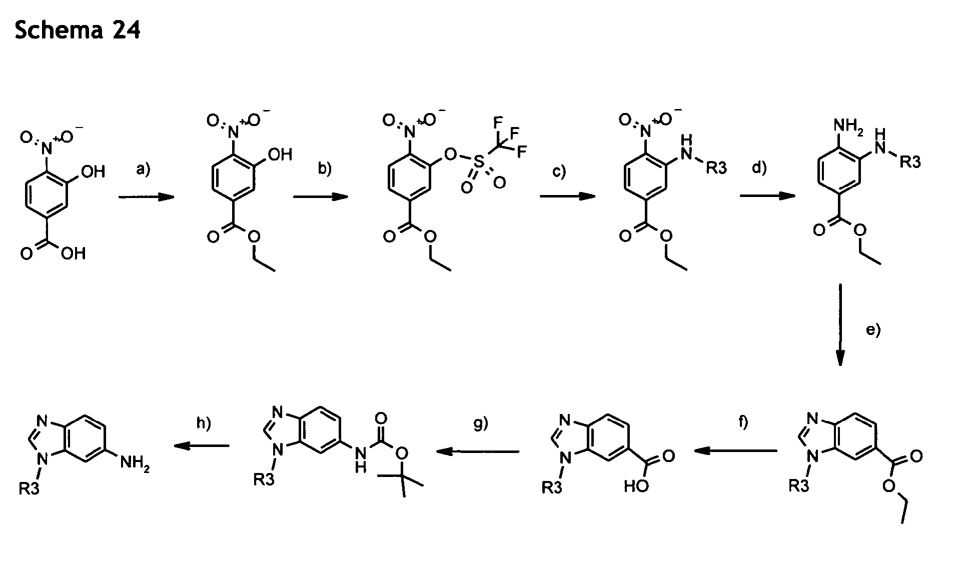

***Schema 24**.* Übersicht zur Synthese von Aminoverbindungen der allgemeinen Formel ( IV ), wobei R3 der Definition in der Beschreibung und den Ansprüchen entspricht.
a) Veresterung; b) Alkohol zu dem Triflat umsetzen; c) Palladium katalysierte Aminierung; d) Hydrierung; e) Säurekatalysierte Umsetzung mit einem Orthoester; f) Esterhydrolyse; g) Säureabbau zum Boc-geschützten Amin; h) Entschützung.

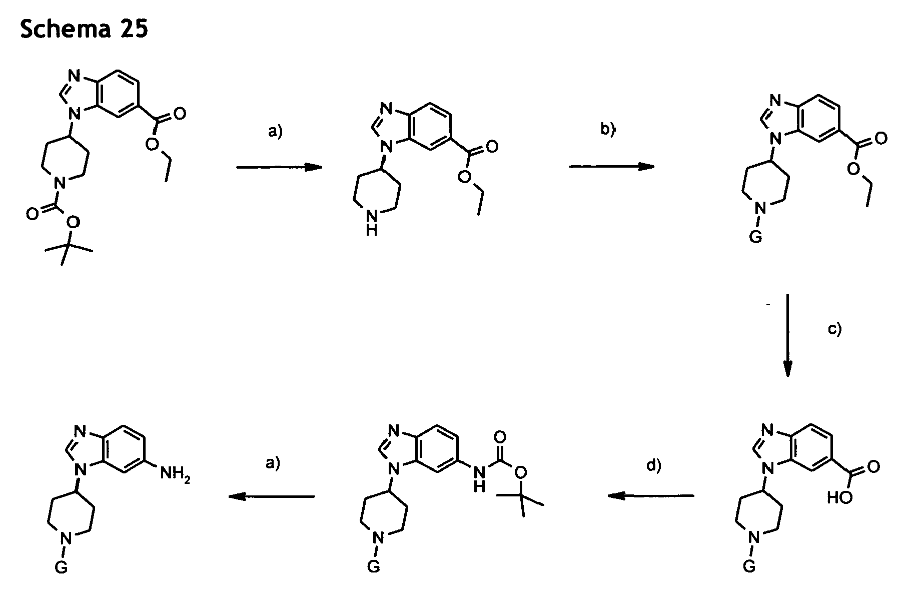

***Schema 25**.* Übersicht zur Synthese von Aminoverbindungen der allgemeinen Formel ( IV ), wobei G C₁-C₆-alkyl, C₁-C₆-haloalkyl, C₁-C₆-alkoxy-C₁-C₆-alkyl, halo-C₁-C₆-alkoxy-C₁-C₆-alkyl, C₂-C₆-alkenyl, C₂-C₆-alkinyl, C₃-C₁₀-cycloalkyl, C₃-C₁₀-heterocycloalkyl, R8, -(CH₂)ₘ-C₆-C₁₀-aryl, oder -(CH₂)ₙheteroaryl entspricht.
a) Entschützung; b) Reduktive Aminierung; c) Esterhydrolyse; d) Säureabbau zum Boc-geschützten Amin.

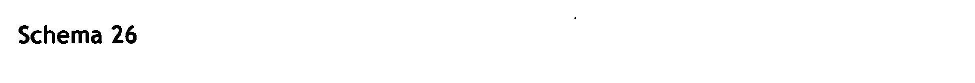

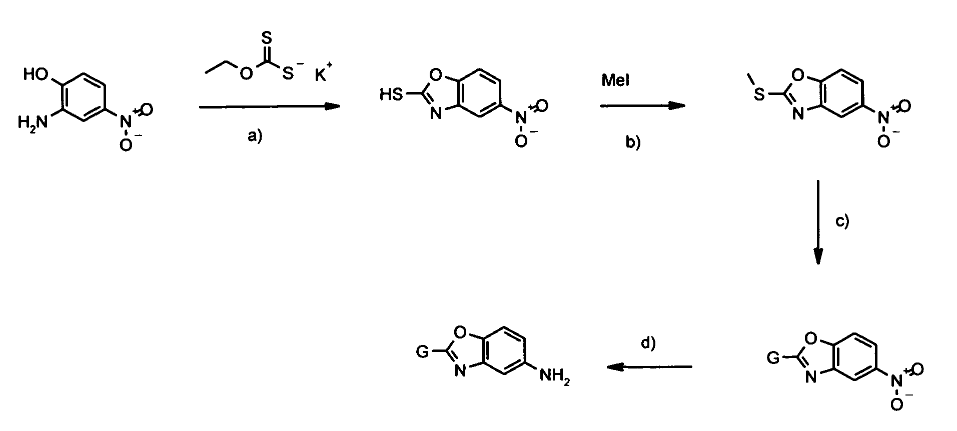

***Schema 26**.* Übersicht zur Synthese von Aminoverbindungen der allgemeinen Formel ( IV ), wobei G R8 oder -NR4R5 entspricht und R8, R4 und R5 den Definitionen in der Beschreibung und den Ansprüchen entsprechen.
a) Cyctisierung; b) Alkylierung; c) Substitution; d) Hydrierung.

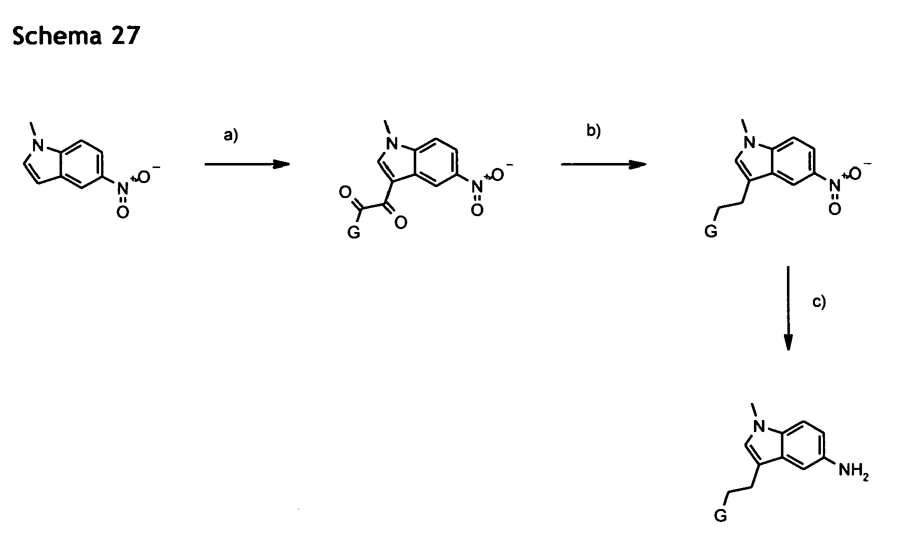

***Schema 27.*** Übersicht zur Synthese von Aminoverbindungen der allgemeinen Formel ( IV ), wobei G R8 oder -NR4R5 entspricht und R8, R4 und R5 den Definitionen in der Beschreibung und den Ansprüchen entsprechen. a) Sequenzielle Umsetzung mit Oxalylchlorid und einem Amin; b) Reduktion; c) Hydrierung.

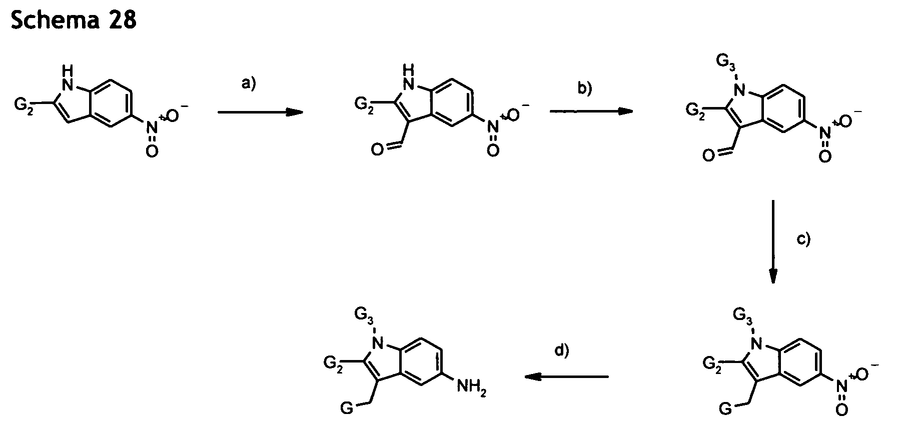

***Schema 28**.* Übersicht zur Synthese von Aminoverbindungen der allgemeinen Formel ( IV ), wobei G3 C₁-C₆-alkyl entspricht. G entspricht R8 oder -NR4R5. G2 entspricht H oder C₁-C₆-alkyl.
a) Formylierung; b) Alkylierung; c) Reduktive Aminierung; d) Hydrierung.

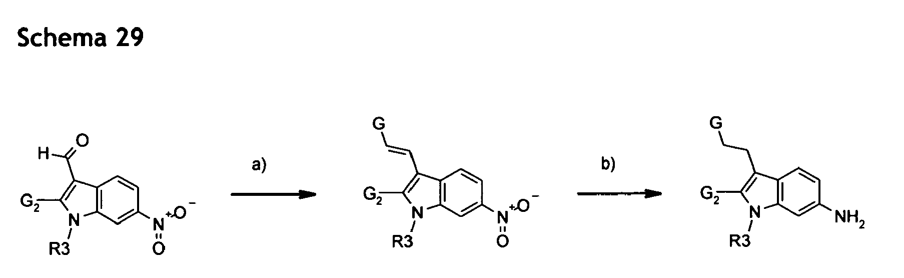

***Schema* 29.** Übersicht zur Synthese von Aminoverbindungen der allgemeinen Formel ( IV ), wobei R3 der Definition in der Beschreibung und den Ansprüchen entspricht. G entspricht einem H-Atom, C₁-C₆-alkyl, C₃-C₁₀-cycloalkyl, R8, -(CH₂)ₘ-C₆-C₁₀-aryl, oder - (CH₂)ₙheteroaryl. G2 entspricht H oder C₁-C₆-alkyl.
a) Wittig-Reaktion; b) Hydrierung.

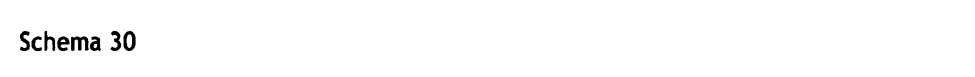

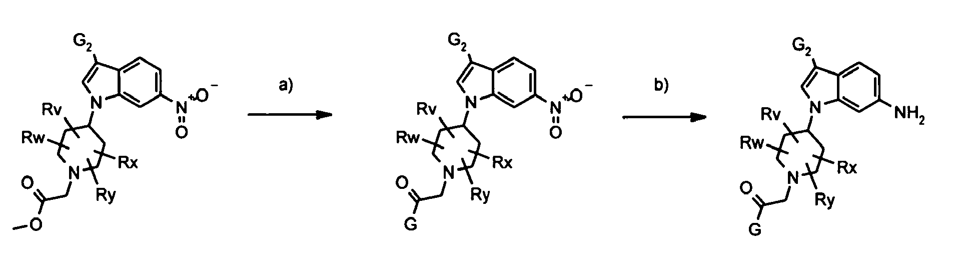

***Schema 30.*** Übersicht zur Synthese von Aminoverbindungen der allgemeinen Formel ( IV ), wobei G R8, -NH₂ oder -NR4R5 entspricht und R8, R4 und R5 den Definitionen in der Beschreibung und den Ansprüchen entsprechen. G2 entspricht H oder C₁-C₆-alkyl. Rv, Rw, Rx und Ry stehen unabhängig voneinander für H oder C₁-C₆-alkyl, oder zwei von diesen Substituenten gemeinsam stehen für -(CH₂)ₙ- (mit n= 1-3).
a) Umsetzung mit einem Amin oder mit Ammoniak; b) Hydrierung.

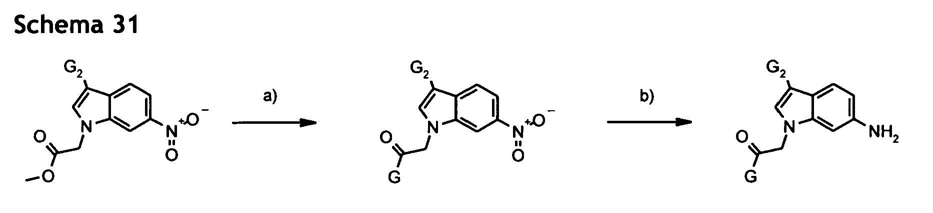

***Schema 31.*** Übersicht zur Synthese von Aminoverbindungen der allgemeinen Formel ( IV ), wobei G R8, -NH₂ oder -NR4R5 entspricht und R8, R4 und R5 den Definitionen in der Beschreibung und den Ansprüchen entsprechen. G2 entspricht H oder C₁-C₆-alkyl.
a) Umsetzung mit einem Amin oder mit Ammoniak; b) Hydrierung.

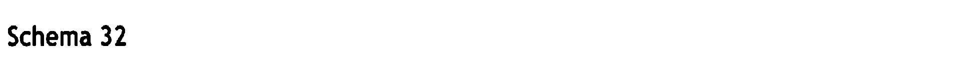

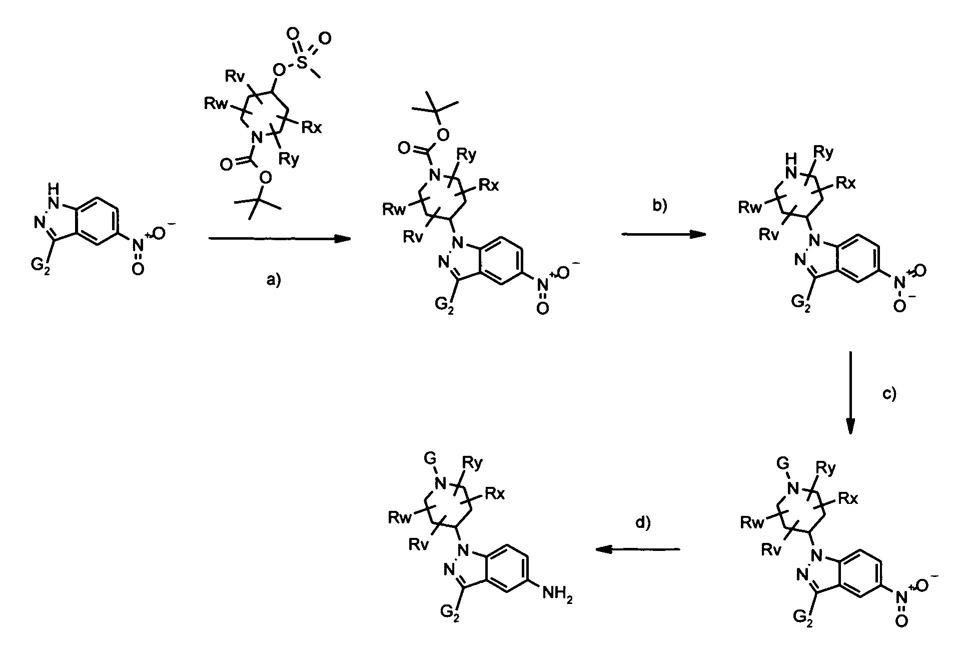

***Schema 32.*** Übersicht zur Synthese von Aminoverbindungen der allgemeinen Formel ( IV ), wobei G2 H oder C₁-C₆-alkyl entspricht. Rv, Rw, Rx und Ry stehen unabhängig voneinander für H oder C₁-C₆-alkyl oder zwei von diesen Substituenten gemeinsam stehen für -(CH₂)ₙ- (mit n= 1-3).
a) Substitution; b) Entschützung; c) Reduktive Aminierung oder Alkylierung; d) Hydrierung oder Reduktion.

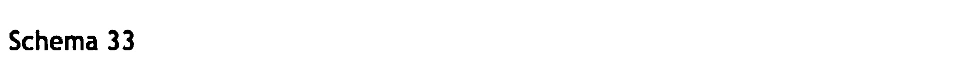

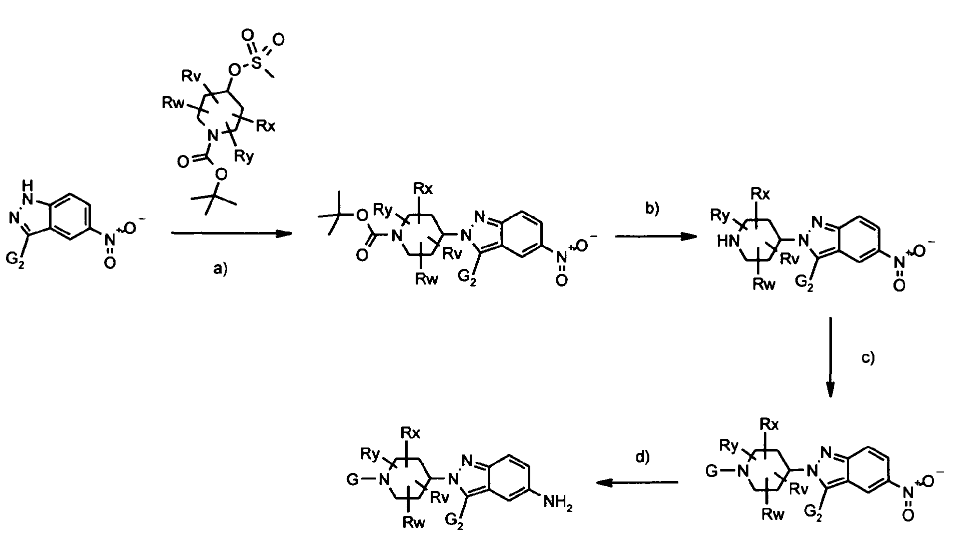

***Schema 33**.* Übersicht zur Synthese von Aminoverbindungen der allgemeinen Formel ( IV ), wobei G2 H oder C₁-C₆-alkyl entspricht. Rv, Rw, Rx und Ry stehen unabhängig voneinander für H oder C₁-C₆-alkyl, oder zwei von diesen Substituenten gemeinsam stehen für -(CH₂)ₙ- (mit n= 1-3).
a) Substitution; b) Entschützung; c) Reduktive Aminierung oder Alkylierung; d) Hydrierung oder Reduktion.

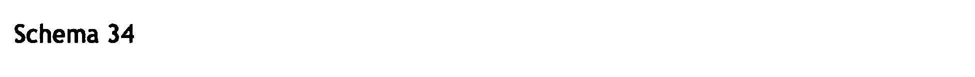

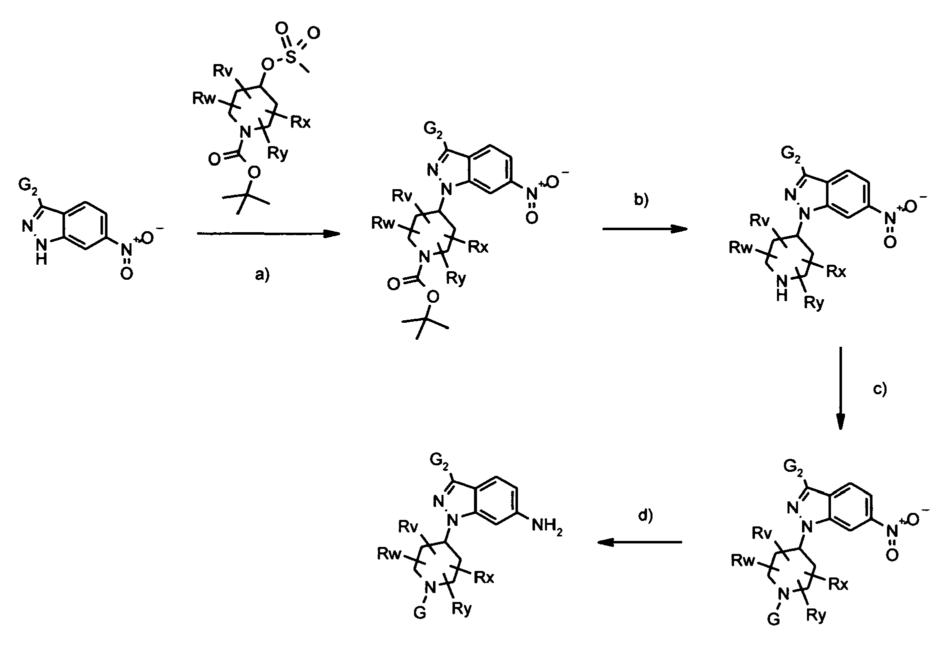

***Schema 34***. Übersicht zur Synthese von Aminoverbindungen der allgemeinen Formel ( IV ), wobei G2 H oder C₁-C₆-alkyl entspricht. Rv, Rw, Rx und Ry stehen unabhängig voneinander für H oder C₁-C₆-alkyl, oder zwei von diesen Substituenten gemeinsam stehen für -(CH₂)ₙ- (mit n= 1-3).
a) Substitution; b) Entschützung; c) Reduktive Aminierung oder Alkylierung; d) Hydrierung oder Reduktion.

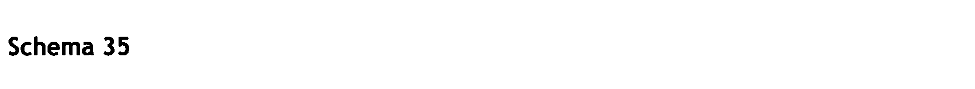

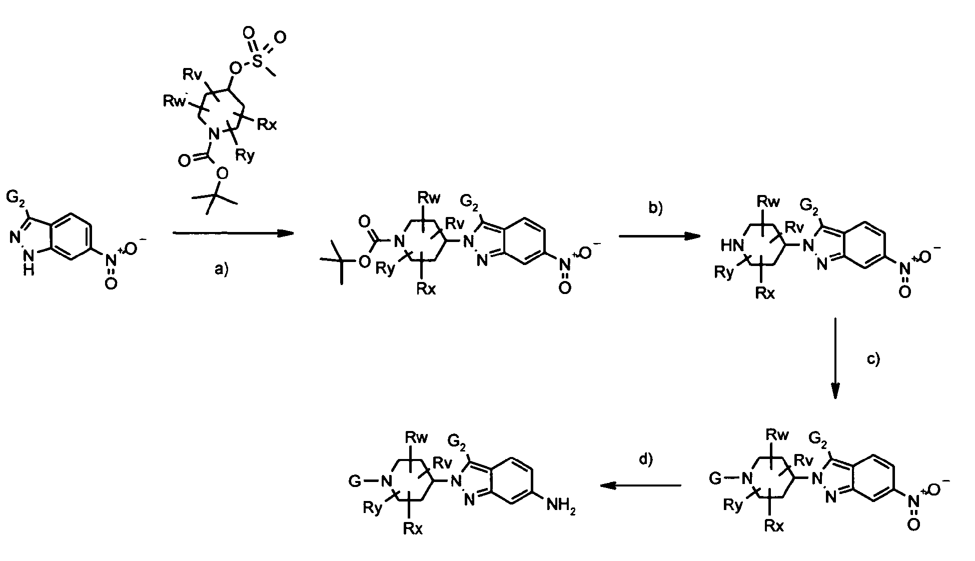

***Schema 35.*** Übersicht zur Synthese von Aminoverbindungen der allgemeinen Formel ( IV ), wobei G2 H oder C₁-C₆-alkyl entspricht. Rv, Rw, Rx und Ry stehen unabhängig voneinander für H oder C₁-C₆-alkyl, oder zwei von diesen Substituenten gemeinsam stehen für -(CH₂)ₙ- (mit n= 1-3).
a) Substitution; b) Entschützung; c) Reduktive Aminierung oder Alkylierung; d) Hydrierung oder Reduktion.

### EXPERIMENTELLER TEIL

### 1. Synthese von Anilin-Bausteinen

### Intermediat INT1

### 1-Methyl-5-nitro-1H-indol

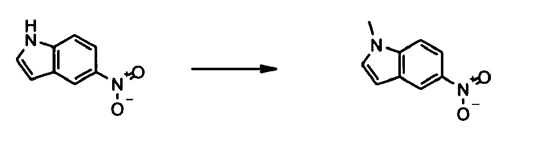

1 g 6-Nitro-1*H*-indol wird in 7 ml *N,N*-Dimethylformamid gelöst und nacheinander mit 1,1 g Kaliumcarbonat und 0,37 ml Methyliodid versetzt und 15 Stunden bei Raumtemperatur gerührt. Es werden weitere 0,1 ml Methyliodid zugegeben und 24 Stunden bei Raumtemperatur gerührt. Die Reaktionsmischung wird auf 500 ml Wasser gegossen, und der ausgefallene Feststoff wird abfiltriert. Es werden 1,0 g der Titelverbindung als Feststoff erhalten.
¹H-NMR, 300 MHz, (DMSO-*d*₆): δ = 3,89 (s, 3H); 6,74 (d, 1 H); 7,60 (d, 1 H); 7,65 (d, 1 H); 8,05 (dd, 1 H); 8,57 (d, 1 H) ppm.

### Intermediat INT2

### 1-Methyl-3-(1-methyl-1,2,3,6-tetrahydro-4-pyridinyl)-5-nitro-1H-indol

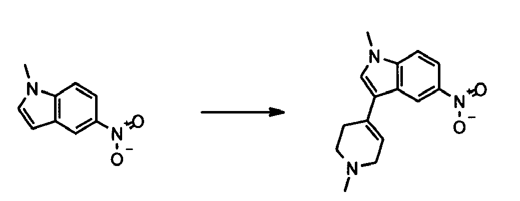

2,0 g der als **INT1** beschriebenen Verbindung werden bei 120°C zu einer Lösung von 5,0 ml *N*-Methyl-4-piperidon in 30 ml Essigsäure und 20 ml Trifluoressigsäure gegeben und es wird zwei Stunden bei 120°C gerührt. Die Reaktionsmischung wird eingeengt und nach Umkristallisieren aus Aceton werden 3,1 g der Titelverbindung als Salz erhalten.
¹H-NMR, 400 MHz, (DMSO-*d*₆): δ = 2,72-2,82 (m, 2H); 2,89 (s, 3H); 3,21-3,35 (m, 1H); 3,55-3,67 (m, 1H); 3,71-3,90 (m, 4H); 3,96-4,11 (m, 1H); 6,21 (s, b, 1H); 7,67 (d, 1H); 7,80 (s, 1 H); 8,08 (dd, 1 H); 8,70 (d, 1 H); 9,88 (s, b, 1 H) ppm.

### Intermediat INT3

### 1-Methyl-3-(1-methyl-4-piperidinyl)-1H-indol-5-amin

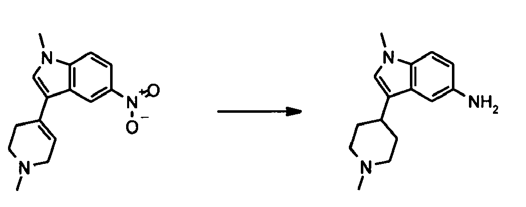

2,2 g der als **INT2** beschriebenen Verbindung werden in 37 ml Ethanol und 6,5 ml Essigsäure gelöst, und mit 184 mg Platin(IV)oxid versetzt. Es wird vier Stunden unter Wasserstoffatmosphäre bei Raumtemperatur gerührt. Die Reaktionsmischung wird über Kieselgur filtriert und das Lösungsmittel wird am Rotationsverdampfer abkondensiert. Der Rückstand wird in Dichlormethan gelöst, mit 5%iger wässriger Natronlauge gewaschen, über Natriumsulfat getrocknet und nach Abkondensieren des Lösungsmittels am Rotationsverdampfer werden 1,25 g der Titelverbindung erhalten.
¹H-NMR, 400 MHz, (DMSO-*d*₆): δ = 1,51-1,69 (m, 2H); 1,76-1,88 (m, 2H); 1,89-1,99 (m, 2H); 2,15 (s, 3H); 2,48-2,56 (m, 1H); 2,75-2,85 (m, 2H); 3,56 (s, 3H); 4,42 (s, b, 2H); 6,47 (dd, 1 H); 6,67 (d, 1 H); 6,80 (s, 1 H); 7,00 (d, 1 H) ppm.

### Intermediat INT4

### 1-Methyl-6-nitro-1H-indol

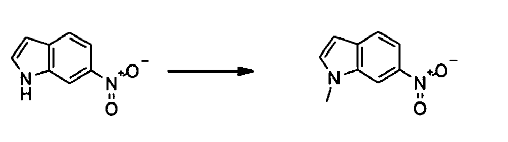

1,0 g 6-Nitro-1*H*-indol wird in 7 ml *N,N*-Dimethylformamid gelöst und nacheinander mit 1,1 g Kaliumcarbonat und 0,37 ml Methyliodid versetzt und 15 Stunden bei Raumtemperatur gerührt. Es werden weitere 0,1 ml Methyliodid zugegeben und 24 Stunden bei Raumtemperatur gerührt. Die Reaktionsmischung wird auf 500 ml Wasser gegossen, und der ausgefallene Feststoff wird abfiltriert. Es werden 1,0 g der Titelverbindung als Feststoff erhalten.
¹H-NMR, 300 MHz, (CDCl₃): δ = 3,09 (s, 3H); 6,62 (d, 1 H); 7,38 (d, 1 H); 7,68 (d, 1 H); 8,04 (dd, 1 H); 8,34 (d, 1 H) ppm.

### Intermediat INT5

### 1-Methyl-6-nitro-3-(1,2,3,6-tetrahydro-1-methyl-4-pyridinyl)-1H-indol

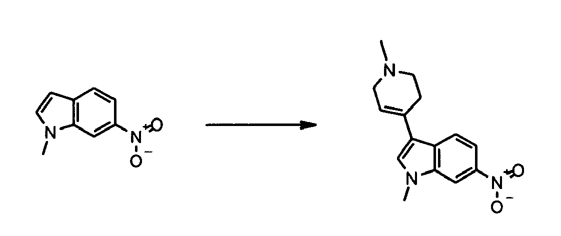

200 mg der als **INT4** beschriebenen Verbindung werden bei 120°C zu einer Lösung von 0,5 ml N-Methyl-4-piperidon in 3,1 ml Essigsäure und 2,0 ml Trifluoressigsäure gegeben und es wird eine Stunde bei 120°C gerührt. Die Reaktionsmischung wird eingeengt und nach Umkristallisieren aus Essigsäureethylester werden 330 mg der Titelverbindung als Salz erhalten.
¹H-NMR, 300 MHz, (DMSO-*d*₆): δ = 2,74-2,86 (m, 2H); 2,91 (s, 3H); 3,42-3,54 (m, 2H); 3,81-4,00 (m, 5H); 6,24 (t, b, 1H); 7,92-8,01 (m, 2H); 8,04 (d, 1H); 8,50 (d, 1H); 10,0-10,35 (b, 1 H) ppm.

### Intermediat INT6

### 1-Methyl-3-(1-methyl-4-piperidinyl)-1H-indol-6-amin

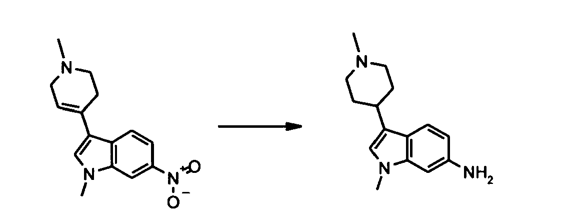

330 mg der als **INT5** beschriebenen Verbindung werden in 50 ml Ethanol und 1,0 ml Essigsäure gelöst, und mit 28 mg Platin(IV)oxid versetzt. Es wird 15 Stunden unter Wasserstoffatmosphäre bei Raumtemperatur gerührt. Die Reaktionsmischung wird über Kieselgur filtriert und das Lösungsmittel wird am Rotationsverdampfer abkondensiert. Der Rückstand wird in Dichlormethan gelöst, mit 5%iger wässriger Natronlauge gewaschen, über Natriumsulfat getrocknet und das Lösungsmittel wird am Rotationsverdampfer abkondensiert. Nach Filtration durch Aminophasenkieselgel werden 200 mg der Titelverbindung erhalten.
¹H-NMR, 300 MHz, (CDCl₃): δ = 1,73-1,92 (m, 2H); 1,98-2,21 (m, 4H); 2,37 (s, 3H); 2,66-2,84 (m, 1H); 2,94-3,07 (m, 2H); 3,59-3,72 (m, 5H); 6,50-6,71 (m, 3H); 7,44 (d, 1H) ppm.

### Intermediat INT7

### 1-Methyl-6-nitro-3-[1,2,3,6-tetrahydro-1-(2-methylpropyl)-4-pyridinyl]-1H-indol

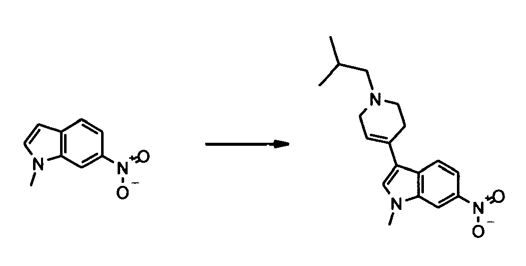

Bei der Umsetzung von 390 mg der als **INT4** beschriebenen Verbindung mit 1,16 ml N-Isobutyl-4-piperidon gemäß der bei **INT5** beschriebenen Vorschrift werden nach Reinigung durch Chromatographie an Aminophasenkieselgel 350 mg der Titelverbindung erhalten.
¹H-NMR, 300 MHz, (CDCl₃): δ = 0,96 (d, 6H); 1,80-1,98 (m, 1H); 2,24 (d, 2H); 2,52-2,64 (m, 2H); 2,69 (t, 2H); 3,11-3,22 (m, 2H); 3,87 (s, 3H); 6,07-6,24 (m, 1H); 7,28 (s, 1H); 7,90 (d, 1 H); 8,01 (dd, 1 H); 8,27 (d, 1 H) ppm.

### Intermediat INT8

### 1-Methyl-3-[1-(2-methylpropyl)-4-piperidinyl]-1H-indol-6-amin

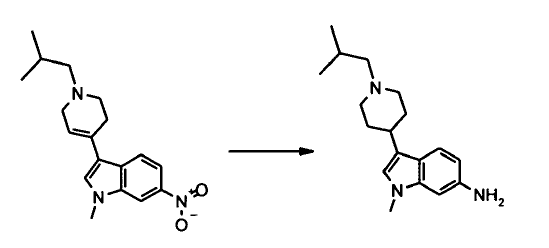

Bei der Umsetzung von 350 mg der als **INT7** beschriebenen Verbindung gemäß der bei **INT6** beschriebenen Vorschrift werden 190 mg der Titelverbindung erhalten.
¹H-NMR, 300 MHz, (DMSO-*d*₆): δ = 0,87 (d, 6H); 1,51-1,70 (m, 2H); 1,70-2,12 (m, 7H); 2,52-2,68 (m, 1H); 2,80-2,92 (m, 2H); 3,53 (s, 3H); 4,75 (s, b, 2H); 6,38 (dd, 1H); 6,44 (d, 1H); 6,69 (s, 1H); 7,18 (d, 1H) ppm.

### Intermediat INT9

### 6-Nitro-1-[2-(1-Pyrrolidinyl)ethyl]-1H-indol

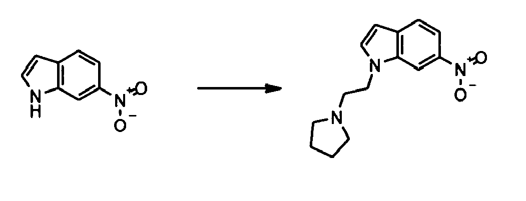

1,91 g Natriumhydrid in Öl (60%ig w/w) werden zweimal mit je 100 ml Hexan gewaschen, und mit 10 ml DMF versetzt. 0,8 g 6-Nitro-1*H*-indol, gelöst in 3 ml DMF werden bei 0°C zugetropft. Es wird zwei Stunden bei Raumtemperatur gerührt. Dann wird eine Lösung von 1-(2-Chlorethyl)pyrrolidin in Toluol bei 0°C zugetropft, die frisch hergestellt wurde aus 1,66 g 1-(2-Chlorethyl)pyrrolidiniumchlorid durch Zugabe von 10 ml 40%iger Natronlauge und Extraktion mit 40 ml Toluol. Es wird über Nacht bei Raumtemperatur gerührt. Dann wird erneut eine Lösung von 1-(2-Chlorethyl)pyrrolidin in Toluol (hergestellt aus 0,83 g 1-(2-Chlorethyl)pyrrolidiniumchlorid, wie oben beschrieben) zugegeben und es wird weitere 60 Stunden bei 50°C gerührt. Das Reaktionsgemisch wird auf 5°C abgekühlt tropfenweise mit 80 ml Wasser versetzt und mit einem Gemisch aus Essigsäureethylester und Hexan (200 ml + 200 ml) extrahiert. Die organische Phase wird nacheinander mit 200 ml halbgesättigter Natriumhydrogencarbonatlösung, und 100 ml gesättigter Kochsalzlösung gewaschen und über Natriumsulfat getrocknet. Nach Reinigung durch Chromatographie an Kieselgel und anschließend an Aminophasenkieselgel werden 1,0 g der Titelverbindung erhalten.
¹H-NMR, 300 MHz, (CDCl₃): δ = 1,74-1,86 (m, 4H); 2,45-2,66 (m, 4H); 2,91 (t, 2H); 4,33 (t, 2H); 6,58 (d, 1 H); 7,45 (d, 1 H); 7,63 (d, 1 H); 7,99 (dd, 1 H); 8,36 (d, 1 H) ppm.

### Intermediat INT10

### 1-[2-(1-Pyrrolidinyl)ethyl]-1H-indol-6-amin

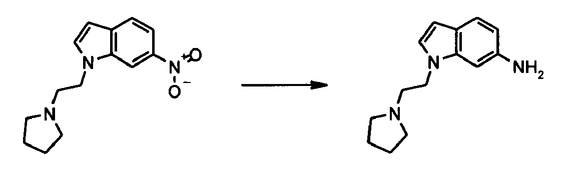

200 mg der als **INT9** beschriebenen Verbindung werden in 100 ml Ethanol gelöst, und mit 5 mg Raney-Nickel versetzt. Es wird 2 Stunden unter Wasserstoffatmosphäre bei Raumtemperatur gerührt. Die Reaktionsmischung wird über Kieselgur filtriert und das Lösungsmittel wird am Rotationsverdampfer abkondensiert. Nach Reinigung durch Chromatographie an Aminophasenkieselgel werden 90 mg der Titelverbindung erhalten.
¹H-NMR, 300 MHz, (CDCl₃): δ = 1,76-1,92 (m, 4H); 2,54-2,68 (m, 4H); 2,90 (t, 2H); 3,05-3,95 (b, 2H); 4,21 (t, 2H); 6,40 (d, 1 H); 6,60 (dd, 1 H); 6,70 (d, 1 H); 6,97 (d, 1 H); 7,43 (d, 1 H) ppm.

### Intermediat INT11

### 1-(2-Methoxyethyl)-5-nitro-1H-indol

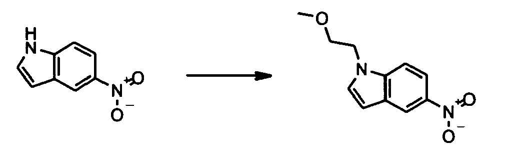

4,93 g Natriumhydrid in Öl (60%ig w/w) werden mit 100 ml Hexan gewaschen, und mit 150 ml DMF versetzt. 10,0 g 5-Nitro-1*H*-indol, gelöst in 17 ml DMF werden bei 0°C zugetropft. Es wird zwei Stunden bei Raumtemperatur gerührt. Dann werden 11,3 ml 1-Chlor-2-methoxyethan zugetropft, und es wird zwei Stunden bei 50°C und 15 Stunden bei Raumtemperatur gerührt. Das Reaktionsgemisch wird auf 5°C abgekühlt tropfenweise mit 100 ml Wasser versetzt und mit 500 ml Essigsäureethylester extrahiert. Die organische Phase wird nacheinander zweimal mit je 200 ml halbgesättigter Natriumhydrogencarbonatlösung, und mit 100 ml gesättigter Kochsalzlösung gewaschen und über Natriumsulfat getrocknet. Nach Umkristallisieren aus Ethanol werden 13,5 g der Titelverbindung erhalten.
¹H-NMR, 300 MHz, (DMSO-*d*₆): δ = 3,21 (s, 3H); 3,68 (t, 2H); 4,44 (t, 2H); 6,75 (d, 1H); 7,63 (d, 1 H); 7,71 (d, 1 H); 8,03 (dd, 1 H); 8,57 (d, 1 H) ppm.

### Intermediat INT12

### 1-(2-Methoxyethyl)-5-nitro-1H-indol-3-carbaldehyd

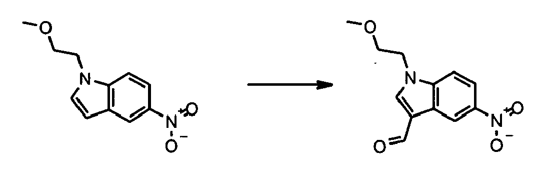

1,0 g der als **INT11** beschriebenen Verbindung werden in 12 ml N,N-Dimethylformamid gelöst und bei 0°C tropfenweise mit 0,47 ml Phosphoroxychlorid versetzt. Es wird 15 Stunden bei Raumtemperatur gerührt, die Reaktionsmischung wird auf Eiswasser gegossen und mit 2 N Natronlauge basisch gestellt. Der ausgefallene Feststoff wird abfiltriert und mit Wasser gewaschen. Der Festoff wird in 100 ml eines Gemisches aus Dichlormethan und Methanol (100 ml + 1 ml) gelöst, und über Natriumsulfat getrocknet. Nach Filtration und Abkondensieren des Lösungsmittels am Rotationsverdampfer werden 1,06 g der Titelverbindung erhalten.
¹H-NMR, 300 MHz, (DMSO-*d*₆): δ = 3,19 (s, 3H); 3,69 (t, 2H); 4,51 (t, 2H); 7,85 (d, 1H); 8,14 (dd, 1 H); 8,52 (s, 1 H); 8,90 (d, 1 H); 9,97 (s, 1 H) ppm.

### Intermediat INT13

### 1-(2-Methoxyethyl)-5-nitro-1H-indol-3-carbonsäure

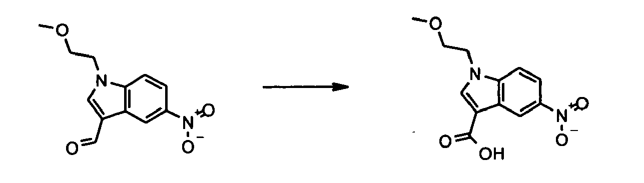

1,05 g der als **INT12** beschriebenen Verbindung werden in 160 ml *tert*-Butanol und 20 ml 2-Methyl-2-buten gelöst und bei 0°C tropfenweise mit einer Lösung von 3,52 g Natriumchlorit und 3,55 g Natriumdihydrogenphosphat in 40 ml Wasser versetzt. Es wird 15 Stunden bei Raumtemperatur gerührt, die Reaktionsmischung wird mit Wasser versetzt, mit Essigsäureethylester gewaschen, mit Phosphorsäure auf pH 3,5 angesäuert und mit Essigsäureethylester extrahiert. Es wird über Natriumsulfat getrocknet und nach Filtration und Abkondensieren des Lösungsmittels am Rotationsverdampfer werden 460 mg der Titelverbindung erhalten.
¹H-NMR, 300 MHz, (DMSO-*d*₆): δ = 3,22 (s, 3H); 3,71 (t, 2H); 4,52 (t, 2H); 7,85 (d, 1H); 8,13 (dd, 1 H); 8,30 (s, 1 H); 8,89 (d, 1 H); 12,59 (s, b, 1 H) ppm.

### Intermediat INT14

### 1-(2-Methoxyethyl)-3-[(4-methyl-1-piperazinyl)carbonyl]-5-nitro-1H-indol

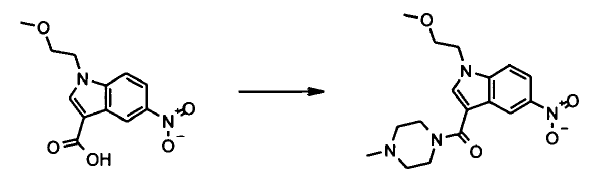

450 mg der als **INT13** beschriebenen Verbindung werden in 15 ml *N,N-*Dimethylformamid gelöst und mit 1,17 g Kaliumcarbonat und 0,38 ml N-Methylpiperidin und 1,1 g TBTU versetzt. Es wird 15 Stunden bei Raumtemperatur gerührt, die Reaktionsmischung wird mit 10 ml Wasser versetzt und mit Essigsäureethylester extrahiert. Die organische Phase wird dreimal mit halbgesättigter Kochsalzlösung gewaschen und über Natriumsulfat getrocknet. Nach Reinigung durch Chromatographie an Kieselgel werden 263 mg der Titelverbindung erhalten.
¹H-NMR, 400 MHz, (DMSO-*d*₆): δ = 2,18 (s, 3H); 2,26-2,38 (m, 4H); 3,18 (s, 3H); 3,57-3,71 (m, 6H); 4,45 (t, 2H); 7,76 (d, 1 H); 7,96 (s, 1 H); 8,05 (dd, 1 H); 8,60 (d, 1 H) ppm.

### Intermediat INT15

### 1-(2-Methoxyethyl)-3-[(4-methyl-1-piperazinyl)carbonyl]-1H-indol-5-amin

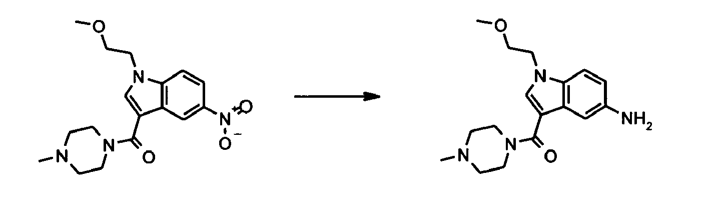

255 mg der als **INT14** beschriebenen Verbindung werden in 10 ml Ethanol gelöst, und mit 80 mg Palladium auf Kohle (10 %ig) versetzt. Es wird 15 Stunden unter Wasserstoffatmosphäre bei Raumtemperatur gerührt. Nach Filtration über Kieselgur und Abkondensieren des Lösungsmittels am Rotationsverdampfer werden 230 mg der Titelverbindung erhalten.
¹H-NMR, 400 MHz, (DMSO-*d*₆): δ = 2,22 (s, 3H); 2,31-2,43 (m,b, 4H); 3,18 (s, 3H); 3,49-3,63 (m, 6H); 4,20 (t, 2H); 4,50-5,40 (b, 2H); 6,52 (dd, 1H); 6,79 (d, 1H); 7,17 (d, 1H); 7,45 (s, 1 H) ppm.

### Intermediat INT16

### 1-(2-Methoxyethyl)-6-nitro-1H-indol

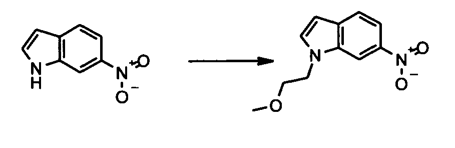

4,79 g Natriumhydrid in Öl (60%ig w/w) werden zweimal mit je 30 ml Hexan gewaschen, und mit 11 ml DMF versetzt. 2,0 g 5-Nitro-1*H*-indol, gelöst in 15 ml DMF werden bei 0°C zugetropft. Es wird zwei Stunden bei Raumtemperatur gerührt. Dann werden 2,23 ml 1-Chlor-2-methoxyethane zugetropft, und es wird 15 Stunden bei 50°C gerührt. Das Reaktionsgemisch wird auf 5°C abgekühlt tropfenweise mit 100 ml Wasser versetzt und mit einem Gemisch aus Essigsäureethylester und Hexan (150 ml + 150 ml) extrahiert. Die organische Phase wird nacheinander mit 300 ml halbgesättigter Natriumhydrogencarbonatlösung, und mit 200 ml gesättigter Kochsalzlösung gewaschen und über Natriumsulfat getrocknet. Nach Reinigung durch Chromatographie an Aminophasenkieselgel werden 2,43 g der Titelverbindung erhalten.
¹H-NMR, 300 MHz, (CDCl₃): δ = 3,37 (s, 3H); 3,78 (t, 2H); 4,42 (t, 2H); 6,64 (d, 1H); 7,50 (d, 1 H); 7,69 (d, 1 H); 8,05 (dd, 1 H); 8,40 (d, 1 H) ppm.

### Intermediat INT17

### 1-(2-Methoxyethyl)-6-nitro-1H-indol-3-carbaldehyde

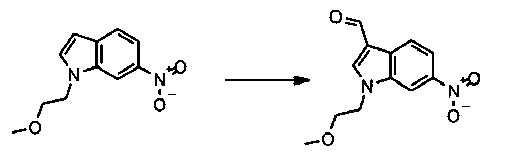

400 mg der als **INT16** beschriebenen Verbindung werden in 5 ml N,N-Dimethylformamid gelöst und bei 0°C tropfenweise mit 0,2 ml Phosphoroxychlorid versetzt. Es wird 15 Stunden bei Raumtemperatur gerührt, die Reaktionsmischung wird auf Eiswasser gegossen und mit 2 N Natronlauge basisch gestellt. Der ausgefallene Feststoff wird abfiltriert und mit Wasser gewaschen. Der Festoff wird in 50 ml eines Gemisches aus Dichlormethan und Methanol (100 ml + 1 ml) gelöst, und über Natriumsulfat getrocknet. Nach Filtration und Abkondensieren des Lösungsmittels am Rotationsverdampfer werden 410 mg der Titelverbindung erhalten.
¹H-NMR, 400 MHz, (DMSO-*d*₆): δ = 3,20 (s, 3H); 3,69 (t, 2H); 4,59 (t, 2H); 8,09 (dd, 1 H); 8,23 (d, 1 H); 8,59 (s, 1 H); 8,65 (d, 1 H); 9,96 (s, 1 H) ppm.

### Intermediat INT18

### 1-(2-Methoxyethyl)-6-nitro-1H-indol-3-carbonsäure

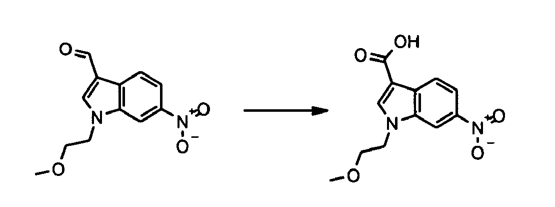

770 mg der als **INT17** beschriebenen Verbindung werden in 120 ml tert-Butanol und 15 ml 2-Metyl-2-buten gelöst und bei 0°C tropfenweise mit einer Lösung von 3,23 g Natriumchlorit und 2,61 g Natriumdihydrogenphosphat in 30 ml Wasser versetzt. Es wird 15 Stunden bei Raumtemperatur gerührt, die Reaktionsmischung wird mit 500 ml Wasser versetzt, mit gesättigter Natriumhydrogencarbonatlösung auf pH8 gebracht und zweimal mit je 400 Dichlormethan gewaschen. Die wässrige Phase wird mit 2 N Salzsäure auf pH 3,5 angesäuert und zweimal mit je 750 ml eines Gemisches aus Dichlormethan und Methanol (100:1) extrahiert. Es wird über Natriumsulfat getrocknet und nach Filtration und Abkondensieren des Lösungsmittels am Rotationsverdampfer werden 480 mg der Titelverbindung erhalten.
¹H-NMR, 300 MHz, (DMSO-*d*₆): δ = 3,19 (s, 3H); 3,66 (t, 2H); 4,55 (t, 2H); 8,03 (dd, 1 H); 8,14 (d, 1 H); 8,32 (s, 1 H); 8,60 (d, 1 H); 11,0-13,0 (b, 1 H) ppm.

### Intermediat INT19

### 1-(2-Methoxyethyl)-3-[(4-methyl-1-piperazinyl)carbonyl]-6-nitro-1H-indol

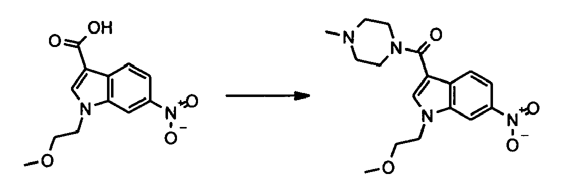

280 mg der als **INT18** beschriebenen Verbindung werden in 15 ml *N,N-*Dimethylformamid gelöst und mit 730 mg Kaliumcarbonat und 0,18 ml N-Methylpiperazin und 510 mg TBTU versetzt. Es wird 15 Stunden bei Raumtemperatur gerührt. Das Lösungsmittel wird am Rotationsverdampfer abkondensiert. Der Rückstand wird in einem Gemisch aus Chloroform und Methanol (100 ml + 10 ml) aufgenommen und mit halbgesättigter Natriumhydrogencarbonatlösung gewaschen und über Natriumsulfat getrocknet. Nach Reinigung durch Chromatographie an Kieselgel werden 250 mg der Titelverbindung erhalten.
¹H-NMR, 300 MHz, (DMSO-*d*₆): δ = 2,17 (s, 3H); 2,26-2,35 (m, 4H); 3,18 (s, 3H); 3,53-3,62 (m, 4H); 3,66 (t, 2H); 4,52 (t, 2H); 7,81 (d, 1 H); 7,97 (dd, 1 H); 8,05 (s, 1 H); 8,58 (d, 1H) ppm.

### Intermediat INT20

### 1-(2-Methoxyethyl)-3-[(4-methyl-1-piperazinyl)carbonyl]-1H-indol-6-amin

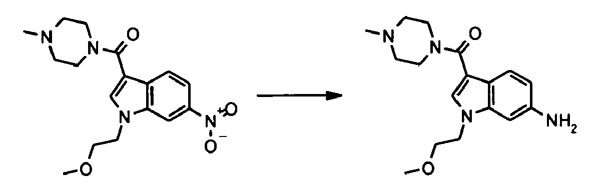

250 mg der als **INT19** beschriebenen Verbindung werden in 95 ml Ethanol gelöst, und mit 12 mg Raney-Nickel versetzt. Es wird 15 Stunden unter Wasserstoffatmosphäre bei Raumtemperatur gerührt. Die Reaktionsmischung wird über Kieselgur filtriert und das Lösungsmittel wird am Rotationsverdampfer abkondensiert. Nach Reinigung durch Chromatographie an Aminophasenkieselgel werden 230 mg der Titelverbindung erhalten.
¹H-NMR, 300 MHz, (CDCl₃), ausgewählte Signale: δ = 2,37 (s, 3H); 2,43-2,56 (m, 4H); 3,36 (s, 3H); 3,69-3,85 (m, 6H); 4,21 (t, 2H); 6,60-6,74 (m, 2H); 7,34 (s, 1H); 7,53 (dd, 1 H) ppm.

### Intermediat INT21

### 1-(2-Methoxyethyl)-3-[(4-methyl-1-piperazinyl)methyl]-6-nitro-1H-indol

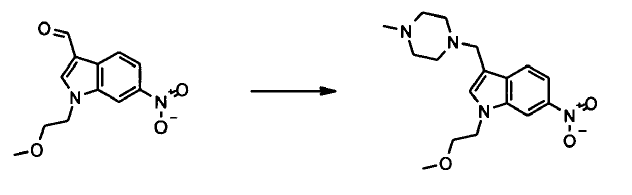

200 mg der als **INT17** beschriebenen Verbindung werden in 8,5 ml Dichlorethan gelöst und mit 0,11 ml N-Methyl-piperazin, 55 µl Eisessig und 215 mg Natriumtrisacetoxyborhydrid versetzt. Es wird 63 Stunden bei Raumtemperatur gerührt. Die Reaktionsmischung wird mit Dichlormethan versetzt, mit gesättigter Natriumhydrogencarbonatlösung gewaschen und über Natriumsulfat getrocknet. Nach Reinigung durch Chromatographie an Kieselgel werden 200 mg der Titelverbindung erhalten.
¹H-NMR, 300 MHz, (CDCl₃): δ = 1,86-2,02 (m, 2H); 2,27 (s, 3H); 2,32-2,66 (m, 6H); 3,32 (s, 3H); 3,70 (s, 2H); 3,72 (t, 2H); 4,32 (t, 2H); 7,38 (s, 1 H); 7,77 (d, 1 H); 7,99 (dd, 1 H); 8,31 (d, 1H) ppm.

### Intermediat INT22

### 1-(2-Methoxyethyl)-3-[(4-methyl-1-piperazinyl)methyl]-1 H-indol-6-amin

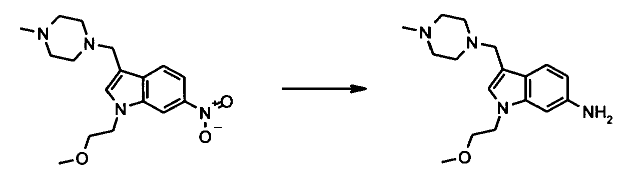

150 mg der als **INT21** beschriebenen Verbindung werden in 100 ml Ethanol gelöst, und mit 5 mg Raney-Nickel versetzt. Es wird 2 Stunden unter Wasserstoffatmosphäre bei Raumtemperatur gerührt. Die Reaktionsmischung wird über Aminophasenkieselgel filtriert und das Lösungsmittel wird am Rotationsverdampfer abkondensiert. Es werden 133 mg der Titelverbindung erhalten.
¹H-NMR, 300 MHz, (CDCl₃): δ = 2,26 (s, 3H); 2,30-2,75 (m, 8H); 3,31 (s, 3H); 3,53-3,75 (m, 6H); 4,12 (t, 2H); 6,56 (dd, 1 H); 6,60 (d, 1 H); 6,88 (s, 1 H); 7,48 (d, 1 H) ppm.

### Intermediat INT23

### 3-Chlor-1-(2-methoxyethyl)-6-nitro-1H-indol

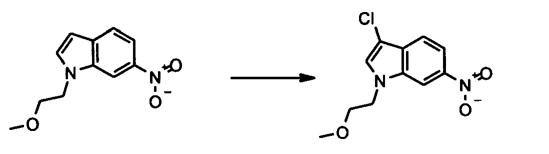

250 mg der als **INT16** beschriebenen Verbindung werden in 5 ml Acetonitril gelöst mit 182 mg *N*-Chlorsuccinimid versetzt. Es wird 15 Stunden bei Raumtemperatur gerührt. Das Lösungsmittel wird am Rotationsverdampfer abkondensiert. Nach Reinigung durch Chromatographie an Kieselgel und anschließende Umkristallisation aus Diethylether werden 150 mg der Titelverbindung erhalten.
¹H-NMR, 400 MHz, (CDCl₃): δ = 3,33 (s, 3H); 3,72 (t, 2H); 4,34 (t, 2H); 7,45 (s, 1H); 7,67 (d, 1 H); 8,06 (dd, 1 H); 8,35 (d, 1 H) ppm.

### Intermediat INT24

### 3-Chlor-1-(2-methoxyethyl)-1H-indol-6-amin

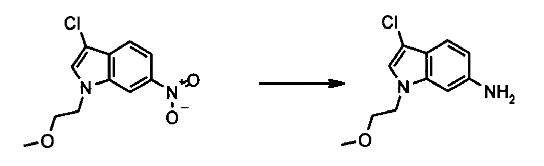

100 mg der als **INT23** beschriebenen Verbindung werden in 10 ml Tetrahydrofuran gelöst, und mit 3,1 ml einer 15%igen Titan(III)chlorid-Lösung versetzt. Es wird 15 Stunden bei Raumtemperatur gerührt. Es werden weitere 0,3 ml einer 15%igen Titan(III)chlorid-Lösung zugegeben und es wird weitere zwei Stunden bei Raumtemperatur gerührt. Die Reaktionsmischung wird mit 2 N Natronlauge basisch gestellt, mit gesättigter Natriumhydrogencarbonatlösung versetzt und mit Essigsäureethylester extrahiert. Die organische Phase wird über Natriumsulfat getrocknet. Nach Reinigung durch Chromatographie an Aminophasenkieselgel und anschließend an Kieselgel werden 60 mg der Titelverbindung erhalten.
¹H-NMR, 400 MHz, (DMSO-*d*₆): δ = 3,18 (s, 3H); 3,56 (t, 2H); 4,06 (t, 2H); 4,98 (s, b, 2H); 6,47 (dd, 1H); 6,52 (d, 1H); 7,00-7,10 (m, 2H) ppm.

### Intermediat INT25

### 1-(3-Chlorpropyl)-6-nitro-1H-indol

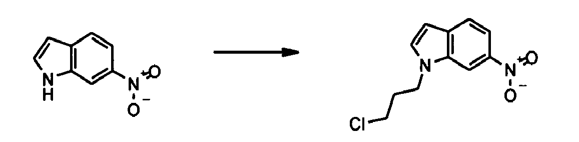

1,0 g 5-Nitro-1*H*-indol werden in 7 ml DMF gelöst, mit 1,07 g Kaliumcarbonat und 0,71 ml 1-Brom-3-chlorpropan versetzt und 30 Stunden bei Raumtemperatur gerührt. Die Reaktionsmischung wird auf 500 ml Wasser gegossen und der ausgefallene Feststoff wird abfiltriert. Der Feststoff wird in Dichlormethan gelöst, die Lösung wird mit gesättigter Kochsalzlösung gewaschen und über Natriumsulfat getrocknet. Nach Abdestillieren des Lösungsmittels am Rotationsverdampfer werden 1,4 g der Titelverbindung als Feststoff erhalten.
¹H-NMR, 300 MHz, (CDCl3): δ = 2,32 (pent., 2H); 3,47 (t, 2H); 4,45 (t, 2H); 6,62 (dd, 1 H); 7,44 (d, 1 H); 7,67 (d, 1 H); 8,02 (dd, 1 H); 8,35 (d, 1 H) ppm.

### Intermediat INT26

### 1-[3-(4-Methyl-1-piperazinyl)propyl]-6-nitro-1H-indol

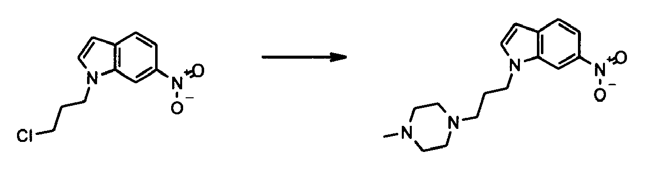

200 mg der als **INT25** beschriebenen Verbindung werden in 2 ml *N,N-*Dimethylformamid gelöst, mit 0,35 g Kaliumcarbonat, 2 mg Kaliumiodid und 0,1 ml 1-Methylpiperazin versetzt und 4 Stunden bei 70°C gerührt. Die Reaktionsmischung wird auf Eiswasser gegossen und das Produkt wird mit Dichlormethan extrahiert. Die organische Phase wird mit gesättigter Kochsalzlösung gewaschen und über Natriumsulfat getrocknet. Nach Reinigung durch Chromatographie an Aminophasenkieselgel werden 160 mg der Titelverbindung erhalten.
¹H-NMR, 400 MHz, (CDCl3): δ = 2,01 (pent., 2H); 2,24 (t, 2H); 2,33 (s, 3H); 2,35-2,73 (m, b, 8H); 4,31 (t, 3H); 6,59 (d, 1H); 7,39 (d, 1H); 7,64 (d, 1H); 7,99 (dd, 1H); 8,41 (d, 1 H) ppm.

### Intermediat INT27

### 1-[3-(4-Methyl-1-piperazinyl)propyl]-1H-indol-6-amin

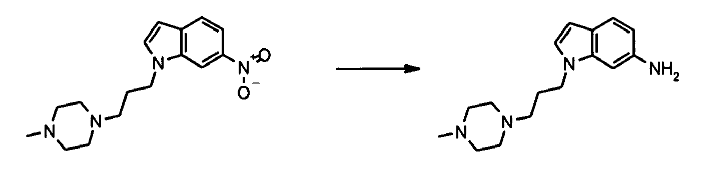

160 mg der als **INT26** beschriebenen Verbindung werden in 68 ml Ethanol gelöst, und mit 5 mg Raney-Nickel versetzt. Es wird 30 Stunden unter Wasserstoffatmosphäre bei Raumtemperatur gerührt. Die Reaktionsmischung wird über Kieselgur filtriert und das Lösungsmittel wird am Rotationsverdampfer abkondensiert. Es werden 130 mg der Titelverbindung erhalten.
¹H-NMR, 300 MHz, (CDCl3): δ = 1,95 (pent., 2H); 2,14-2,70 (m, 13H); 3,61 (s, b, 2H); 4,06 (t, 2H); 6,34 (d, 1 H); 6,54 (d, 1 H); 6,66 (s, b, 1 H); 6,90 (d, 1 H); 7,38 (d, 1 H) ppm.

### Intermediat INT28

### (E)-2-(2-Brom-4-nitrophenyl)-N,N-dimethylethenamin

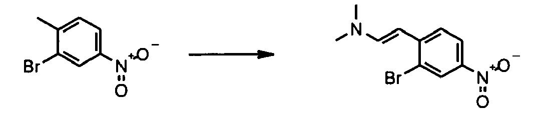

7,0 g 2-Brom-1-methyl-4-nitro-benzol, 10,1 ml Bis(dimethylamino)-*tert*-butoxymethan und 7,48 ml *N,N*-Dimethylformamid werden eine Stunde bei 100°C gerührt. Zu der erkalteten Reaktionsmischung wird Ethanol gegeben und der dunkelrote Feststoff wird abfiltriert. Es werden 8,3 g der Titelverbindung erhalten.
¹H-NMR, 300 MHz, (DMSO-*d*₆): δ = 2,94 (s, 6H); 5,23 (d, 1H); 7,59 (d, 1H); 7,63 (d, 1H); 7,89 (dd, 1 H); 8,18 (d, 1 H) ppm.

### Intermediat INT29

### 1-(1-Methyl-4-piperidinyl)-6-nitro-1H-indol

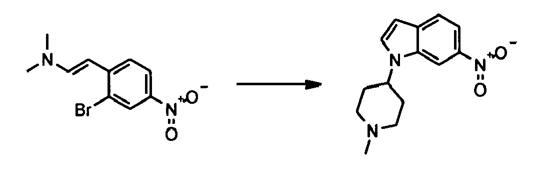

5,6 g der als **INT28** beschriebenen Verbindung, 2,73 g 4-Amino-1-methylpiperidin, 945 mg Tris(dibenzylidenaceton)dipalladium(0), 1,29 g *rac*-BINAP und 3,97 g Natrium-*tert-*butylat werden in 98 ml Toluol eine Stunde bei 110°C gerührt. Die Reaktionsmischung wird mit Wasser und Essigsäureethylester versetzt und mit vier normaler Salzsäure leicht angesäuert. Die organische Phase wird abgetrennt und die wässrige Phase wird mit ein normaler Natronlauge basisch gestellt und zweimal mit einem Gemisch aus Dichlormethan und Methanol (10:1) extrahiert. Die organische Phase wird nacheinander mit halbgesättigter Natriumhydrogencarbonatlösung und mit gesättigter Kochsalzlösung gewaschen und über Natriumsulfat getrocknet. Nach Reinigung durch Chromatographie an Kieselgel werden 3,6 g der Titelverbindung erhalten.
¹H-NMR, 300 MHz, (DMSO-*d*₆): δ = 1,81-2,08 (m, 4H); 2,09-2,25 (m, 5H); 2,78-2,92 (m, 2H); 4,50-4,66 (m, 1 H); 6,65 (d, 1 H); 7,68 (d, 1 H); 7,86 (dd, 1 H); 7,92 (d, 1 H); 8,57 (d, 1 H) ppm.

### Intermediat INT30

### 1-(1-Methyl-4-piperidinyl)-1H-indol-6-amin

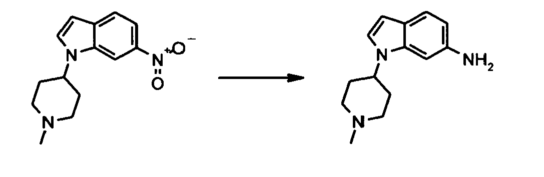

3,6 g der als **INT29** beschriebenen Verbindung werden in 80 ml Ethanol gelöst, und mit 300 mg Palladium auf Kohle (10 %ig) versetzt. Es wird 4 Stunden unter Wasserstoffatmosphäre bei Raumtemperatur gerührt. Die Reaktionsmischung wird über Kieselgur filtriert und das Lösungsmittel wird am Rotationsverdampfer abkondensiert. Nach Reinigung durch Chromatographie an Aminophasenkieselgel werden 3,15 g der Titelverbindung erhalten.
¹H-NMR, 400 MHz, (DMSO-*d*₆): δ = 1,77-1,97 (m, 4H); 1,99-2,09 (m, 2H); 2,19 (s, 3H); 2,82-2,91 (m, 2H); 3,92-4,02 (m, 1H); 4,70 (s, b, 2H); 6,15 (d, 1H); 6,36 (dd, 1H); 6,55 (d, 1H); 7,04 (d, 1H); 7,13 (d, 1H) ppm.

### Intermediat INT31

### 1-[1-(1-Methyl-4-piperidinyl)-6-nitro-1H-indol-3-yl]ethanon

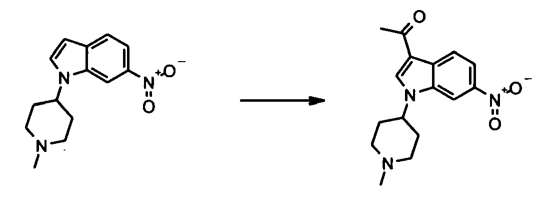

100 mg der als **INT29** beschriebenen Verbindung werden in 10 ml Dichlormethan gelöst und mit 41 µl Acetanhydrid und 10 mg Aluminium(III)chtorid versetzt. Es wird 15 Stunden bei Raumtemperatur gerührt. Die Reaktionsmischung wird mit verdünnter Natronlauge vesetzt und mit einem Gemisch aus Dichlormethan und Methanol (10:1) extrahiert. Die organische Phase wird mit gesättigter Kochsalzlösung gewaschen und über Natriumsulfat getrocknet. Nach Reinigung durch Chromatographie an Kieselgel und Umkristallisieren aus Ethanol werden 59 mg der Titelverbindung erhalten.
¹H-NMR, 400 MHz, (DMSO-*d*₆), ausgewählte Signale: δ = 1,87-1,99 (m, 2H); 2,02-2,27 (m, 7H); 2,83-2,93 (m, 2H); 4,60-4,78 (m, 1 H); 8,06 (dd, 1 H); 8,32 (d, 1 H); 8,69 (d, 1 H); 8,82 (s, 1 H) ppm.

### Intermediat INT32

### 1-[6-Amino-1-(1-methyl-4-piperidinyl)-1H-indol-3-yl]ethanon

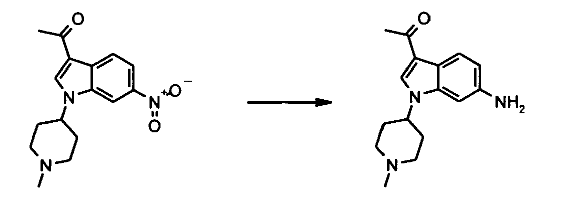

200 mg der als **INT31** beschriebenen Verbindung werden in 10 ml Ethanol gelöst, und mit 10 mg Palladium auf Kohle (10 %ig) versetzt. Es wird 15 Stunden unter Wasserstoffatmosphäre bei Raumtemperatur gerührt. Die Reaktionsmischung wird über Kieselgur filtriert und das Lösungsmittel wird am Rotationsverdampfer abkondensiert. Nach Reinigung durch Chromatographie an Aminophasenkieselgel werden 180 mg der Titelverbindung erhalten.
¹H-NMR, 300 MHz, (DMSO-*d*₆), ausgewählte Signale: δ = 1,95-2,30 (m, 4H); 2,39 (s, 3H); 3,10-3,23 (m, 2H); 4,17-4,33 (m, 1H); 6,57 (dd, 1H); 6,69 (d, 1H); 7,83 (d, 1H); 8,07 (s, 1 H) ppm.

### Intermediat INT33

### 1-(1-Methyl-4-piperidinyl)-6-nitro-1H-indol-3-carbaldehyd

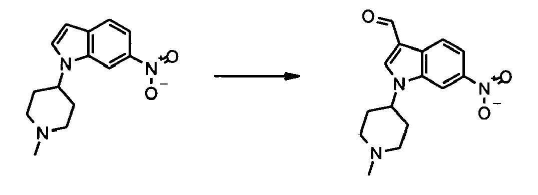

1,0 g der als **INT29** beschriebenen Verbindung werden in 10 ml *N,N*-Dimethylformamid gelöst und bei 0°C werden 0,43 ml Phosphoroxychlorid zugetropft. Es wird 15 Stunden bei Raumtemperatur gerührt. Die Reaktionsmischung wird auf Eiswasser gegossen, mit zwei normaler Natronlauge basisch gestellt und mit einem Gemisch aus Dichlormethan und Methanol (100:1) extrahiert. Die organische Phase wird mit gesättigter Natriumhydrogencarbonatlösung gewaschen und über Natriumsulfat getrocknet. Nach Reinigung durch Chromatographie an Kieselgel werden 850 mg der Titelverbindung erhalten.
¹H-NMR, 300 MHz, (DMSO-*d*₆): δ = 1,91-2,10 (m, 4H); 2,11-2,27 (m, 5H); 2,81-2,94 (m, 2H); 4,64-4,79 (m, 1H); 8,10 (dd, 1H); 8,23 (d, 1H); 8,74 (d, 1H); 8,84 (s, 1H); 9,94 (s, 1 H) ppm.

### Intermediat INT34

### 1-(1-Methyl-4-piperidinyl)-6-nitro-1H-indol-3-carbaldehyd oxim

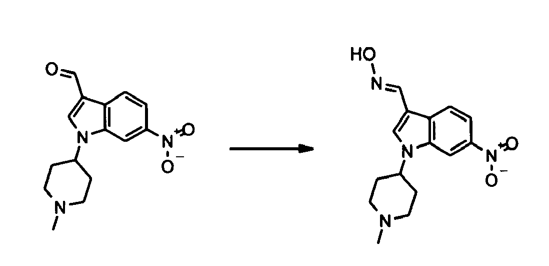

850 mg der als **INT33** beschriebenen Verbindung werden in 68 ml Ethanol gelöst, und mit 230 mg Hydroxylamin-hydrochlorid und einer Lösung von 340 mg Kaliumhydroxid in 170 ml Ethanol versetzt. Es wird zwei Stunden unter Rückfluss gerührt. Es werden weitere 50 mg Hydroxylamin Hydrochlorid zugegeben und es wird weitere zwei Stunden unter Rückfluss gerührt. Die Reaktionsmischung wird am Rotationsverdampfer zur Trockene eingeengt. Der Rückstand wird in einem Gemisch aus Dichlormethan und Methanol (10:1) gelöst und mit halbgesättigter Natriumhydrogencarbonatlösung gewaschen. Die organische Phase wird über Natriumsulfat getrocknet. Nach Reinigung durch Chromatographie an Aminophasenkieselgel werden 650 mg der Titelverbindung erhalten.
¹H-NMR, 300 MHz, (DMSO-*d*₆): δ = 1,90-2,10 (m, 4H); 2,12-2,34 (m, 5H); 2,81-2,99 (m, 2H); 4,53-4,86 (m, 1H); 7,90 (s, 1H, Isomer 1); 8,00-8,06 (m, 1H); 8,11-8,17 (m, 1H); 8,23 (s, 1 H, Isomer 2); 8,29 (s, 1 H, Isomer 2); 8,63 (s, 1 H, Isomer 1); 8,65-8,72 (m, 1 H) ppm.

### Intermediat INT35

### 1-(1-Methyl-4-piperidinyl)-6-nitro-1H-indol-3-carbonitril

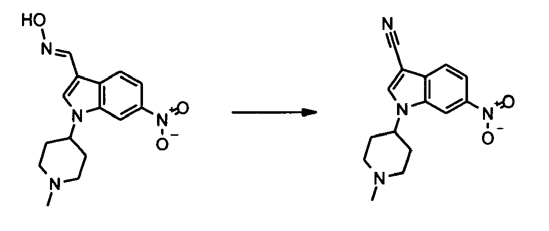

550 mg der als **INT34** beschriebenen Verbindung werden in 80 ml Dichlormethan gelöst und mit 0,76 ml Triethylamin und 0,31 ml Trichloracetylchlorid versetzt. Es wird zwei Stunden bei Raumtemperatur gerührt. Die Reaktionsmischung wird am Rotationsverdampfer zur Trockene eingeengt. Nach Reinigung durch Chromatographie an Kieselgel werden 440 mg der Titelverbindung erhalten.
¹H-NMR, 300 MHz, (DMSO-*d*₆): δ = 1,88-2,08 (m, 4H); 2,10-2,23 (m, 5H); 2,79-2,92 (m, 2H); 4,67-4,82 (m, 1 H); 7,82 (d, 1 H); 8,09 (dd, 1 H); 8,82 (d, 1 H); 8,84 (s, 1 H) ppm. FT-IR, ausgewählte Signale:
ν = 2219 (s), 1516 (vs), 1342 (vs) cm⁻¹.

### Intermediat INT36

### 6-Amino-1-(1-methyl-4-piperidinyl)-1H-indol-3-carbonitril

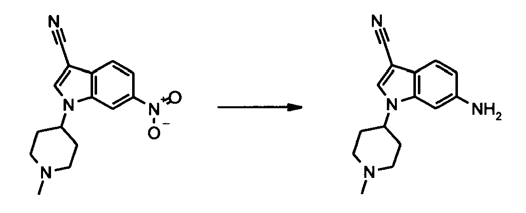

370 mg der als **INT35** beschriebenen Verbindung werden in 60 ml Ethanol vorgelegt, und mit 61 mg Raney-Nickel versetzt. Es wird 15 Stunden unter Wasserstoffatmosphäre bei Raumtemperatur gerührt. Die Reaktionsmischung wird über Kieselgur filtriert und das Lösungsmittel wird am Rotationsverdampfer abkondensiert. Nach Reinigung durch Chromatographie an Aminophasenkieselgel werden 250 mg der Titelverbindung erhalten.
¹H-NMR, 300 MHz, (DMSO-*d*₆): δ = 1,77-2,14 (m, 6H); 2,19 (s, 3H); 2,77-2,95 (m, 2H); 4,00-4,16 (m, 1 H); 5,08 (s, b, 2H); 6,58 (dd, 1 H); 6,69 (d, 1 H); 7,22 (d, 1 H); 8,01 (s, 1 H) ppm.

### Intermediat INT37

### 3-Chlor-1-(1-methyl-4-piperidinyl)-6-nitro-1H-indol

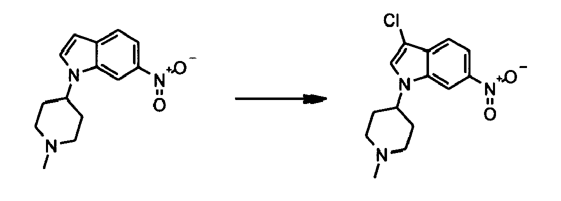

600 mg der als **INT29** beschriebenen Verbindung werden in 10 ml Acetonitril gelöst mit 370 mg *N*-Chlorsuccinimid versetzt. Es wird 4 Stunden bei Raumtemperatur gerührt. Die Reaktionsmischung wird mit halbgesättigter Natriumhydrogencarbonatlösung versetzt und mit einem Gemisch aus Essigsäureethylester und Methanol (10:1) extrahiert. Die organische Phase wird mit gesättigter Kochsalzlösung gewaschen und über Natriumsulfat getrocknet. Nach Reinigung durch Umkristallisieren aus Ethanol werden 457 mg der Titelverbindung erhalten.
¹H-NMR, 300 MHz, (DMSO-*d*₆): δ = 1,81-1,91 (m, 2H); 1,91-2,08 (m, 2H); 2,09-2,25 (m, 5H); 2,78-2,90 (m, 2H); 4,51-4,77 (m, 1H); 7,63 (d, 1H); 7,96 (dd, 1H); 8,18 (s, 1H); 8,69 (d, 1 H) ppm.

### Intermediat INT38

### 3-Chlor-1-(1-methyl-4-piperidinyl)-1H-indol-6-amin

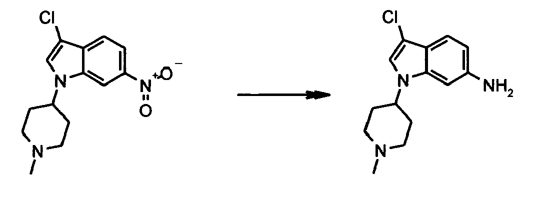

450 mg der als **INT37** beschriebenen Verbindung werden in 20 ml Tetrahydrofuran gelöst, und mit 3,6 ml einer 15%igen Titan(III)chlorid-Lösung versetzt. Es wird 4 Stunden bei Raumtemperatur gerührt. Die Reaktionsmischung wird mit 20-prozentiger Natronlauge basisch gestellt und mit 20 ml Methanol versetzt. Der Niederschlag wird abfiltriert, die organische Phase wird mit Essigsäureethylester versetzt, mit gesättigter Kochsalzlösung gewaschen und über Natriumsulfat getrocknet. Nach Reinigung durch Chromatographie an Aminophasenkieselgel werden 352 mg der Titelverbindung erhalten.
¹H-NMR, 300 MHz, (DMSO-*d*₆): δ = 1,78-2,16 (m, 6H); 2,22 (s, 3H); 2,80-2,98 (m, 2H); 3,93-4,11 (m, 1H); 4,96 (s, b, 2H); 6,51 (d, 1H); 6,62 (s, 1H); 7,12 (d, 1H); 7,26 (s, 1H) ppm.

### Intermediat INT39

### 2,3-Dichlor-1-(1-methyl-4-piperidinyl)-6-nitro-1H-indol

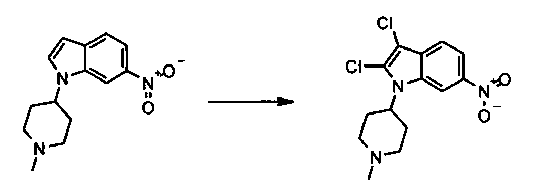

200 mg der als **INT29** beschriebenen Verbindung werden in 5 ml Dichlormethan gelöst und bei 0°C mit 0,16 ml Sulfurylchlorid versetzt. Es wird 72 Stunden bei Raumtemperatur gerührt. Die Reaktionsmischung wird mit 5-prozentiger Natronlauge und Dichlormethan versetzt. Die organische Phase wird abgetrennt, mit gesättigter Kochsalzlösung gewaschen und über Natriumsulfat getrocknet. Nach Reinigung durch Chromatographie an Kieselgel werden 220 mg der Titelverbindung erhalten.
¹H-NMR, 300 MHz, (DMSO-*d*₆): δ = 1,60-1,78 (m, 2H); 2,01-2,17 (m, 2H); 2,21 (s, 3H); 2,24-2,41 (m, 2H); 2,76-2,93 (m, 2H); 4,11-4,30 (m, 1H); 8,00 (d, 1H); 8,05 (dd, 1H); 8,13 (d, 1 H) ppm.

### Intermediat INT40

### 6-Amino-1,3-dihydro-1-(1-methyl-4-piperidinyl)-2H-indol-2-one

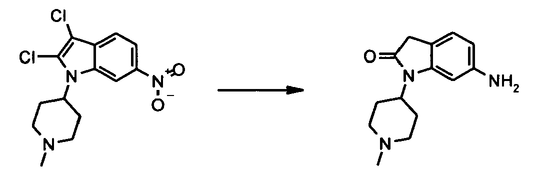

440 mg der als **INT39** beschriebenen Verbindung werden in 33 ml Tetrahydrofuran gelöst, und mit 6,8 ml einer 15%igen Titan(III)chlorid-Lösung versetzt. Es wird 4 Stunden bei Raumtemperatur gerührt. Die Reaktionsmischung wird mit 20-prozentiger Natronlauge basisch gestellt und mit 20 ml Methanol versetzt. Der Niederschlag wird abfiltriert, die organische Phase wird mit Essigsäureethylester versetzt, mit gesättigter Kochsalzlösung gewaschen und über Natriumsulfat getrocknet. Nach Reinigung durch Chromatographie an Aminophasenkieselgel werden 136 mg der Titelverbindung erhalten.
¹H-NMR, 300 MHz, (DMSO-*d*₆): δ = 1,43-1,62 (m, 2H); 1,98 (t, b, 2H); 2,20 (s, 3H); 2,26-2,44 (m, 2H); 2,80-2,95 (m, 2H); 3,31 (s, 2H); 3,91-4,15 (m, 1H); 5,09 (s, b, 2H); 6,17 (d, 1H); 6,49 (s, 1H); 6,85 (d, 1H) ppm.

### Intermediat INT41

### (trans)-4-(6-Nitro-1H-indol-1-yl)cyclohexanamin

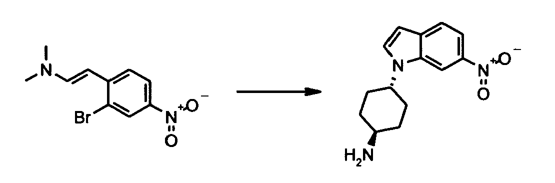

1,5 g der als **INT28** beschriebenen Verbindung, 947 mg trans-1,4-Diaminocyclohexan, 253 mg Tris(dibenzylidenaceton)dipalladium(0), 344 mg *rac*-BINAP und 1,06 g Natrium-*tert*-butylat werden in 33 ml Toluol zwei Stunden bei 110°C gerührt. Die Reaktionsmischung wird mit Wasser und Essigsäureethylester versetzt und mit vier normaler Salzsäure leicht angesäuert. Die organische Phase wird abgetrennt und die wässrige Phase wird mit ein normaler Natronlauge basisch gestellt und zweimal mit einem Gemisch aus Dichlormethan und Methanol (10:1) extrahiert. Die organische Phase wird mit gesättigter Kochsalzlösung gewaschen und über Natriumsulfat getrocknet. Nach Reinigung durch Chromatographie an Kieselgel werden 520 mg der Titelverbindung erhalten.
¹H-NMR, 300 MHz, (DMSO-*d*₆): δ = 1,25-1,44 (m, 2H); 1,70-1,96 (m, 6H); 2,59-2,72 (m, 1 H); 3,27 (b, 2H); 4,49-4,65 (m, 1 H); 6,63 (d, 1 H); 7,67 (d, 1 H); 7,86 (dd, 1 H); 7,90 (d, 1 H); 8,55 (d, 1 H) ppm.

### Intermediat INT42

### (trans)-N,N-Dimethyl-4-(6-nitro-1H-indol-1-yl)cyclohexanamin

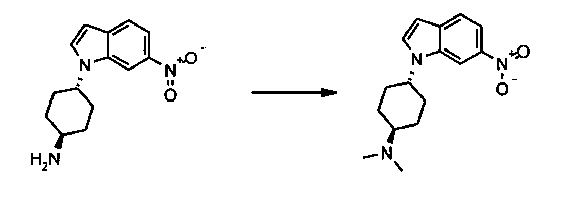

513 mg der als **INT41** beschriebenen Verbindung werden in 16 ml Methanol und 7 ml Dichlormethan gelöst und mit 594 mg Paraformaldehyd, 1,24 g Natriumcyanoborhydrid und 1,13 ml Eisessig versetzt. Es wird zwei Stunden bei Raumtemperatur gerührt. Die Reaktionsmischung wird mit Wasser versetzt, mit zwei molarer Natronlauge basisch gestellt und mit Essigsäureethylester extrahiert. Die organische Phase wird mit gesättigter Kochsalzlösung gewaschen und über Natriumsulfat getrocknet. Nach Reinigung durch Chromatographie an Kieselgel werden 378 mg der Titelverbindung erhalten.
¹H-NMR, 300 MHz, (DMSO-*d*₆): δ = 1,42-1,62 (m, 2H); 1,71-2,03 (m, 6H); 2,19 (s, 6H); 2,23-2,36 (m, 1H); 4,52-4,68 (m, 1H); 4,64 (d, 1H); 7,67 (d, 1H); 7,82-7,91 (m, 2H); 8,58 (d, 1 H) ppm.

### Intermediat INT43

### (trans)-1-[4-(Dimethylamino)cyclohexyl]-1H-indol-6-amin

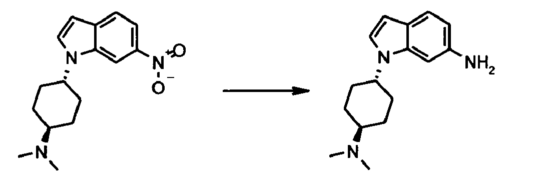

150 mg der als **INT42** beschriebenen Verbindung werden in 5 ml Ethanol gelöst, und mit 50 mg Palladium auf Kohle (10 %ig) versetzt. Es wird 15 Stunden unter Wasserstoffatmosphäre bei Raumtemperatur gerührt. Die Reaktionsmischung wird über Kieselgur filtriert und das Lösungsmittel wird am Rotationsverdampfer abkondensiert. Es werden 133 mg der Titelverbindung erhalten.
¹H-NMR, 400 MHz, (DMSO-*d*₆): δ = 1,39 (q, b, 2H); 1,70 (q, b, 2H); 1,91 (t, b, 4H); 2,18 (s, 6H); 2,22-2,33 (m, 1 H); 3,92-4,04 (m, 1 H); 4,69 (s, b, 2H); 6,14 (d, 1 H); 6,36 (dd, 1H); 6,55 (d, 1H); 7,02 (d, 1H); 7,12 (d, 1H) ppm.

### Intermediat INT44

### (trans)-4-(3-Chlor-6-nitro-1H-indol-1-yl)-N,N-dimethylcyclohexanamin

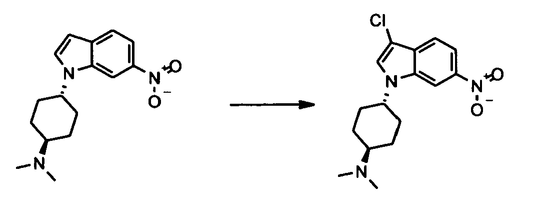

250 mg der als **INT42** beschriebenen Verbindung werden in 5 ml Acetonitril gelöst mit 140 mg N-Chlor-Succinimid versetzt. Es wird eine Stunde bei Raumtemperatur gerührt. Die Reaktionsmischung wird mit halbgesättigter Natriumhydrogencarbonatlösung versetzt und mit einem Gemisch aus Essigsäureethylester und Methanol (5:1) extrahiert. Die organische Phase wird nacheinander mit 5-prozentiger Natriumthiosulfatlösung und mit gesättigter Kochsalzlösung gewaschen und über Natriumsulfat getrocknet. Nach Reinigung durch Chromatographie an Kieselgel werden 115 mg der Titelverbindung erhalten.
¹H-NMR, 400 MHz, (DMSO-*d*₆): δ = 1,54 (q, b, 2H); 1,73-2,03 (m, 6H); 2,24 (s, 6H); 2,31-2,43 (m, 1H); 4,62-4,73 (m, 1H); 7,63 (d, 1H); 7,96 (dd, 1H); 8,14 (s, 1H); 8,70 (d, 1H) ppm.

### Intermediat INT45

### (trans)-3-Chlor-1-[4-(dimethylamino)cyclohexyl]-1H-indol-6-amin

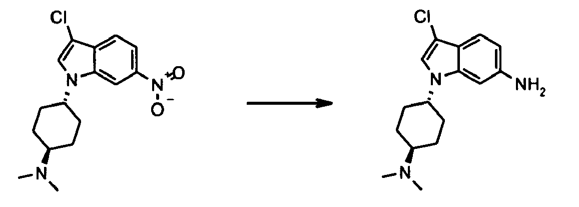

265 mg der als **INT44** beschriebenen Verbindung werden in 20 ml Tetrahydrofuran gelöst, und mit 4,2 ml einer 15%igen Titan(III)chlorid-Lösung versetzt. Es wird 4 Stunden bei Raumtemperatur gerührt. Die Reaktionsmischung wird mit 20-prozentiger Natronlauge basisch gestellt und mit 20 ml Methanol versetzt. Der Niederschlag wird abfiltriert, die organische Phase wird mit Essigsäureethylester versetzt, mit gesättigter Kochsalzlösung gewaschen und über Natriumsulfat getrocknet. Nach Reinigung durch Chromatographie an Aminophasenkieselgel werden 166 mg der Titelverbindung erhalten.
¹H-NMR, 300 MHz, (DMSO-*d*₆): δ = 1,28-1,48 (m, 2H); 1,58-1,79 (m, 2H); 1,81-1,97 (m, 4H); 2,09-2,31 (m, 7H); 3,91-4,08 (m, 1 H); 4,90 (s, b, 2H); 6,46 (dd, 1 H); 6,57 (d, 1 H); 7,06 (d, 1 H); 7,18 (s, 1 H) ppm.

### Intermediat INT46

### Ethyl 3-Hydroxy-4-nitrobenzoat

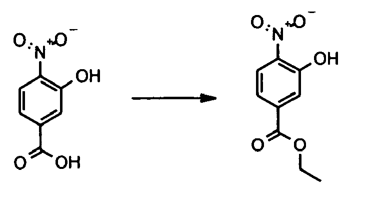

20 g 3-Hydroxy-4-nitrobenzoesäure werden in 350 ml Chloroform suspendiert, mit 2 g *p*-Toluolsulfonsäure versetzt und 48 Stunden am inversen Wasserabscheider unter Rückfuß gerührt. Die Reaktionsmischung wird mit Wasser gewaschen und die wässrige Phase wird mit Essigsäureethylester extrahiert. Die vereinigten organischen Phasen werden mit gesättigter Kochsalzlösung gewaschen und über Natriumsulfat getrocknet. Nach Reinigung durch Chromatographie an Kieselgel werden 19,4 g der Titelverbindung erhalten.
¹H-NMR, 300 MHz, (DMSO-*d*₆): δ = 1,28 (t, 3H); 4,28 (q, 2H); 7,38 (dd, 1H); 7,62 (d, 1 H); 7,89 (d, 1 H) ppm.

### Intermediat INT47

### Ethyl 4-nitro-3-[[(trifluormethyl)sulfonyl]oxy]benzoat

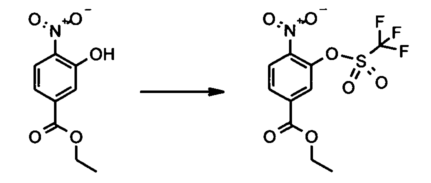

12,4 g der als **INT46** beschriebenen Verbindung werden in 300 ml Tetrahydrofuran gelöst und bei 0°C mit 20 ml Diisopropylethylamin und 21,4 g *N-*Phenylbis(trifluormethansulfonimid) versetzt. Es wird zwei Stunden bei Raumtemperatur gerührt. Die Reaktionsmischung wird mit gesättigter Kochsalzlösung versetzt und mit Essigsäureethylester extrahiert. Die organische Phase wird über Natriumsulfat getrocknet. Nach Filtration durch Kieselgel und Umkristallisieren aus Dichlormethan (20 ml) / Hexan (200 ml) werden 18,5 g der Titelverbindung erhalten.
¹H-NMR, 300 MHz, (DMSO-*d*₆): δ = 1,31 (t, 3H); 4,36 (q, 2H); 8,10 (d, 1H); 8,22 (dd, 1H); 8,42 (d, 1H) ppm.

### Intermediat INT48

### Ethyl 3-[(1-methyl-4-piperidinyl)amino]-4-nitrobenzoat

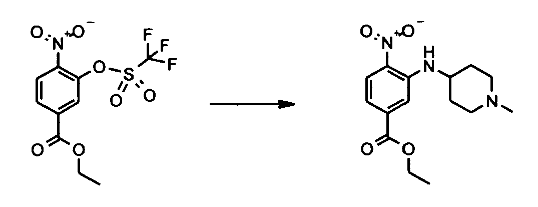

6,45 g der als **INT47** beschriebenen Verbindung, 3,22 g 1-Methyl-4-aminopiperidin, 1,08 g Tetrakis(triphenylphosphin)palladium(0), 0,49 g Triphenylphosphin und 5,2 g Kaliumcarbonat werden in 39 ml Toluol eine Stunde bei 110°C gerührt. Es werden weitere 1,08 g Tetrakis(triphenylphosphin)palladium(0) und 0,49 g Triphenylphosphin hinzugegeben und es wird erneut eine Stunde bei 110°C gerührt. Die Reaktionsmischung wird mit Wasser versetzt und mit Essigsäureethylester extrahiert. Die organische Phase wird mit gesättigter Kochsalzlösung gewaschen und über Natriumsulfat getrocknet. Nach Reinigung durch Chromatographie an Aminophasenkieselgel und anschließend an Kieselgel werden 2,31 g der Titelverbindung erhalten.
¹H-NMR, 300 MHz, (DMSO-*d*₆): δ = 1,29 (t, 3H); 1,47-1,67 (m, 2H); 1,83-1,98 (m, 2H); 2,06-2,20 (m, 5H); 2,55-2,68 (m, 2H); 3,55-3,73 (m, 1H); 4,30 (q, 2H); 7,11 (dd, 1H); 7,50 (d, 1 H); 7,88 (d, 1 H); 8,14 (d, 1 H) ppm.

### Intermediat INT49

### Ethyl 4-amino-3-[(1-methyl-4-piperidinyl)amino]benzoat

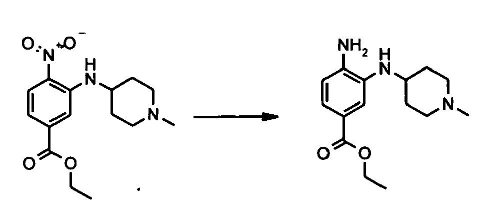

2,34 g der als **INT48** beschriebenen Verbindung werden in 160 ml Ethanol gelöst, und mit 100 mg Palladium auf Kohle (10 %ig) versetzt. Es wird 15 Stunden unter Wasserstoffatmosphäre bei Raumtemperatur gerührt. Die Reaktionsmischung wird über Kieselgur filtriert und das Lösungsmittel wird am Rotationsverdampfer abkondensiert. Es werden 2,1 g der Titelverbindung erhalten.
¹H-NMR, 400 MHz, (DMSO-*d*₆): δ = 1,22 (t, 3H); 1,39-1,54 (m, 2H); 1,84-1,96 (m, 2H); 2,11-2,31 (m, 5H); 2,79-2,91 (m, 2H); 3,11-3,26 (m, 1H); 4,15 (q, 2H); 4,37 (d, 1H); 5,40 (s, b, 2H); 6,50 (d, 1H); 6,98 (d, 1H); 7,10 (dd, 1H) ppm.

### Intermediat INT50

### Ethyl 1-(1-methyl-4-piperidinyl)-1H-benzimidazol-6-carboxylat

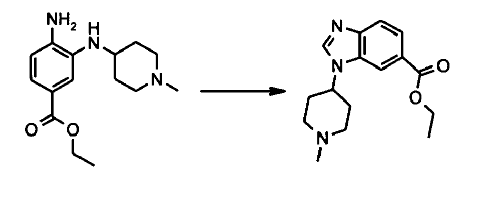

2,1 g der als **INT49** beschriebenen Verbindung werden in 10 ml Dichlormethan gelöst, mit 20 ml Triethylorthoformiat und 190 mg Pyridinium-*para*-toluolsulfonat versetzt und zwei Stunden bei 60°C gerührt. Die Reaktionsmischung wird mit wenig Triethylamin versetzt, und nach Reinigung durch Chromatographie an Aminophasenkieselgel werden 1,36 g der Titelverbindung erhalten.
¹H-NMR, 300 MHz, (DMSO-*d*₆): δ = 1,32 (t, 3H); 1,89-2,18 (m, 6H); 2,21 (s, 3H); 2,80-2,93 (m, 2H); 4,32 (q, 2H); 4,40-4,55 (m, 1H); 7,71 (d, 1H); 7,81 (dd, 1H); 8,23 (d, 1H); 8,54 (s, 1H) ppm.

### Intermediat INT51

### 1-(1-Methyl-4-piperidinyl)-1H-benzimidazol-6-carbonsäure

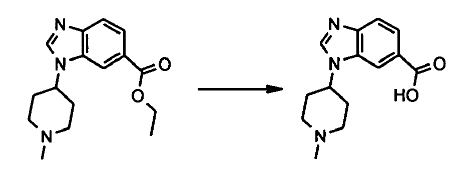

484 mg der als **INT50** beschriebenen Verbindung werden in 19 ml Ethanol gelöst, und mit 1,26 ml einer zwei molaren Natriumhydroxidlösung versetzt. Es wird 2 Stunden bei 80°C gerührt. Die Reaktionsmischung wird mit 2,53 ml einnormaler Salzsäure in Diethylether versetzt und das Lösungsmittel wird am Rotationsverdampfer abdestilliert. Der Rückstand wird dreimal nacheinander mit einem Gemisch aus Chloroform und Toluol (3:1) versetzt und bis zur Trockene eingengt. Das erhaltene Rohprodukt wird ohne Reinigung weiter umgesetzt.
¹H-NMR, 300 MHz, (DMSO-*d*₆), ausgewählte Signale: δ = 1,80-2,27 (m, 9H); 2,79-2,97 (m, 2H); 4,32-4,54 (m, 1H); 7,62 (d, 1H); 7,79 (dd, 1H); 8,19 (d, 1H); 8,45 (s, 1H) ppm.

### Intermediat INT52

### 1,1-Dimethylethyl [1-(1-methyl-4-piperidinyl)-1H-benzimidazol-6-yl]carbamat

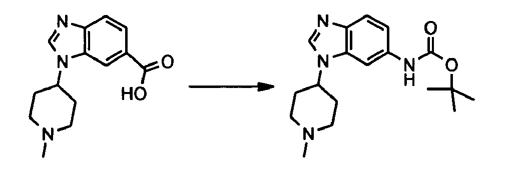

1,68 mmol des als **INT51** beschriebenen Rohproduktes werden in 5 ml Toluol suspendiert, mit 0,26 ml Triethylamin und 0,40 ml Diphenylphosphorylchlorid versetzt und zwei Stunden bei 90°C gerührt. Es werden 1,6 ml *tert*-Butanol zugegeben und es wird weitere zwei Stunden bei 90°C gerührt. Die Reaktionsmischung wird mit halbgesättigter Natriumhydrogencarbonatlösung versetzt und mit Dichlormethan extrahiert. Die organische Phase wird mit gesättigter Kochsalzlösung gewaschen und über Natriumsulfat getrocknet. Nach Reinigung durch Chromatographie an Aminophasenkieselgel werden 203 mg der Titelverbindung erhalten.
¹H-NMR, 300 MHz, (DMSO-*d*₆): δ = 1,45 (s, 9H); 1,82-2,14 (m, 6H); 2,20 (s, 3H); 2,17-2,25 (m, 2H); 4,08-4,26 (m, 1H); 7,07 (dd, 1H); 7,45 (d, 1H); 7,85 (d, 1H); 8,16 (s, 1H); 9,31 (s, 1H) ppm.

### Intermediat INT53

### 1-(1-Methyl-4-piperidinyl)-1H-benzimidazol-6-amin

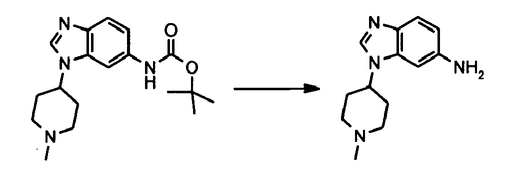

387 mg der als **INT52** beschriebenen Verbindung werden in 12 ml Dichlormethan gelöst und mit 5,5 ml Trifluoressigsäure versetzt. Es wird eine Stunde bei Raumtemperatur gerührt. Die Reaktionsmischung wird am Rotationsverdampfer zur Trockene eingeengt, und in Dichlormethan suspendiert. Die organische Phase wird mit gesättigter Kaliumcarbonatlösung gewaschen und über Natriumsulfat getrocknet. Nach Reinigung durch Chromatographie an Aminophasenkieselgel werden 215 mg der Titelverbindung erhalten.
¹H-NMR, 300 MHz, (DMSO-*d*₆): δ = 1,85-2,18 (m, 6H); 2,24 (s, 3H); 2,85-2,98 (m, 2H); 3,97-4,20 (m, 1H); 4,94 (s, 1H); 6,52 (dd, 1H); 6,68 (d, 1H); 7,28 (d, 1H); 7,95 (s, 1H) ppm.

### Intermediat INT54

### Ethyl 4-nitro-3-[[2-(1-pyrrolidinyl)ethyl]amino]benzoat

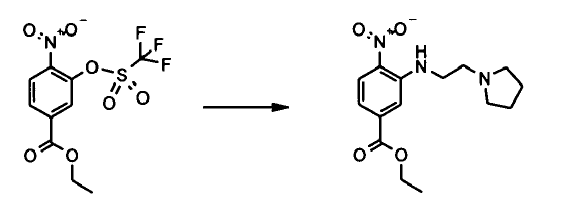

Analog zu der unter **INT48** beschriebenen Vorschrift wird ausgehend von **INT47** und 1-Pyrrolidinethanamin die Titelverbindung erhalten.
¹H-NMR, 300 MHz, (DMSO-*d*₆): δ = 1,34 (t, 3H); 1,62-1,79 (m, 4H); 2,52-2,61 (m, 4H); 2,74 (t, 2H); 3,47 (q, 2H); 4,35 (q, 2H); 7,15 (dd, 1H); 7,54 (d, 1H); 8,17 (d, 1H); 8,37 (t, 1H) ppm.

### Intermediat INT55

### Ethyl 4-amino-3-[[2-(1-pyrrolidinyl)ethyl]amino]benzoat

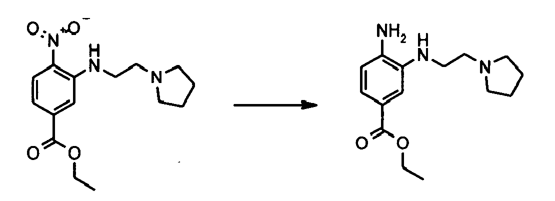

Analog zu der unter **INT49** beschriebenen Vorschrift wird ausgehend von **INT54** die Titelverbindung erhalten.
¹H-NMR, 300 MHz, (DMSO-*d*₆): δ = 1,23 (t, 3H); 1,60-1,85 (m, 4H); 2,56-2,79 (m, 4H); 2,79-2,94 (m, 2H); 3,14-3,24 (m, 2H); 4,15 (q, 2H); 4,63 (b, 1H); 5,40 (s, b, 2H); 6,51 (d, 1H); 6,96 (d, 1H); 7,13 (dd, 1H) ppm.

### Intermediat INT56

### Ethyl 1-[2-(1-pyrrolidinyl)ethyl]-1H-benzimidazol-6-carboxylat

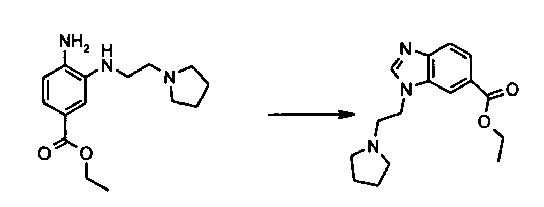

Analog zu der unter **INT50** beschriebenen Vorschrift wird ausgehend von **INT55** die Titelverbindung erhalten.
¹H-NMR, 300 MHz, (DMSO-*d*₆): δ = 1,31 (t, 3H); 1,54-1,72 (m, 4H); 2,40-2,55 (m, 4H); 2,71-2,90 (m, 2H); 4,31 (q, 2H); 4,41 (t, 2H); 7,68 (d, 1H); 7,80 (dd, 1H); 8,21 (d, 1H); 8,38 (s, 1H) ppm.

### Intermediat INT57

### 1,1-Dimethylethyl [1-[2-(1-pyrrolidinyl)ethyl]-1H-benzimidazol-6-yl]carbamat

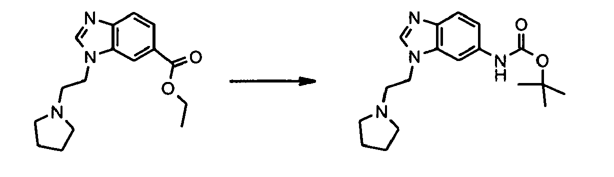

Analog zu den unter **INT51** und **INT52** beschriebenen Vorschriften wird ausgehend von **INT56** die Titelverbindung erhalten.
¹H-NMR, 300 MHz, (DMSO-*d*₆): δ = 1,45 (s, 9H); 1,57-1,66 (m, 4H); 2,35-2,50 (m, 4H); 2,75 (t, 2H); 4,21 (t, 2H); 7,09 (dd, 1H); 7,44 (d, 1H); 7,79 (d, 1H); 8,05 (s, 1H); 9,32 (s, b, 1H) ppm.

### Intermediat INT58

### 1-[2-(1-Pyrrolidinyl)ethyl]-1H-benzimidazol-6-amin

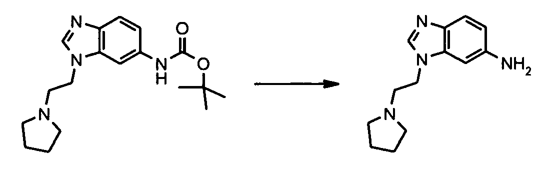

Analog zu der unter **INT53** beschriebenen Vorschrift wird ausgehend von **INT57** die Titelverbindung erhalten.
¹H-NMR, 400 MHz, (DMSO-*d*₆): δ = 1,54-1,70 (m, 4H); 2,35-2,45 (m, 4H); 2,72 (t, 2H); 4,11 (t, 2H); 4,90 (s, 2H); 6,48 (dd, 1H); 6,56 (d, 1H); 7,23 (d, 1H); 7,80 (s, 1H) ppm.

### Intermediat INT59

### 1,1-Dimethylethyl 4-[[5-(Ethoxycarbonyl)-2-nitrophenyl]amino]piperidin-1-carboxylat

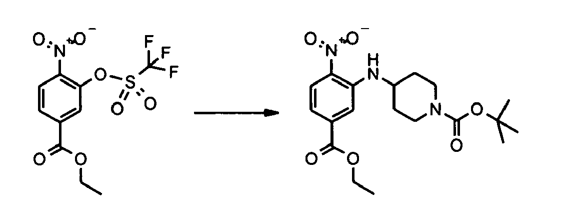

6,3 g der als **INT47** beschriebenen Verbindung, 4,04 g 1,1-Dimethylethyl 4-aminopiperidin-1-carboxylat, 1,06 g Tetrakis(triphenylphosphin)palladium(0), 0,48 g Triphenylphosphin und 5,1 g Kaliumcarbonat werden in 38 ml Toluol eine Stunde bei 110°C gerührt. Es werden weitere 1,06 g Tetrakis(triphenylphosphin)palladium(0) und 0,48 g Triphenylphosphin hinzugegeben und es wird erneut eine Stunde bei 110°C gerührt. Die Reaktionsmischung wird mit Wasser versetzt und mit Essigsäureethylester extrahiert. Die organische Phase wird mit gesättigter Kochsalzlösung gewaschen und über Natriumsulfat getrocknet. Nach Reinigung durch Chromatographie an Aminophasenkieselgel und anschließend an Kieselgel werden 2,88 g der Titelverbindung erhalten.
¹H-NMR, 400 MHz, (DMSO-*d*₆): δ = 1,29 (t, 3H); 1,37 (s, 9H); 1,40-1,51 (m, 2H); 1,82-1,95 (m, 2H); 2,83-3,11 (m, 2H); 3,75-3,95 (m, 3H); 4,31 (q, 2H); 7,12 (dd, 1H); 7,54 (d, 1H); 7,84 (d, 1H); 8,14 (d, 1H) ppm.

### Intermediat INT60

### 1,1-Dimethylethyl 4-[[2-amino-5-(ethoxycarbonyl)phenyl]amino]piperidin-1-carboxylat

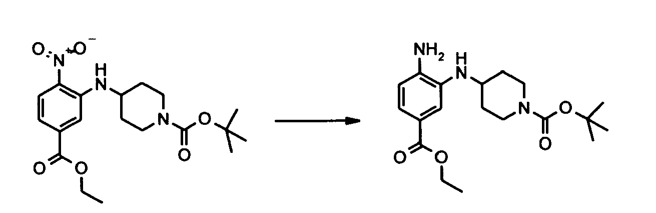

5,32 g der als **INT59** beschriebenen Verbindung werden in 280 ml Ethanol gelöst, und mit 145 mg Palladium auf Kohle (10 %ig) versetzt. Es wird 15 Stunden unter Wasserstoffatmosphäre bei Raumtemperatur gerührt. Die Reaktionsmischung wird über Kieselgur filtriert und das Lösungsmittel wird am Rotationsverdampfer abkondensiert. Nach Reinigung durch Chromatographie an Kieselgel werden 4,28 g der Titelverbindung erhalten.
¹H-NMR, 300 MHz, (DMSO-*d*₆): δ = 1,17-1,27 (m, 5H); 1,37 (s, 9H); 1,77-1,93 (m, 2H); 2,75-3,06 (m, 2H); 3,31-3,49 (m, 1H); 3,76-3,93 (m, 2H); 4,15 (q, 2H); 4,34 (d, 1H); 5,38 (s, 2H); 6,50 (d, 1H); 7,01 (d, 1H); 7,10 (dd, 1H) ppm.

### Intermediat INT61

### Ethyl 1-[1-[(1,1-dimethylethoxy)carbonyl]-4-piperidinyl]-1H-benzimidazol-6-carboxylat

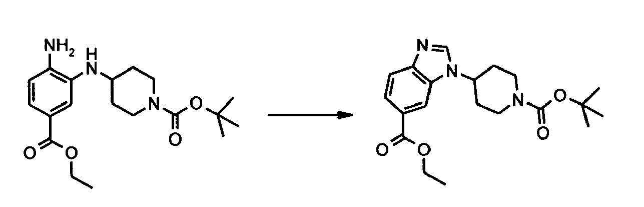

4,2 g der als **INT60** beschriebenen Verbindung werden in 15 ml Dichlormethan gelöst, mit 30 ml Triethylorthoformiat und 290 mg Pyridinium-*para*-toluolsulfonat versetzt und 30 Minuten bei Raumtemperatur gerührt. Die Reaktionsmischung wird mit wenig Triethylamin versetzt, und nach Reinigung durch Chromatographie an Kieselgel werden 3,28 g der Titelverbindung erhalten.
¹H-NMR, 300 MHz, (DMSO-*d*₆): δ = 1,31 (t, 3H); 1,40 (s, 9H); 1,80-2,07 (m, 4H); 2,85-3,12 (m, 2H); 4,02-4,18 (m, 2H); 4,31 (q, 2H); 4,65-4,83 (m, 1H); 7,70 (d, 1H); 7,81 (dd, 1H); 8,26 (d, 1H); 8,57 (s, 1H) ppm.

### Intermediat INT62

### Ethyl 1-(4-Piperidinyl)-1H-benzimidazol-6-carboxylat

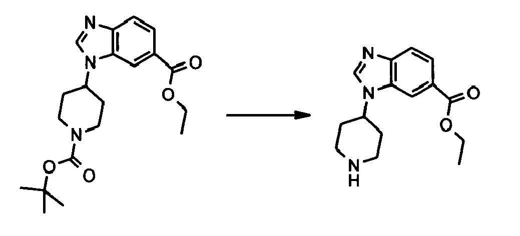

3,27 g der als **INT61** beschriebenen Verbindung werden in 85 ml Dichlormethan gelöst und mit 40 ml Trifluoressigsäure versetzt. Es wird eine Stunde bei Raumtemperatur gerührt. Die Reaktionsmischung wird am Rotationsverdampfer zur Trockene eingeengt. Der Rückstand wird mit Essigsäureethylester versetzt und mit gesättigter Natriumhydrogencarbonatlösung gewaschen. Nach Reinigung durch Chromatographie an Kieselgel werden 2,35 g der Titelverbindung erhalten.
¹H-NMR, 400 MHz, (DMSO-*d*₆): δ = 1,32 (t, 3H); 2,14-2,32 (m, 4H); 3,10-3,21 (m, 2H); 3,40-3,54 (m, 2H); 4,33 (q, 2H); 4,78-4,69 (m, 1H); 7,73 (d, 1H); 7,83 (dd, 1H); 8,33 (d, 1H); 8,49 (s, 1H); 8,83 (b, 1H) ppm.

### Intermediat INT63

### Ethyl 1-(1-Cyclopentyl-4-piperidinyl)-1H-benzimidazol-6-carboxylat

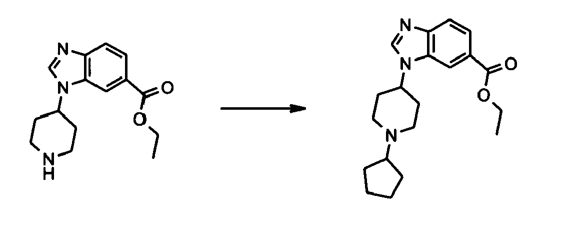

650 mg der als **INT62** beschriebenen Verbindung werden in 17 ml Dichlorethan gelöst und mit 0,18 ml Cyclopentanon, 492 mg Natriumtrisacetoxyborhydrid und 95 µl Eisessig versetzt. Es wird 17 Stunden bei Raumtemperatur gerührt. Die Reaktionsmischung wird mit Dichlormethan versetzt, mit halbgesättigter Natriumhydrogencarbonatlösung gewaschen und über Natriumsulfat getrocknet. Nach Reinigung durch Chromatographie an Kieselgel werden 502 mg der Titelverbindung erhalten.
¹H-NMR, 300 MHz, (DMSO-*d*₆): δ = 1,22-1,40 (m, 5H); 1,41-1,67 (m, 4H); 1,71-1,83 (m, 2H); 1,91-2,21 (m, 6H); 2,50-2,58 (m, 1H); 2,94-3,10 (m, 2H); 4,31 (q, 2H); 4,42-4,59 (m, 1 H); 7,69 (d, 1 H); 7,80 (dd, 1 H); 8,23 (d, 1 H); 8,55 (s, 1 H) ppm.

### Intermediat INT64

### 1-(1-Cyclopentyl-4-piperidinyl)-1H-benzimidazol-6-carbonsäure

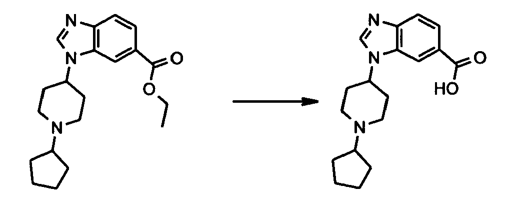

895 mg der als **INT63** beschriebenen Verbindung werden in 30 ml Ethanol gelöst, und mit 1,97 ml einer zwei molaren Natriumhydroxidlösung versetzt. Es wird 2 Stunden bei 80°C gerührt. Die Reaktionsmischung wird mit 3,93 ml einnormaler Salzsäure in Diethylether versetzt und das Lösungsmittel wird am Rotationsverdampfer abdestilliert. Der Rückstand wird dreimal nacheinander mit einem Gemisch aus Chloroform und Toluol (3:1) versetzt und bis zur Trockene eingengt. Das erhaltene Rohprodukt wird ohne Reinigung weiter umgesetzt.
¹H-NMR, 300 MHz, (DMSO-*d*₆), ausgewählte Signale: δ = 1,28-1,45 (m, 2H); 1,46-1,70 (m, 4H); 1,72-1,87 (m, 2H); 1,95-2,25 (m, 6H); 2,50-2,63 (m, 1H); 3,01-3,15 (m, 2H); 4,32-4,53 (m, 1H); 7,59 (d, 1H); 7,84 (dd, 1H); 8,19 (d, 1H); 8,42 (s, 1H) ppm.

### Intermediat INT65

### 1,1-Dimethylethyl [1-(1-cyclopentyl-4-piperidinyl)-1H-benzimidazol-6-yl]carbamat

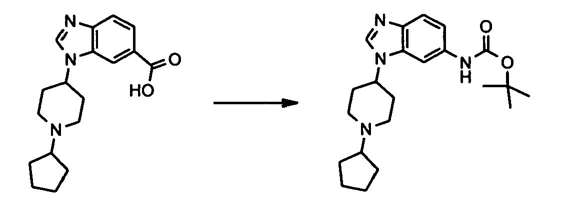

2,62 mmol des als **INT64** beschriebenen Rohproduktes werden in 5 ml Toluol suspendiert, mit 0,40 ml Triethylamin und 0,62 ml Diphenylphosphorylchlorid versetzt und drei Stunden bei 90°C gerührt. Es werden 2,5 ml *tert*-Butanol zugegeben und es wird weitere drei Stunden bei 90°C gerührt. Die Reaktionsmischung wird mit halbgesättigter Natriumhydrogencarbonatlösung versetzt und mit Dichlormethan extrahiert. Die organische Phase wird mit gesättigter Kochsalzlösung gewaschen und über Natriumsulfat getrocknet. Nach Reinigung durch Chromatographie an Kieselgel werden 192 mg der Titelverbindung erhalten.
¹H-NMR, 300 MHz, (DMSO-*d*₆): δ = 1,20-1,69 (m, 15H); 1,70-1,86 (m, 2H); 1,88-2,18 (m, 6H); 2,46-2,60 (m, 1H); 2,99-3,11 (m, 2H); 4,08-4,27 (m, 1H); 7,08 (dd, 1H); 7,45 (d, 1 H); 7,82 (d, 1 H); 8,17 (s, 1 H); 9,31 (s, b, 1 H) ppm.

### Intermediat INT66

### 1-(1-Cyclopentyl-4-piperidinyl)-1H-benzimidazol-6-amin

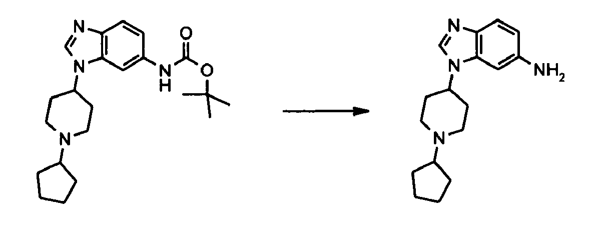

185 mg der als **INT65** beschriebenen Verbindung werden in 5 ml Dichlormethan gelöst und mit 2,5 ml Trifluoressigsäure versetzt. Es wird eine Stunde bei Raumtemperatur gerührt. Die Reaktionsmischung wird am Rotationsverdampfer zur Trockene eingeengt, und in eienm Gemisch aus Dichlormethan und Methanol (5:1) suspendiert. Die organische Phase wird mit gesättigter Kaliumcarbonatlösung gewaschen und über Natriumsulfat getrocknet. Nach Reinigung durch Chromatographie an Aminophasenkieselgel werden 99 mg der Titelverbindung erhalten.
¹H-NMR, 300 MHz, (DMSO-*d*₆): δ = 1,30-1,45 (m, 2H); 1,45-1,70 (m, 4H); 1,75-1,90 (m, 2H); 1,90-2,17 (m, 6H); 2,45-2,62 (m, 1H); 3,01-3,16 (m, 2H); 4,02-4,17 (m, 1H); 4,94 (s, b, 2H); 6,52 (dd, 1H); 6,67 (d, 1H); 7,28 (d, 1H); 7,95 (s, 1H) ppm.

### Intermediat INT67

### 5-Nitro-2-benzoxazolthiol

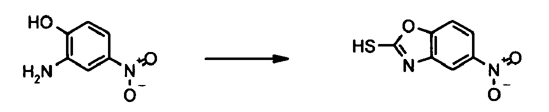

2,0 g 2-Amino-4-nitrophenol werden in 50 ml Ethanol gelöst, mit 2,5 g *O*-Ethyl-Kaliumxanthat versetzt und 2 Stunden unter Rückfluss gerührt. Aus der erkalteten Reaktionslösung wird der ausgefallene Niederschlag abfiltriert. Es werden 2,45 g der Titelverbindung erhalten.
¹H-NMR, 300 MHz, (DMSO-*d*₆): δ = 7,23 (d, 1H); 7,70-7,90 (m, 2H) ppm.

### Intermediat INT68

### 2-(Methylthio)-5-nitrobenzoxazol

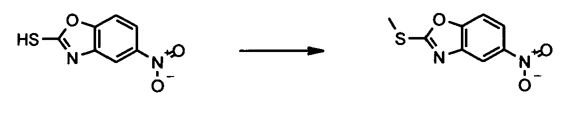

2,2 g der als **INT67** beschriebenen Verbindung werden in 45 ml *N*,*N*-Dimethylformamid gelöst, und bei 0°C mit 2,32 g Kaliumcarboant und 0,87 ml Metyliodid versetzt. Es wird 2 Stunden bei Raumtemperatur gerührt. Die Reaktionsmischung wird mit Wasser versetzt und mit Essigsäureethylester extrahiert. Die organische Phase wird mit gesättigter Kochsalzlösung gewaschen und über Natriumsulfat getrocknet. Es werden 1,62 g der Titelverbindung erhalten.
¹H-NMR, 300 MHz, (DMSO-*d*₆): δ = 2,77 (s, 3H); 7,85 (d, 1H); 8,20 (dd, 1H); 8,43 (d, 1H) ppm.

### Intermediat INT69

### 2-(4-Methyl-1-piperazinyl)-5-nitrobenzoxazol

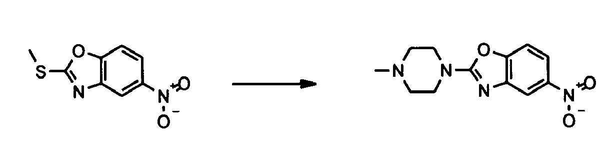

1,62 g der als **INT68** beschriebenen Verbindung werden in 162 ml 1-Propanol gelöst und mit 8,6 ml 1-Methylpiperazin versetzt. Es wird 17 Stunden unter Rückfluss gerührt. Die Reaktionsmischung wird mit Wasser versetzt und mit Essigsäureethylester extrahiert. Die organische Phase wird mit gesättigter Kochsalzlösung gewaschen und über Natriumsulfat getrocknet. Nach Reinigung durch Chromatographie an Kieselgel werden 1,79 g der Titelverbindung erhalten.
¹H-NMR, 300 MHz, (DMSO-*d*₆): δ = 2,19 (s, 3H); 2,40 (t, b, 4H); 3,61 (t, b, 4H); 7,58 (d, 1H); 7,93 (dd, 1H); 8,01 (d, 1H) ppm.

### Intermediat INT70

### 2-(4-Methyl-1-piperazinyl)benzoxazol-5-amin

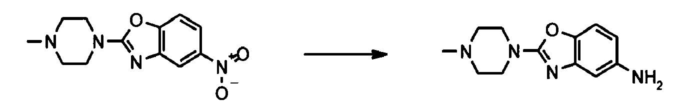

1,8 g der als **INT69** beschriebenen Verbindung werden in 200 ml Ethanol gelöst, und mit 365 mg Palladium auf Kohle (10 %ig) versetzt. Es wird 15 Stunden unter Wasserstoffatmosphäre bei Raumtemperatur gerührt. Die Reaktionsmischung wird über Kieselgur filtriert und das Lösungsmittel wird am Rotationsverdampfer abkondensiert. Es werden 1,7 g der Titelverbindung erhalten.
¹H-NMR, 400 MHz, (DMSO-*d*₆): δ = 2,33 (s, 3H); 2,53-2,68 (m, 4H); 3,47-3,68 (m, 4H); 5,30-5,80 (s, b, 2H); 6,21 (dd, 1H); 6,46 (d, 1H); 6,99 (d, 1H) ppm.

### Intermediat INT71

### 3-(1,2,3,6-Tetrahydro-1-methyl-4-pyridinyl)-5-nitro-1H-indol

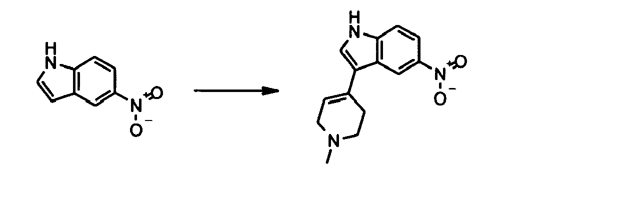

3,91 ml 1-Methyl-4-piperidinon werden in 12 ml Essigsäure und 16 ml Trifluoressigsäure vorgelegt. Bei 105°C werden 1,43 g 5-Nitro-1*H*-indol, suspendiert in 11 ml Essigsäure langsam zugetropft. Es wird eine weitere Stunde bei 105°C gerührt. Die Reaktionsmischung wird am Rotationsverdampfer zur Trockene eingeengt und der Rückstand wird aus Aceton umkristallisiert. Der Feststoff wird in einem Gemisch aus Dichlormethan und Methanol (10:1) gelöst und mit zwei normaler Natronlauge gewaschen. Die organische Phase wird über Natriumsulfat getrocknet. Nach Abdestillieren des Lösungsmittels werden 0,91 g der Titelverbindung erhalten.
¹H-NMR, 300 MHz, (DMSO-*d*₆), ausgewählte Signale: δ = 2,25 (s, 3H); 2,48-2,59 (m, 4H); 2,97-3,09 (m, 2H); 6,06-6,18 (m, 1H); 7,51 (d, 1H); 7,62 (s, 1H); 7,97 (dd, 1H); 8,64 (d, 1H) ppm.

### Intermediat INT72

### 1-(2,2-Dimethyl-1-oxopropyl)-5-nitro-3-(1,2,3,6-tetrahydro-1-methyl-4-pyridinyl)-1H-indol

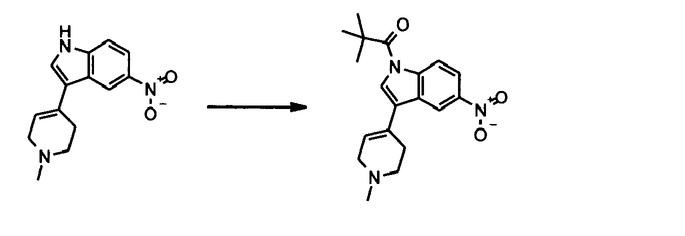

553 mg der als **INT71** beschriebenen Verbindung werden in 8,5 ml *N,N-*Dimethylformamid gelöst, und bei 0°C mit 130 mg Natriumhydrid (60%ig in Öl) versetzt. Es wird 15 Minuten bei Raumtemperatur gerührt. Es werden 0,32 ml Pivaloylchlorid zugegeben und es wird weitere zwei Stunden bei Raumtemperatur gerührt. Die Reaktionsmischung wird mit halbgesättigter Natriumhydrogencarbonatlösung versetzt und mit Dichlormethan extrahiert. Die organische Phase wird mit gesättigter Kochsalzlösung gewaschen und über Natriumsulfat getrocknet. Nach Reinigung durch Chromatographie an Kieselgel werden 465 mg der Titelverbindung erhalten.
¹H-NMR, 300 MHz, (DMSO-*d*₆): δ = 1,48 (s, 9H); 2,32 (s, 3H); 2,59-2,67 (m, b, 4H);3,05-3,16 (m, 2H); 6,31 (t, b, 1H); 8,10 (s, 1H); 8,25 (dd, 1H); 8,56 (d, 1H); 8,60 (d, 1H) ppm.

### Intermediat INT73

### 1-(2,2-Dimethyl-1-oxopropyl)-3-(1-methyl-4-piperidinyl)-1H-indol-5-amin

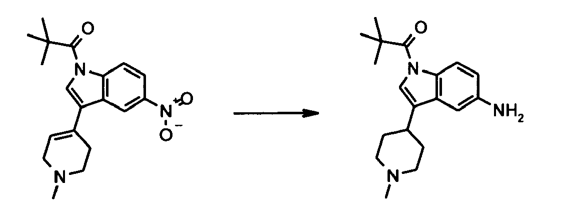

460 mg der als **INT72** beschriebenen Verbindung werden in 6 ml Ethanol und 1 ml Essigsäure gelöst, und mit 184 mg Platin(IV)oxid versetzt. Es wird vier Stunden unter Wasserstoffatmosphäre bei Raumtemperatur gerührt. Die Reaktionsmischung wird über Kieselgur filtriert und das Lösungsmittel wird am Rotationsverdampfer abkondensiert. Der Rückstand wird in Dichlormethan gelöst, mit 5%iger wässriger Natronlauge gewaschen, über Natriumsulfat getrocknet und nach Abkondensieren des Lösungsmittels am Rotationsverdampfer werden 66 mg der Titelverbindung erhalten.
¹H-NMR, 300 MHz, (DMSO-*d*₆): δ = 1,36 (s, 9H); 1,66-1,89 (m, 4H); 1,90-2,04 (m, 2H); 2,17 (s, 3H); 2,50-2,61 (m, 1H); 2,77-2,89 (m, 2H); 4,88 (s, b, 2H); 6,53 (dd, 1H); 6,71 (d, 1H); 7,45 (s, 1H); 8,00 (d, 1H) ppm.

### Intermediat INT74

### 1-(1-Methyl-4-piperidinyl)-6-nitro-1H-indol-3-carboxamid

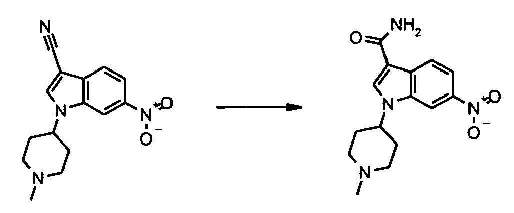

900 mg der als **INT35** beschriebenen Verbindung werden in 7 ml Trifluoressigsäure und 1,8 ml konz. Schwefelsäure vorgelegt. Es wird zwei Stunden bei 80°C und 15 Stunden bei Raumtemperatur gerührt. Die Reaktionsmischung wird mit 200 ml Wasser und mit 20 ml zwei normaler Natronlauge versetzt und mit gesättigter Natriumhydrogencarbonatlösung auf pH 8 eingestellt. Es wird mit einem Gemisch aus Dichlormethan und Methanol (100:1) extrahiert. Die organische Phase wird mit gesättigter Natriumhydrogencarbonatlösung gewaschen und über Natriumsulfat getrocknet. Nach Abdestillieren des Lösungsmittels werden 690 mg der Titelverbindung erhalten.
¹H-NMR, 300 MHz, (DMSO-*d*₆): δ = 1,83-2,09 (m, 4H); 2,14-2,31 (m, 5H); 2,85-2,98 (m, 2H); 4,61-4,78 (m, 1H); 7,06 (s, b, 1H); 7,57 (s, b, 1H); 8,02 (dd, 1H); 8,35 (d, 1H); 8,62 (s, 1H); 8,68 (d, 1H) ppm.

### Intermediat INT75

### 6-Amino-1-(1-methyl-4-piperidinyl)-1H-indol-3-carboxamid

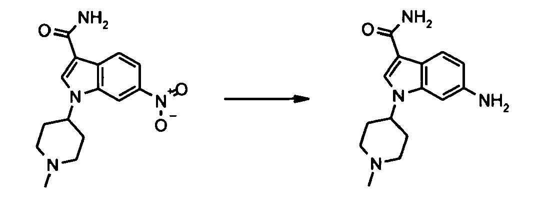

590 mg der als **INT74** beschriebenen Verbindung werden in 45 ml Ethanol gelöst, und mit 200 mg Palladium auf Kohle (10 %ig) versetzt. Es wird 20 Stunden unter Wasserstoffatmosphäre bei Raumtemperatur gerührt. Die Reaktionsmischung wird über Kieselgur filtriert und das Lösungsmittel wird am Rotationsverdampfer abkondensiert. Nach Reinigung durch Chromatographie an Aminophasenkieselgel werden 500 mg der Titelverbindung erhalten.
¹H-NMR, 400 MHz, (DMSO-*d*₆): δ = 1,99-2,12 (m, 4H); 2,12-2,25 (m, 2H); 2,36 (s, 3H); 2,94-3,09 (m, 2H); 4,00-4,12 (m, 1H); 5,60 (s, b, 2H); 6,63-6,77 (m, 2H); 7,64 (s, 1H); 7,73 (d, 1H) ppm.

### Intermediat INT76

### 3-Fluor-1-(1-methyl-4-piperidinyl)-6-nitro-1H-indol

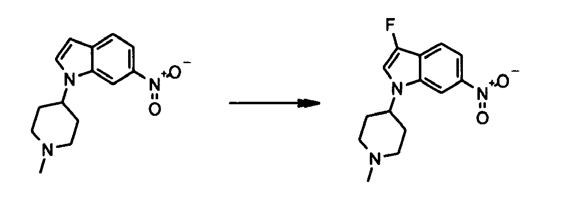

300 mg der als **INT29** beschriebenen Verbindung werden in 12 ml Acetonitril suspendiert und mit 320 mg 2,6-Dichlor-1-fluor-4-methylpyridinium tetrafluoroborat versetzt. Es wird eine Stunde bei 50°C gerührt. Es werden weitere 160 mg 2,6-Dichlor-1-fluor-4-methylpyridinium tetrafluoroborat zugegeben und es wird eine weitere Stunde bei 50°C gerührt. Es werden weitere 60 mg 2,6-Dichlor-1-fluor-4-methylpyridinium tetrafluoroborat zugegeben und es wird eine weitere Stunde bei 50°C gerührt. Die Reaktionsmischung wird mit halbgesättigter Natriumhydrogencarbonatlösung versetzt und mit einem Gemisch aus Dichlormethan und Methanol (100:1) extrahiert. Die organische Phase wird mit gesättigter Kochsalzlösung gewaschen und über Natriumsulfat getrocknet. Nach Reinigung durch Chromatographie an Kieselgel werden 60 mg der Titelverbindung erhalten.
¹H-NMR, 400 MHz, (DMSO-*d*₆): δ = 1,78-1,89 (m, 2H); 1,89-2,04 (m, 2H); 2,08-2,25 (m, 5H); 2,80-2,88 (m, 2H); 4,57-4,71 (m, 1H); 7,70 (d, 1H); 7,89 (dd, 1H); 8,05 (d, 1H); 8,67 (s, b, 1H) ppm.

### Intermediat INT77

### 3-Fluor-1-(1-methyl-4-piperidinyl)-1H-indol-6-amin

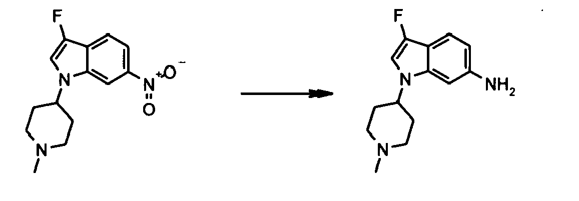

80 mg der als **INT76** beschriebenen Verbindung werden in 8 ml Tetrahydrofuran gelöst, und mit 3,2 ml einer 15%igen Titan(III)chlorid-Lösung versetzt. Es wird 17 Stunden bei Raumtemperatur gerührt. Die Reaktionsmischung wird mit zwei normaler Natronlauge basisch gestellt. Der Niederschlag wird abfiltriert, die Lösung wird mit einem Gemisch aus Dichlormethan und Methanol (100:1) extrahiert, mit gesättigter Kochsalzlösung gewaschen und über Natriumsulfat getrocknet. Nach Reinigung durch Chromatographie an Aminophasenkieselgel werden 30 mg der Titelverbindung erhalten.
¹H-NMR, 300 MHz, (DMSO-*d*₆): δ = 1,70-1,93 (m, 4H); 1,94-2,90 (m, 2H); 2,17 (s, 3H); 2,75-2,91 (m, 2H); 3,82-4,05 (m, 1H); 4,88 (s, b, 2H); 6,41 (dd, 1H); 6,52 (s, b, 1H); 7,02 (d, 1H); 7,11 (d, 1H) ppm.

### Intermediat INT78

### 1-(2-Methoxyethyl)-6-nitro-3-(1-pyrrolidinylmethyl)-1H-indol

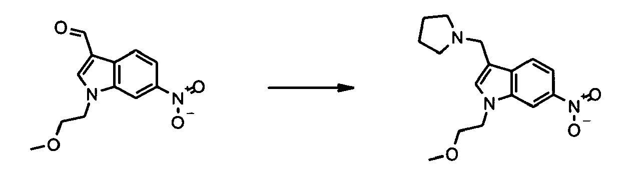

400 mg der als **INT17** beschriebenen Verbindung werden in 16 ml Dichlorethan gelöst und mit 0,16 ml Pyrrolidin, 0,11 ml Eisessig und 429 mg Natriumtrisacetoxyborhydrid versetzt. Es wird 64 Stunden bei Raumtemperatur gerührt. Die Reaktionsmischung wird mit Dichlormethan versetzt, mit gesättigter Natriumhydrogencarbonatlösung gewaschen und über Natriumsulfat getrocknet. Nach Reinigung durch Chromatographie an Kieselgel werden 460 mg der Titelverbindung erhalten.
¹H-NMR, 300 MHz, (CDCl₃): δ = 1,70-1,85 (m, 4H); 2,47-2,64 (m, 4H); 3,32 (s, 3H); 3,72 (t, 2H); 4,33 (t, 2H); 7,42 (s, 1H); 7,73 (d, 1H); 8,00 (dd, 1H); 8,32 (d, 1H) ppm.

### Intermediat INT79

### 1-(2-Methoxyethyl)-3-(1-pyrrolidinylmethyl)-1H-indol-6-amin

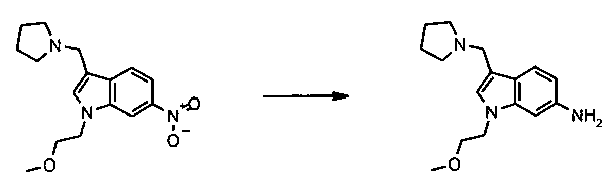

460 mg der als **INT78** beschriebenen Verbindung werden in 190 ml Ethanol gelöst, und mit 9 mg Raney-Nickel versetzt. Es wird 17 Stunden unter Wasserstoffatmosphäre bei Raumtemperatur gerührt. Die Reaktionsmischung wird über Aminophasenkieselgel filtriert und das Lösungsmittel wird am Rotationsverdampfer abkondensiert. Es werden 400 mg der Titelverbindung erhalten.
¹H-NMR, 300 MHz, (CDCl₃): δ = 1,75-1,86 (m, 4H); 2,50-2,69 (m, 4H); 3,36 (s, 3H); 3,28-3,74 (m, 6H); 4,18 (t, 2H); 6,60 (dd, 1H); 6,65 (d, 1H); 6,95 (s, 1H); 7,49 (d, 1H) ppm.

### Intermediat INT80

### 3-Chlor-6-nitro-1-[2-(1-pyrrolidinyl)ethyl]-1H-indol

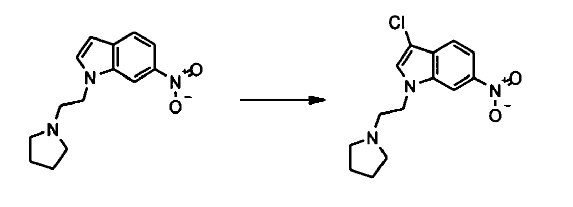

470 mg der als **INT9** beschriebenen Verbindung werden in 5 ml Acetonitril gelöst und mit 290 mg *N*-Chlorsuccinimid versetzt. Es wird 17 Stunden bei Raumtemperatur gerührt. Die Reaktionsmischung wird mit halbgesättigter Natriumhydrogencarbonatlösung versetzt und mit Essigsäureethylester extrahiert. Die organische Phase wird mit gesättigter Kochsalzlösung gewaschen und über Natriumsulfat getrocknet. Nach Reinigung durch Chromatographie an Kieselgel und anschließend an Aminophasenkieselgel werden 440 mg der Titelverbindung erhalten.
¹H-NMR, 400 MHz, (CDCl₃): δ = 1,71-1,86 (m, 4H); 2,50-2,62 (m, 4H); 2,91 (t, 2H); 4,29 (t, 2H); 7,47 (s, 1H); 7,66 (d, 1H); 8,05 (dd, 1H); 8,35 (d, 1H) ppm.

### Intermediat INT81

### 3-Chlor-1-[2-(1-pyrrolidinyl)ethyl]-1H-indol-6-amin

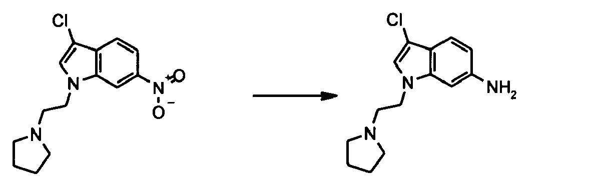

440 mg der als **INT80** beschriebenen Verbindung werden in 40 ml Tetrahydrofuran gelöst, und mit 11,6 ml einer 15%igen Titan(III)chlorid-Lösung versetzt. Es wird 15 Stunden bei Raumtemperatur gerührt. Die Reaktionsmischung wird Wasser versetzt und mit zwei normaler Natronlauge basisch gestellt. Der Niederschlag wird abfiltriert, und mit einem Gemisch aus Dichlormethan und Methanol (10:1) gewaschen. Die organische Phase wird abgetrennt, mit gesättigter Kochsalzlösung gewaschen und über Natriumsulfat getrocknet. Nach Reinigung durch Chromatographie an Aminophasenkieselgel werden 230 mg der Titelverbindung erhalten.
¹H-NMR, 300 MHz, (DMSO-*d*₆): δ = 1,57-1,74 (m, 4H); 2,41-2,52 (m, 4H); 2,73 (t, 2H); 4,05 (t, 2H); 4,97 (s, b, 2H); 6,51 (dd, 1H); 6,56 (d, 1H); 7,12 (d, 1H); 7,18 (s, 1H) ppm.

### Intermediat INT82

### 6-Nitro-1-[2-(1-pyrrolidinyl)ethyl]-1H-indol-3-carbaldehyd

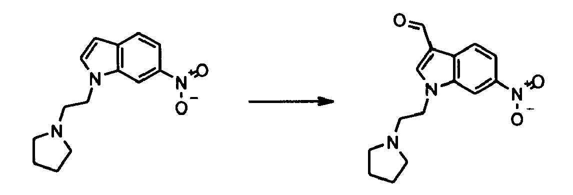

600 mg der als **INT9** beschriebenen Verbindung werden in 7 ml *N,N*-Dimethylformamid gelöst, und bei 0°C werden 0,26 ml Phosphoroxychlorid zugetropft. Es wird 15 Stunden bei Raumtemperatur gerührt. Die Reaktionsmischung wird auf Eiswasser gegossen, mit zwei normaler Natronlauge basisch gestellt und mit einem Gemisch aus Dichlormethan und Methanol (100:1) extrahiert. Die organische Phase wird mit gesättigter Natriumhydrogencarbonatlösung gewaschen und über Natriumsulfat getrocknet. Nach Reinigung durch Chromatographie an Kieselgel werden 450 mg der Titelverbindung erhalten.
¹H-NMR, 300 MHz, (DMSO-*d*₆): δ = 1,54-1,67 (m, 4H); 2,37-2,50 (m, 4H); 2,83 (t, 2H); 4,50 (t, 2H); 8,09 (dd, 1H); 8,22 (d, 1H); 8,57-8,68 (m, 2H); 9,96 (s, 1H) ppm.

### Intermediat INT83

### 3-[(4-Methyl-1-piperazinyl)methyl]-6-nitro-1-[2-(1-pyrrolidinyl)ethyl]-1H-indol

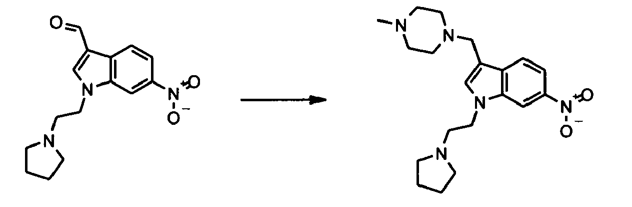

450 mg der als **INT82** beschriebenen Verbindung werden in 16 ml Dichlorethan gelöst und mit 0,21 ml 1-Methylpiperazin, 105 µl Eisessig und 420 mg Natriumtrisacetoxyborhydrid versetzt. Es wird 17 Stunden bei Raumtemperatur gerührt. Die Reaktionsmischung wird mit Dichlormethan versetzt, mit gesättigter Natriumhydrogencarbonatlösung gewaschen und über Natriumsulfat getrocknet. Nach Reinigung durch Chromatographie an Aminophasenkieselgel werden 460 mg der Titelverbindung erhalten.
¹H-NMR, 300 MHz, (CDCl₃): δ = 1,75-1,85 (m, 4H); 2,27 (s, 3H); 2,30-2,72 (m, 12H); 2,90 (t, 2H); 3,69 (s, 2H); 4,29 (t, 2H); 7,37 (s, 1H); 7,76 (d, 1H); 7,99 (dd, 1H); 8,32 (d, 1H) ppm.

### Intermediat INT84

### 3-[(4-Methyl-1-piperazinyl)methyl]-1-[2-(1-pyrrolidinyl)ethyl]-1H-indol-6-amin

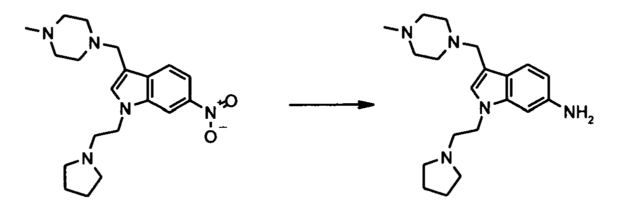

460 mg der als **INT83** beschriebenen Verbindung werden in 160 ml Ethanol gelöst, und mit 8 mg Raney-Nickel versetzt. Es wird 2 Stunden unter Wasserstoffatmosphäre bei Raumtemperatur gerührt. Die Reaktionsmischung wird über Kieselgur filtriert und das Lösungsmittel wird am Rotationsverdampfer abkondensiert. Nach Reinigung durch Chromatographie an Aminophasenkieselgel werden 360 mg der Titelverbindung erhalten.
¹H-NMR, 300 MHz, (CDCl₃): δ = 1,72-1,84 (m, 4H); 1,85-2,06 (m, b, 2H); 2,26 (s, 3H); 2,32-2,67 (m, 10H); 2,83 (t, 2H); 3,62 (s, b, 2H); 4,11 (t, 2H); 6,55 (dd, 1H); 6,61 (d, 1H); 6,86 (s, 1H); 7,47 (d, 1H) ppm.

### Intermediat INT85

### 2,2-Dimethyl-1-[1-(1-methyl-4-piperidinyl)-6-nitro-1H-indol-3-yl]-1-propanon

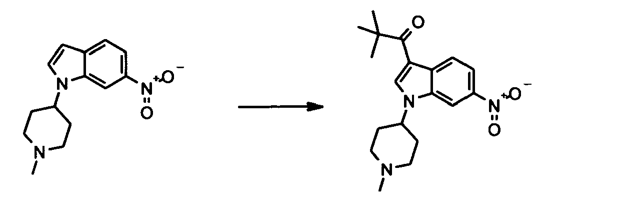

660 mg der als **INT29** beschriebenen Verbindung werden in 66 ml Dichlormethan gelöst und mit 0,94 ml Pivaloylchlorid und 135 mg Aluminium(III)chlorid versetzt. Es wird 15 Stunden bei Raumtemperatur gerührt. Es werden weitere 0,45 ml Pivaloylchlorid und 65 mg Aluminium(III)chlorid zugegeben und es wird weitere zwei Stunden bei Raumtemperatur gerührt. Die Reaktionsmischung wird mit verdünnter Natronlauge vesetzt und mit einem Gemisch aus Dichlormethan und Methanol (10:1) extrahiert. Die organische Phase wird mit gesättigter Kochsalzlösung gewaschen und über Natriumsulfat getrocknet. Nach Reinigung durch Chromatographie an Kieselgel und Umkristallisieren aus Ethanol werden 420 mg der Titelverbindung erhalten.
¹H-NMR, 300 MHz, (DMSO-*d*₆): δ = 1,36 (s, 9H); 1,84-2,03 (m, 2H); 2,12-2,36 (m, 7H); 2,85-2,97 (m, 2H); 4,65-4,84 (m, 1H); 8,08 (dd, 1H); 8,46 (d, 1H); 8,71 (d, 1H); 8,77 (s, 1H) ppm.

### Intermediat INT86

### 1-[6-Amino-1-(1-methyl-4-piperidinyl)-1H-indol-3-yl]-2,2-dimethyl-1-propanon

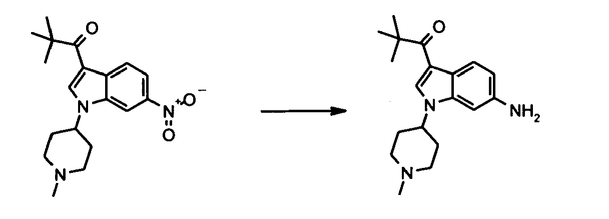

410 mg der als **INT85** beschriebenen Verbindung werden in 10 ml Ethanol gelöst, und mit 120 mg Palladium auf Kohle (10 %ig) versetzt. Es wird 15 Stunden unter Wasserstoffatmosphäre bei Raumtemperatur gerührt. Die Reaktionsmischung wird über Kieselgur filtriert und das Lösungsmittel wird am Rotationsverdampfer abkondensiert. Nach Reinigung durch Chromatographie an Aminophasenkieselgel werden 370 mg der Titelverbindung erhalten.
¹H-NMR, 400 MHz, (DMSO-*d*₆): δ = 1,27 (s, 9H); 1,82-2,02 (m, 2H); 2,08-2,24 (m, 2H); 2,25-2,43 (m, 5H); 2,93-3,13 (m, 2H); 4,10-4,29 (m, 1H); 4,60-5,50 (b, 2H); 6,50 (dd, 1H); 6,64 (d, 1H); 7,90 (d, 1H); 7,95 (s, 1H) ppm.

### Intermediat INT87

### (endo)-8-Methyl-3-(6-nitro-1H-indol-1-yl)-8-azabicyclo[3.2.1]octan

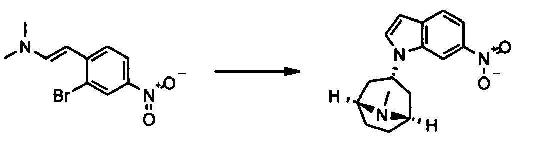

600 mg der als **INT28** beschriebenen Verbindung, 341 mg (endo)-8-Methyl-8-azabicyclo[3.2.1]octan-3-amin, 101 mg Tris(dibenzylidenaceton)dipalladium(0), 137 mg *rac*-BINAP und 425 mg Natrium-*tert*-butylat werden in 8 ml Toluol zwei Stunden bei 110°C gerührt. Die Reaktionsmischung wird mit Wasser und einem Gemisch aus Dichlormethan und Methanol (20:1) versetzt und mit vier normaler Salzsäure leicht angesäuert. Die organische Phase wird abgetrennt und die wässrige Phase wird mit ein normaler Natronlauge basisch gestellt und zweimal mit einem Gemisch aus Dichlormethan und Methanol (10:1) extrahiert. Die organischen Phasen werden vereinigt und mit gesättigter Natriumhydrogencarbonatlösung gewaschen und über Natriumsulfat getrocknet. Nach Reinigung durch Chromatographie an Kieselgel und anschließend an Aminophasenkieselgel werden 440 mg der Titelverbindung erhalten.
¹H-NMR, 300 MHz, (CDCl₃): δ = 1,52-1,74 (m, 4H); 2,15-2,32 (m, 5H); 2,62-2,82 (m, 2H); 3,26-3,42 (m, 2H); 4,76-4,96 (m, 1H); 6,59 (d, 1H); 7,48 (d, 1H); 7,62 (d, 1H); 7,99 (dd, 1H); 8,42 (d, 1H) ppm.

### Intermediat INT88

### (endo)-1-(8-Methyl-8-azabicyclo[3.2.1]oct-3-yl)-1H-indol-6-amin

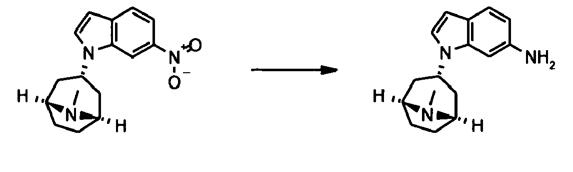

350 mg der als **INT87** beschriebenen Verbindung werden in 50 ml Ethanol gelöst, und mit 130 mg Palladium auf Kohle (10 %ig) versetzt. Es wird 17 Stunden unter Wasserstoffatmosphäre bei Raumtemperatur gerührt. Die Reaktionsmischung wird über Kieselgur filtriert und das Lösungsmittel wird am Rotationsverdampfer abkondensiert. Nach Reinigung durch Chromatographie an Aminophasenkieselgel werden 310 g der Titelverbindung erhalten.
¹H-NMR, 300 MHz, (DMSO-*d*₆): δ = 1,40-1,60 (m, 4H); 1,90-2,14 (m, 5H); 2,28-2,43 (m, 2H); 3,13-3,23 (m, 2H); 4,34-4,52 (m, 1H); 4,72 (s, 2H); 6,12 (d, 1H); 6,33 (dd, 1H); 6,48 (d, 1H); 7,04 (d, 1H); 7,10 (d, 1H) ppm.

### Intermediat INT89

### 6-Nitro-1-(1,2,2,6,6-pentamethyl-4-piperidinyl)-1H-indol

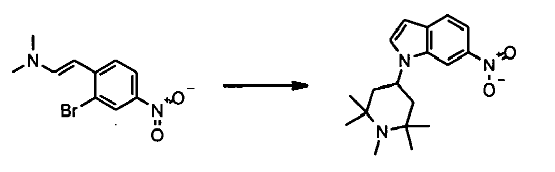

600 g der als **INT28** beschriebenen Verbindung, 0,28 ml 4-Amino-1,2,2,6,6-pentamethylpiperidin, 101 mg Tris(dibenzylidenaceton)dipalladium(0), 137 mg *rac-*BINAP und 425 mg Natrium-*tert*-butylat werden in 8 ml Toluol eine Stunde bei 110°C gerührt. Die Reaktionsmischung wird mit Wasser und Dichlormethan versetzt und mit vier normaler Salzsäure leicht angesäuert. Die organische Phase wird abgetrennt und die wässrige Phase wird mit ein normaler Natronlauge basisch gestellt und zweimal mit einem Gemisch aus Dichlormethan und Methanol (10:1) extrahiert. Die organischen Phasen werden vereinigt, nacheinander mit gesättigter Natriumhydrogencarbonatlösung und mit gesättigter Kochsalzlösung gewaschen und über Natriumsulfat getrocknet. Nach Reinigung durch Chromatographie an Aminophasenkieselgel werden 350 mg der Titelverbindung erhalten.
¹H-NMR, 400 MHz, (DMSO-*d*₆): δ = 1,12 (s, 6H); 1,18 (s, 6H); 1,80-1,89 (m, 4H); 2,21 (s, 3H); 4,78-4,97 (m, 1 H); 6,65 (d, 1 H); 7,70 (d, 1 H); 7,88 (dd, 1 H); 7,94 (d, 1 H); 8,57 (d, 1 H) ppm.

### Intermediat INT90

### 1-(1,2,2,6,6-Pentamethyl-4-piperidinyl)-1H-indol-6-amin

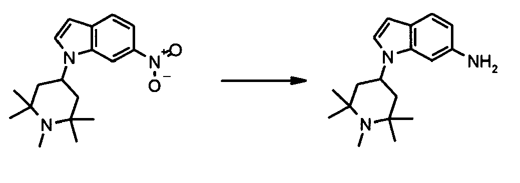

200 mg der als **INT89** beschriebenen Verbindung werden in 100 ml Ethanol gelöst, und mit 67 mg Palladium auf Kohle (10%ig) versetzt. Es wird 2 Stunden unter Wasserstoffatmosphäre bei Raumtemperatur gerührt. Die Reaktionsmischung wird über Kieselgur filtriert und das Lösungsmittel wird am Rotationsverdampfer abkondensiert. Es werden 180 mg der Titelverbindung erhalten.
¹H-NMR, 400 MHz, (DMSO-*d*₆): δ = 1,11 (s, 6H); 1,13 (s, 6H); 1,71-1,80 (m, 4H); 2,20 (s, 3H); 4,29-4,42 (m, 1H); 4,73 (s, b, 2H); 6,15 (d, 1H); 6,37 (dd, 1H); 6,57 (d, 1H); 7,05 (d, 1 H); 7,14 (d, 1H) ppm.

### Intermediat INT91

### (cis)-4-(6-Nitro-1H-indol-1-yl)cyclohexanamin

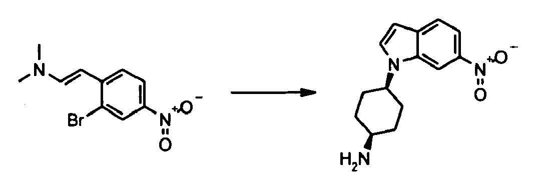

2,0 g der als **INT28** beschriebenen Verbindung, 1,26 g cis-1,4-Diaminocyclohexan, 337 mg Tris(dibenzylidenaceton)dipalladium(0), 459 mg *rac*-BINAP und 1,42 g Natrium-*tert*-butylat werden in 43 ml Toluol eine Stunde bei 110°C gerührt. Die Reaktionsmischung wird mit Wasser und Essigsäureethylester versetzt und mit vier normaler Salzsäure leicht angesäuert. Die organische Phase wird abgetrennt und die wässrige Phase wird mit ein normaler Natronlauge basisch gestellt und zweimal mit einem Gemisch aus Dichlormethan und Methanol (10:1) extrahiert. Die organische Phase wird mit gesättigter Kochsalzlösung gewaschen und über Natriumsulfat getrocknet. Nach Reinigung durch Chromatographie an Aminophasenkieselgel und anschließend an Kieselgel werden 635 mg der Titelverbindung erhalten.
¹H-NMR, 400 MHz, (DMSO-*d*₆): δ = 1,55 (s, b, 2H); 1,61-1,73 (m, 4H); 1,73-1,86 (m, 2H); 2,13-2,29 (m, 2H); 3,07-3,20 (m, 1H); 4,52-4,67 (m, 1H); 6,68 (d, 1H); 7,72 (d, 1 H); 7,90 (dd, 1 H); 8,00 (d, 1 H); 8,60 (d, 1 H) ppm.

### Intermediat INT92

### (cis)-N,N-Dimethyl-4-(6-nitro-1H-indol-1-yl)cyclohexanamin

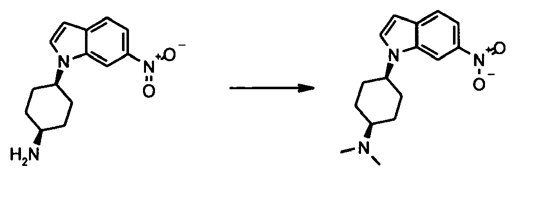

630 mg der als **INT91** beschriebenen Verbindung werden in 20 ml Methanol und 9 ml Dichlormethan gelöst und mit 730 mg Paraformaldehyd, 1,53 g Natriumcyanoborhydrid und 1,4 ml Eisessig versetzt. Es wird zwei Stunden bei Raumtemperatur gerührt. Die Reaktionsmischung wird mit Wasser versetzt, mit zwei molarer Natronlauge basisch gestellt und mit Essigsäureethylester extrahiert. Die organische Phase wird mit gesättigter Kochsalzlösung gewaschen und über Natriumsulfat getrocknet. Nach Reinigung durch Chromatographie an Kieselgel werden 590 mg der Titelverbindung erhalten.
¹H-NMR, 400 MHz, (DMSO-*d*₆): δ = 1,61-1,78 (m, 4H); 2,00-2,14 (m, 5H); 2,22 (s, 6H); 4,64-4,78 (m, 1 H); 6,67 (d, 1 H); 7,73 (d, 1 H); 7,87 (d, 1 H); 7,91 (dd, 1 H); 8,63 (d, 1 H) ppm.

### Intermediat INT93

### (cis)-1-[4-(Dimethylamino)cyclohexyl]-1H-indol-6-amin

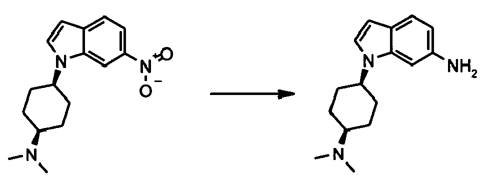

585 mg der als **INT92** beschriebenen Verbindung werden in 12 ml Ethanol gelöst, und mit 210 mg Palladium auf Kohle (10 %ig) versetzt. Es wird 15 Stunden unter Wasserstoffatmosphäre bei Raumtemperatur gerührt. Die Reaktionsmischung wird über Kieselgur filtriert und das Lösungsmittel wird am Rotationsverdampfer abkondensiert. Nach Reinigung durch Chromatographie an Aminophasenkieselgel werden 430 mg der Titelverbindung erhalten.
¹H-NMR, 300 MHz, (DMSO-*d*₆): δ = 1,41-1,74 (m, 4H); 1,85-2,05 (m, 5H); 2,16 (s, 6H); 4,00-4,18 (m, 1H); 4,70 (s, b, 2H); 6,14 (d, 1H); 6,35 (dd, 1H); 6,54 (d, 1H); 6,99 (d, 1H); 7,13 (d, 1H) ppm.

### Intermediat INT94

### (1α,2α)-2-(6-Nitro-1H-indol-1-yl)cyclohexanamin

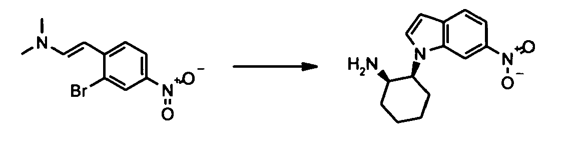

1,5 g der als **INT28** beschriebenen Verbindung, 1,250 g cis-1,2-Diaminocyclohexan Hydrochlorid, 253 mg Tris(dibenzylidenaceton)dipalladium(0), 344 mg *rac*-BINAP und 1,06 g Natrium-*tert*-butylat werden in 33 ml Toluol eine Stunde bei 110°C gerührt. Die Reaktionsmischung wird mit Wasser und Essigsäureethylester versetzt und mit vier normaler Salzsäure leicht angesäuert. Die organische Phase wird abgetrennt und die wässrige Phase wird mit ein normaler Natronlauge basisch gestellt und zweimal mit einem Gemisch aus Dichlormethan und Methanol (10:1) extrahiert. Die organische Phase wird mit gesättigter Kochsalzlösung gewaschen und über Natriumsulfat getrocknet. Nach Reinigung durch Chromatographie an Kieselgel werden 355 mg der Titelverbindung erhalten.
¹H-NMR, 300 MHz, (DMSO-*d*₆): δ = 1,71 (s, b, 2H); 1,33-1,71 (m, 7H); 2,25-2,42 (m, 1 H); 3,13-3,22 (m, 1 H); 4,69 (dt, 1 H); 6,59 (d, 1 H); 7,69 (d, 1 H); 7,82-7,90 (m, 2H); 8,54 (d, 1H) ppm.

### Intermediat INT95

### (1α,2α)-N,N-Dimethyl-2-(6-nitro-1H-indol-1-yl)cyclohexanamin

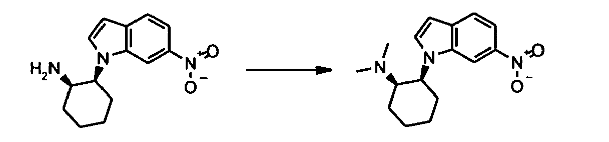

350 mg der als **INT94** beschriebenen Verbindung werden in 11 ml Methanol und 5 ml Dichlormethan gelöst und mit 405 mg Paraformaldehyd, 850 mg Natriumcyanoborhydrid und 0,77 ml Eisessig versetzt. Es wird zwei Stunden bei Raumtemperatur gerührt. Die Reaktionsmischung wird mit Wasser versetzt, mit zwei molarer Natronlauge basisch gestellt und mit Essigsäureethylester extrahiert. Die organische Phase wird mit gesättigter Kochsalzlösung gewaschen und über Natriumsulfat getrocknet. Nach Reinigung durch Chromatographie an Kieselgel werden 358 mg der Titelverbindung erhalten.
¹H-NMR, 300 MHz, (DMSO-*d*₆): δ = 1,35-1,54 (m, 3H); 1,63-1,94 (m, 10H); 2,01-2,15 (m, 1H); 2,52-2,62 (m, 1H); 4,96-5,08 (m, 1H); 6,60 (d, 1H); 7,68 (d, 1H); 7,87 (dd, 1H); 7,96 (d, 1H); 8,53 (d, 1H) ppm.

### Intermediat INT96

### (1α,2α)-1-[2-(Dimethylamino)cyclohexyl]-1H-indol-6-amin

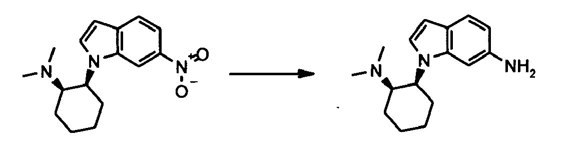

350 mg der als **INT95** beschriebenen Verbindung werden in 7 ml Ethanol gelöst, und mit 120 mg Palladium auf Kohle (10 %ig) versetzt. Es wird 15 Stunden unter Wasserstoffatmosphäre bei Raumtemperatur gerührt. Die Reaktionsmischung wird über Kieselgur filtriert und das Lösungsmittel wird am Rotationsverdampfer abkondensiert. Nach Reinigung durch Chromatographie an Aminophasenkieselgel werden 195 mg der Titelverbindung erhalten.
¹H-NMR, 300 MHz, (DMSO-*d*₆): δ = 1,28-1,58 (m, 3H); 1,58-1,92 (m, 10H); 1,99-2,18 (m, 1H); 2,50-2,60 (m, 1H); 4,39-4,47 (m, 1H); 4,69 (s, b, 2H); 6,11 (d, 1H); 6,36 (dd, 1H); 6,53 (d, 1H); 7,07-7,17 (m, 2H) ppm.

### Intermediat INT97

### 3-(1-Cyclopropyl-1,2,3,6-tetrahydro-4-pyridinyl)-1-methyl-5-nitro-1H-indol

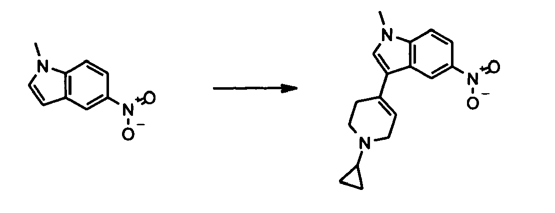

Bei der Umsetzung von 350 mg der als **INT1** beschriebenen Verbindung mit 995 mg 1 - Cyclopropyl-4-piperidinon gemäß der bei **INT2** beschriebenen Vorschrift werden 542 mg der Titelverbindung als Salz erhalten.
¹H-NMR, 300 MHz, (DMSO-*d*₆): δ = 0,79-1,11 (m, 4H); 2,77-2,91 (m, b, 2H); 2,94-3,11 (m, b, 1 H); 3,40-3,66 (m, b, 1 H); 3,68-3,82 (m, b, 1 H); 3,89 (s, 3H); 3,95-4,27 (m, b, 2H); 6,26 (s, b, 1 H); 7,72 (d, 1 H); 7,85 (s, 1 H); 8,12 (dd, 1 H); 8,74 (d, 1 H); 9,58 (b, 1 H) ppm.

### Intermediat INT98

### 3-(1-Cyclopropyl-4-piperidinyl)-1-methyl-1H-indol-5-amin

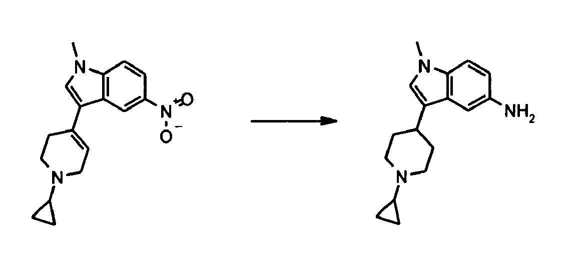

Bei der Umsetzung von 540 mg der als **INT97** beschriebenen Verbindung gemäß der bei **INT3** beschriebenen Vorschrift werden 245 mg der Titelverbindung erhalten.
¹H-NMR, 400 MHz, (DMSO-*d*₆): δ = 0,22-0,30 (m, 2H); 0,34-0,43 (m, 2H); 1,44-1,62 (m, 3H); 1,81 (d, b, 2H); 2,23 (t, b, 2H); 2,49-2,61 (m, 1H); 2,97 (d, b, 2H); 3,55 (s, 3H); 4,42 (s, b, 2H); 6,47 (dd, 1 H); 6,66 (d, 1 H); 6,79 (s, 1 H); 6,99 (d, 1 H) ppm.

### Intermediat INT99

### 3-(1-Ethyl-1,2,3,6-tetrahydro-4-pyridinyl)-1-methyl-5-nitro-1H-indol

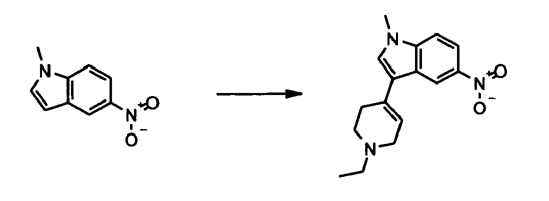

Bei der Umsetzung von 600 mg der als **INT1** beschriebenen Verbindung mit 1,77 ml 1-Ethyl-4-piperidinon gemäß der bei **INT2** beschriebenen Vorschrift werden 995 mg der Titelverbindung als Salz erhalten.
¹H-NMR, 400 MHz, (DMSO-*d*₆): δ = 1,26 (t, 3H); 2,74-2,83 (m, b, 2H); 3,16-3,33 (m, 3H); 3,62-3,72 (m, 1H); 3,72-3,82 (m, 1H); 3,85 (s, 3H); 4,00-4,13 (m, 1H); 6,21 (s, b, 1H); 7,68 (d, 1H); 7,81 (s, 1H); 8,08 (d, 1H); 8,70 (d, 1H); 9,61 (s, b, 1H) ppm.

### Intermediat INT100

### 3-(1-Ethyl-4-piperidinyl)-1-methyl-1H-indol-5-amin

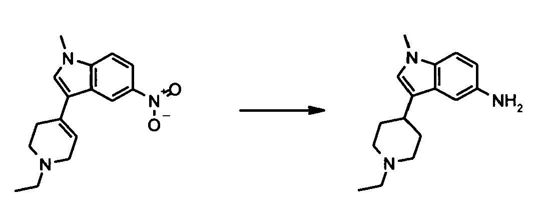

Bei der Umsetzung von 995 mg der als **INT99** beschriebenen Verbindung gemäß der bei **INT3** beschriebenen Vorschrift werden 470 mg der Titelverbindung erhalten.
¹H-NMR, 400 MHz, (DMSO-*d*₆): δ = 0,98 (t, 3H); 1,59 (dq, 2H); 1,83 (d, b, 2H); 1,93 (t, b, 2H); 2,31 (q, 2H); 2,48-2,56 (m, 1H); 2,91 (d, b, 2H); 3,56 (s, 3H); 4,43 (s, b, 2H); 6,47 (dd, 1H); 6,67 (d, 1H); 6,80 (s, 1H); 7,00 (d, 1H) ppm.

### Intermediat INT101

### 1-Methyl-3-[1-(2-methylpropyl)-1,2,3,6-tetrahydro-4-pyridinyl]-5-nitro-1H-indol

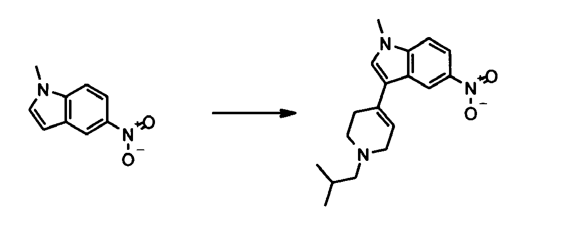

Bei der Umsetzung von 600 mg der als **INT1** beschriebenen Verbindung mit 2,2 ml 1-Isobutyl-4-piperidinon gemäß der bei **INT2** beschriebenen Vorschrift werden 1,1 g der Titelverbindung als Salz erhalten.
¹H-NMR, 300 MHz, (DMSO-*d*₆): δ = 0,88-1,05 (m, 6H); 1,99-2,20 (m, 1 H); 2,65-2,93 (m, 2H); 3,03 (t, 2H); 3,17-3,39 (m, 1H); 3,59-3,90 (m, 5H); 4,00-4,17 (m, 1H); 6,19 (s, b, 1H); 7,67 (d, 1H); 7,80 (s, 1H); 8,08 (dd, 1H); 8,71 (d, 1H); 9,41 (s, b, 1H) ppm.

### Intermediat INT102

### 1-Methyl-3-[1-(2-methylpropyl)-4-piperidinyl]-1H-indol-5-amin

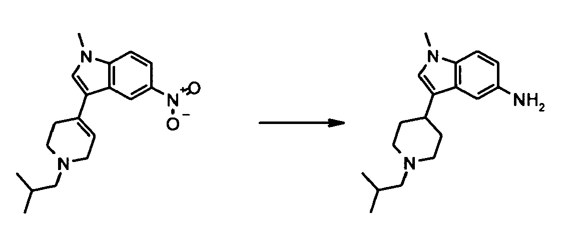

Bei der Umsetzung von 1,1 g der als **INT101** beschriebenen Verbindung gemäß der bei **INT3** beschriebenen Vorschrift werden 520 mg der Titelverbindung erhalten.
¹H-NMR, 300 MHz, (DMSO-*d*₆): δ = 0,73-0,91 (m, 6H); 1,47-2,05 (m, 9H); 2,49-2,57 (m, 1H); 2,78-2,95 (m, 2H); 3,56 (s, 3H); 4,44 (s, b, 2H); 6,47 (dd, 1H); 6,65 (d, 1H); 6,82 (s, 1H); 7,00 (d, 1H) ppm.

### Intermediat INT103

### 1-Methyt-3-[1-(1-methylethyl)-1,2,3,6-tetrahydro-4-pyridinyl]-5-nitro-1H-indol

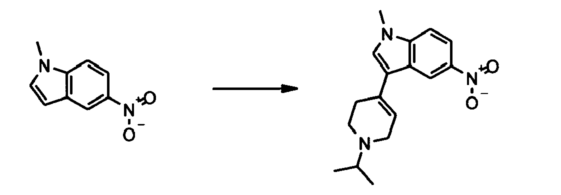

Bei der Umsetzung von 600 mg der als **INT1** beschriebenen Verbindung mit 1,92 ml 1-Isopropyl-4-piperidinon gemäß der bei **INT2** beschriebenen Vorschrift werden 811 mg der Titelverbindung als Salz erhalten.
¹H-NMR, 300 MHz, (DMSO-*d*₆), ausgewählte Signale: δ = 1,05-1,29 (m, b, 6H); 2,54-3,75 (m, 7H); 3,83 (s, 3H); 6,19 (s, b, 1H); 7,64 (d, 1H); 7,68-7,77 (s, b, 1H); 8,06 (dd, 1H); 8,68 (d, 1H) ppm.

### Intermediat INT104

### 1-Methyl-3-[1-(1-methylethyl)-4-piperidinyl]-1H-indol-5-amin

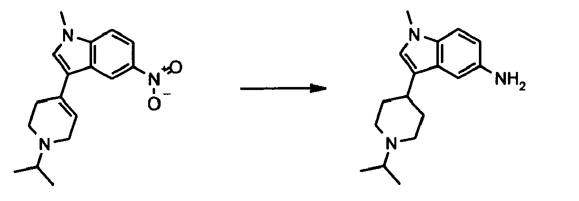

Bei der Umsetzung von 811 mg der als **INT103** beschriebenen Verbindung gemäß der bei **INT3** beschriebenen Vorschrift werden 270 mg der Titelverbindung erhalten.
¹H-NMR, 300 MHz, (DMSO-*d*₆): δ = 1,00 (d, 6H); 1,59 (dq, 2H); 1,89 (d, b, 2H); 2,23 (t, b, 2H); 2,53-2,76 (m, 2H); 2,86 (d, b, 2H); 3,61 (s, 3H); 4,46 (s, b, 2H); 6,51 (dd, 1H); 6,71 (d, 1H); 6,84 (s, 1H); 7,04 (d, 1H) ppm.

### Intermediat INT105

### 3-(1-Acetyl-1,2,3,6-tetrahydro-4-pyridinyl)-1-methyl-5-nitro-1H-indol

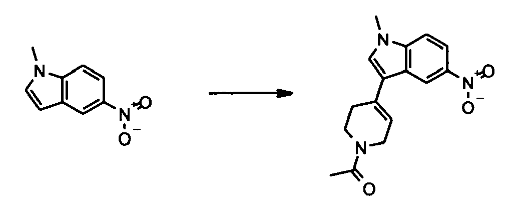

600 mg der als **INT1** beschriebenen Verbindung, suspendiert in 4 ml Essigsäure, werden bei 130°C zu einer Lösung von 1,6 ml 1-Acetyl-4-piperidinon in 5 ml Essigsäure und 6 ml Trifluoressigsäure gegeben und es wird eine Stunde bei 110°C gerührt. Die Reaktionsmischung wird eingeengt und nach Reinigung durch Chromatographie an Kieselgel und Umkristallisieren aus Essigsäureethylester werden 261 mg der Titelverbindung erhalten.
¹H-NMR, 300 MHz, (DMSO-*d*₆): δ = 1,98-2,11 (m, 3H); 2,49-2,61 (m, 2H); 3,64 (dt, 2H); 3,82 (s, 3H); 4,15 (m, 2H); 6,17 (s, b, 1H); 7,57-7,72 (m, 2H); 7,98-8,09 (m, 1H); 8,67 (dd, 1H) ppm.

### Intermediat INT106

### 3-(1-Acetyl-4-piperidinyl)-1-methyl-1H-indol-5-amin

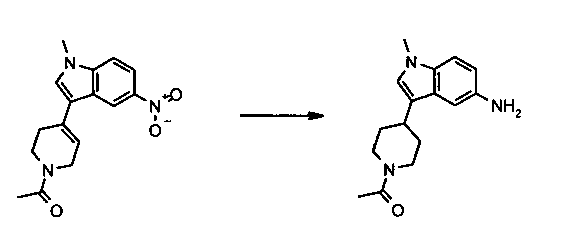

Bei der Umsetzung von 250 mg der als **INT105** beschriebenen Verbindung gemäß der bei **INT3** beschriebenen Vorschrift werden 199 mg der Titelverbindung erhalten.
¹H-NMR, 400 MHz, (DMSO-*d*₆): δ = 1,29-1,57 (m, 2H); 1,87 (t, b, 2H); 1,99 (s, 3H); 2,60 (t, b, 1H); 2,74-2,89 (m, 1H); 3,13 (t, b, 1H); 3,56 (s, 3H); 3,86 (d, b, 1H); 4,37-4,54 (m, 3H); 6,48 (dd, 1H); 6,67 (d, 1H); 6,83 (s, 1H); 7,01 (d, 1H) ppm.

### Intermediat INT107

### 2,3-Dihydro-1-(1-methyl-4-piperidinyl)-6-nitro-1H-indol

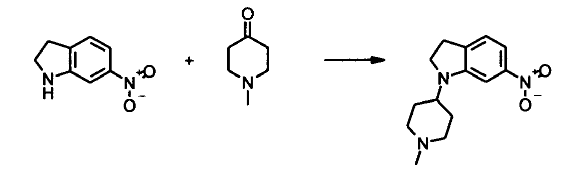

Eine Lösung von 25g 2,3-Dihydro-6-nitro-1*H*-indol und 17,77 ml 1-Methyl-4-piperidinon in 1,5 l Dichlormethan wird 30 Minuten bei Raumtemperatur gerührt, dann mit 64,25 g Natriumtriacetoxyborhydrid versetzt, und über Nacht bei Raumtemperatur gerührt. Der Ansatz wird mit gesättigter wässriger Natriumhydrogencarbonatlösung versetzt, 30 Minuten gerührt und mit Dichlormethan extrahiert. Die organische Phase wird mit Wasser gewaschen, über Natriumsulfat getrocknet und am Vakuum eingeengt. Flashchromatographie des Rückstands ergibt 8,65 g der Titelverbindung als dunkelrote Kristalle.
¹H-NMR (300 MHz, CDCl₃): δ = 1,74 (m, 2H); 1,78 (m, 2H); 2,08 (m, 2H); 2,31 (s, 3H); 2,96 (m, 2H); 3,01 (tbr, 2H); 3,39 (m, 1H); 3,51 (t, 2H); 7,05 (d, 1H); 7,06 (d, 1H); 7,45 (dd, 1H) ppm.

### Intermediat INT108

### 2,3-Dihydro-1-(1-methyl-4-piperidinyl)-1H-indol-6-amin

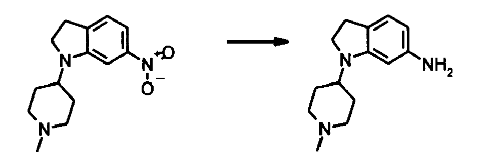

Eine Lösung von 525 mg der als **INT107** beschriebenen Verbindung in 20 ml Tetrahydrofuran wird mit 7,8 ml einer 10 Gewichts-prozentigen Lösung von Titan(III)chlorid in 20 bis 30 Gewichts-prozentiger wässriger Chlorwasserstofflösung versetzt. Hierbei tritt leichte Erwärmung auf. Das Reaktionsgemisch wird mit wässriger Natronlauge basisch gestellt und mit Ethylacetat extrahiert. Die organische Phase wird mit Wasser gewaschen, über Natriumsulfat getrocknet und am Vakuum eingeengt. Flashchromatographie des Rückstands ergibt 458 mg der Titelverbindung als hellbraunen Feststoff.
¹H-NMR (300 MHz, CD₃OD): δ = 1,72 (m, 2H); 1,77 (m, 2H); 2,14 (m, 2H); 2,30 (s, 3H); 2,77 (tbr, 2H); 2,96 (m, 2H); 3,30 (t, 2H); 3,37 (m, 1H); 5,99 (d, 1H); 6,04 (dd, 1H); 6,77 (d, 1H) ppm.

Die nachfolgenden Verbindungen werden analog nach dem für INT108 beschriebenen Verfahren hergestellt.

| Beispiel | Struktur Name | ¹H-NMR | Molmasse MS | Edukt |
|---|---|---|---|---|
| **INT108A** | 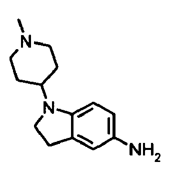 | (300 MHz, CDCl₃): δ = 1,71 (dddd, 2H); 1,77 (dbr, 2H); 2,02 (ddd, 2H); 2,29 (s, 3H); 2,85 (t, 2H); 2,94 (dbr, 2H); 3,16 (sbr, 2H); 3,24 (m, 1H); 3,27 (t, 2H); 6,27 (d, 1H); 6,44 (dd, 1H); 6,54 (sbr, 1H) ppm. | MW: 231,34 MS (ESI) [M+1]⁺: 232 | **INT109** |
| | 1-(1-Methyl-4-piperidinyl)-1*H*-indol-5-amin | | | |
| **INT108B** | 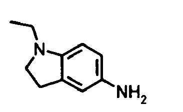 | (300 MHz, CDCl₃): δ = 1,07 (t, 3H); 2,71 (tbr, 2H); 2,89 (q, 1H); 3,06 (tbr, 2H); 6,27 (sbr, 2H); 6,40 (sbr, 1H) ppm. | MW: 162,24 MS (ESI) [M+1]⁺: 163 | **INT110** |
| | 1-Ethyl-1*H*-indol-5-amin | | | |

### Verfahren 2 zur Synthese von Intermediat INT30

### 1-(1-Methyl-4-piperidinyl)-1H-indol-6-amin

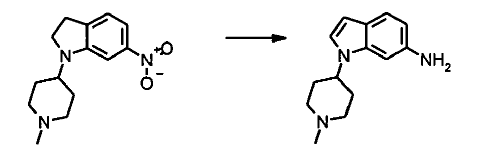

Eine Lösung von 560 mg der als **INT107** beschriebenen Verbindung in 21 ml Ethanol wird mit 114 mg Palladium auf Aktivkohle (5% Pd) 15 Minuten in Wasserstoffatmosphäre gerührt. Dann wird der Wasserstoff durch Stickstoff verdrängt. Das Reaktionsgemisch wird 5 Stunden zum Sieden erhitzt, nach dem Abkühlen über Celite^{®} filtriert, mit einem Gemisch aus Dichlormethan und Methanol (9:1) nachgewaschen und am Vakuum eingeengt. Flashchromatographie des Rückstands ergibt 321 mg der Titelverbindung als hellbraune Kristalle.

### Intermediate INT109 und INT110

### 2,3-Dihydro-1-(1-methyl-4-piperidinyl)-5-nitro-1H-indol (INT109) und 1-Ethyl-2,3-dihydro-5-nitro-1H-indol (INT110)

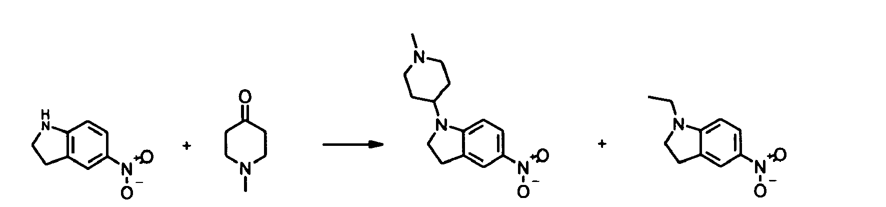

Eine Lösung von 1 g 2,3-Dihydro-6-nitro-1*H*-indol und 0,71 ml 1-Methyl-4-piperidinon in 60 ml Dichlormethan wird 15 Minuten bei Raumtemperatur gerührt, dann mit 2,57 g Natriumtriacetoxyborhydrid versetzt, über Nacht bei Raumtemperatur und 6 Stunden bei 50°C gerührt. Der Ansatz wird mit gesättigter wässriger Natriumhydrogencarbonatlösung versetzt, 30 Minuten gerührt und mit Dichlormethan extrahiert. Die organische Phase wird mit Wasser gewaschen, über Natriumsulfat getrocknet und am Vakuum eingeengt. Flashchromatographie des Rückstands ergibt 108 mg **INT109** als gelbe Kristalle und 305 mg **INT110** als orangefarbene Kristalle.
**INT109:** ¹H-NMR (300 MHz, CDCl₃): δ = 1,80 (m, 2H); 1,83 (m, 2H); 2,07 (m, 2H); 2,32 (s, 3H); 2,98 (m, 2H); 3,05 (tbr, 2H); 3,45 (m, 1H); 3,65 (t, 2H); 6,25 (d, 1H); 7,87 (dbr, 1H); 8,04 (dd, 1H) ppm.
**INT110**: ¹H-NMR (300 MHz, CDCl₃): δ = 1,21 (t, 3H); 3,06 (tbr, 2H); 3,30 (q, 1H); 3,65 (t, 2H); 6,26 (d, 1H); 7,87 (dbr, 1H); 8,05 (dd, 1H) ppm.

### Intermediat INT111

### 1,1-Dimethylethyl 4-(6-nitro-1H-indol-1-yl)-1-piperidincarboxylat

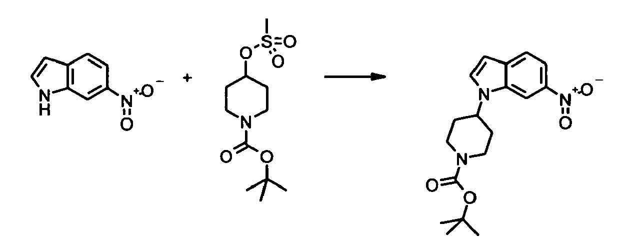

Zu einer Suspension von 1,23 g 60%igem Natriumhydrid in Paraffinöl in 3 ml *N,N-*Dimethylformamid wird unter Eisbadkühlung eine Lösung von 4,54 g 6-Nitro-1*H*-indol in 45 ml *N,N*-Dimethylformamid getropft. Die Mischung wird 30 Minuten bei Raumtemperatur nachgerührt. Dann wird der Ansatz auf 100°C erwärmt. Im Abstand von 30 Minuten wird bei dieser Temperatur eine Lösung von 7,82 g 1,1-Dimethylethyl 4-[(methylsulfonyl)oxy]-1-piperidincarboxylat in 50 ml *N,N*-Dimethylformamid in zwei Portionen hinzugetropft. Das Reaktionsgemisch wird über Nacht bei 100°C gerührt. Nach dem Abkühlen wird mit gesättigter wässriger Natriumhydrogencarbonatlösung verdünnt und mit Ethylacetat extrahiert. Die vereinigten organischen Phasen werden mit gesättigter wässriger Natriumhydrogencarbonatlösung und mit gesättigter wässriger Natriumchloridlösung gewaschen, über Natriumsulfat getrocknet und am Vakuum eingeengt. Flashchromatographie des Rückstands ergibt 2,26 g der Titelverbindung als gelben kristallinen Feststoff.
¹H-NMR (300 MHz, CDCl₃): δ = 1,50 (s, 9H); 1,93 (ddddbr, 2H); 2,11 (dbr, 2H); 2,97 (ddbr, 2H); 4,37 (m, 2H); 4,48 (tt, 1H); 6,64 (d, 1H); 7,47 (d, 1H); 7,66 (d, 1H); 8,01 (dd, 1H); 8,37 (d, 1H) ppm.

Die nachfolgenden Verbindungen werden analog nach dem für **INT111** beschriebenen Verfahren hergestellt.

| Beispiel | Struktur Name | ¹H-NMR | Molmasse MS | Edukt |
|---|---|---|---|---|
| **INT111A** | 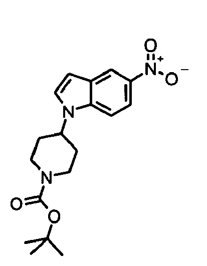 | (300 MHz, CDCl₃): δ = 1,50 (s, 9H); 1,93 (ddddbr, 2H); 2,09 (dbr, 2H); 2,94 (ddbr, 2H); 4,37 (m, 2H); 4,42 (tt, 1H); 6,72 (dbr, 1H); 7,33 (d, 1H); 7,40 (d, 1H); 8,12 (dd, 1H); 8,59 (d, 1H) ppm. | MW: 345,40 MS (ESI) [M+1]⁺: 346 | 5-Nitro-1*H*-indol |
| | 1,1-Dimethylethyl 4-(5-nitro-1*H*-indol-1-yl)-1-piperidincarboxylat | | | |
| **INT111B** | 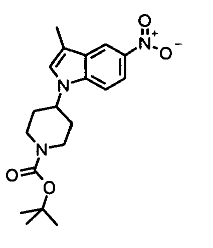 | (300 MHz, CDCl₃): δ = 1,49 (s, 9H); 1,89 (ddddbr, 2H); 2,04 (dbr, 2H); 2,35 (s, 3H); 2,92 (m, 2H); 4,34 (m, 2H); 4,35 (tt, 1H); 7,09 (sbr, 1H); 7,33 (d, 1H); 8,03 (dd, 1H); 8,52 (d, 1H) ppm. | MW: 359,43 MS (ESI) [M+1]⁺: 360 | 3-Methyl-5-nitro-1*H*-indol |
| | 1,1-Dimethylethyl 4-(3-methyl-5-nitro-1*H*-indol-1-yl)-1-piperidincarboxylat | | | |
| **INT111C** | 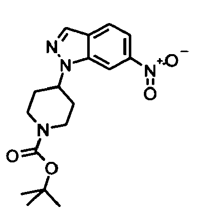 | (300 MHz, CDCl₃): δ = 1,50 (s, 9H); 2,04 (dbr, 2H); 2,26 (ddddbr, 2H); 3,01 (ddbr, 2H); 4,34 (m, 2H); 4,66 (tt, 1H); 7,85 (d, 1H); 8,03 (dd, 1H); 8,14 (s, 1H); 8,43 (m, 1H) ppm. | MW: 346,39 MS (ESI) [M+1]⁺: 347 | 6-Nitro-1*H*-indazol |
| | 1,1-Dimethylethyl 4-(6-nitro-1*H*-indazol-1-yl)-1-piperidincarboxylat | | | |
| **INT111D** | 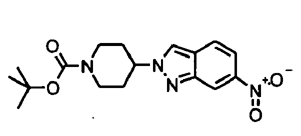 | (300 MHz, CDCl₃): δ = 1,49 (s, 9H); 2,11 (ddddbr, 2H); 2,27 (dbr, 2H); 2,97 (ddbr, 2H); 4,34 (m, 2H); 4,64 (tt, 1H); 7,76 (d, 1H); 7,90 (dd, 1H); 8,08 (s, 1H); 8,70 (m, 1H) ppm. | MW: 346,39 MS (ESI) [M+1]⁺: 347 | 6-Nitro-1*H*-indazol |
| | 1,1-Dimethylethyl 4-(6-nitro-2*H*-indazol-2-yl)-1-piperidincarboxylat | | | |
| **INT111E** | 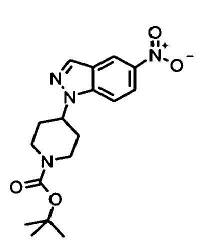 | (300 MHz, DMSO-*d*₆): δ = 1,43 (s, 9H); 1,91...1,97 (m, 2H); 3,0 (br, 2H); 4,11 (dbr, 2H); 4,96...5,04 (m, 1H); 7,97 (d, 1H); 8,24 (dd, 1H); 8,43 (s, 1H); 8,83 (d, 1H) ppm. | MW: 346,39 MS (ESI) [M+1]⁺: 347 | 5-Nitro-1*H*-indazol |
| | 1,1-Dimethylethyl 4-(5-nitro-1*H*-indazol-1-yl)-1-piperidincarboxylat | | | |
| **INT111F** | 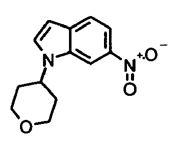 | (300 MHz, CDCl₃): δ = 2,09 (m, 2H); 2,13 (dddd, 2H); 3,67 (ddd, 2H); 4,19 (dbr, 2H); 4,58 (m, 1H); 6,65 (d, 1H); 7,51 (d, 1H); 7,66 (d, 1H); 8,02 (dd, 1H); 8,39 (d, 1H) ppm. | MW: 246,27 MS (ESI) [M+1]⁺: 247 | 6-Nitro-1*H*-indol und Tetrahydro-2H-pyran-4-ylmethan-sulfonat |
| | 6-Nitro-1-(tetrahydro-2*H*-pyran-4-yl)-1*H*-indol | | | |

### Intermediat INT112

### 6-Nitro-1-(4-piperidinyl)-1H-indol

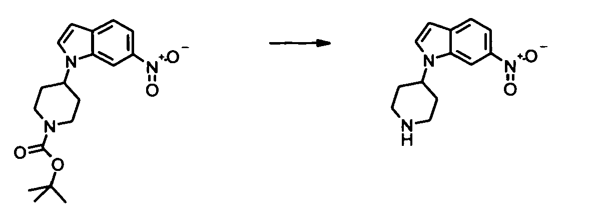

564 mg der als **INT111** beschriebenen Verbindung werden mit 1,26 ml Trifluoressigsäure in 16 ml Dichlormethan zwei Stunden bei Raumtemperatur gerührt. Das Reaktionsgemisch wird am Vakuum eingeengt. Flashchromatographie des Rückstands ergibt 357 mg der Titelverbindung als gelben kristallinen Feststoff.
¹H-NMR (300 MHz, CD₃OD): δ = 1,98 (dddd, 2H); 2,07 (dbr, 2H); 2,89 (ddd, 2H); 3,22 (dbr, 2H); 4,64 (tt, 1H); 6,66 (d, 1H); 7,67 (d, 1H); 7,73 (d, 1H); 7,95 (dd, 1H); 8,52 (d, 1H) ppm.

Die nachfolgenden Verbindungen werden analog nach dem für **INT112** beschriebenen Verfahren hergestellt.

| Beispiel | Struktur Name | ¹H-NMR | Molmasse MS | Edukt |
|---|---|---|---|---|
| **INT112 A** | 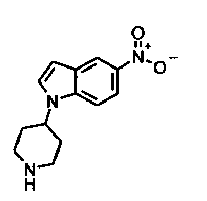 | (300 MHz, CDCl₃): δ = 1,96 (ddddbr, 2H); 2,04 (dbr, 2H); 2,85 (ddbr, 2H); 3,21 (dbr, 2H); 4,56 (tt, 1H); 6,73 (dbr, 1H); 7,58 (d, 1H); 7,63 (d, 1H); 8,06 (dd, 1H); 8,53 (d, 1H) ppm. | MW: 245,28 MS (ESI) [M+1]⁺: 246 | **INT111A** |
| | 5-Nitro-1-(4-piperidinyl)-1*H*-indol | | | |
| **INT112 B** | 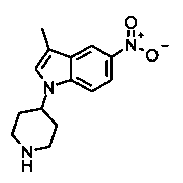 | (300 MHz, CDCl₃): δ = 1,93 (ddddbr, 2H); 2,00 (m, 2H); 2,35 (s, 3H); 2,83 (dddbr, 2H); 3,19 (dbr, 2H); 4,49 (m, 1H); 7,35 (sbr, 1H); 7,55 (d, 1H); 8,04 (dd, 1H); 8,47 (d, 1H) ppm. | MW: 259,31 MS (ESI) [M+1]⁺: 260 | **INT111B** |
| | 3-Methyl-5-nitro-1-(4-piperidinyl)-1*H*-indol | | | |
| **INT112 C** | 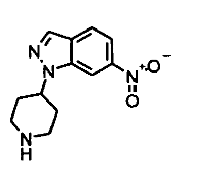 | (300 MHz, DMSO-*d*₆): δ = 1,88 (dbr, 2H); 2,00 (ddddbr, 2H); 2,74 (ddbr, 2H); 3,09 (dbr, 2H); 4,99 (tt, 1H); 7,95 (dd, 1H); 8,00 (d, 1H); 8,32 (s, 1H); 8,84 (m, 1H) ppm. | MW: 246,27 MS (ESI) [M+1]⁺: 247 | **INT111C** |
| | 6-Nitro-1-(4-piperidinyl)-1*H*-indazol | | | |
| **INT112 D** | 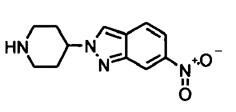 | (300 MHz, DMSO-*d*₆): δ = 1,96 (ddddbr, 2H); 2,07 (dbr, 2H); 2,65 (dddbr, 2H); 3,08 (dbr, 2H); 4,66 (tt, 1H); 7,80 (dd, 1H); 7,96 (d, 1H); 8,63 (m, 1H); 8,68 (s, 1H) ppm. | MW: 246,27 MS (ESI) [M+1]⁺: 247 | **INT111D** |
| | 6-Nitro-2-(4-piperidinyl)-2*H*-indazol | | | |
| **INT112 E** | 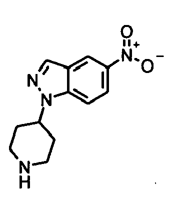 | (300 MHz, DMSO-*d*₆): δ = 1,84...1,89 (m, 2H); 1,93...2,07 (m, 2H); 2,70 (dt, 2H); 3,07...3,11 (m, 2H); 3,29 (br, =NH); 4,76...4,86 (m, 1H); 7,97 (d, 1H); 8,21 (dd, 1H); 8,41 (s, 1H); 8,82 (d, 1H) ppm. | MW: 246,27 MS (ESI) [M+1]⁺: 247 | **INT111E** |
| | 5-Nitro-1-(4-piperidinyl)-1*H*-indazol | | | |

### Intermediat INT113

### 1-[1-(1-Methylethyl)-4-piperidinyl]-6-nitro-1H-indol

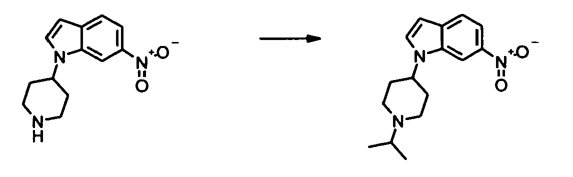

Eine Lösung von 543 mg der als **INT112** beschriebenen Verbindung und 0,49 ml Aceton in 22 ml Dichlormethan wird 30 Minuten bei Raumtemperatur gerührt, dann mit 700 mg Natriumtriacetoxyborhydrid versetzt, und über Nacht bei Raumtemperatur gerührt. Der Ansatz wird mit gesättigter wässriger Natriumhydrogencarbonatlösung versetzt, 60 Minuten gerührt und mit einem Gemisch aus Dichlormethan und Methanol (9:1) extrahiert. Die organische Phase wird mit gesättigter wässriger Natriumhydrogencarbonatlösung und mit gesättigter wässriger Natriumchloridlösung gewaschen, über Natriumsulfat getrocknet und am Vakuum eingeengt. Flashchromatographie des Rückstands ergibt 540 mg der Titelverbindung als gelben kristallinen Feststoff.
¹H-NMR (300 MHz, CDCl₃): δ = 1,11 (d, 6H); 2,06 (m, 2H); 2,13 (m, 2H); 2,43 (ddd, 2H); 2,85 (sept, 1H); 3,08 (dbr, 2H); 4,31 (tt, 1H); 6,62 (dbr, 1H); 7,53 (d, 1H); 7,65 (d, 1 H); 8,00 (dd, 1 H); 8,37 (dbr, 1 H) ppm.

Die nachfolgenden Verbindungen werden analog nach dem für **INT113** beschriebenen Verfahren hergestellt.

| Beispiel | Struktur Name | ¹H-NMR | Molmasse MS | Edukte |
|---|---|---|---|---|
| **INT113 A** | 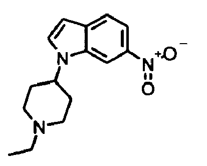 | (300 MHz, CDCl₃): δ = 1,16 (t, 3H); 2,06...2,28 (m, 6H); 2,53 (q, 2H); 3,17 (dbr, 2H); 4,34 (tt, 1 H); 6,62 (dbr, 1 H); 7,52 (d, 1 H); 7,65 (d, 1 H); 8,01 (dd, 1 H); 8,37 (dbr, 1H) ppm. | MW: 273,34 MS (ESI) [M+1]⁺: 274 | **INT112** Ethanal |
| | 1-(1-Ethyl-4-piperidinyl)-6-nitro-1*H*-indol | | | |
| **INT113 B** | 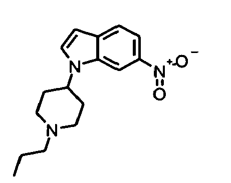 | (300 MHz, CDCl₃): δ = 0,95 (t, 3H); 1,57 (m, 2H); 2,01...2,28 (m, 6H); 2,41 (m, 2H); 3,14 (dbr, 2H); 4,33 (tt, 1H); 6,62 (dbr, 1H); 7,52 (d, 1H); 7,65 (d, 1H); 8,00 (dd, 1 H); 8,37 (dbr, 1 H) ppm. | MW: 287,36 MS (ESI) [M+1]⁺: 288 | **INT112** Propana l |
| | 6-Nitro-1-(1-propyl-4-piperidinyl)-1*H*-indol | | | |
| **INT113 C** | 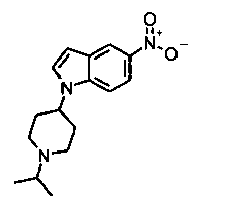 | (300 MHz, CDCl₃): δ = 1,10 (d, 6H); 2,07 (m, 2H); 2,11 (m, 2H); 2,40 (ddd, 2H); 2,84 (sept, 1H); 3,07 (dbr, 2H); 4,25 (m, 1 H); 6,70 (dbr, 1 H); 7,39 (d, 1H); 7,39 (d, 1H); 8,10 (dd, 1H); 8,58 (dbr, 1 H) ppm. | MW: 287,36 MS (ESI) [M+1]⁺: 288 | **INT112 A** Aceton |
| | 1-[1-(1-Methylethyl)-4-piperidinyl]-5-nitro-1*H*- indol | | | |
| **INT113 D** | 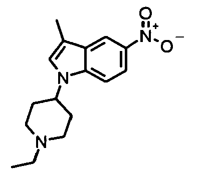 | (300 MHz, CDCl₃): δ = 1,14 (t, 3H); 2,00...2,21 (m, 6H); 2,35 (sbr, 3H); 2,50 (q, 2H); 3,14 (dbr, 2H); 4,21 (m, 1 H); 7,12 (sbr, 1 H); 7,32 (d, 1H); 8,08 (dd, 1 H); 8,52 (d, 1 H) ppm. | MW: 287,36 MS (ESI) [M+1]⁺: 288 | **INT112 B** Ethanal |
| | 1-(1-Ethyl-4-piperidinyl)-3-methyl-6-nitro-1*H*-indol | | | |
| **INT113 E** | 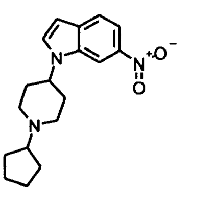 | (300 MHz, DMSO-*d*₆): δ = 1,35 (m, 2H); 1,52 (m, 2H); 1,62 (m, 2H); 1,82 (m, 2H); 1,94 (dbr, 2H); 2,01 (dddd, 2H); 2,21 (ddbr, 2H); 2,56 (m, 2H); 3,06 (dbr, 2H); 4,64 (tt, 1 H); 6,68 (dbr, 1 H); 7,72 (d, 1 H); 7,90 (dd, 1 H); 7,99 (d, 1 H); 8,62 (dbr, 1 H) ppm. | MW: 313,40 MS (ESI) [M+1]⁺: 314 | **INT112** Cyclope ntanon |
| | 1-(1-Cyclopentyl-4-piperidinyl)-6-nitro-1*H*-indol | | | |
| **INT113 F** | 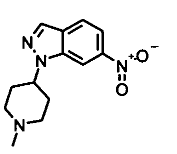 | (300 MHz, DMSO-*d*₆): δ = 1,87...1,94 (m, 2H); 2,07...2,19 (m, 4H); 2,24 (s, 3H); 2,88...2,92 (m, 2H); 4,88...4,96 (m, 1H); 7,94 (dd, 1H); 8,0 (dd, 1H); 8,33 (s, 1 H); 8,35 (m, 1 H) ppm. | MW: 260,30 MS (ESI) [M+1]⁺: 261 | **INT112 C** Formaldehyd |
| | 1-(1-Methyl-4-piperidinyl)-6-nitro-1*H*-indazol | | | |
| **INT113 G** | 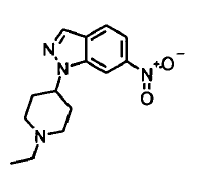 | (300 MHz, DMSO-*d*₆): δ = 1,04 (t, 3H); 1,90...1,94 (m, 2H); 2,06...2,20 (m, 4H); 2,40 (q, 2H); 2,99...3,01 (m, 2H); 4,89...4,97 (m, 1 H); 7,75 (dd, 1H); 8,0 (dd, 1H); 8,33 (s, 1 H); 8,85 (m, 1 H) ppm. | MW: 274,33 MS (ESI) [M+1]⁺: 275 | **INT112 C** Acetald ehyd |
| | 1-(1-Ethyl-4-piperidinyl)-6-nitro-1*H*-indazol | | | |
| **INT113 H** | 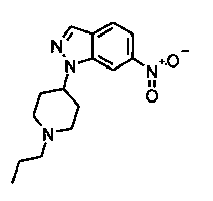 | (300 MHz, CDCl₃): δ = 0,94 (t, 3H); 1,56 (m, 2H); 2,05 (dbr, 2H); 2,21 (dddbr, 2H); 2,40 (tbr, 2H); 2,41 (ddddbr, 2H); 3,14 (dbr, 2H); 4,51 (tt, 1 H); 7,83 (d, 1H); 8,01 (dd, 1H); 8,12 (s, 1H); 8,45 (dbr, 1H) ppm. | MW: 288,35 MS (ESI) [M+1]⁺: 289 | **INT112 C** Propana l |
| | 6-Nitro-1-(1-propyl-4-piperidinyl)-1*H*-indazol | | | |
| **INT113 I** | 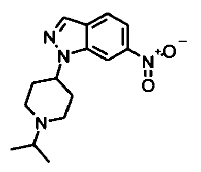 | (300 MHz, CDCl₃): δ = 1,11 (d, 6H); 2,07 (m, 2H); 2,38 (m, 2H); 2,44 (m, 2H); 2,86 (sept, 2H); 3,09 (dbr, 2H); 4,49 (m, 1 H); 7,83 (d, 1 H); 8,01 (dd, 1H); 8,11 (s, 1H); 8,45 (sbr, 1H) ppm. | MW: 288,35 MS (ESI) [M+1]⁺: 289 | **INT112 C** Aceton |
| | 1-[1-(1-Methylethyl)-4-piperidinyl]-6-nitro-1*H*-indazol | | | |
| **INT113 J** | 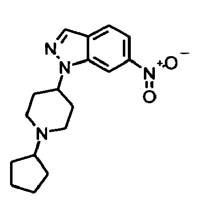 | (300 MHz, DMSO-*d*₆): δ = 1,31...1,43 (m, 2H); 1,46...1,65 (m, 4H); 1,78...1,87 (m, 2H); 1,90...1,95 (m, 2H); 2,05...2,26 (m, 4H); 2,57 (m, 1 H); 3,05...3,08 (dbr, 2H); 4,88...4,98 (m, 1H); 7,94 (dd, 1 H); 8,01 (dd, 1H); 8,33 (s, 1 H); 8,84 (m, 1H) ppm. | MW: 314,39 MS (ESI) [M+1]⁺: 315 | **INT112 C** Cyclopentano n |
| | 1-(1-Cyclopentyl-4-piperidinyl)-6-nitro-1*H*-indazol | | | |
| **INT113 K** | 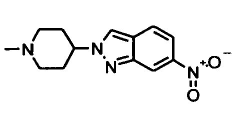 | (300 MHz, CDCl₃): δ = 2,13...2,37 (m, 6H); 2,37 (s, 3H); 3,06 (m, 2H); 4,50 (m, 1H); 7,75 (dbr, 1H); 7,90 (dd, 1H); 8,09 (sbr, 1H); 8,70 (dbr, 1 H) ppm. | MW: 260,30 MS (ESI) [M+1]⁺: 261 | **INT112 D** Parafor maldehyd |
| | 2-(1-Methyl-4-piperidinyl)-6-nitro-2*H*-indazol | | | |
| **INT113 L** | 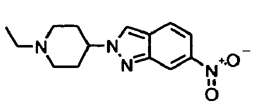 | (300 MHz, DMSO-*d*₆): δ = 1,03 (t, 3H); 2,08...2,16 (m, 6H); 2,40 (q, 2H); 2,99...3,03 (m, 2H); 4,55...4,64 (m, 1H); 7,81 (dd, 1 H); 7,96 (dd, 1 H); 8,64 (m, 1 H); 8,72 (m, 1 H) ppm. | MW: 274,33 MS (ESI) [M+1]⁺: 275 | **INT112 D** Ethanal |
| | 2-(1-Ethyl-4-piperidinyl)-6-nitro-2*H*-indazol | | | |
| **INT113 M** | 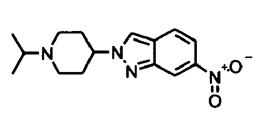 | (300 MHz, DMSO-*d*₆): δ = 1,02 (d, 6H); 2,07...2,19 (m, 4H); 2,28...2,45 (m, 2H); 2,82 (br, 1H); 2,95...2,99 (m, 2H); 4,53...4,64 (m, 1 H); 7,81 (dd, 1 H); 7,96 (dd, 1 H); 8,63 (m, 1 H); 8,61 (d, 1H)ppm. | MW: 288,35 MS (ESI) [M+1]⁺: 289 | **INT112 D** Aceton |
| | 2-[1-(1-Methylethyl)-4-piperidinyl]-6-nitro-2*H*-indazol | | | |
| **INT113 N** | 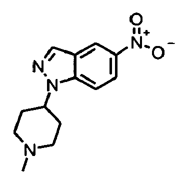 | (300 MHz, DMSO-*d*₆): δ = 1,87....1,95 (m, 2H); 2,12...2,17 (m, 4H); 2,23 (s, 3H); 2,89...2,92 (m, 2H); 4,67...4,77 (m, 1H); 7,96 (d, 1H); 8,21 (dd, 1 H); 8,42 (s, 1 H); 8,82 (d, 1 H) ppm. | MW: 260,30 MS (ESI) [M+1]⁺: 261 | **INT112 E** Formaldehyd |
| | 1-(1-Methyl-4-piperidinyl)-5-nitro-1*H*-indazol | | | |
| **INT113 O** | 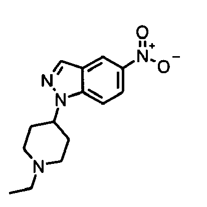 | (300 MHz, DMSO-*d*₆): δ = 1,04 (t, 3H); 1,90...1,94 (m, 2H); 2,07...2,17 (m, 4H); 2,40 (q, 2H); 3,0...3,02 (m, 2H); 4,71...4,78 (m, 1 H); 7,97 (d, 1H); 8,21 (dd, 1H); 8,42 (s, 1 H); 8,83 (d, 1 H) ppm. | MW: 274,33 MS (ESI) [M+1]⁺: 275 | **INT112 E** Ethanal |
| | 1-(1-Ethyl-4-piperidinyl)-5-nitro-1*H*-indazol | | | |
| **INT113 P** | 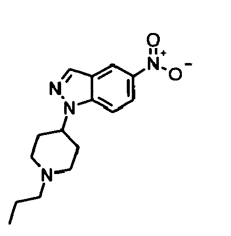 | (300 MHz, DMSO-*d*₆): δ = 0,88 (t, 3H); 1,41...1,54 (m, 2H); 1,89...1,93 (m, 2H); 2,10...2,20 (m, 4H); 2,29...2,34 (m, 2H); 2,99...3,02 (m, 2H); 4,69...4,80 (m, 1 H); 7,97 (d, 1 H); 8,21 (dd, 1 H); 8,42 (s, 1 H); 8,83 (d, 1 H) ppm. | MW: 288,35 MS (ESI) [M+1]⁺: 289 | **INT112 E** Propana l |
| | 5-Nitro-1-(1-propyl-4-piperidinyl)-1*H*-indazol | | | |
| **INT113 Q** | 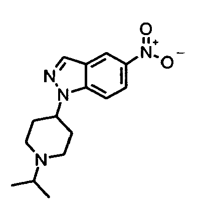 | (300 MHz, DMSO-*d*₆): δ = 1,06 (d, 6H); 1,96...2,01 (m, 2H); 2,09...2,22 (m, 2H); 2,51...2,60 (m, 2H); 2,87...2,98 (m, 1H); 3,02...3,07 (m, 2H); 4,71...4,81 (m, 1H); 7,92 (d, 1 H); 8,21 (dd, 1 H); 8,42 (s, 1 H); 8,81 (d, 1 H) ppm. | MW: 288,35 MS (ESI) [M+1]⁺: 289 | **INT112 E** Aceton |
| | 1-[1-(1-Methylethyl)-4-piperidinyl]-5-nitro-1*H*-indazol | | | |
| **INT113 R** | 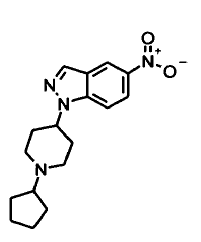 | (300 MHz, DMSO-*d*₆): δ = 1,30...1,42 (m, 2H); 1,47...1,65 (m, 4H); 1,77...1,86 (m, 2H); 1,85...1,94 (m, 2H); 2,05...2,22 (m, 4H); 2,52...2,62 (m, 1H); 3,06...3,09 (m, 2H); 4,69...4,79 (m, 1H); 7,96 (d, 1 H); 8,21 (dd, 1 H); 8,42 (s, 1 H); 8,82 (dd, 1 H) ppm. | MW: 314,39 MS (ESI) [M+1]⁺: 315 | **INT112 E** Cyclope ntanon |
| | 1-(1-Cyclopentyl-4-piperidinyl)-5-nitro-1*H*-indazol | | | |

### Intermediat INT114

### 1-(1-Methyl-4-piperidinyl)-5-nitro-1H-indol

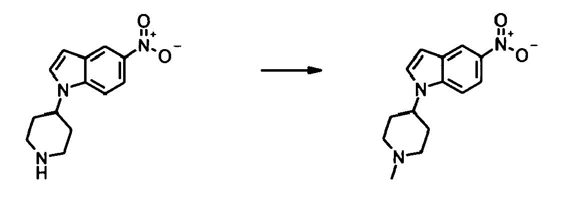

Zu einer Suspension von 196 mg 60%igem Natriumhydrid in Paraffinöl in 3 ml *N,N-*Dimethylformamid wird unter Eisbadkühlung eine Lösung von 1 g der als **INT112A** beschriebenen Verbindung in 24 ml *N,N*-Dimethylformamid getropft. 305 µl lodmethan werden hinzugegeben. Der Ansatz wird 2 Stunden bei Raumtemperatur gerührt, mit gesättigter wässriger Natriumhydrogencarbonatlösung versetzt, und mit Ethylacetat extrahiert. Die organische Phase wird über Natriumsulfat getrocknet und am Vakuum eingeengt. Flashchromatographie des Rückstands ergibt 666 mg der Titelverbindung als orange-gelben kristallinen Feststoff.
¹H-NMR (300 MHz, CDCl₃): δ = 2,00...2,25 (m, 6H); 2,35 (s, 3H); 3,02 (dbr, 2H); 4,24 (m, 1 H); 6,66 (d, 1 H); 7,35 (d, 1 H); 7,37 (d, 1 H); 8,05 (dd, 1 H); 8,53 (dbr, 1 H) ppm.

Die nachfolgenden Verbindungen werden analog nach dem für **INT114** beschriebenen Verfahren hergestellt.

| Beispiel | Struktur Name | ¹H-NMR | Molmasse MS | Edukte |
|---|---|---|---|---|
| **INT114 A** | 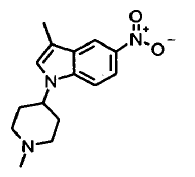 | (300 MHz, CDCl₃): δ = 2,03 (dbr, 2H); 2,08 (ddddbr, 2H); 2,19 (dddbr, 2H); 2,35 (d, 3H); 2,37 (s, 3H); 3,04 (dbr, 2H); 4,19 (tt, 1H); 7,12 (q, 1H); 7,32 (d, 1 H); 8,08 (dd, 1 H); 8,52 (d, 1 H) ppm. | MW: 273,34 MS (ESI) [M+1]⁺: 274 | **INT112 B** Iodmet han |
| | 3-Methyl-1-(1-methyl-4-piperidinyl)-5-nitro-1*H*-indol | | | |
| **INT114 B** | 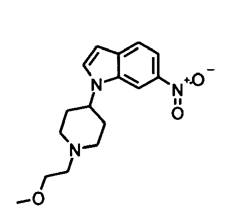 | (300 MHz, CDCl₃): δ = 2,08 (m, 2H); 2,15 (m, 2H); 2,29 (dddbr, 2H); 2,68 (t, 2H); 3,18 (dbr, 2H); 3,39 (s, 3H); 3,56 (t, 2H); 4,33 (m, 1 H); 6,61 (dbr, 1 H); 7,51 (d, 1H); 7,64 (d, 1H); 7,99 (dd, 1 H); 8,36 (dbr, 1 H) ppm. | MW: 303,36 MS (ESI) [M+1]⁺: 304 | **INT112** 1-Brom-2-methox yethan |
| | 1-[1-(2-Methoxyethyl)-4-piperidinyl]-6-nitro-1*H*-indol | | | |
| **INT114 C** | 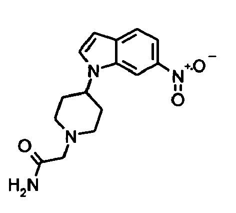 | (300 MHz, CDCl₃): δ = 2,12 (m, 4H); 2,49 (dddbr, 2H); 3,11 (dbr, 2H); 3,12 (s, 2H); 4,36 (m, 1H); 6,07 (sbr, 1H); 6,63 (dbr, 1H); 7,00 (sbr, 1H); 7,50 (d, 1H); 7,65 (d, 1H); 7,99 (dd, 1H); 8,35 (dbr, 1H) ppm. | MW: 302,34 MS (ESI) [M+1]⁺: 303 | **INT112** 2-Bromac etamid |
| | 4-(6-Nitro-1*H*-indol-1-yl)-1-piperidinacetamid | | | |
| **INT114 D** | 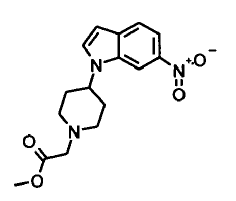 | (300 MHz, CDCl₃): δ = 2,11 (dbr, 2H); 2,19 (ddddbr, 2H); 2,52 (dddbr, 2H); 3,16 (dbr, 2H); 3,35 (s, 2H); 3,77 (s, 3H); 4,35 (tt, 1H); 6,63 (dbr, 1H); 7,51 (d, 1 H); 7,65 (d, 1 H); 8,01 (dd, 1H); 8,36 (dbr, 1H) ppm. | MW: 317,35 MS (ESI) [M+1]⁺: 318 | **INT112** Methyl bromac etat |
| | Methyl 4-(6-nitro-1*H*-indol-1-yl)-1-piperidinacetat | | | |
| **INT114 E** | 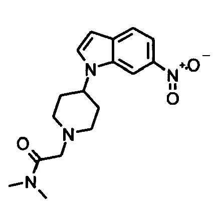 | (300 MHz, CDCl₃): δ = 2,09 (dbr, 2H); 2,17 (ddddbr, 2H); 2,42 (dddbr, 2H); 2,97 (s, 3H); 3,09 (s, 3H); 3,16 (dbr, 2H); 3,28 (s, 2H); 4,34 (ttbr, 1H); 6,61 (dbr, 1 H); 7,51 (d, 1 H); 7,64 (d, 1 H); 7,99 (dd, 1H); 8,36 (dbr, 1 H) ppm. | MW: 330,39 MS (ESI) [M+1]⁺: 331 | **INT112** 2-Chlor-*N,N*-dimeth ylaceta mid |
| | *N,N*-Dimethyl-4-(6-nitro-1*H*-indol-1-yl)-1-piperidinacetamid | | | |

### Intermediat INT115

### 1-(2-Brom-4-nitrophenyl)-N,N-dimethyl-1-propen-2-amin

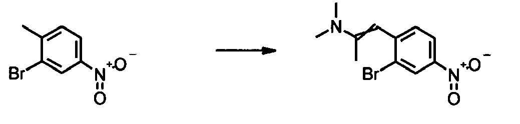

Eine Lösung von 5 g 2-Brom-1-methyl-4-nitrobenzen (CAS 7745-93-9) und 7,5 ml 1,1-Dimethoxy-*N,N*-dimethylethanamin in 6,5 ml *N,N*-Dimethylacetamid wird 4 Stunden bei 110°C gerührt. Das beim Abkühlen erstarrte Reaktionsgemisch wird in 5 ml Ethanol aufgeschlämmt und filtriert. Man erhält 3,27 g der Titelverbindung als rote Kristalle.
¹H-NMR (300 MHz, CDCl₃): δ = 2,12 (s, 3H); 2,99 (s, 6H); 5,27 (s, 1 H); 7,23 (d, 1 H); 8,03 (dd, 1H); 8,40 (d, 1H) ppm.

### Intermediat INT116

### 2-Methyl-1-(1-methyl-4-piperidinyl)-6-nitro-1H-indol

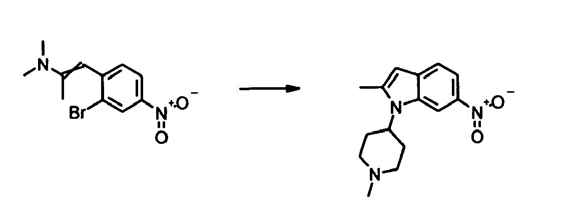

Eine Lösung von 1,44 g der als **INT115** beschriebenen Verbindung in 22 ml Toluol wird zusammen mit 634 mg 1-Methyl-4-piperidinamin, 231 mg Tris(dibenzylidenaceton)dipalladium(0), 314 mg [1,1'-Binaphthalen]-2,2'-diylbis[diphenylphosphin] und 534 mg Natrium-*tert*-butylat 90 Minuten bei 120°C gerührt. Nach dem Abkühlen wird mit gesättigter wässriger Natriumhydrogencarbonatlösung versetzt und mit Ethylacetat extrahiert. Die vereinigten organischen Phasen werden mit gesättigter wässriger Natriumchloridlösung gewaschen, über Natriumsulfat getrocknet und am Vakuum eingeengt. Flashchromatographie des Rückstands ergibt 210 mg der Titelverbindung als gelbe Kristalle.
¹H-NMR (300 MHz, CDCl₃): δ = 1,86 (dbr, 2H); 2,17 (dddbr, 2H); 2,37 (s, 3H); 2,52 (s, 3H); 2,58 (ddddbr, 2H); 3,07 (dbr, 2H); 4,22 (tt, 1 H); 6,34 (s, 1 H); 7,49 (d, 1 H); 7,95 (dd, 1 H); 8,48 (sbr, 1 H) ppm.

Die nachfolgenden Verbindungen werden analog aus nach dem für **INT116** beschriebenen Verfahren hergestellt.

| Beispiel | Struktur Name | ¹H-NMR | Molmas se MS | Edukte |
|---|---|---|---|---|
| **INT116A** | 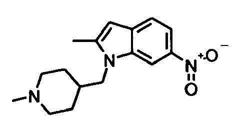 | (300 MHz, CDCl₃): δ = 1,46 (ddddbr, 2H); 1,56 (dbr, 2H); 1,87 (m, 1H); 1,88 (dddbr, 2H); 2,26 (s, 3H); 2,47 (s, 3H); 2,87 (dbr, 2H); 4,02 (d, 2H); 6,34 (s, 1H); 7,50 (d, 1 H); 7,96 (dd, 1H); 8,20 (d, 1 H) ppm. | MW: 287,36 MS (ESI) [M+1]⁺: 288 | **INT115** und 1-Methyl-4-piperidinmet hanamin |
| | 2-Methyl-1-[(1-methyl-4-piperidinyl)methyl]-6-nitro-1*H*-indol | | | |
| **INT116B** | 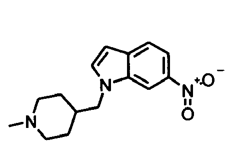 | (300 MHz, CDCl₃): δ = 1,38 (ddddbr, 2H); 1,55 (dbr, 2H); 1,82 (m, 1 H); 1,87 (dddbr, 2H); 2,24 (s, 3H); 2,83 (dbr, 2H); 4,06 (d, 2H); 6,56 (dbr, 1 H); 7,33 (d, 1 H); 7,62 (d, 1 H); 7,96 (dd, 1H); 8,28 (dbr, 1 H) ppm. | MW: 273,34 MS (ESI) [M+1]⁺: 274 | **INT28** und 1-Methyl-4-piperidinmet hanamin |
| | 1-[(1-Methyl-4-piperidinyl)methyl]-6-nitro-1*H*-indol | | | |
| **INT116C** | 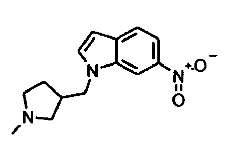 | (300 MHz, CDCl₃): δ = 1,78 (m, 1H); 2,13 (m, 1H); 2,54 (s, 3H); 2,66 (dd, 1 H); 2,68 (m, 1 H); 2,70 (m, 1 H); 2,78 (dd, 1H); 3,12 (m, 1 H); 4,29 (dd, 1 H); 4,38 (dd, 1H); 6,59 (dbr, 1H); 7,53 (d, 1 H); 7,65 (d, 1 H); 8,00 (dd, 1H); 8,37 (dbr, 1 H) ppm. | MW: 259,31 MS (ESI) [M+1]⁺: 260 | **INT28** und 1-Methyl-3-pyrrolidinme thanamin |
| | 1-[(1-Methyl-3-pyrrolidinyl)methyl]-6-nitro-1*H*-indol | | | |
| **INT116D** | 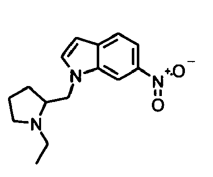 | (300 MHz, CDCl₃): δ = 1,04 (t, 3H); 1,52 (m, 1 H); 1,66 (m, 1 H); 1,79 (m, 1 H); 2,17 (m, 1H); 2,20 (m, 1H); 2,27 (m, 1H); 2,54 (m, 1H); 2,87 (m, 1H); 3,16 (m, 1H); 4,11 (dd, 1H); 4,20 (dd, 1H); 6,56 (dbr, 1 H); 7,47 (d, 1 H); 7,62 (d, 1 H); 7,97 (dd, 1 H); 8,36 (dbr, 1 H) ppm. | MW: 273,34 MS (ESI) [M+1]⁺: 274 | **INT28** und 1-Ethyl-2-pyrrolidinme thanamin |
| | 1-[(1-Ethyl-2-pyrrolidinyl)methyl]-6-nitro-1*H*-indol | | | |
| **INT116E** | 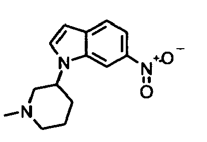 | (300 MHz, CDCl₃): δ = 1,38 (m, 3H); 2,12 (m, 2H); 2,31 (m, 1H); 2,33 (s, 3H); 2,81 (dbr, 1H); 3,05 (dbr, 1H); 4,57 (m, 1H); 6,59 (dbr, 1H); 7,62 (d, 1H); 7,62 (d, 1 H); 7,97 (dd, 1 H); 8,40 (dbr, 1 H) ppm. | MW: 259,31 MS (ESI) [M+1]⁺: 260 | **INT28** und (±)-1-Methyl-3-piperidinami n |
| | 1-(1-Methyl-3-piperidinyl)-6-nitro-1*H*-indol | | | |

### Verfahren 2 zur Synthese von Intermediat INT1

### 1-Methyl-5-nitro-1H-indol

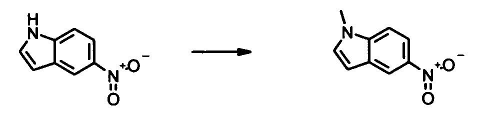

Zu einer Suspension von 7,4 g 60%igem Natriumhydrid in Paraffinöl in 100 ml *N,N-*Dimethylformamid wird unter Eisbadkühlung eine Lösung von 25 g 5-Nitro-1*H*-indol (CAS 6146-52-7) in 316 ml *N*,*N*-Dimethylformamid getropft. Die Mischung wird 15 Minuten nachgerührt. Dann werden 11,5 ml Iodmethan hinzugetropft. Das Reaktionsgemisch wird vier Stunden bei Raumtemperatur gerührt, anschließend auf 2 l halbkonzentrierte wässrige Natriumhydrogencarbonatlösung im Gemisch mit Eis gegossen und eine Stunde gerührt. Der Niederschlag wird abgesaugt. Man erhält 27 g der Titelverbindung als gelben kristallinen Feststoff.
¹H-NMR (300 MHz, CDCl₃): δ = 3,86 (s, 3H); 6,67 (dbr, 1 H); 7,20 (d, 1 H); 7,34 (d, 1 H); 8,13 (dd, 1H); 8,59 (sbr, 1H) ppm.

Die nachfolgende Verbindung wird analog nach dem für **INT1** beschriebenen Verfahren 2 hergestellt.

| Beispiel | Struktur Name | ¹H-NMR | Molmasse MS | Edukt |
|---|---|---|---|---|
| **INT117** | 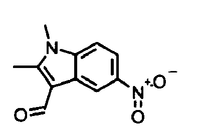 | (300 MHz, CDCl₃): δ = 2,74 (s, 3H); 3,78 (s, 3H); 7,33 (d, 1 H); 8,13 (dd, 1 H); 9,07 (dbr, 1H); 10,17 (s, 1H) ppm. | MW: 218,21 MS (ESI) [M+1]⁺: 219 | 2-Methyl-5-nitro-1*H*-indol-3-carbaldehyd; CAS 3558-17-6 |
| | 1,2-Dimethyl-5-nitro-1*H*-indol-3-carbaldehyd | | | |

### Intermediat INT118

### 1-(1-Methyl-5-nitro-1 H-indol-3-yl)-2-(1-pyrrolidinyl)-2-oxoethanon

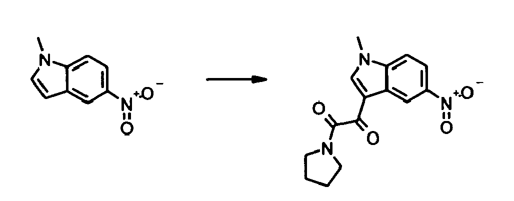

Zu einer Lösung von 1 g der als **INT1** beschriebenen Verbindung in 60 ml Dichlormethan werden vorsichtig 537 µl Oxalylchlorid getropft. Die Mischung wird 6 Stunden bei Raumtemperatur gerührt. Dann werden 1,41 ml Pyrrolidin hizugetropft. Das Reaktionsgemisch wird über Nacht bei Raumtemperatur gerührt. Der Ansatz wird mit gesättigter wässriger Natriumhydrogencarbonatlösung versetzt und mit Dichlormethan extrahiert. Die organische Phase wird mit Wasser gewaschen, über Natriumsulfat getrocknet und am Vakuum eingeengt. Flashchromatographie des Rückstands ergibt 794 mg der Titelverbindung als gelbliche Kristalle.
¹H-NMR (300 MHz, DMSO-*d*₆): δ = 1,83 (m, 2H); 1,87 (m, 2H); 3,45 (m, 2H); 3,50 (m, 2H); 3,98 (s, 3H); 7,84 (dbr, 1 H); 8,22 (dd, 1 H); 8,54 (s, 1 H); 8,99 (d, 1 H) ppm.

Die nachfolgenden Verbindungen werden analog aus **INT1** nach dem für **INT118** beschriebenen Verfahren hergestellt.

| Beispiel | Struktur Name | ¹H-NMR | Molmasse MS | Edukt |
|---|---|---|---|---|
| **INT118A** | 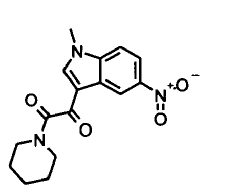 | (300 MHz, DMSO-*d*₆): δ = 1,46 (m, 2H); 1,63 (m, 4H); 3,32 (m, 2H); 3,60 (m, 2H); 3,98 (s, 3H); 7,86 (dbr, 1H); 8,23 (dd, 1H); 8,49 (s, 1 H); 8,96 (d, 1 H) ppm. | MW: 315,33 MS (ESI) [M+1]⁺: 316 | Piperidin |
| | 1-(1-Methyl-5-nitro-1*H*-indol-3-yl)-2-(1-piperidinyl)-2-oxoethanon | | | |
| **INT118B** | 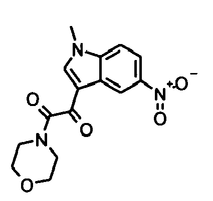 | (300 MHz, DMSO-*d*₆): δ = 3,33 (m, 2H); 3,39 (m, 2H); 3,64 (m, 2H); 3,73 (m, 2H); 3,99 (s, 3H); 7,86 (dbr, 1H); 8,24 (dd, 1H); 8,55 (s, 1 H); 8,97 (d, 1 H) ppm. | MW: 317,30 MS (ESI) [M+1]⁺: 318 | Morpholin |
| | 1-(1-Methyl-5-nitro-1*H*-indol-3-yl)-2-(4-morpholinyl)-2-oxoethanon | | | |
| **INT118C** | 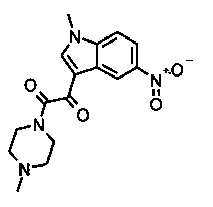 | (300 MHz, DMSO-*d*₆): δ = 2,20 (s, 3H); 2,27 (m, 2H); 2,42 (m, 2H); 3,35 (m, 2H); 3,63 (m, 2H); 3,99 (s, 3H); 7,86 (dbr, 1H); 8,24 (dd, 1H); 8,51 (s, 1H); 8,96 (d, 1H) ppm. | MW: 330,35 MS (ESI) [M+1]⁺: 331 | 1-Methylpiperazin |
| | 1-(1-Methyl-5-nitro-1*H*-indol-3-yl)-2-(4-methyl-1-piperazinyl)-2-oxoethanon | | | |
| **INT118D** | 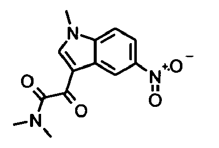 | (300 MHz, DMSO-*d*₆): δ = 2,95 (s, 3H); 3,01 (s, 3H); 3,97 (s, 3H); 7,86 (dbr, 1H); 8,23 (dd, 1H); 8,47 (s, 1H); 8,97 (d, 1H) ppm. | MW: 275,27 MS (ESI) [M+1]⁺: 276 | Dimethylamin, 2 M in Tetrahydrofuran |
| | 2-(Dimethylamino)-1-(1-methyl-5-nitro-1*H*-indol-3-yl)-2-oxoethanon | | | |
| **INT118E** | 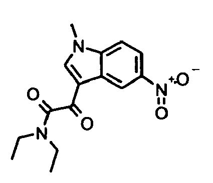 | (300 MHz, DMSO-*d*₆): δ = 1,10 (t, 3H); 1,21 (t, 3H); 3,27 (q, 2H); 3,47 (q, 2H); 3,98 (s, 3H); 7,85 (dbr, 1H); 8,22 (dd, 1H); 8,42 (s, 1 H); 8,95 (d, 1 H) ppm. | MW: 303,32 MS (ESI) [M+1]⁺: 304 | Diethylamin |
| | 2-(Diethylamino)-1-(1-methyl-5-nitro-1*H*-indol-3-yl)-2-oxoethanon | | | |

### Intermediat INT119

### 1-Methyl-5-nitro-3-[2-(1-pyrrolidinyl)ethyl]-1H-indol

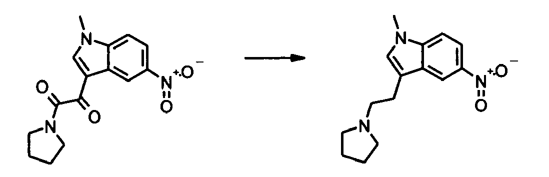

Zu einer Lösung von 790 mg der als **INT118** beschriebenen Verbindung in 24 ml Tertahydrofuran werden 853 mg Natriumcarbonat und 853 mg Cäsiumfluorid gegeben, sowie vorsichtig 21 ml Boran-Tetrahydrofuran-Komplex getropft. Die Mischung wird über Nacht bei Raumtemperatur gerührt. Dann wird mit gesättigter wässriger Natriumhydrogencarbonatlösung versetzt und mit Ethylacetat extrahiert. Die organische Phase wird mit Wasser gewaschen, über Natriumsulfat getrocknet und am Vakuum eingeengt. Flashchromatographie des Rückstands ergibt 268 mg der Titelverbindung als gelbliche Kristalle.
¹H-NMR (300 MHz, CDCl₃): δ = 1,95 (m, 4H); 2,79 (m, 4H); 3,05 (m, 2H); 3,35 (m, 2H); 3,81 (s, 3H); 7,07 (s, 1 H); 7,31 (d, 1 H); 8,14 (dd, 1 H); 8,53 (dbr, 1 H) ppm.

Die nachfolgenden Verbindungen werden analog nach dem für **INT119** beschriebenen Verfahren hergestellt.

| Beispiel | Struktur Name | ¹H-NMR | Molmasse MS | Edukt |
|---|---|---|---|---|
| **INT119A** | 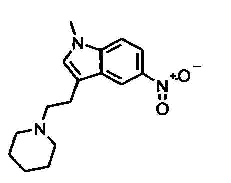 | (300 MHz, CDCl₃): δ = 1,47 (m, 2H); 1,64 (m, 4H); 2,51 (m, 4H); 2,63 (m, 2H); 2,97 (m, 2H); 3,79 (s, 3H); 7,03 (s, 1H); 7,27 (d, 1H); 8,10 (dd, 1H); 8,57 (dbr, 1 H) ppm. | MW: 287,36 MS (ESI) [M+1]⁺: 288 | **INT118A** |
| | 1-Methyl-5-nitro-3-[2-(1-piperidinyl)ethyl]-1*H*-indol | | | |
| **INT119B** | 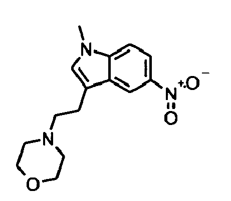 | (300 MHz, CDCl₃): δ = 2,55 (m, 4H); 2,66 (m, 2H); 2,96 (m, 2H); 3,76 (m, 4H); 3,80 (s, 3H); 7,05 (s, 1 H); 7,27 (d, 1 H); 8,10 (dd, 1H); 8,57 (dbr, 1H) ppm. | MW: 289,34 MS (ESI) [M+1]⁺: 290 | **INT118B** |
| | 1-Methyl-3-[2-(4-morpholinyl)ethyl]-5-nitro-1*H*-indol | | | |
| **INT119C** | 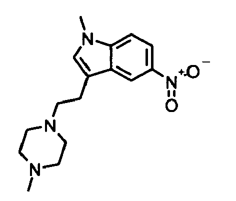 | (300 MHz, CDCl₃): δ = 2,62 (m, 4H); 2,66 (s, 3H); 2,75 (m, 2H); 2,95 (m, 2H); 3,12 (m, 4H); 3,81 (s, 3H); 7,04 (s, 1H); 7,29 (d, 1H); 8,11 (dd, 1H); 8,59 (dbr, 1H) ppm. | MW: 302,38 MS (ESI) [M+1]⁺: 303 | **INT118C** |
| | 1-Methyl-3-[2-(4-methyl-1-piperazinyl)ethyl]-5-nitro-1*H*-indol | | | |
| **INT119D** | 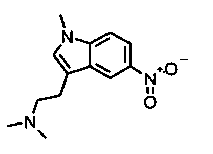 | (300 MHz, CDCl₃): δ = 2,31 (s, 6H); 2,60 (m, 2H); 2,91 (m, 2H); 3,77 (s, 3H); 7,03 (s, 1H); 7,24 (d, 1H); 8,06 (dd, 1H); 8,51 (dbr, 1 H) ppm. | MW: 247,30 MS (ESI) [M+1]⁺: 248 | **INT118D** |
| | *N,N*,1-Trimethyl-5-nitro-1*H*-indol-3-ethanamin | | | |
| **INT119E** | 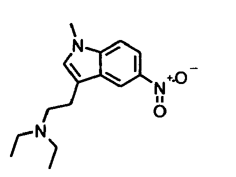 | (300 MHz, CDCl₃): δ = 1,22 (t, 6H); 2,90 (m, 2H); 2,97 (q, 4H); 2,97 (m, 2H); 3,82 (s, 3H); 7,06 (s, 1 H); 7,31 (d, 1H); 8,13 (dd, 1H); 8,53 (dbr, 1 H) ppm. | MW: 275,35 MS (ESI) [M+1]⁺: 276 | **INT118E** |
| | *N,N*-Diethyl-1-methyl-5-nitro-1*H*-indol-3-ethanamin | | | |

### Intermediat INT120

### 1,2-Dimethyl-3-(4-morpholinylmethyl)-5-nitro-1H-indol

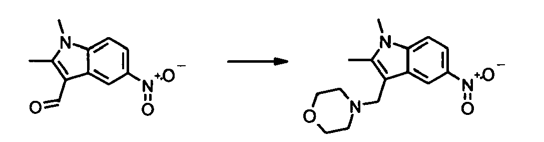

Eine Lösung von 500 mg der als **INT117** beschriebenen Verbindung und 265 µl Morpholin in 23 ml Dichlormethan wird 60 Minuten bei Raumtemperatur gerührt, dann mit 967 mg Natriumtriacetoxyborhydrid versetzt, und über Nacht bei Raumtemperatur gerührt. Der Ansatz wird mit gesättigter wässriger Natriumhydrogencarbonatlösung versetzt, 30 Minuten gerührt und mit Dichlormethan extrahiert. Die organische Phase wird mit gesättigter wässriger Natriumhydrogencarbonatlösung und mit gesättigter wässriger Natriumchloridlösung gewaschen, über Natriumsulfat getrocknet und am Vakuum eingeengt. Flashchromatographie des Rückstands ergibt 497 mg der Titelverbindung als gelben kristallinen Feststoff.
¹H-NMR (300 MHz, CDCl₃): 8 = 2,44 (s, 3H); 2,45 (m, 4H); 3,65 (s, 2H); 3,68 (m, 4H); 3,72 (s, 3H); 7,24 (d, 1 H); 8,05 (dd, 1 H); 8,62 (dbr, 1 H) ppm.

Die nachfolgenden Verbindungen werden analog nach dem für **INT120** beschriebenen Verfahren hergestellt.

| Beispiel | Struktur Name | ¹H-NMR | Molmasse MS | Edukte |
|---|---|---|---|---|
| **INT120A** | 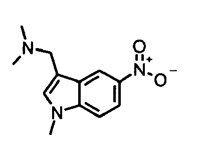 | (300 MHz, CDCl₃): δ = 2,27 (s, 6H); 3,61 (s, 2H); 3,81 (s, 3H); 7,14 (s, 1H); 7,28 (d, 1H); 8,09 (dd, 1H); 8,64 (d, 1 H) ppm. | MW: 233,27 MS (ESI) [M+1]⁺: 234 | **INT121B** und 2M Dimethylamin in Tetrahydrofuran |
| | *N,N*,1-Trimethyl-5-nitro-1*H*-indol-3-methanamin | | | |
| **INT120B** | 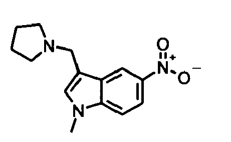 | (300 MHz, CDCl₃): δ = 1,79 (m, 4H); 2,57 (m, 4H); 3,82 (s, 2H); 3,82 (s, 3H); 7,18 (s, 1H); 7,29 (d, 1H); 8,11 (dd, 1H); 8,67 (d, 1 H) ppm. | MW: 259,31 MS (ESI) [M+1]⁺: 260 | **INT121B** und Pyrrolidin |
| | 1-Methyl-5-nitro-3-(1-pyrrolidinylmethyl)-1*H*-indol | | | |
| **INT120C** | 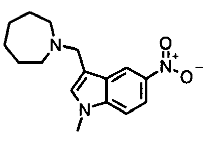 | (300 MHz, CDCl₃): δ = 1,62 (m, 8H); 2,66 (m, 4H); 3,81 (s, 2H); 3,81 (s, 3H); 7,13 (s, 1 H); 7,28 (d, 1H); 8,11 (dd, 1 H); 8,75 (d, 1H)ppm. | MW: 287,36 MS (ESI) [M+1]⁺: 288 | **INT121B** und Hexahydro-1*H*-azepin |
| | 3-[(Hexahydro-1*H*-azepin-1-yl)methyl]-1-methyl-5-nitro-1*H*-indol | | | |
| **INT120D** | 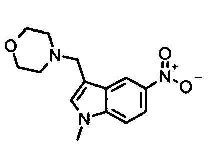 | (300 MHz, CDCl₃): δ = 2,48 (m, 4H); 3,69 (s, 2H); 3,71 (m, 4H); 3,82 (s, 3H); 7,13 (s, 1H); 7,30 (d, 1H); 8,13 (dd, 1 H); 8,72 (d, 1H) ppm. | MW: 275,31 MS (ESI) [M+1]⁺: 276 | **INT121B** und Morpholin |
| | 1-Methyl-3-(4-morpholinylmethyl)-5-nitro-1*H*-indol | | | |
| **INT120E** | 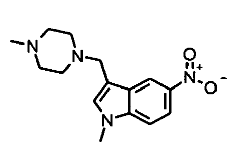 | (300 MHz, CDCl₃): = 2,28 (s, 3H); 2,47 (m, 8H); 3,71 (s, 2H); 3,81 (s, 3H); 7,12 (s, 1H); 7,29 (d, 1H); 8,12 (dd, 1H); 8,71 (d, 1 H) ppm. | MW: 288,35 MS (ESI) [M+1]⁺: 289 | **INT121B** und 1-Methylpiperazin |
| | 1-Methyl-3-[(4-methyl-1-piperazinyl)methyl]-5-nitro-1*H*-indol | | | |
| **INT120F** | 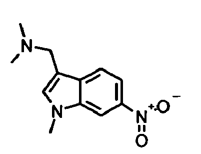 | (300 MHz, CDCl₃): δ = 2,28 (s, 6H); 3,63 (s, 2H); 3,87 (s, 3H); 7,31 (s, 1H); 7,73 (d, 1H); 8,01 (dd, 1H); 8,29 (d, 1 H) ppm. | MW: 233,27 MS (ESI) [M+1]⁺: 234 | **INT121C** und 2 M Dimethylamin in Tetrahydrofuran |
| | *N,N*,1-Trimethyl-6-nitro-1*H*-indol-3-methanamin | | | |
| **INT120G** | 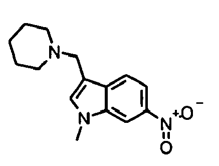 | (300 MHz, CDCl₃): δ = 1,43 (m, 2H); 1,57 (m, 4H); 2,42 (m, 4H); 3,66 (s, 2H); 3,86 (s, 3H); 7,30 (s, 1H); 7,72 (d, 1 H); 8,00 (dd, 1H); 8,28 (d, 1H) ppm. | MW: 273,34 MS (ESI) [M+1]⁺: 274 | **INT121C** und Piperidin |
| | 1-Methyl-6-nitro-3-(1-piperidinylmethyl)-1*H*-indol | | | |
| **INT120H** | 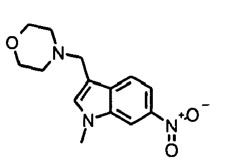 | (300 MHz, CDCl₃): δ = 2,47 (m, 4H); 3,68 (s, 2H); 3,70 (m, 4H); 3,87 (s, 3H); 7,28 (s, 1H); 7,79 (d, 1H); 8,01 (dd, 1H); 8,29 (d, 1 H) ppm. | MW: 275,31 MS (ESI) [M+1]⁺: 276 | **INT121C** und Morpholin |
| | 1-Methyl-3-(4-morpholinylmethyl)-6-nitro-1*H*-indol | | | |
| **INT120I** | 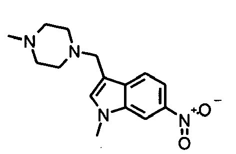 | (300 MHz, CDCl₃): δ = 2,28 (s, 3H); 2,45 (m, 4H); 2,52 (m, 4H); 3,70 (s, 2H); 3,86 (s, 3H); 7,28 (s, 1H); 7,78 (d, 1 H); 8,00 (dd, 1 H); 8,28 (d, 1H) ppm. | MW: 288,35 MS (ESI) [M+1]⁺: 289 | **INT121C** und 1-Methylpiperazin |
| | 1-Methyl-3-[(4-methyl-1-piperazinyl)methyl]-6-nitro-1*H*-indol | | | |
| **INT120J** | 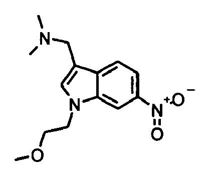 | (300 MHz, DMSO-*d*₆): δ = 2,28 (s, 6H); 3,32 (s, 3H); 3,63 (s, 2H); 3,72 (t, 2H); 4,34 (t, 2H); 7,41 (s, 1 H); 7,73 (d, 1 H); 8,00 (dd, 1 H); 8, 32 (d, 1 H) ppm. | MW: 277,33 MS (ESI) [M+1]⁺: 278 | **INT17** und 2 M Dimethylamin in Tetrahydrofuran |
| | 1-(2-Methoxyethyl)-*N,N*-dimethyl-6-nitro-1*H*-indol-3-methanamin | | | |
| **INT120K** | 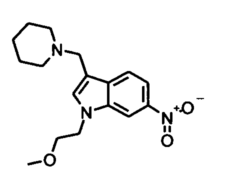 | (300 MHz, DMSO-*d*₆): δ = 1,42 (m, 2H); 1,57 (m, 4H); 2,42 (m, 4H); 3,32 (s, 3H); 3,66 (s, 2H); 3,72 (t, 2H); 4,33 (t, 2H); 7,39 (s, 1 H); 7,76 (d, 1 H); 8,00 (dd, 1 H); 8,32 (d, 1 H) ppm. | MW: 317,39 MS (ESI) [M+1]⁺: 318 | **INT17** und Piperidin |
| | 1-(2-Methoxyethyl)-6-nitro-3-(1-piperidinylmethyl)-1*H*-indol | | | |
| **INT120L** | 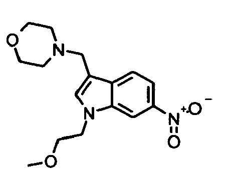 | (300 MHz, DMSO-*d*₆): δ = 2,47 (m, 4H); 3,32 (s, 3H); 3,69 (s, 2H); 3,70 (m, 4H); 3,72 (t, 2H); 4,33 (t, 2H); 7,38 (s, 1 H); 7,79 (d, 1 H); 8,01 (dd, 1H); 8,32 (d, 1H) ppm. | MW: 319,36 MS (ESI) [M+1]⁺: 320 | **INT17** und Morpholin |
| | 1-(2-Methoxyethyl)-3-(4-morpholinylmethyl)-6-nitro-1*H*-indol | | | |
| **INT120M** | 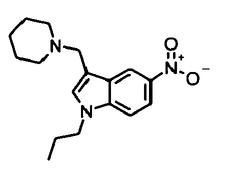 | (300 MHz, CDCl₃): δ = 0,93 (t, 3H); 1,43 (m, 2H); 1,57 (m, 4H); 1,87 (m, 2H); 2,42 (m, 4H); 3,67 (s, 2H); 4,08 (t, 2H); 7,18 (s, 1 H); 7,30 (d, 1H); 8,09 (dd, 1 H); 8,69 (d, 1 H) ppm. | MW: 301,39 MS (ESI) [M+1]⁺: 302 | **INT121D** und Piperidin |
| | 5-Nitro-3-(1-piperidinylmethyl)-1-propyl-1*H*-indol | | | |
| **INT120N** | 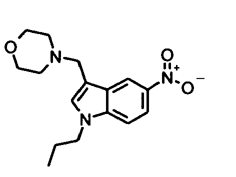 | (300 MHz, CDCl₃): δ = 0,93 (t, 3H); 1,88 (qt, 2H); 2,48 (m, 4H); 3,70 (s, 2H); 3,71 (m, 4H); 4,09 (t, 2H); 7,17 (s, 1H); 7,32 (d, 1H); 8,10 (dd, 1H); 8,72 (d, 1 H) ppm. | MW: 303,36 MS (ESI) [M+1]⁺: 304 | **INT121D** und Morpholin |
| | 3-(4-Morpholinylmethyl)-5-nitro-1-propyl-1*H*-indol | | | |

Die nachfolgenden Verbindungen werden analog nach dem für **INT33** beschriebenen Verfahren hergestellt.

| Beispiel | Struktur Name | ¹H-NMR | Molmasse MS | Edukt |
|---|---|---|---|---|
| **INT121A** | 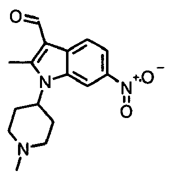 | (300 MHz, CDCl₃): δ = 1,89 (dbr, 2H); 2,20 (ddd, 2H); 2,39 (s, 3H); 2,66 (ddddbr, 2H); 2,82 (s, 3H); 3,11 (dbr, 2H); 4,32 (tt, 1H); 8,15 (dd, 1H); 8, 37 (d, 1H); 8, 54 (d, 1H); 10,23 (s, 1H) ppm. | MW: 301,35 MS (ESI) [M+1]⁺: 302 | **INT116** |
| | 2-Methyl-1-(1-methyl-4-piperidinyl)-6-nitro-1*H*-indol-3-carbaldehyd | | | |
| **INT121B** | 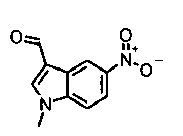 | (300 MHz, DMSO-*d*₆): δ = 3,97 (s, 3H); 7,81 (d, 1H); 8,19 (dd, 1H); 8,54 (s, 1 H); 8,92 (d, 1H); 9,92 (s, 1H) ppm. | MW: 204,19 MS (ESI) [M+1]⁺: 205 | **INT1** |
| | 1-Methyl-5-nitro-1*H*-indol-3-carbaldehyd | | | |
| **INT121C** | 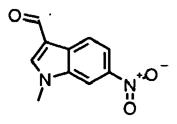 | (300 MHz, DMSO-*d*₆): δ = 4,03 (s, 3H); 8,14 (dd, 1H); 8,27 (d, 1H); 8,60 (d, 1H); 8,62 (s, 1H); 9,98 (s, 1H) ppm. | MW: 204,19 MS (ESI) [M+1]⁺: 205 | **INT4** |
| | 1-Methyl-6-nitro-1*H*-indol-3-carbaldehyd | | | |
| **INT121D** | 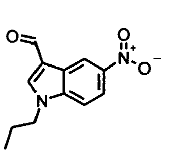 | (300 MHz, DMSO-*d*₆): δ = 0,87 (t, 3H); 1,85 (qt, 2H); 4,34 (t, 2H); 7,90 (d, 1H); 8,18 (dd, 1H); 8,62 (s, 1H); 8,94 (d, 1H); 10,00 (s, 1H) ppm. | MW: 232,24 MS (ESI) [M+1]⁺: 233 | **INT124** |
| | 5-Nitro-1-propyl-1*H*-indol-3-carbaldehyd | | | |

### Intermediat INT122

### 3-Ethenyl-6-nitro-1-(1-methyl-4-piperidinyl)-1H-indol

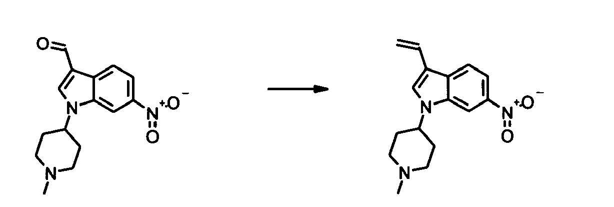

Zu einer Lösung von 260 mg der als **INT33** beschriebenen Verbindung und 698 mg Methyltriphenylphosphoniumbromid in 9 ml Tetrahydrofuran werden 219 mg Kalium*tertiär*-butylat gegeben. Die Mischung wird über Nacht bei Raumtemperatur gerührt. Das Reaktionsgemisch wird mit Wasser und mit gesättigter wässriger Natriumchloridlösung verdünnt, und mit Ethylacetat extrahiert. Die organische Phase wird mit gesättigter wässriger Natriumchloridlösung gewaschen und am Vakuum eingeengt. Flashchromatographie des Rückstands ergibt 148 mg der Titelverbindung als gelben kristallinen Feststoff.
¹H-NMR (300 MHz, CDCl₃): δ = 2,11 (m, 4H); 2,24 (m, 2H); 2,39 (s, 3H); 3,06 (dbr, 2H); 4,30 (m, 1 H); 5,25 (dd, 1 H); 5,69 (dd, 1 H); 6,85 (dd, 1 H); 7,56 (s, 1 H); 7,87 (d, 1 H); 8,05 (dd, 1H); 8,34 (d, 1H) ppm.

Die nachfolgende Verbindung wird analog nach dem für **INT122** beschriebenen Verfahren hergestellt.

| Beispiel | Struktur Name | ¹H-NMR | Molmasse MS | Edukt |
|---|---|---|---|---|
| **INT122A** | 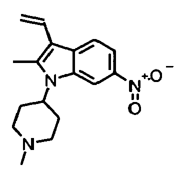 | (300 MHz, CDCl₃): δ = 1,85 (m, 2H); 2,18 (ddd, 2H); 2,37 (s, 3H); 2,54 (s, 3H); 2,61 (ddddbr, 2H); 3,08 (dbr, 2H); 4,26 (tt, 1H); 5,34 (dd, 1H); 5,64 (dd, 1H); 6,83 (dd, 1H); 7,82 (d, 1H); 8,02 (dd, 1 H); 8,49 (d, 1 H) ppm. | MW: 299,38 MS (ESI) [M+1]⁺: 300 | **INT121A** |
| | 3-Ethenyl-6-nitro-2-methyl-1-(1-methyl-4-piperidinyl)-1*H*-indol | | | |

### Intermediat INT123

### 3-Ethyl-1-(1-methyl-4-piperidinyl)-1H-indol-6-amin

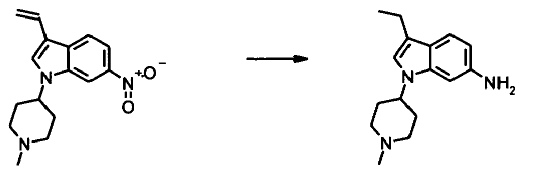

Eine Lösung von 60 mg der als **INT122** beschriebenen Verbindung in 4 ml Methanol wird mit 114 mg Palladium auf Aktivkohle (10% Pd) über Nacht in Wasserstoffatmosphäre gerührt. Dann wird der Wasserstoff durch Stickstoff verdrängt. Das Reaktionsgemisch wird über Celite^{®} filtriert, mit einem Gemisch aus Dichlormethan und Methanol (9:1) nachgewaschen und am Vakuum eingeengt. Flashchromatographie des Rückstands ergibt 40 mg der Titelverbindung als braunen öligen Feststoff.
¹H-NMR (300 MHz, CDCl₃): δ = 1,28 (t, 3H); 2,04 (m, 4H); 2,14 (m, 2H); 2,35 (s, 3H); 2,71 (qbr, 2H); 3,01 (dbr, 2H); 4,01 (m, 1 H); 6,54 (dd, 1 H); 6,63 (d, 1 H); 6,78 (s, 1 H); 7,36 (d, 1 H) ppm.
Die nachfolgende Verbindung wird analog nach dem für **INT123** beschriebenen Verfahren hergestellt.

| Beispiel | Struktur Name | ¹H-NMR | Molmasse MS | Edukt |
|---|---|---|---|---|
| **INT123A** | 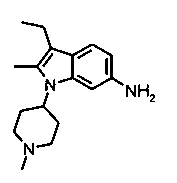 | (300 MHz, CD₃OD): δ = 1,13 (t, 3H); 1,72 (dbr, 2H); 2,22 (dddbr, 2H); 2,29 (s, 3H); 2,37 (s, 3H); 2,59 (m, 2H); 2,63 (q, 2H); 3,03 (dbr, 2H); 4,14 (m, 1H); 6,52 (dd, 1H); 6,97 (d, 1 H); 7,20 (d, 1 H) ppm. | MW: 271,41 MS (ESI) [M+1]⁺: 272 | **INT122A** |
| | 3-Ethenyl-6-nitro-2-methyl-1-(1-methyl-4-piperidinyl)-1*H*-indol | | | |

### Intermediat INT124

### 5-Nitro-1-propyl-1H-indol

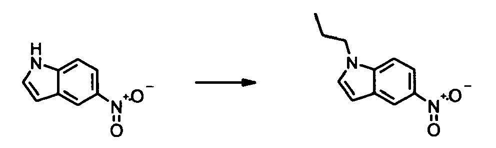

Zu einer Suspension von 1,35 g 60%igem Natriumhydrid in Paraffinöl in 50 ml N,N-Dimethylacetamid wird unter Eisbadkühlung eine Lösung von 5 g 5-Nitro-1*H*-indol (CAS 6146-52-7) in 100 ml *N,N*-Dimethylacetamid getropft. Die Mischung wird 15 Minuten nachgerührt. Dann werden 3,7 ml 1-Iodpropan hinzugetropft. Das Reaktionsgemisch wird vier Stunden bei Raumtemperatur gerührt, anschließend auf konzentrierte wässrige Natriumhydrogencarbonatlösung im Gemisch mit Eis gegossen. Die Mischung wird mit Ethylacetat extrahiert. Die organische Phase wird mit gesättigter wässriger Natriumchloridlösung gewaschen, über Natriumsulfat getrocknet und anschließend am Vakuum eingeengt. Flashchromatographie des Rückstands ergibt 6,01 g der Titelverbindung als gelbes Öl.
¹H-NMR (300 MHz, CDCl₃): δ = 0,94 (t, 3H); 1,09 (qt, 2H); 4,13 (t, 2H); 6,67 (d, 1H); 7,25 (d, 1H); 7,35 (d, 1H); 8,10 (dd, 1H); 8,58 (d, 1H) ppm.

Die nachfolgende Verbindung wird analog nach dem für **INT124** beschriebenen Verfahren hergestellt.

| Beispiel | Struktur Name | ¹H-NMR | Molmasse MS | Edukte |
|---|---|---|---|---|
| **INT124A** | 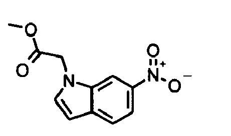 | (300 MHz, CDCl₃): δ = 3,80 (s, 3H); 4,96 (s, 2H); 6,68 (dbr, 1H); 7,38 (d, 1 H); 7,69 (d, 1 H); 8,04 (dd, 1 H); 8,24 (dbr, 1 H) ppm. | MW: 234,21 MS (ESI) [M+1]⁺: 235 | 6-Nitro-1*H*-indol; Methyl bromacetat |
| | Methyl 6-nitro-1*H*-indol-1-acetat | | | |

### Intermediat INT125

### N-Methyl-4-(6-nitro-1H-indol-1-yl)-1-piperidinacetamid

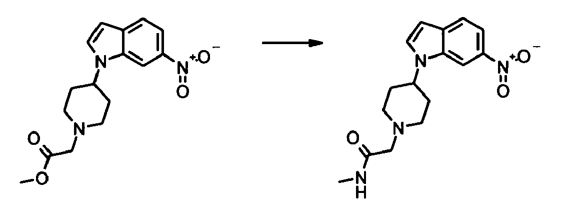

200 mg der unter **INT114E** hergestellten Verbindung werden mit 6,3 ml einer 2 M Lösung von Methylamin in Methanol über Nacht im Druckgefäß bei 80°C gerührt. Nach dem Abkühlen wird das Reaktionsgemisch am Vakuum eigeengt. Flashchromatographie des Rückstands ergibt 113 mg der Titelverbindung als orangefarbenen Feststoff.
¹H-NMR (300 MHz, CDCl₃): δ = 2,11 (m, 4H); 2,50 (dddbr, 2H); 2,89 (d, 3H); 3,06 (dbr, 2H); 3,12 (s, 2H); 4,36 (m, 1H); 6,64 (dbr, 1H); 7,51 (d, 1H); 7,66 (d, 1H); 8,01 (dd, 1 H); 8,36 (dbr, 1 H) ppm.
Die nachfolgenden Verbindungen werden analog nach dem für **INT125** beschriebenen Verfahren hergestellt.

| Beispiel | Struktur Name | ¹H-NMR | Molmasse MS | Edukte |
|---|---|---|---|---|
| **INT125A** | 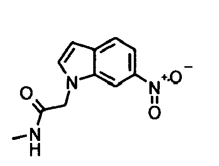 | (300 MHz, DMSO-*d*₆): δ = 2,63 (d, 3H); 5,01 (s, 2H); 6,67 (dbr, 1H); 7,74 (d, 1 H); 7,74 (d, 1H); 7,92 (dd, 1H); 8,20 (m, 1H); 8,43 (dbr, 1 H) ppm. | MW: 233,23 MS (ESI) [M+1]⁺: 234 | **INT124A;** 2 M NH₂Me in Methanol |
| | *N*-Methyl-6-nitro-1*H*-indol-1-acetamid | | | |
| **INT125B** | 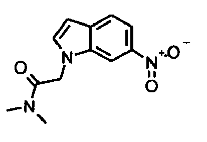 | (300 MHz, DMSO-*d*₆): δ = 2,87 (s, 3H); 3,13 (s, 3H); 5,37 (s, 2H); 6,66 (dbr, 1H); 7,66 (d, 1H); 7,73 (d, 1H); 7,91 (dd, 1H); 8,48 (dbr, 1H) ppm. | MW: 247,26 MS (ESI) [M+1]⁺: 248 | **INT124A;** 2 M NHMe₂ in Tetrahydrofuran |
| | *N,N*-Dimethyl-6-nitro-1*H*-indol-1-acetamid | | | |
| **INT125C** | 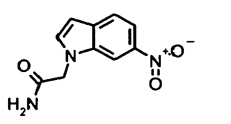 | (300 MHz, DMSO-*d*₆): δ = 5,00 (s, 2H); 6,66 (dbr, 1 H); 7,33 (sbr, 1 H); 7,72 (sbr, 1 H); 7,74 (d, 1 H); 7,75 (d, 1H); 7,92 (dd, 1 H); 8,41 (dbr, 1 H) ppm. | MW: 219, 20 MS (ESI) [M+1]⁺: 220 | **INT124A;** 3 M NH₃ in Methanol |
| | 6-Nitro-1*H*-indol-1- acetamid | | | |
| **INT125D** | 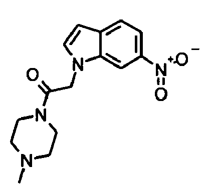 | (300 MHz, CDCl₃): δ = 2,35 (s, 3H); 2,44 (m, 2H); 2,50 (m, 2H); 3,59 (m, 2H); 3,67 (m, 2H); 5,01 (s, 2H); 6,67 (dbr, 1H); 7,38 (d, 1 H); 7,67 (d, 1 H); 8,02 (dd, 1 H); 8,22 (dbr, 1 H) ppm. | MW: 302,34 MS (ESI) [M+1]⁺: 303 | **INT124A;** 2 M 1-Methylpiperazin in Methanol |
| | 1-[2-(4-Methyl-1-piperazinyl)-2-oxoethyl]-6-nitro-1*H*-indol | | | |

### Intermediat INT126

### 1-[1-(1-Methylethyl)-4-piperidinyl]-1H-indol-6-amin

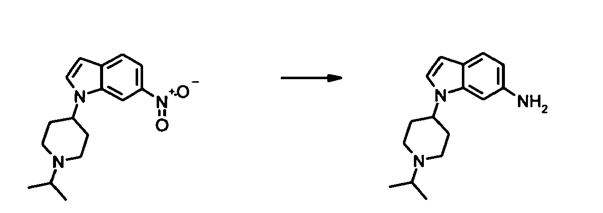

Eine Lösung von 533 mg der als **INT113** beschriebenen Verbindung in 19 ml Methanol und 19 ml Tetrahydrofuran wird mit einer Spatelspitze Raney-Nickel über Nacht in Wasserstoffatmosphäre gerührt. Dann wird der Wasserstoff durch Stickstoff verdrängt. Das Raney-Nickel wird mit einem Magneten aus der Reaktionslösung entfernt. Der Ansatz wird über Celite^{®} filtriert, mit einem Gemisch aus Dichlormethan und Methanol (9:1) nachgewaschen und am Vakuum eingeengt. Flashchromatographie des Rückstands ergibt 438 mg der Titelverbindung als hellbraunes Öl.
¹H-NMR (300 MHz, CDCl₃): δ = 1,10 (d, 6H); 2,01 (m, 2H); 2,08 (m, 2H); 2,35 (ddd, 2H); 2,82 (sept, 1H); 3,05 (dbr, 2H); 3,62 (m, 2H); 4,05 (m, 1H); 6,38 (dbr, 1H); 6,56 (dd, 1 H); 6,68 (dbr, 1 H); 7,05 (d, 1 H); 7,39 (d, 1 H) ppm.

Die nachfolgenden Verbindungen werden analog nach dem für **INT126** beschriebenen Verfahren hergestellt.

| Beispiel | Struktur Name | ¹H-NMR | Molmasse MS | Edukte |
|---|---|---|---|---|
| **INT126A** | 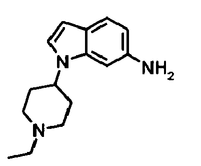 | (300MHz, CDCl₃): δ=1,14 (t, 3H); 2,01...2,20 (m, 6H); 2,49 (q, 2H); 3,13 (m, 2H); 3,62 (m, 2H); 4,08 (m, 1H); 6,38 (dbr, 1H); 6,56 (dd, 1H); 6,67 (dbr, 1H); 7,04 (d, 1 H); 7,39 (d, 1 H) ppm. | MW: 243,35 MS (ESI) [M+1]⁺: 244 | **INT113A** |
| | 1-(1-Ethyl-4-piperidinyl)-1*H*-indol-6-amin | | | |
| **INT126B** | 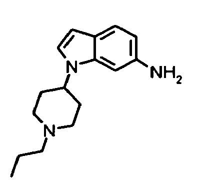 | (300 MHz, CDCl₃): δ = 0,94 (t, 3H); 1,56 (m, 2H); 2,00...2,21 (m, 6H); 2,37 (m, 2H); 3,10 (dbr, 2H); 3,63 (m, 2H); 4,07 (m, 1 H); 6,38 (dbr, 1H); 6,56 (dd, 1 H); 6,67 (dbr, 1 H); 7,04 (d, 1 H); 7,39 (d, 1 H) ppm. | MW: 257,38 MS (ESI) [M+1]⁺: 258 | **INT113B** |
| | 1-(1-Propyl-4-piperidinyl)-1*H*-indol-6-amin | | | |
| **INT126C** | 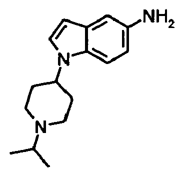 | (300 MHz, CD₃OD): δ = 1,13 (d, 6H); 2,03 (m, 4H); 2,45 (m, 2H); 2,82 (sept, 1 H); 3,05 (dbr, 2H); 4,21 (m, 1H); 6,25 (d, 1H); 6,72 (dd, 1H); 6,94 (d, 1 H); 7,20 (d, 1 H); 7,25 (d, 1 H) ppm. | MW: 257,38 MS (ESI) [M+1]⁺: 258 | **INT113C** |
| | 1-[1-(1-Methylethyl)-4-piperidinyl]-1*H*-indol-5-amin | | | |
| **INT126D** | 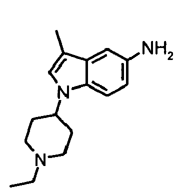 | (300 MHz, CD₃OD): δ = 1,11 (t, 3H); 1,90 (m, 2H); 1,96 (m, 2H); 2,10 (dddbr, 2H); 2,21 (sbr, 3H); 2,45 (q, 2H); 3,02 (dbr, 2H); 4,09 (m, 1 H); 6,69 (dd, 1 H); 6,88 (d, 1H); 6,94 (sbr, 1H); 7,16 (d, 1H) ppm. | MW: 257,38 MS (ESI) [M+1]⁺: 258 | **INT113D** |
| | 1-(1-Ethyl-4-piperidinyl)-3-methyl-1*H*-indol-5-amin | | | |
| **INT126E** | 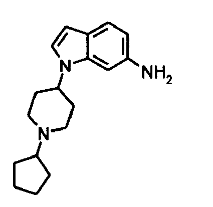 | (300 MHz, DMSO-*d*₆): δ = 1,37...1,72 (m, 6H); 1,77...2,06 (m, 6H); 3,14 (m, 2H); 3,34 (m, 2H); 3,44 (m, 1H); 4,09 (m, 1H); 6,20 (d, 1 H); 6,40 (dd, 1 H); 6,59 (sbr, 1H); 7,08 (d, 1 H); 7,17 (d, 1 H) ppm. | MW: 283,42 MS (ESI) [M+1]⁺: 284 | **INT113E** |
| | 1-(1-Cyclopentyl-4-piperidinyl)-1*H*-indol- 6-amin | | | |
| **INT126F** | 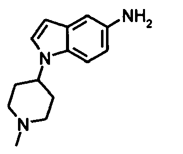 | (300 MHz, CD₃OD): δ = 2,03 (m, 2H); 2,06 (m, 2H); 2,30 (m, 2H); 2,36 (s, 3H); 3,02 (dbr, 2H); 4,25 (m, 1H); 6,26 (d, 1H); 6,72 (dd, 1H); 6,95 (d, 1H); 7,20 (d, 1H); 7,24 (d, 1H) ppm. | MW: 229,33 MS (ESI) [M+1]⁺: 230 | **INT114** |
| | 1-(1-Methyl-4-piperidinyl)-1*H*-indol-5-amin | | | |
| **INT126G** | 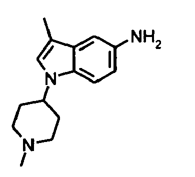 | (300 MHz, CD₃OD): δ = 1,89 (dbr, 2H); 1,96 (dddd, 2H); 2,17 (ddd, 2H); 2,21 (sbr, 3H); 2,30 (s, 3H); 2,92 (dbr, 2H); 4,08 (tt, 1H); 6,70 (dd, 1H); 6,88 (d, 1H); 6,94 (sbr, 1H); 7,16 (d, 1H) ppm. | MW: 243,35 MS (ESI) [M+1]⁺: 244 | **INT114A** |
| | 3-Methyl-1-(1-methyl-4-piperidinyl)-1*H*-indol-5-amin | | | |
| **INT126H** | 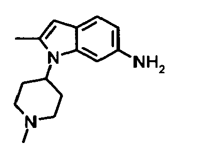 | (300 MHz, CD₃OD): δ = 1,72 (dbr, 2H); 2,22 (ddd, 2H); 2,36 (sbr, 3H); 2,37 (s, 3H); 2,59 (dddd, 2H); 3,03 (dbr, 2H); 4,14 (tt, 1H); 6,00 (sbr, 1 H); 6,51 (dd, 1 H); 6,98 (sbr, 1 H); 7,16 (d, 1H) ppm. | MW: 243,35 MS (ESI) [M+1]⁺: 244 | **INT116** |
| | 2-Methyl-1-(1-methyl-4-piperidinyl)-1*H*-indol-6-amin | | | |
| **INT126I** | 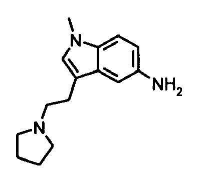 | (300 MHz, CD₃OD): δ = 1,81 (m, 4H); 2,59 (m, 4H); 2,71 (m, 2H); 2,86 (m, 2H); 3,61 (s, 3H); 6,71 (dd, 1H); 6,82 (s, 1 H); 6,94 (dbr, 1 H); 7,07 (d, 1H) ppm. | MW: 243,35 MS (ESI) [M+1]⁺: 244 | **INT119** |
| | 1-Methyl-3-[2-(1-pyrrolidinyl)ethyl]-1*H*-indol-5-amin | | | |
| **INT126J** | 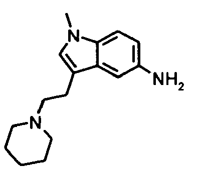 | (300 MHz, CD₃OD): δ = 1,48 (m, 2H); 1,64 (m, 4H); 2,51 (m, 4H); 2,58 (m, 2H); 2,85 (m, 2H); 3,62 (s, 3H); 6,71 (dd, 1 H); 6,82 (s, 1 H); 6,94 (dbr, 1 H); 7,08 (d, 1 H) ppm. | MW: 257,38 MS (ESI) [M+1]⁺: 258 | **INT119A** |
| | 1-Methyl-3-[2-(1-piperidinyl)ethyl]-1*H*-indol-5-amin | | | |
| **INT126K** | 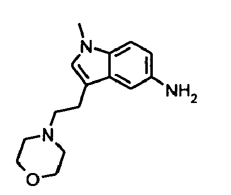 | (300 MHz, CD₃OD): δ = 2,46 (m, 4H); 2,56 (m, 2H); 2,81 (m, 2H); 3,68 (m, 4H); 3,57 (s, 3H); 6,70 (dd, 1H); 6,80 (s, 1H); 6,92 (dbr, 1H); 7,05 (d, 1H) ppm. | MW: 259,35 MS (ESI) [M+1]⁺: 260 | **INT119B** |
| | 1-Methyl-3-[2-(4-morpholinyl)ethyl]-1*H*-indol-5-amin | | | |
| **INT126L** | 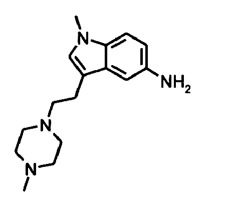 | (300 MHz, CD₃OD): δ = 2,28 (s, 3H); 2,54 (m, 8H); 2,62 (m, 2H); 2,84 (m, 2H); 3,62 (s, 3H); 6,71 (dd, 1 H); 6,83 (s, 1H); 6,93 (dbr, 1H); 7,08 (d, 1 H) ppm. | MW: 272,40 MS (ESI) [M+1]⁺: 273 | **INT119C** |
| | 1-Methyl-3-[2-(4-methyl-1-piperazinyl)ethyl]-1*H*-indol-5-amin | | | |
| **INT126M** | 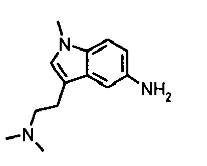 | (300 MHz, CD₃OD): δ = 2,33 (s, 6H); 2,61 (m, 2H); 2,85 (m, 2H); 3,66 (s, 3H); 6,73 (dd, 1 H); 6,86 (s, 1 H); 6,94 (dbr, 1 H); 7,10 (d, 1 H) ppm. | MW: 217,32 MS (ESI) [M+1]⁺: 218 | **INT119D** |
| | 5-Amino-*N,N*,1-trimethyl-1*H*-indol-3-ethanamin | | | |
| **INT126N** | 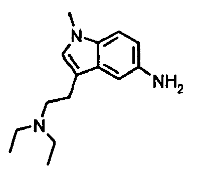 | (300 MHz, CD₃OD): δ = 1,10 (t, 6H); 2,67 (q, 4H); 2,74 (m, 2H); 2,81 (m, 2H); 3,65 (s, 3H); 6,72 (dd, 1H); 6,85 (s, 1H); 6,94 (dbr, 1H); 7,10 (d, 1H) ppm. | MW: 245,37 MS (ESI) [M+1]⁺: 246 | **INT119E** |
| | 5-Amino-*N,N*-diethyl-1-methyl-1*H*-indol-3-ethanamin | | | |
| **INT1260** | 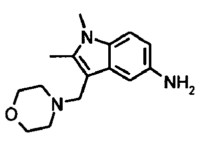 | (300 MHz, CD₃OD): δ = 2,36 (s, 3H); 2,51 (m, 4H); 3,61 (s, 2H); 3,61 (s, 3H); 3,65 (m, 4H); 6,67 (dd, 1H); 6,99 (dbr, 1 H); 7,08 (d, 1H) ppm. | MW: 259,35 MS (ESI) [M+1]⁺: 260 | **INT120** |
| | 1,2-Dimethyl-3-(4-morpholinylmethyl)-1*H*-indol-5-amin | | | |
| **INT126P** | 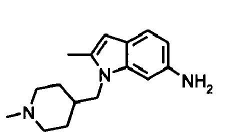 | (300 MHz, CD₃OD): δ = 1,37 (ddddbr, 2H); 1,55 (dbr, 2H); 1,83 (m, 1 H); 1,89 (dddbr, 2H); 2,23 (s, 3H); 2,35 (s, 3H); 2,84 (dbr, 2H); 3,87 (d, 2H); 6,03 (s, 1H); 6,52 (dd, 1 H); 6,70 (d, 1 H); 7,17 (d, 1 H) ppm. | MW: 257,38 MS (ESI) [M+1]⁺: 258 | **INT116A** |
| | 2-Methyl-1-[(1-methyl-4-piperidinyl)methyl]-1*H*-indol-6-amin | | | |
| **INT126Q** | 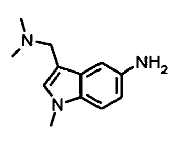 | (300 MHz, CD₃OD): δ = 2,25 (s, 6H); 3,58 (s, 2H); 3,71 (s, 3H); 6,74 (dd, 1H); 7,01 (d, 1H); 7,01 (s, 1H); 7,14 (d, 1H) ppm. | MW: 203,29 MS (ESI) [M+1]⁺: 204 | **INT120A** |
| | 5-Amino-*N,N*,1-trimethyl-1*H*-indol-3-methanamin | | | |
| **INT126R** | 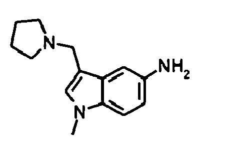 | (300 MHz, DMSO-*d*₆): δ = 1,65 (m, 4H); 2,40 (m, 4H); 3,56 (s, 2H); 3,62 (s, 3H); 6,52 (dd, 1H); 6,75 (d, 1 H); 6,97 (s, 1 H); 7,04 (d, 1 H) ppm. | MW: 229,33 MS (ESI) [M+1]⁺: 230 | **INT120B** |
| | 1-Methyl-3-(1-pyrrolidinylmethyl)-1*H*-indol-5-amin | | | |
| **INT126S** | 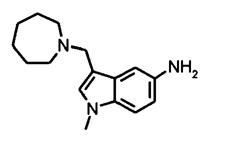 | (300 MHz, DMSO-*d*₆): δ = 1,55 (m, 8H); 2,55 (m, 4H); 3,57 (s, 2H); 3,62 (s, 3H); 6,52 (dd, 1H); 6,78 (d, 1H); 6,97 (s, 1H); 7,04 (d, 1H) ppm. | MW: 257,38 MS (ESI) [M+1]⁺: 258 | **INT120C** |
| | 3-[(Hexahydro-1*H*-azepin-1-yl)methyl]-1-methyl-1*H*-indol-5-amin | | | |
| **INT126T** | 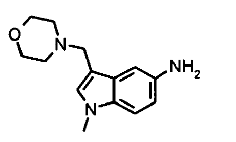 | (300 MHz, DMSO-*d*₆): δ = 2,34 (m, 4H); 3,45 (s, 2H); 3,54 (m, 4H); 3,63 (s, 3H); 6,52 (dd, 1 H); 6,76 (d, 1 H); 7,00 (s, 1 H); 7,05 (d, 1 H) ppm. | MW: 245,33 MS (ESI) [M+1]⁺: 246 | **INT120D** |
| | 1-Methyl-3-(4-morpholinylmethyl)-1*H*-indol-5-amin | | | |
| **INT126U** | 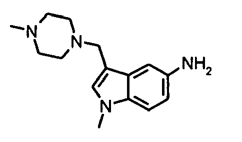 | (300 MHz, DMSO-*d*₆): δ = 2,13 (s, 3H); 3,34 (m, 8H); 3,44 (s, 2H); 3,62 (s, 3H); 6,52 (dd, 1H); 6,74 (d, 1 H); 6,98 (s, 1 H); 7,05 (d, 1 H) ppm. | MW: 258,37 MS (ESI) [M+1]⁺: 259 | **INT120E** |
| | 1-Methyl-3-[(4-methyl-1-piperazinyl)methyl]-1*H*-indol-5-amin | | | |
| **INT126V** | 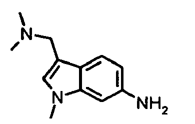 | (300 MHz, CDCl₃): δ = 2,26 (s, 6H); 3,56 (s, 2H); 3,64 (s, 3H); 6,57 (dd, 1H); 6,58 (sbr, 1H); 6,80 (s, 1H); 7,44 (d, 1H) ppm. | MW: 203,29 MS (ESI) [M+1]⁺: 204 | **INT120F** |
| | 6-Amino-*N,N*,1-trimethyl-1*H*-indol-3-methanamin | | | |
| **INT126X** | 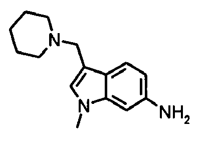 | (300 MHz, CDCl₃): δ = 1,40 (m, 2H); 1,57 (m, 4H); 2,45 (m, 4H); 3,63 (s, 2H); 3,64 (s, 3H); 6,57 (dd, 1H); 6,58 (sbr, 1H); 6,82 (s, 1H); 7,46 (d, 1 H) ppm. | MW: 243,35 MS (ESI) [M+1]⁺: 244 | **INT120G** |
| | 1-Methyl-3-(1-piperidinylmethyl)-1*H*-indol-6-amin | | | |
| **INT126Y** | 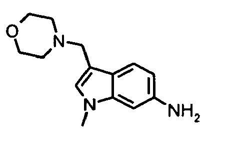 | (300 MHz, CDCl₃): δ = 2,53 (m, 4H); 3,67 (s, 2H); 3,69 (s, 3H); 3,74 (m, 4H); 6,62 (dd, 1H); 6,62 (d, 1H); 6,84 (s, 1H); 7,54 (d, 1H) ppm. | MW: 245,33 MS (ESI) [M+1]⁺: 246 | **INT120H** |
| | 1-Methyl-3-(4-morpholinylmethyl)-1*H*-indol-6-amin | | | |
| **INT126Z** | 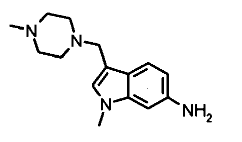 | (300 MHz, CDCl₃): δ = 2,26 (s, 3H); 2,44 (m, 4H); 2,52 (m, 4H); 3,63 (s, 2H); 3,63 (s, 3H); 6,56 (dd, 1H); 6,57 (d, 1H); 6,80 (s, 1H); 7,48 (d, 1H) ppm. | MW: 258,37 MS (ESI) [M+1]⁺: 259 | **INT120I** |
| | 1-Methyl-3-[(4-methyl-1-piperazinyl)methyl]-1*H*-indol-6-amin | | | |
| **INT126AA** | 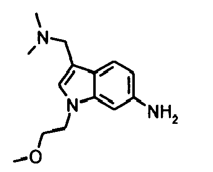 | (300 MHz, CDCl₃): δ = 2,27 (s, 6H); 3,31 (s, 3H); 3,56 (s, 2H); 3,67 (t, 2H); 4,14 (t, 2H); 6,57 (dd, 1 H); 6,61 (d, 1 H); 6,89 (s, 1 H); 7,44 (d, 1 H) ppm. | MW: 247,34 MS (ESI) [M+1]⁺: 248 | **INT120J** |
| | 6-Amino-1-(2-methoxyethyl)-*N,N*-dimethyl-1*H*-indol-3-methanamin | | | |
| **INT126AB** | 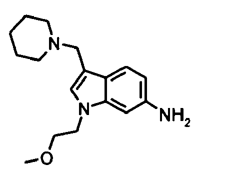 | (300 MHz, CDCl₃): δ = 1,39 (m, 2H); 1,56 (m, 4H); 2,44 (m, 4H); 3,31 (s, 3H); 3,61 (s, 2H); 3,67 (t, 2H); 4,13 (t, 2H); 6,56 (dd, 1H); 6,61 (d, 1H); 6,89 (s, 1H); 7,47 (d, 1H) ppm. | MW: 287,41 MS (ESI) [M+1]⁺: 288 | **INT120K** |
| | 1-(2-Methoxyethyl)-3-(1-piperidinylmethyl)-1*H*-indol-6-amin | | | |
| **INT126AC** | 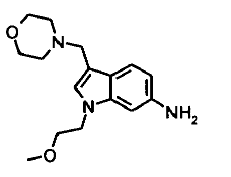 | (300 MHz, CDCl₃): δ = 2,48 (m, 4H); 3,31 (s, 3H); 3,62 (s, 2H); 3,67 (t, 2H); 3,70 (m, 4H); 4,13 (t, 2H); 6,57 (dd, 1 H); 6,61 (d, 1 H); 6,88 (s, 1 H); 7,49 (d, 1 H) ppm. | MW: 289,38 MS (ESI) [M+1]⁺: 290 | **INT120L** |
| | 1-(2-Methoxyethyl)-3-(4-morpholinylmethyl)-1*H*-indol-6-amin | | | |
| **INT126AD** | 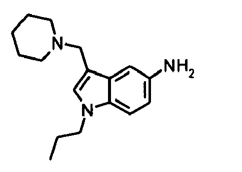 | (300 MHz, CD₃OD): δ = 0,87 (t, 3H); 1,42 (m, 2H); 1,80 (qt, 2H); 1,58 (m, 4H); 2,49 (m, 4H); 3,62 (s, 2H); 4,03 (t, 2H); 6,72 (dd, 1H); 7,01 (d, 1 H); 7,08 (s, 1 H); 7,15 (d, 1 H) ppm. | MW: 271,41 MS (ESI) [M+1]⁺: 272 | **INT120M** |
| | 3-(1-Piperidinylmethyl)-1-propyl-1*H*-indol-5-amin | | | |
| **INT126AE** | 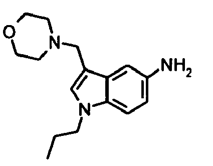 | (300 MHz, CD₃OD): δ = 0,86 (t, 3H); 1,79 (qt, 2H); 2,50 (m, 4H); 3,62 (s, 2H); 3,66 (m, 4H); 4,01 (t, 2H); 6,72 (dd, 1 H); 7,04 (d, 1 H); 7,07 (s, 1H); 7,15 (d, 1H) ppm. | MW: 273,38 MS (ESI) [M+1]⁺: 274 | **INT120N** |
| | 3-(4-Morpholinylmethyl)-1-propyl-1*H*-indol-5-amin | | | |
| **INT126AF** | 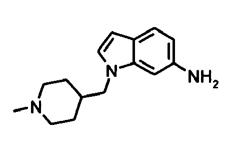 | (300 MHz, CD₃OD): δ = 1,30 (ddddbr, 2H); 1,51 (dbr, 2H); 1,78 (m, 1H); 1,84 (dddbr, 2H); 2,18 (s, 3H); 2,78 (dbr, 2H); 3,86 (d, 2H); 6,26 (dbr, 1H); 6,56 (dd, 1 H); 6,72 (dbr, 1 H); 6,89 (d, 1 H); 7,28 (d, 1 H) ppm. | MW: 243,35 MS (ESI) [M+1]⁺: 244 | **INT116B** |
| | 1-[(1-Methyl-4-piperidinyl)methyl]-1*H*-indol-6-amin | | | |
| **INT126AG** | 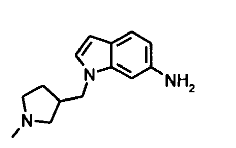 | (300 MHz, CD₃OD): δ = 1,55 (m, 1H); 1,90 (m, 1H); 2,29 (s, 3H); 2,33 (dd, 1H); 2,45 (dd, 1 H); 2,49 (m, 1 H); 2,61 (m, 1 H); 2,74 (m, 1 H); 3,95 (m, 2H); 6,27 (dbr, 1H); 6,57 (dd, 1H); 6,76 (dbr, 1 H); 6,95 (d, 1H); 7,29 (d, 1 H) ppm. | MW: 229,33 MS (ESI) [M+1]⁺: 230 | **INT116C** |
| | 1-[(1-Methyl-3-pyrrolidinyl)methyl]-1*H*-indol-6-amin | | | |
| **INT126AH** | 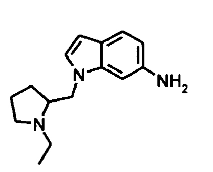 | (300 MHz, CD₃OD): δ = 1,09 (t, 3H); 1,58 (m, 1H); 1,74 (m, 3H); 2,21 (m, 2H); 2,72 (m, 1H); 2,84 (m, 1H); 3,18 (m, 1H); 3,88 (dd, 1H); 4,14 (dd, 1H); 6,27 (dbr, 1H); 6,57 (dd, 1H); 6,76 (dbr, 1 H); 6,98 (d, 1H); 7,29 (d, 1 H) ppm. | MW: 243,35 MS (ESI) [M+1]⁺: 244 | **INT116D** |
| | 1-[(1-Ethyl-2-pyrrolidinyl)methyl]-1*H*-indol-6-amin | | | |
| **INT126AI** | 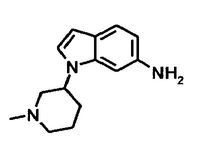 | (300 MHz, CD₃OD): δ = 1,63...1,88 (m, 3H); 1,99 (m, 2H); 2,10 (dd, 1H); 2,25 (s, 3H); 2,88 (dbr, 1 H); 2,97 (dbr, 1H); 4,28 (m, 1H); 6,30 (dbr, 1H); 6,56 (dd, 1 H); 6,76 (dbr, 1 H); 7,02 (d, 1H); 7,28 (d, 1H) ppm. | MW: 229,33 MS (ESI) [M+1]⁺: 230 | **INT116E** |
| | 1-(1-Methyl-3-piperidinyl)-1*H*-indol-6-amin | | | |
| **INT126AJ** | 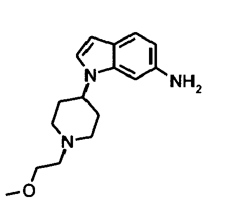 | (300 MHz, CDCl₃): δ = 1,92 (dbr, 2H); 2,01 (ddddbr, 2H); 2,22 (ddbr, 2H); 2,61 (t, 2H); 3,06 (dbr, 2H); 3,34 (s, 3H); 3,53 (t, 2H); 4,10 (ttbr, 1 H); 6,29 (dbr, 1H); 6,57 (dd, 1H); 6,78 (sbr, 1 H); 7,05 (d, 1 H); 7,29 (d, 1H) ppm. | MW: 273,38 MS (ESI) [M+1]⁺: 274 | **INT114B** |
| | 1-[1-(2-Methoxyethyl)-4-piperidinyl]-1*H*-indol-6-amin | | | |
| **INT126AK** | 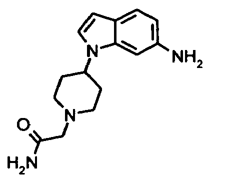 | (300 MHz, CD₃OD): δ = 1,98 (dbr, 2H); 2,10 (ddddbr, 2H); 2,39 (dddbr, 2H); 3,03 (dbr, 2H); 3,07 (s, 3H); 4,15 (ttbr, 1H); 6,30 (dbr, 1H); 6,58 (dd, 1H); 6,81 (dbr, 1 H); 7,10 (d, 1 H); 7,29 (d, 1 H) ppm. | MW: 272,35 MS (ESI) [M+1]⁺: 273 | **INT114C** |
| | 4-(6-Amino-1*H*-indol-1-yl)-1-piperidinacetamid | | | |
| **INT126AL** | 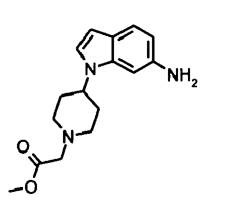 | (300 MHz, CDCl₃): δ = 2,05 (dbr, 2H); 2,15 (ddddbr, 2H); 2,46 (dddbr, 2H); 3,11 (dbr, 2H); 3,33 (s, 2H); 3,76 (s, 3H); 4,09 (tt, 1 H); 6,39 (dbr, 1H); 6,56 (dd, 1H); 6,66 (dbr, 1 H); 7,03 (d, 1 H); 7,39 (d, 1H) ppm. | MW: 287,36 MS (ESI) [M+1]⁺: 288 | **INT114D** |
| | Methyl 4-(6-amino-1*H*-indol-1-yl)-1-piperidinacetat | | | |
| **INT126AM** | 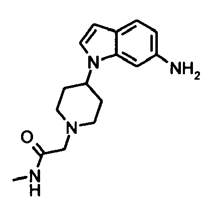 | (300 MHz, CD₃OD): δ = 1,93 (dbr, 2H); 2,08 (ddddbr, 2H); 2,35 (dddbr, 2H); 2,79 (s, 3H); 2,94 (dbr, 2H); 3,05 (s, 2H); 4,11 (tt, 1H); 6,31 (dbr, 1 H); 6,58 (dd, 1H); 6,79 (dbr, 1 H); 7,08 (d, 1 H); 7,29 (d, 1 H) ppm. | MW: 286,38 MS (ESI) [M+1]⁺: 287 | **INT125** |
| | 4-(6-Amino-1*H*-indol-1-yl)-*N*-methyl-1-piperidinacetamid | | | |
| **INT126AN** | 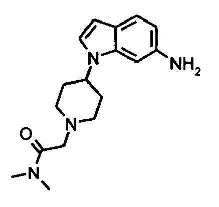 | (300 MHz, CD₃OD): δ = 1,99 (dbr, 2H); 2,09 (ddddbr, 2H); 2,36 (dddbr, 2H); 2,95 (s, 3H); 3,09 (dbr, 2H); 3,12 (s, 3H); 3,31 (s, 2H); 4,17 (ttbr, 1 H); 6,29 (dbr, 1 H); 6,57 (dd, 1H); 6,81 (dbr, 1 H); 7,09 (d, 1 H); 7,28 (d, 1 H) ppm. | MW: 300,41 MS (ESI) [M+1]⁺: 301 | **INT114E** |
| | 4-(6-Amino-1*H*-indol-1-yl)-*N,N*-dimethyl-1-piperidinacetamid | | | |
| **INT126AO** | 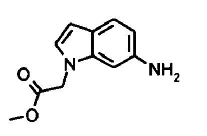 | (300 MHz, CDCl₃): δ = 3,74 (s, 3H); 4,74 (s, 2H); 6,43 (dbr, 1 H); 6,53 (dbr, 1H); 6,58 (dd, 1H); 6,89 (d, 1H); 7,40 (d, 1 H) ppm. | MW: 204,23 MS (ESI) [M+1]⁺ : 205 | **INT124A** |
| | Methyl 6-amino-1*H*-indol-1-acetat | | | |
| **INT126AP** | 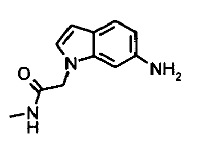 | (300 MHz, CDCl₃): δ = 2,71 (d, 3H); 4,67 (s, 2H); 5,41 (sbr, 1H); 6,48 (dbr, 1H); 6,52 (dbr, 1 H); 6,61 (dd, 1 H); 6,85 (d, 1 H); 7,42 (d, 1 H) ppm. | MW: 203,25 MS (ESI) [M+1]⁺: 204 | **INT125A** |
| | 6-Amino-*N*-methyl-1*H*-indol-1-acetamid | | | |
| **INT126AQ** | 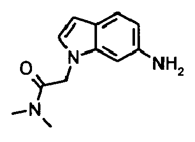 | (300 MHz, CDCl₃): δ = 2,97 (s, 3H); 2,98 (s, 3H); 4,78 (s, 2H); 6,43 (dbr, 1H); 6,56 (dd, 1H); 6,58 (dbr, 1H); 6,88 (d, 1 H); 7,39 (d, 1 H) ppm. | MW: 217,27 MS (ESI) [M+1]⁺: 218 | **INT125B** |
| | 6-Amino-*N,N*-dimethyl-1*H*-indol-1-acetamid | | | |
| **INT126AR** | 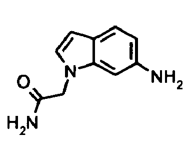 | (300 MHz, DMSO-*d*₆): δ = 4,57 (s, 2H); 4,77 (sbr, 2H); 6,18 (dbr, 1 H); 6,41 (m, 2H); 6,94 (d, 1H); 7,17 (d, 1 H); 7,18 (sbr, 1 H); 7,29 (sbr, 1 H) ppm. | MW: 189,22 MS (ESI) [M+1]⁺: 190 | **INT125C** |
| | 6-Amino-1*H*-indol-1-acetamid | | | |
| **INT126AS** | 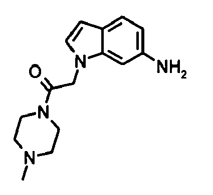 | (300 MHz, CDCl₃): δ = 2,22 (m, 2H); 2,25 (s, 3H); 2,36 (m, 2H); 3,41 (m, 2H); 3,65 (m, 2H); 4,78 (s, 2H); 6,43 (dbr, 1H); 6,56 (m, 1H); 6,57 (m, 1 H); 6,87 (d, 1H); 7,39 (d, 1H) ppm. | MW: 272,35 MS (ESI) [M+1]⁺: 273 | **INT125D** |
| | 1-[2-(4-Methyl-1-piperazinyl)-2-oxoethyl]-1*H*-indol-6-amin | | | |
| **INT126AT** | 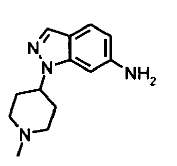 | (300 MHz, DMSO-*d*₆): δ = 1,78...1,86 (m, 2H); 2,02...2,12 (m, 4H); 2,22 (s, 3H); 2,87...2,9 (m, 2H); 4,14...4,24 (m, 1H); 5,27 (br, 2H); 6,49 (dd, 1H); 6,54 (d, 1H); 7,34 (d, 1H); 7,7 (s, 1 H) ppm. | MW: 230,32 MS (ESI) [M+1]⁺: 231 | **INT113F** |
| | 1-(1-Methyl-4-piperidinyl)-1*H*-indazol-6-amin | | | |
| **INT126AU** | 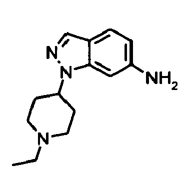 | (300 MHz, DMSO-*d*₆): δ = 1,03 (t, 3H); 1,82...1,88 (m, 2H); 2,01...2,10 (m, 4H); 2,38 (q, 2H); 2,98...3,01 (m, 2H); 4,16...4,26 (m, 1 H); 5,27 (br, 2H); 6,49 (dd, 2H); 6,54 (dd, 1 H); 7,34 (d, 1 H); 7,70 (s, 1 H) ppm. | MW: 244,34 MS (ESI) [M+1]⁺: 245 | **INT113G** |
| | 1-(1-Ethyl-4-piperidinyl)-1*H*-indazol-6-amin | | | |
| **INT126AV** | 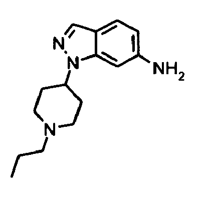 | (300 MHz, CDCl₃): δ = 0,93 (t, 3H); 1,55 (m, 2H); 1,99 (dbr, 2H); 2,14 (dddbr, 2H); 2,35 (ddddbr, 2H); 2,36 (m, 2H); 3,10 (dbr, 2H); 3,84 (sbr, 2H); 4,26 (tt, 1H); 6,56 (dd, 1 H); 6,64 (sbr, 1 H); 7,48 (d, 1 H); 7,80 (s, 1 H) ppm. | MW: 258,37 MS (ESI) [M+1]⁺: 259 | **INT113H** |
| | 1-(1-Propyl-4-piperidinyl)-1*H*-indazol-6-amin | | | |
| **INT126AW** | 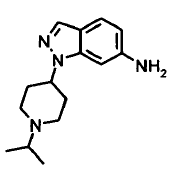 | (300 MHz, CDCl₃): δ = 1,09 (d, 6H); 2,03 (m, 2H); 2,31 (m, 2H); 2,36 (m, 2H); 2,82 (sept, 2H); 3,04 (dbr, 2H); 3,84 (sbr, 2H); 4,24 (m, 1H); 6,55 (dd, 1H); 6,65 (dbr, 1 H); 7,47 (d, 1 H); 7,80 (s, 1 H) ppm. | MW: 258,37 MS (ESI) [M+1]⁺: 259 | **INT113I** |
| | 1-[1-(1-Methylethyl)-4-piperidinyl]-1*H*-indazol-6-amin | | | |
| **INT126AX** | 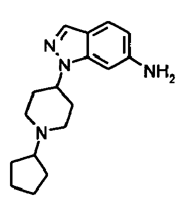 | (300 MHz, DMSO-*d*₆): δ = 1,30...1,40 (m, 2H); 1,47...1,55 (m, 2H); 1,58...1,65 (m, 2H); 1,77...1,86 (m, 4H); 2,01...2,13 (m, 4H); 2,49...2,57 (m, 1H); 3,02...3,07 (m, 2H); 4,17...4,25 (m, 1H); 5,27 (br, 2H); 6,48 (dd, 1 H); 6,54 (s, 1H); 7,34 (d,1H); 7,7 (s, 1 H) ppm. | MW: 284,41 MS (ESI) [M+1]⁺: 285 | **INT113J** |
| | 1-(1-Cyclopentyl-4-piperidinyl)-1*H*-indazol-6-amin | | | |
| **INT126AY** | 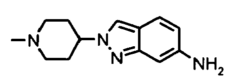 | (300 MHz, CDCl₃): δ = 2,05...2,31 (m, 6H); 2,34 (s, 3H); 3,01 (m, 2H); 3,74 (m, 2H); 4,33 (m, 1H); 6,58 (dd, 1 H); 6,80 (dbr, 1 H); 7,46 (d, 1 H); 7,80 (s, 1 H) ppm. | MW: 230,32 MS (ESI) [M+1]⁺: 231 | **INT113K** |
| | 2-(1-Methyl-4-piperidinyl)-2*H*-indazol-6-amin | | | |
| **INT126AZ** | 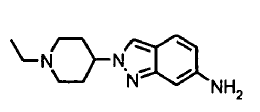 | (300 MHz, DMSO-*d*₆): δ = 1,02 (t, 3H); 1,98...2,06 (m, 6H); 2,37 (q, 2H); 2,96...2,99 (m, 2H); 4,19...4,29 (m, 1H); 4,99 (br, 2H); 6,46 (m, 1H); 6,50 (dd, 1 H); 7,33 (d, 1 H); 8,07 (s, 1H) ppm. | MW: 244,34 MS (ESI) [M+1]⁺: 245 | **INT113L** |
| | 2-(1-Ethyl-4-piperidinyl)-2*H*-indazol-6-amin | | | |
| **INT126BA** | 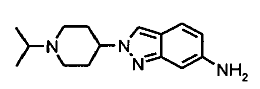 | (300 MHz, DMSO-d₆): δ = 0,99 (d, 6H); 1,89...2,07 (m, 4H); 2,27 (td, 2H); 2,70...2,79 (m, 1 H); 2,87...2,91 (m, 2H); 4,16...4,26 (m, 1 H); 4,99 (br, 2H); 6,46 (m, 1H); 6,49 (dd, 1 H); 7,32 (d, 1 H); 8,06 (s, 1H) ppm. | MW: 258,37 MS (ESI) [M+1]⁺: 259 | **INT113M** |
| | 2-[1-(1-Methylethyl)-4-piperidinyl]-2*H*-indazol-6-amin | | | |
| **INT126BB** | 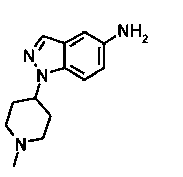 | (300 MHz, DMSO-d₆): δ = 1,80...1,88 (m, 2H); 2,01...2,14 (m, 4H); 2,22 (s, 3H); 2,86...2,89 (m , 2H); 4,33...4,43 (m, 1 H); 4,78 (br, 2H); 6,73 (d, 1 H); 6,78 (dd, 1 H); 7,38 d, 1H); 7,69 (s, 1 H) ppm. | MW: 230,32 MS (ESI) [M+1]⁺: 231 | **INT113N** |
| | 1-(1-Methyl-4-piperidinyl)-1*H*-indazol-5-amin | | | |
| **INT126BC** | 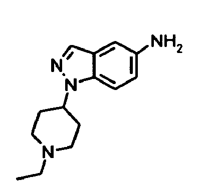 | (300 MHz, DMSO-*d*₆): δ = 1,02 (t, 3H); 1,83...1,86 (m, 2H); 2,0...2,11 (m, 4H); 2,37 (q, 2H); 2,94...3,01 (m, 2H); 4,36...4,45 (m, 1H); 4,78 (br, 2H); 6,72 (d, 1H); 6,78 (dd, 1H); 7,38 (d, 1H); 7,69 (s, 1H) ppm. | MW: 244,34 MS (ESI) [M+1]⁺: 245 | **INT113O** |
| | 1-(1-Ethyl-4-piperidinyl)-1*H*-indazol-5-amin | | | |
| **INT126BD** | 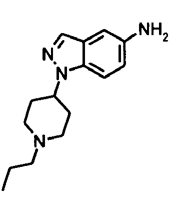 | (300 MHz, DMSO-*d*₆): δ = 0,87 (t, 3H); 1,40...1,52 (m, 2H); 1,81...1,90 (m, 2H); 1,99...2,13 (m, 4H); 2,28 (dd, 2H); 2,91...3,0 (m, 2H); 4,35...4,45 (m, 1H); 4,78, (br, 2H); 6,73 (d, 1H); 6,78 (dd, 1H); 7,38 (d, 1H); 7,69 (s, 1 H) ppm. | MW: 258,37 MS (ESI) [M+]⁺: 259 | **INT113P** |
| | 1-(1-Propyl-4-piperidinyl)-1*H*-indazol-5-amin | | | |
| **INT126BE** | 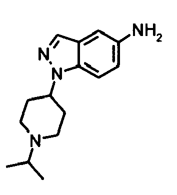 | (300 MHz, DMSO-*d*₆): δ = 1,00 (d, 6H); 1,84...1,88 (m, 2H); 1,95...2,08 (m, 2H); 2,28...2,37 (m, 2H); 2,71...2,8 (m, 1H); 2,87...2,91 (m, 2H); 4,32...4,42 (m, 1H); 4,77 (br, 2H); 6,72 (d, 1 H); 6,78 (d, 1H); 7,38 (d, 1H) ppm. | MW: 258,37 MS (ESI) [M+1]⁺: 259 | **INT113Q** |
| | 1-[1-(1-Methylethyl)-4-piperidinyl]-1*H*-indazol-5-amin | | | |
| **INT126BF** | 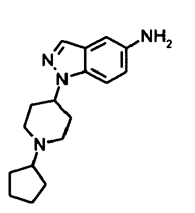 | (300 MHz, DMSO-*d*₆): δ = 1,29...1,41 (m, 2H); 1,47...1,67 (m, 4H); 1,76...1,87 (m, 4H); 1,98...2,16 (m, 4H); 2,5...2,56 (m, 1H); 3,03...3,06 (m, 2H); 4,35...4,45 (m, 1H); 4,77 (br, 2H); 6,72 (d, 1 H); 6,78 (dd, 1 H); 7,39 (d, 1 H); 7,69 (s, 1 H) ppm. | MW: 284,41 MS (ESI) [M+]⁺: 285 | **INT113R** |
| | 1-(1-Cyclopentyl-4-piperidinyl)-1*H*-indazol-5-amin | | | |
| **INT126BG** | 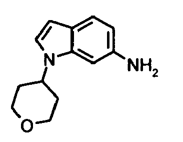 | (300 MHz, CD₃OD): δ = 1,91 (dbr, 2H); 2,01 (dddd, 2H); 3,60 (ddd, 2H); 4,05 (ddbr, 2H); 4,36 (m, 1H); 6,31 (d, 1H); 6,59 (dd, 1H); 6,82 (d, 1H); 7,07 (d, 1H); 7,29 (d, 1H) ppm. | MW: 216,29 MS (ESI) [M+1]⁺: 217 | **INT111F** |
| | 1-(Tetrahydro-2*H*-pyran-4-yl)-1*H*-indol-6-amin | | | |

### 2. Synthese der Template

### Intermediat INTT1

### 2-Propenyl 2-cyan-3-(ethylamino)-3-thioxopropanoat

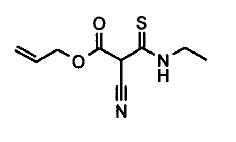

Zu einer Mischung aus 22,4 g Cyanessigsäureallylester und 30 ml Triethylamin werden bei Raumtemperatur 19 ml Ethylisothiocyanat zugegeben. Es wird 8 Stunden bei 60°C und 15 Stunden bei Raumtemperatur gerührt. Das Reaktionsgemisch auf eiskalte 1 normale Salzsäure gegossen und es wird 30 Minuten bei Raumtemperatur gerührt. Das Reaktionsgemisch wird mit Essigsäureethylester extrahiert und anschließend mit gesättigter Kochsalzlösung gewaschen und über Natriumsulfat getrocknet. Es werden 37,3 g der Titelverbindung als Rohprodukt erhalten, und ohne weitere Aufreinigung weiter umgesetz.
Molmasse = 200,261; MS (ESI): [M+1]⁺ = 201.

### Intermediat INTT2

### 2-Propenyl (Z)-cyan(3-ethyl-4-oxo-2-thiazolidinyliden)acetat

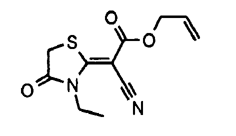

37,3 g der als **INTT1** beschriebenen Verbindung werden in 750 ml Tetrahydrofuran gelöst. Bei Raumtemperatur werden 17 ml Bromacetylchlorid zugetropft und es wird weitere 15 Stunden bei Raumtemperatur gerührt. Das Reaktionsgemisch wird auf gesättigte Natriumhydrogencarbonat-Lösung gegossen und mit Essigsäureethylester extrahiert. Die organische Phase wird mit Wasser und anschließend mit gesättigter Kochsalzlösung gewaschen und über Natriumsulfat getrocknet. Nach Reinigung durch zweimalige Kristallisation aus Ethanol werden 21,3 g der Titelverbindung erhalten.
¹H-NMR, 300 MHz, (DMSO-*d*₆): δ = 1,18 (t, 3H); 3,92 (s, 2H); 4,07 (q, 2H); 4,60-4,74 (m, 2H); 5,15-5,40 (m, 2H); 5,80-6,04 (m, 1H) ppm.

### Intermediat INTT3

### 2-Propenyl (Z,E/Z)-Cyan[5-(ethoxymethylen)-3-ethyl-4-oxo-2-thiazolidinyliden]acetat

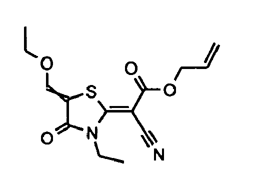

25 g der als **INTT2** beschriebenen Verbindung, 21,5 ml Acetanhydrid und 99 ml Triethylorthoformiat werden 15 Stunden bei 100°C gerührt. Die Reaktionsmischung wird auf Eiswasser gegossen und mit Dichlormethan extrahiert. Nach Reinigung durch zweimaliges Umkristallisieren aus Ethanol werden 22,3 g der Titelverbindung erhalten.
¹H-NMR, 400 MHz, (DMSO-*d*₆): δ = 1,19 (t, 3H); 1,26 (t, 3H); 4,16 (q, 2H); 4,34 (q, 2H); 4,67 (d, b, 2H); 5,15-5,44 (m, 2H); 5,79-6,03 (m, 1H); 8,12 (s, 1H) ppm.

### Intermediat INTT4

### (Z)-2-Cyan-2-(3-ethyl-4-oxo-2-thiazolidinyliden)-N-(2,2,2-trifluorethyl)acetamid

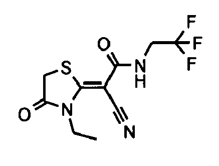

10,0 g der als **INTT3** beschriebenen Verbindung werden in 50 ml N,N-Dimethylformamid und 500 ml Tetrahydrofuran gelöst. Es werden 12,38 g N,N-Dimethylbarbitursäure und 916 mg Tetrakis(triphenylphosphin)palladium(0) zugegeben und es wird 3,5 Stunden bei Raumtemperatur gerührt. Anschließend werden 38,18 g TBTU, 16,65 g Natriumhydrogencarbonat und 9,35 ml (2,2,2-Trifluorethyl)amin zugegeben und es wird 15 Stunden bei Raumtemperatur gerührt. Die Reaktionsmischung wird mit 2 l eines Gemisches aus Hexan und Essigsäureethylester (1:3) versetzt, dreimal mit je 400 ml Wasser, einmal mit 400 ml gesättigter Natriumhydrogencarbonatlösung und einmal mit 400 ml gesättigter Kochsalzlösung gewaschen. Die Lösung wird über Natriumsulfat getrocknet, und das Lösungsmittel wird am Rotationsverdampfer abkondensiert. Nach Reinigung durch Kristallisation aus Ethanol werden 9,8 g der Titelverbindung erhalten.
¹H-NMR, 300 MHz, (DMSO-*d*₆): δ = 1,18 (t, 3H); 3,81 (s, 2H); 3,83-3,99 (m, 2H); 4,06 (q, 2H); 8,29 (t, b, 1 H) ppm.

Die nachfolgenden Verbindungen werden analog der oben beschriebenen Verfahren hergestellt.

| **Beispiel Nr.** | **Struktur und Name** | **¹H-NMR** | **Mol. Gewicht** | **Edukt / Synthese analo g** |
|---|---|---|---|---|
| **INTT5** | 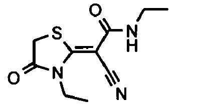 | (300 MHz, DMSO-*d*₆): δ = 1,00 (t, 3H); 1,17 (t, 3H); 3,13 (dq, 2H); 3,76 (s, 2H); 4,05 (q, 2H); 7,77 (t, 1 H) ppm. | 239,30 | INTT3 / INTT4 |
| | (*Z*)-2-Cyan-*N*-ethyl-2-(3-ethyl-4-oxo-2-thiazolidinyliden)acetamid | | | |
| **INTT6** | 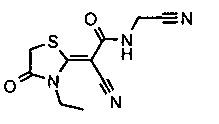 | (400 MHz, DMSO-*d*₆): δ = 1,18 (t, 3H); 3,83 (s, 2H); 4,06 (q, 2H); 4,11 (d, 2H); 8,43 (t, 1 H) ppm. | 250,28 | INTT3 / INTT4 |
| | (*Z*)-2-Cyan-*N*-(cyanmethyl)-2-(3-ethyl-4-oxo-2-thiazolidinyliden)acetamid | | | |
| **INTT7** | 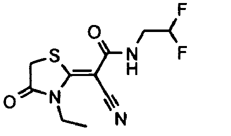 | (300 MHz, DMSO-*d*₆): δ = 1.18 (t, 3H); 3.52 (ddt, 2H); 3.80 (s, 2H); 4.06 (q, 2H); 6.01 (tt, 1 H); 8.03 (t, 1 H) ppm. | 275,28 | INTT3 / INTT4 |
| | (*Z*)-2-Cyan-*N*-(2,2-difluorethyl)-2-(3-ethyl-4-oxo-2-thiazolidinyliden)acetamid | | | |
| **INTT8** | 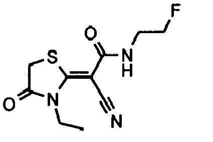 | (400 MHz, CDCl₃): δ = 1,36 (t, 3H); 3,58-3,76 (m, 4H); 4,28 (q, 2H); 4,55 (dt, 2H); 6,61 (t, b, 1 H) ppm. | 257,29 | INTT3 / INTT4 |
| | (*Z*)-2-Cyan-2-(3-ethyl-4-oxo-2-thiazolidinyliden)-*N*-(2-fluorethyl)acetamid | | | |
| **INTT9** | 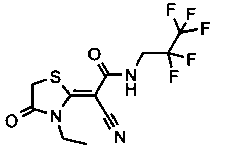 | (300 MHz, DMSO-*d*₆): δ = 1,23 (t, 3H); 3,86 (s, 2H); 4,00 (dt, 2H); 4,11 (q, 2H); 8,31 (t, 1H) ppm. | 343,28 | INTT3 / INTT4 |
| | (*Z*)-2-Cyan-2-(3-ethyl-4-oxo-2-thiazolidinyliden)-*N*-(2,2,3,3,3-pentafluorpropyl)acetamid | | | |
| **INTT10** | 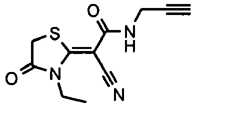 | (300 MHz, DMSO-*d*₆): δ = 1.21 (t, 3H); 3.07 (m, 1H); 3.83 (s, 2H); 3.90 (m, 2H); 4.10 (q, 2H); 8.22 (t, 1 H) ppm. | 249,29 | INTT3 / INTT4 |
| | (*Z*)-2-Cyan-2-(3-ethyl-4-oxo-2-thiazolidinyliden)-*N*-(2-propinyl)acetamid | | | |
| **INTT11** | 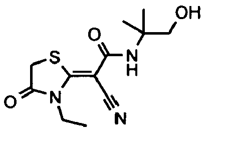 | (300 MHz, CDCl₃): δ = 1,34 (t, 3H); 1,37 (s, 6H); 3,60 (s, 2H); 3,65 (m, 2H); 4,26 (q, 2H); 6,32 (sbr, 1 H) ppm. | 283,35 | INTT3 / INTT4 |
| | (*Z*)-2-Cyan-2-(3-ethyl-4-oxo-2-thiazolidinyliden)-*N*-(2-hydroxy-1,1-dimethylethyl)acetamid | | | |
| **INTT12** | 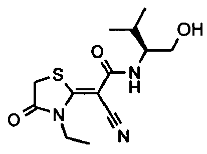 | (300 MHz, CD₃OD): δ = 0,94 (d, 3H); 0,99 (d, 3H); 1,32 (t, 3H); 1,94 (m, 1 H); 3,62 (dd, 1 H); 3,68 (dd, 1 H); 3,73 (m, 2H); 4,25 (q, 2H) ppm. | 297,38 | INTT3 / INTT4 |
| | [S-(Z)]-2-Cyan-2-(3-ethyl-4-oxo-2-thiazolidinyliden)-*N*-[-1-(hydroxymethyl)-2-methylpropyl]acetamid | | | |

### Intermediat INTT13

### (Z,E/Z)-2-Cyan-2-[5-(ethoxymethylen)-3-ethyl-4-oxo-2-thiazolidinyliden]-N-(2,2,2-trifluorethyl)acetamid

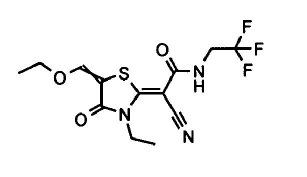

20,0 g der als **INTT4** beschriebenen Verbindung, 200 ml Triethylorthoformiat und 59 ml Acetanhydrid werden 20 Stunden bei 150°C Ölbadtemperatur gerührt. Die erkaltete Reaktionsmischung wird mit Ethanol versetzt, und der Feststoff wird abfiltriert. Nach Reinigung durch Kristallisation aus Ethanol werden 19,5 g der Titelverbindung erhalten.
¹H-NMR, 400 MHz, (DMSO-*d*₆): δ =1,19 (t, 3H); 1,25 (t, 3H); 3,90 (q, 2H); 4,15 (q, 2H); 4,30 (q, 2H); 8,01 (s, 1H); 8,31 (s, b, 1H) ppm.

Die nachfolgenden Verbindungen werden analog der oben beschriebenen Verfahren hergestellt.

| Beispiel Nr. | Struktur und Name | ¹H-NMR | Mol. Gewicht | Edukt / Synthese analog |
|---|---|---|---|---|
| **INTT14** | 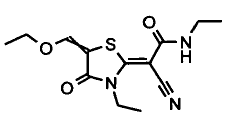 | (300 MHz, DMSO-*d*₆): δ = 1,01 (t, 3H); 1,18 (t, 3H); 1,25 (t, 3H); 3,14 (dq, 2H); 4,13 (q, 2H); 4,28 (q, 2H); 7,78 (t, 1 H); 7,93 (s, 1H) ppm. | 295,36 | INTT5 / INTT13 |
| | (*Z*,*E*/*Z*)-2-Cyan-2-[5-(ethoxymethylen)-3-ethyl-4-oxo-2-thiazolidinyliden]-*N*-ethylacetamid | | | |
| **INTT15** | 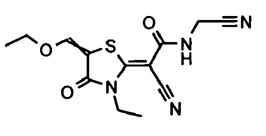 | (300 MHz, DMSO-*d*₆): δ = 1,19 (t, 3H); 1,26 (t, 3H); 4,05-4,22 (m, 4H); 4,31 (q, 2H); 8,02 (s, 1 H); 8,43 (t, 1H) ppm. | 306,35 | INTT6 / INTT13 |
| | (*Z*,*E*/*Z*)-2-Cyan-*N*-(cyanmethyl)-2-[5-(ethoxymethylen)-3-ethyl-4-oxo-2-thiazolidinyliden]acetamid | | | |
| **INTT16** | 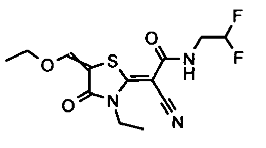 | (300 MHz, DMSO-*d*₆): δ = 1.19 (t, 3H); 1.25 (t, 3H); 3.52 (ddt, 2H); 4.14 (q, 2H); 4.30 (q, 2H); 6.01 (tt, 1H); 7.99 (s, 1H); 8.05 (t, 1H) ppm. | 331,34 | INTT7 / INTT13 |
| | (*Z*,*E*/*Z*)-2-Cyan-*N*-(2,2-difluorethyl)-2-[5-(ethoxymethylen)-3-ethyl-4-oxo-2-thiazolidinyliden]acetamid | | | |
| **INTT17** | 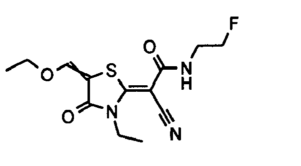 | (300 MHz, DMSO-*d*₆): δ = 1,22 (t, 3H); 1,28 (t, 3H); 3,44 (q, 1H); 3,49 (q, 1H); 4,18 (q, 2H); 4,33 (q, 2H); 4,41 (t, 1H); 4,53 (t, 1 H); 7,94 (t, 1 H); 8,0 (s, 1 H) ppm. | 313,35 | INTT8 / INTT13 |
| | (*Z*,*E*/*Z*)-2-Cyan-2-[5-(ethoxymethylen)-3-ethyl-4-oxo-2-thiazolidinyliden]-*N*-(2-fluorethyl)acetamid | | | |
| **INTT18** | 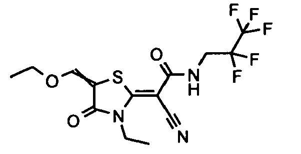 | (300 MHz, DMSO-*d*₆): δ = 1,19 (t, 3H); 1,25 (t, 3H); 3,95 (dt, 2H); 4,14 (q, 2H); 4,30 (q, 2H); 8,01 (s, 1 H); 8,28 (t, 1 H) ppm. | 399, 34 | INTT9 / INTT13 |
| | (*Z*,*E*/*Z*)-2-Cyan-2-[5-(ethoxymethylen)-3-ethyl-4-oxo-2-thiazolidinyliden]-*N*-(2,2,3,3,3-pentafluorpropyl)acetamid | | | |

### Intermediat INTT19

### 2-Propenyl (Z,E/Z)-cyan[3-ethyl-5-[[[1-(1-methyl-4-piperidinyl)-1H-indol-6-yl]amino]methylen]-4-oxo-2-thiazolidinyliden]acetat

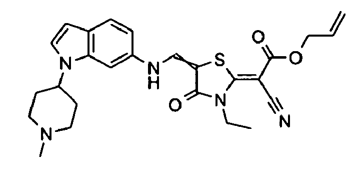

720 mg der als **INTT3** beschriebenen Verbindung und 800 mg der als **INT30** beschriebenen Verbindung werden in 40 ml Ethanol gelöst und fünf Stunden unter Rückfluss gerührt. Nach Reinigung durch Chromatographie an Kieselgel, anschließend an Aminophasenkieselgel und anschließender Kristallisation aus Ethanol werden 1,10 g der Titelverbindung erhalten.
¹H-NMR, 300 MHz, (DMSO-*d*₆): δ = 1,26 (t, 3H); 1,82-2,09 (m, 4H); 2,16 (t, b, 2H); 2,25 (s, 3H); 2,91 (d, b, 2H); 4,27 (q, 2H); 4,34-4,48 (m, 1H); 4,72 (d, 2H); 5,27 (dd, 1H); 5,39 (dd, 1H); 5,88-6,10 (m, 1H); 6,42 (d, 1H); 7,03 (dd, 1H); 7,46 (d, 1H); 7,51 (d, 1H); 7,56 (s, 1H); 8,36 (s, 1H); 10,61 (s, b, 1H) ppm.

### 1. Synthese der Endverbindungen

### Verfahren V1

### Beispiel B118 (Z,E/Z)-2-Cyan-2-[3-ethyl-5-[[[1-methyl-3-(1-methyl-4-piperidinyl)-1H-indol-5-yl]amino]methylen]-4-oxo-2-thiazolidinyliden]-N-(2,2,2-trifluorethyl)acetamid

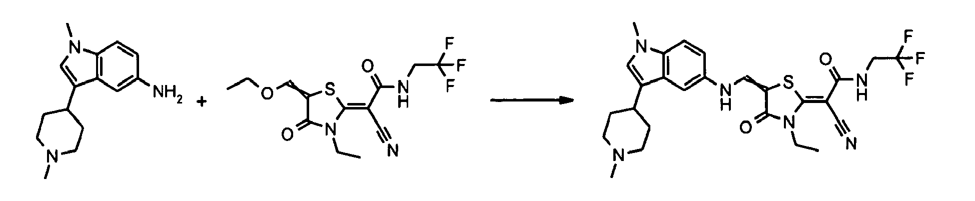

120 mg der als **INTT13** beschriebenen Verbindung werden in 10 ml Ethanol gelöst. Es werden 125 mg der als **INT3** beschriebenen Verbindung zugegeben. Es wird 12 Stunden unter Rückfluss gerührt. Die Reaktionsmischung wird eingeengt. Nach Reinigung durch Chromatographie an Aminophasenkieselgel, anschließend an Kieselgel und anschließender Kristallisation aus Ethanol werden 121 mg der Titelverbindung erhalten. Die spektroskopischen Daten sind in der nachfolgenden Tabelle aufgeführt.

### Verfahren V2

### Beispiel B174 (Z,E/Z)-2-Cyan-N-(2,2-difluorethyl)-2-[3-ethyl-5-[[[1-(1-methyl-4-piperidinyl)-1H-indol-6-yl]amino]methylen]-4-oxo-2-thiazolidinyliden]acetamid

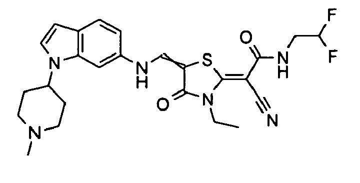

200 mg der als **INTT19** beschriebenen Verbindung werden in 0,41 ml *N,N-*Dimethylformamid und 4,1 ml Tetrahydrofuran gelöst. Es werden 130 mg *N,N-*Dimethylbarbitursäure und 10 mg Tetrakis(triphenylphosphin)palladium(0) zugegeben und es wird drei Stunden bei Raumtemperatur gerührt. Anschließend werden 260 mg TBTU, 170 mg Natriumhydrogencarbonat und 99 mg 2,2-Difluorethylamin zugegeben und es wird 15 Stunden bei Raumtemperatur gerührt. Die Reaktionsmischung wird mit 300 ml eines Gemisches aus Dichlormethan und Methanol (10:1) versetzt und mit 100 ml halbgesättigter Natriumhydrogencarbonatlösung gewaschen. Die wässrige Phase wird mit 200 ml Dichlormethan extrahiert und die vereinigten organischen Phasen werden über Natriumsulfat getrocknet. Nach Reinigung durch Chromatographie an Kieselgel werden 160 mg der Titelverbindung erhalten. Die spektroskopischen Daten sind in der nachfolgenden Tabelle aufgeführt.

### Verfahren V3

### Beispiel B168 (Z,E/Z)-2-Cyan-N-(cyanmethyl)-2-[3-ethyl-5-[[[2-(4-methyl-1-piperazinyl)-5-benzoxazolyl]amino]methylen]-4-oxo-2-thiazolidinyliden]acetamid

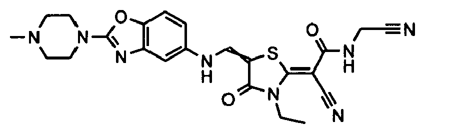

215 mg der als **INTT6** beschriebenen Verbindung werden in 10 ml Ethanol gelöst. Es werden 260 mg der als **INT70** beschriebenen Verbindung und 0,39 ml Triethylorthoformiat zugegeben und es wird 2 Stunden unter Rückfluss gerührt. Es werden weitere 0,19 ml Triethylorthoformiat zugegeben und es wird weitere 4 Stunden unter Rückfluss gerührt. Nach dem Erkalten der Reaktionsmischung wird der ausgefallene Feststoff abfiltriert. Nach Reinigung durch Chromatographie an Aminophasenkieselgel und anschließender Kristallisation aus Ethanol werden 284 mg der Titelverbindung erhalten. Die spektroskopischen Daten sind in der nachfolgenden Tabelle aufgeführt.

### Verfahren V4

### Beispiel B73 (Z,E/Z)-4-[6-[[[2-[1-Cyan-2-oxo-2-[(2,2,2-trifluorethyl)amino]ethyliden]-3-ethyl-4-oxo-5-thiazolidinyliden]methyl]amino]-1H-indol-1-yl]-1-piperidinessigsäure

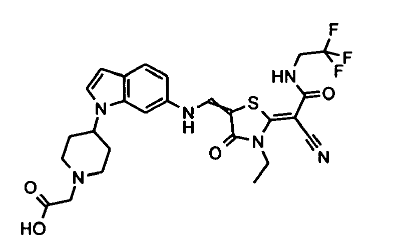

50 mg der als **B72** beschriebenen Verbindung werden mit 420 µl einer zwei molaren wässrigen Natriumhydroxidlösung in 10 ml Methanol vier Stunden auf 50°C erhitzt. Das Reaktionsgemisch wird am vakuum eingeengt, in Wasser aufgenommen, mit ein molarer wässriger Salzsäure auf pH 7 bis 8 eingestellt, und mit Butanol extrahiert. Die organische Phase wird eingeengt und aus Ethanol kristallisiert. Man erhält 45 mg der Titelverbindung als hellbraune Kristalle. Die spektroskopischen Daten sind in der nachfolgenden Tabelle aufgeführt.

Die nachfolgenden Verbindungen werden analog der oben beschriebenen Verfahren hergestellt.

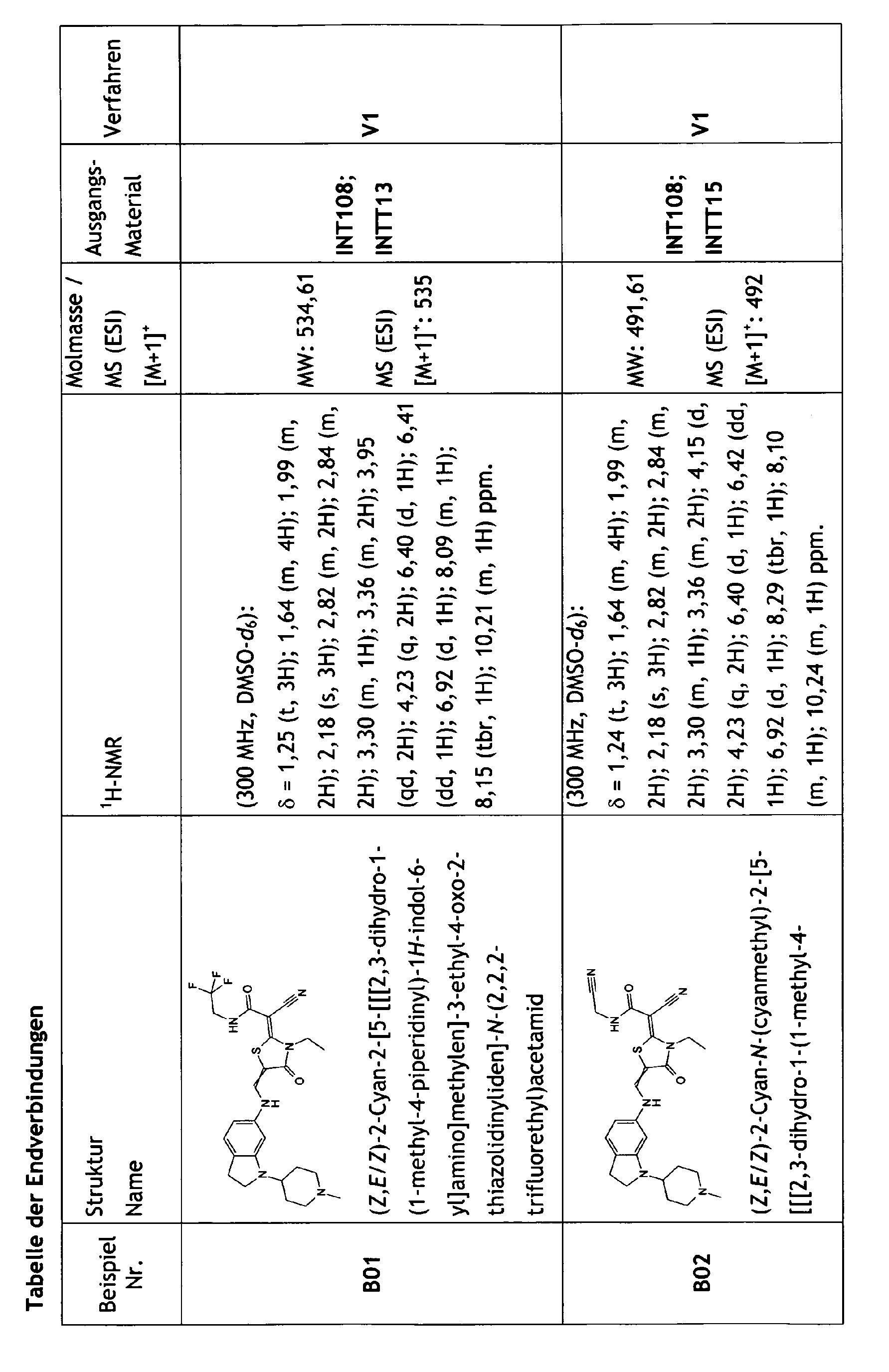

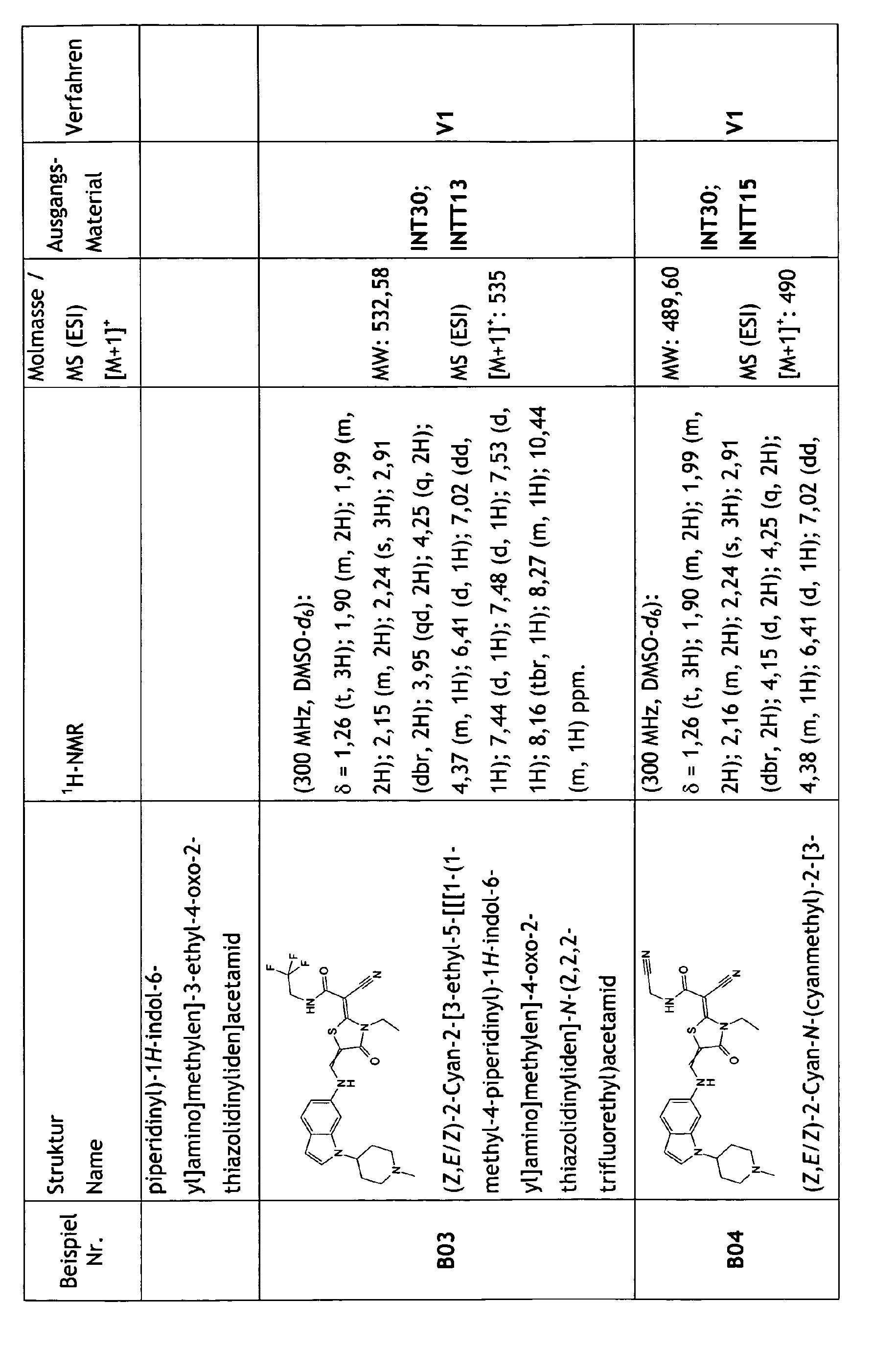

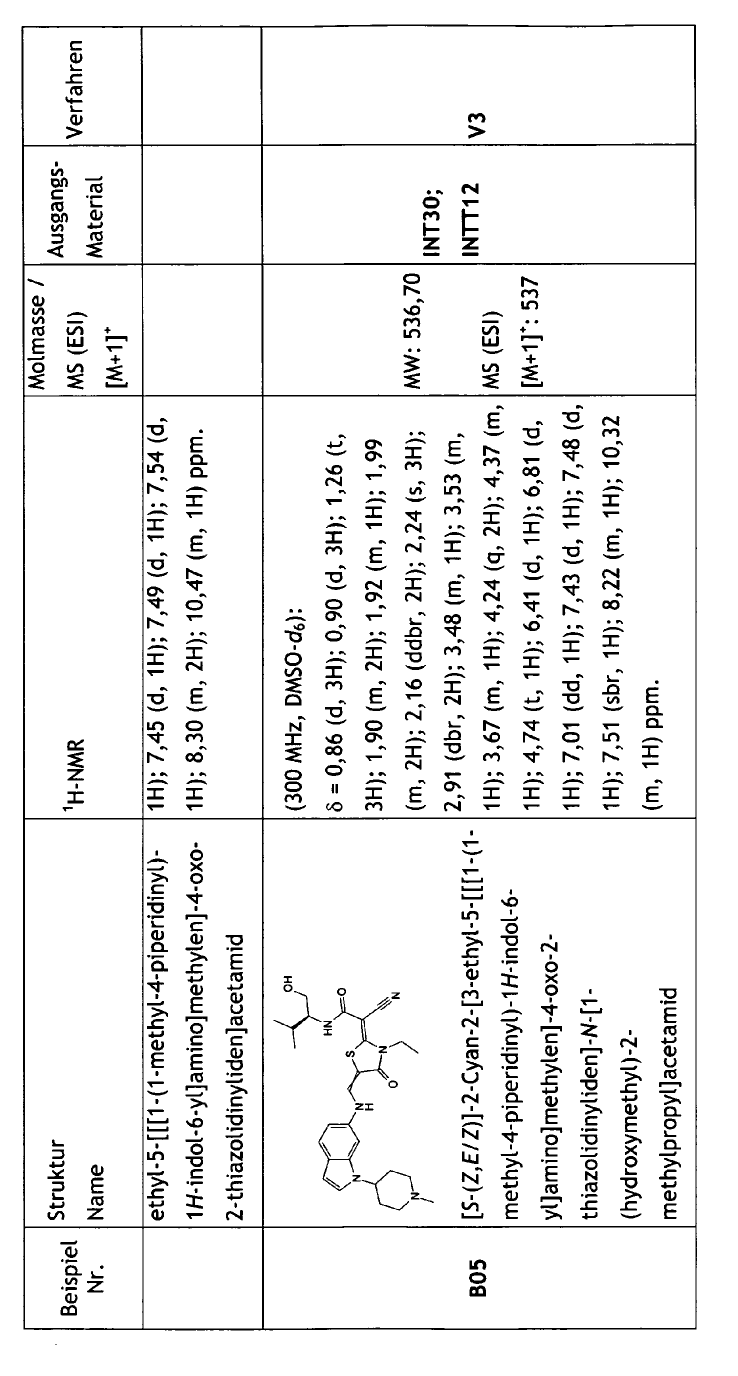

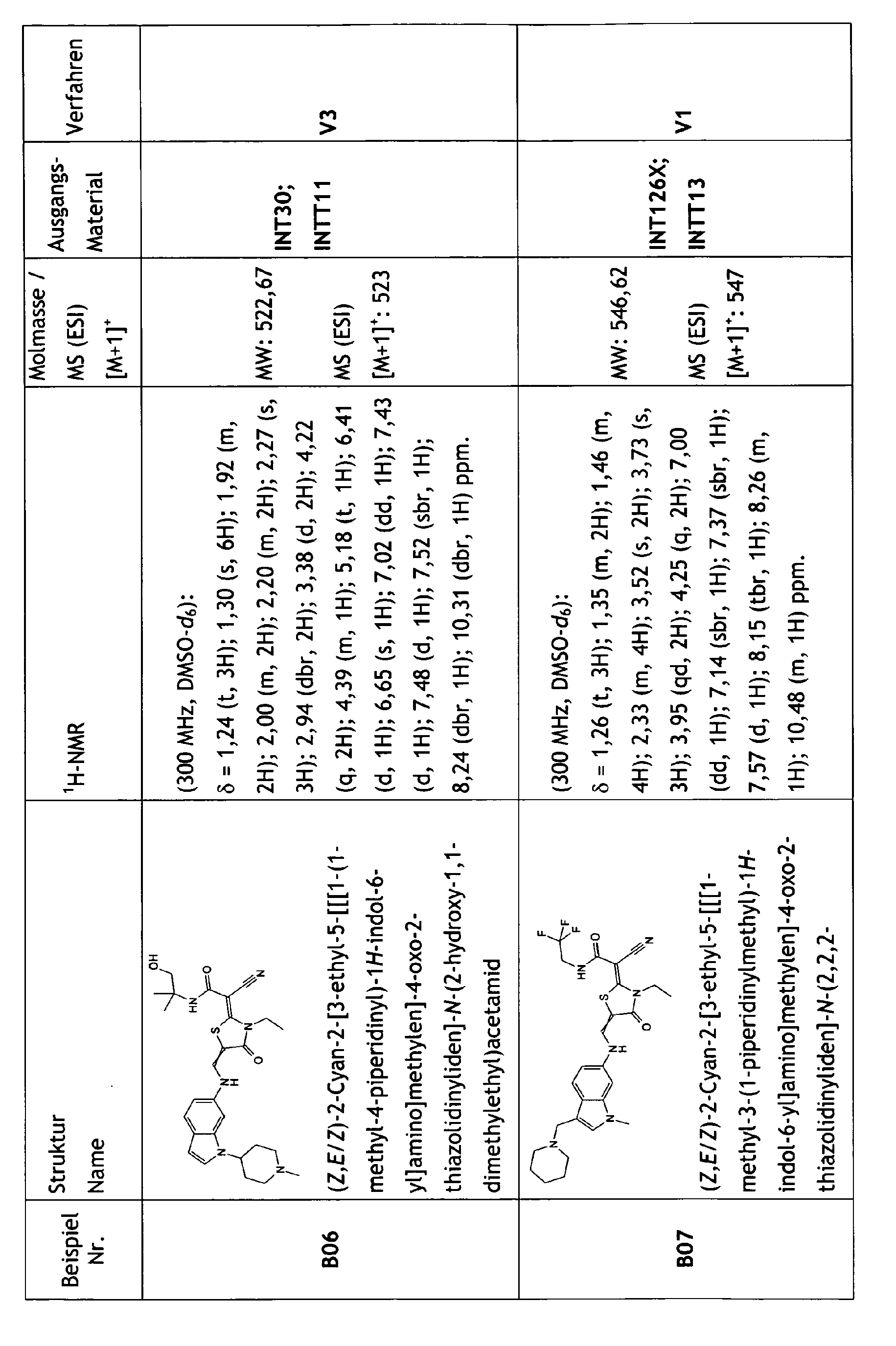

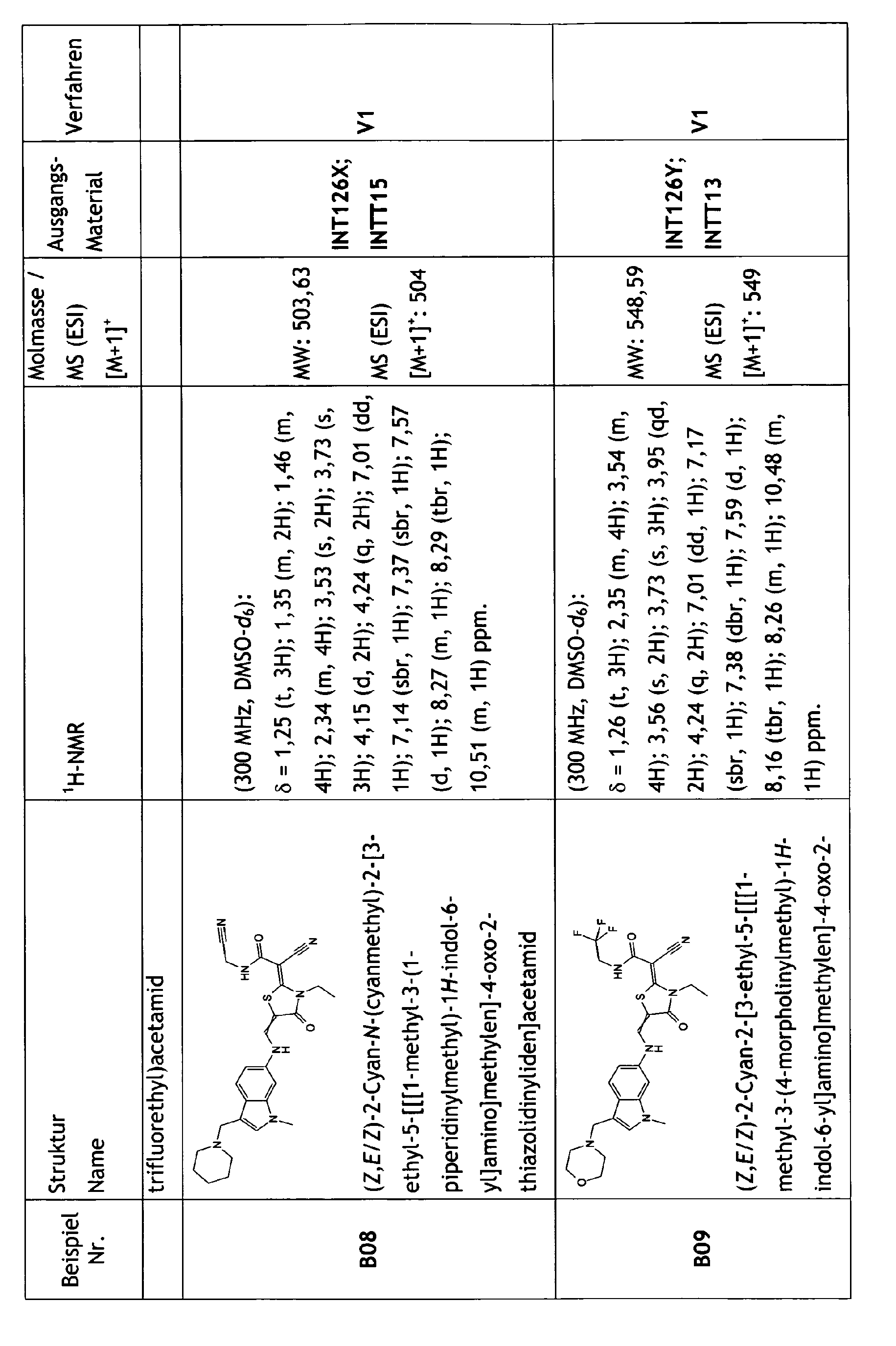

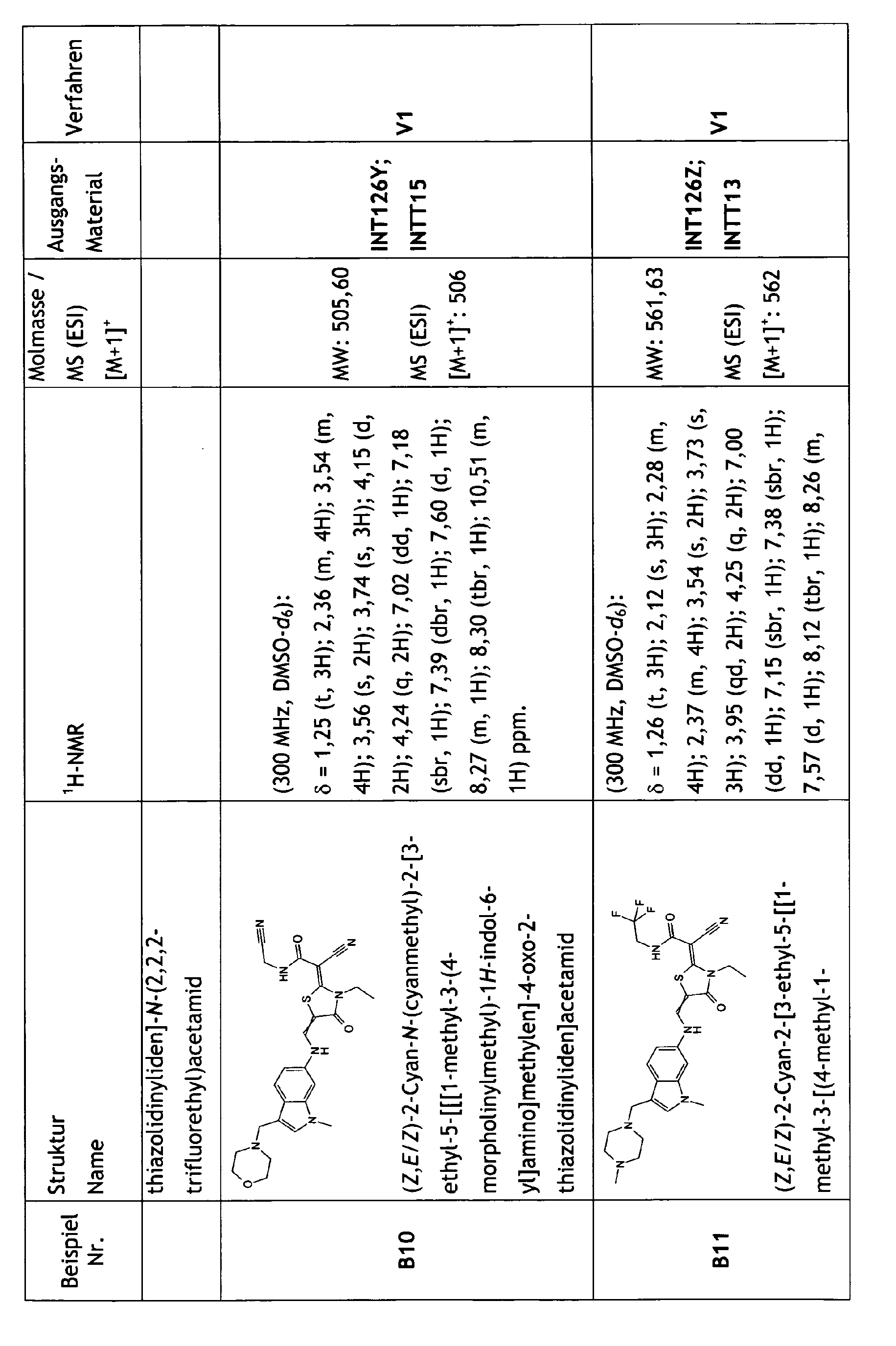

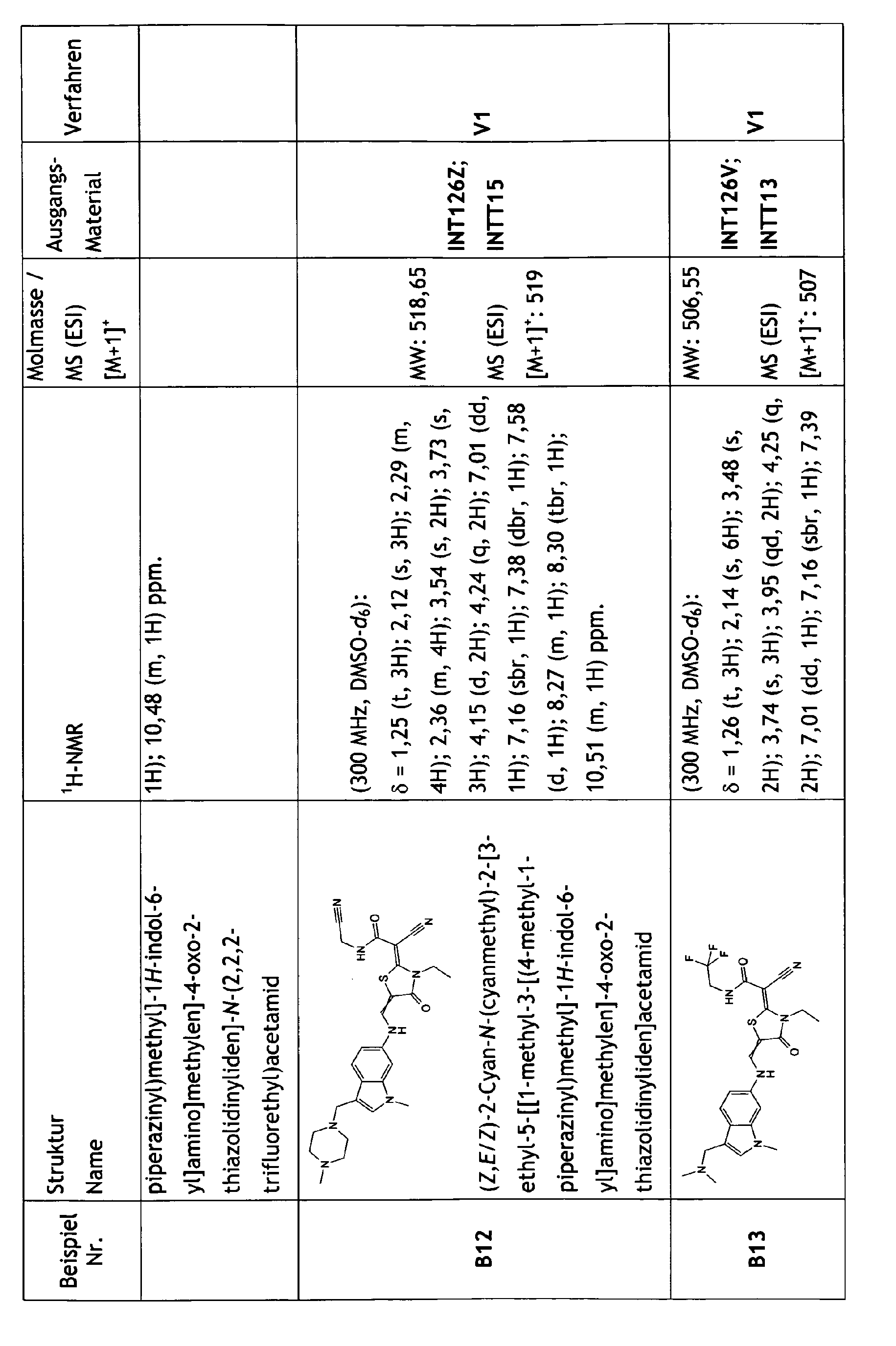

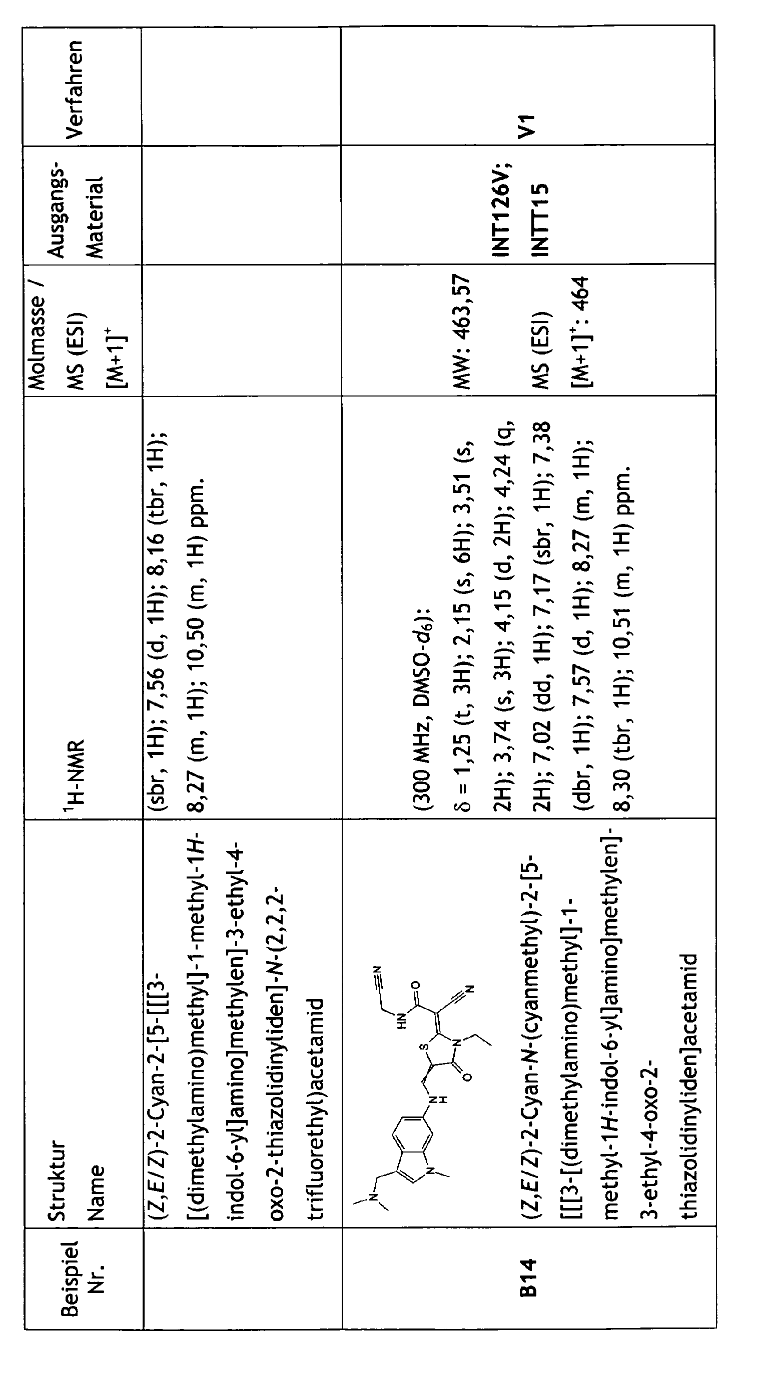

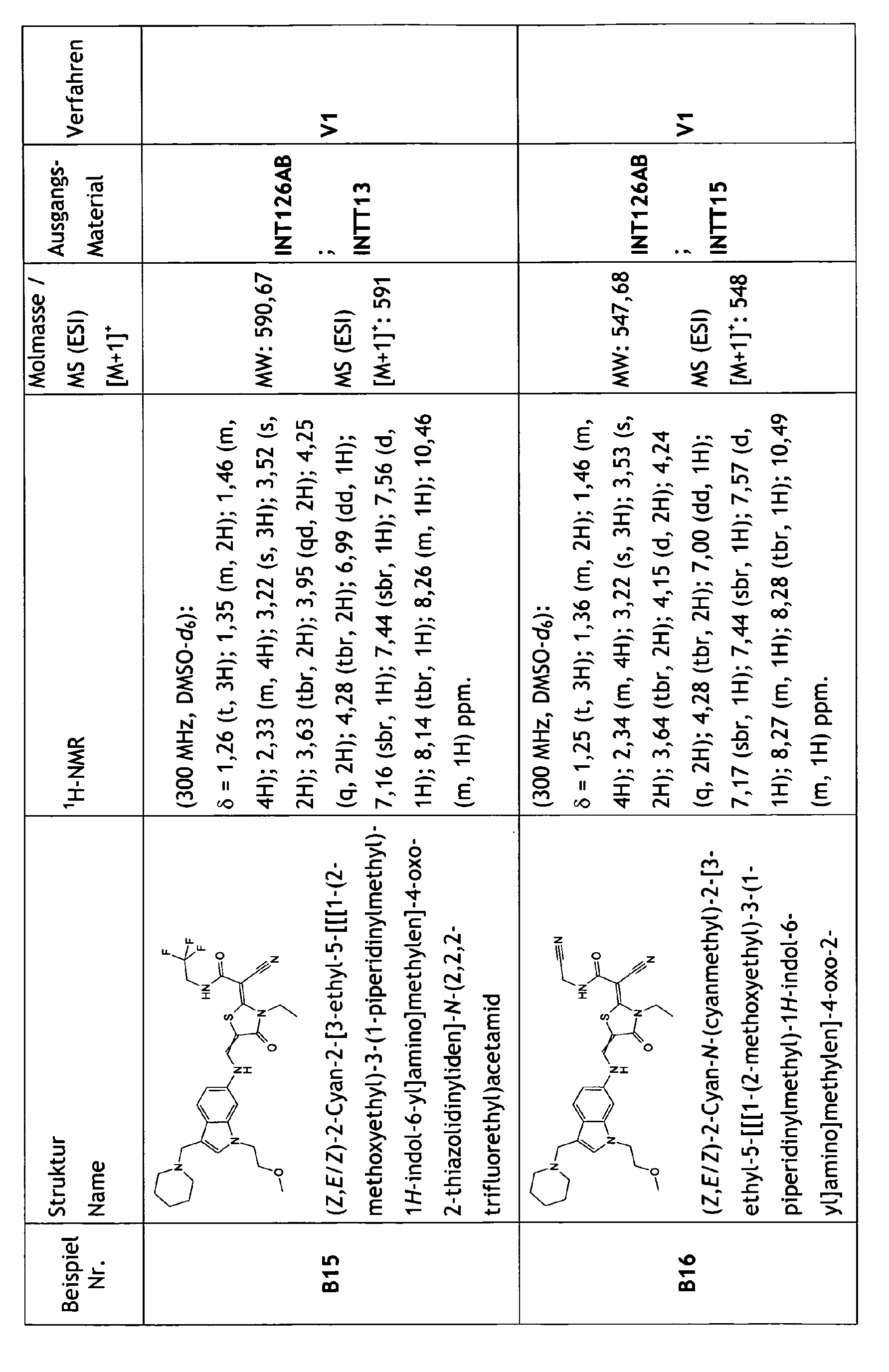

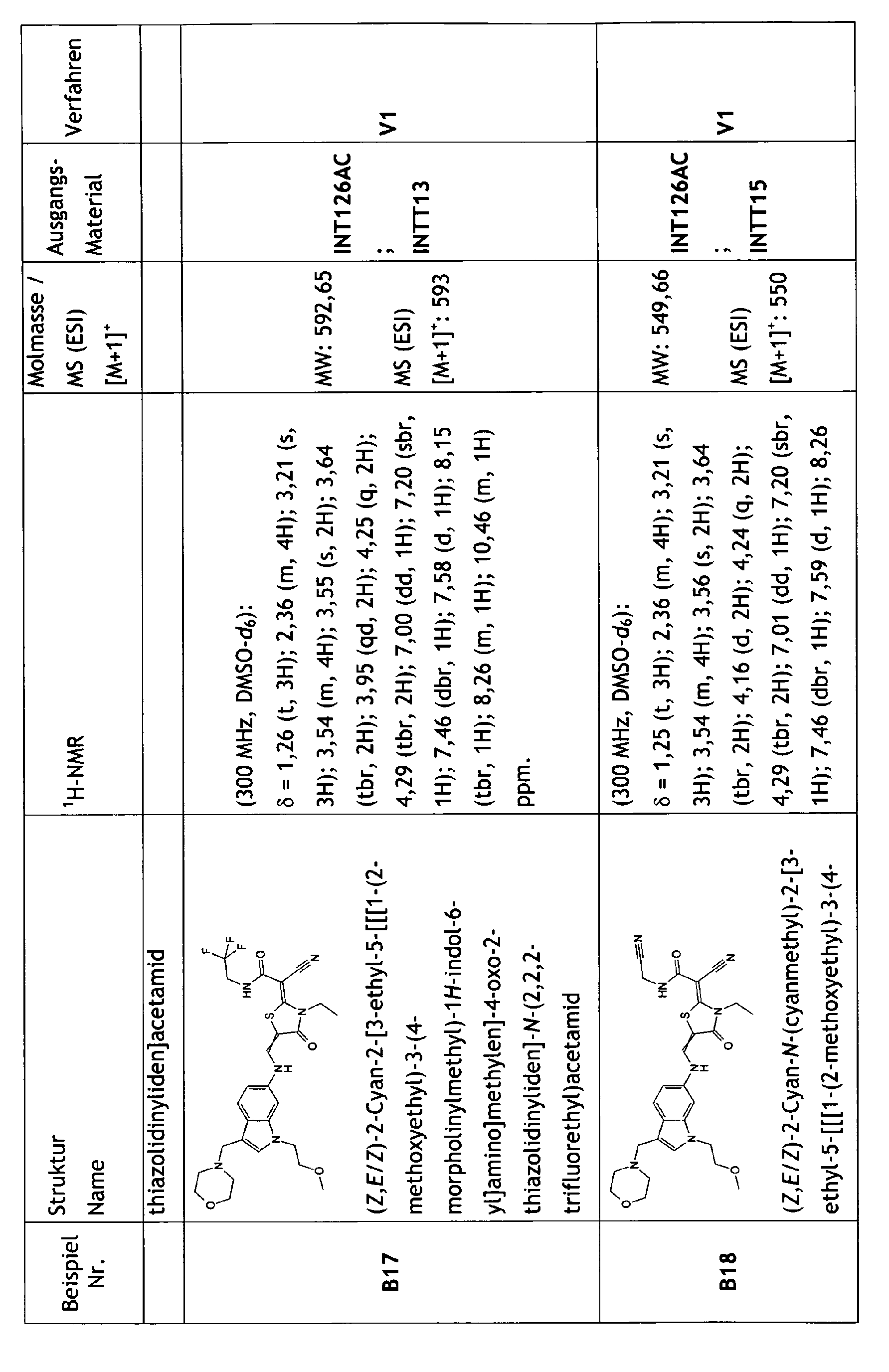

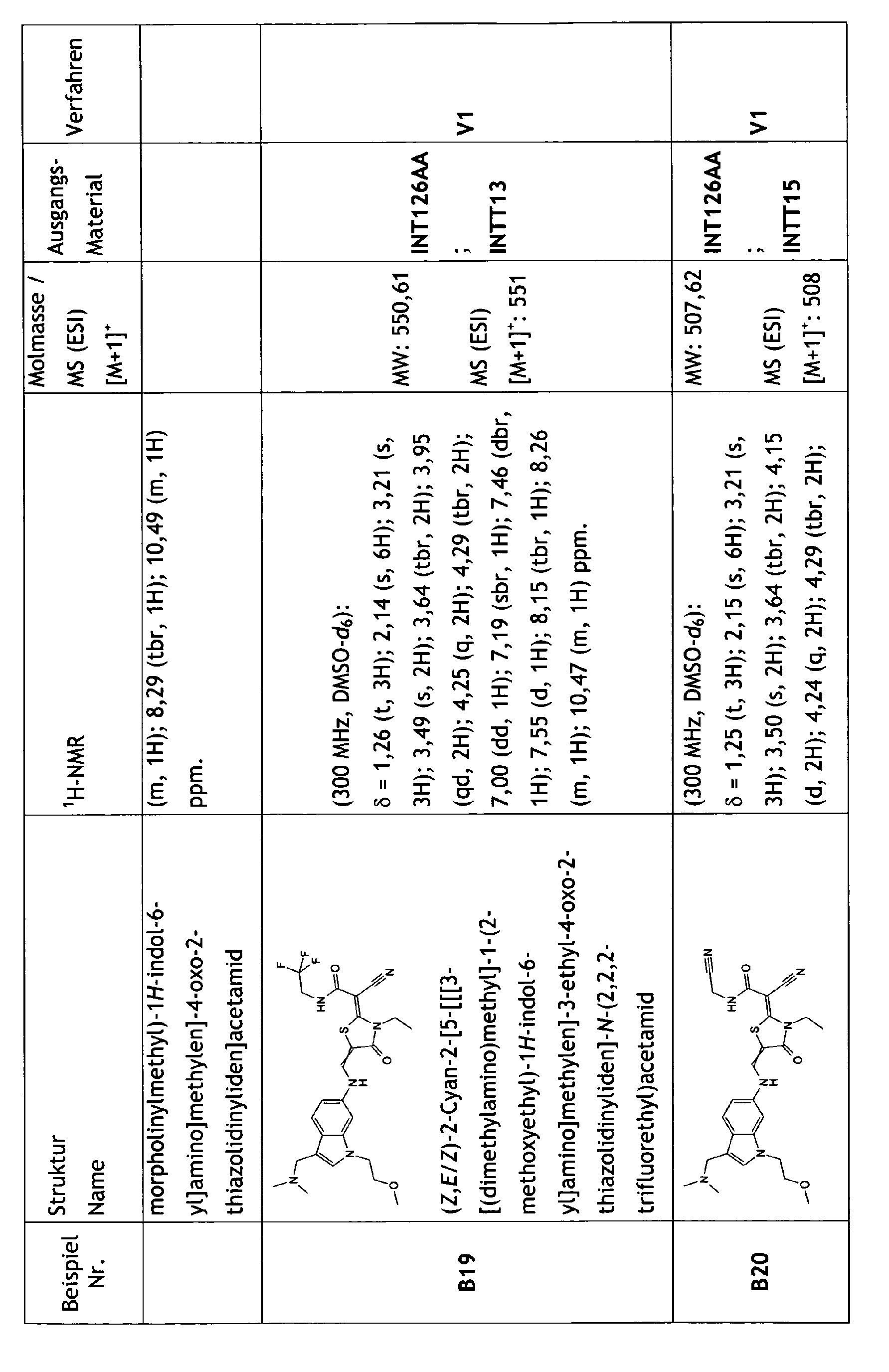

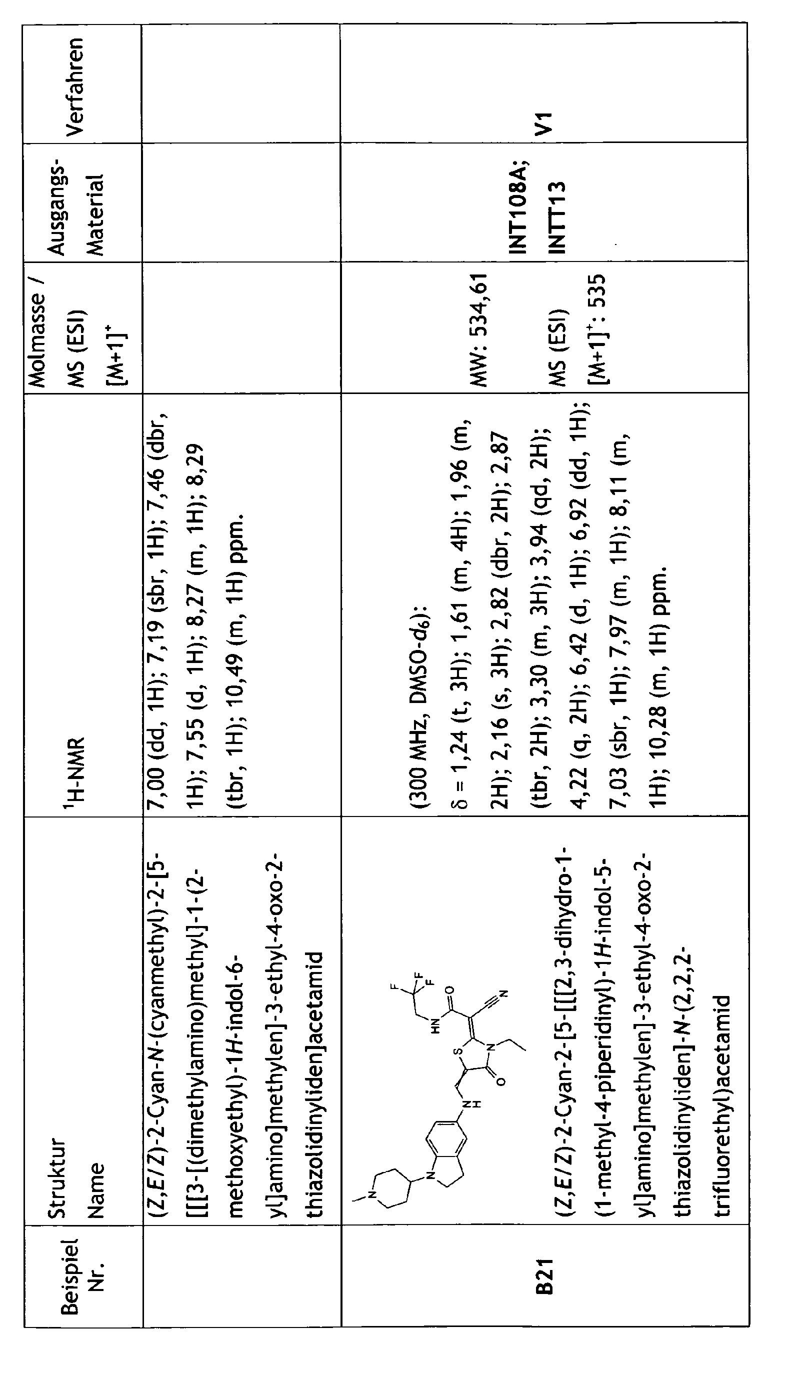

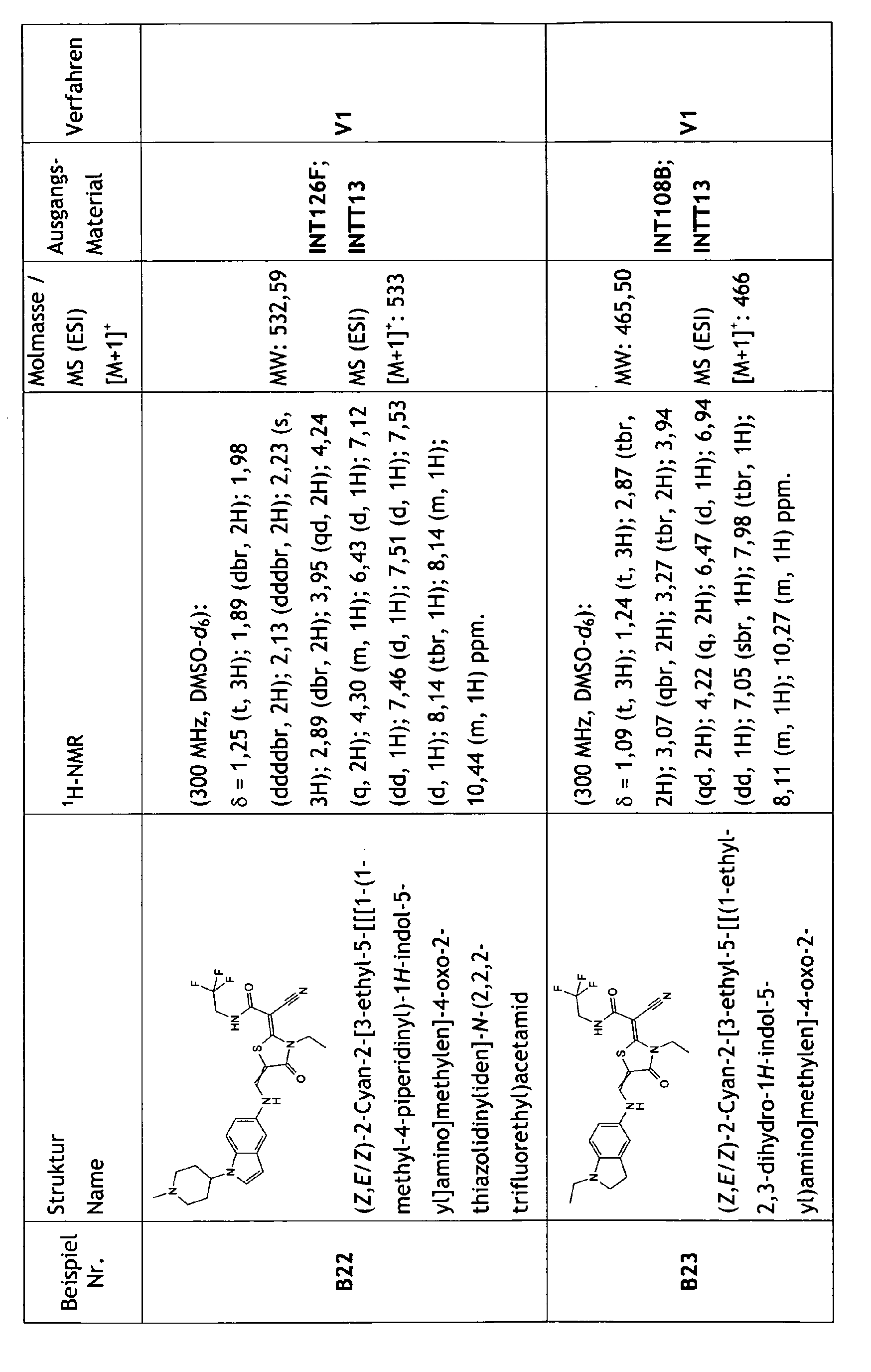

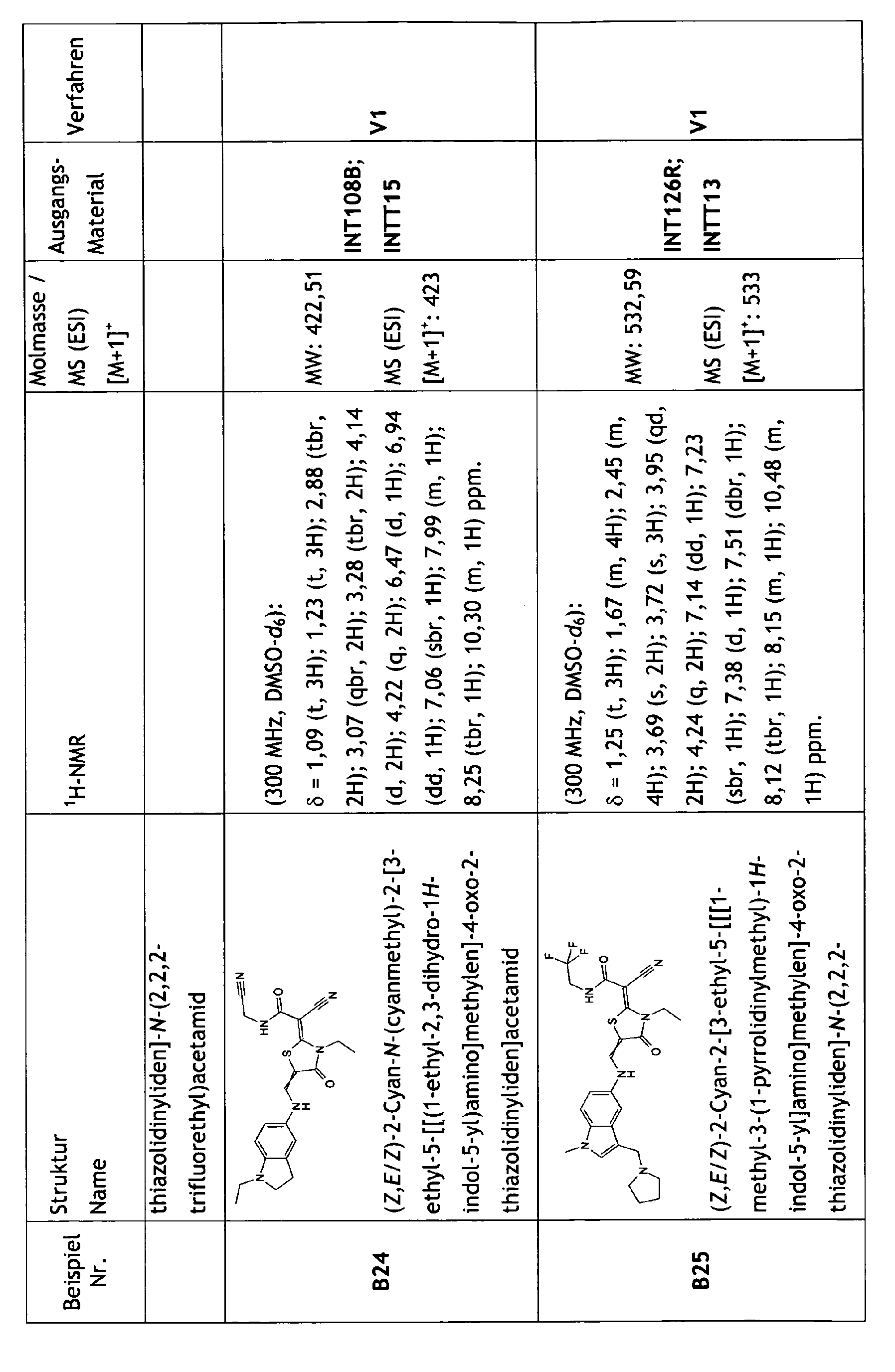

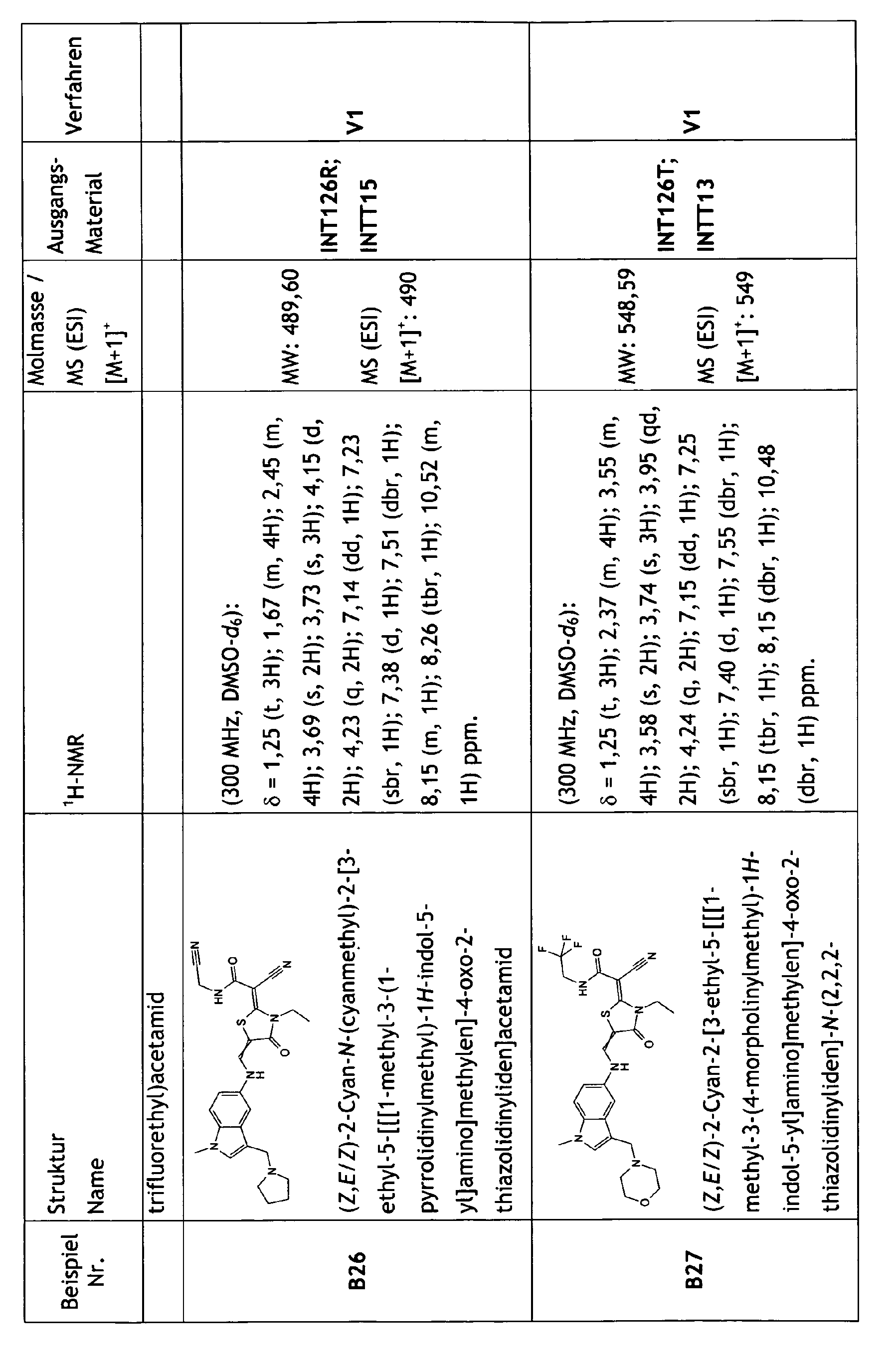

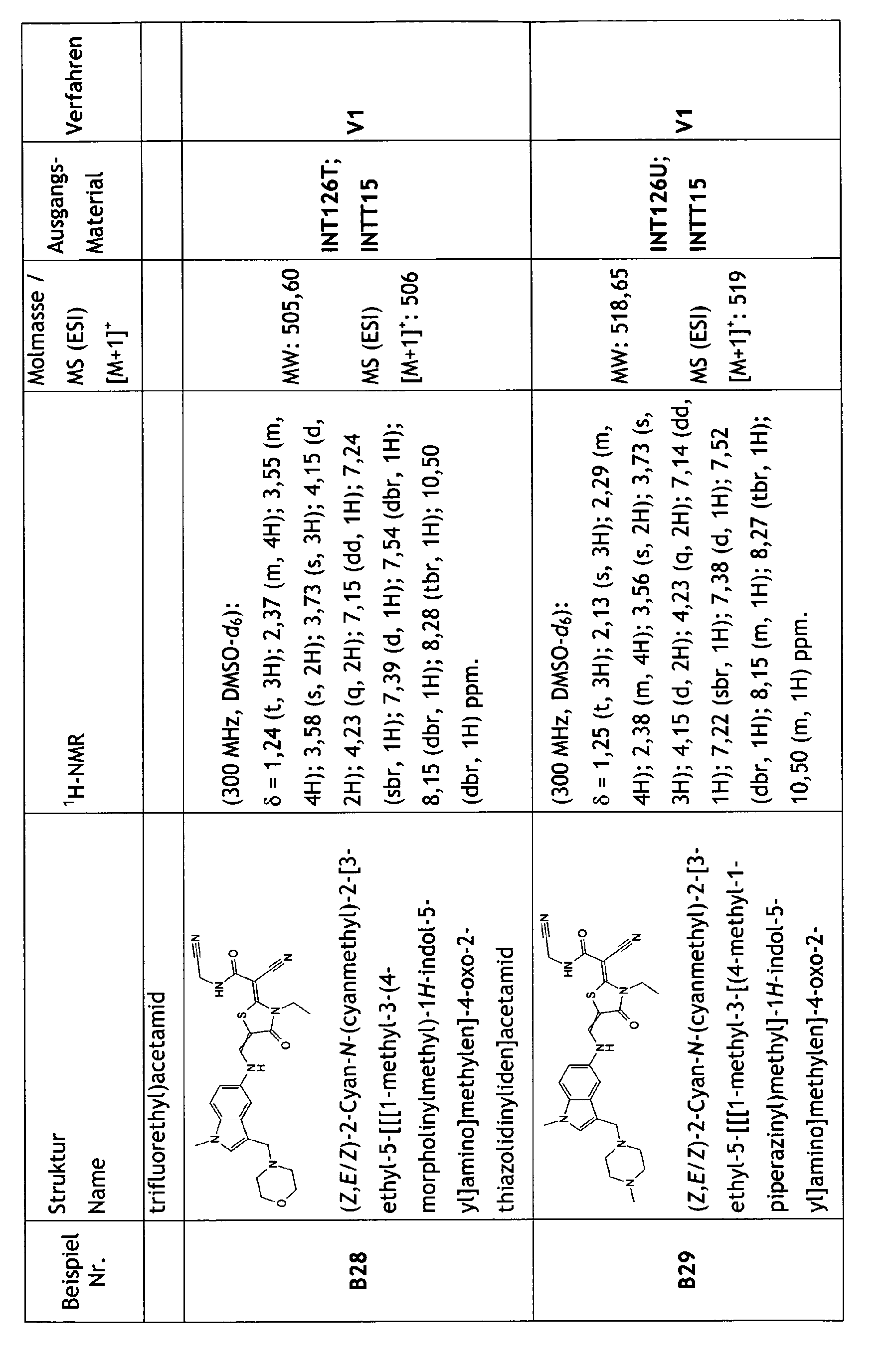

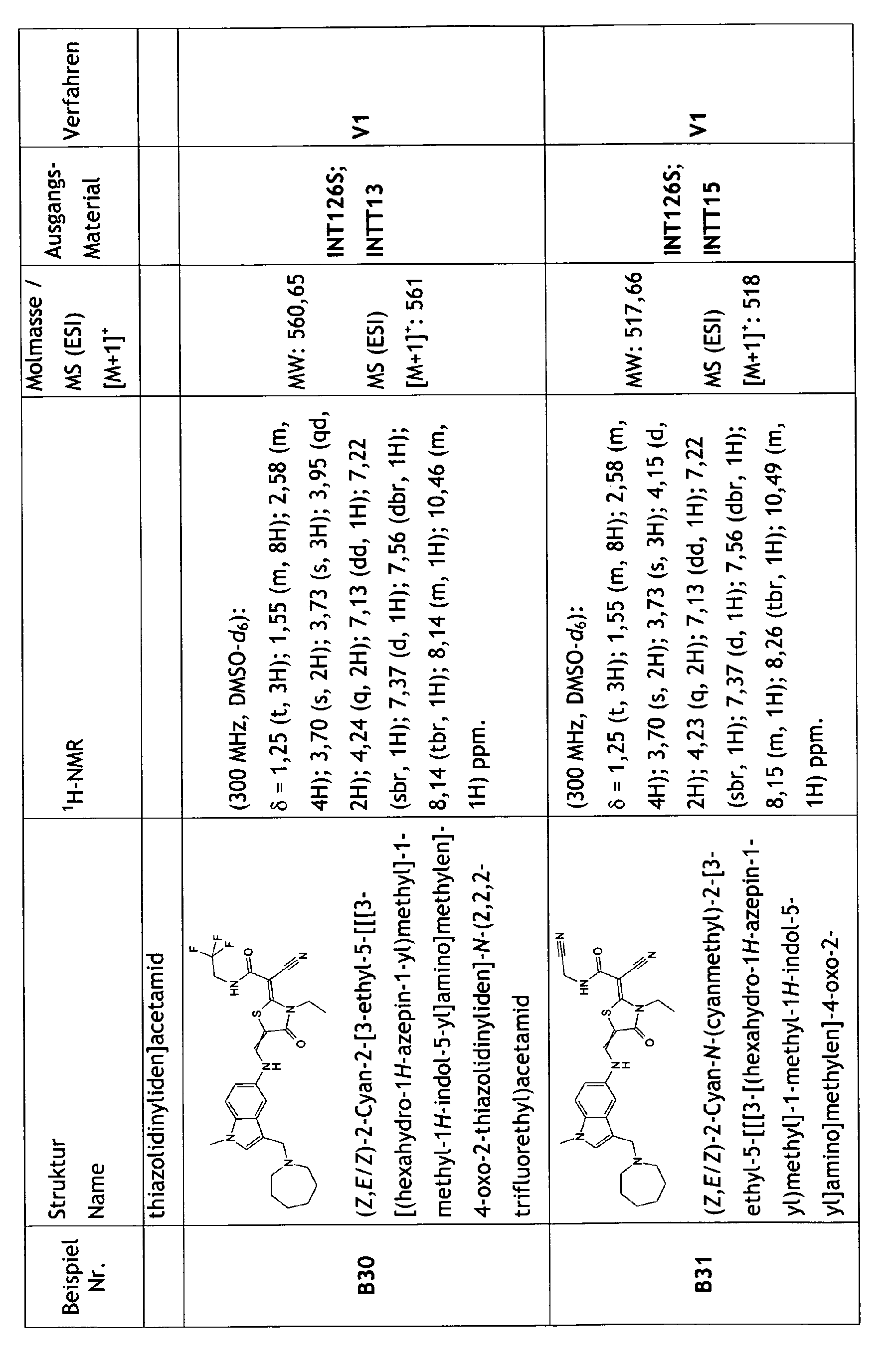

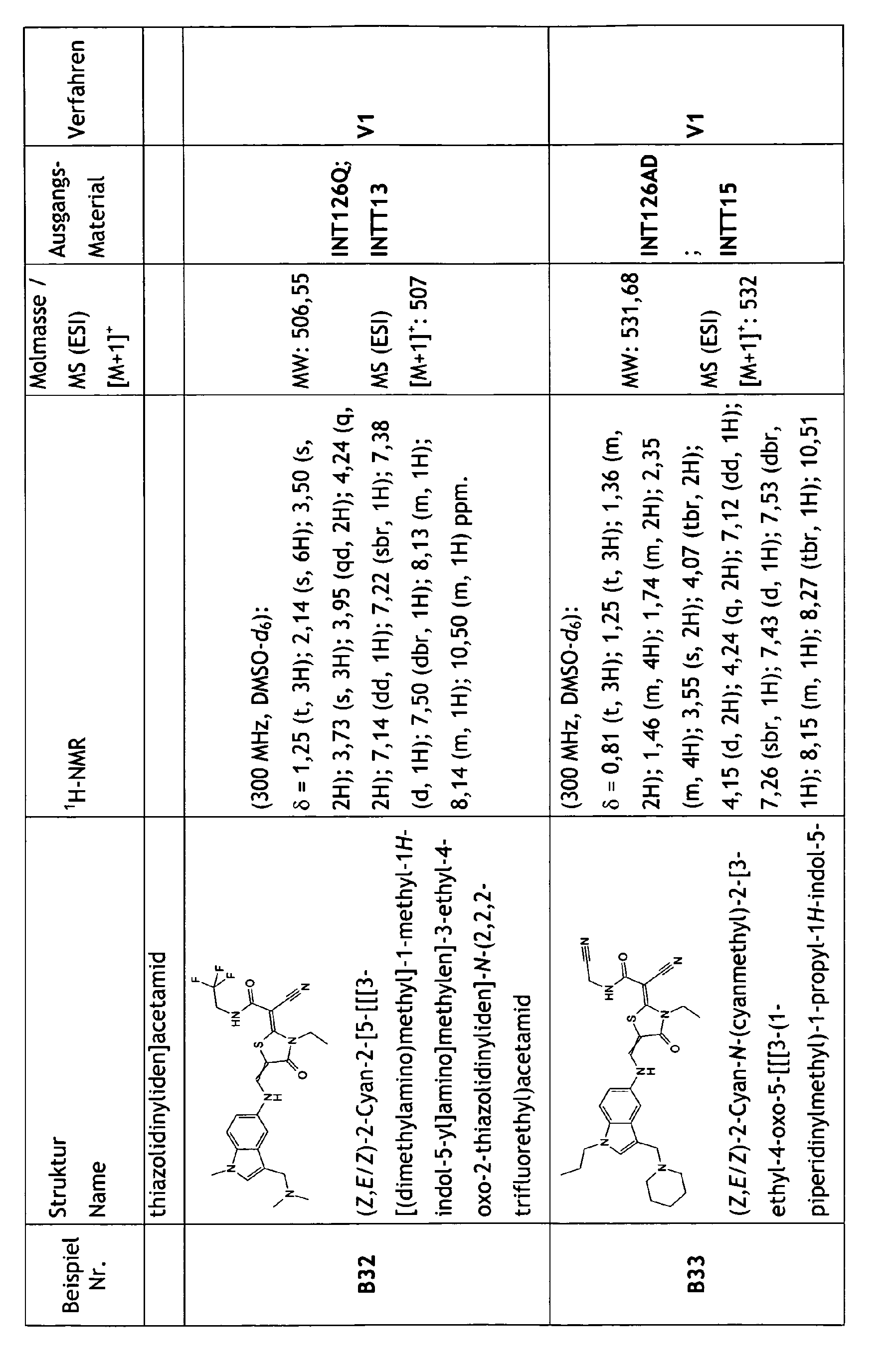

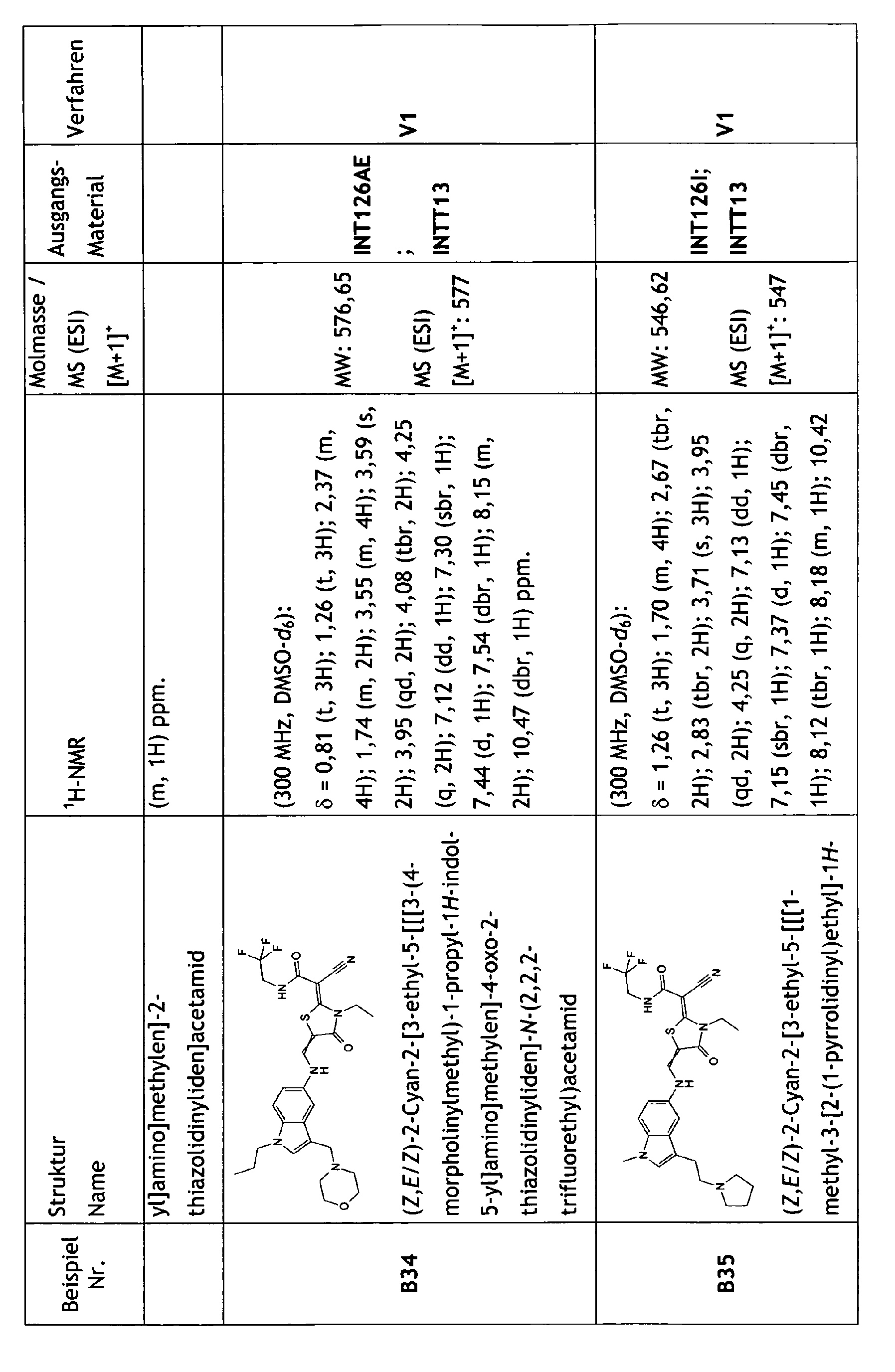

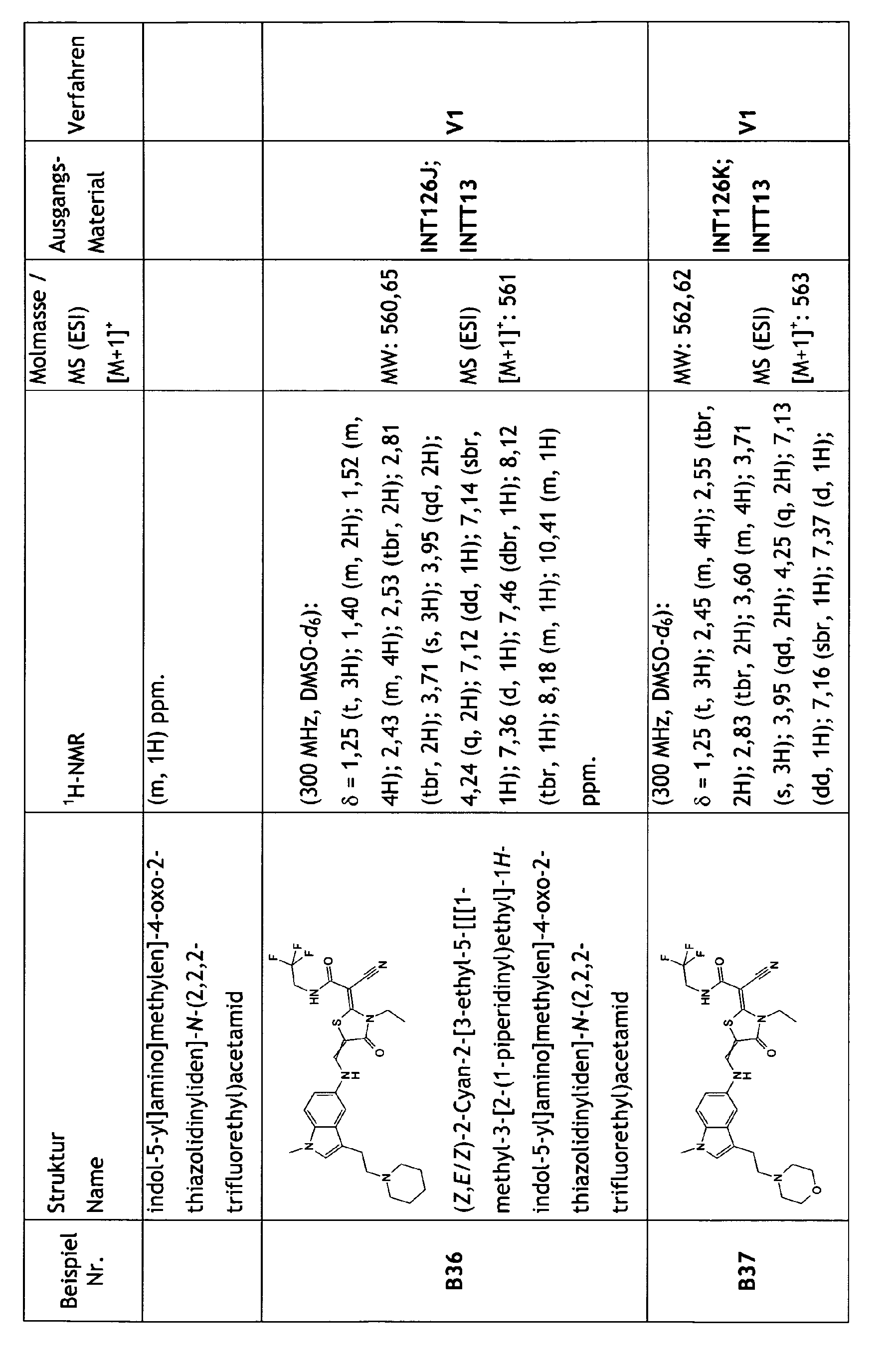

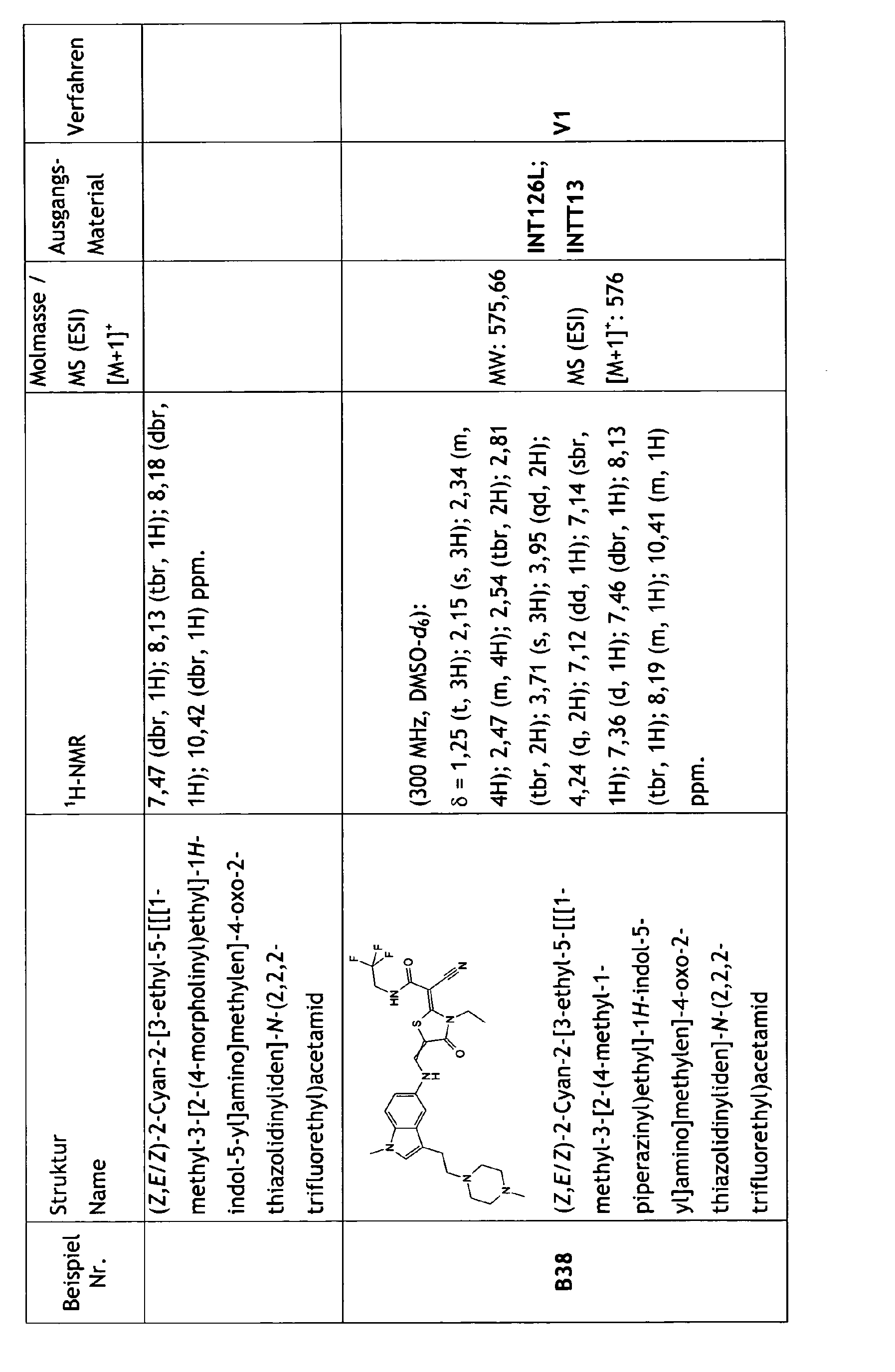

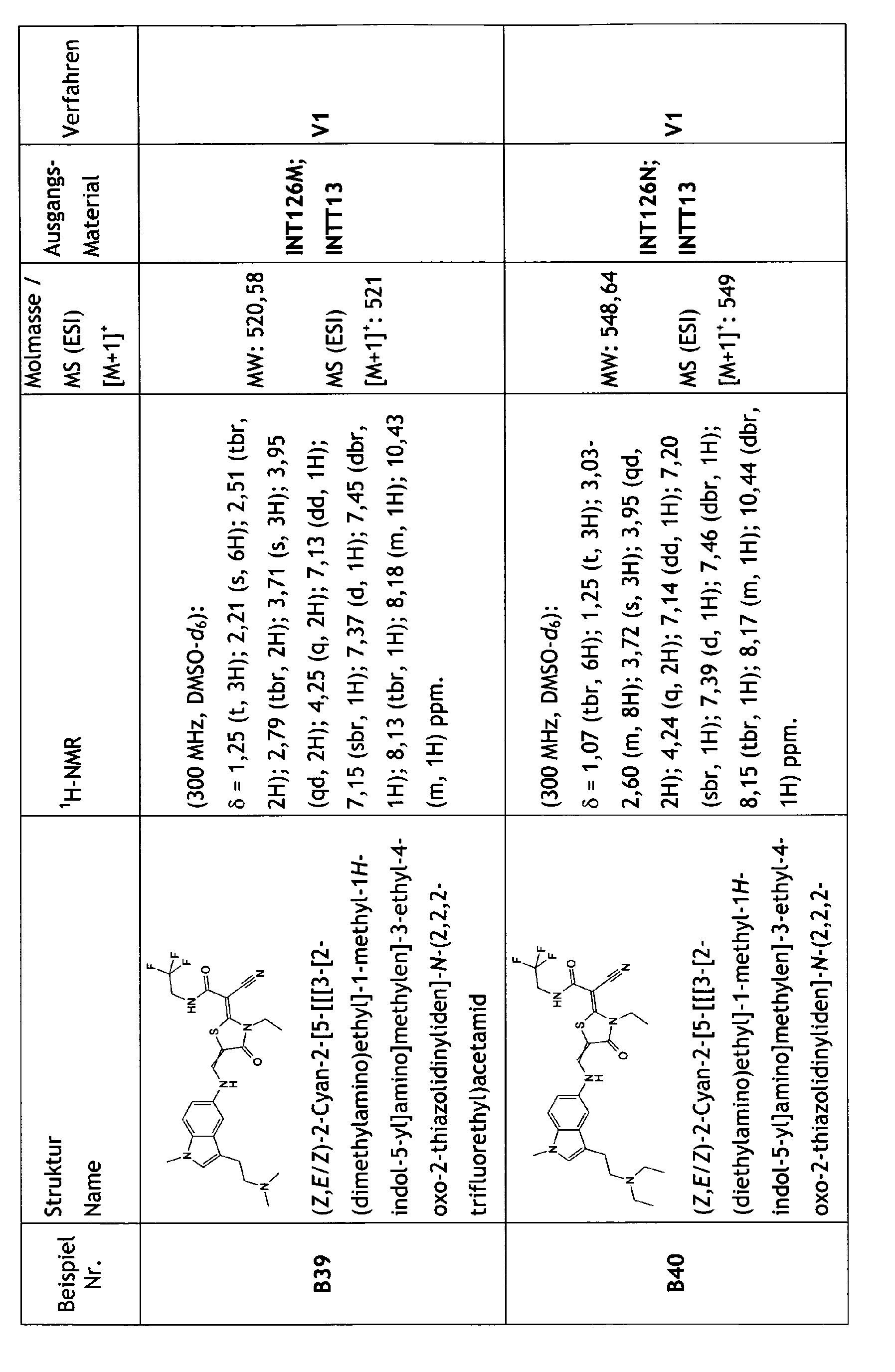

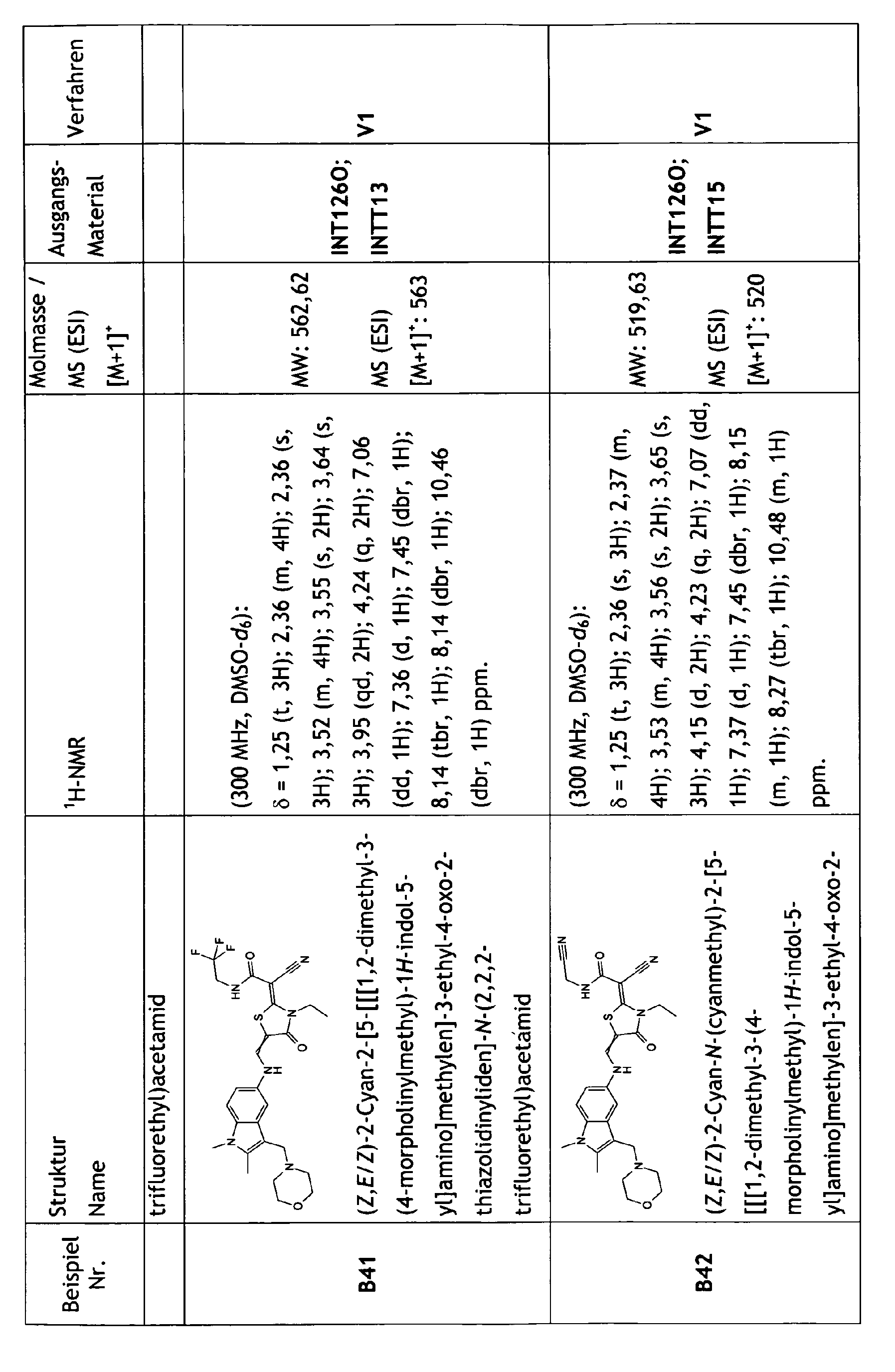

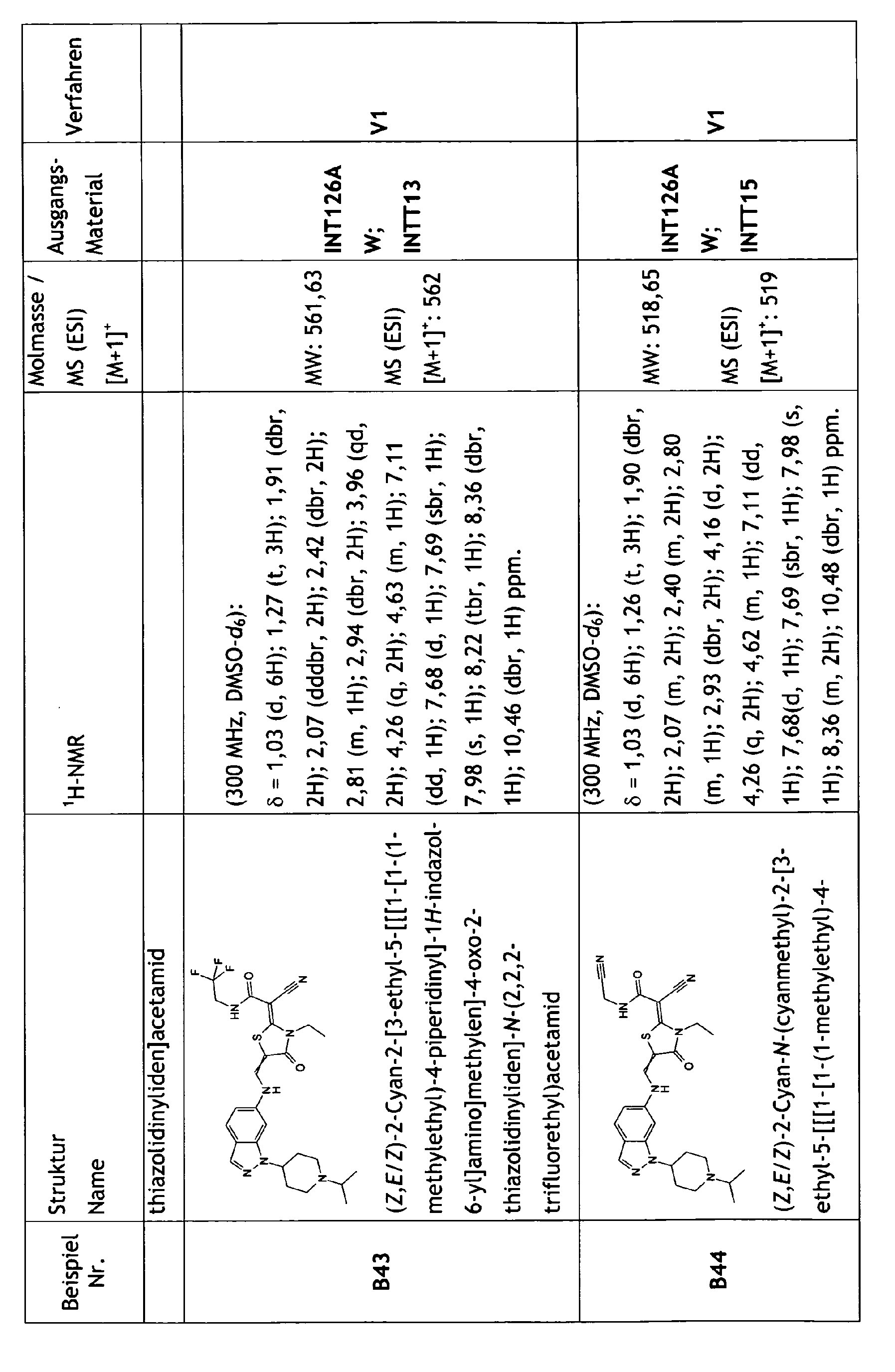

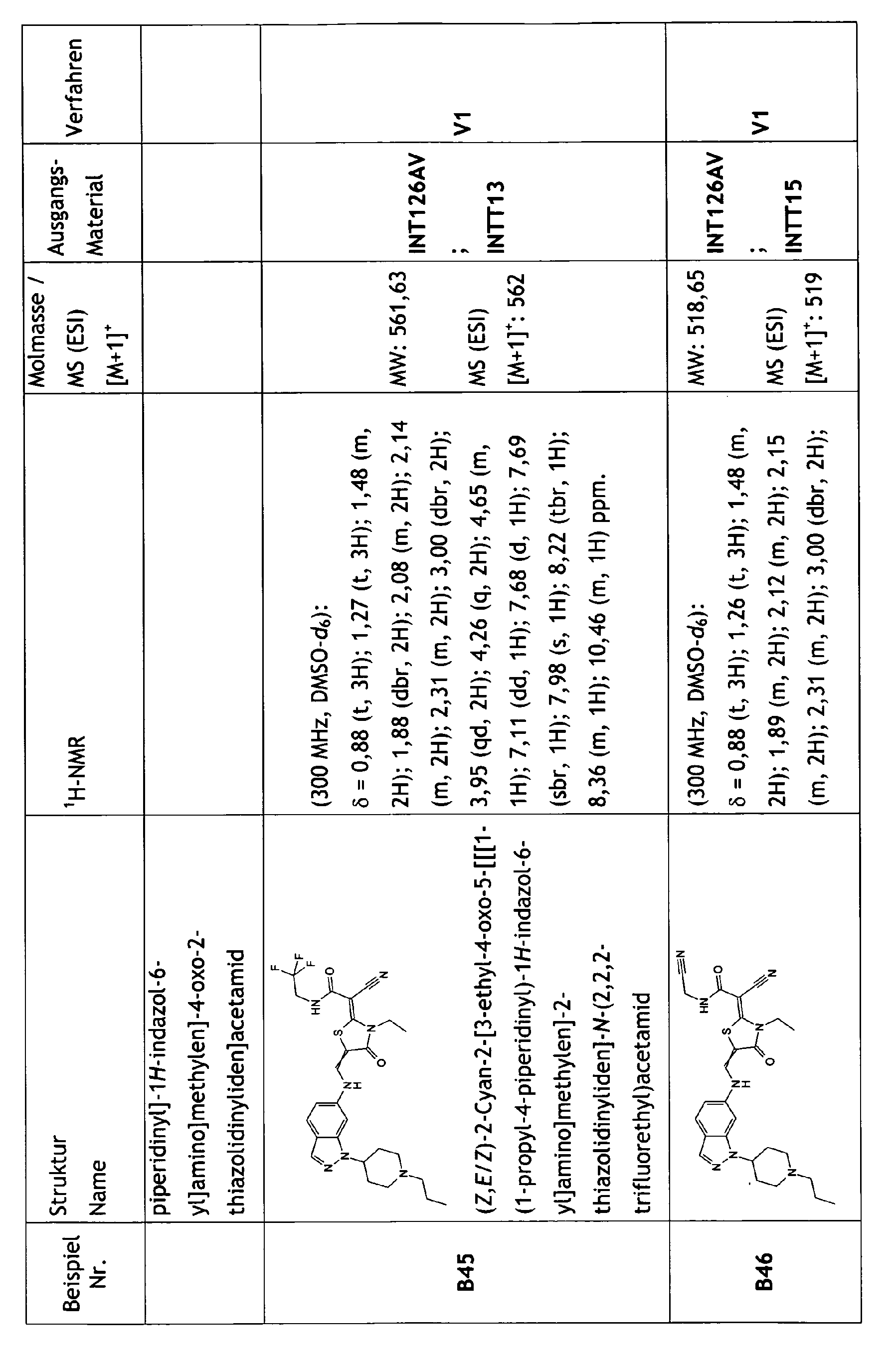

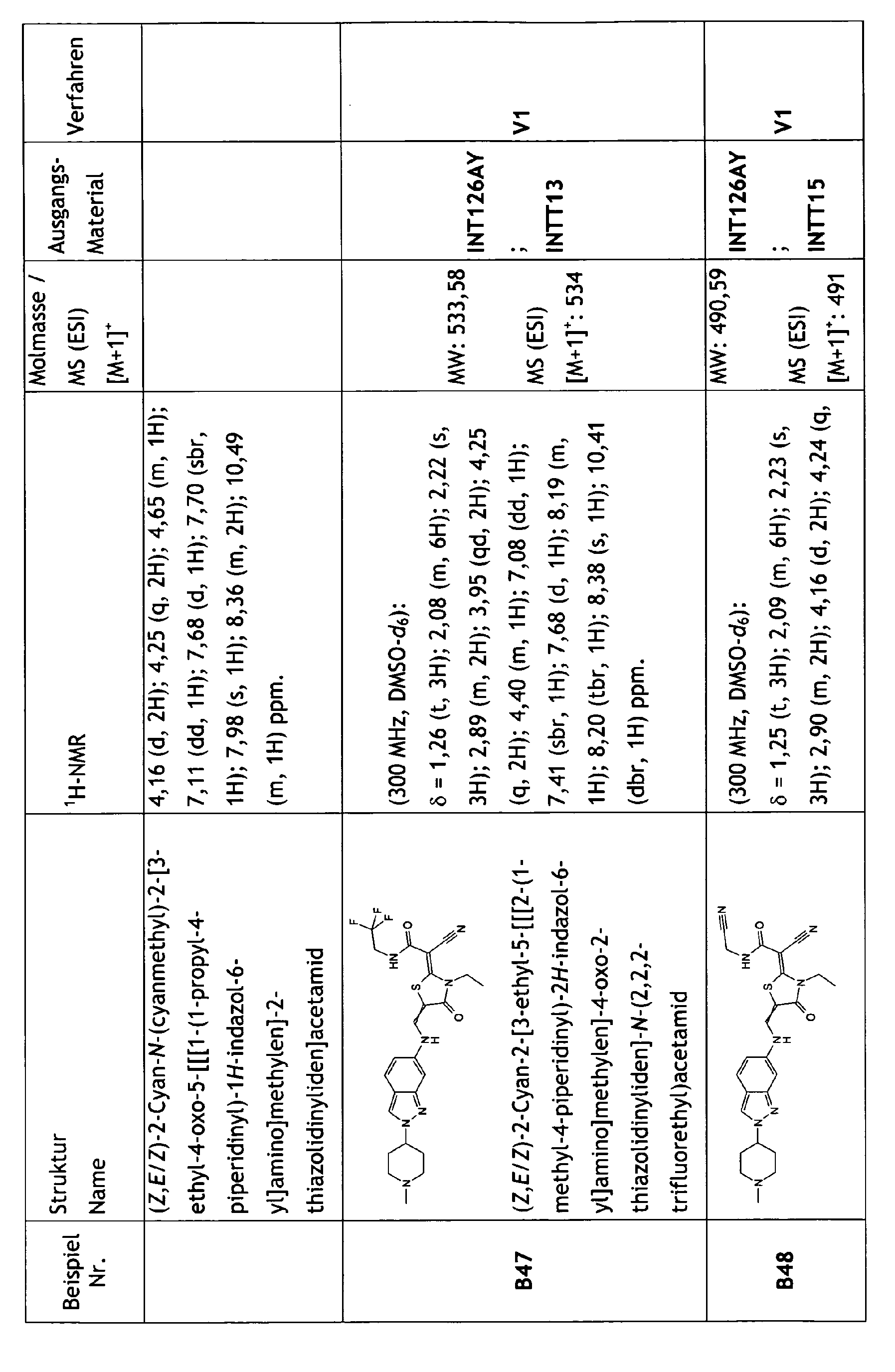

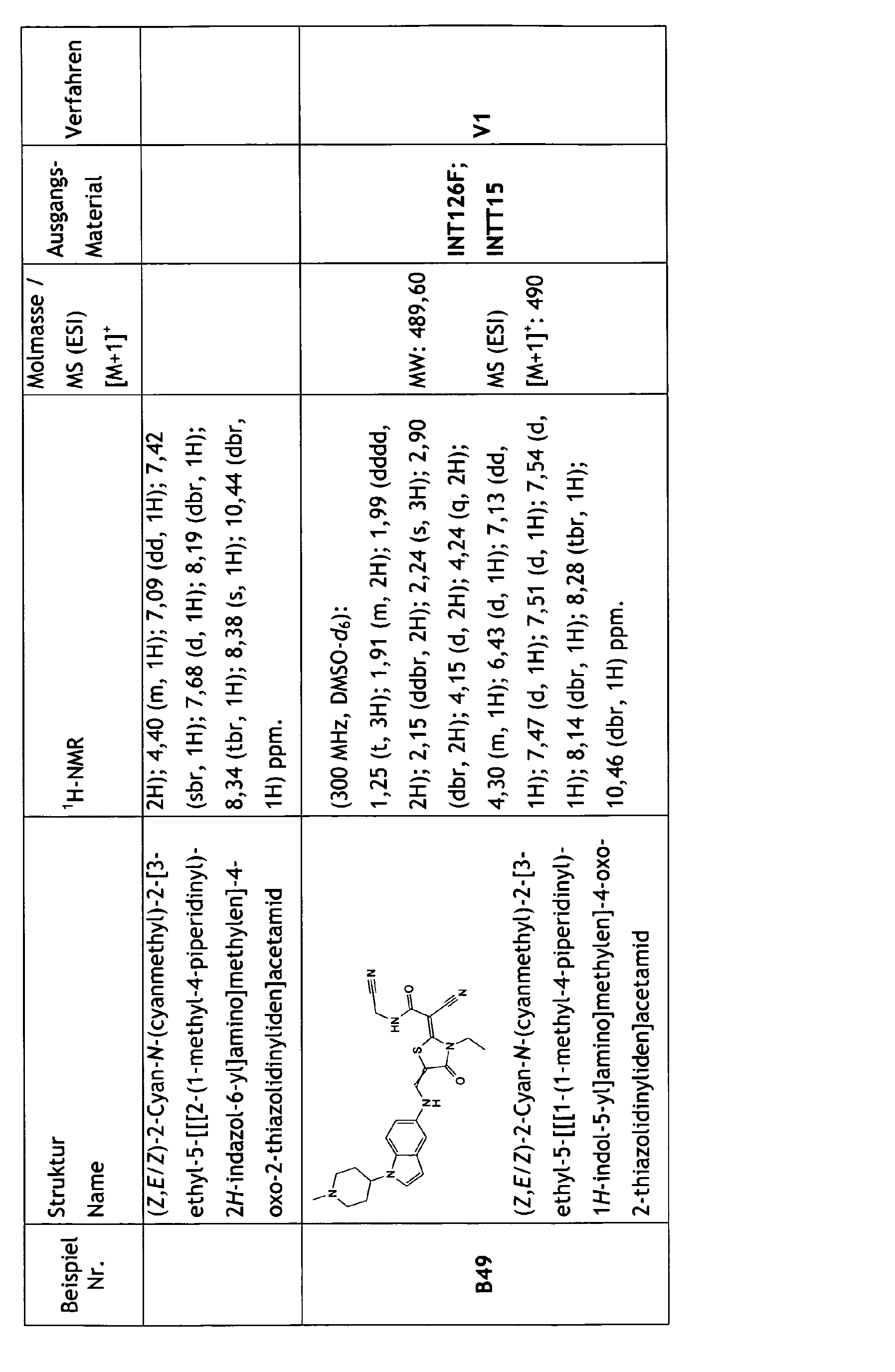

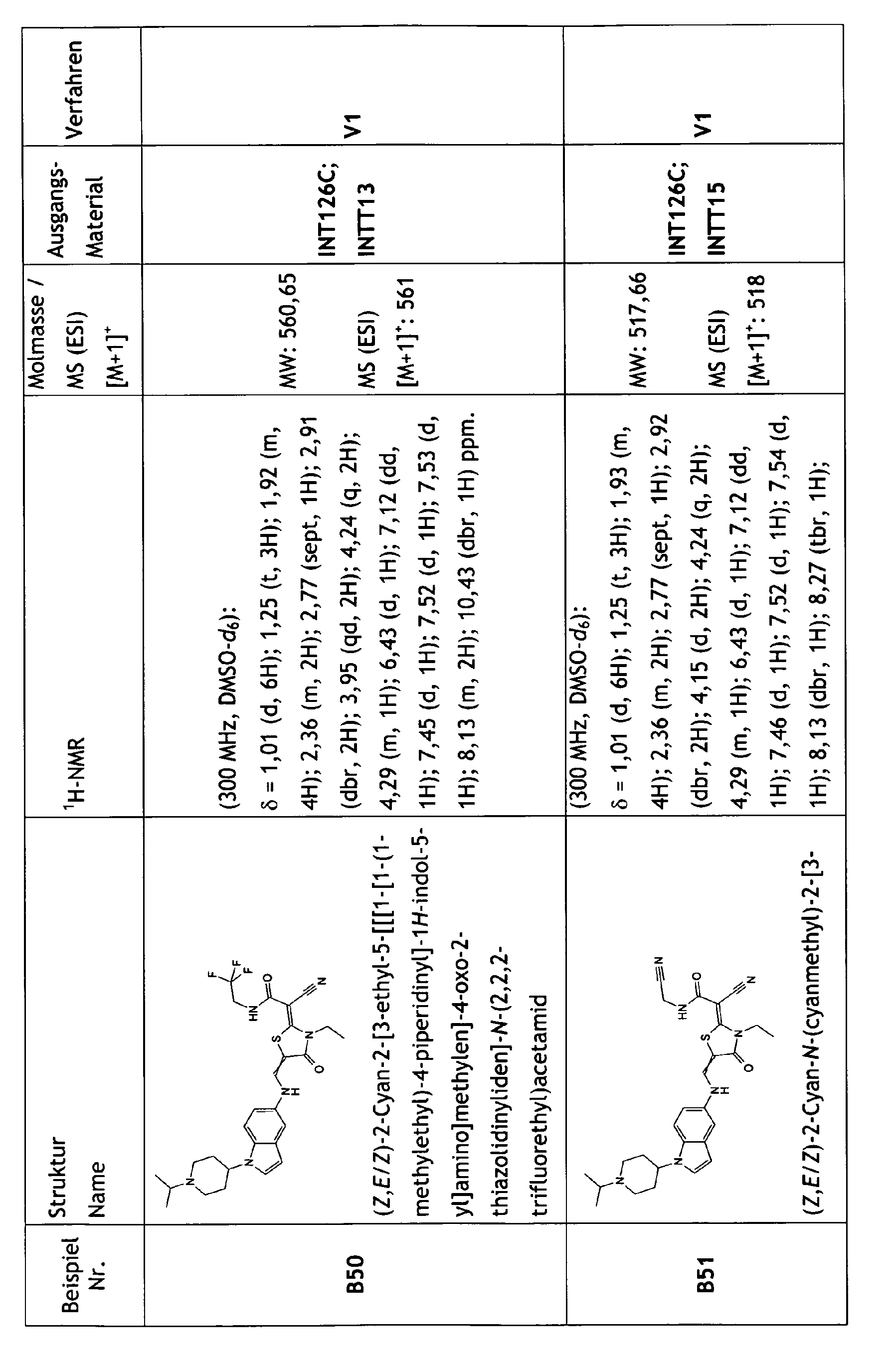

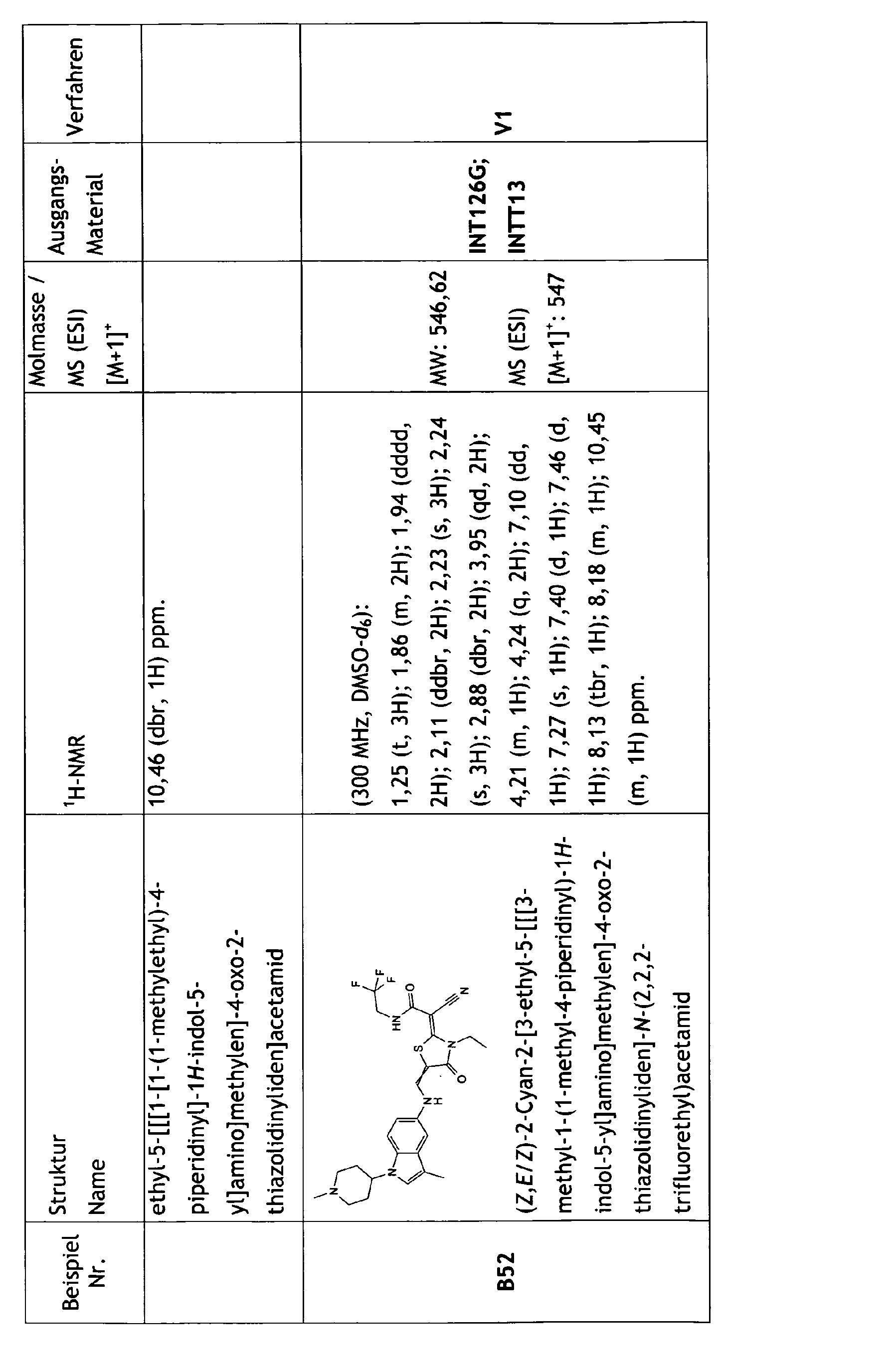

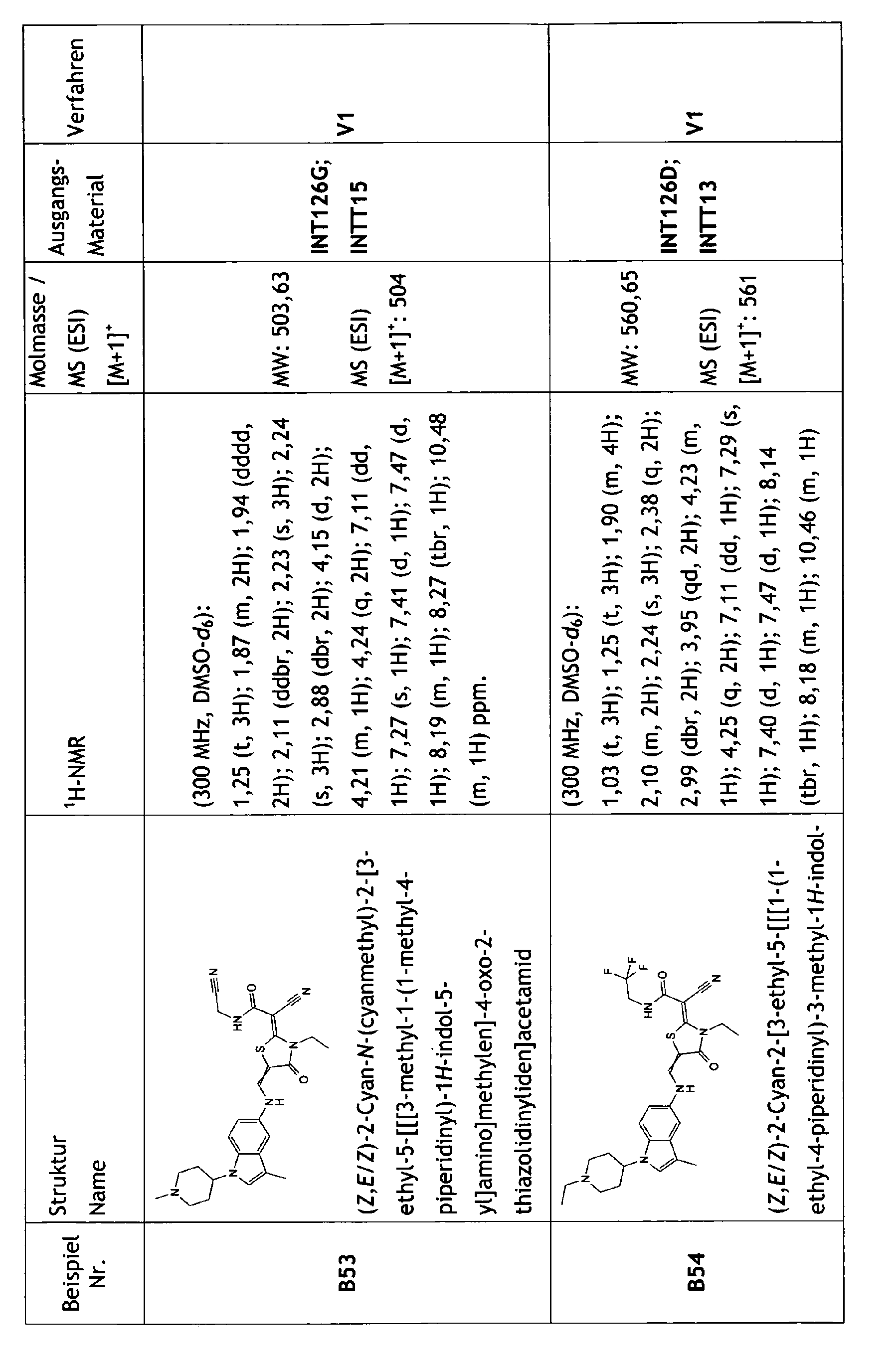

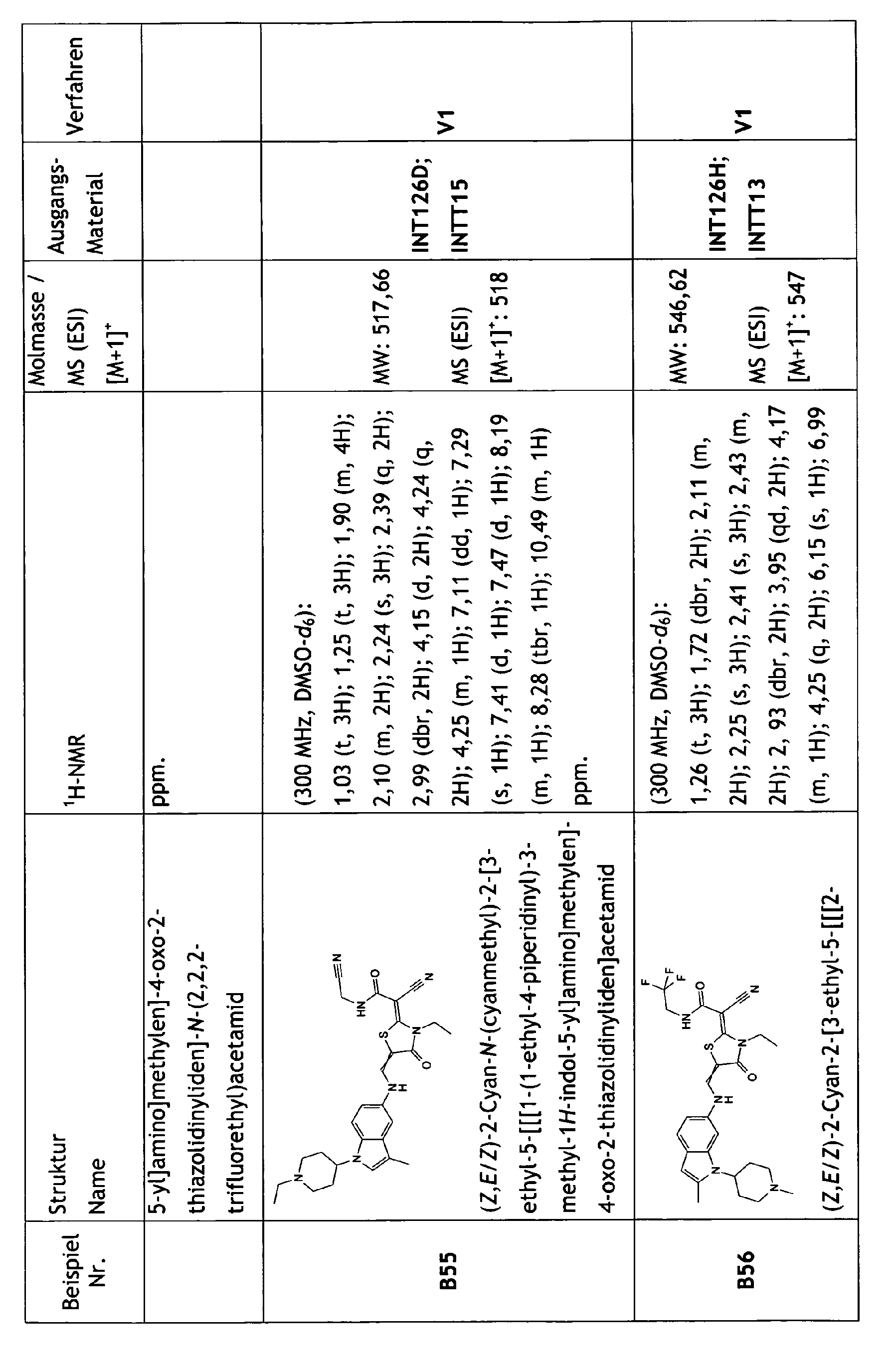

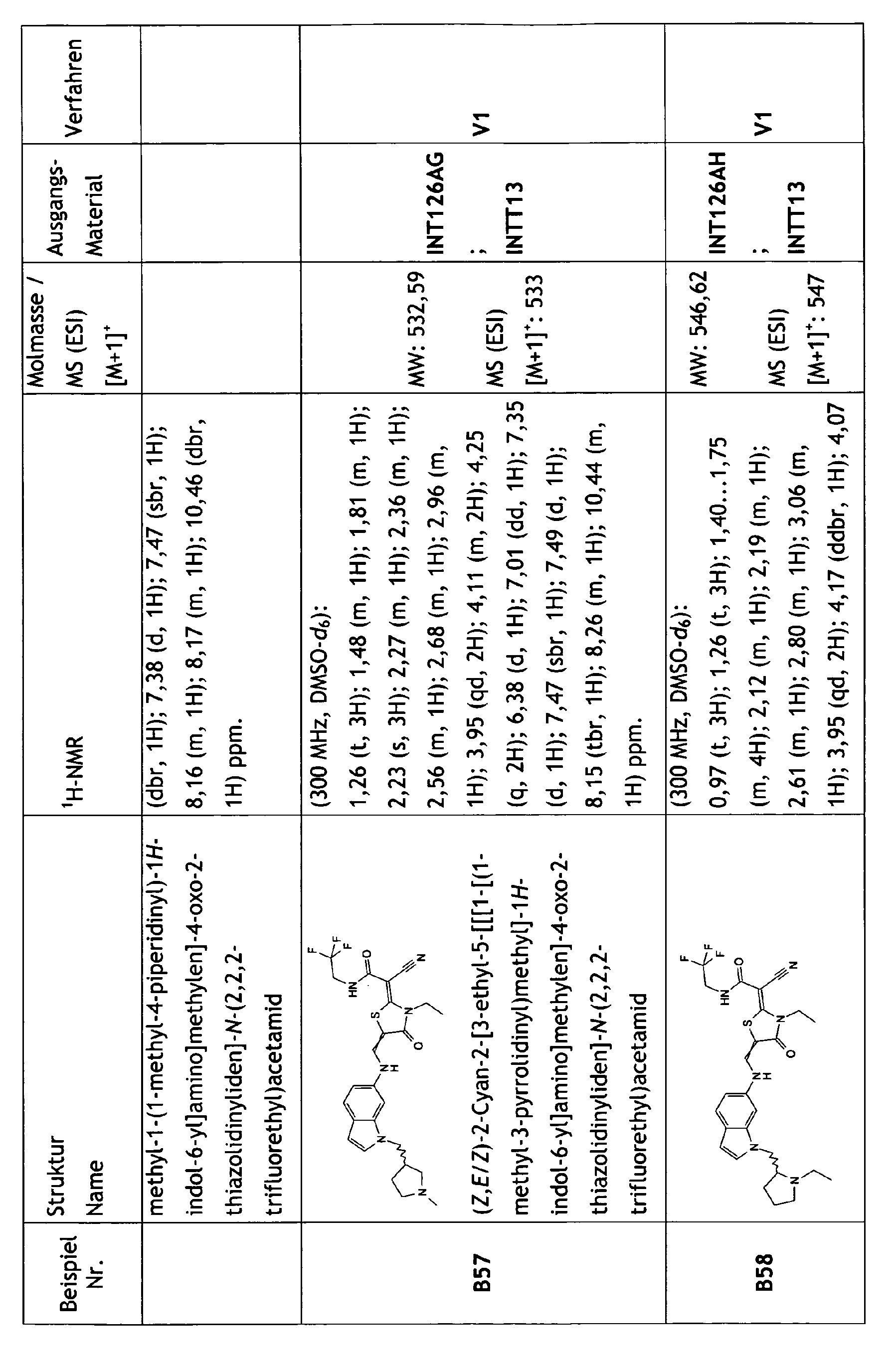

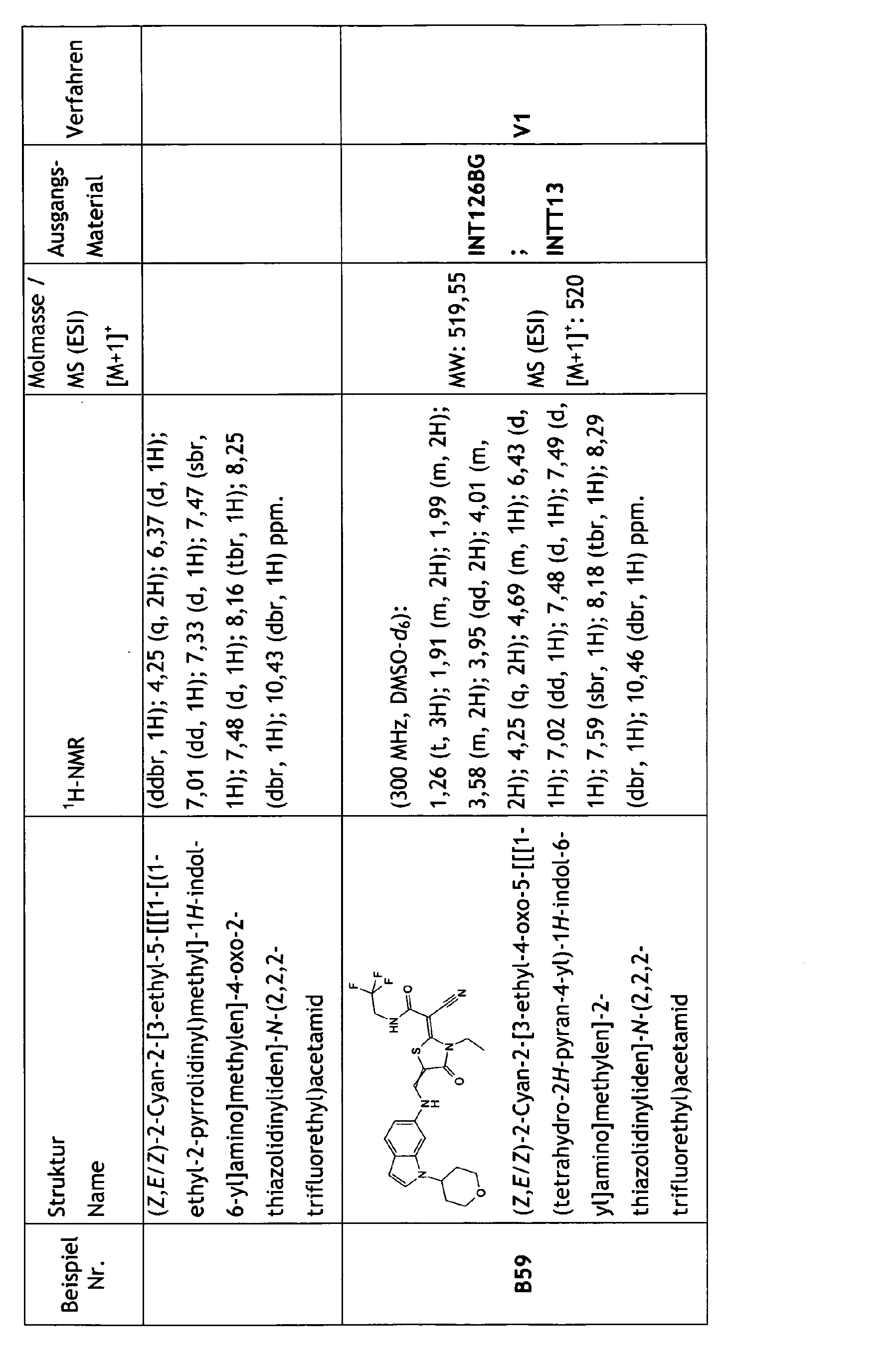

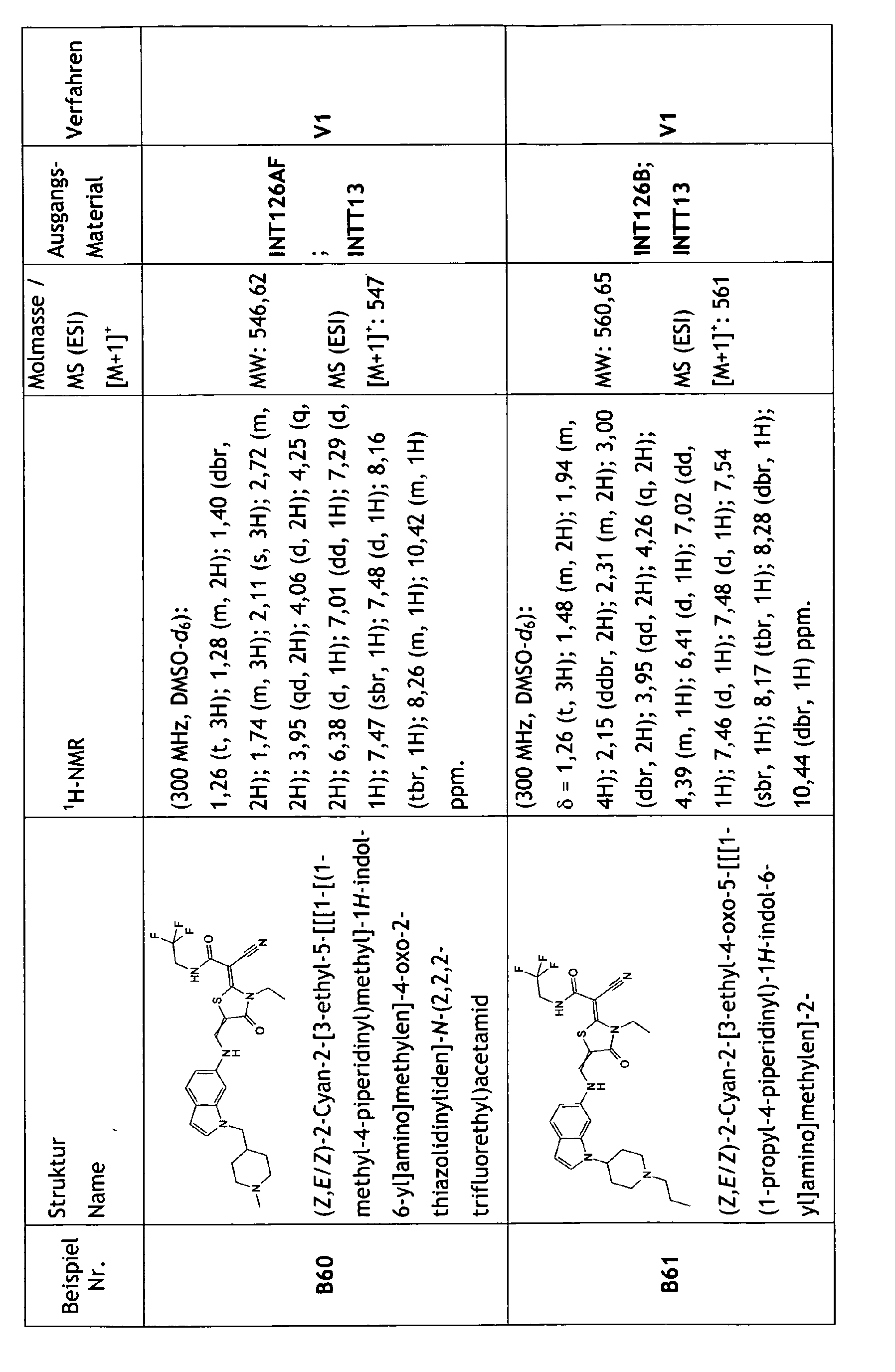

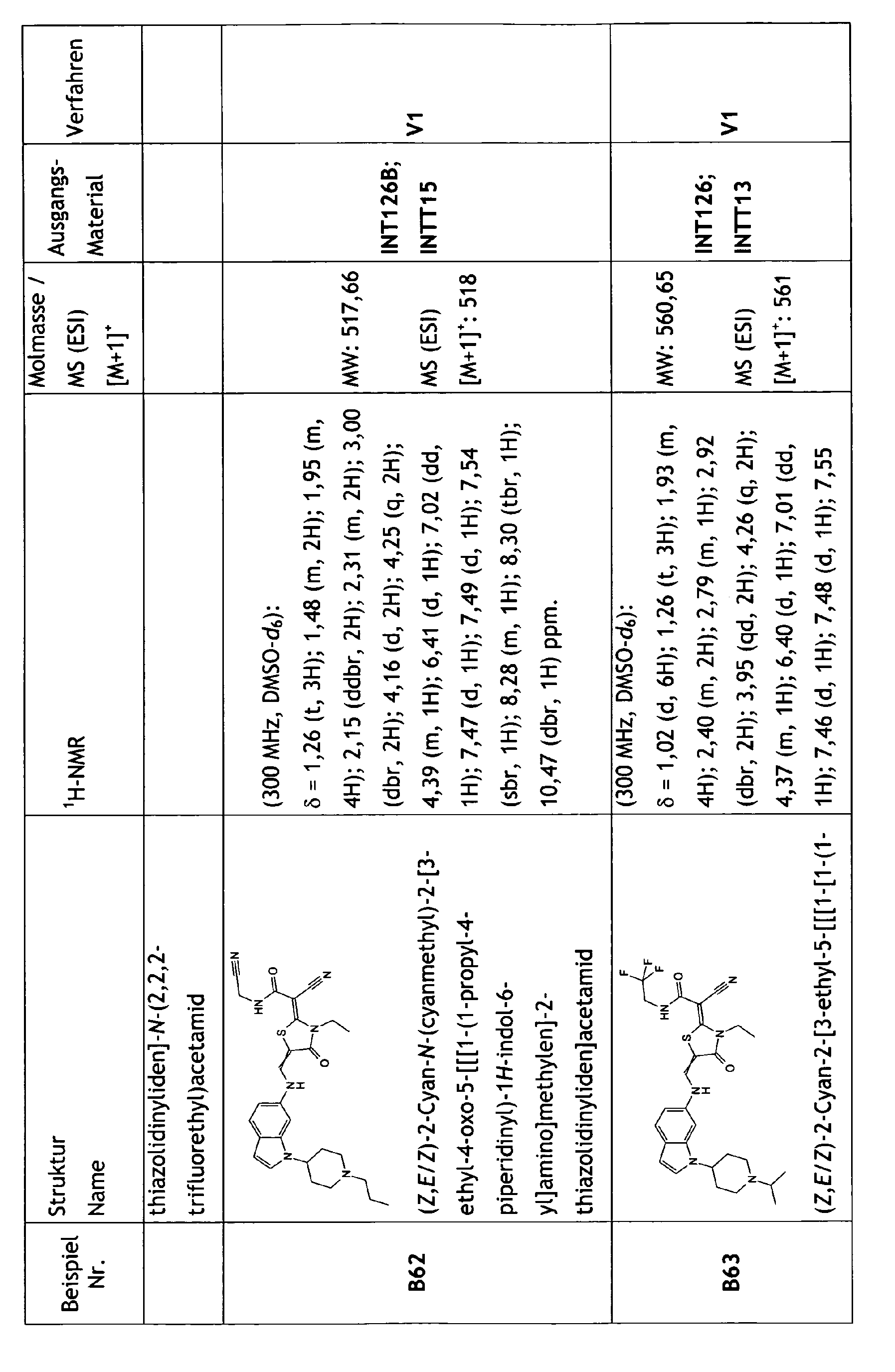

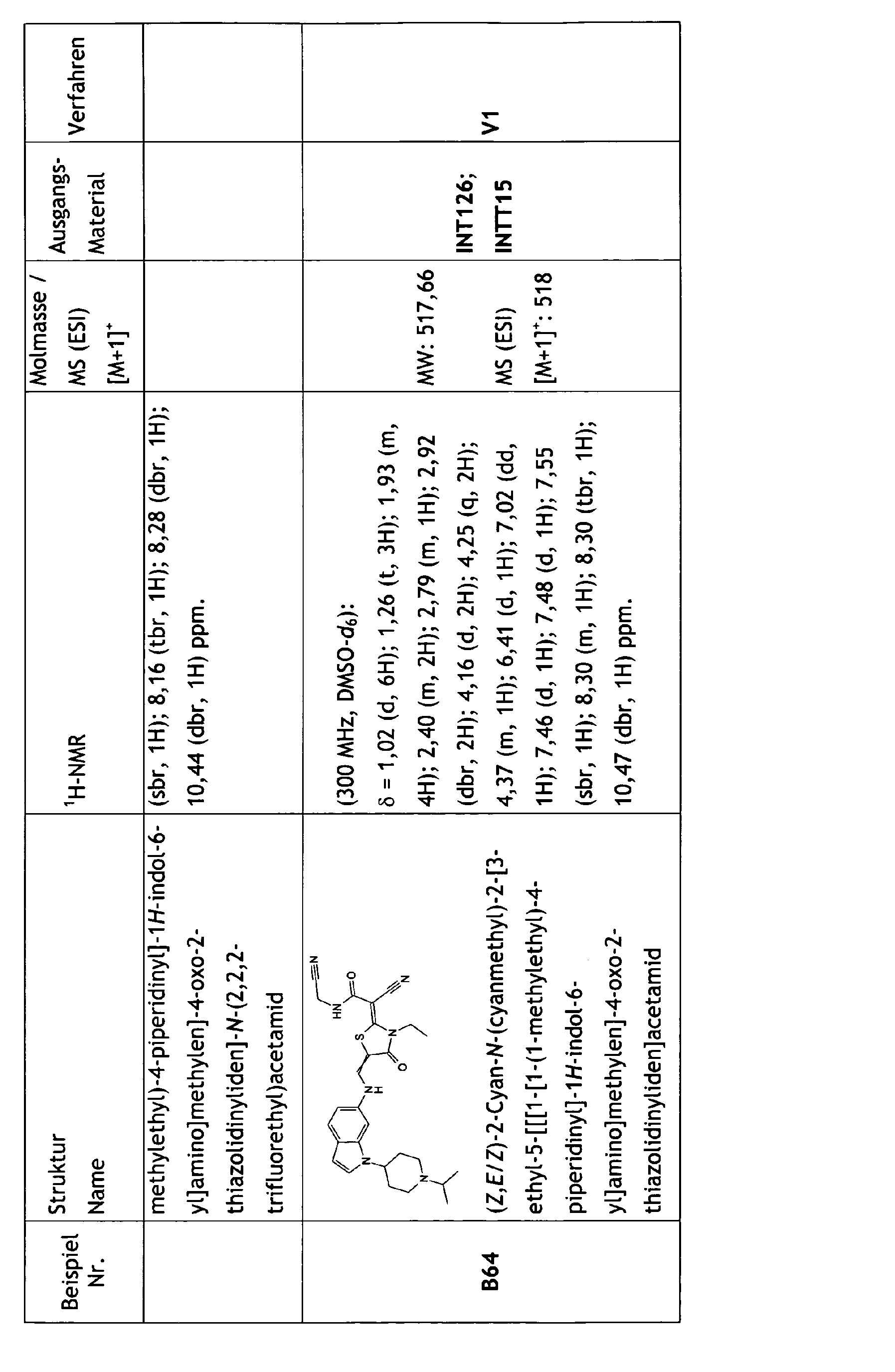

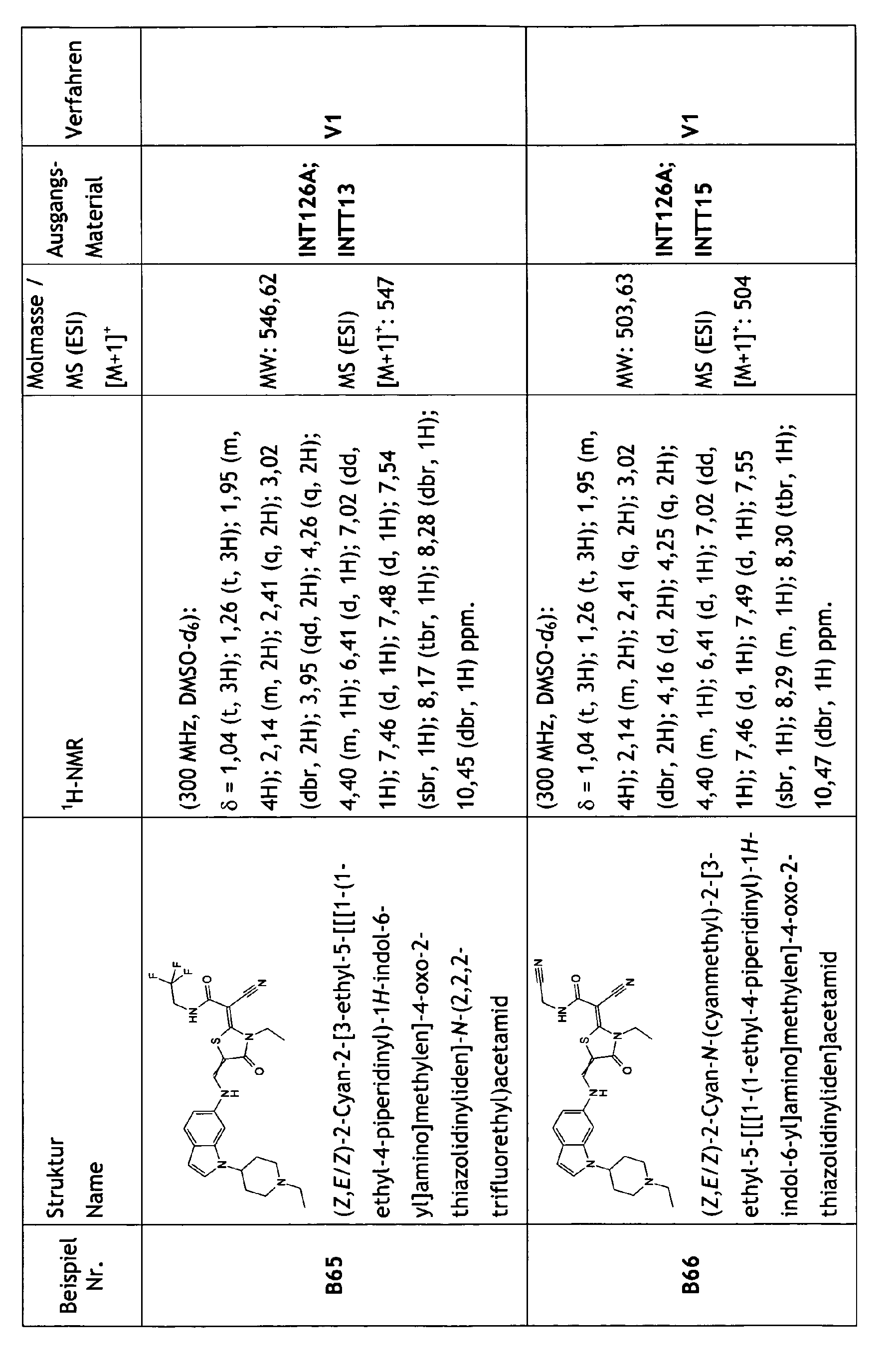

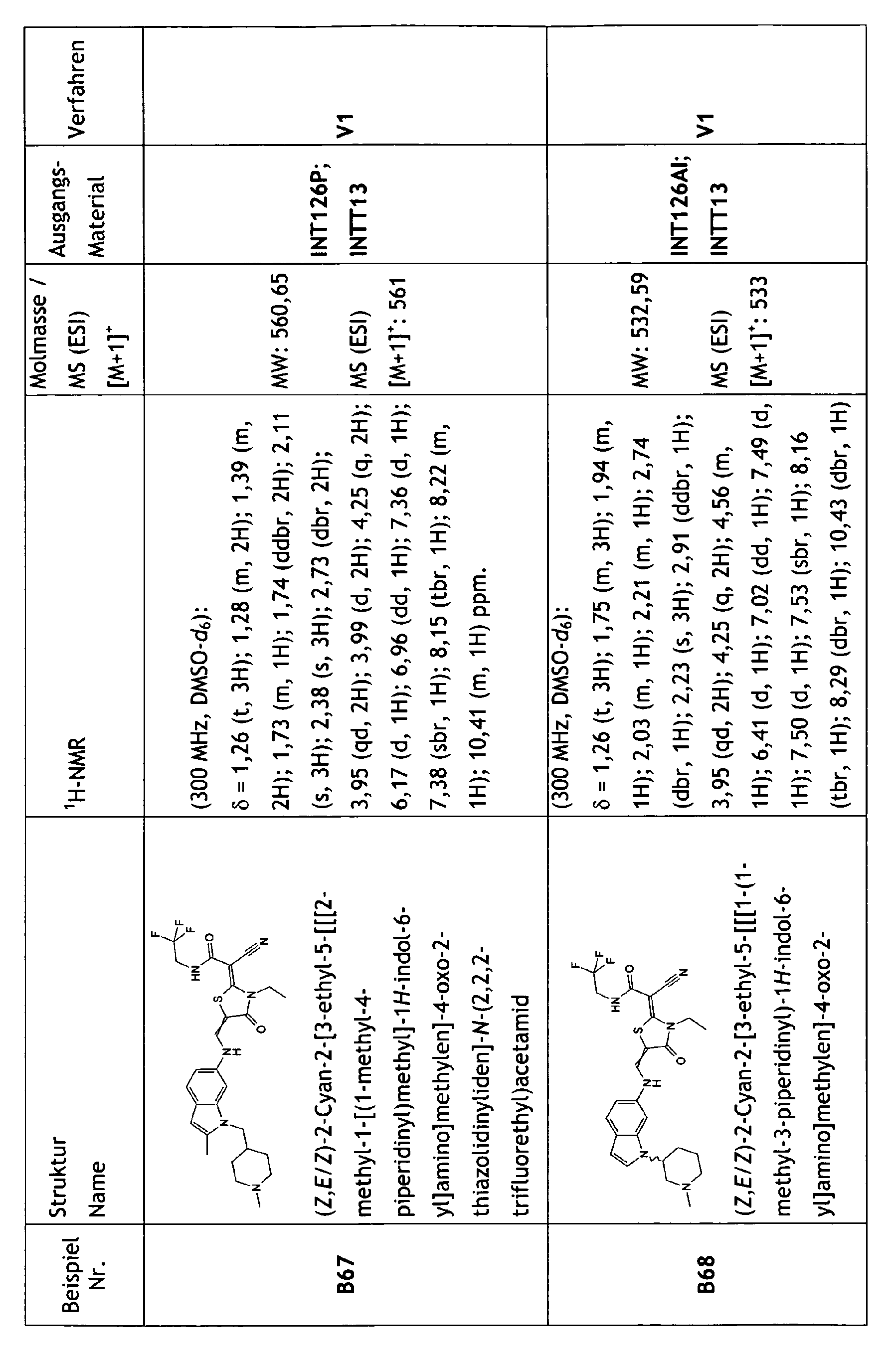

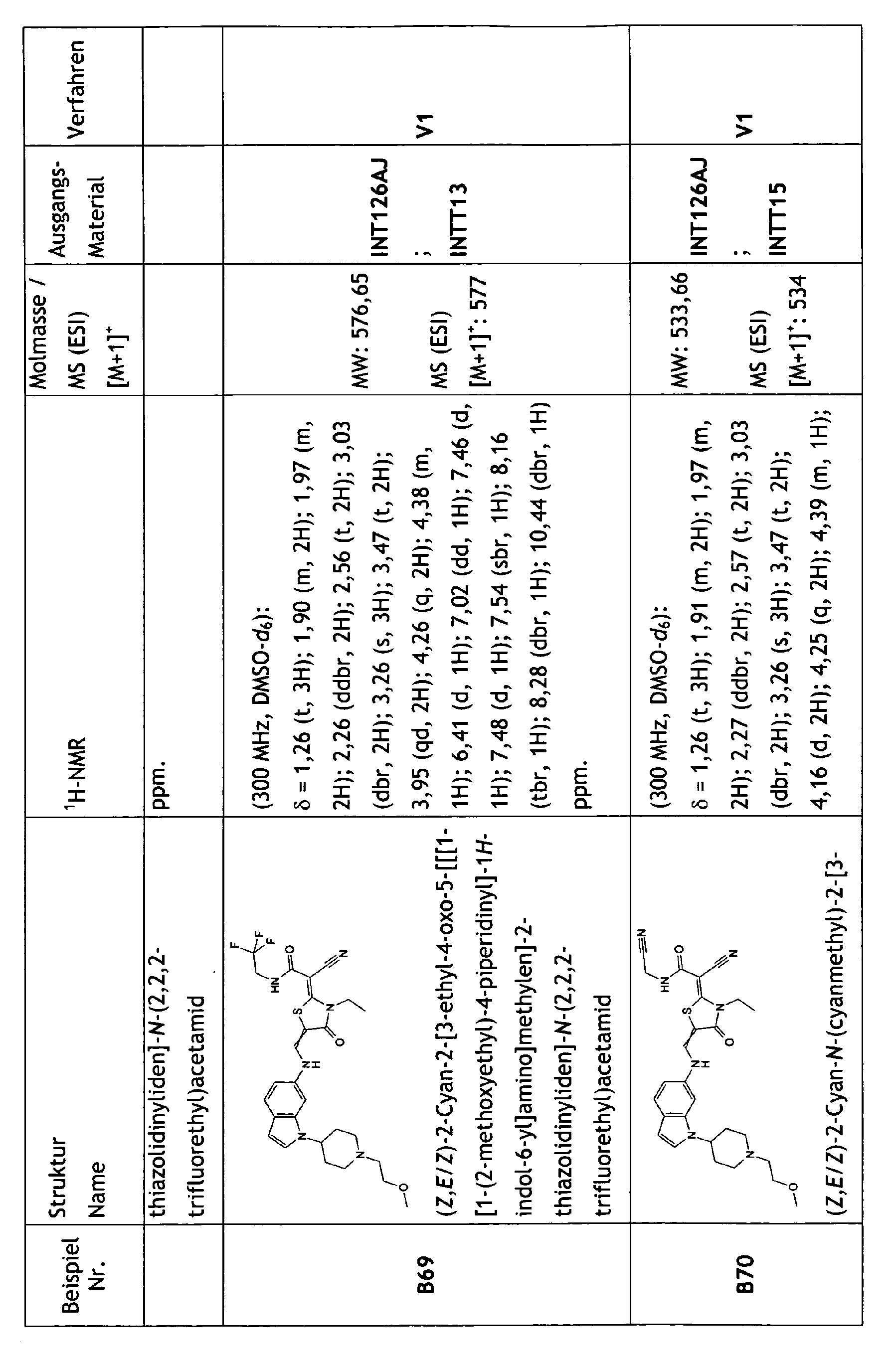

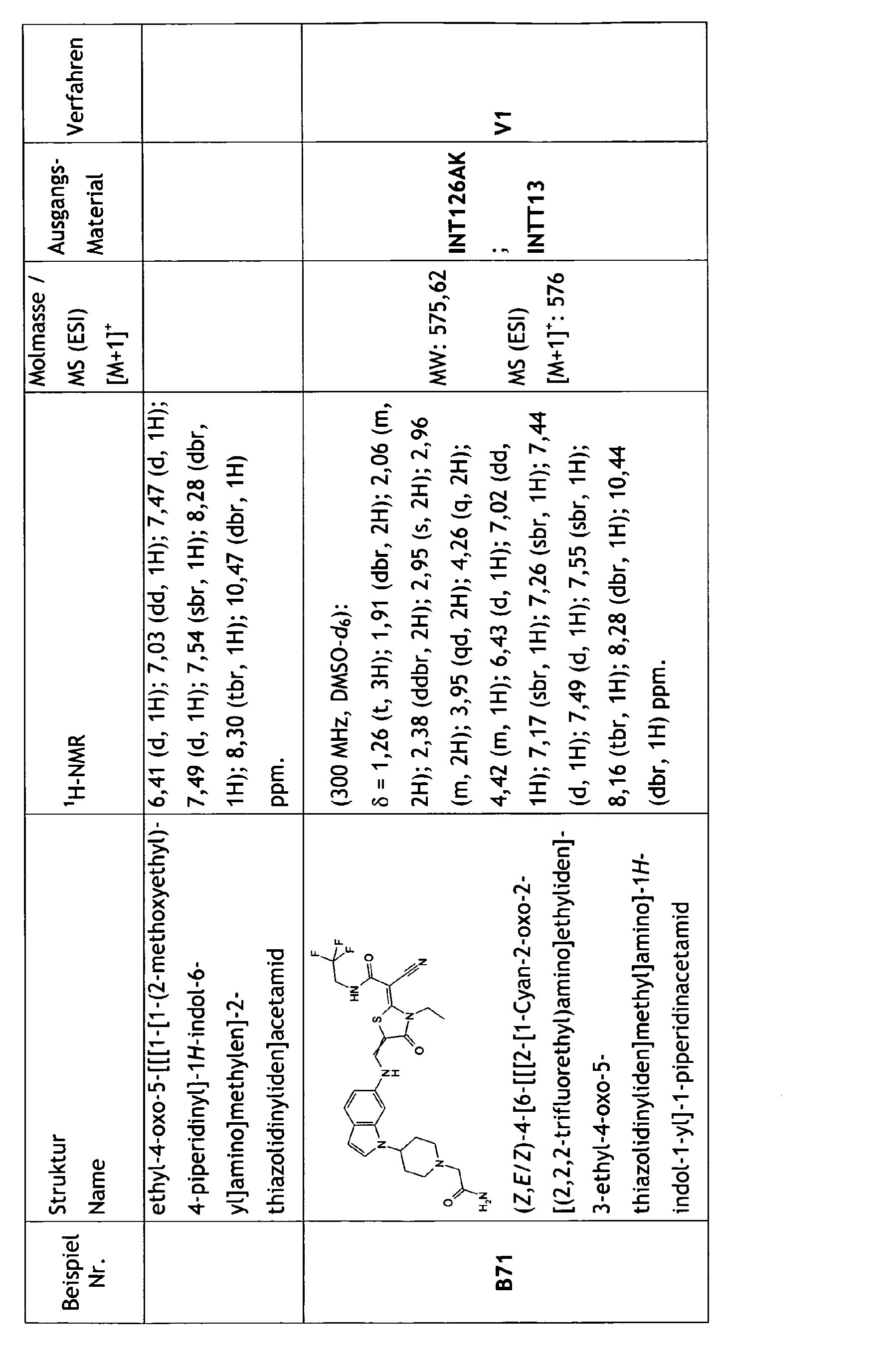

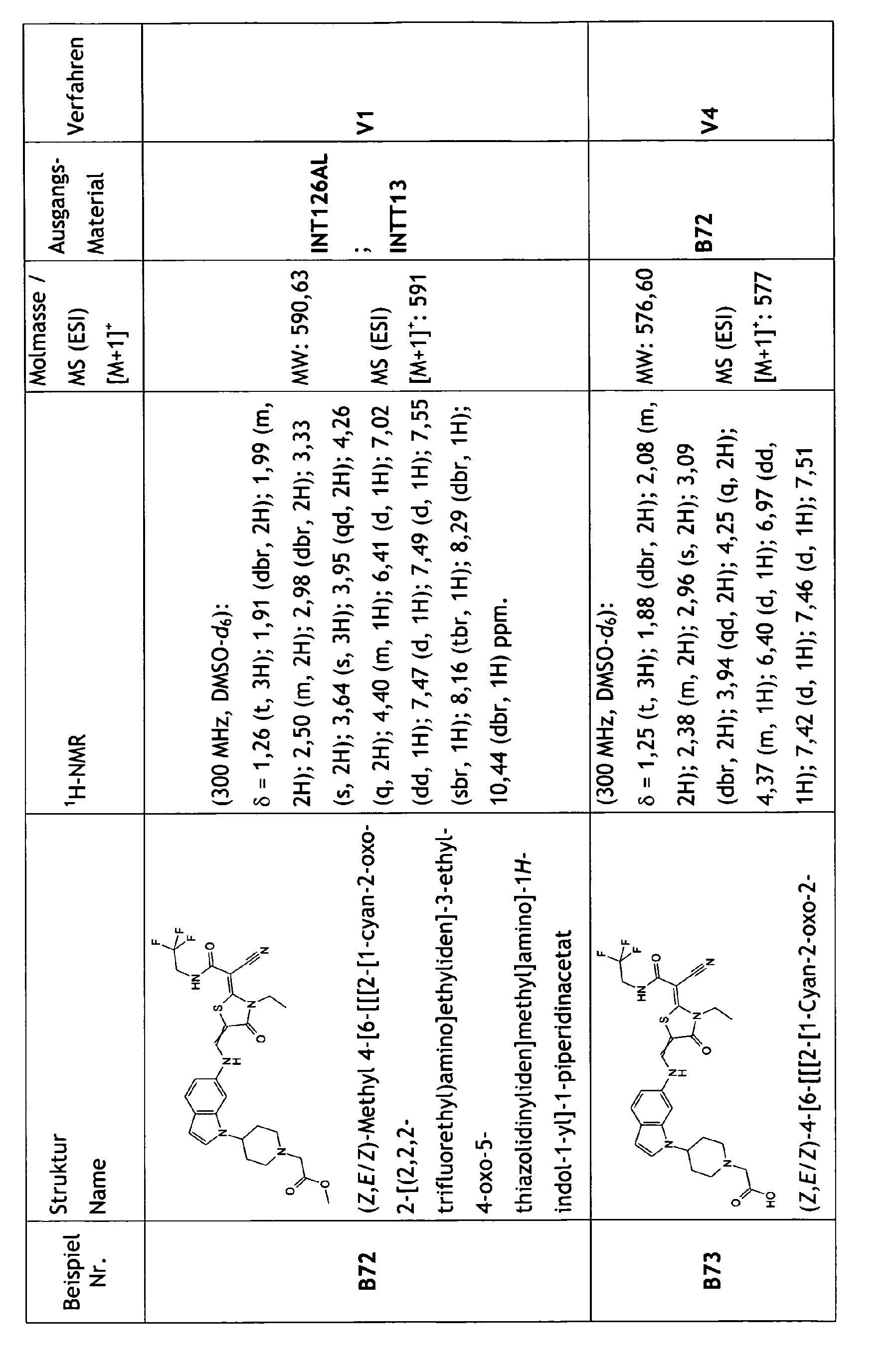

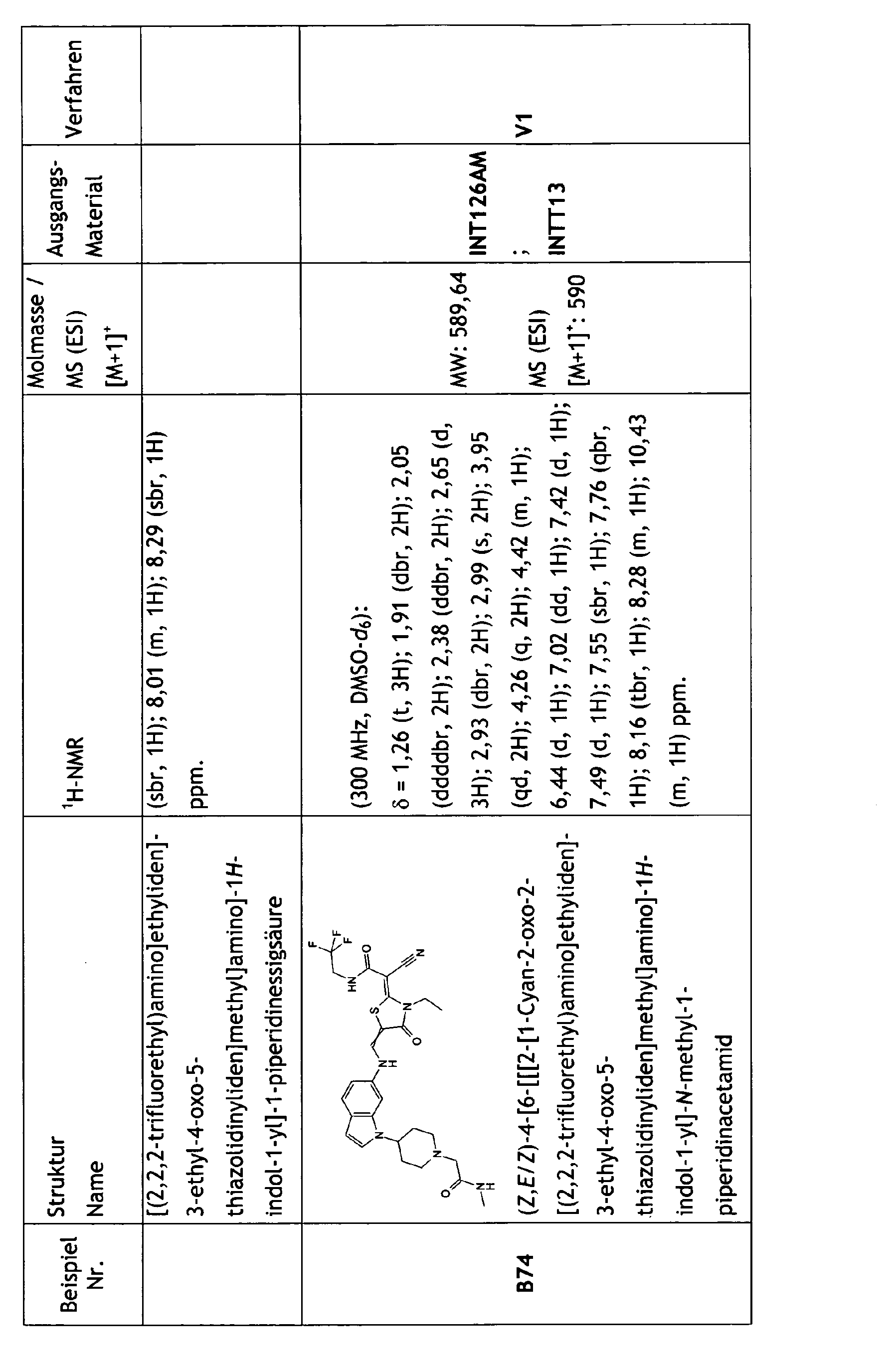

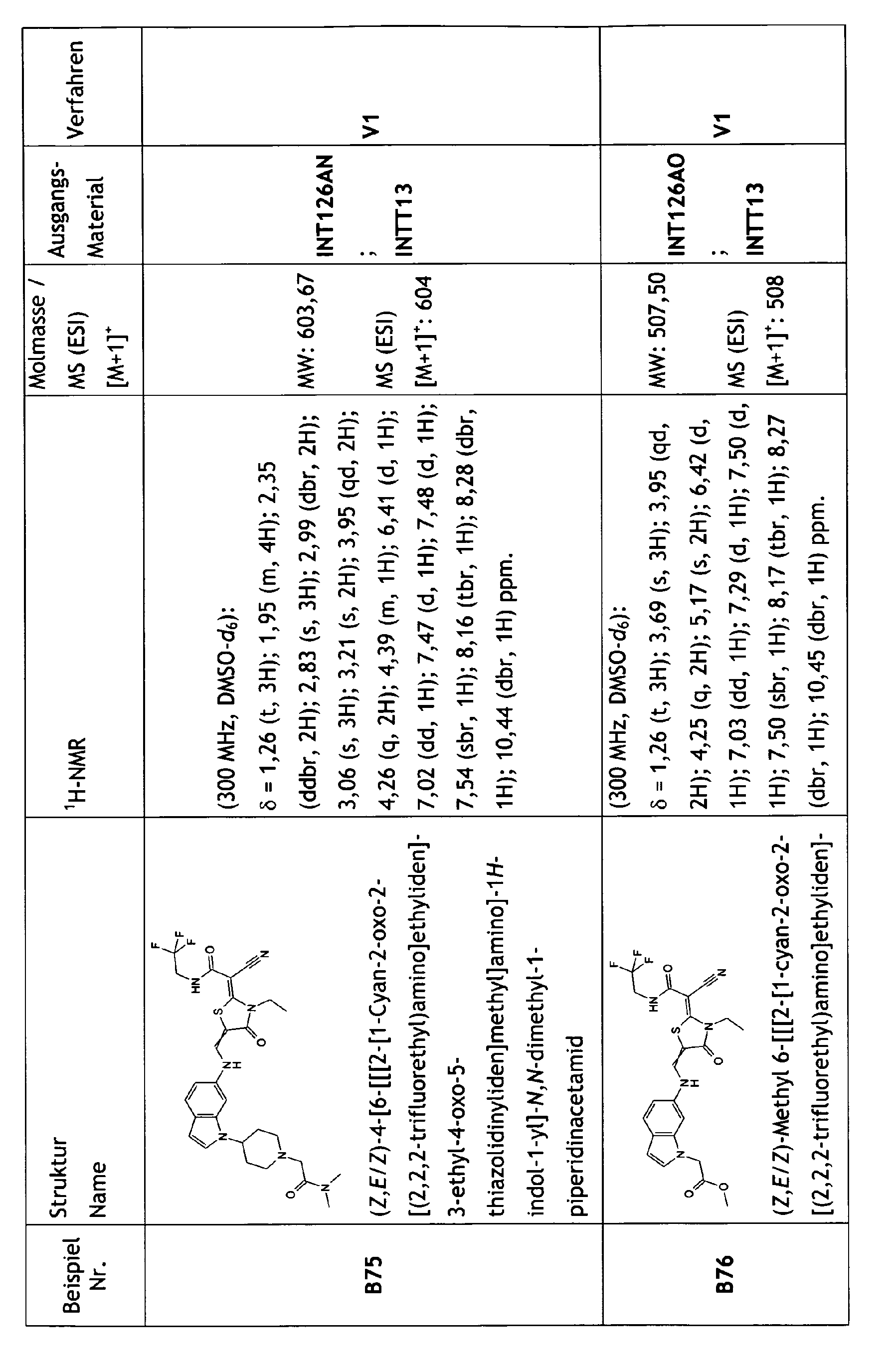

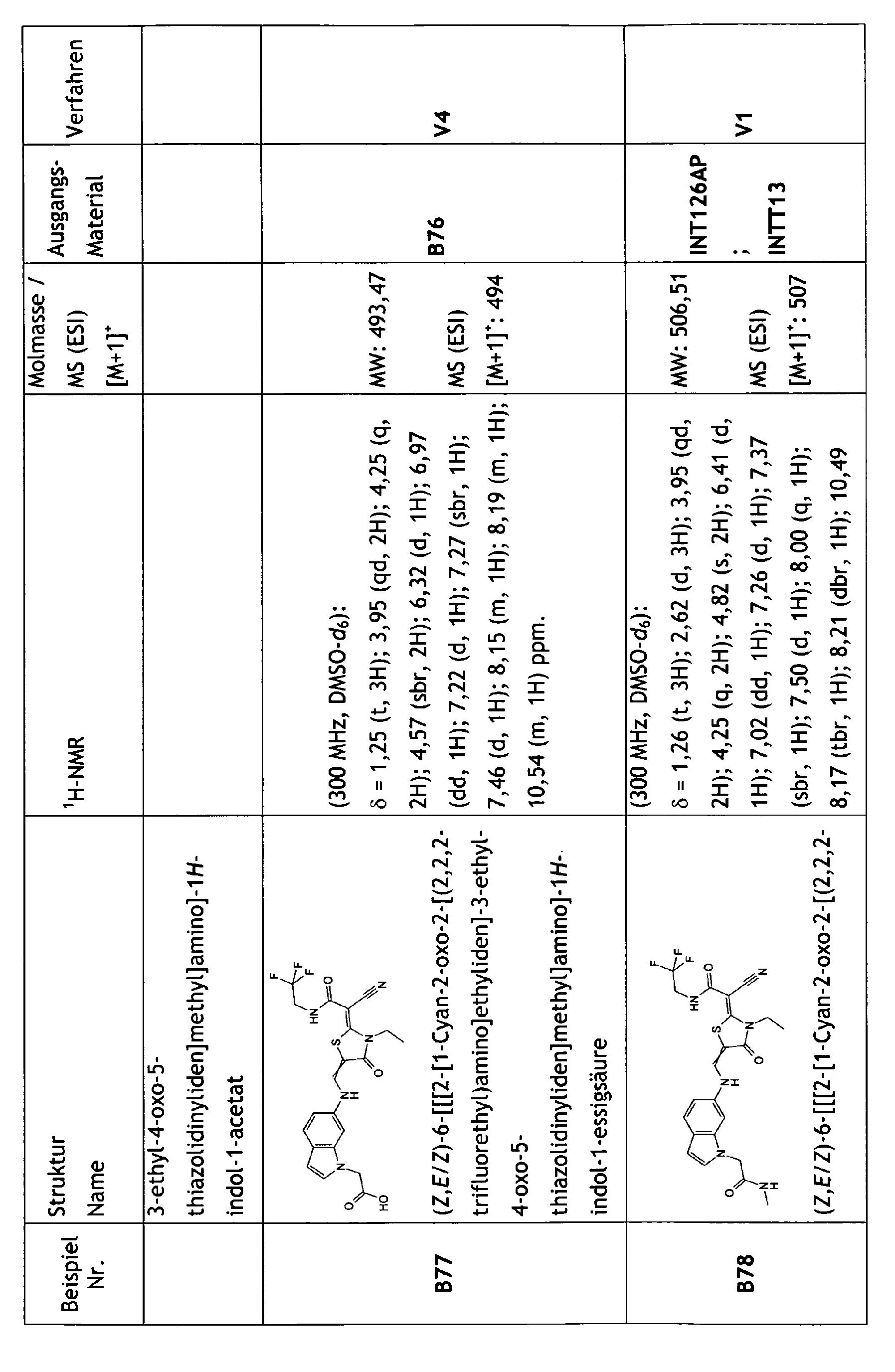

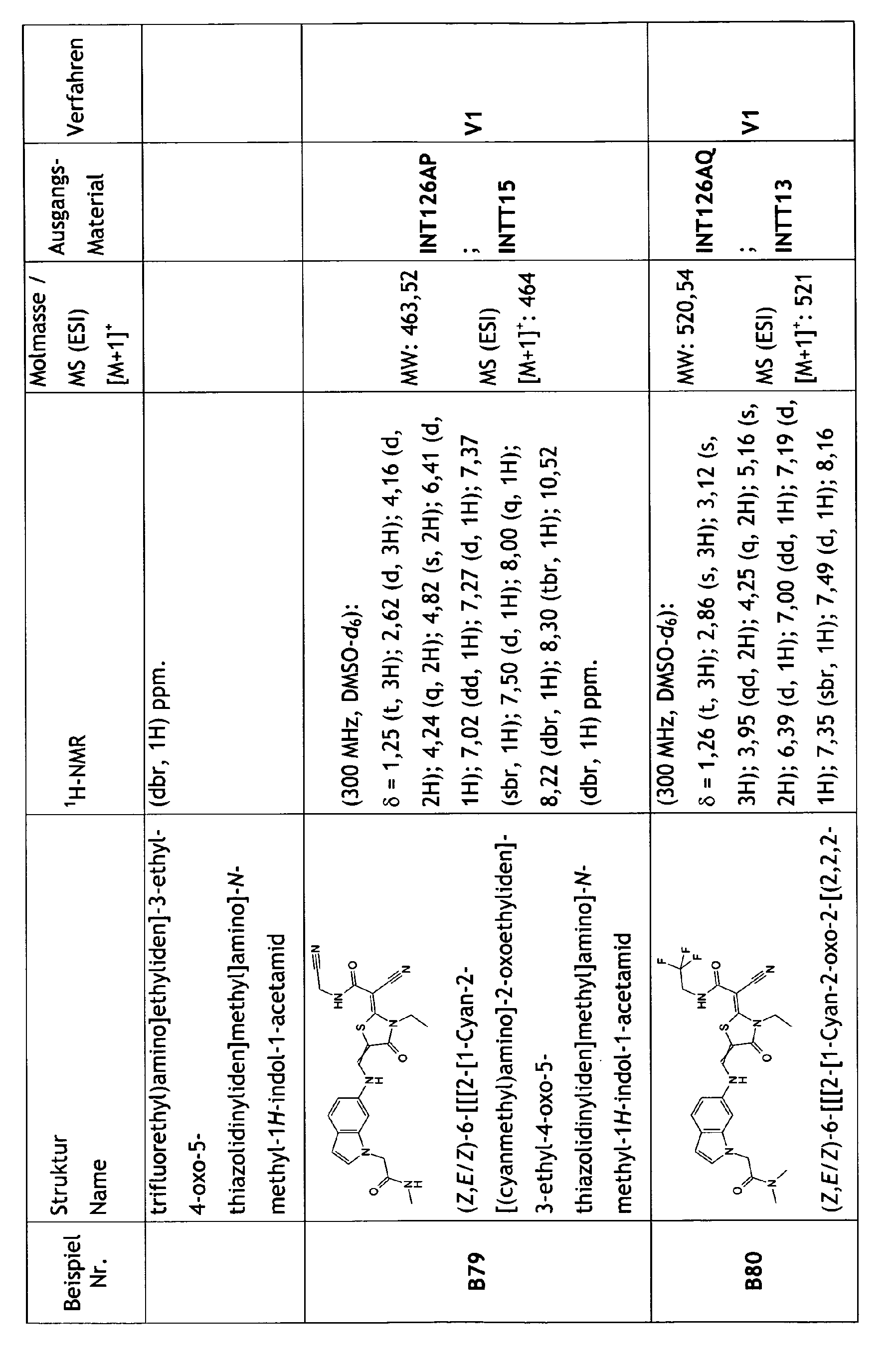

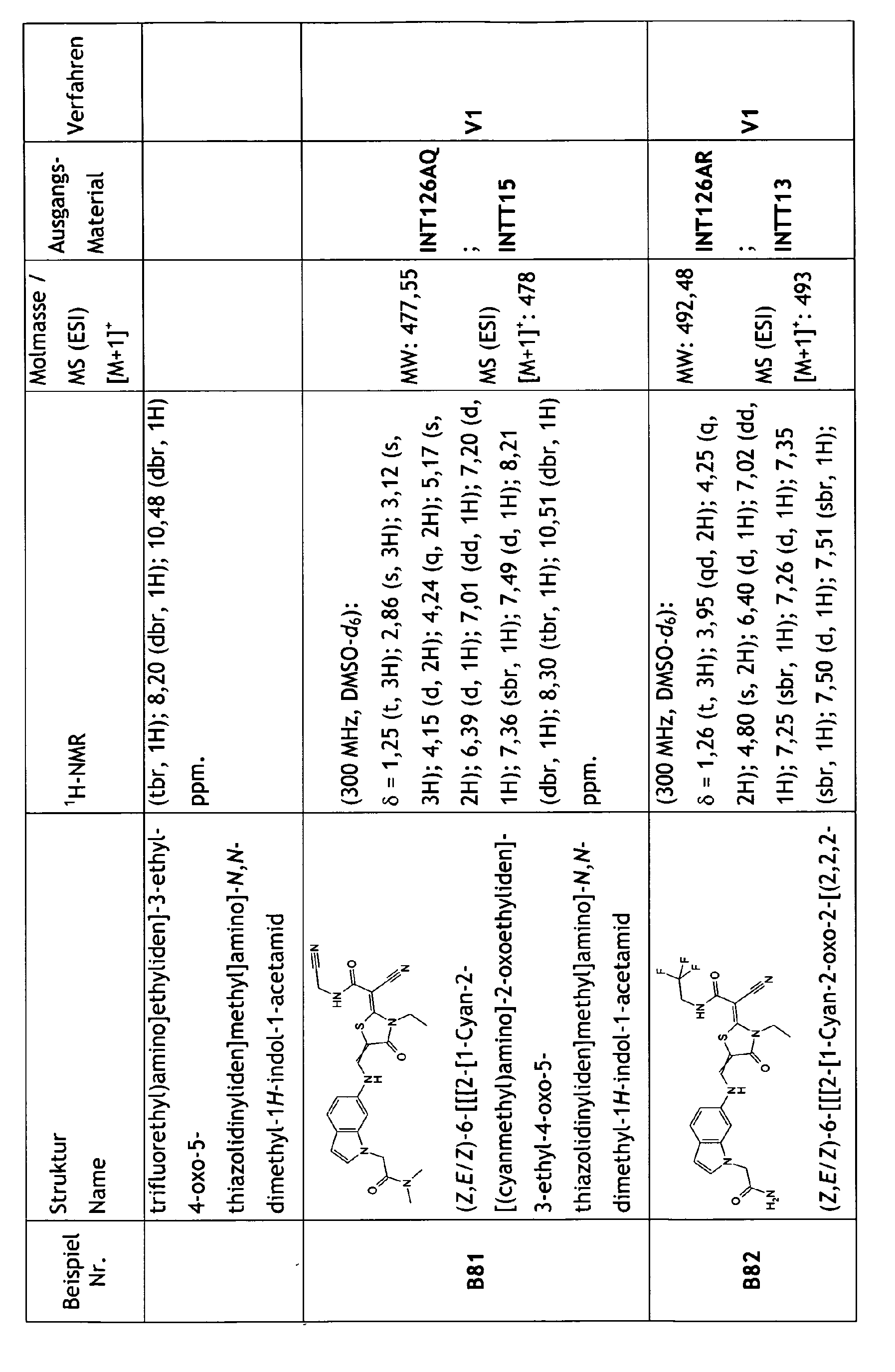

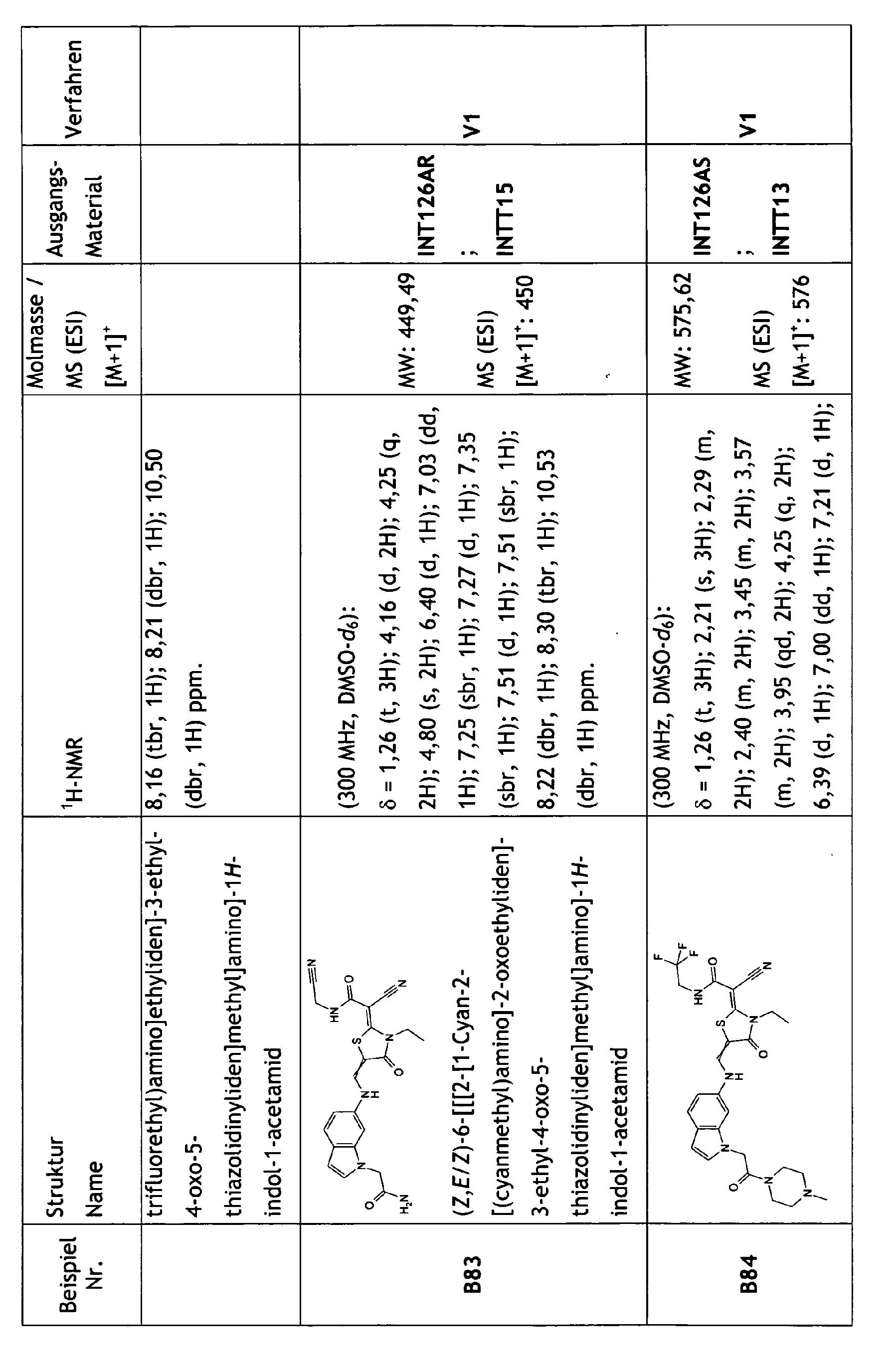

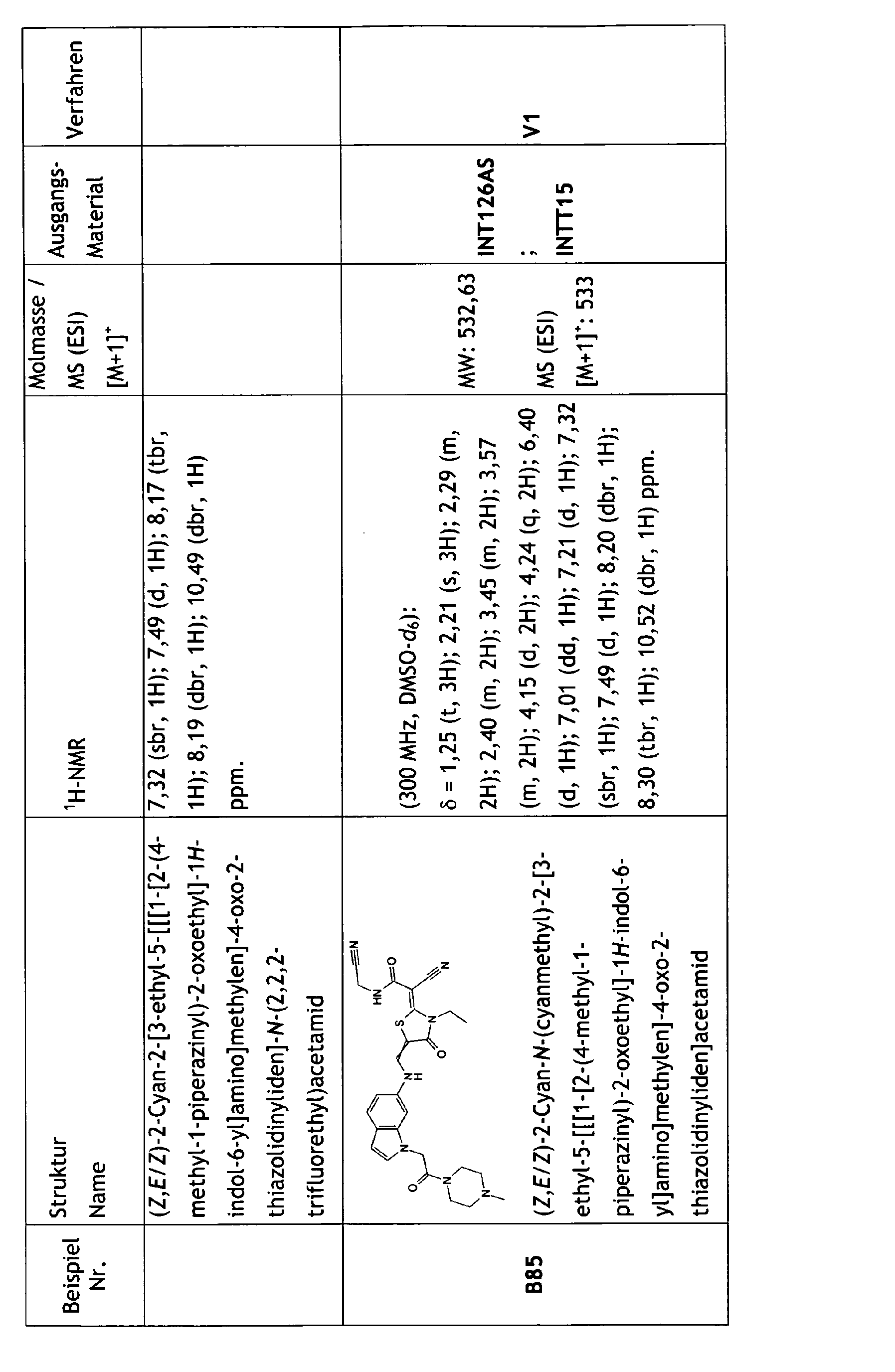

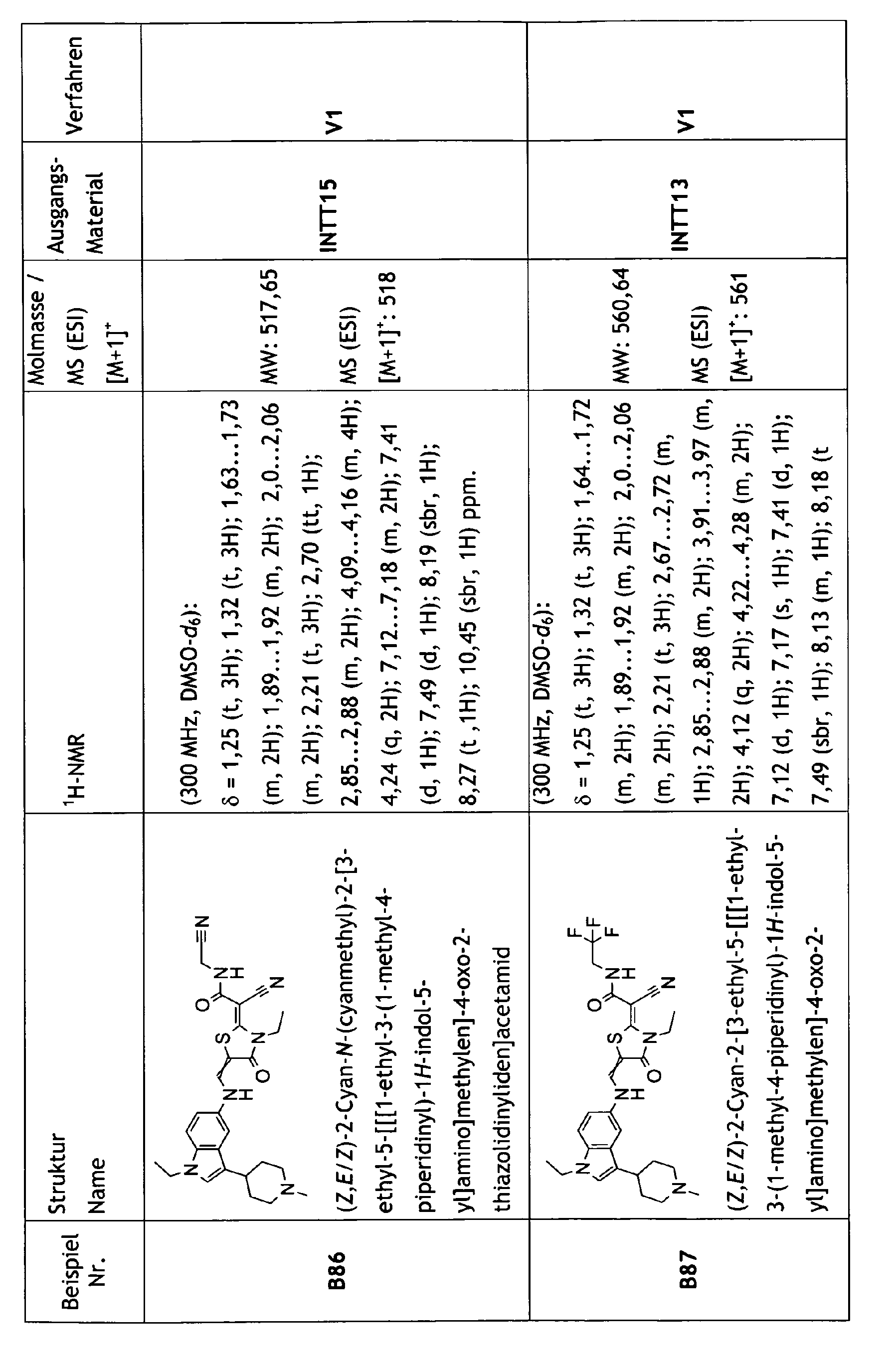

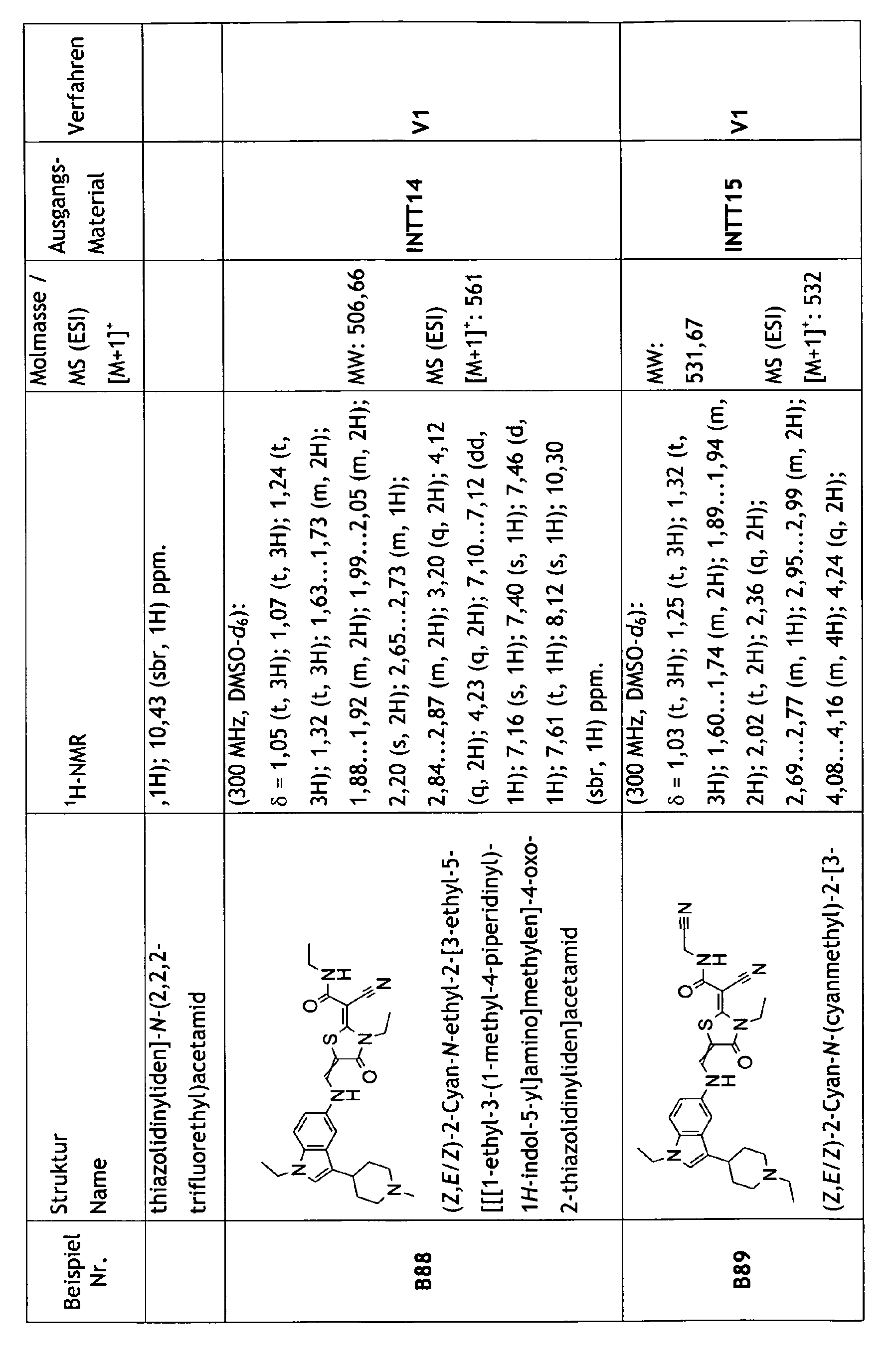

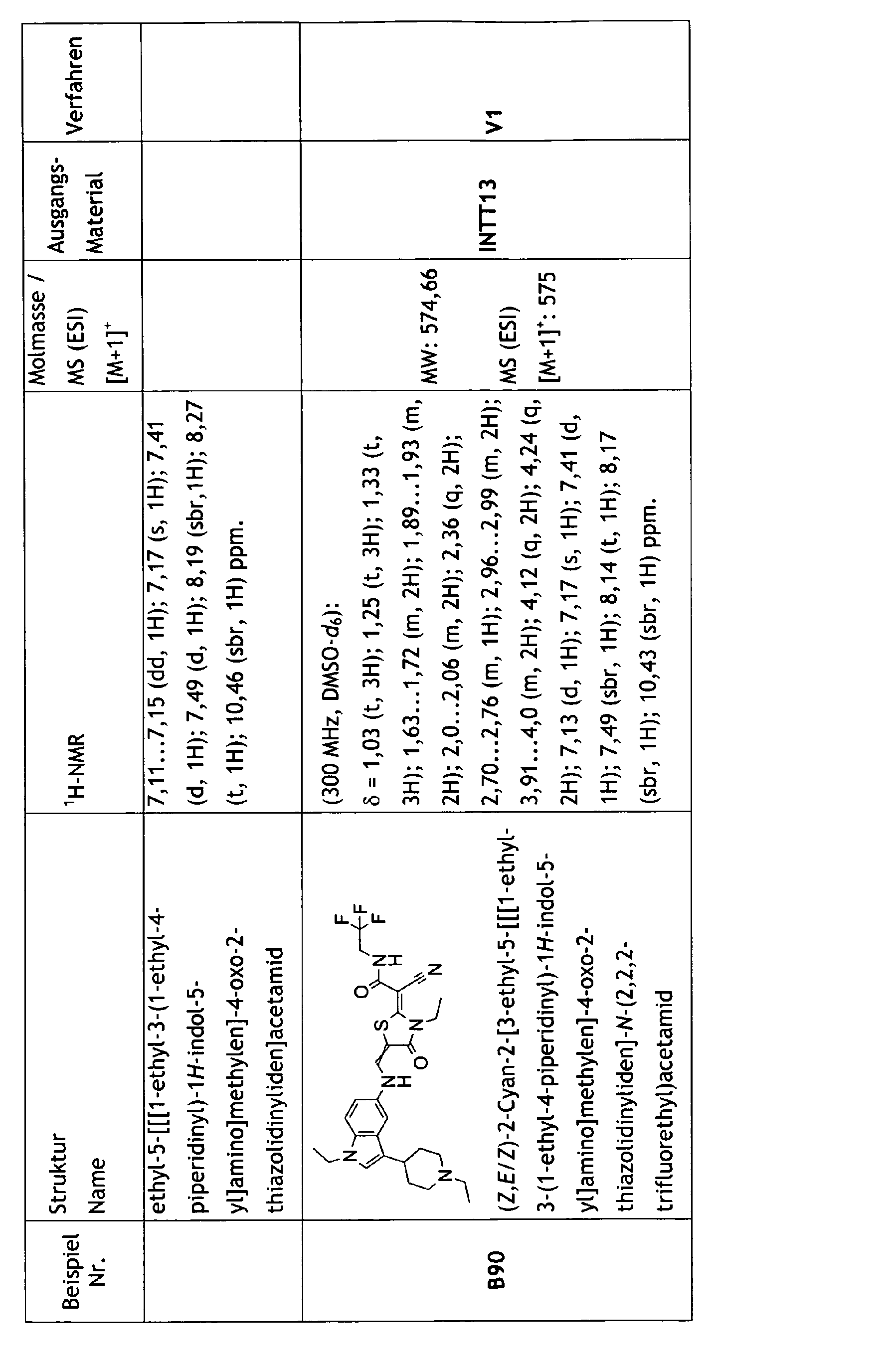

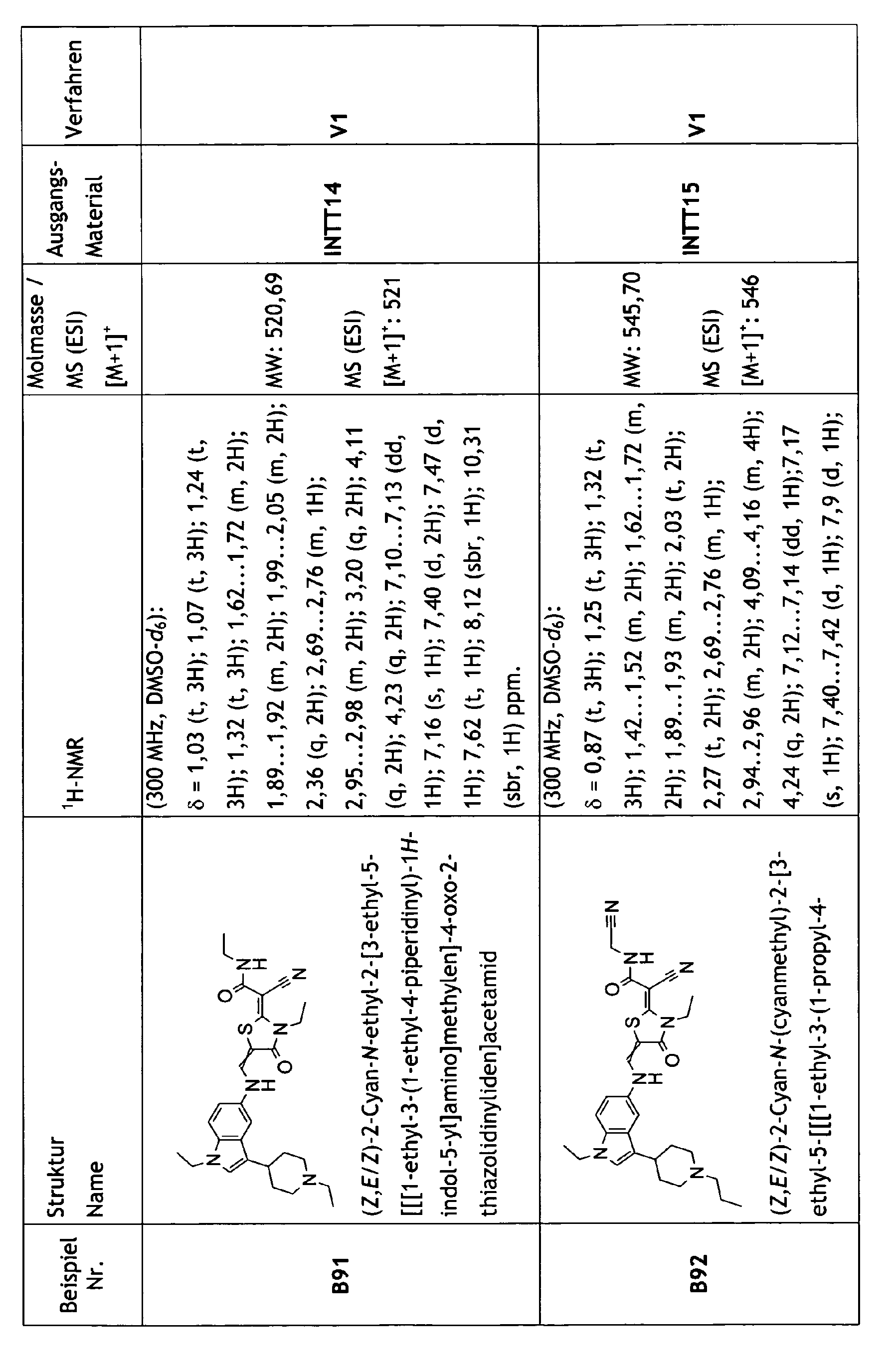

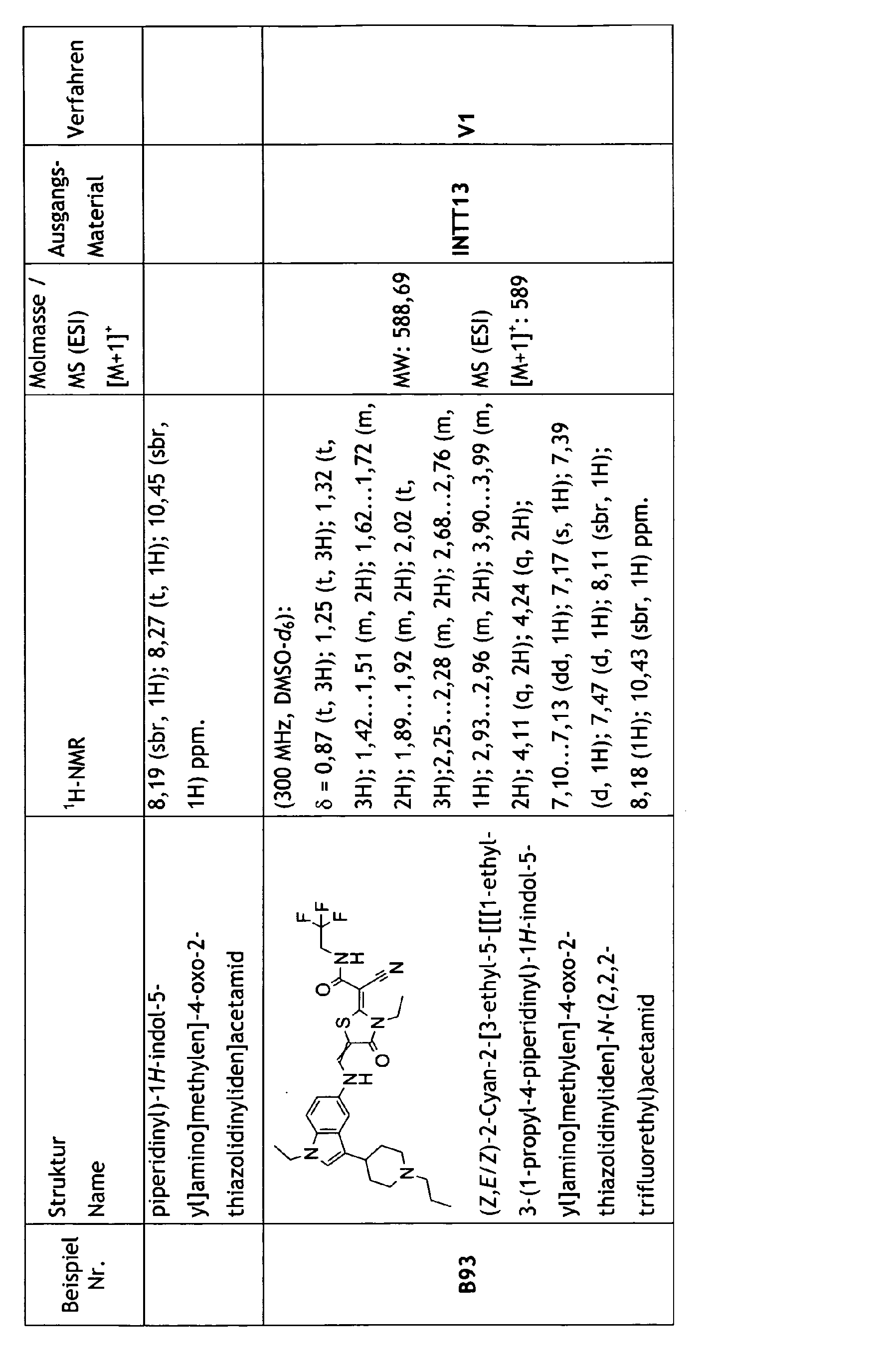

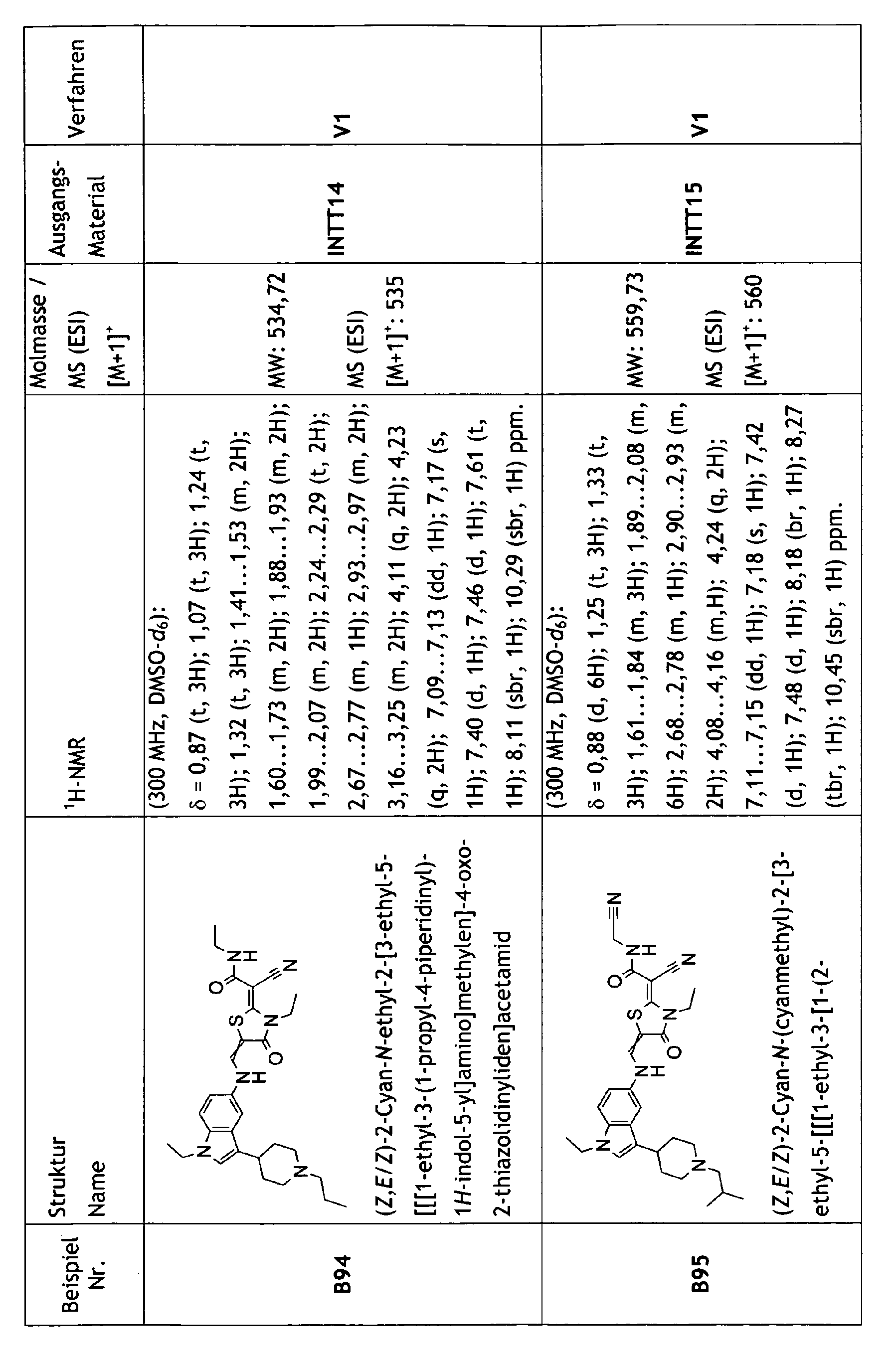

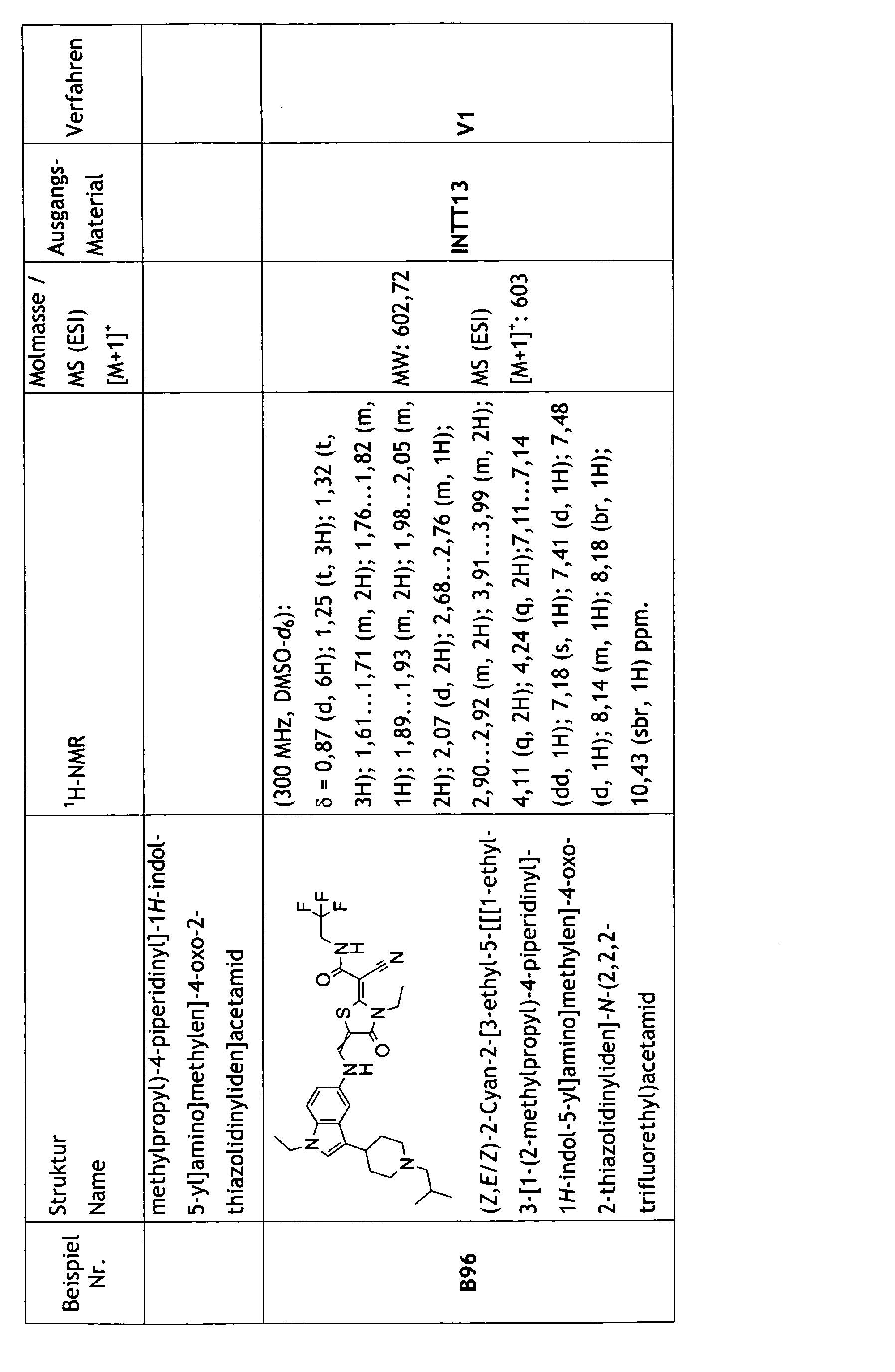

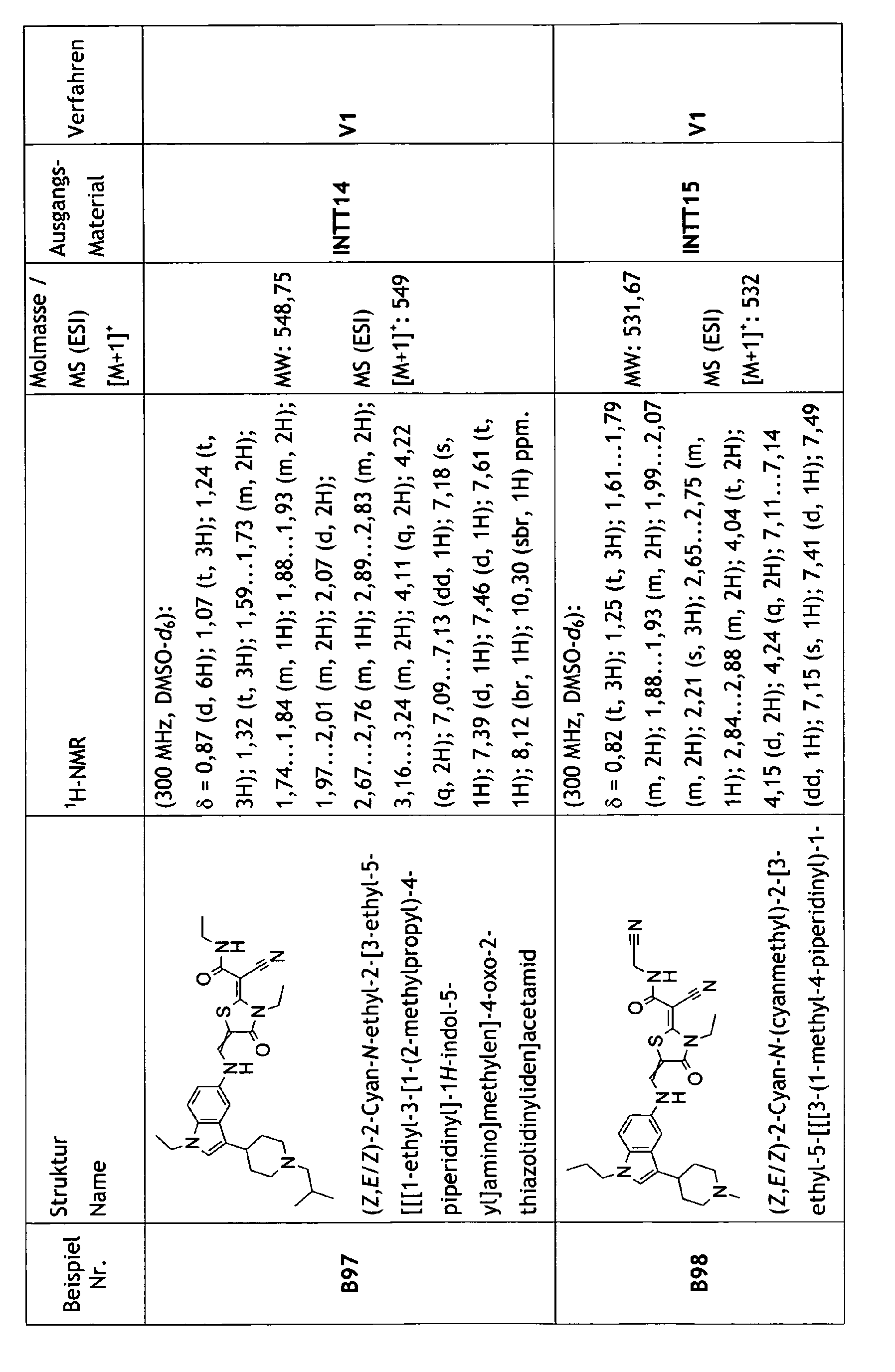

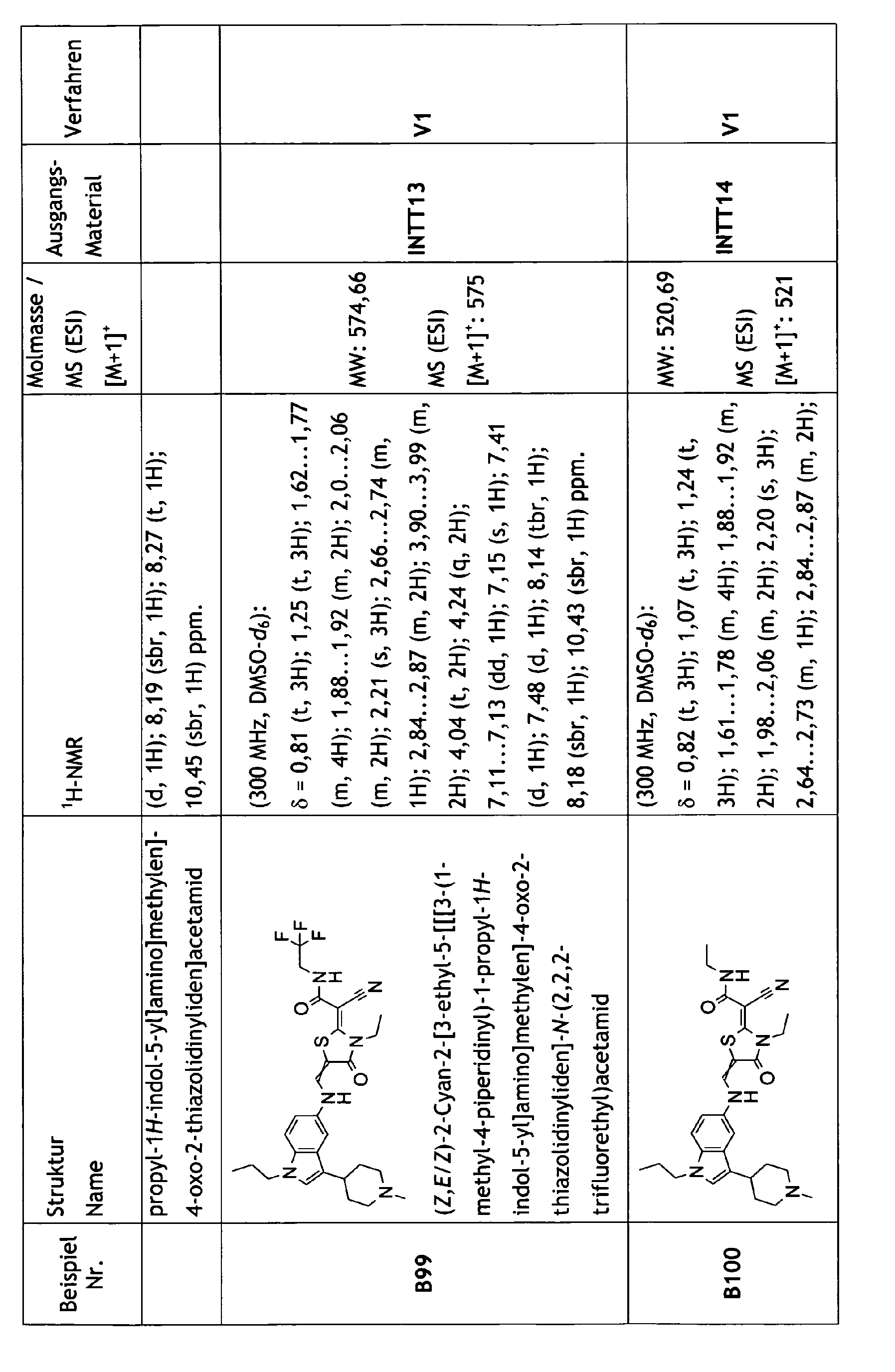

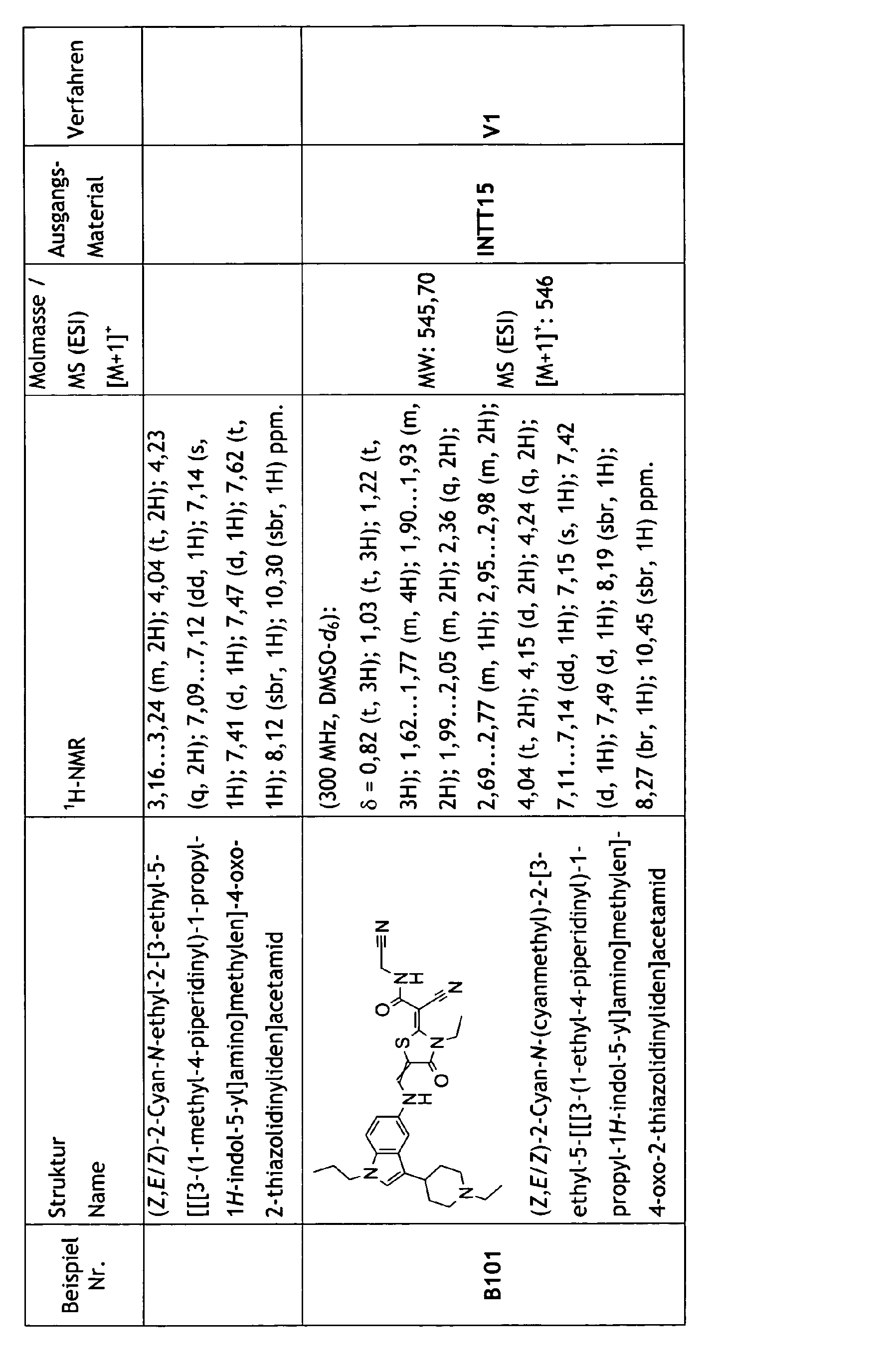

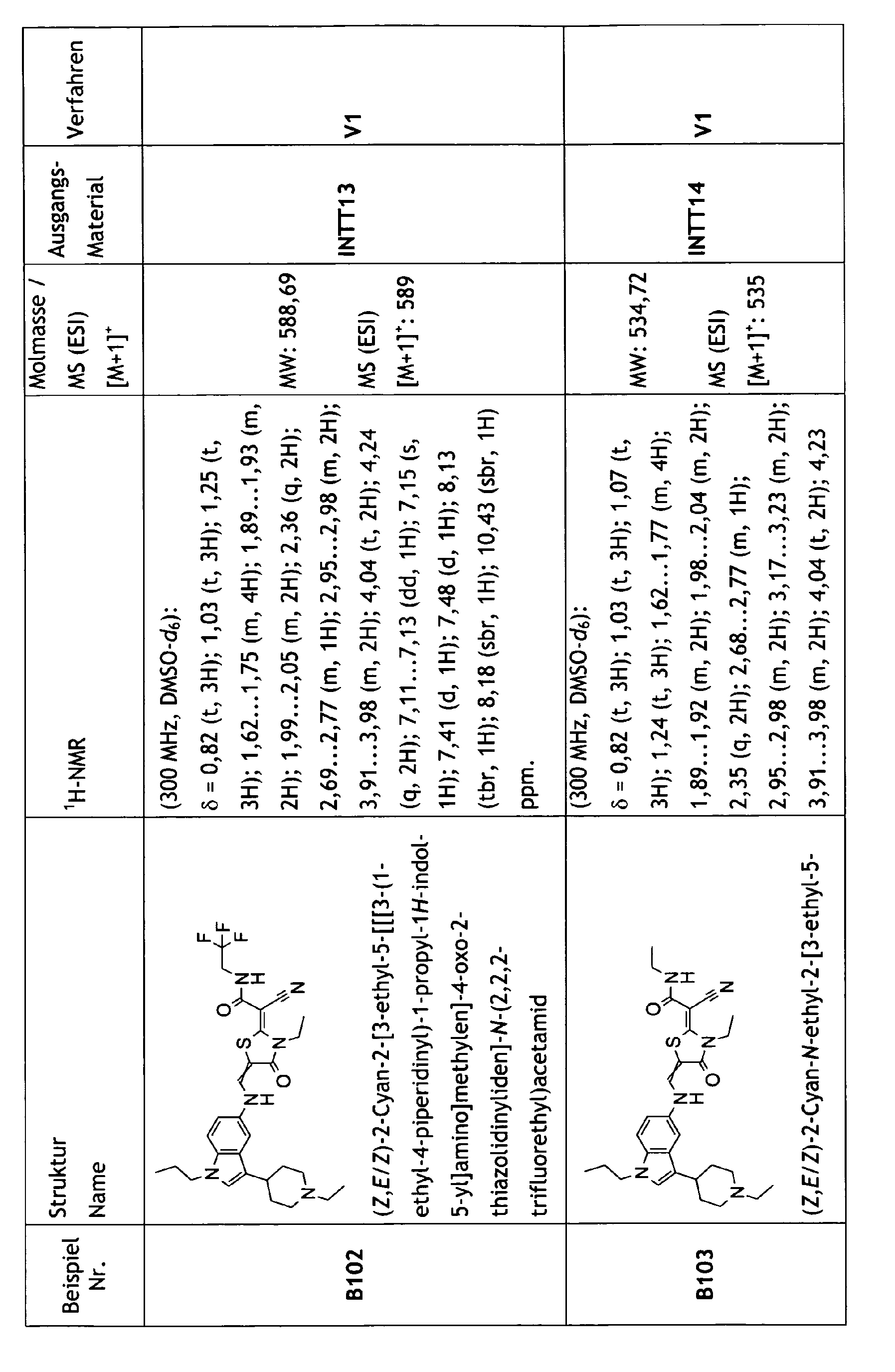

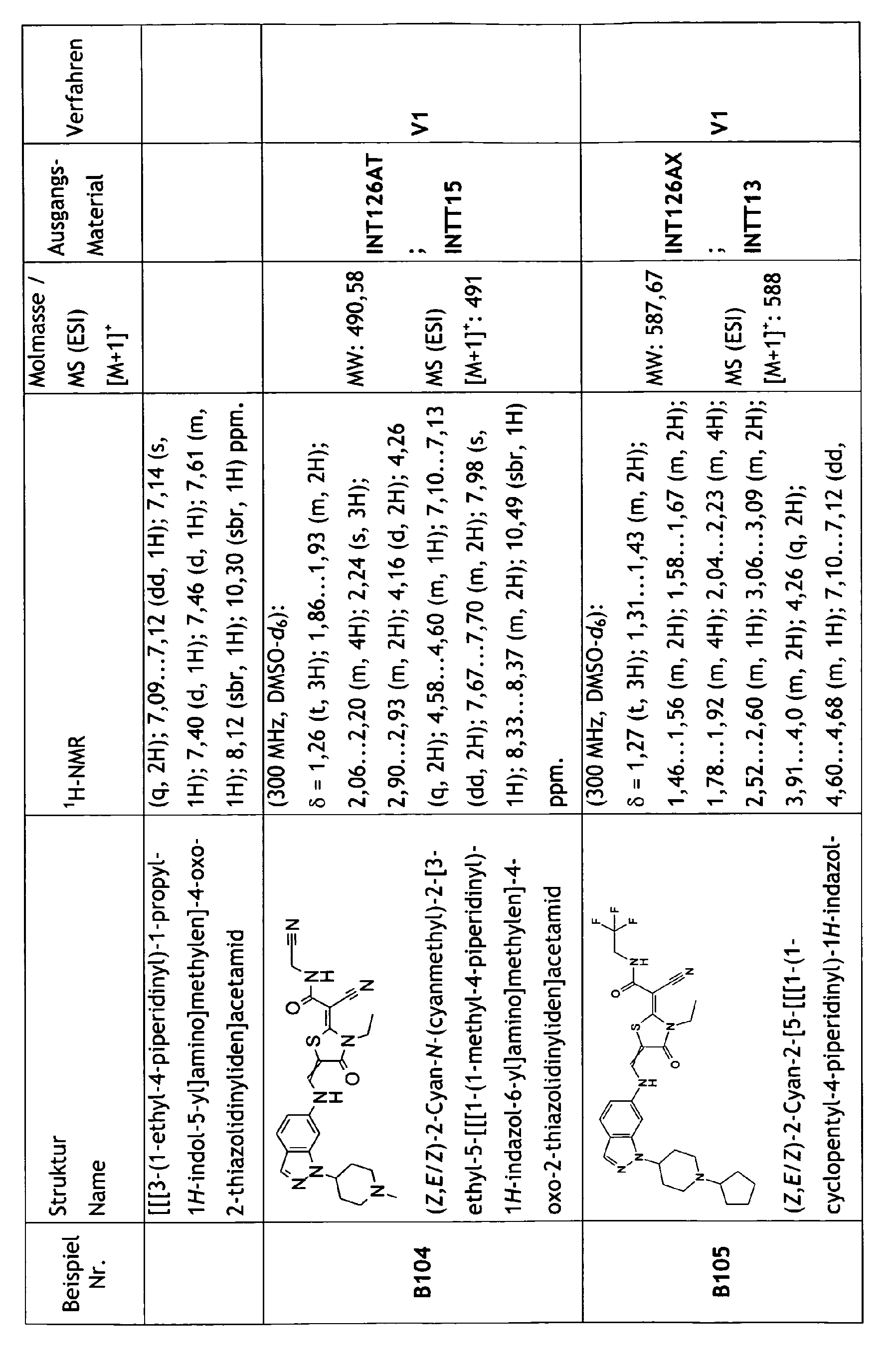

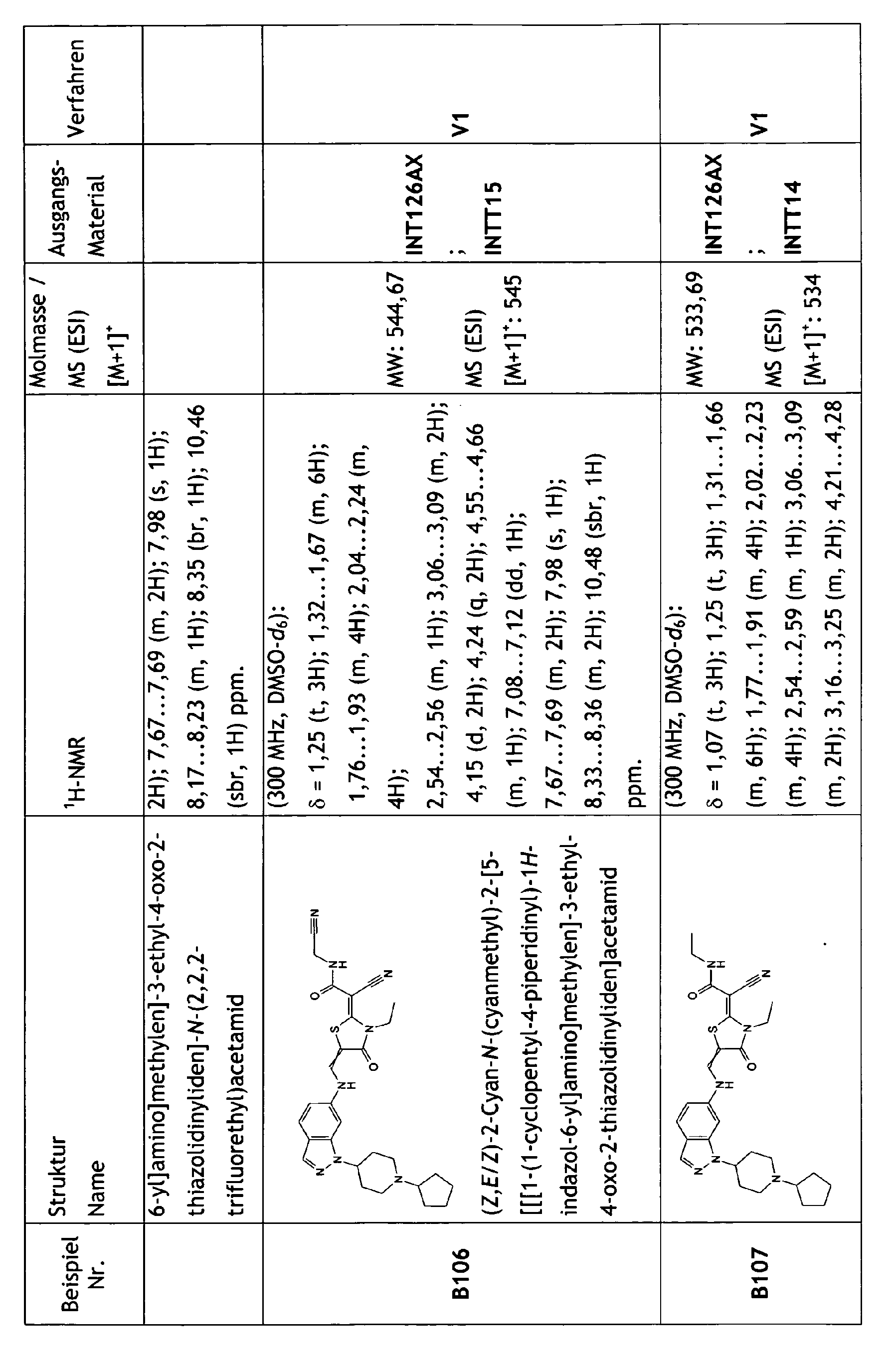

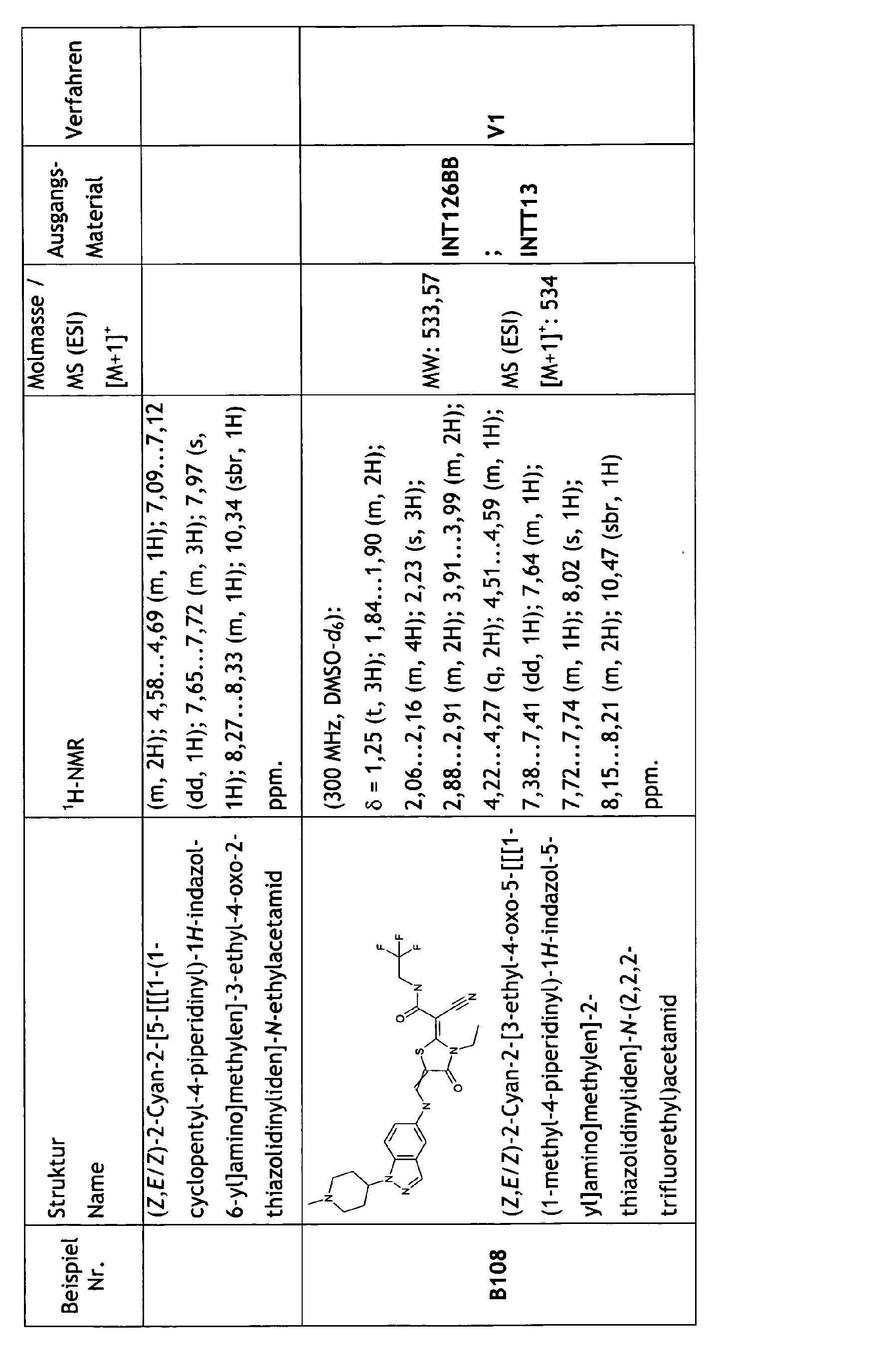

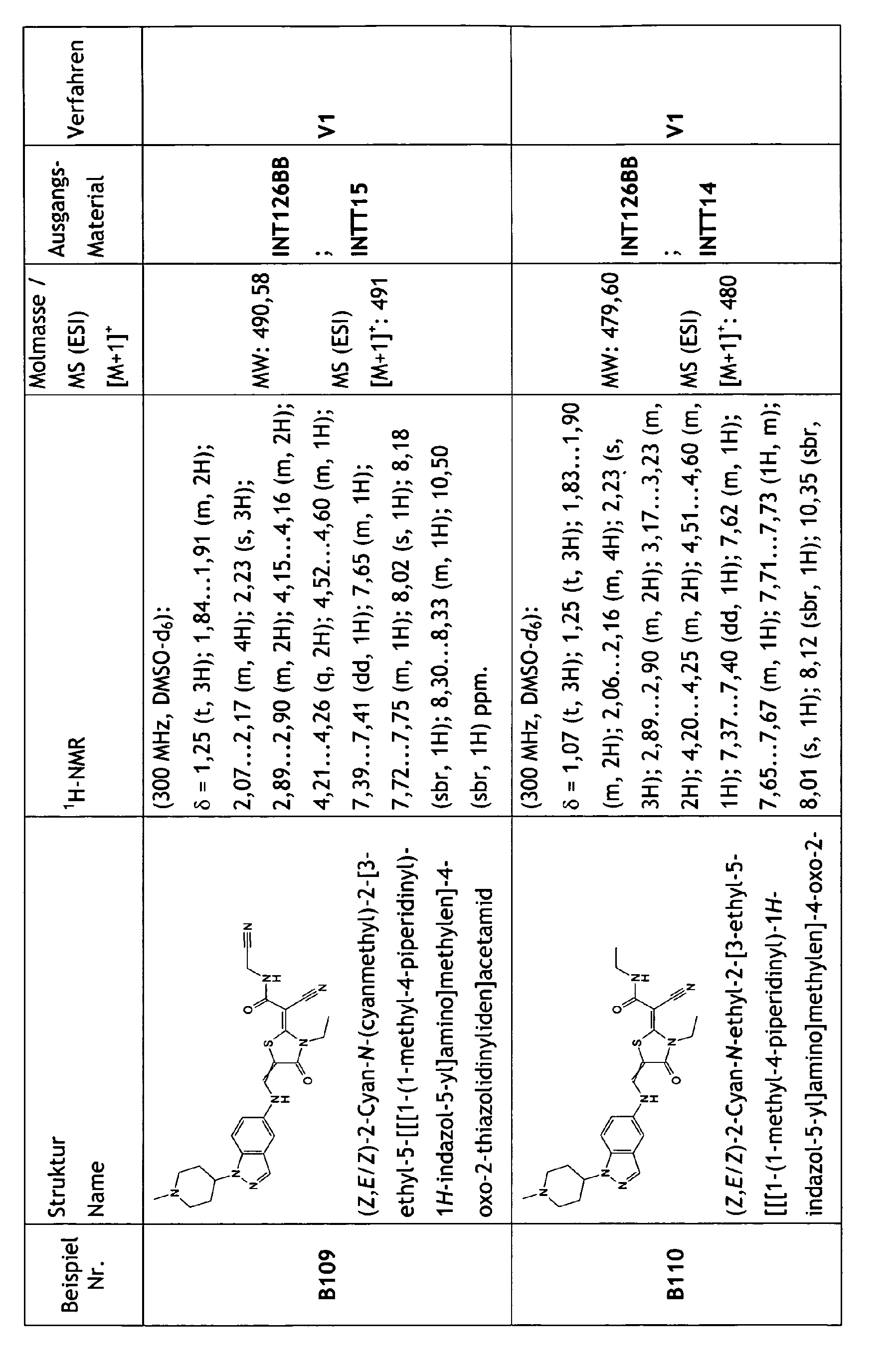

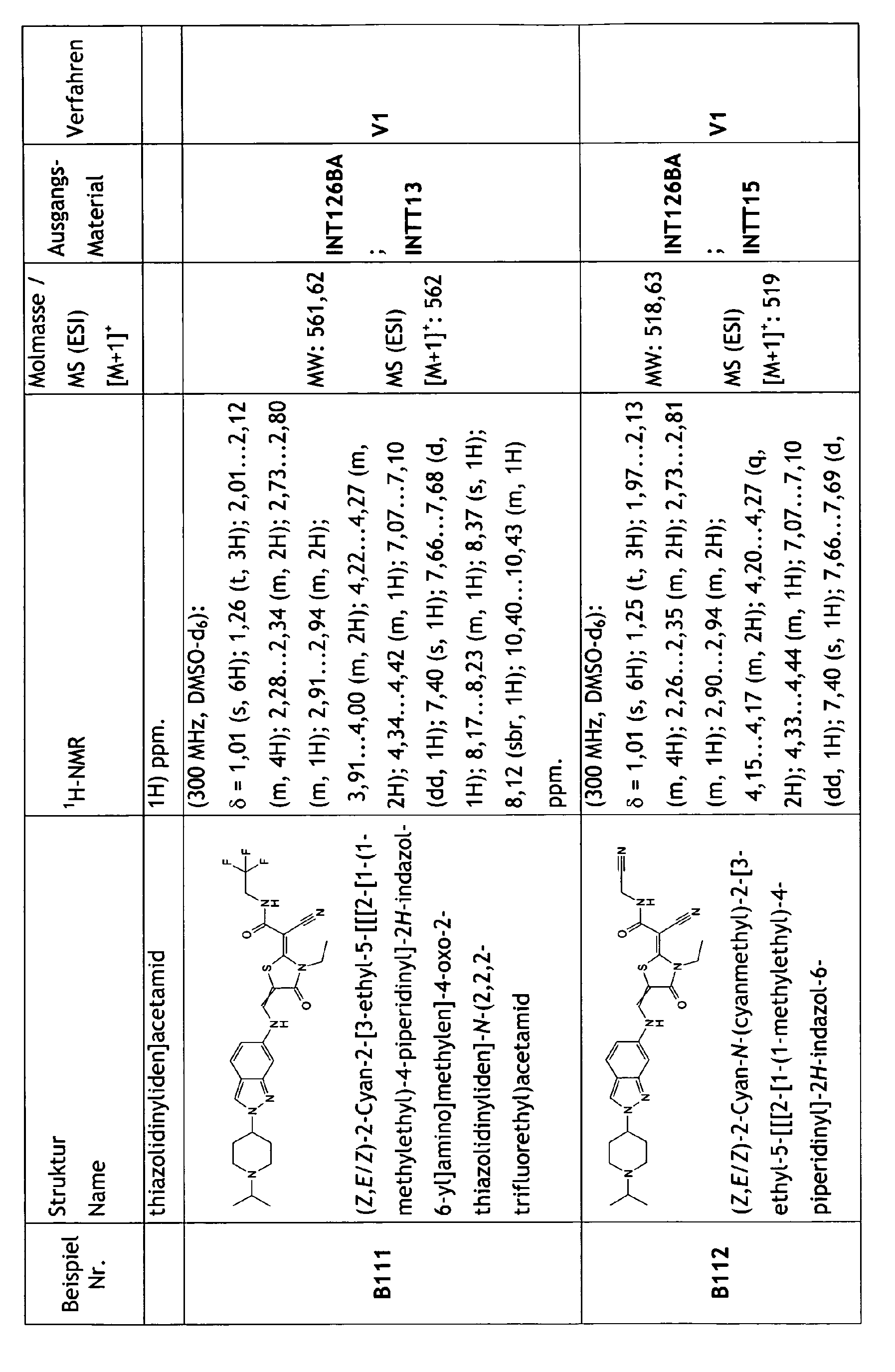

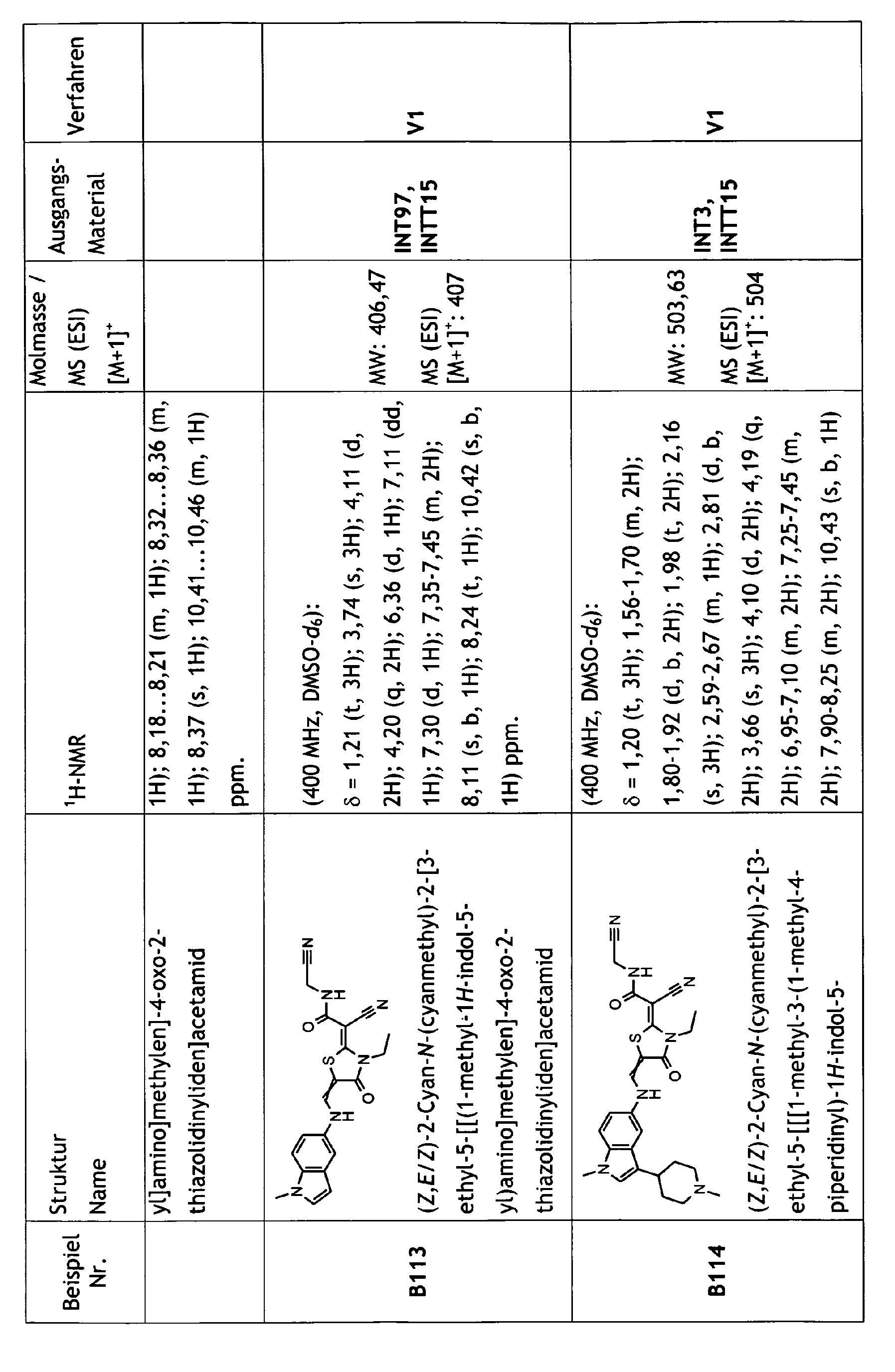

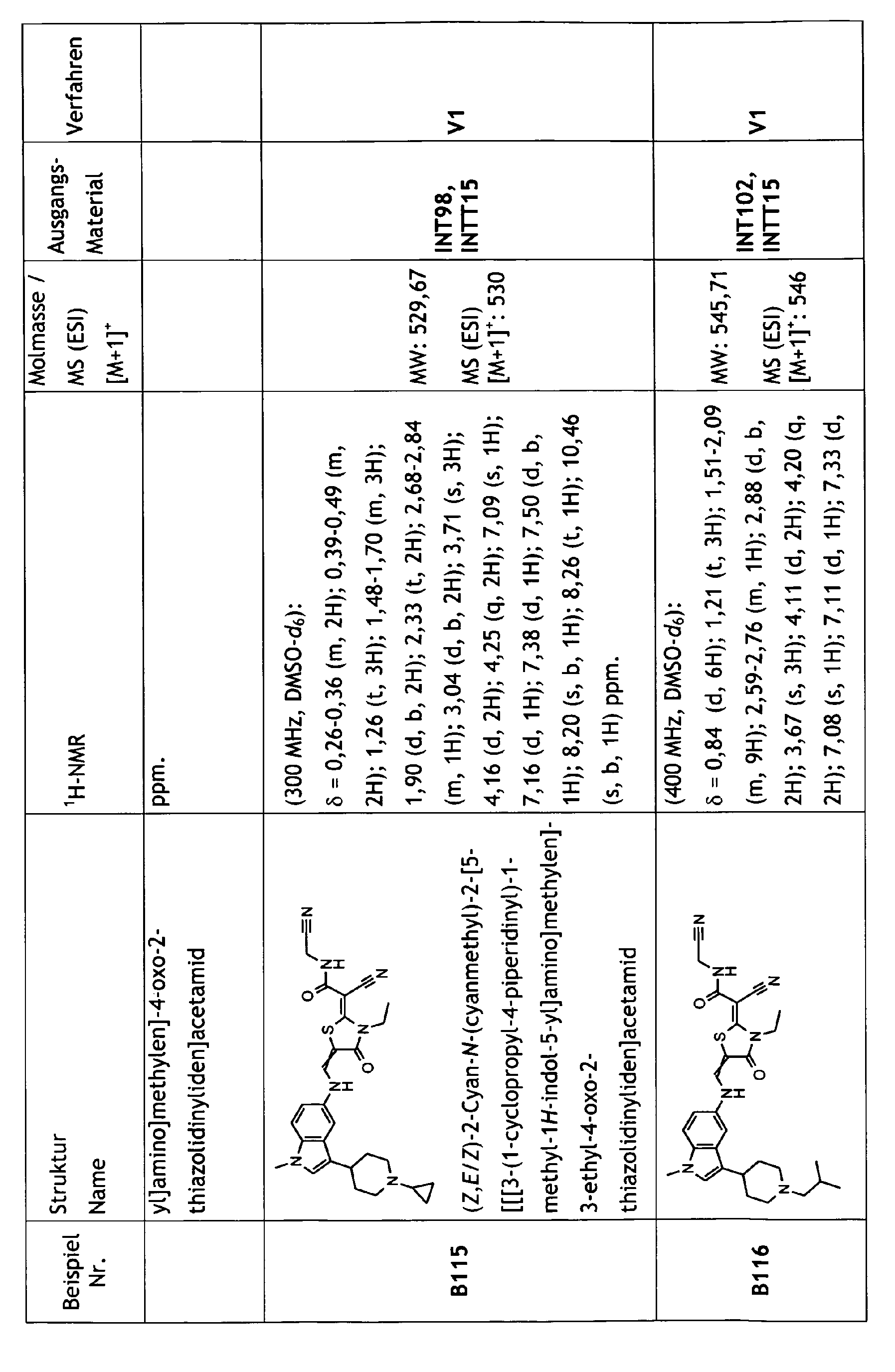

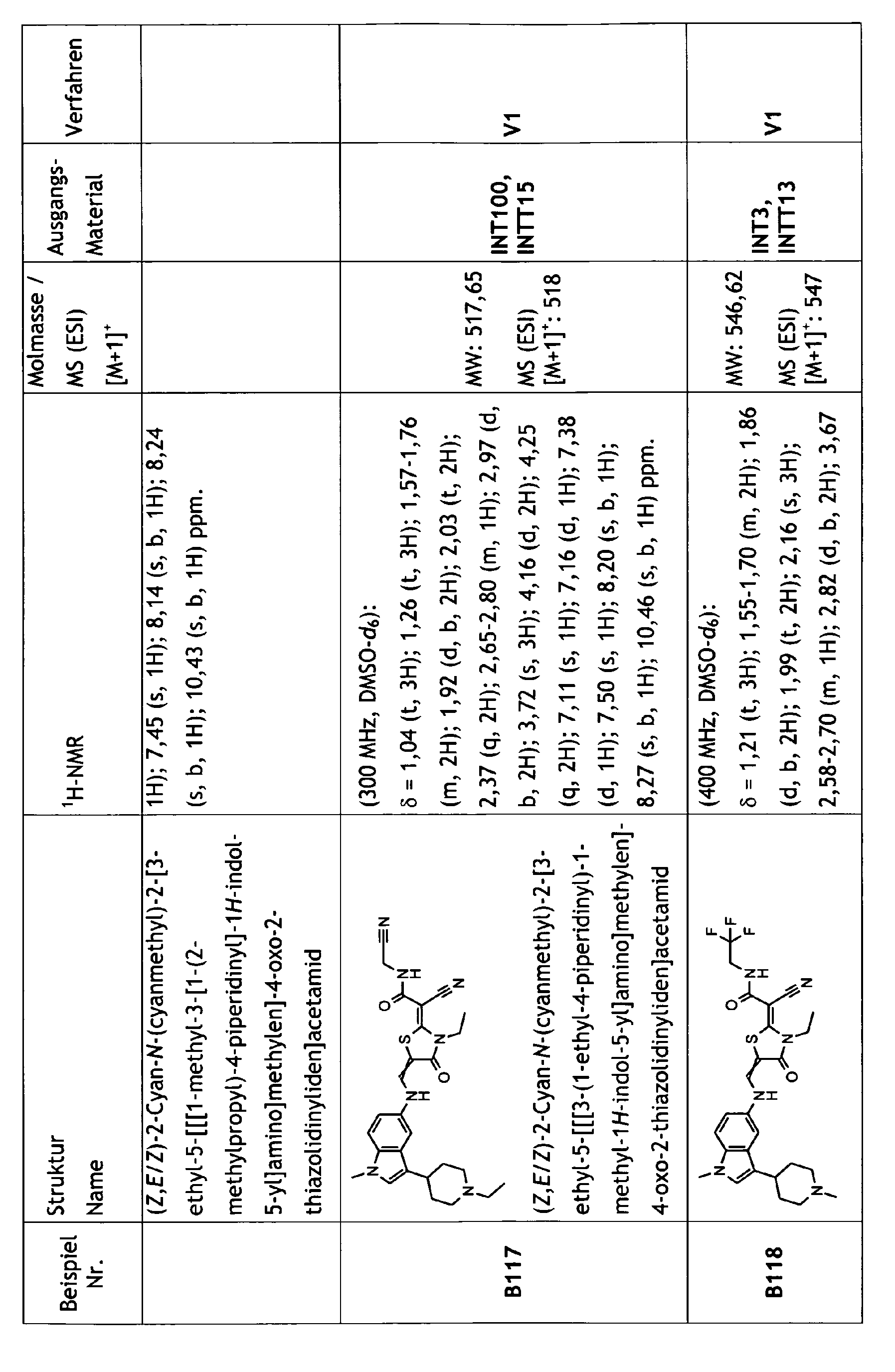

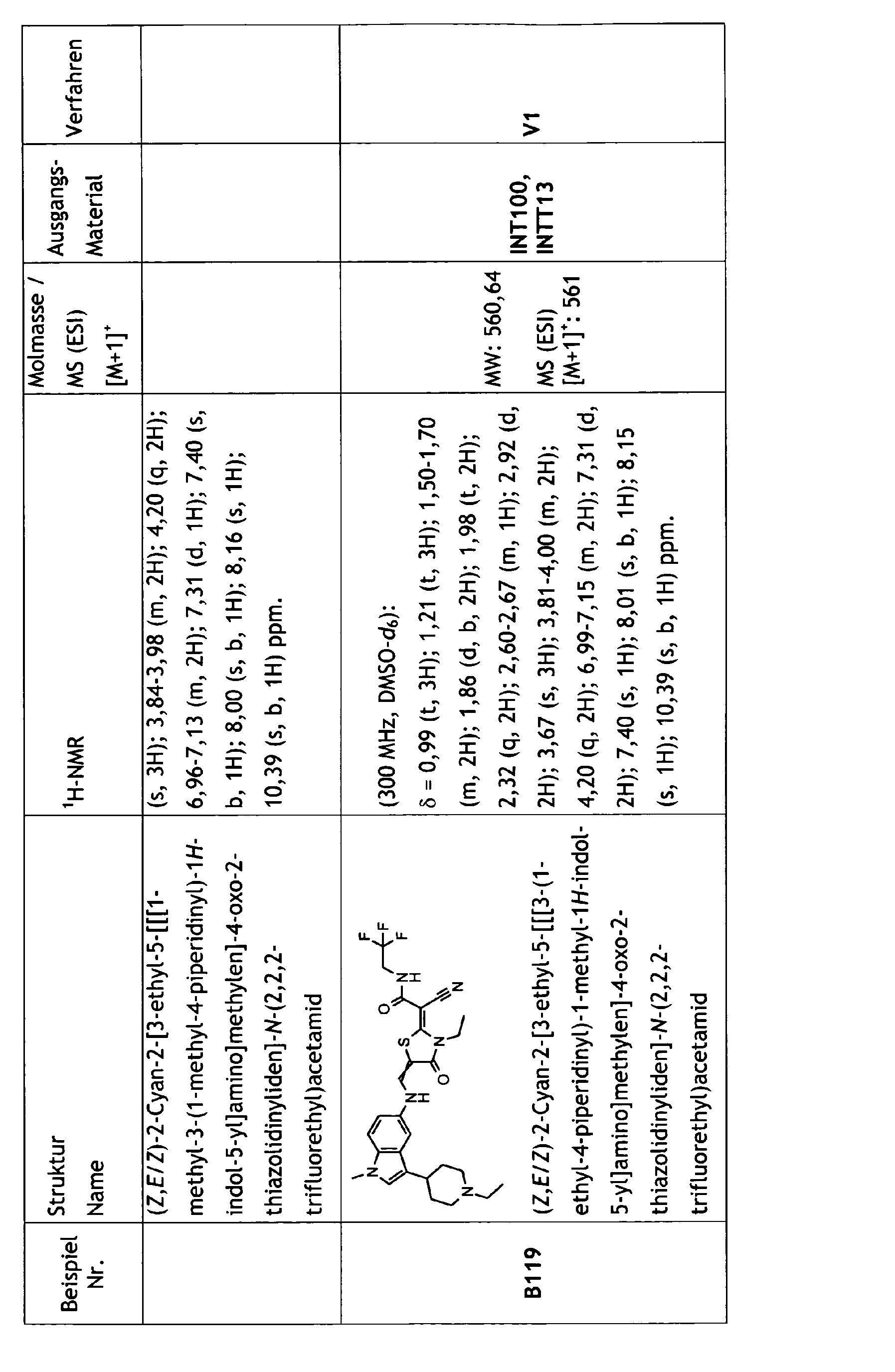

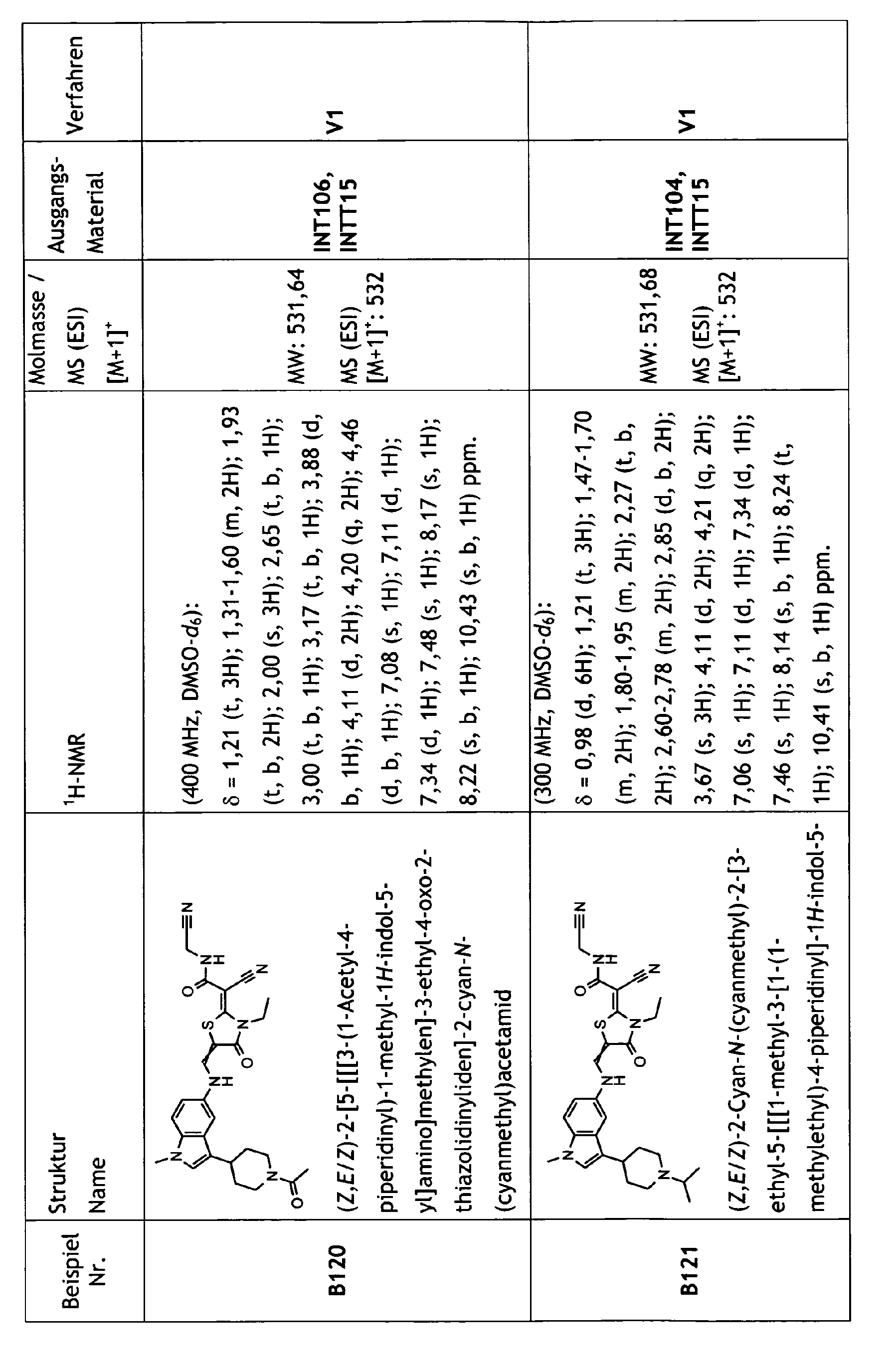

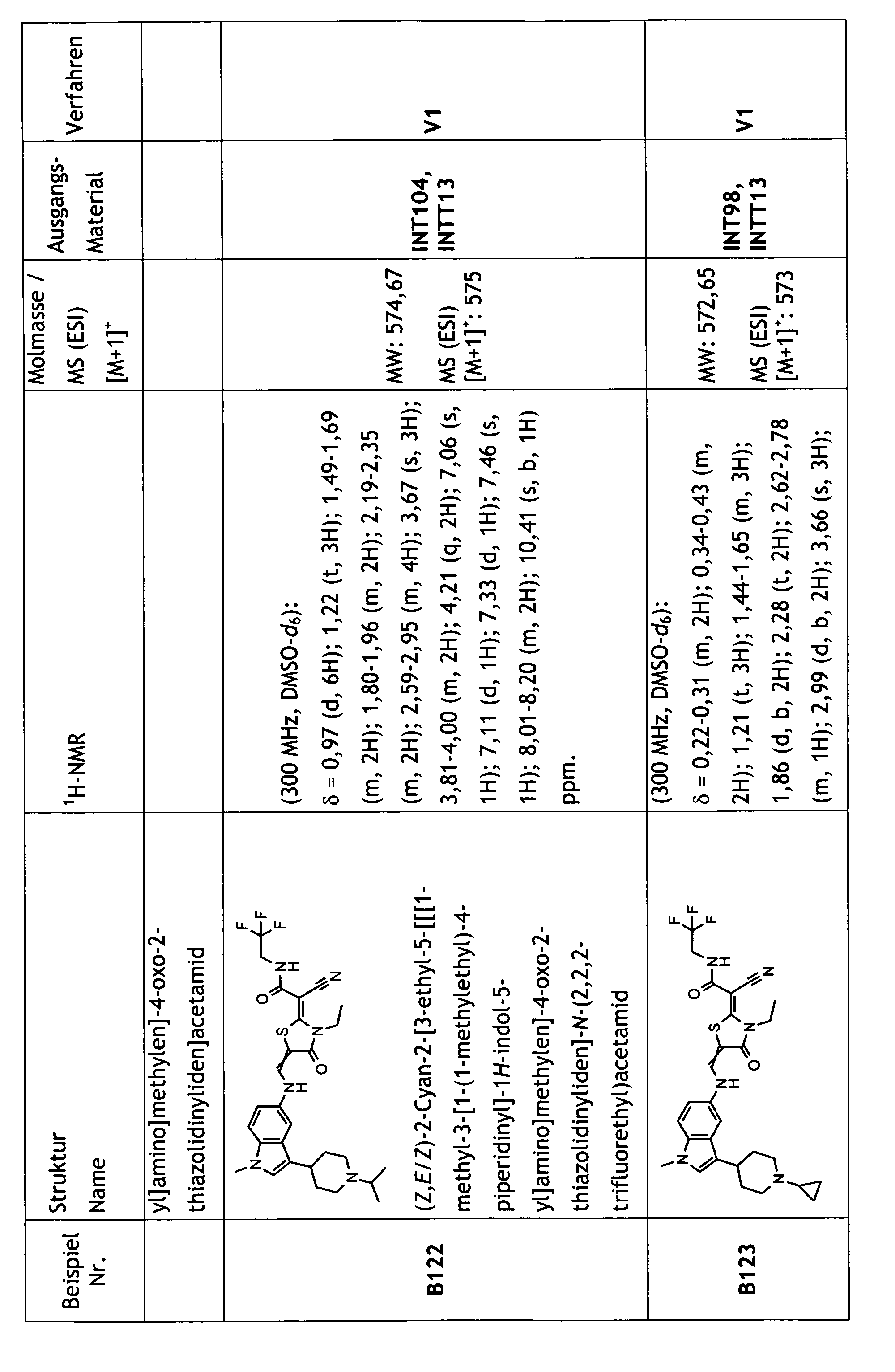

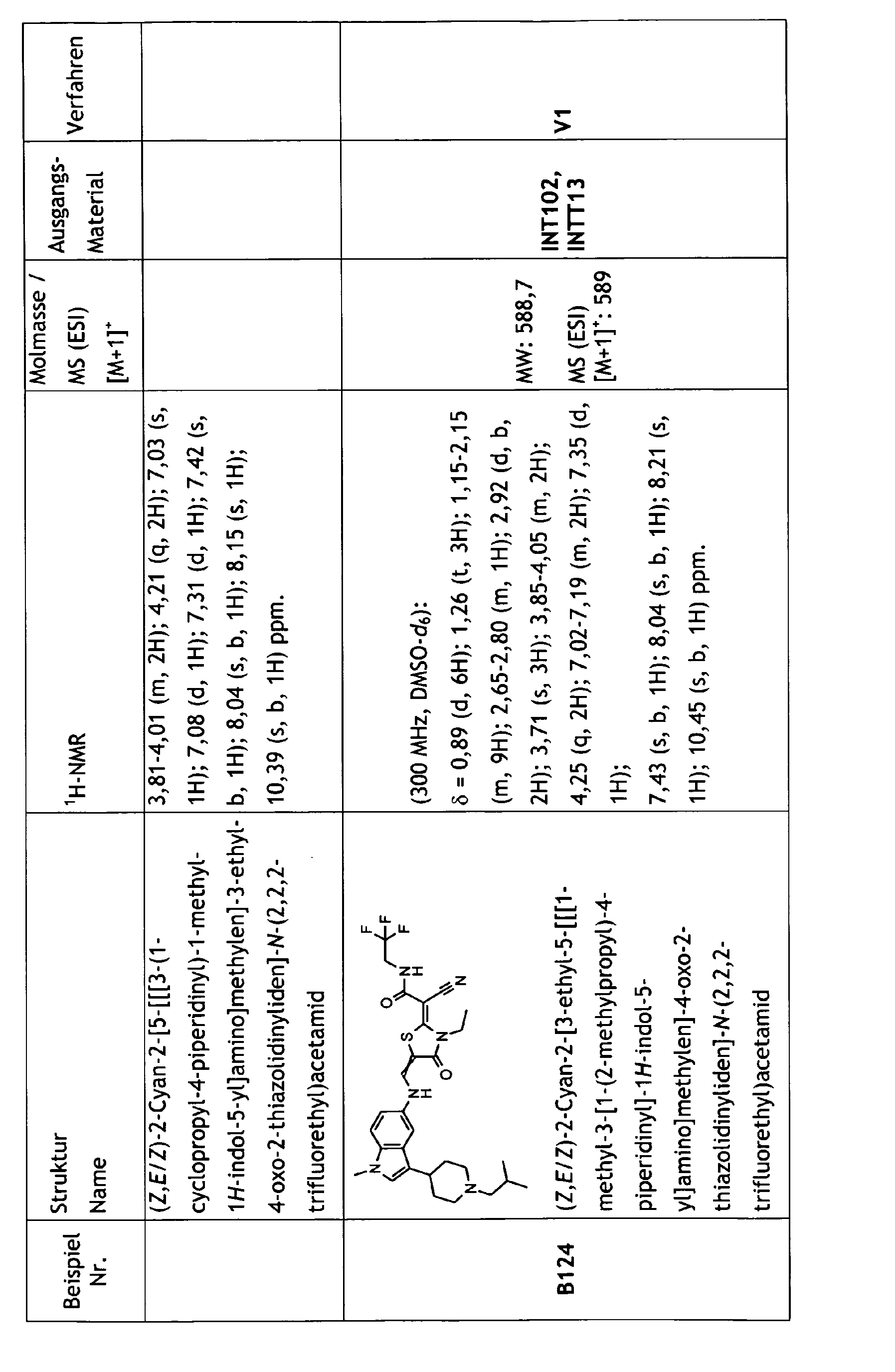

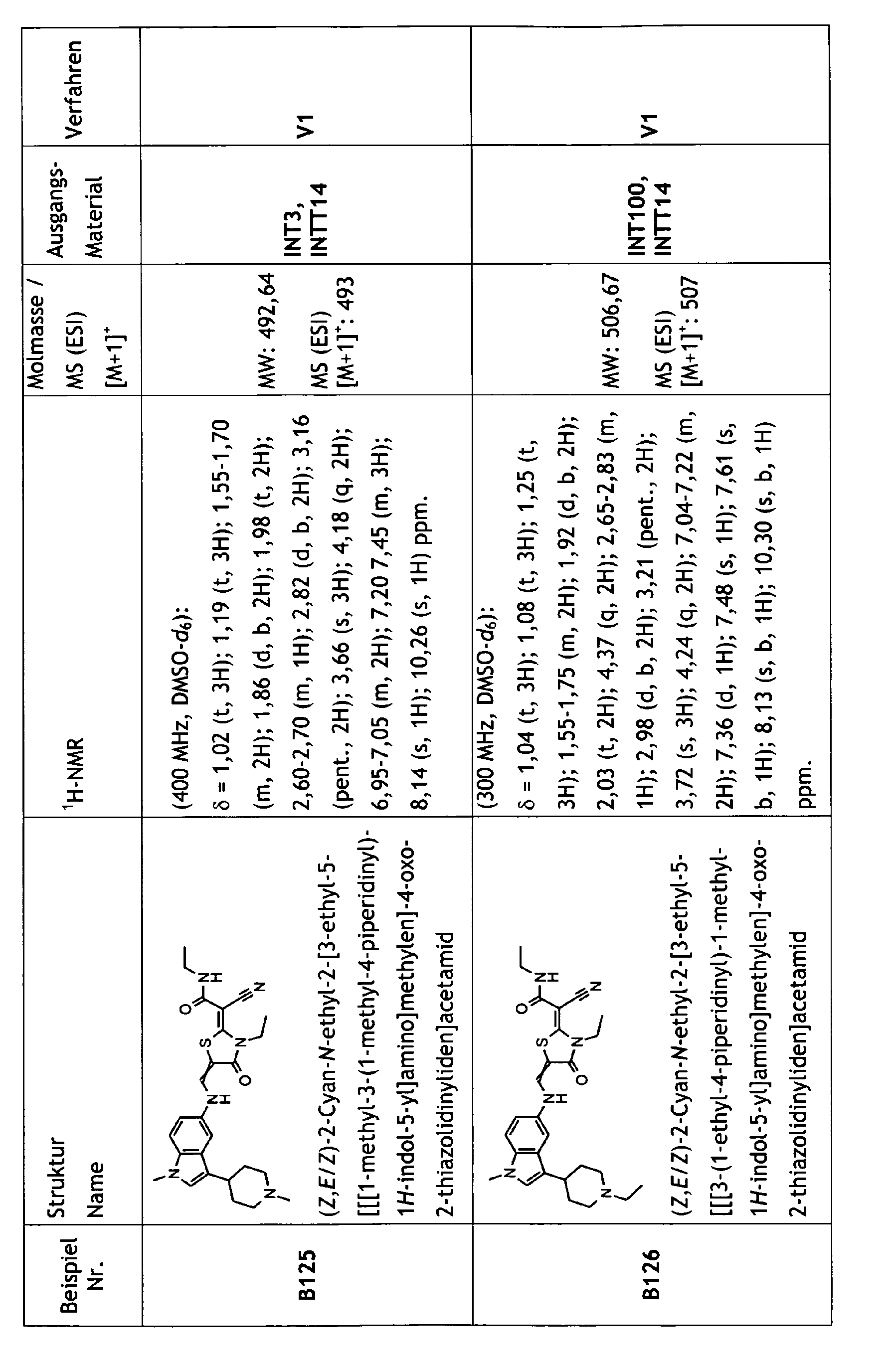

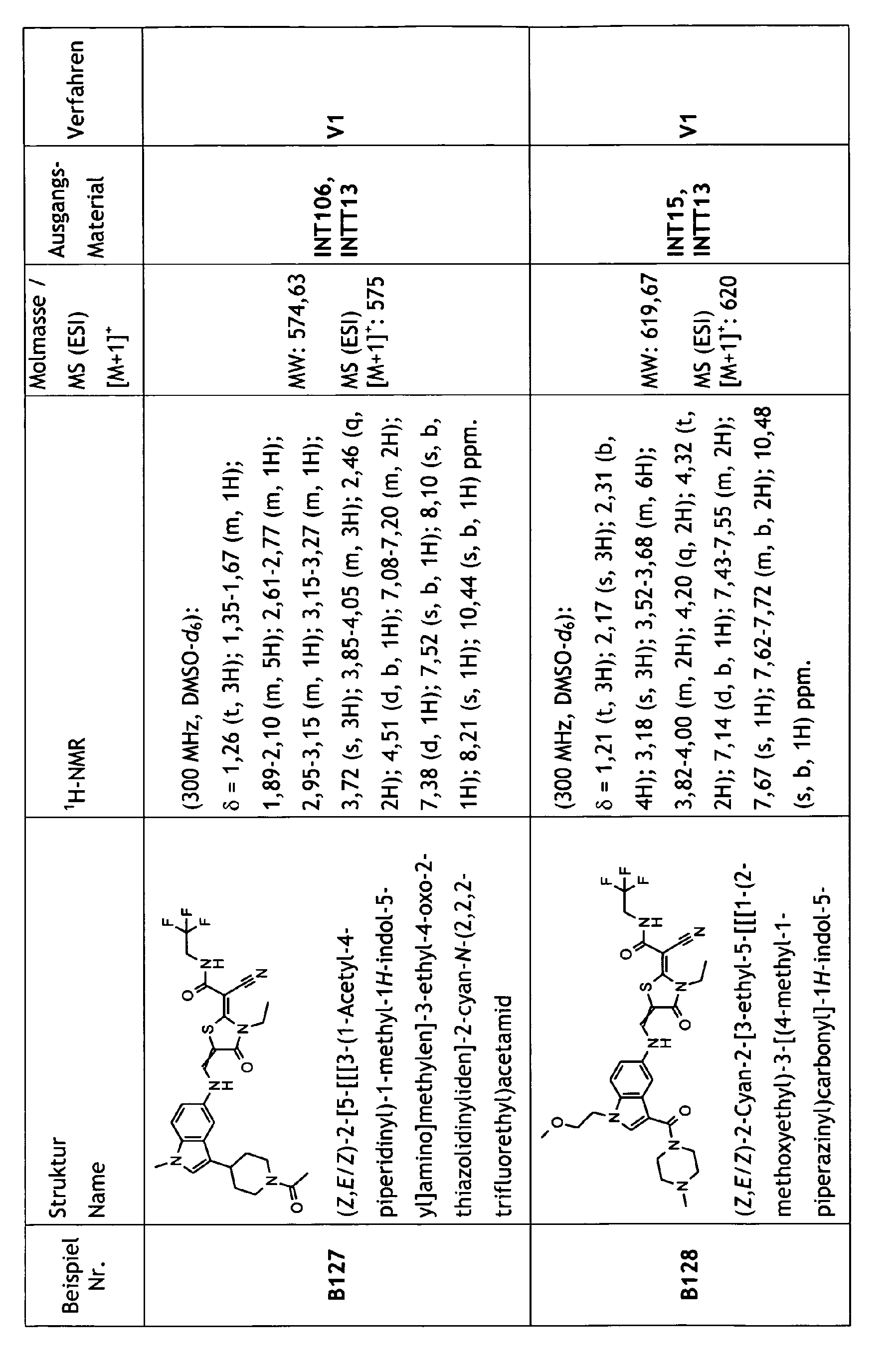

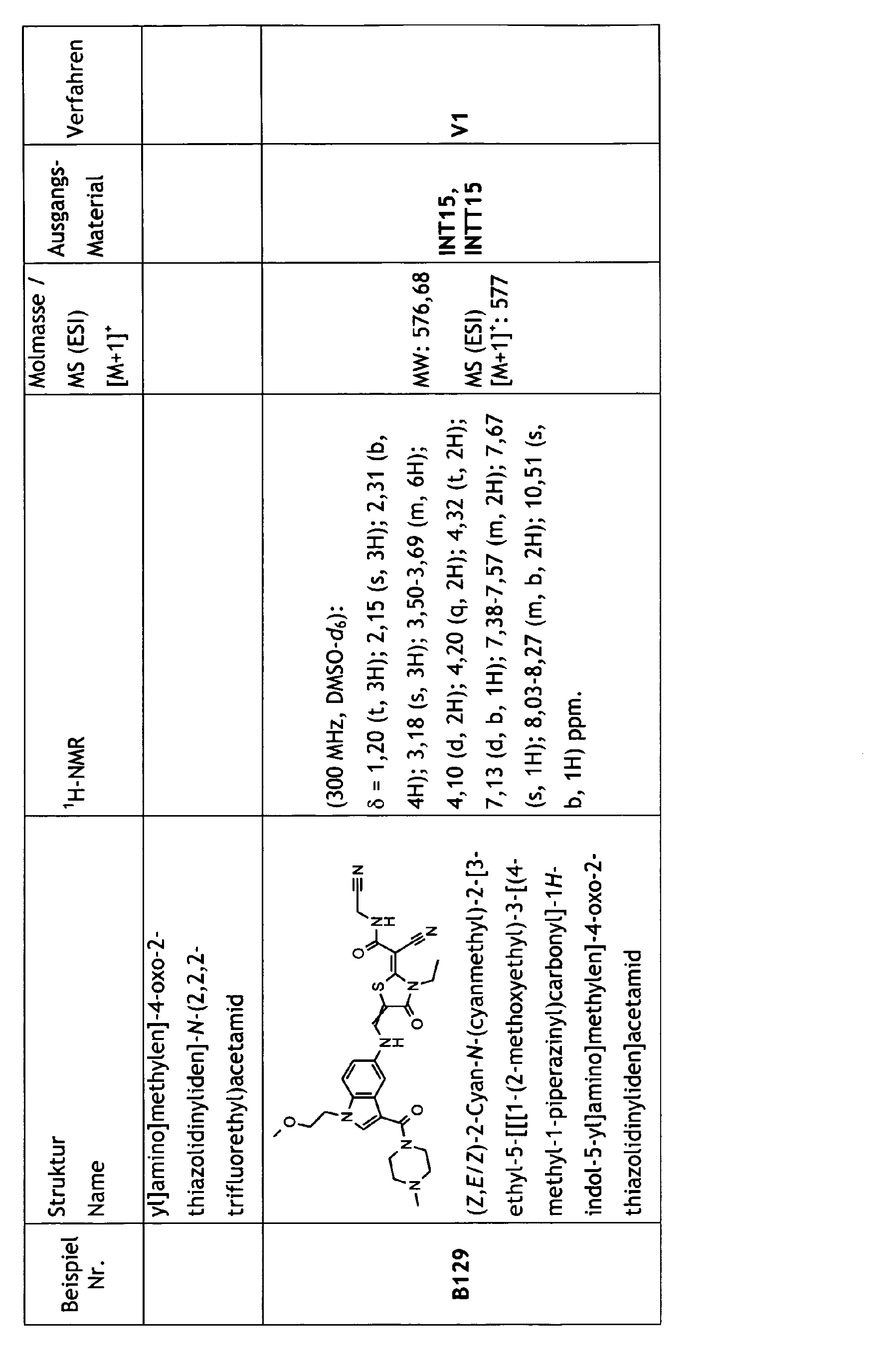

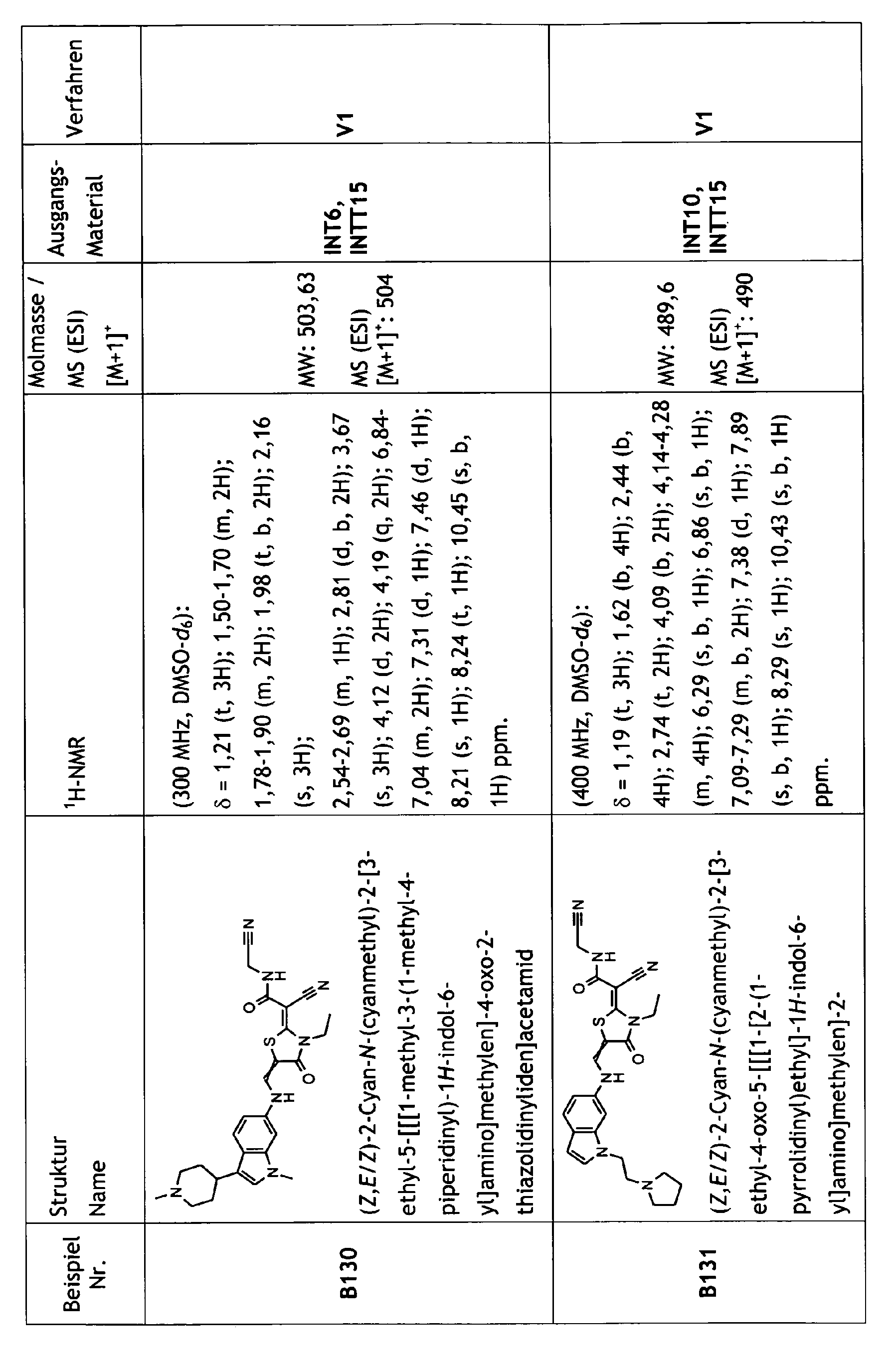

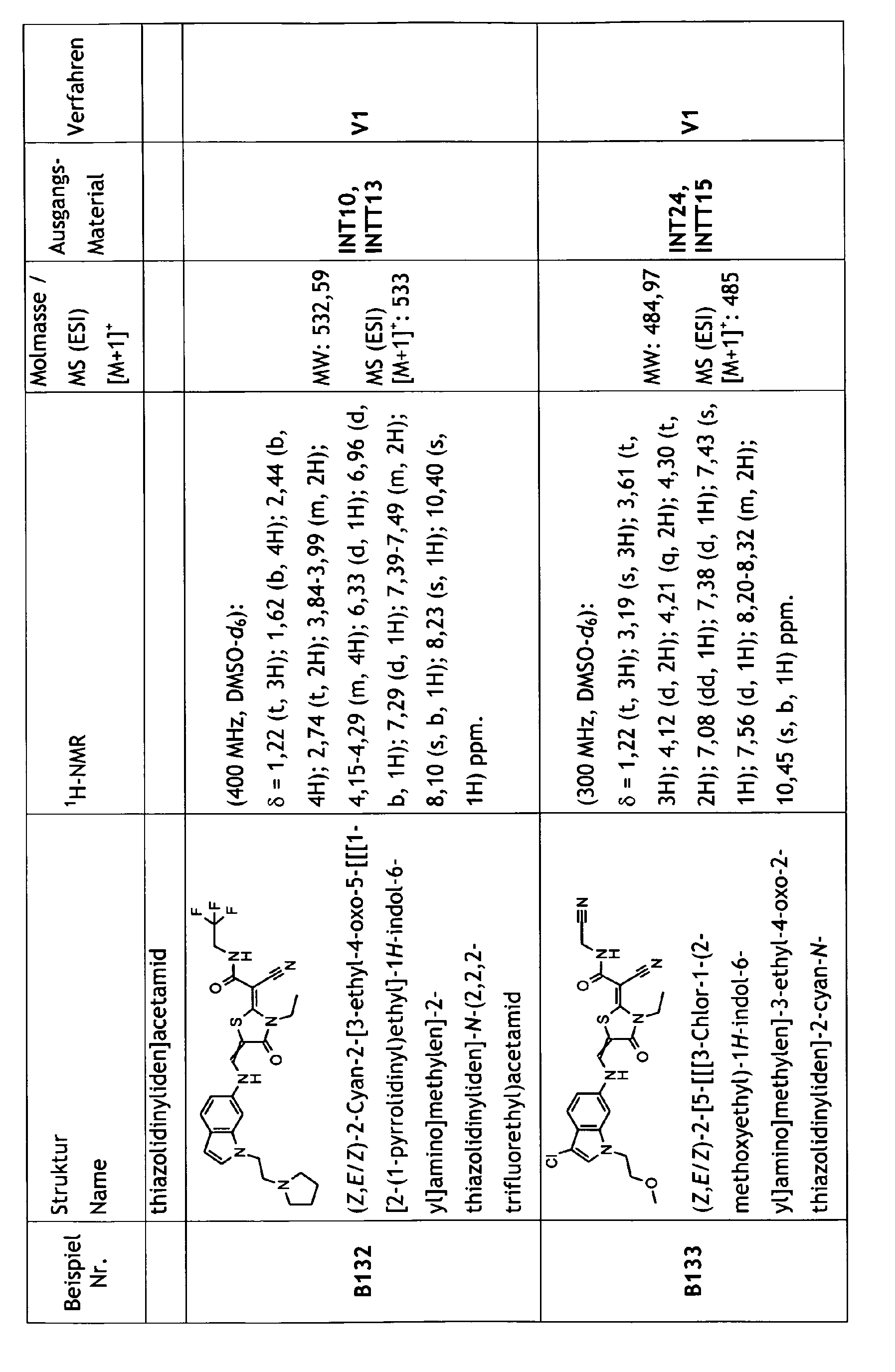

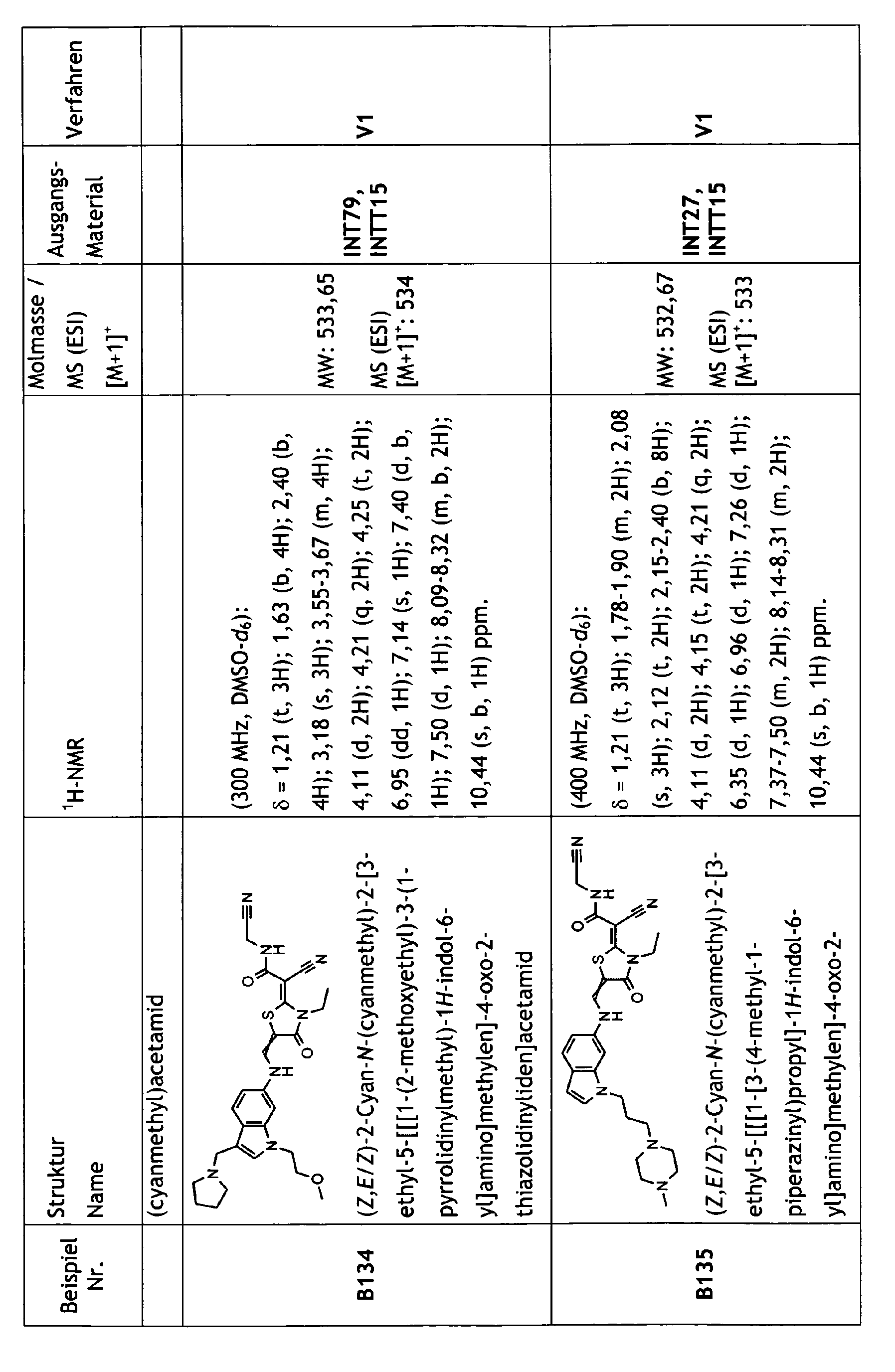

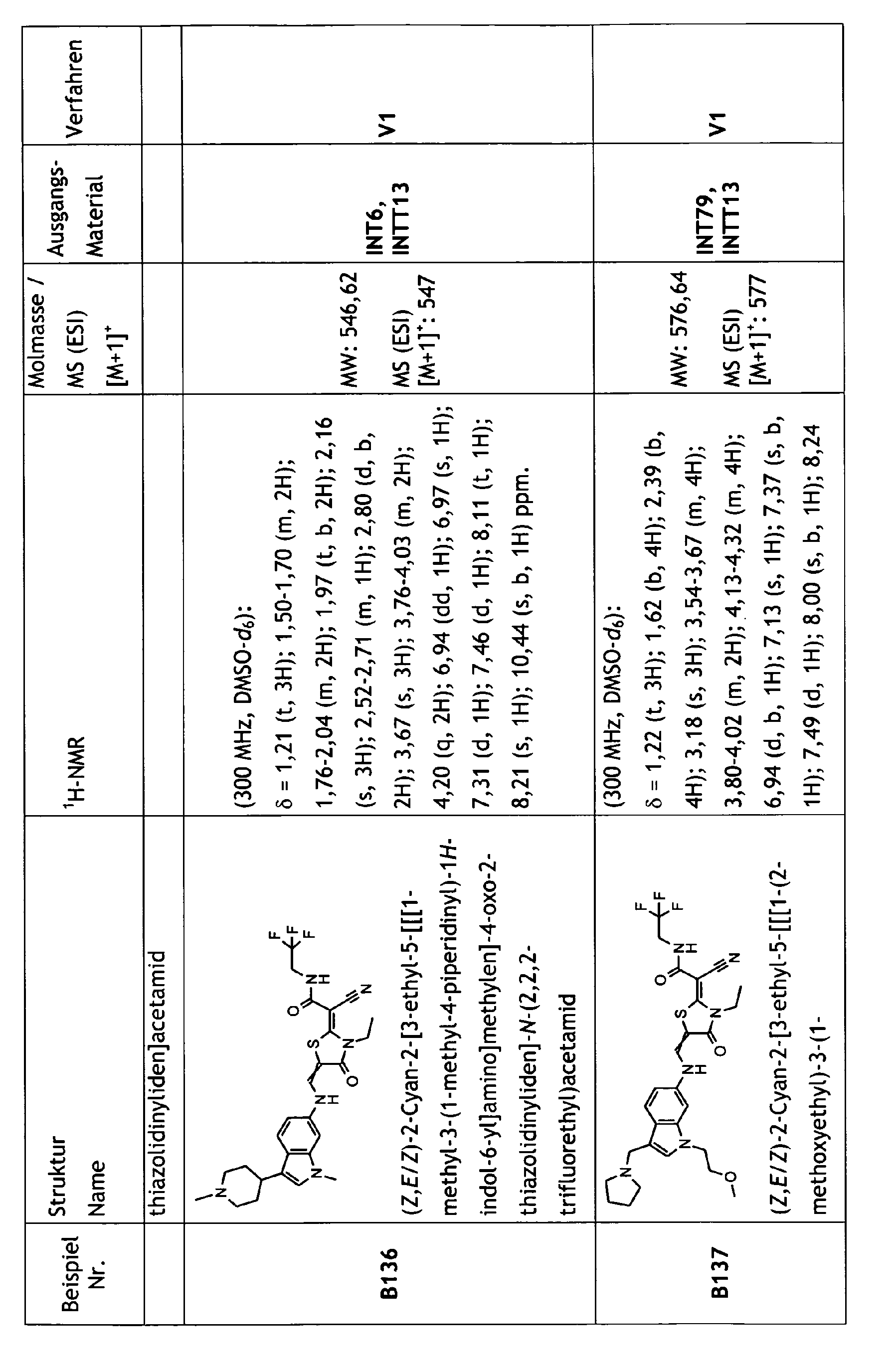

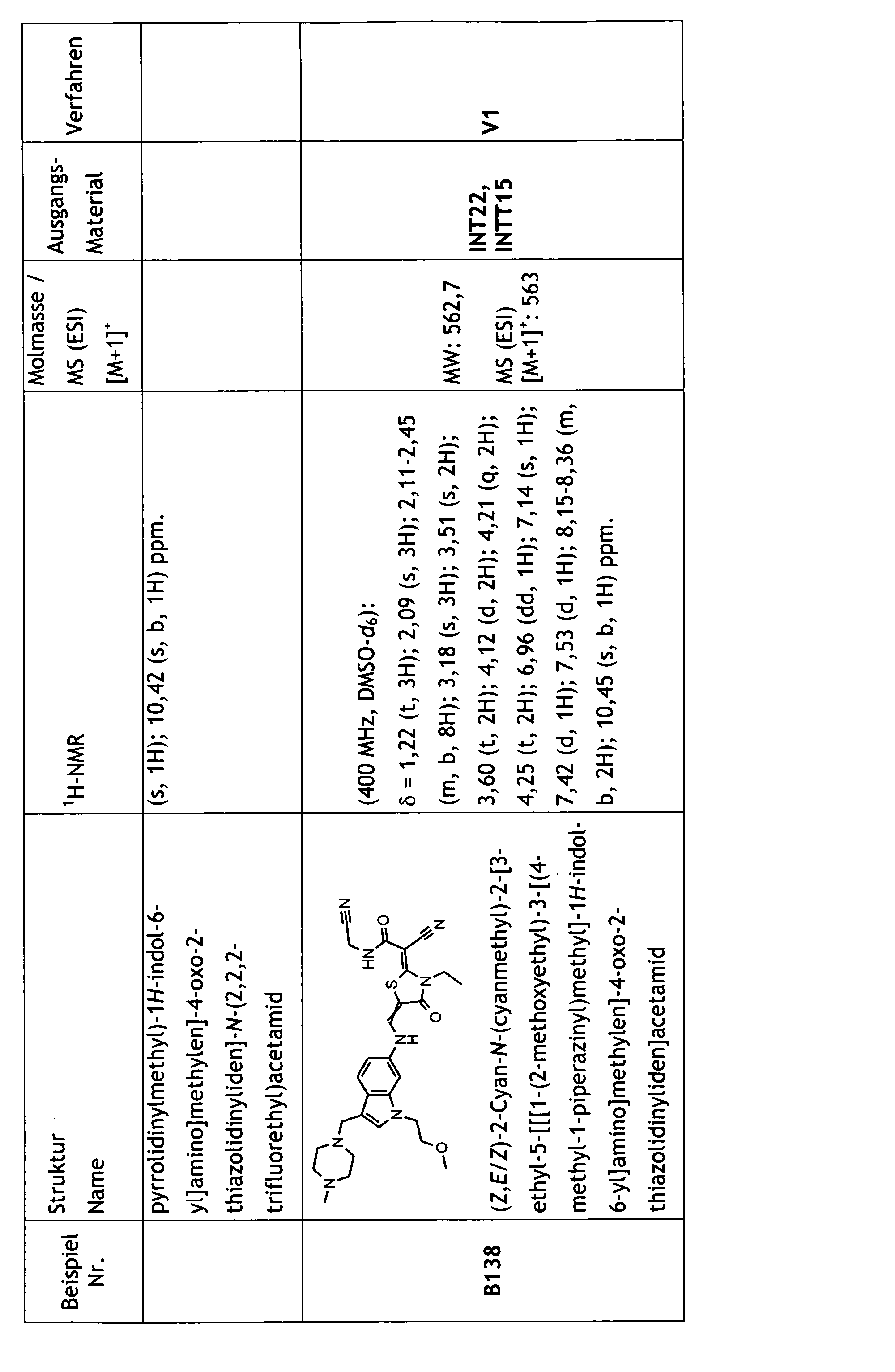

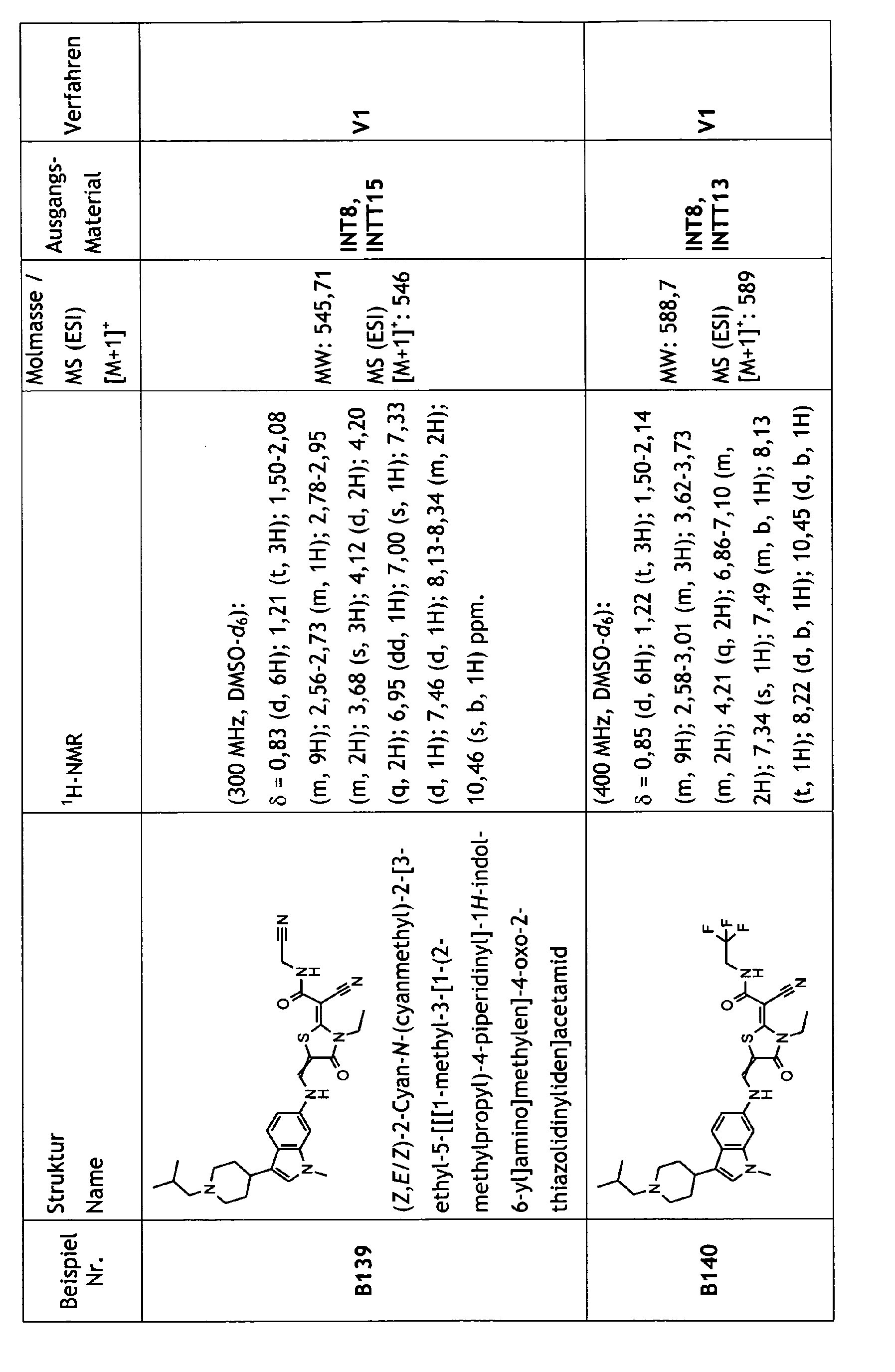

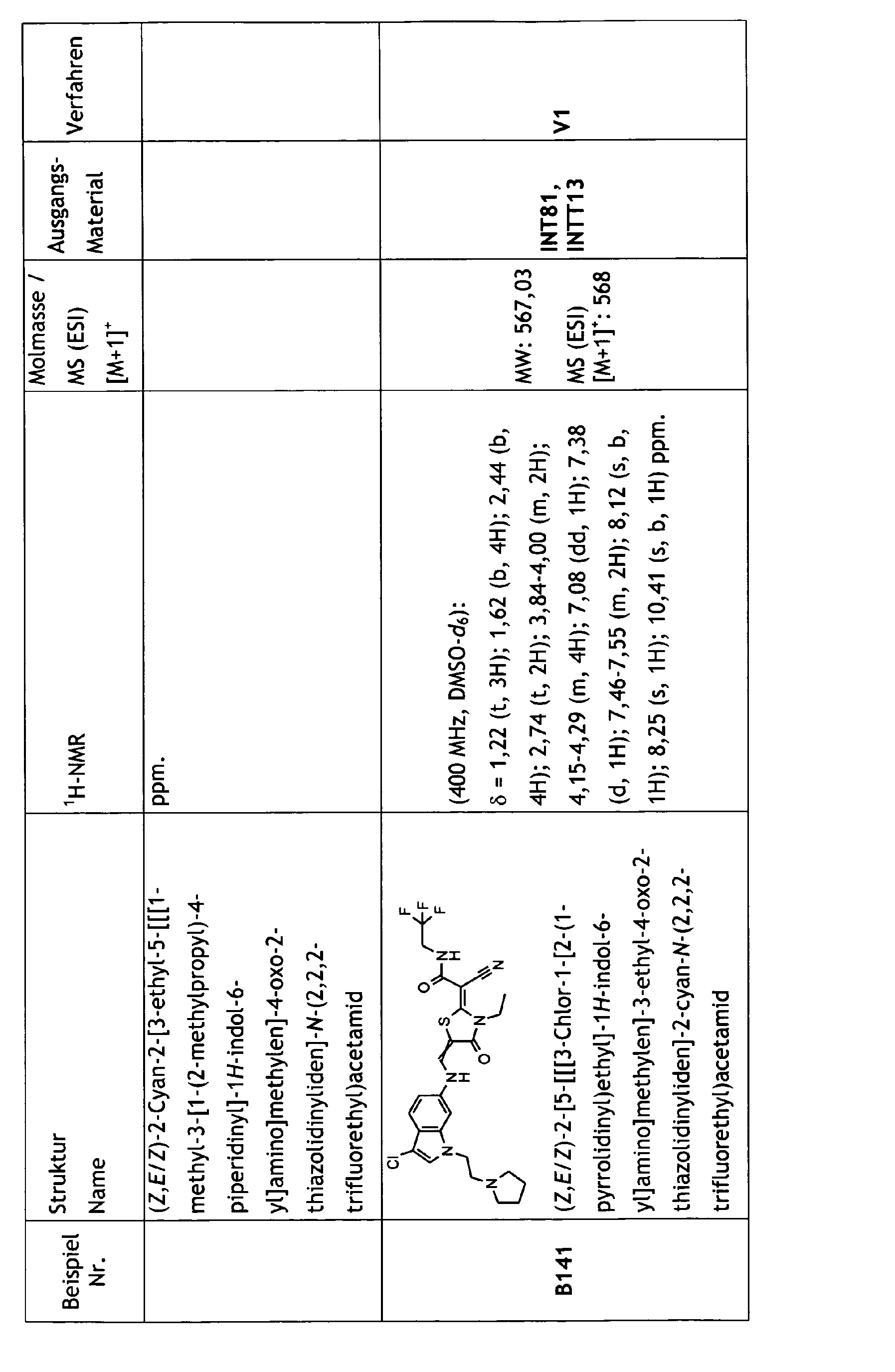

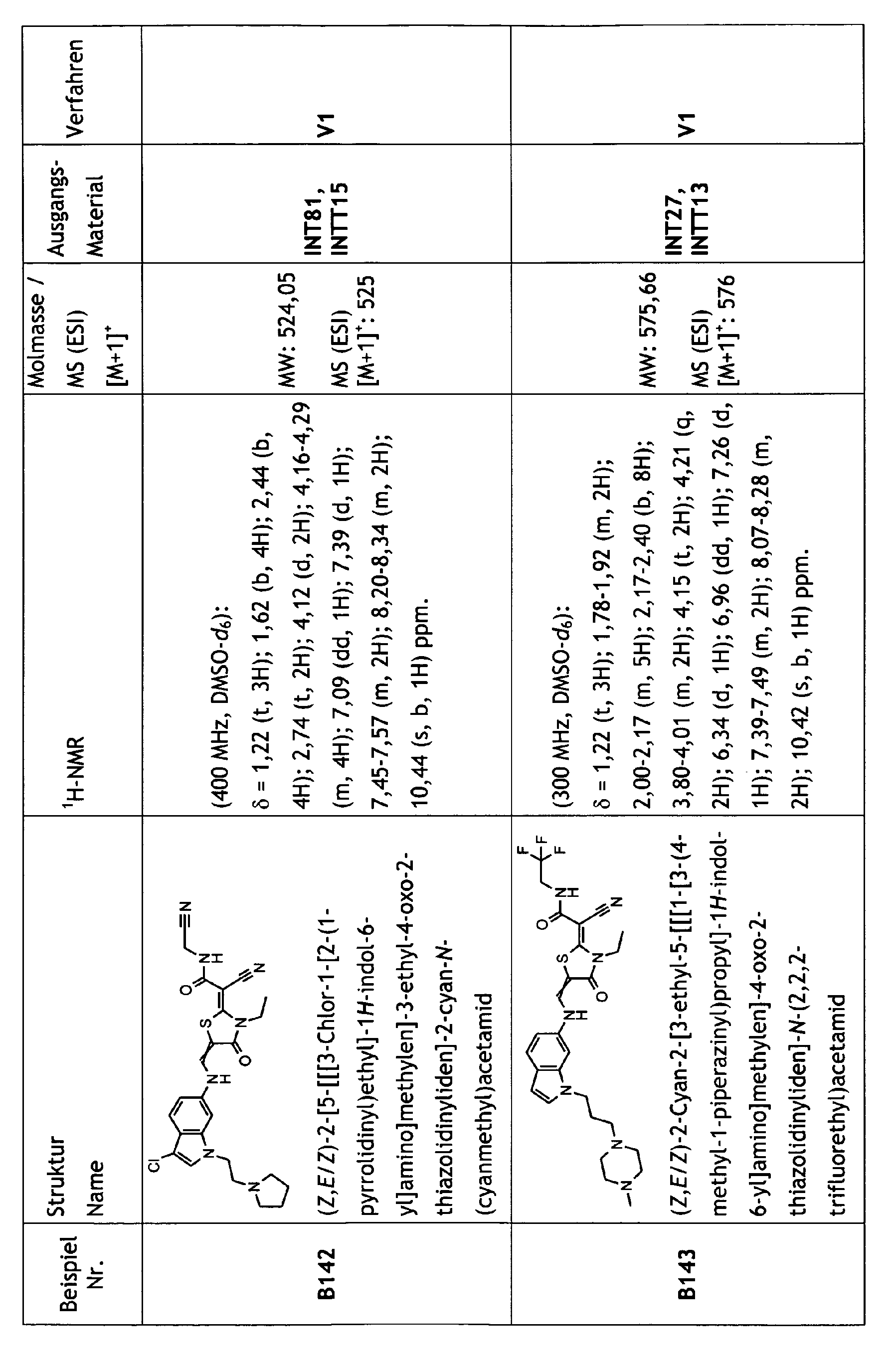

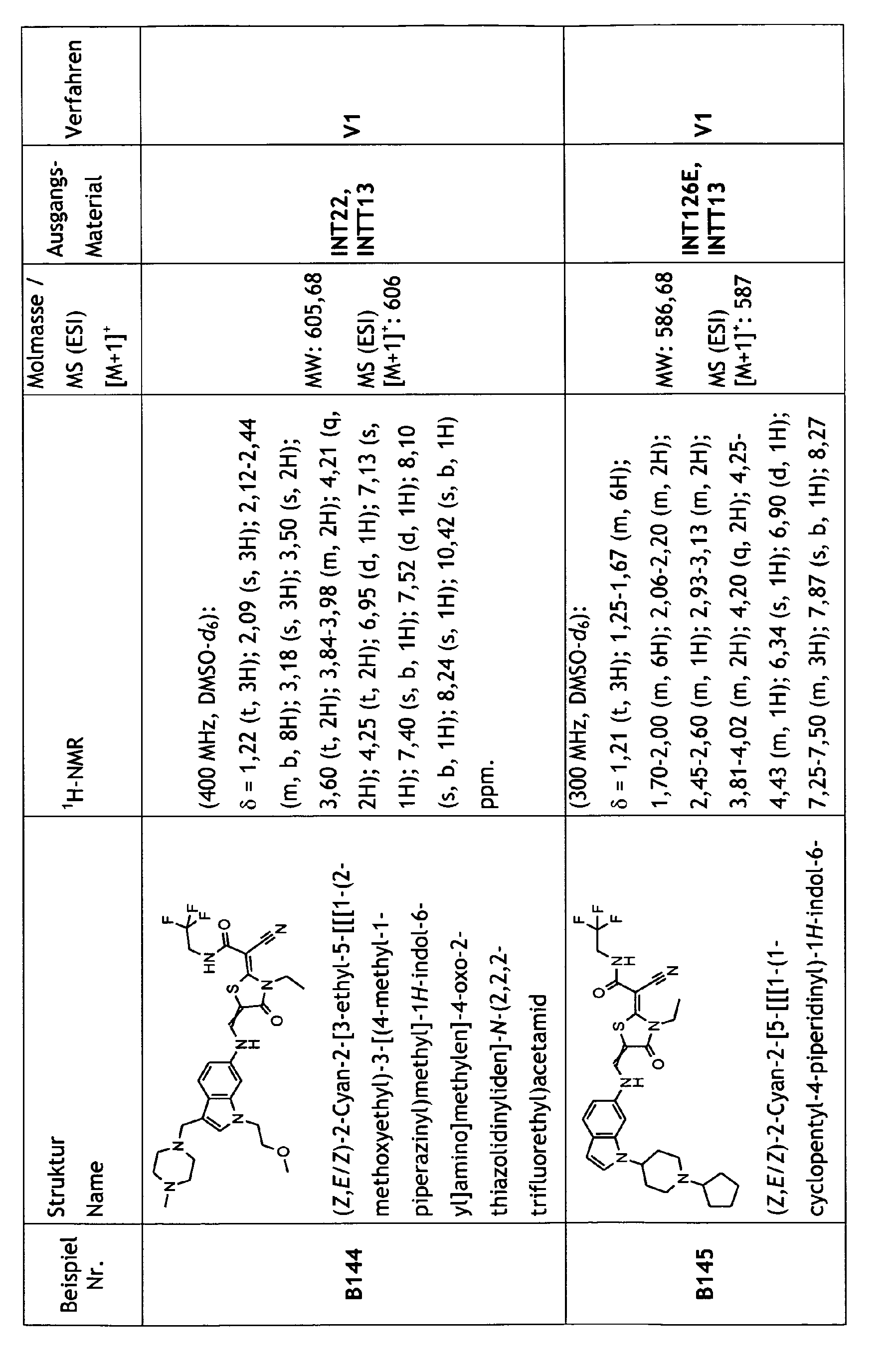

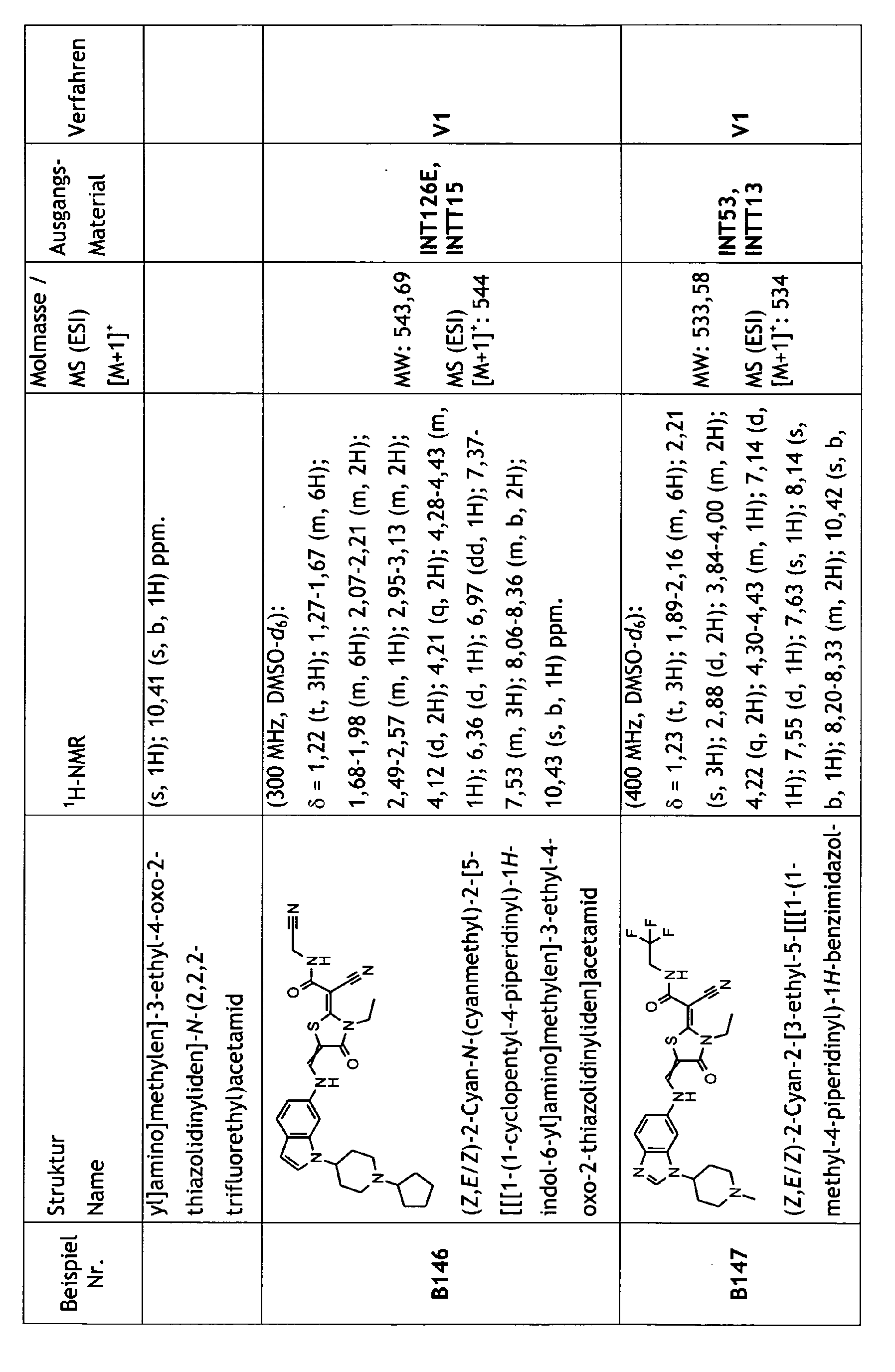

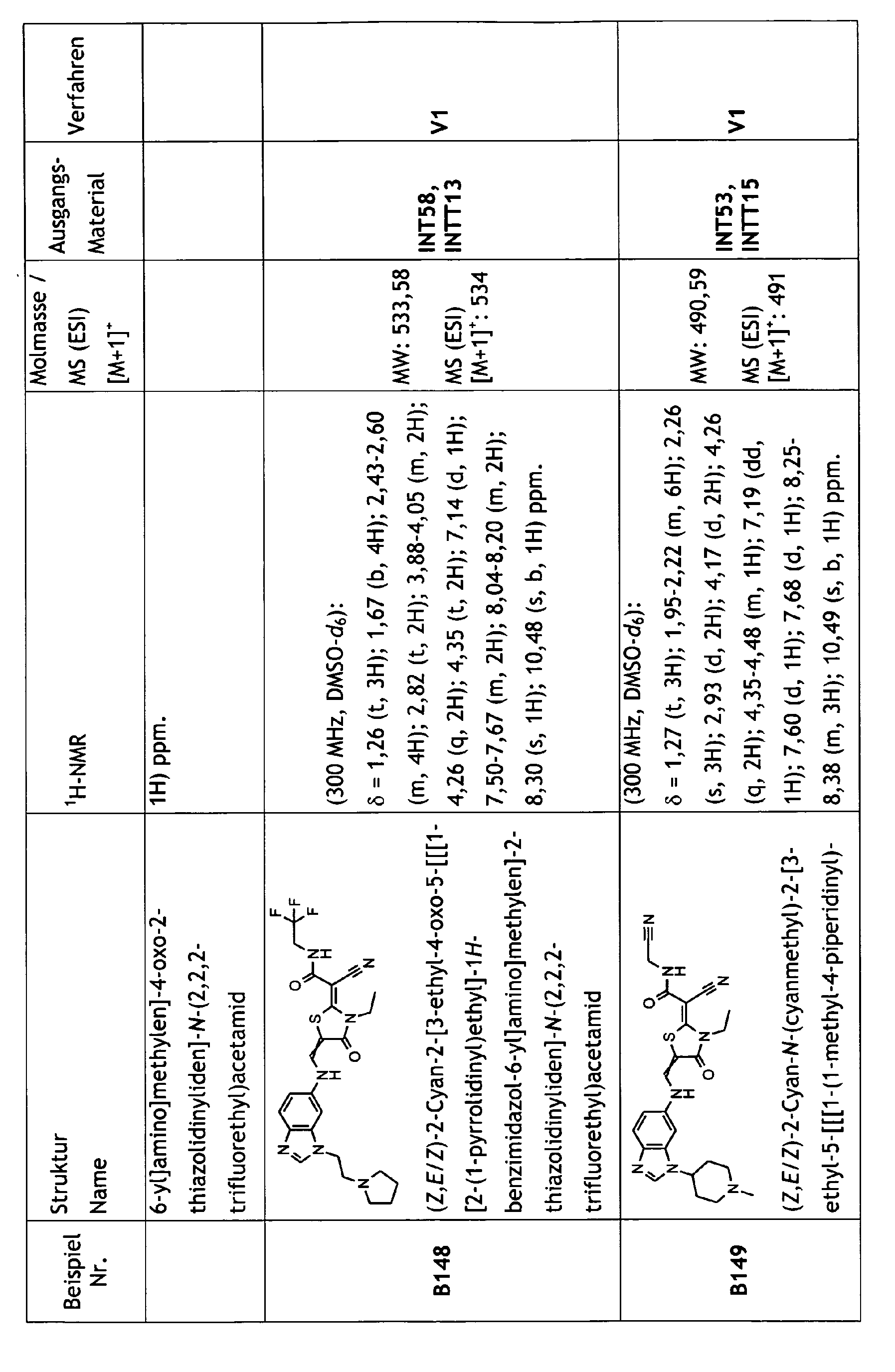

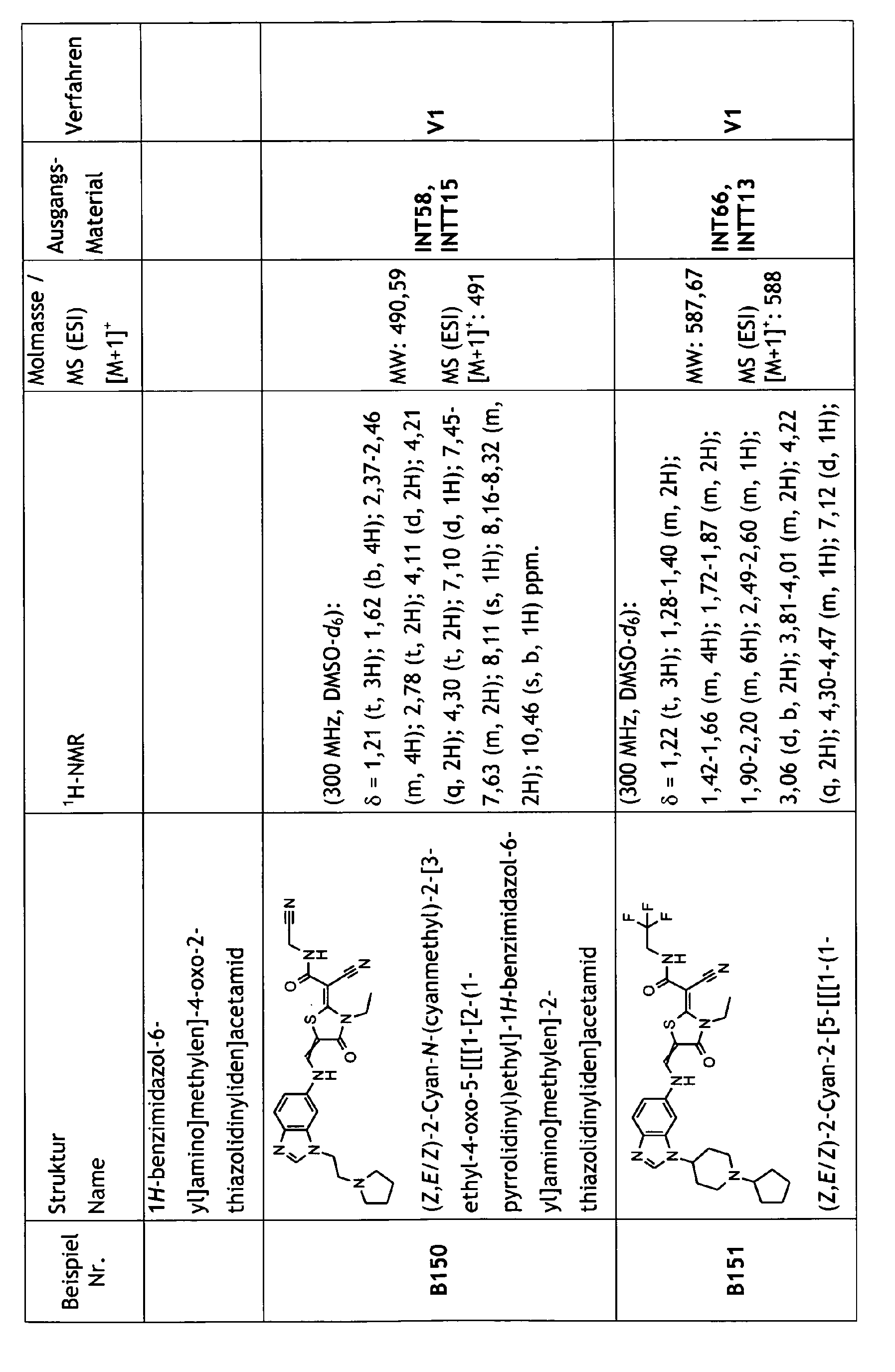

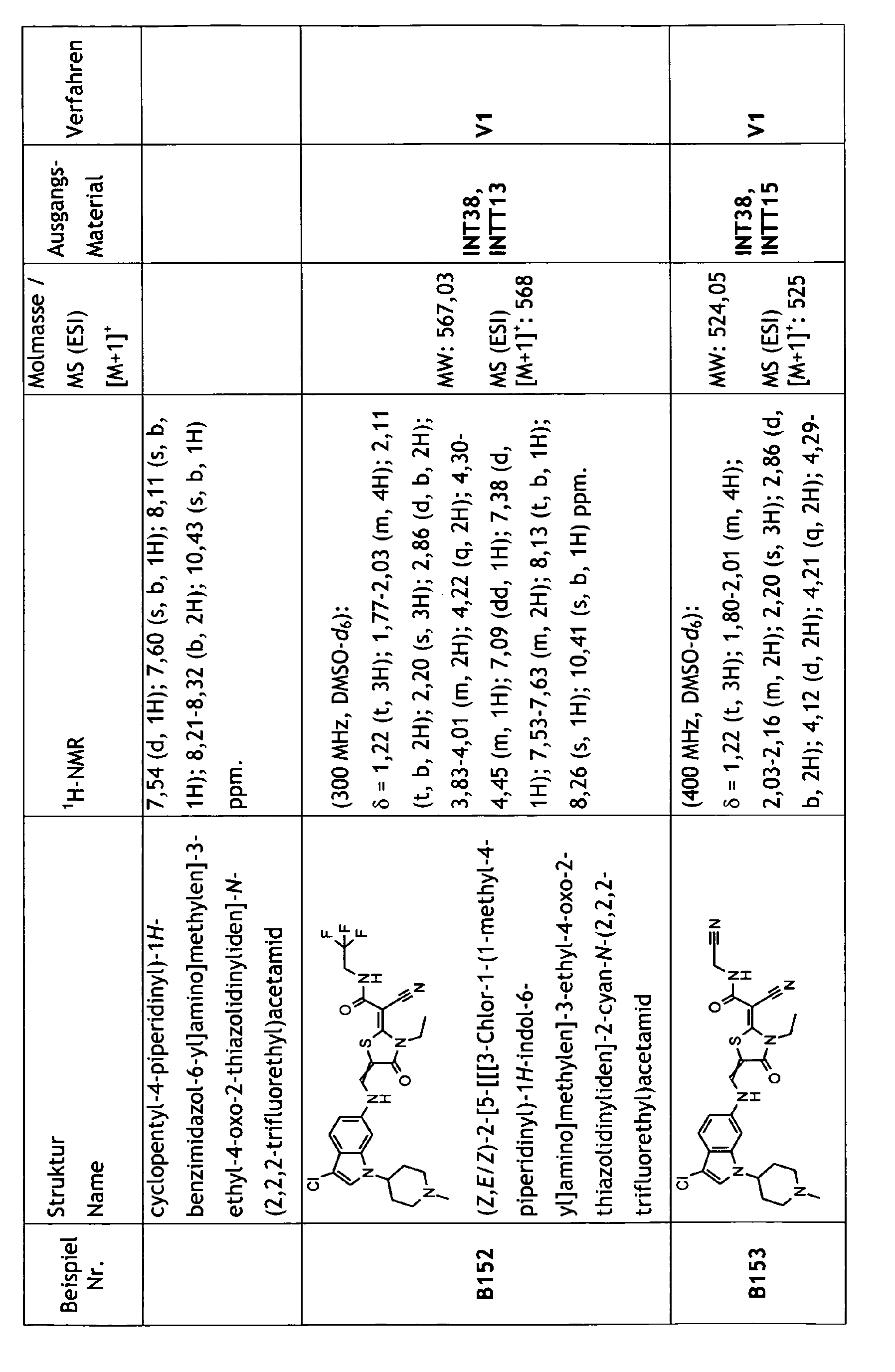

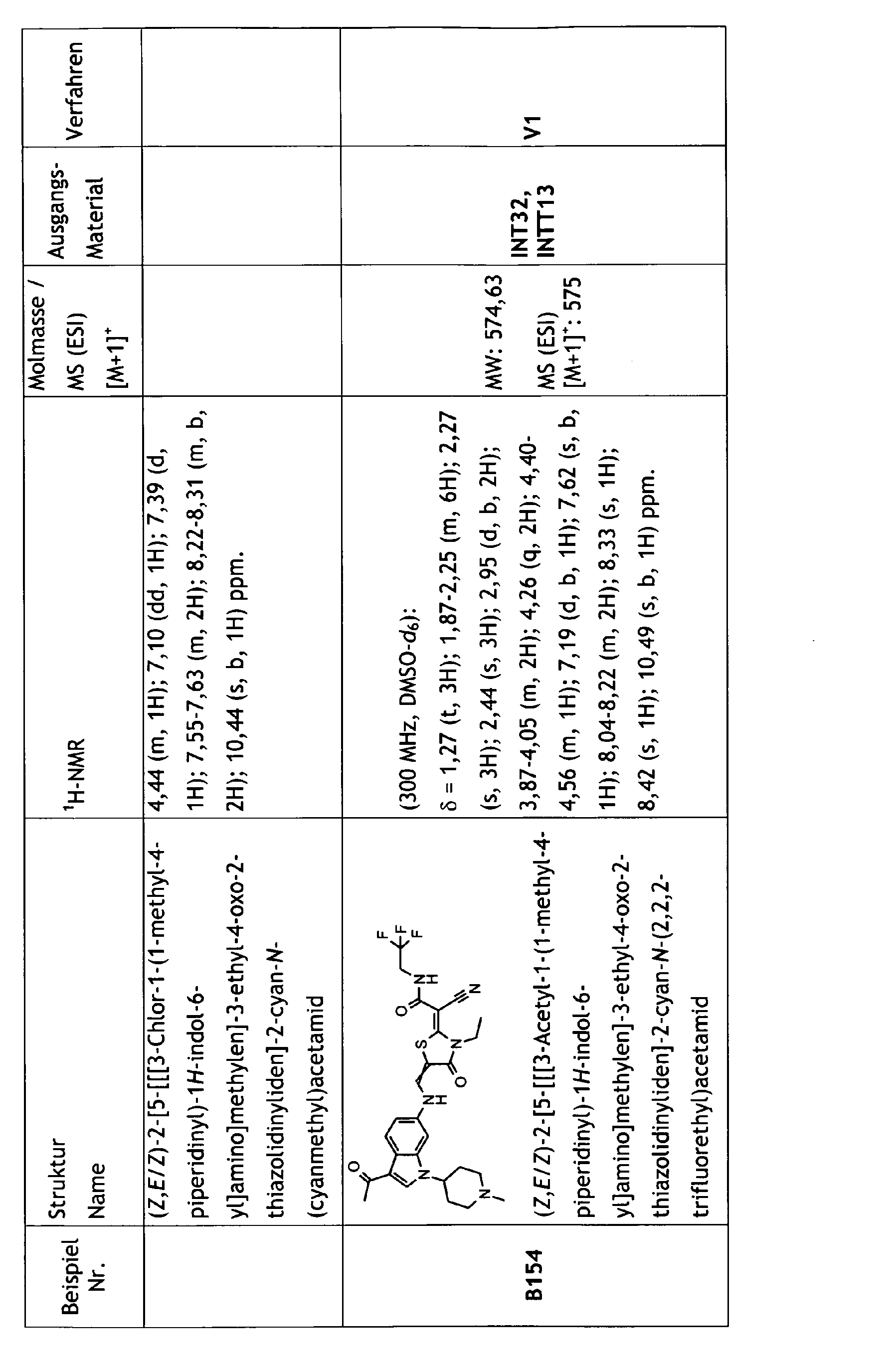

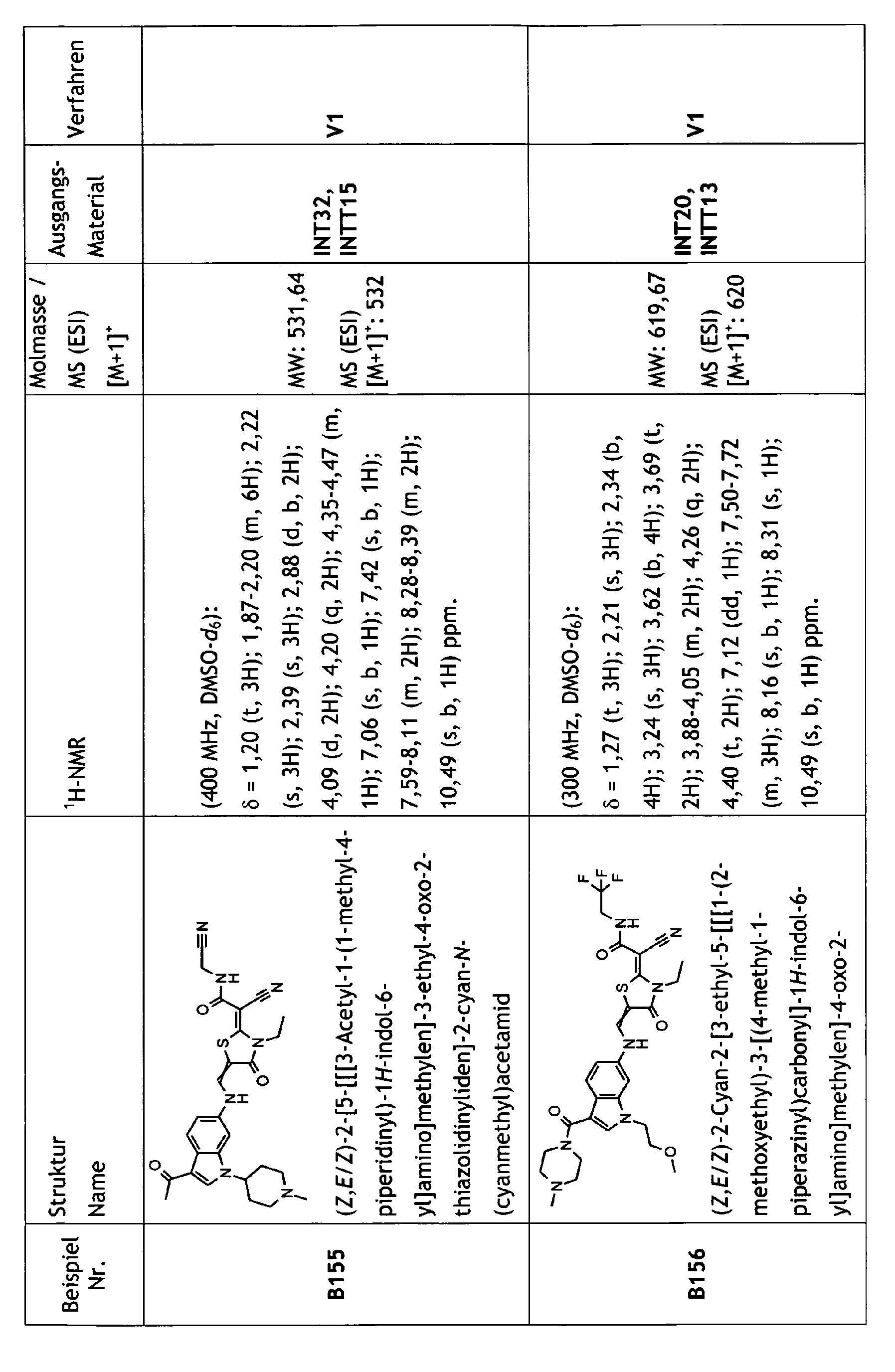

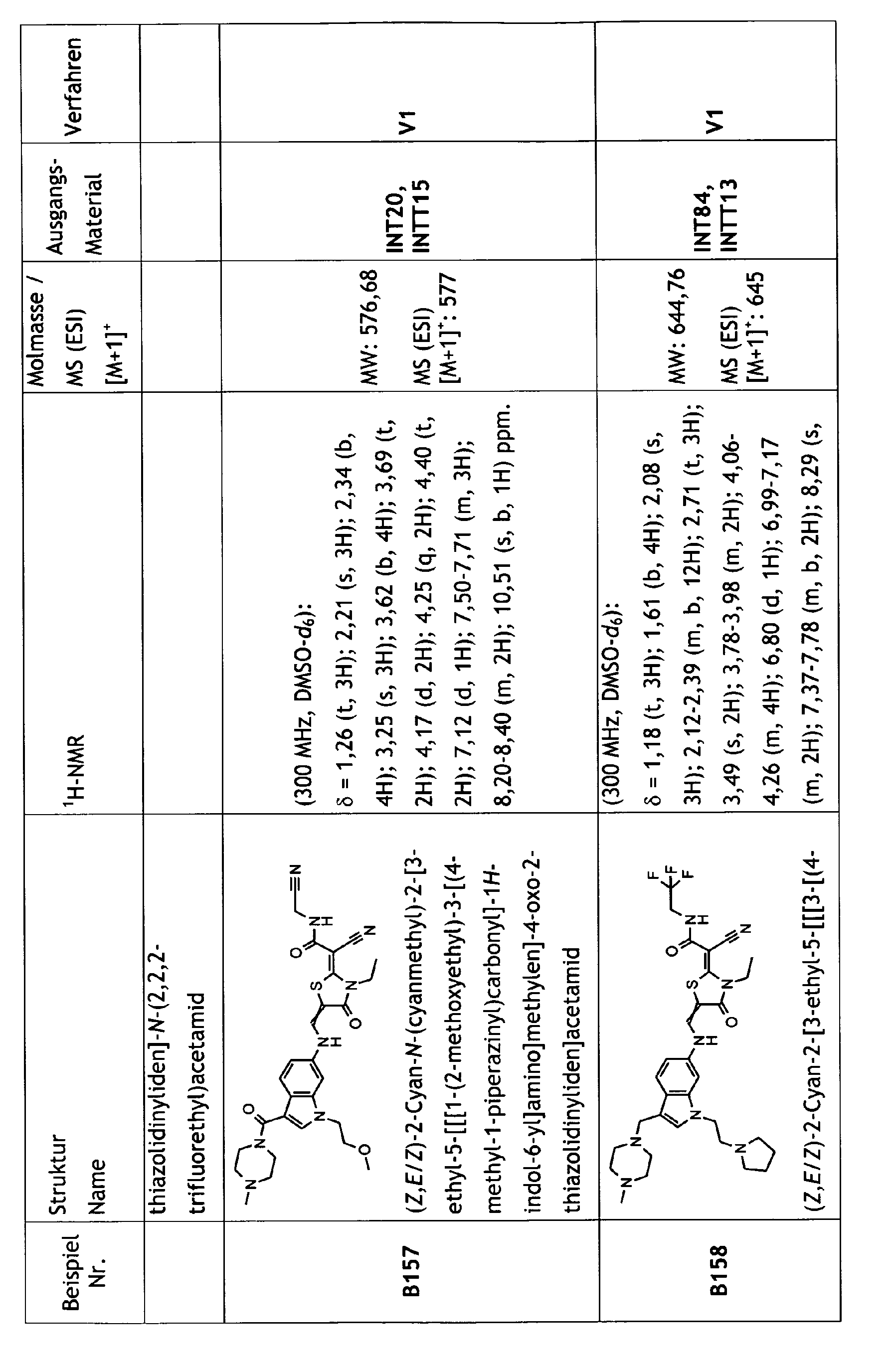

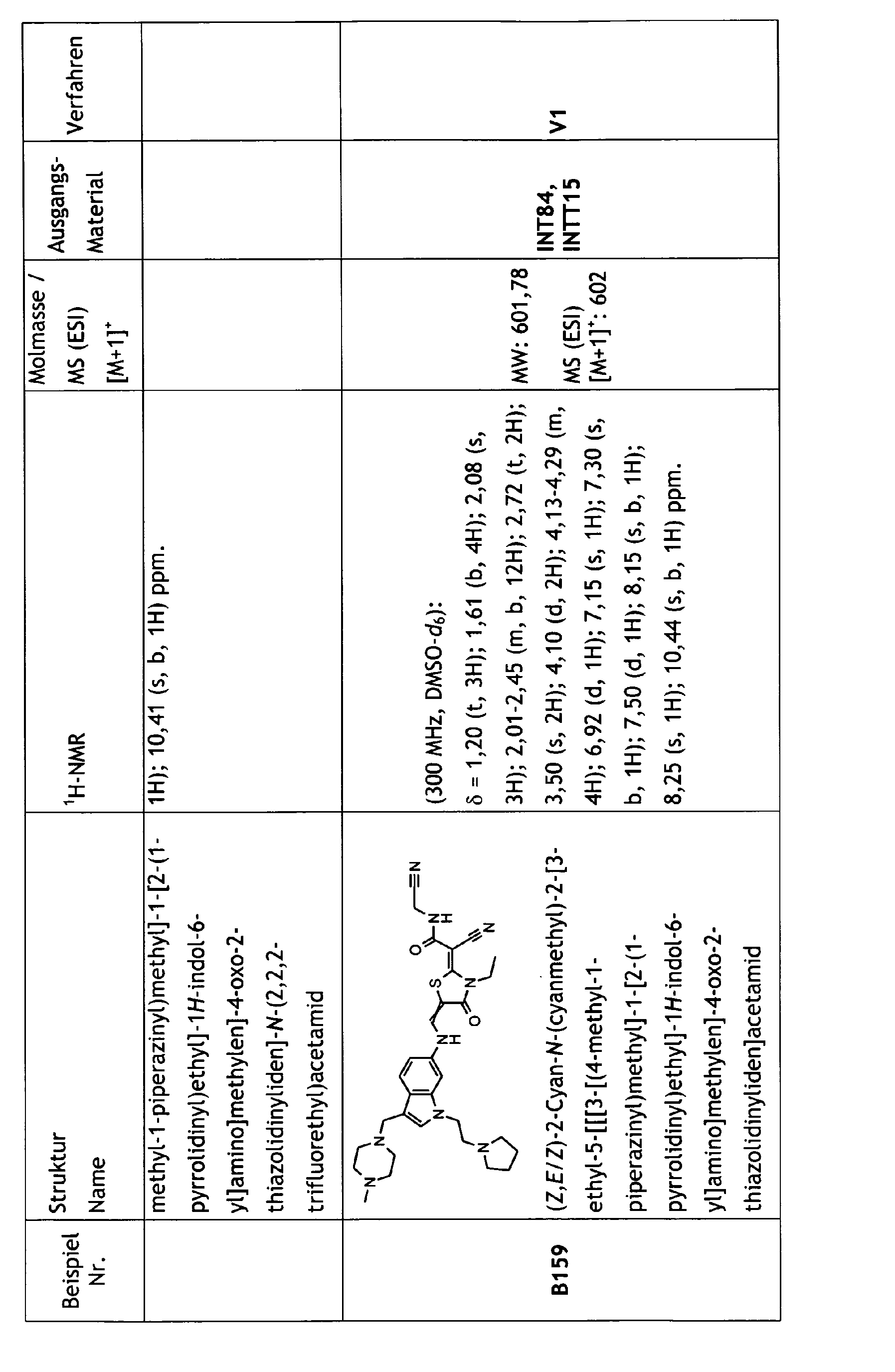

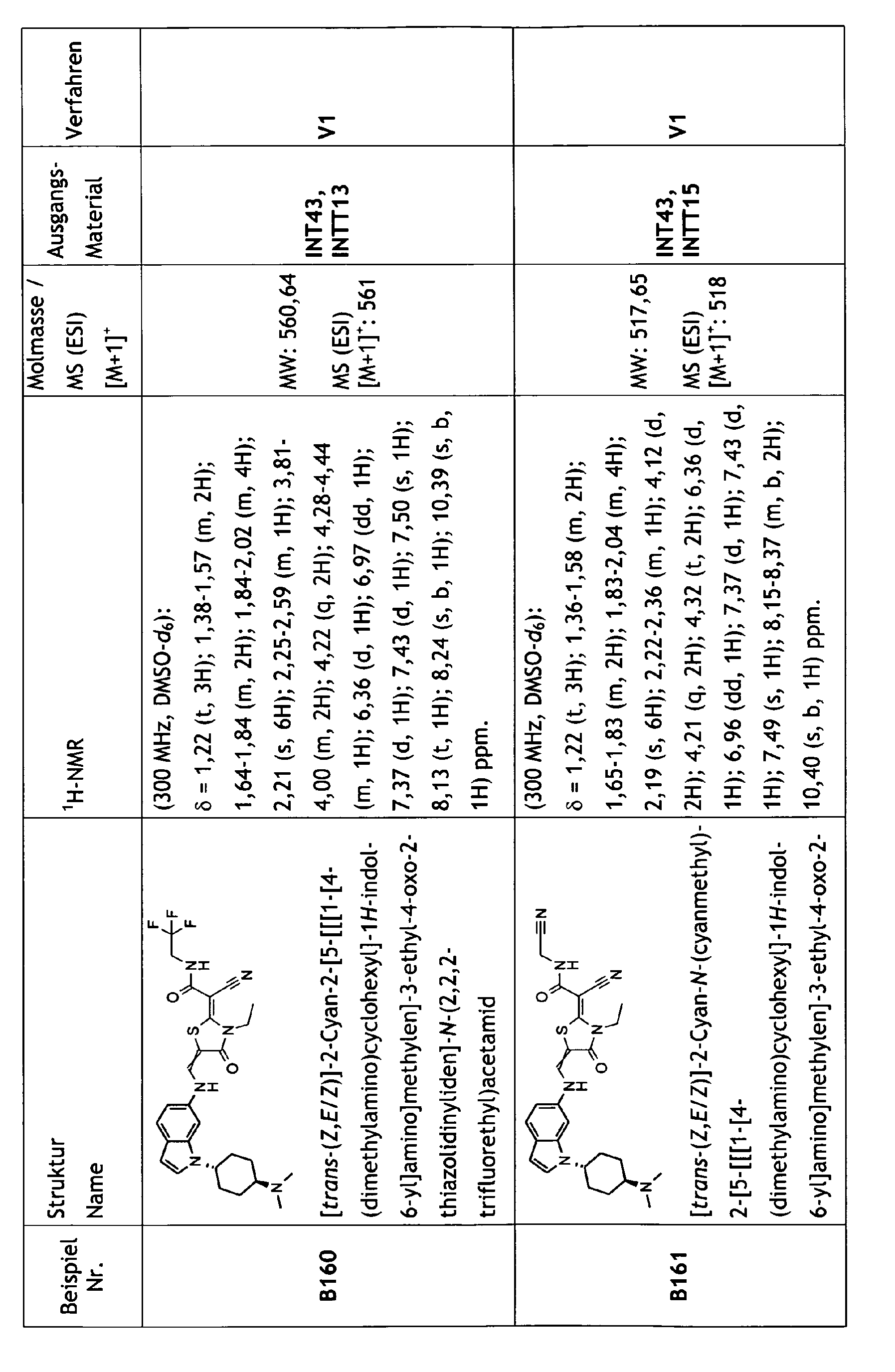

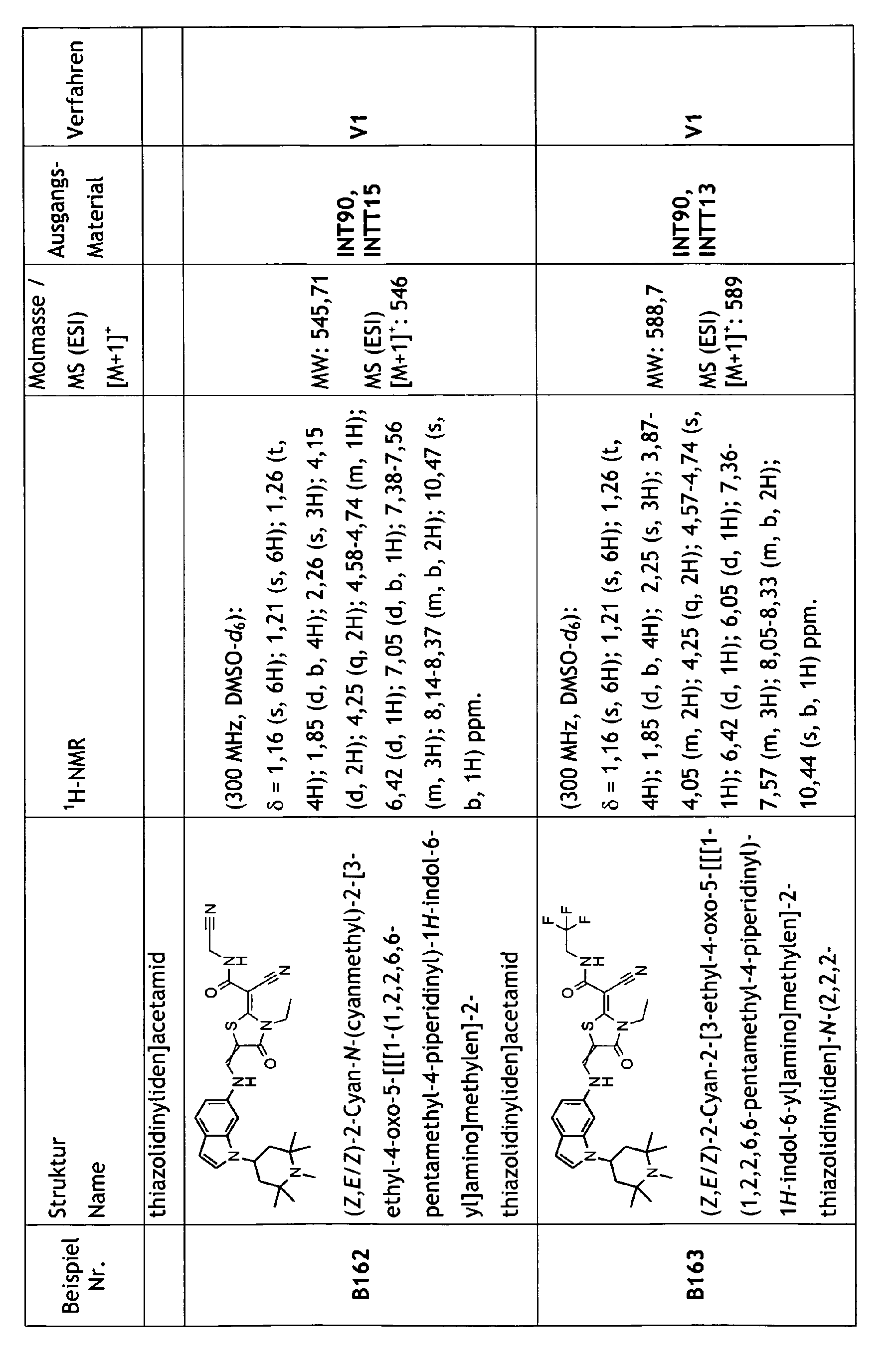

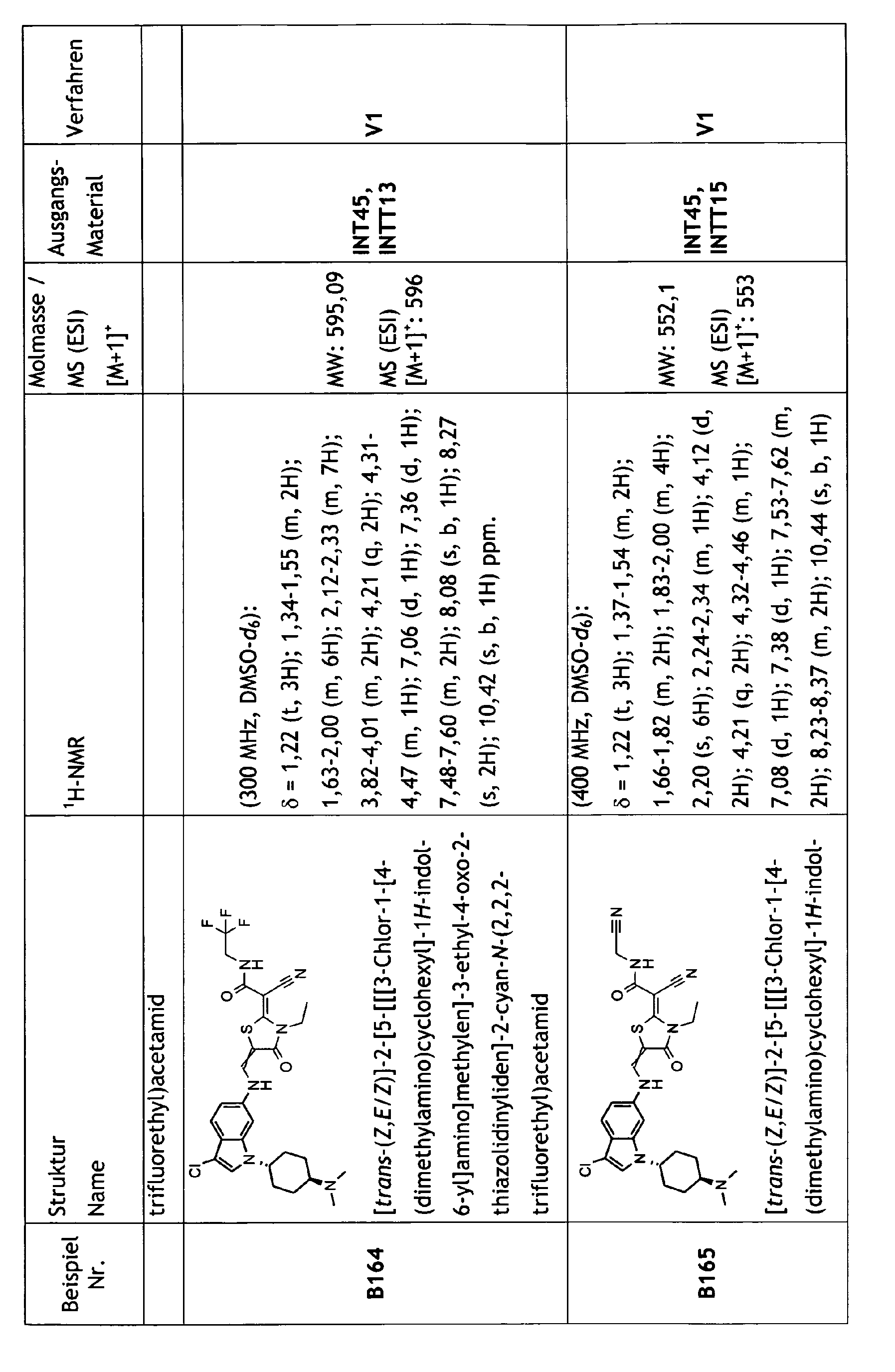

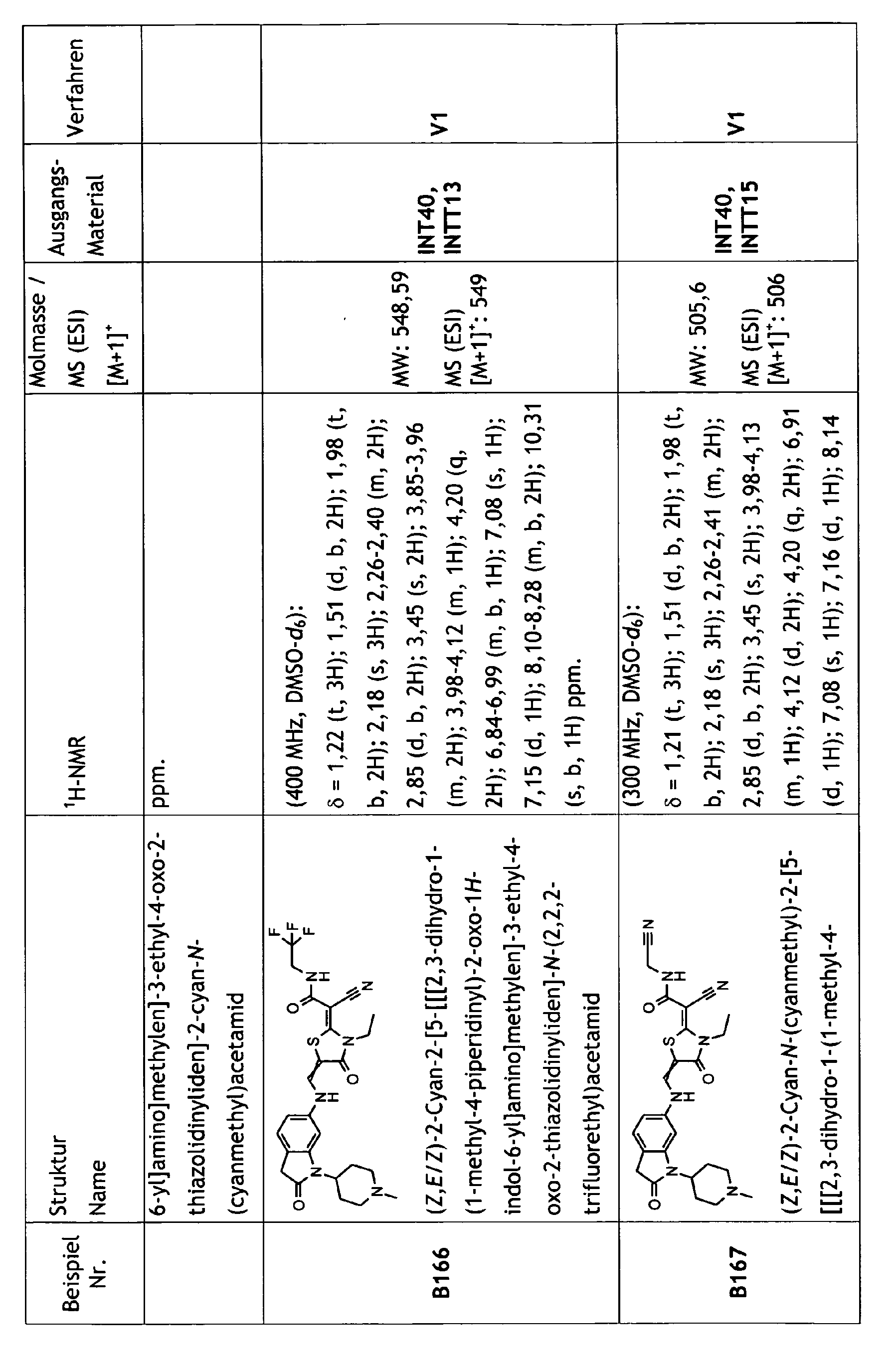

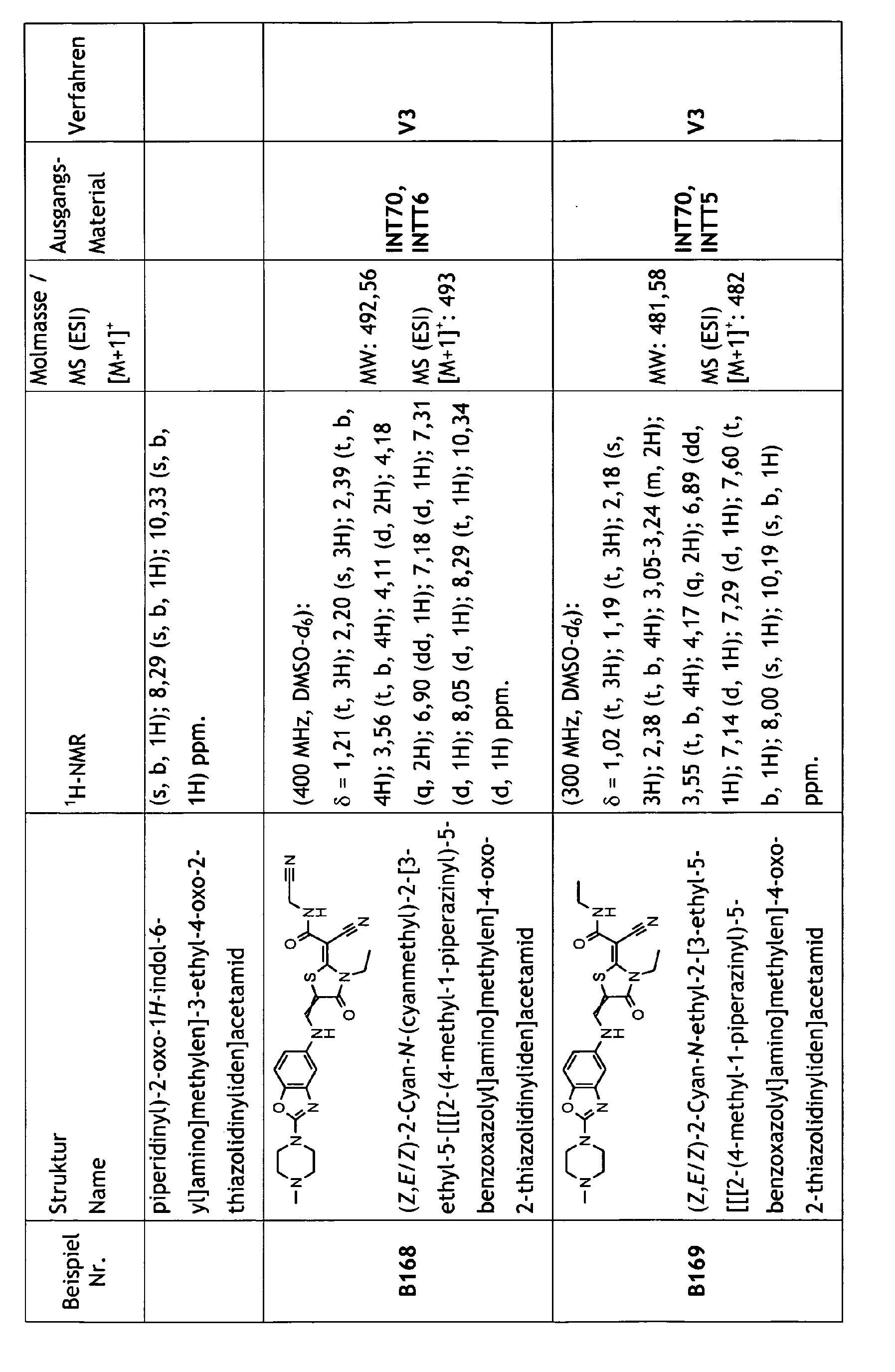

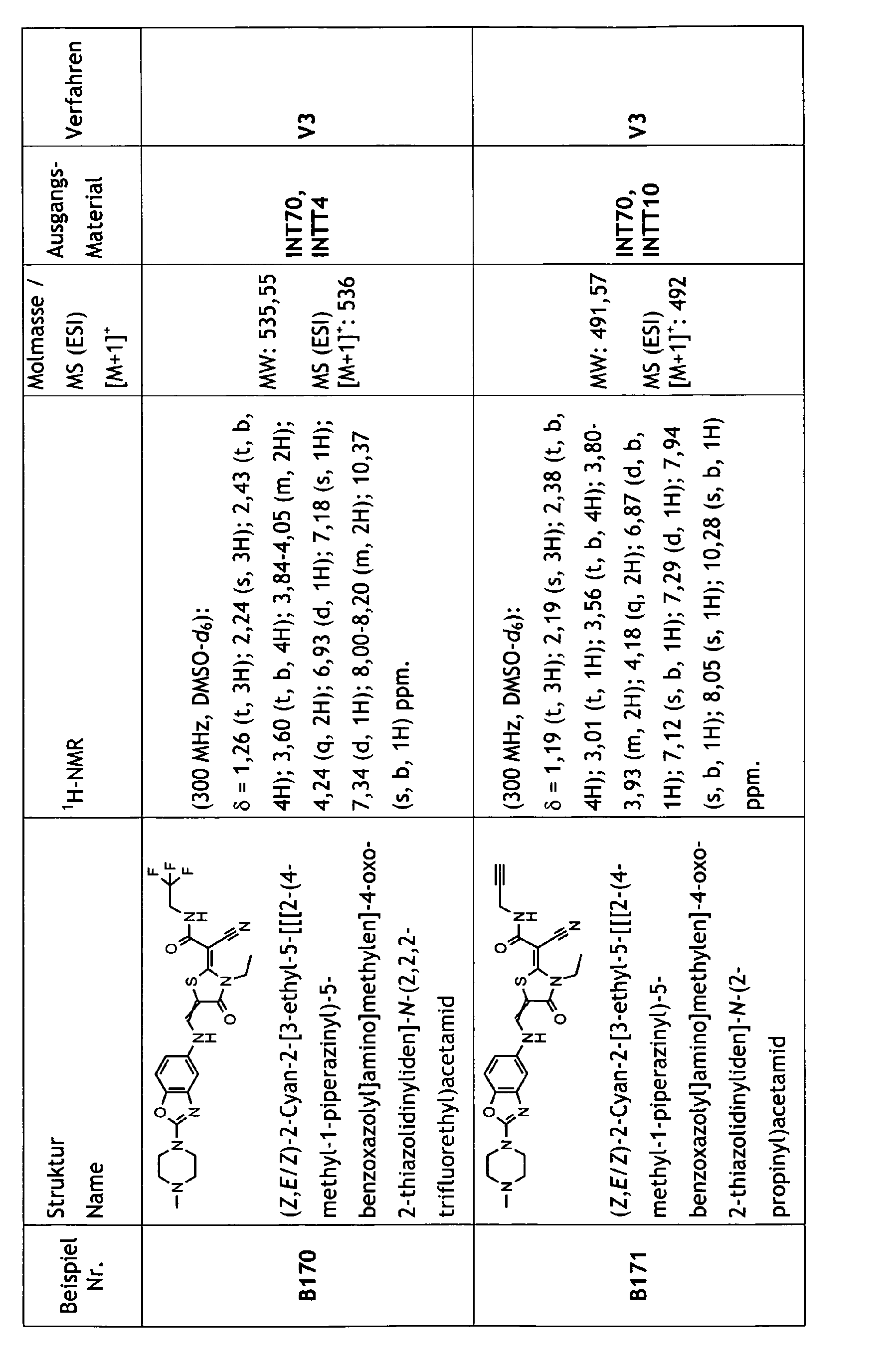

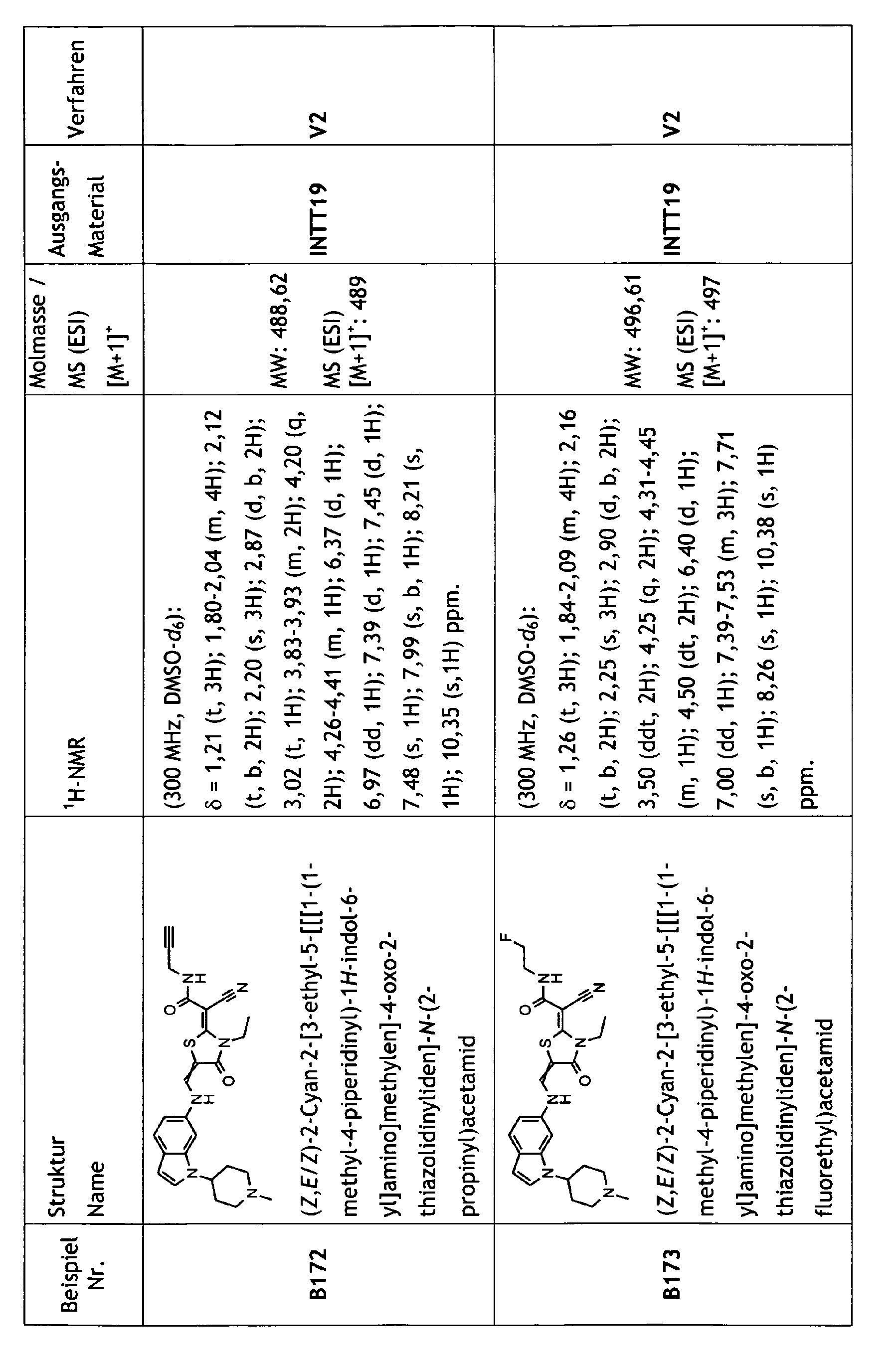

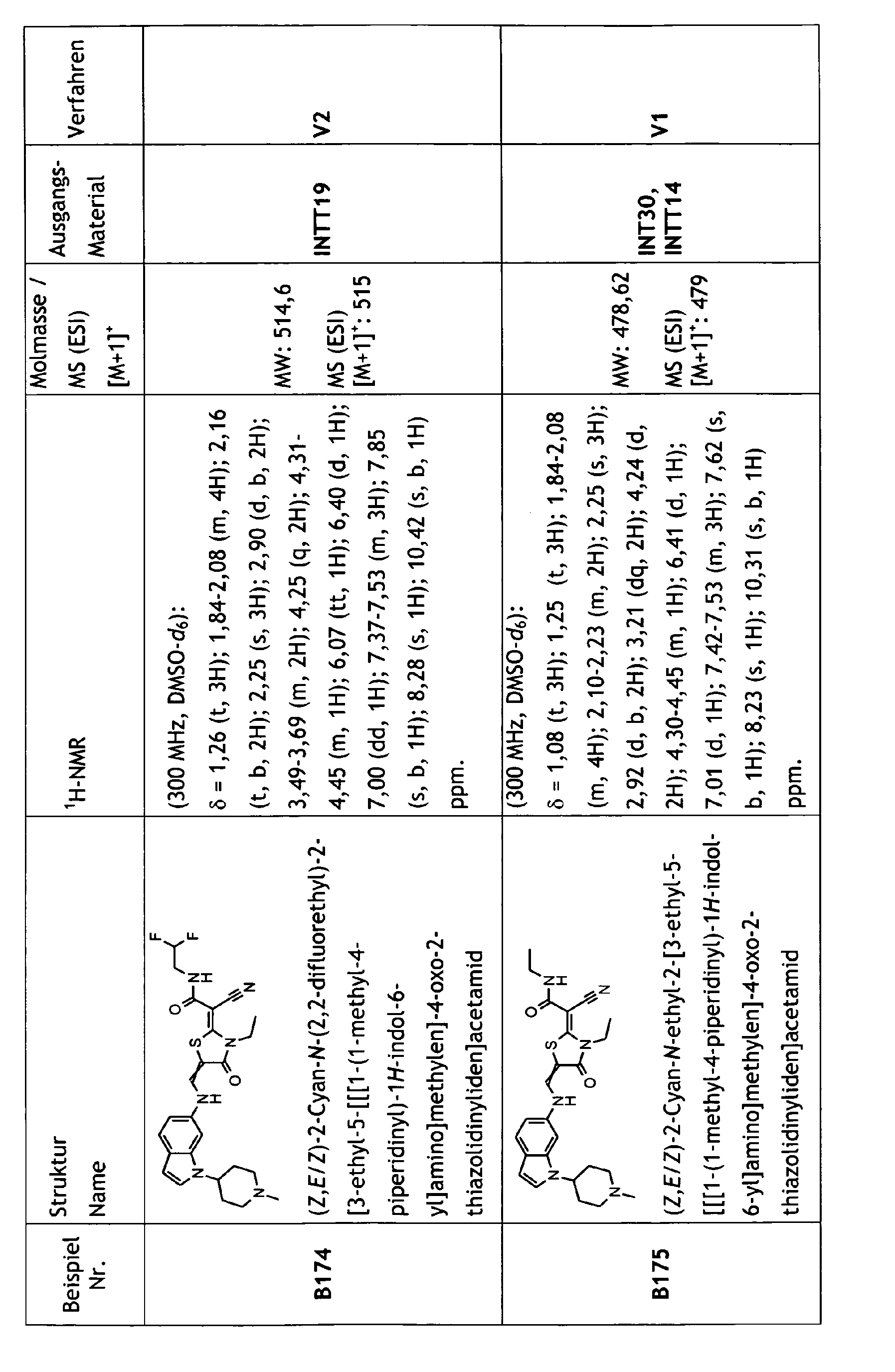

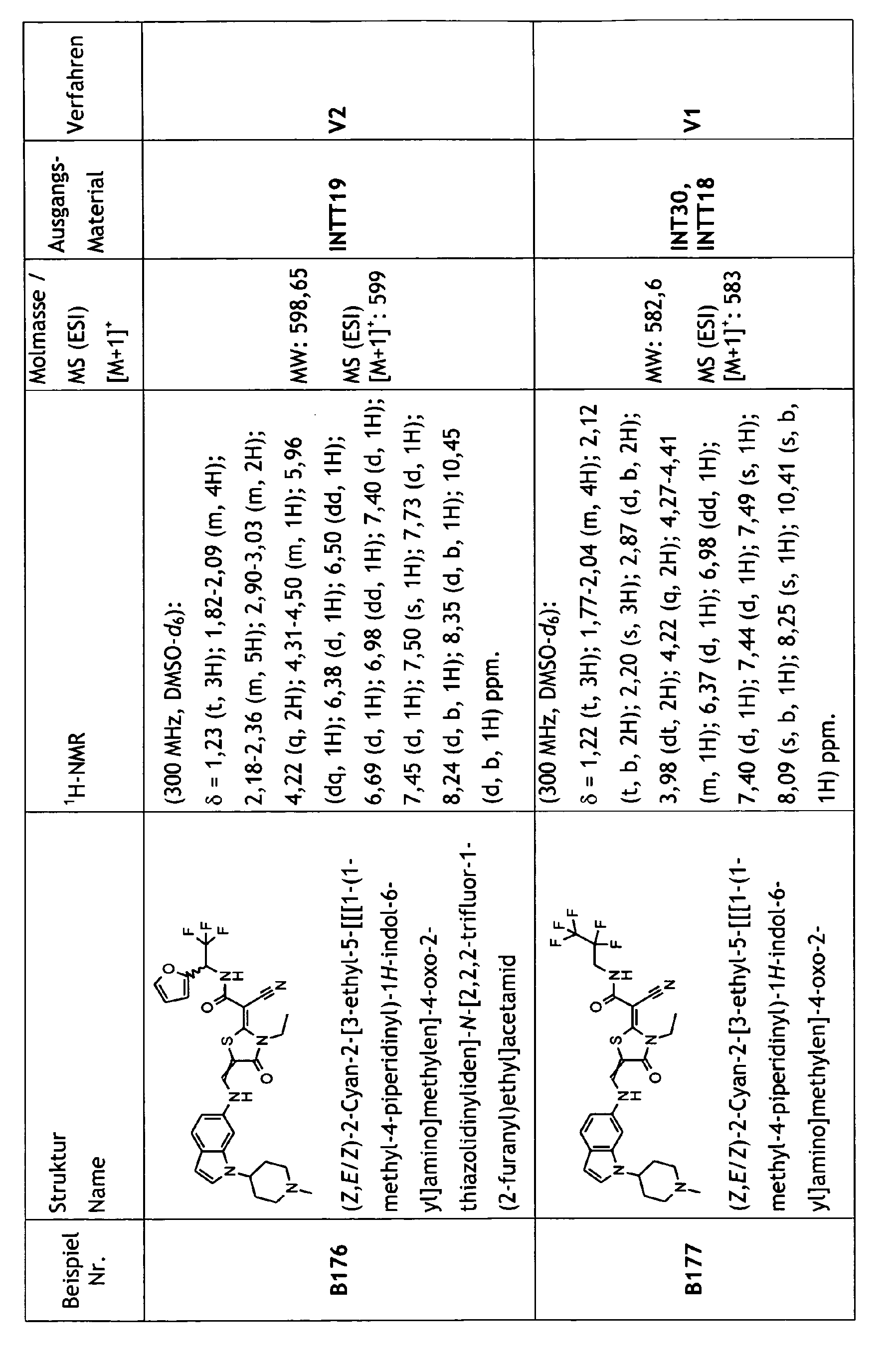

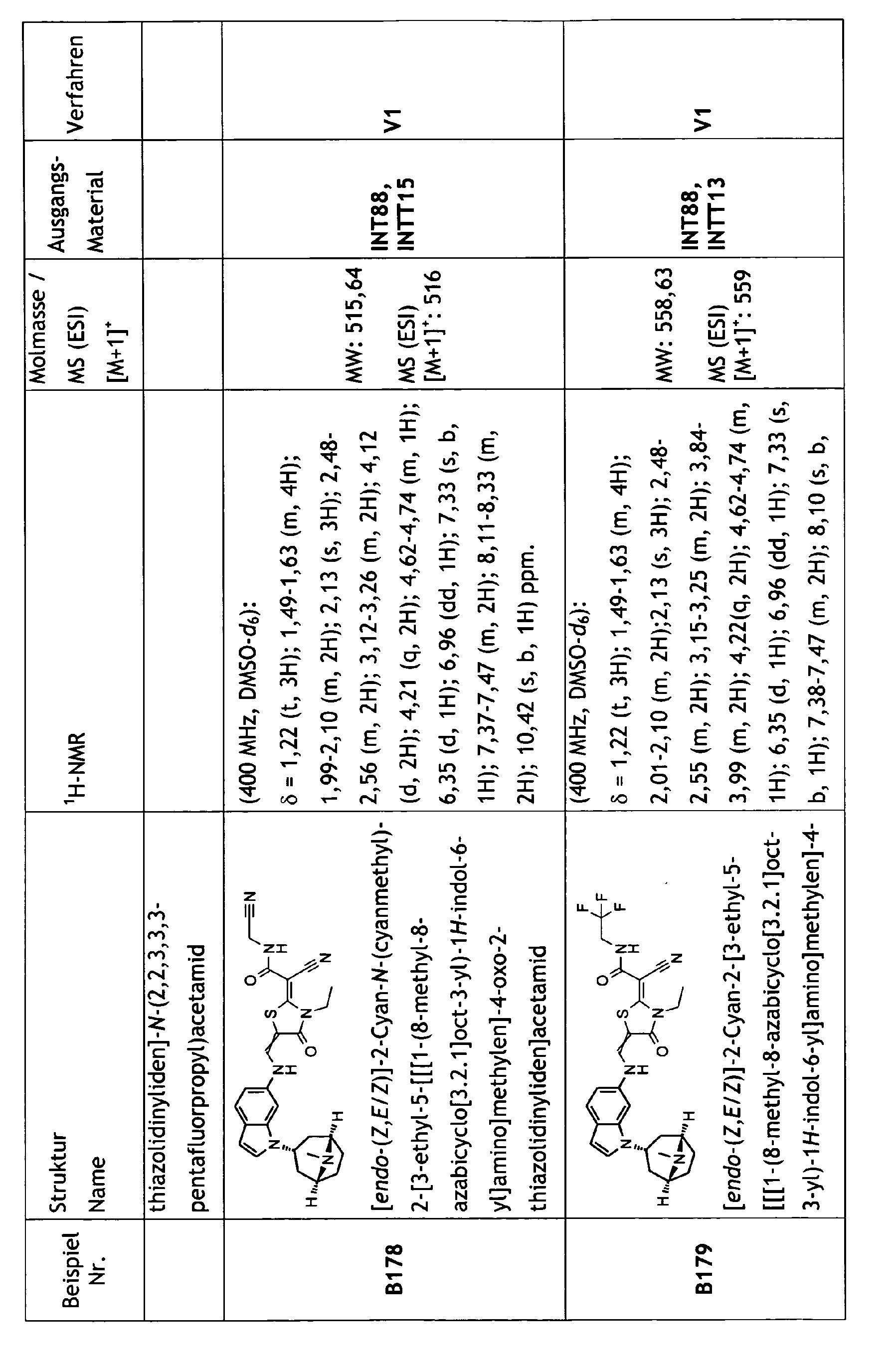

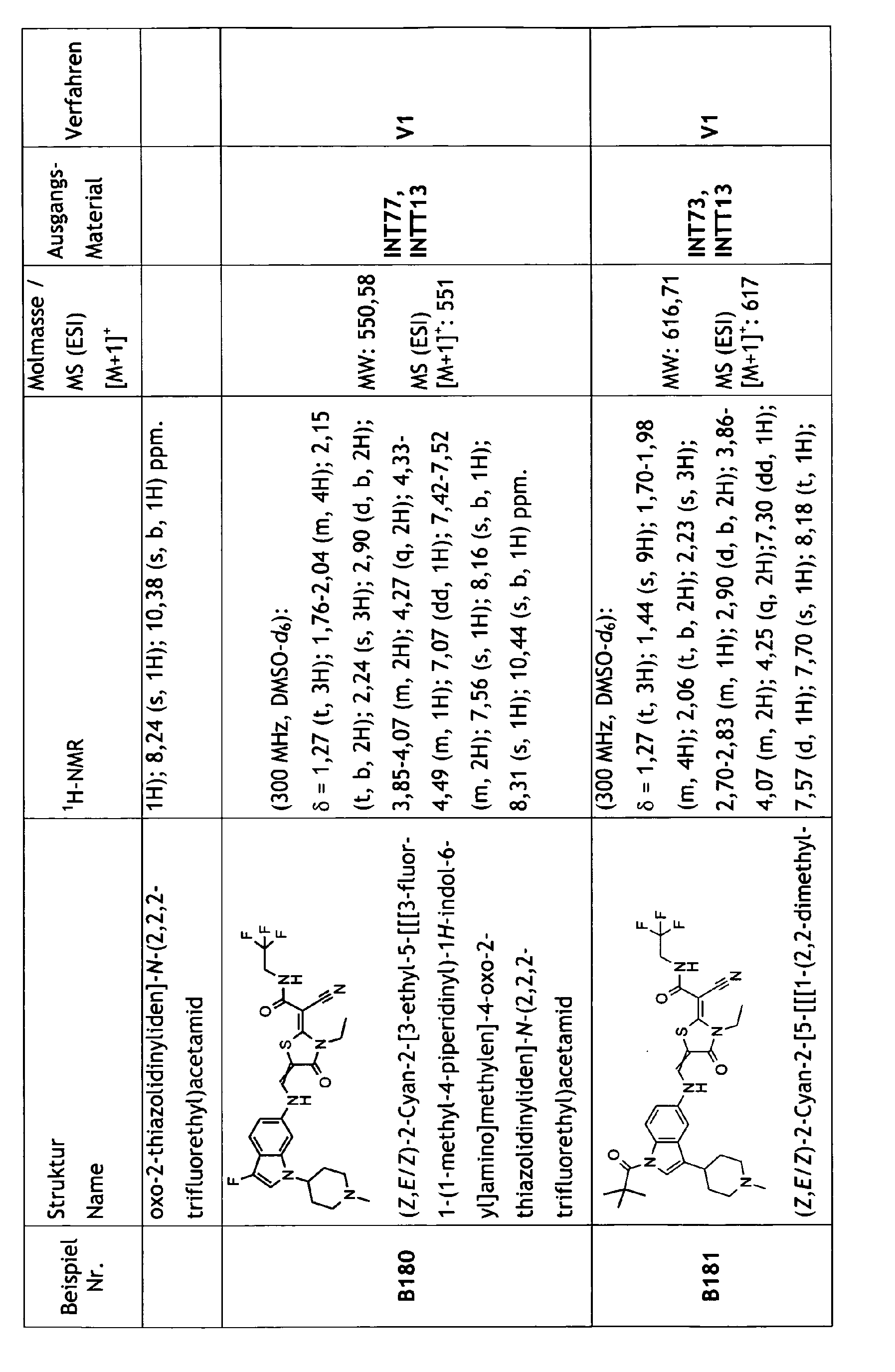

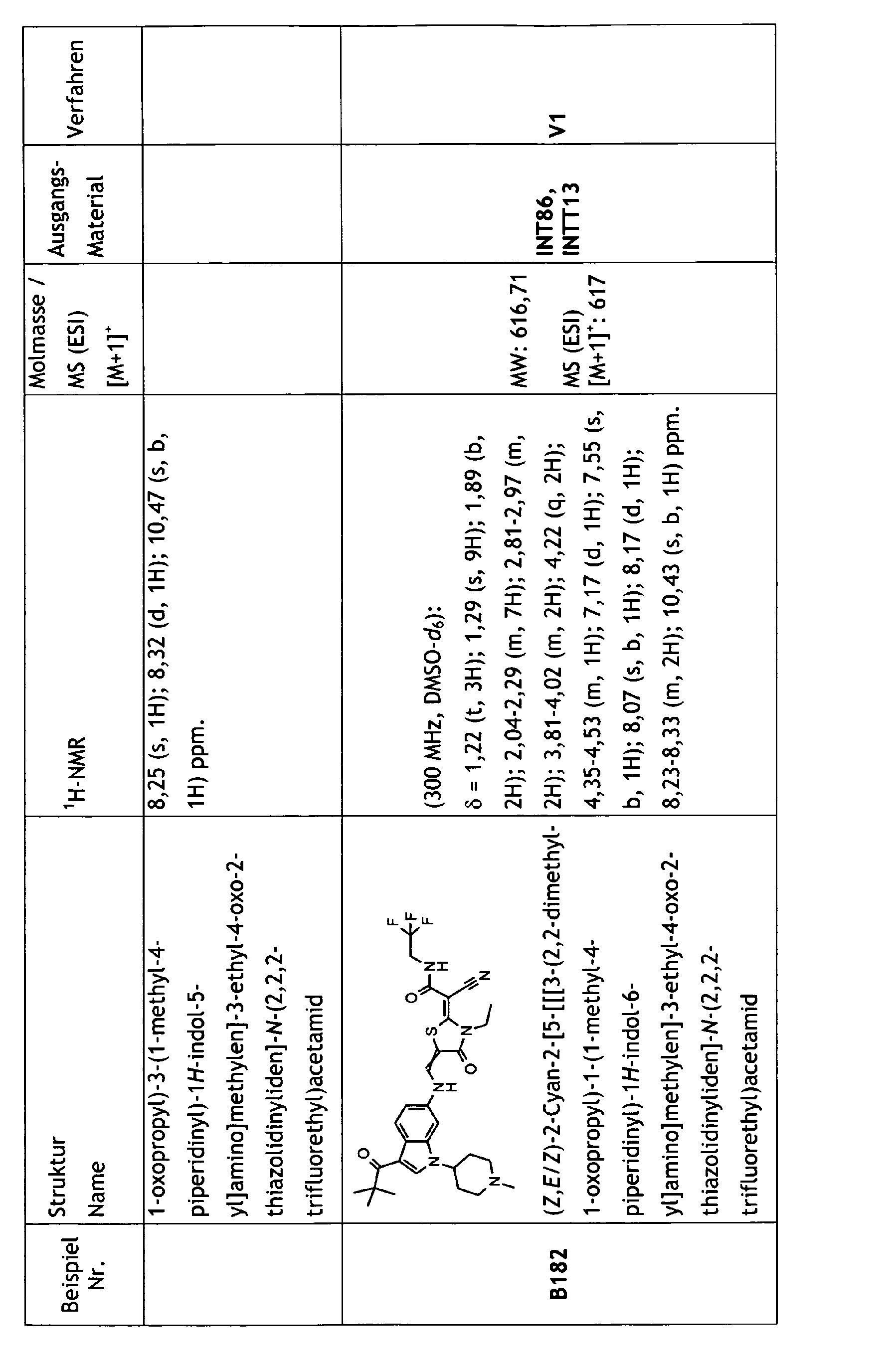

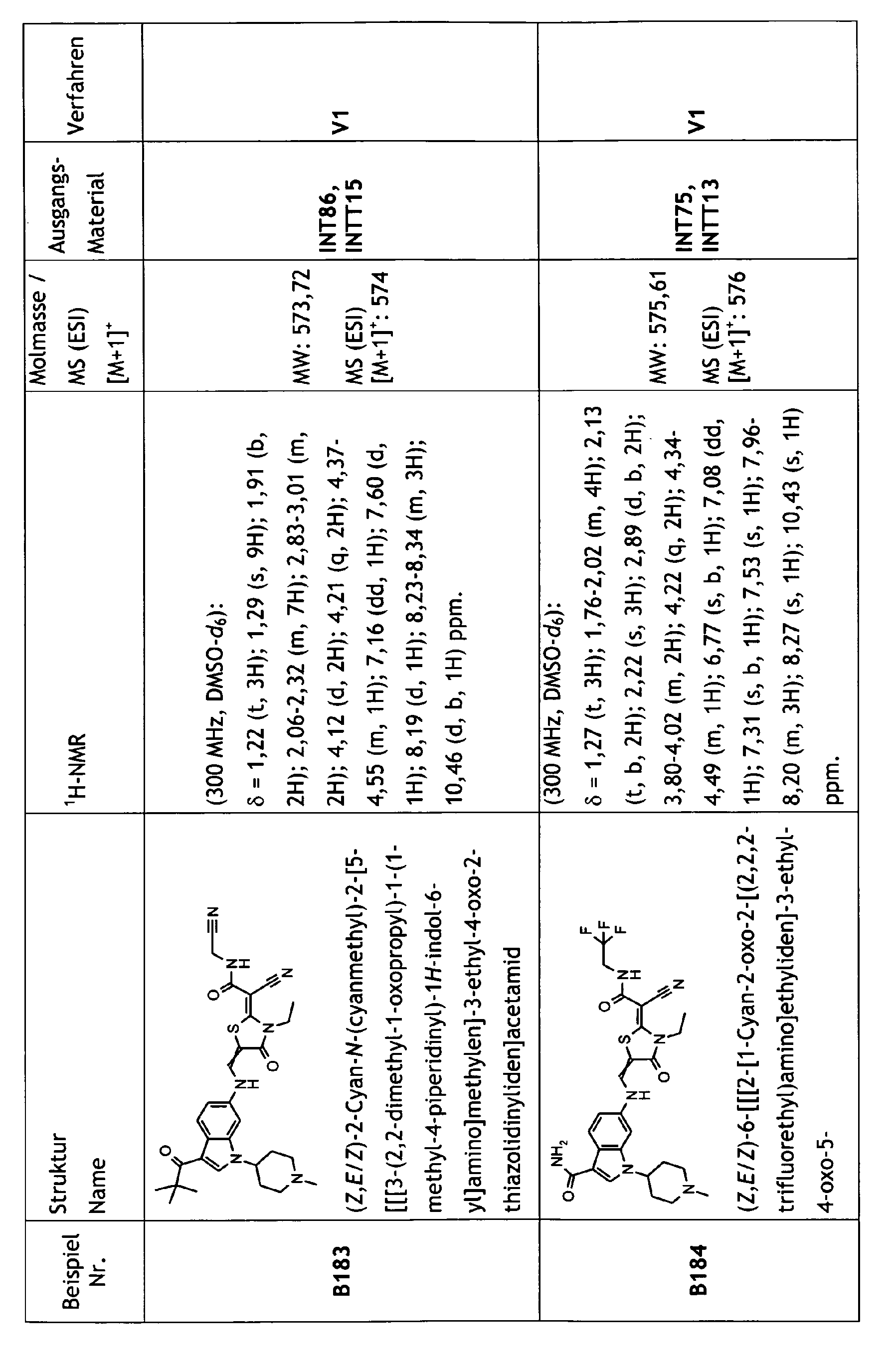

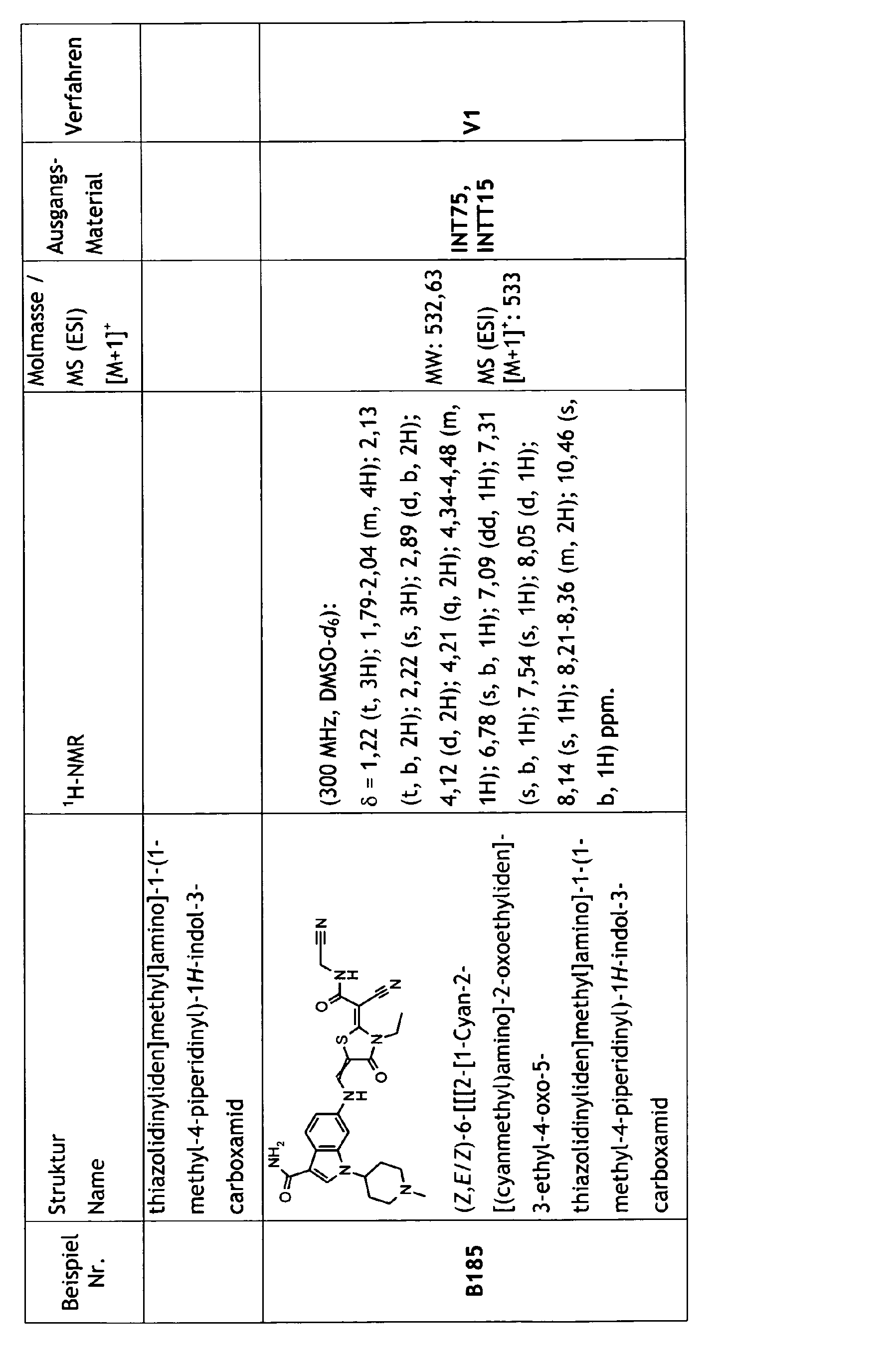

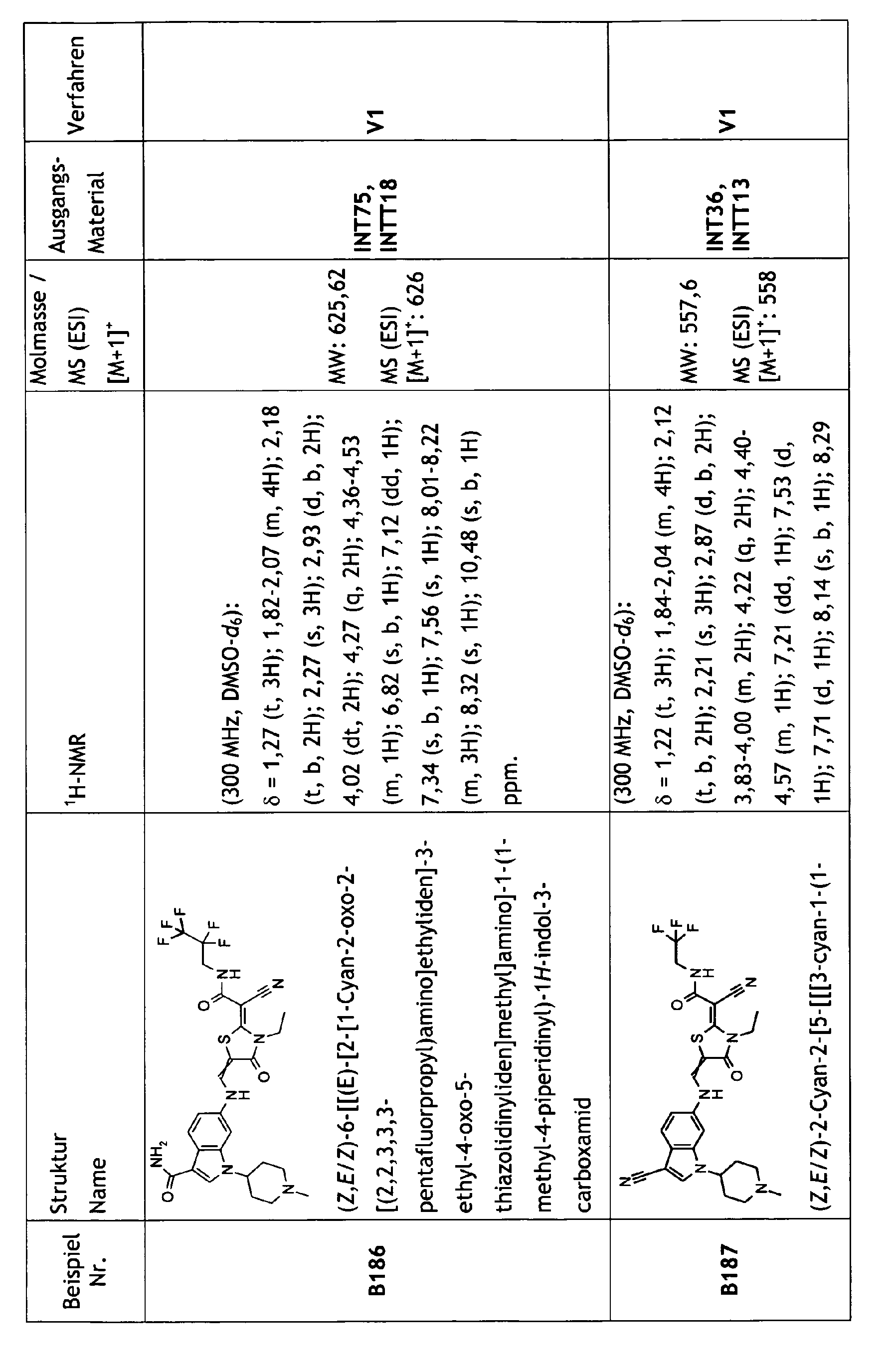

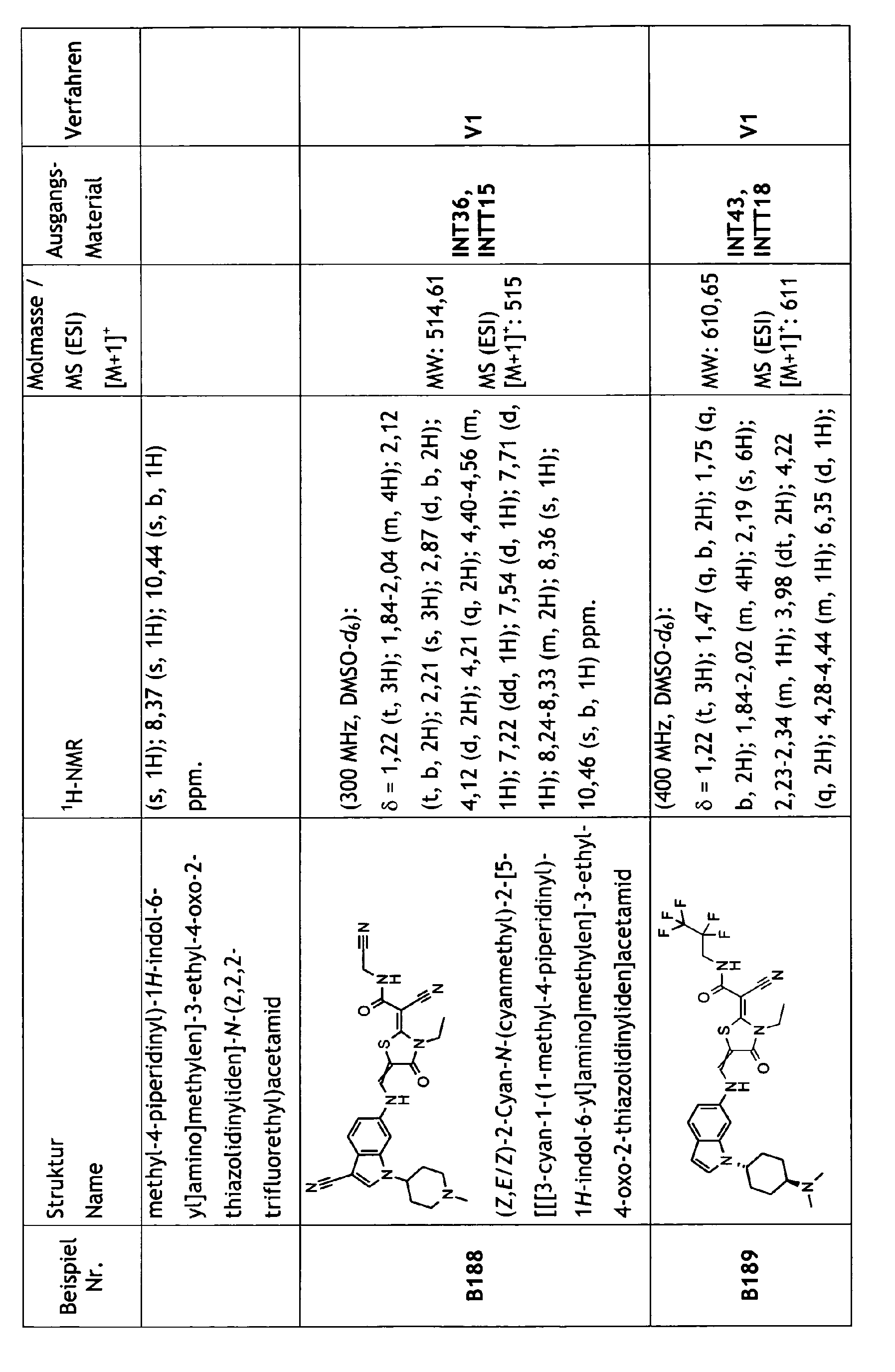

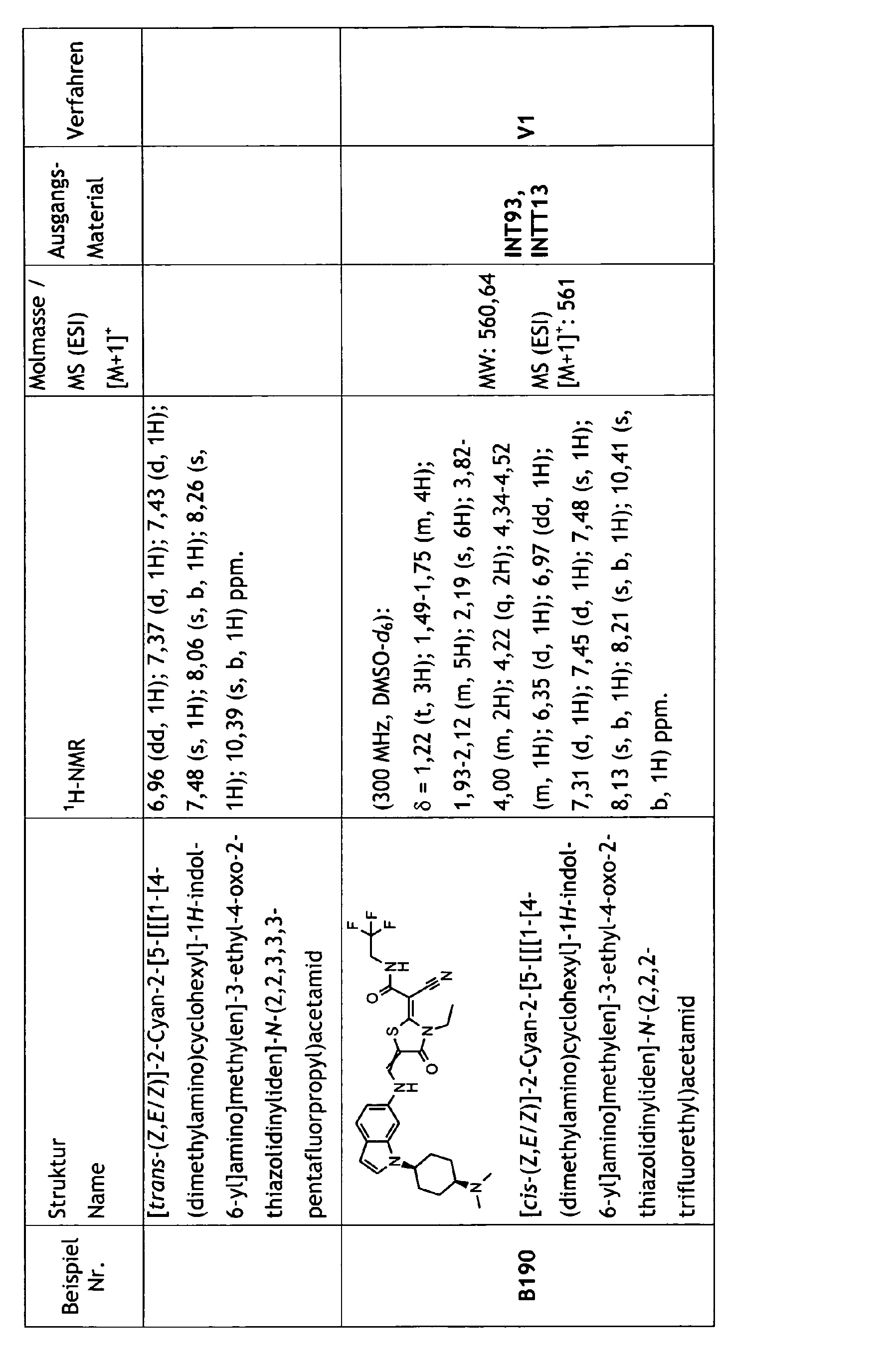

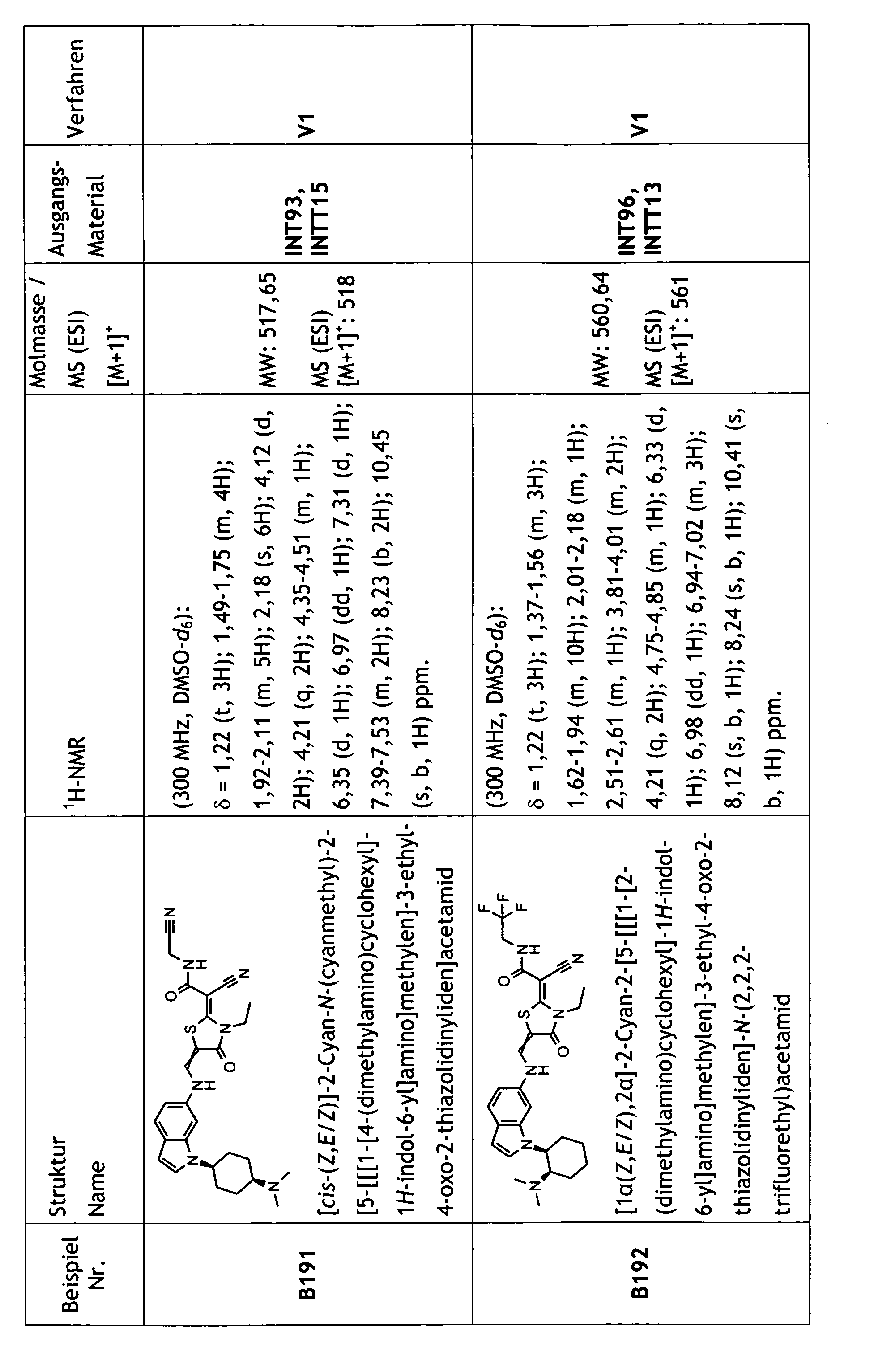

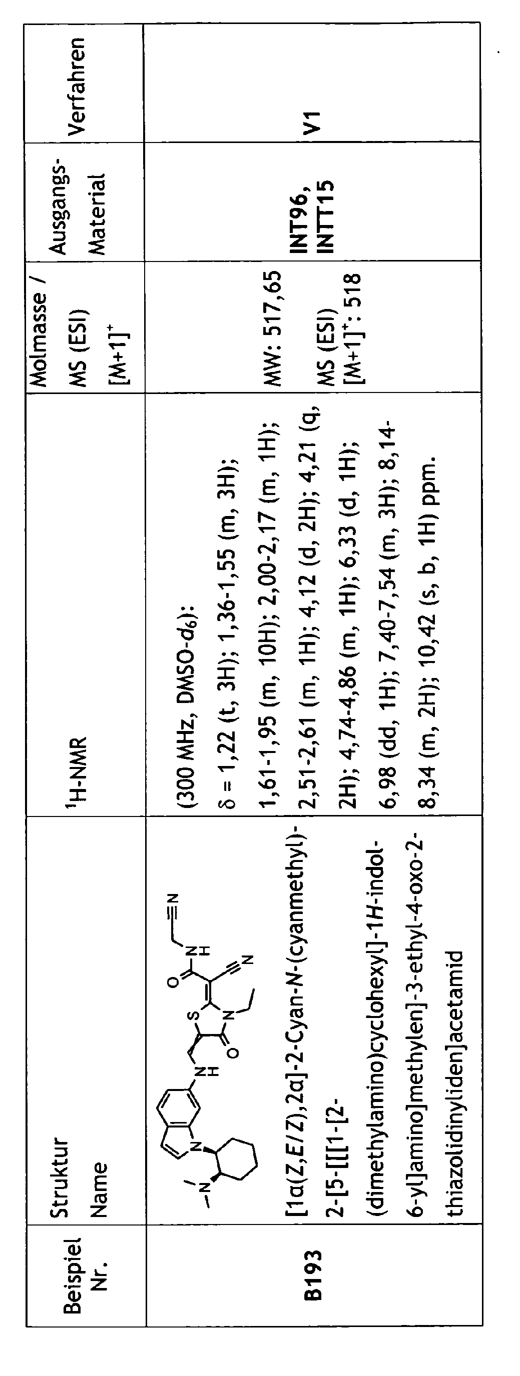

Die nachfolgenden Beispiele beschreiben die biologische Wirkung der erfindungsgemäßen Verbindungen:

### PLK Enzym-Assay

Rekombinantes humanes Plk-1 (6xHis) wird aus Bakulovirus-infizierten Insektenzetten (Hi5) gereinigt.

10 ng (rekombinant hergestelltes, gereinigtes) PLK Enzym wird für 90 min bei Raumtemperatur mit biotinyliertem Casein und 33P-γ-ATP als Substrat in einem Volumen von 15 µl in 384well Greiner Small Volume Microtiterplatten inkubiert (Endkonzentrationen im Puffer: 660 ng/ml PLK; 0,7 µM Casein; 0,5 µM ATP inkl. 400 nCi/ml 33P-γ-ATP; 10 mM MgCl₂, 1 mM MnCl₂; 0,01% NP40; 1 mM DTT, Proteaseinhibitoren; 0,1 mM Na₂VO₃ in 50 mM HEPES pH 7,5). Zum Beenden der Reaktion wird 5 µl Stoplösung (500 µM ATP; 500 mM EDTA; 1% Triton X100; 100 mg/ml Streptavidin coated SPA Beads in PBS) zugesetzt. Nach Verschließen der Microtiterplatte durch Folie werden die Beads durch Zentrifugation (10 min, 1500 rpm) sedimentiert. Der Einbau von 33P-γ-ATP in Casein wird als Maß der Enzymaktivität durch β-Counting bestimmt. Das Maß der Inhibitoraktivität wird gegen eine Lösungsmittelkontrolle (= ungehemmte Enzymaktivität = 0% Inhibition) und den Mittelwert von mehreren Ansätzen die 300 µM Wortmannin enthalten (= voll gehemmte Enzymaktivität = 100% Inhibition) referenziert.
Testsubstanzen werden in verschiedenen Konzentrationen (0 µM, sowie im Bereich 0,01 - 30 µM) eingesetzt. Die finale Konzentration des Lösungsmittels Dimethylsulfoxid beträgt in allen Ansätzen 1,5%.

### PLK Enzym-Assay (10 mM ATP)

Für den Kinaseassay wird aus Insektenzellen (Hi5) exprimiertes, durch Glutathion-Sepharose-Affinitätschromatographie und anschließender Gel-filtration (Suerdex 75) gereinigtes rekombinantes Fusionsprotein bestehend aus GST und Plk (Kinase Domäne 33-345; MW 36 kDa, conc 0,8 µg/µl) verwendet. Aliqots hiervon werden in flüssigem Stickstoff weggefroren und bei -80°C gelagert und nach dem Auftauen nur einmal verwendet.

Bei dem verwendeten Assay handelt es sich um einen indirekten HTRF-Assay, bei dem folgende Materialien und Verfahrensweisen zum Einsatz kamen.
Als Substrat für die Kinasereaktion wird das biotinylierte Peptid Btn-Ahx-KKLNRTLSFAEPG-Amid x TFA, von Biosyntan, Probennr.: 6178.1 (C-Terminus in Amidform) verwendet. Hierbei handelt es sich um eine artifizielle Sequenz, die von keinem bekannten Protein abgeleitet wurde.

50 nl der in 100% Dimethylsulfoxid (DMSO) gelösten Testverbindungen (Endkonzentrationen : 0 µM und Konzentrationen im Bereich von 0,001 - 20 µM) werden mit 2 µl Enzym Arbeitslösung (0,039 ng/µl Plk-1 im Arbeitspuffer [25 mM MgCl₂; 1mM DTT; 50 mM Hepes pH 7,0; 0,01% NP40; 1x Complete; 0,05% BSA] für 30 min vorinkubiert. Anschließend wird die Kinase-Reaktion durch Zugabe von 3 µl Substratlösung [16,7 mM Adenosintriphosphate (ATP) und 1,4 µM Substratpeptid (Biotin-Ttds- KKLNRTLSFAEPG -NH₂)] in Arbeitspuffer gestartet, und nach 30 min durch Zugabe einer Abstopp-Lösung (100 mM EDTA, 100 mM Hepes pH 7,5, 800 mM Potassiumfluoride, 0,12% BSA, 0,4 µM SA-XLent (0,05 µM, von CIS bio international, Marcoule, Frankreich), Eu³⁺ Cryptate-conjugated Rabbit anti-Mouse IgG (1,5 nM; ein mit Europiumkryptat markierter Anti-Maus-IgG-Antikörper von CIS bio international, Marcoule, Frankreich), 1 nM Anti-phospho-Serine Kinase (ein phospho-spezifischer Antikörper von Upstate Biotechnology, Dundee, Schottland), gestoppt, und bei 4°C über Nacht inkubiert. Danach wird die Menge des phosphorylierten Substratpeptids durch Messung des Resonanzenergietransfers vom Europium-markierten Antikörperkomplex zum Streptavidine-XLent bestimmt. Zu diesem Zweck wird die Fluoreszenzemission bei 620 nm und 665 nm nach Anregung bei 350 nm in einem HTRF-Messgerät, z. B. Rubystar (BMG Labtechnologies, Offenburg, Deutschland) oder Viewlux (Perkin-Elmer), bestimmt. Das Verhältnis der Emissionen bei 665 nm und bei 620 nm wird als Maß für die Menge des phosphorylierten Substratpeptids verwendet. Die Daten werden normalisiert (Enzymreaktion ohne Inhibitor = 0 % Inhibition, alle anderen Assaykomponenten, aber kein Enzym = 100 % Inhibition), und IC₅₀-Werte werden durch einen 4 Parameter-Fit mit einer hauseigenen Software berechnet.

### Proliferationsassay

Kultivierte humane Tumorzellen (MCF7, hormonunabhängige menschliche Mammakarzinomazellen, bezogen von ATCC HTB22; NCI-H460, menschliche nicht kleinzellige Lungenkarzinomazellen, ATCC HTB-177; DU 145, hormonunabhängige menschliche Prostatakarzinomazellen, ATCC HTB-81; HeLa-MaTu, humane Cervixkarzinomazellen, bezogen von EPO-GmbH, Berlin,) werden in einer Dichte von 5000 (MCF7, DU145) bzw. 3000 (NCI-H460, HeLa-MaTu) Zellen/Messpunkt in einer 96-Loch Multititerplatte in 200 µl des entsprechenden Wachstumsmediums ausplattiert. Nach 24 Stunden werden die Zellen einer Platte (Nullpunkt-Platte) mit Kristallviolett gefärbt (s.u.), während das Medium der anderen Platten durch frisches Kulturmedium (200 µl), dem die Testsubstanzen in verschiedenen Konzentrationen (0 µM, sowie im Bereich 0,01 - 30 µM; die finale Konzentration des Lösungsmittels Dimethylsulfoxid beträgt 0,5%) zugesetzt sind, ersetzt. Die Zellen werden für 4 Tage in Anwesenheit der Testsubstanzen inkubiert. Die Zellproliferation wird durch Färbung der Zellen mit Kristallviolett bestimmt: Die Zellen werden durch Zugabe von 20 µl/Messpunkt einer 11%igen Glutaraldehyd-Lösung 15 min bei Raumtemperatur fixiert. Nach dreimaligem Waschen der fixierten Zellen mit Wasser werden die Platten bei Raumtemperatur getrocknet. Die Zellen werden durch Zugabe von 100 µl/Messpunkt einer 0,1%igen Kristallviolett-Lösung (pH durch Zugabe von Essigsäure auf pH3 eingestellt) gefärbt. Nach dreimaligem Waschen der gefärbten Zellen mit Wasser werden die Platten bei Raumtemperatur getrocknet. Der Farbstoff wird durch Zugabe von 100 µl/Messpunkt einer 10%igen Essigsäure-Lösung gelöst. Die Extinktion wird photometrisch bei einer Wellenlänge von 595 nm bestimmt. Die prozentuale Änderung des Zellwachstums wird durch Normalisierung der Messwerte auf die Extinktionwerte der Nullpunktplatte (=0%) und die Extinktion der unbehandelten (0 µM) Zellen (=100%) berechnet. IC₅₀-Werte werden nach einer 4-Parameterkurvenanpassung mit Hilfe eines Firmeneigenen Computerprogramms berechnet.

Die Ergebnisse des PLK-1 Enzym-Assays bei einer ATP-Konzentration von 10 mM und des Proliferations-Assays sind in den nachfolgenden Tabellen aufgeführt :

**Tabelle 1**

| Ergebnisse des PLK-1 Enzym-Assays bei 10 mM ATP: | | |
|---|---|---|
| Beispiel Nr. | Struktur | Inhibition PLK-1 IC₅₀ [M] (10 mM ATP) |
| **B01** | 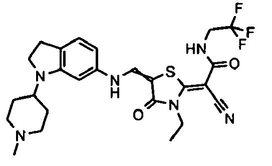 | 5,7E-09 |
| **B03** | 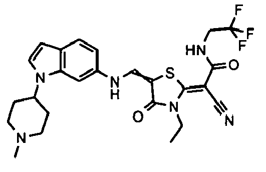 | 1,6E-09 |
| **B04** | 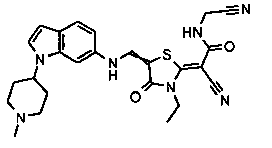 | 2,9E-09 |
| **B06** | 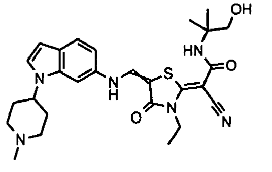 | 1,0E-08 |
| **B44** | 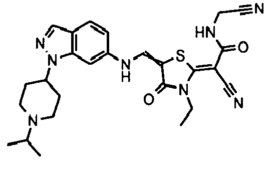 | 1,1E-09 |
| **B59** | 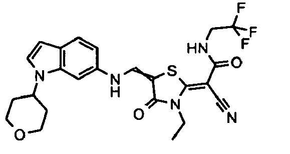 | 3,7E-08 |
| **B71** | 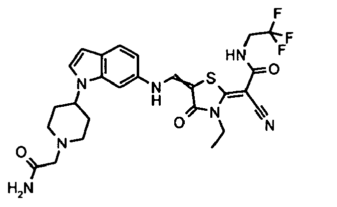 | 8,2E-09 |
| **B72** | 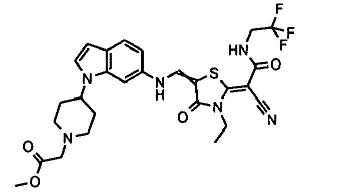 | 3,9E-09 |
| **B102** | 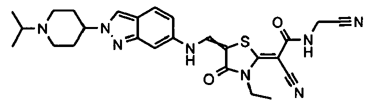 | 2,2E-08 |
| **B109** | 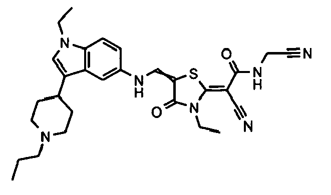 | 3,5E-09 |
| **B115** | 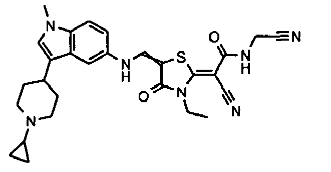 | 1,3E-09 |
| **B118** | 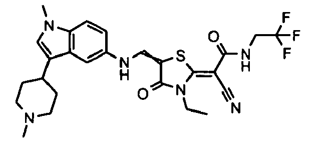 | 2,1 E-09 |
| **B123** | 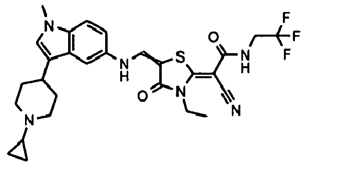 | 1,2E-08 |
| **B130** | 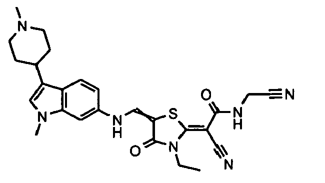 | 1,0E-09 |
| **B147** | 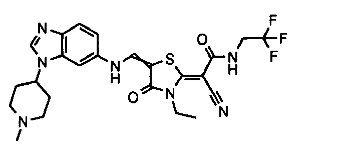 | 3,1 E-09 |
| **B153** | 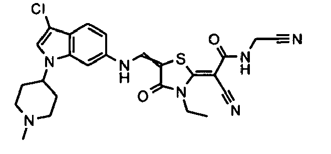 | 1,0E-09 |
| **B162** | 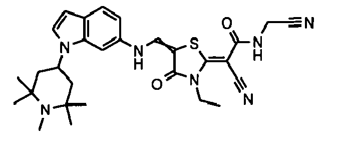 | 3,7E-09 |
| **B165** | 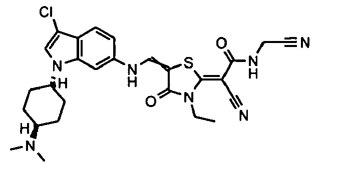 | 1,3E-09 |
| **B176** | 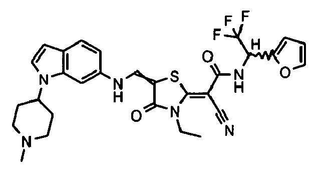 | 6,2E-08 |
| **B183** | 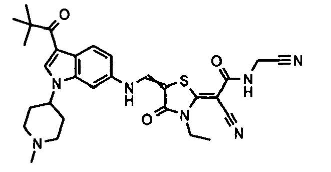 | 4,2E-09 |
| **B189** | 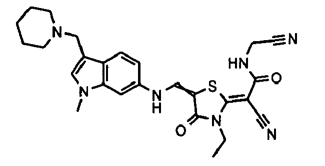 | 4,3E-09 |
| **B193** | 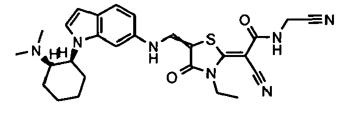 | 1,4E-08 |
| **B08** | 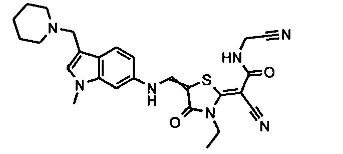 | 5,4E-09 |
| **B35** | 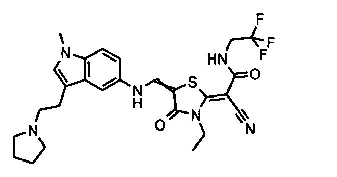 | 5,9E-09 |
| **B39** | 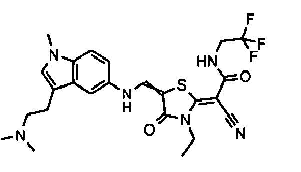 | 6,1 E-09 |
| **B58** | 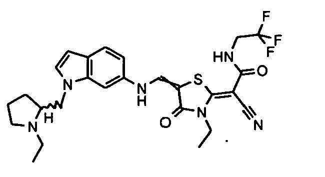 | 2,0E-08 |
| **B80** | 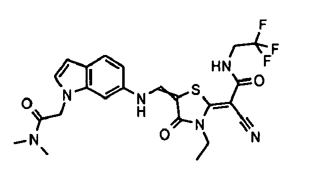 | 4,9E-08 |
| **B132** | 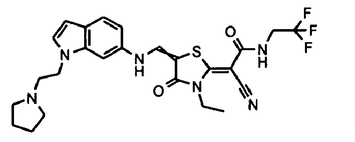 | 3,3E-09 |

**Tabelle 2**

| Daten zur Inhibition der Tumorzell-Proliferation von den Zellinien MCF-7, DU145 und NCI-H460. | | | |
|---|---|---|---|
| Beispiel Nr. | Inhibition IC₅₀ [M] (MCF-7) | Inhibition IC₅₀ [M] (DU145) | Inhibition IC₅₀ [M] (NCI-H460) |
| **B01** | 4,4E-07 | 1,3E-07 | 1,9E-07 |
| **B03** | 1,4E-07 | 5,1E-08 | 9,6E-08 |
| **B04** | 7,2E-08 | 2,9E-08 | 1,4E-07 |
| **B06** | 2,4E-07 | 1,2E-07 | 1,5E-07 |
| **B44** | 2,0E-07 | 1,1E-07 | 4,9E-07 |
| **B59** | 3,9E-07 | 1,7E-07 | 1,8E-07 |
| **B71** | 1,4E-07 | 1,2E-07 | 2,1E-07 |
| **B72** | 1,2E-07 | 1,2E-07 | 1,1E-07 |
| **B102** | 1,9E-07 | 2,8E-07 | 6,2E-07 |
| **B109** | 1,4E-07 | 5,4E-08 | 1,6E-07 |
| **B115** | 1,1E-07 | 3,7E-08 | 1,3E-07 |
| **B118** | 2,9E-07 | 8,5E-08 | 1,7E-07 |
| **B123** | 6,2E-07 | 2,3E-07 | 5,0E-07 |
| **B130** | 1,4E-07 | 8,4E-08 | 3,7E-07 |
| **B147** | 2,4E-07 | 1,3E-07 | 2,4E-07 |
| **B153** | 4,2E-07 | 1,4E-07 | 5,9E-07 |
| **B162** | 1,4E-07 | 3,5E-08 | 3,7E-08 |
| **B165** | 1,7E-07 | 1,8E-07 | 6,1E-07 |
| **B176** | 1,2E-07 | 2,1 E-07 | 3,9E-07 |
| **B183** | 1,6E-07 | 1,3E-07 | 3,2E-07 |
| **B189** | 1,9E-07 | 5,8E-08 | 3,5E-07 |
| **B193** | 1,8E-07 | 1,8E-07 | 2,6E-07 |
| **B08** | 6,1E-07 | 1,4E-07 | 3,3E-07 |
| **B35** | 6,4E-07 | 1,8E-07 | 3,3E-07 |
| **B39** | 3,8E-07 | 1,8E-07 | 4,8E-07 |
| **B58** | 3,7E-07 | 3,2E-07 | 4,8E-07 |
| **B80** | 3,1 E-07 | 5,1 E-07 | 8,9E-07 |
| **B132** | 4,5E-07 | 2,2E-07 | 4,0E-07 |

**Tabelle 3:**

| Vergleich mit Stand der Technik Daten zur Inhibition von PLK-1 bei 10 mM ATP. | | |
|---|---|---|
| Beispiel Nr. | Struktur | Inhibition PLK-1 IC₅₀ [M] (10 mM ATP) |
| **Beispiel 30 aus** PCT/EP2005/013418 | 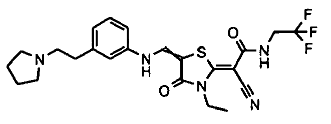 | 1,1E-07 |

**Tabelle 4: Vergleich mit Stand der Technik**

| Daten zur Inhibition der Tumorzell-Proliferation von den Zellinien DU145, NCI-H460 und MCF-7. | | | |
|---|---|---|---|
| Beispiel Nr. | Inhibition IC₅₀ [M] (MCF-7) | Inhibition IC₅₀ [M] (DU145) | Inhibition IC₅₀ [M] (NCI-H460) |
| **Beispiel 30 aus** PCT/EP2005/013418 | 7E-07 | 6E-07 | 9E-07 |

Aus den Tabellen 1 und 2 kann man ersehen, dass die vorliegenden erfinderischen Verbindungen der allgemeinen Formel ( I ) PLK und die Tumorzell-Proliferation inhibieren. Desweiteren kann der Fachmann aus Tabellen 3 und 4 ersehen, dass die vorliegenden erfinderischen Substanzen auch besser als der Stand der Technik von PCT Anmeldung PCT/EP2005/013418 (publiziert als WO 2006/063806) sind. In PCT/EP2005/013418 ist gezeigt, dass die Verbindungen von PCT/EP2005/013418 erfinderisch gegenüber den Verbindungen aus PCT Anmeldung PCT/EP2004/12242 (publiziert als WO 2005/042505) sind. In PCT/EP2004/12242 ist gezeigt, dass die Verbindungen von PCT/EP2004/12242 erfinderisch gegenüber den Verbindungen aus PCT Anmeldung PCT/EP2003/04450 (publiziert als WO 03/093249) sind.

## Patentansprüche

1. Verbindungen der allgemeinen Formel ( I ) : 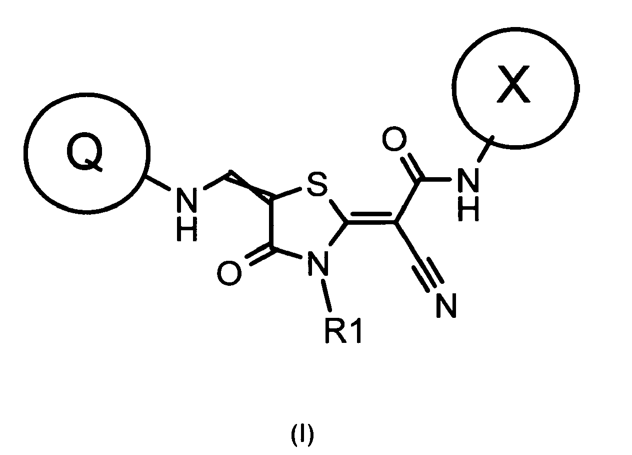 in der :
R1 für gegebenenfalls ein- oder mehrfach, gleich oder verschieden mit einem Halogenatom substituiertes C₁-C₃-Alkyl oder Cyclopropyl steht ;
X für gegebenenfalls ein oder mehrfach, gleich oder verschieden mit Cyan, Methyl, Ethyl, Isopropyl, Ethinyl, Hydroxy, Aryl, Furanyl oder einem Halogenatom substituiertes C₁-C₃-Alkyl steht ;
Q für einen Zyklus : 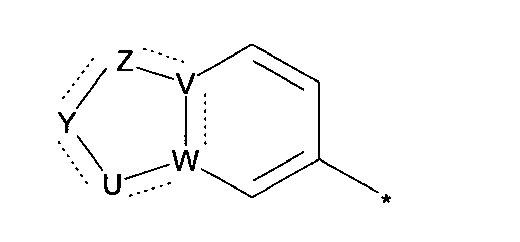
steht,
in dem :
zwischen V und W, W und U, U und Y, Y und Z sowie Z und V unabhängig voneinander jeweils eine Einfachbindung oder eine Doppelbindung besteht, wobei von diesen Bindungen nicht mehr als 2 Doppelbindungen sind,
und
V und W unabhängig voneinander für 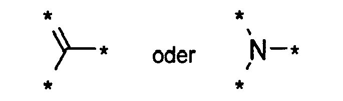 stehen,
U für : 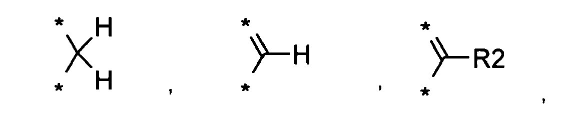 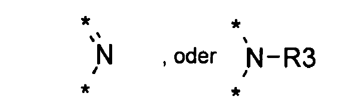 steht,
Y für : 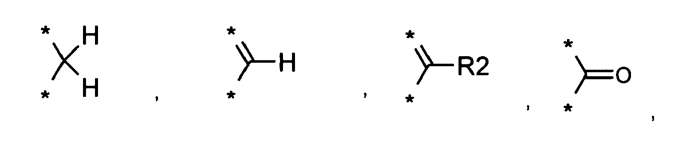 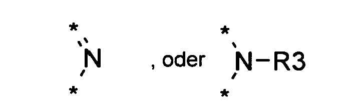 steht und
Z für : 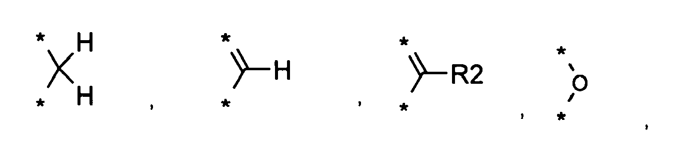 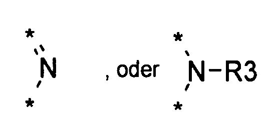 steht,
in dem :
R2 für ein Wasserstoffatom, ein Halogenatom, Cyan, Nitro, C₁-C₆-Alkyl, C₁-C₆-Haloalkyl, C₁-C₆-Alkoxy, C₁-C₆-Haloalkyloxy, C₁-C₆-Alkoxy-C₁-C₆-alkyl, Halo-C₁-C₆-alkoxy-C₁-C₆-alkyl, -(CH₂)ₙC(O)NH₂, -(CH₂)ₙC(O)-R8, -(CH₂)ₙC(O)NR4R5, -(CH₂)ₙC(O)₂R6, C₂-C₆-Alkenyl, C₂-C₆-Alkynyl, C₃-C₁₀-Cycloalkyl, C₃-C₁₀-Heterocycloalkyl, C₆-C₁₀-Aryl, -(CH₂)ₘ-C₆-C₁₀-Aryl, -(CH₂)ₙHeteroaryl, -O(CH2)ₚ-C6-C10-Aryl, -O(CH2)_{q}-Heteroaryl, -C(O)₂R6, -NR4R5, -C(O)NH₂, -C(O)-R8, -S(O)R7, -S(O)₂R7, -S(O)₂R8, -S(O)₂NR4R5, -C(O)H oder -C(O)R7, steht,
wobei das besagte C₁-C₆-Alkyl, C₁-C₆-Hatoatkyl, C₁-C₆-Alkoxy, C₁-C₆-Haloalkyloxy, C₁-C₆-Alkoxy-C₁-C₆-alkyl, Halo-C₁-C₆-alkoxy-C₁-C₆-alkyl, -(CH₂)ₙC(O)R8, -(CH₂)ₙC(O)NR4R5, -(CH₂)ₙC(O)₂R6, C₂-C₆-Alkenyl, C₂-C₆-Alkynyl, C₃-C₁₀-Cycloalkyl, C₃-C₁₀-Heterocycloalkyl, C₆-C₁₀-Aryl, -(CH₂)ₘ-C₆-C₁₀-Aryl, -(CH₂)ₙHeteroaryl, -O(CH₂)ₚ-C₆-C₁₀-aryl, -O(CH₂)_{q}-Heteroaryl, -C(O)₂R6, -NR4R5, -C(O)R8, -S(O)R7, -S(O)₂R7, -S(O)₂R8, -S(O)₂NR4R5, oder -C(O)R7
gegebenenfalls ein- oder mehrfach, gleich oder verschieden mit C₁-C₆-Alkyl, C₁-C₆-Haloalkyl, C₁-C₆-Alkoxy-C₁-C₆-alkyl, Halo-C₁-C₆-alkoxy-C₁-C₆-alkyl, C₂-C₆-Alkenyl, C₂-C₆-Alkynyl, C₃-C₁₀-Cycloalkyl, C₃-C₁₀-Heterocycloalkyl, C₆-C₁₀-Aryl, -(CH₂)ₘ-C₆-C₁₀-Aryl, -(CH₂)ₙHeteroaryl, -C(O)R7, -C(O)₂R6, -NR4R5, -C(O)NR4R5, -S(O)R7, -S(O)₂R7, -S(O)₂NR4R5, R8, R9, -(CH₂)ₙC(O)NR4R5 oder -(CH₂)ₙC(O)₂R6, substituiert ist ;
R3 für Cyan, C₁-C₆-Alkyl, C₁-C₆-Hatoalkyl, C₁-C₆-Alkoxy-C₁-C₆-alkyl, Hal0-C₁-C₆-alkoxy-C₁-C₆-alkyl, -(CH₂)ₙC(O)NH₂, -(CH₂)ₙC(O)R8, -(CH₂)ₙC(O)NR4R5, -(CH₂)ₙC(O)₂R6, C₂-C₆-Alkenyl, C₂-C₆-Alkynyl, C₃-C₁₀-Cycloalkyl, C₃-C₁₀-Heterocycloalkyl, C₆-C₁₀-Aryl, -(CH₂)ₘ-C₆-C₁₀-Aryl, -(CH₂)ₙHeteroaryl, -C(O)₂R6, -C(O)NH₂, -C(O)R8, , -C(O)NR4R5, -S(O)R7, -S(O)₂R7, -S(O)₂R8, -S(O)₂NR4R5, -C(O)H oder -C(O)R7 steht,
wobei das besagte C₁-C₆-Alkyl, C₁-C₆-Haloalkyl, C₁-C₆-Alkoxy-C₁-C₆-alkyl, Halo-C₁-C₆-alkoxy-C₁-C₆-alkyl, -(CH₂)ₙC(O)R8, -(CH₂)ₙC(O)NR4R5, -(CH₂)ₙC(O)₂R6, C₂-C₆-Alkenyl, C₂-C₆-Alkynyl, C₃-C₁₀-Cycloalkyl, C₃-C₁₀-Heterocycloalkyl, C₆-C₁₀-Aryl, -(CH₂)ₘ-C₆-C₁₀-Aryl, -(CH₂)ₙHeteroaryl, -C(O)₂R6, -C(O)R8, -C(O)NR4R5 -S(O)R7, -S(O)₂R7, -S(O)₂R8, -S(O)₂NR4R5, oder -C(O)R7,
gegebenenfalls ein- oder mehrfach, gleich oder verschieden mit C₁-C₆-Alkyl, C₁-C₆-Haloalkyl, C₁-C₆-Alkoxy-C₁-C₆-alkyl, Halo-C₁-C₆-alkoxy-C₁-C₆-alkyl, C₂-C₆-Alkenyl, C₂-C₆-Alkynyl, C₃-C₁₀-Cycloalkyl, C₃-C₁₀-Heterocycloalkyl, C₆-C₁₀-Aryl, -(CH₂)ₘ-C₆-C₁₀-Aryl, -(CH₂)ₙHeteroaryl, -C(O)R7, -C(O)₂R6, -NR4R5, -C(O)NR4R5, -S(O)R7, -S(O)₂R7, -S(O)₂NR4R5, R8, R9, -(CH₂)ₙC(O)NR4R5 oder -(CH₂)ₙC(O)₂R6, substituiert ist ;
R4 und R5 unabhängig voneinander für ein Wasserstoffatom, C₁-C₆-Alkyl, C₁-C₆-Haloalkyl, C₁-C₆-Alkoxy-C₁-C₆-alkyl, Halo-C₁-C₆-alkoxy-C₁-C₆-alkyl, C₂-C₆-Alkenyl, C₂-C₆-Alkynyl, C₃-C₁₀-Cycloalkyl, C₆-C₁₀-Aryl, Heteroaryl, -(CH₂)ₘ-C₆-C₁₀-Aryl, oder -(CH₂)ₙHeteroaryl stehen ;
R6 für ein Wasserstoffatom, C₁-C₆-Alkyl, C₁-C₆-Haloalkyl, C₁-C₆-Alkoxy-C₁-C₆-alkyl, Halo-C₁-C₆-alkoxy-C₁-C₆-alkyl, C₂-C₆-Alkenyl, C₂-C₆-Alkynyl, C₃-C₁₀-Cycloalkyl, C₆-C₁₀-Aryl, Heteroaryl -(CH₂)ₘ-C₆-C₁₀-Aryl, oder -(CH₂)ₙHeteroaryl steht ;
R7 für C₁-C₆-Alkyl, C₁-C₆-Haloalkyl, C₁-C₆-Alkoxy-C₁-C₆-alkyl, Halo-C₁-C₆-alkoxy-C₁-C₆-alkyl, C₂-C₆-Alkenyl, C₂-C₆-Alkynyl, C₃-C₁₀-Cycloalkyl, C₆-C₁₀-Aryl, Heteroaryl, -(CH₂)ₘ-C₆-C₁₀-Aryl, oder -(CH₂)ₙHeteroaryl steht ;
R8 für C₃-C₁₀-Heterocycloalkyl steht, wobei das besagte C₃-C₁₀-Heterocycloalkyl gegebenenfalls ein- oder mehrfach, gleich oder verschieden mit C₁-C₆-Alkyl, C₁-C₆-Haloalkyl, C₁-C₆-Alkoxy-C₁-C₆-alkyl, Halo-C₁-C₆-alkoxy-C₁-C₆-alkyl, C₂-C₆-Alkenyl, C₂-C₆-Alkynyl, C₃-C₁₀-Cycloalkyl, C₃-C₁₀-Heterocycloalkyl, C₆-C₁₀-Aryl, -(CH₂)ₘ-C₆-C₁₀-Aryl, -(CH₂)ₙHeteroaryl, -C(O)R7, -C(O)₂R6, -NR4R5, -C(O)NR4R5, -S(O)R7, -S(O)₂R7, -S(O)₂NR4R5, -(CH₂)ₙC(O)NR4R5 oder -(CH₂)ₙC(O)₂R6, substituiert ist ;
R9 für C₃-C₁₀-Cycloalkyl steht, wobei das besagte C₃-C₁₀-Cycloalkyl gegebenenfalls ein-oder mehrfach, gleich oder verschieden mit C₁-C₆-Alkyl, C₁-C₆-Haloalkyl, C₁-C₆-Alkoxy-C₁-C₆-alkyl, Halo-C₁-C₆-alkoxy-C₁-C₆-alkyl, C₂-C₆-Alkenyl, C₂-C₆-Alkynyl, C₃-C₁₀-Cycloalkyl, C₃-C₁₀-Heterocycloalkyl, C₆-C₁₀-Aryl, -(CH₂)ₘ-C₆-C₁₀-Aryl, -(CH₂)ₙHeteroaryl, -C(O)R7, -C(O)₂R6, -NR4R5, -C(O)NR4R5 -S(O)R7, -S(O)₂R7, -S(O)₂NR4R5, -(CH₂)ₙC(O)NR4R5 oder -(CH₂)ₙC(O)₂R6, substituiert ist ; und
n, m, p und q unabhängig voneinander eine Ganzzahl von 1, 2, 3, oder 4, sind.

2. Verbindungen der allgemeinen Formel ( I ) gemäß dem Anspruch 1, worin :
R1 für gegebenenfalls ein- oder mehrfach, gleich oder verschieden mit einem Halogenatom substituiertes Ethyl, Isopropyl, oder Cyclopropyl steht ;
X für gegebenenfalls ein oder mehrfach, gleich oder verschieden mit Cyan, Methyl, Ethyl, Ethinyl, Hydroxy, Furanyl oder einen Halogenatom substituiertes C₁-C₃-Alkyl steht ;
Q für einen Zyklus : 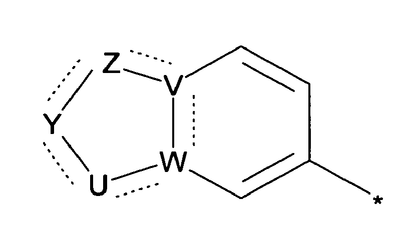 steht,
in dem :
zwischen V und W, W und U, U und Y, Y und Z sowie Z und V unabhängig voneinander jeweils eine Einfachbindung oder eine Doppelbindung besteht, wobei von diesen Bindungen nicht mehr als 2 Doppelbindungen sind,
und
V und W unabhängig voneinander für 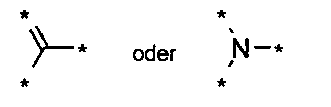 stehen,
U für : 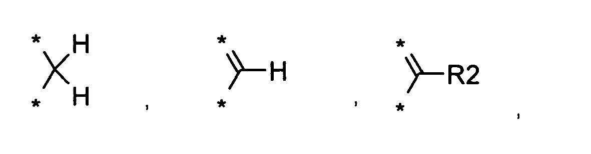 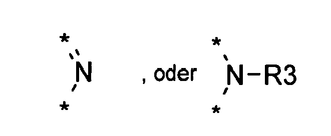 steht,
Y für : 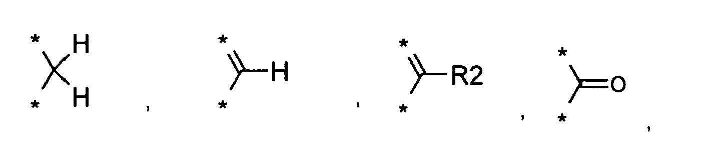 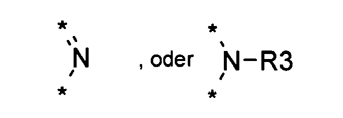 steht,
Z für : 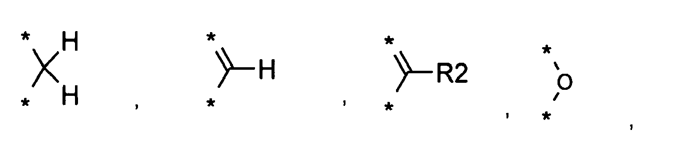 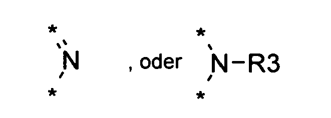 steht,
in dem :
R2 für ein Wasserstoffatom, ein Halogenatom, Cyan, C₁-C₆-Alkyl, C₁-C₆-Haloalkyl, C₁-C₆-Alkoxy-C₁-C₆-alkyl, Halo-C₁-C₆-alkoxy-C₁-C₆-alkyl, (CH₂)ₙC(O)NH₂, -(CH₂)ₙC(O)-R8, -(CH₂)ₙC(O)NR4R5, -(CH₂)ₙC(O)₂R6, C₂-C₆-Alkenyl, C₃-C₁₀-Cycloalkyl, C₃-C₁₀-Heterocycloalkyl, C₆-C₁₀-Aryl, -(CH₂)ₘ-C₆-C₁₀-Aryl, -(CH₂)ₙHeteroaryl, -C(O)₂R6, -NR4R5, -C(O)NH₂, -C(O)-R8, -C(O)H oder -C(O)R7 steht,
wobei das besagte C₁-C₆-Alkyl, C₁-C₆-Haloalkyl, C₁-C₆-Alkoxy-C₁-C₆-alkyl, Halo-C₁-C₆-alkoxy-C₁-C₆-alkyl, -(CH₂)ₙC(O)R8, -(CH₂)ₙC(O)NR4R5, -(CH₂)ₙC(O)₂R6, C₂-C₆-Alkenyl, C₃-C₁₀-Cycloalkyl, C₃-C₁₀-Heterocycloalkyl, C₆-C₁₀-Aryl, -(CH₂)ₘ-C₆-C₁₀-Aryl, -(CH₂)ₙHeteroaryl, -C(O)₂R6, -NR4R5, -C(O)R8, oder -C(O)R7
gegebenenfalls ein- oder mehrfach, gleich oder verschieden mit C₁-C₆-Alkyl, C₁-C₆-Haloalkyl, C₁-C₆-Alkoxy-C₁-C₆-alkyl, Halo-C₁-C₆-alkoxy-C₁-C₆-alkyl, C₂-C₆-Alkenyl, C₃-C₁₀-Cycloalkyl, C₃-C₁₀-Heterocycloalkyl, C₆-C₁₀-Aryl, -(CH₂)ₘ-C₆-C₁₀-Aryl, -(CH₂)ₙHeteroaryl, -C(O)R7, -C(O)₂R6, -NR4R5, -C(O)NR4R5 -S(O)R7, -S(O)₂R7, -S(O)₂NR4R5, R8 oder R9 substituiert ist ;
R3 für Cyan, C₁-C₆-Alkyl, C₁-C₆-Haloalkyl, C₁-C₆-Alkoxy-C₁-C₆-alkyl, Halo-C₁-C₆-alkoxy-C₁-C₆-alkyl, -(CH₂)ₙC(O)NH₂, -(CH₂)ₙC(O)R8, -(CH₂)ₙC(O)NR4R5, -(CH₂)ₙC(O)₂R6, C₂-C₆-Alkenyl, C₃-C₁₀-Cycloalkyl, C₃-C₁₀-Heterocycloalkyl, C₆-C₁₀-Aryl, -(CH₂)ₘ-C₆-C₁₀-Aryl, -(CH₂)ₙHeteroaryl, -C(O)₂R6, -C(O)NH₂, -C(O)R8, , -C(O)NR4R5 -C(O)H oder -C(O)R7 steht,
wobei der besagte C₁-C₆-Alkyl, C₁-C₆-Haloalkyl, C₁-C₆-Alkoxy-C₁-C₆-alkyl, Halo-C₁-C₆-alkoxy-C₁-C₆-alkyl, -(CH₂)ₙC(O)R8, -(CH₂)ₙC(O)NR4R5, -(CH₂)ₙC(O)₂R6, C₂-C₆-Alkenyl, C₃-C₁₀-Cycloalkyl, C₃-C₁₀-Heterocycloalkyl, C₆-C₁₀-Aryl, -(CH₂)ₘ-C₆-C₁₀-Aryl, -(CH₂)ₙHeteroaryl, -C(O)₂R6, -C(O)R8, -C(O)NR4R5, oder -C(O)R7
gegebenenfalls ein- oder mehrfach, gleich oder verschieden mit C₁-C₆-Alkyl, C₁-C₆-Haloalkyl, C₁-C₆-Alkoxy-C₁-C₆-alkyl, Halo-C₁-C₆-alkoxy-C₁-C₆-alkyl, C₂-C₆-Alkenyl, C₃-C₁₀-Cycloalkyl, C₃-C₁₀-Heterocycloalkyl, C₆-C₁₀-Aryl, -(CH₂)ₘ-C₆-C₁₀-Aryl, -(CH₂)ₙHeteroaryl, -C(0)R7, -C(O)₂R6, -NR4R5, -C(O)NR4R5, -S(O)R7, -S(O)₂R7, -S(O)₂NR4R5, R8, R9, -(CH₂)ₙC(O)NR4R5 oder -(CH₂)ₙC(O)₂R6, substituiert ist ; und
n und m unabhängig voneinander eine Ganzzahl von 1, 2, oder 3, sind.

3. Verbindungen der allgemeinen Formel ( I ) gemäß dem Anspruch 1 oder 2, worin :
R1 für gegebenenfalls ein- oder mehrfach, gleich oder verschieden mit einem Halogenatom substituiertes Ethyl steht ;
X für gegebenenfalls ein oder mehrfach, gleich oder verschieden mit Cyan, Methyl, Hydroxy oder einen Halogenatom substituiertes C₁-C₃-Alkyl steht ;
Q für : 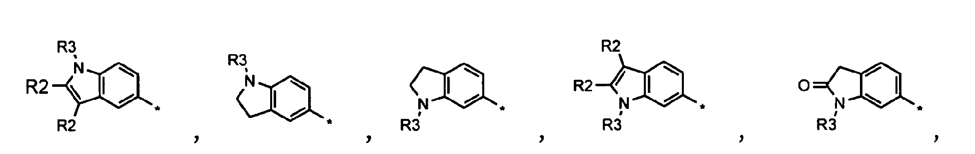 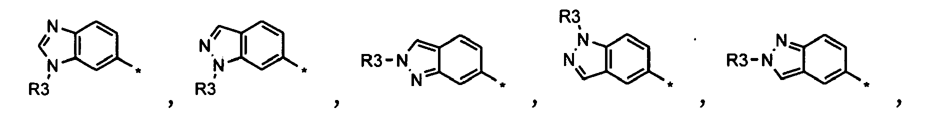 oder 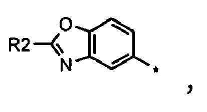 steht, in dem R2 und/oder R3 wie in dem Anpruch 1 oder 2 definiert ist(sind) ; und in dem n, m, p und q unabhängig voneinander 1 oder 2 sind.

4. Verbindungen der allgemeinen Formel ( I ) gemäß dem Anspruch 1, 2 oder 3, worin
Q für : 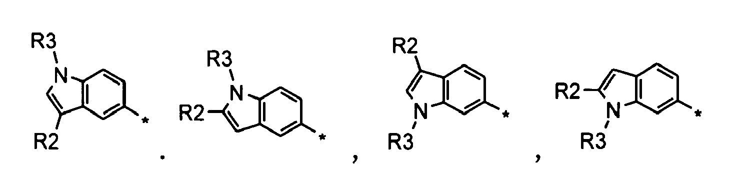 oder 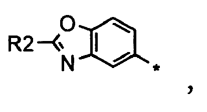
steht, in dem :
R2 für Cyan, C₁-C₆-Alkoxy-C₁-C₆-alkyl, Halo-C₁-C₆-alkoxy-C₁-C₆-alkyl, -(CH₂)ₙC(O)NH₂, - (CH₂)ₙC(O)-R8, -(CH₂)ₙC(O)₂R6, C₂-C₆-Alkenyl, C₃-C₁₀-Cycloalkyl, oder C₃-C₁₀-Heterocycloalkyl steht,
wobei das besagte C₁-C₆-Alkoxy-C₁-C₆-alkyl, Halo-C₁-C₆-alkoxy-C₁-C₆-alkyl, -(CH₂)ₙC(O)R8, -(CH₂)ₙC(O)₂R6, C₂-C₆-Alkenyl, C₃-C₁₀-Cycloalkyl oder C₃-C₁₀-Heterocycloalkyl
gegebenenfalls ein- oder mehrfach, gleich oder verschieden mit C₁-C₆-Alkyl, C₁-C₆-Haloalkyl, C₁-C₆-Alkoxy-C₁-C₆-alkyl, Halo-C₁-C₆-alkoxy-C₁-C₆-alkyl, C₂-C₆-Alkenyl, C₃-C₁₀-Cycloalkyl, C₃-C₁₀-Heterocycloalkyl, C₆-C₁₀-Aryl, -(CH₂)ₘ-C₆-C₁₀-Aryl, -(CH₂)ₙHeteroaryl, -C(O)R7, -C(O)₂R6, -NR4R5, -C(0)NR4R5, -S(O)R7, -S(O)₂R7, -S(O)₂NR4R5, R8 oder R9 substituiert ist.

5. Verbindungen der allgemeinen Formel ( I ) gemäß Anspruch 1, 2 oder 3, worin :
Q für : 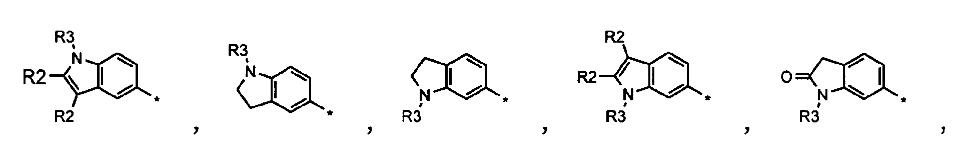 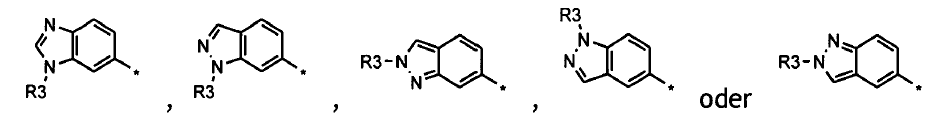
steht, in dem :
R3 für Cyan, C₁-C₆-Alkoxy-C₁-C₆-alkyl, Halo-C₁-C₆-alkoxy-C₁-C₆-alkyl, -(CH₂)ₙC(O)NH₂, - (CH₂)ₙC(O)-R8, -(CH₂)ₙC(O)₂R6, C₂-C₆-Alkenyl, C₃-C₁₀-Cycloalkyl oder C₃-C₁₀-heterocycloalkyl steht,
wobei das besagte C₁-C₆-alkoxy-C₁-C₆-alkyl, halo-C₁-C₆-alkoxy-C₁-C₆-alkyl, -(CH₂)ₙC(O)R8, -(CH₂)ₙC(O)₂R6, C₂-C₆-alkenyl, C₃-C₁₀-cycloalkyl oder C₃-C₁₀-heterocycloalkyl,
gegebenenfalls ein- oder mehrfach, gleich oder verschieden mit C₁-C₆-alkyl, C₁-C₆-haloalkyl, C₁-C₆-alkoxy-C₁-C₆-alkyl, halo-C₁-C₆-alkoxy-C₁-C₆-alkyl, C₂-C₆-alkenyl, C₃-C₁₀-cycloalkyl, C₃-C₁₀-heterocycloalkyl, C₆-C₁₀-aryl, -(CH₂)ₘ-C₆-C₁₀-aryl, -(CH₂)ₙheteroaryl, -C(O)R7, -C(O)₂R6, -NR4R5, -C(O)NR4R5, -S(O)R7, -S(O)₂R7, -S(O)₂NR4R5, R8, R9, -(CH₂)ₙC(O)NR4R5 oder -(CH₂)ₙC(O)₂R6 substituiert ist.

6. Verbindungen der allgemeinen Formel ( I ) gemäß einem der Ansprüche 1, 2, 3 oder 5, worin :
R3 für C₁-C₆-Alkoxy-C₁-C₆-alkyl, Halo-C₁-C₆-alkoxy-C₁-C₆-alkyl, C₃-C₁₀-Cycloalkyl oder C₃-C₁₀-heterocycloalkyl steht,
wobei das besagte C₁-C₆-alkoxy-C₁-C₆-alkyl, halo-C₁-C₆-alkoxy-C₁-C₆-alkyl, C₃-C₁₀-cycloalkyl oder C₃-C₁₀-heterocycloalkyl,
gegebenenfalls ein- oder mehrfach, gleich oder verschieden mit C₁-C₆-alkyl, C₁-C₆-haloalkyl, C₁-C₆-alkoxy-C₁-C₆-alkyl, halo-C₁-C₆-alkoxy-C₁-C₆-alkyl, C₂-C₆-alkenyl, C₃-C₁₀-cycloalkyl, C₃-C₁₀-heterocycloalkyl, C₆-C₁₀-aryl, -(CH₂)ₘ-C₆-C₁₀-aryl, -(CH₂)ₙheteroaryl, -C(O)R7, -C(O)₂R6, -NR4R5, -C(0)NR4R5, -S(O)R7, -S(O)₂R7, -S(O)₂NR4R5, R8, R9, -(CH₂)ₙC(O)NR4R5 oder -(CH₂)ₙC(O)₂R6 substituiert ist.

7. Verbindungen der allgemeinen Formel ( I ) gemäß Anspruch 1, 2 oder 3, worin :
Q für : 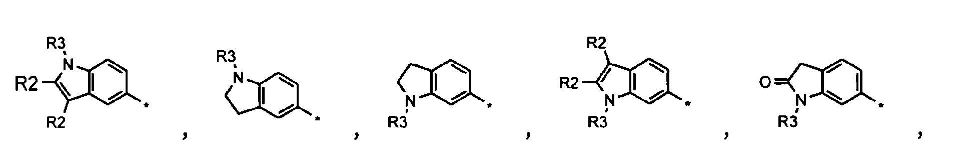 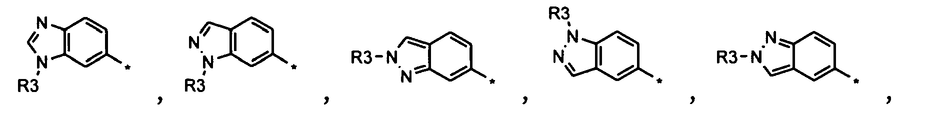 oder 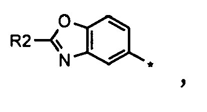
steht, in dem :
R2 für ein Wasserstoffatom, ein Halogenatom, -C₆-C₁₀-aryl, -C(O)₂R6, -NR4R5, -C(O)NH2, -C(O)-R8, -C(O)H oder -C(O)R7 steht,
wobei das besagte C₆-C₁₀-aryl, -C(O)₂R6, -NR4R5, -C(O)R8, oder -C(O)R7, gegebenenfalls ein- oder mehrfach, gleich oder verschieden mit C₁-C₆-Alkyl, C₁-C₆-Haloalkyl, C₁-C₆-Alkoxy-C₁-C₆-alkyl, Halo-C₁-C₆-alkoxy-C₁-C₆-alkyl, C₂-C₆-Alkenyl, C₃-C₁₀-Cycloalkyl, C₃-C₁₀-Heterocycloalkyl, C₆-C₁₀-Aryl, -(CH₂)ₘ-C₆-C₁₀-Aryl, -(CH₂)ₙHeteroaryl, -C(O)R7, -C(O)₂R6, -NR4R5, -C(O)NR4R5 -S(O)R7, -S(O)₂R7, -S(O)₂NR4R5, R8 oder R9 substituiert ist ;
oder für
C₁-C₆-Alkyl, C₁-C₆-Haloalkyl, -(CH₂)ₙC(O)NR4R7, -(CH₂)ₘ-C₆-C₁₀-aryl oder -(CH₂)ₙheteroaryl steht,
wobei das besagte C₁-C₆-Alkyl, C₁-C₆-Haloalkyl, -(CH₂)ₙC(O)NR4R7, -(CH₂)ₘ-C₆-C₁₀-aryl oder -(CH₂)ₙheteroaryl
gegebenenfalls ein- oder mehrfach, gleich oder verschieden mit C₁-C₆-Alkyl, C₁-C₆-Haloalkyl, C₃-C₁₀-Cycloalkyl, C₃-C₁₀-Heterocycloalkyl, C₆-C₁₀-Aryl, -C(O)R7, -NR4R5, -S(O)R7, -S(O)₂R7, -S(O)₂NR4R5, R8 oder R9 substituiert ist ;
R3 für C₆-C₁₀-aryl, -C(O)₂R6, -C(O)NH2, -C(O)R8, -C(O)NR4R5, -C(O)H oder -C(O)R7 steht,
wobei das besagte C₆-C₁₀-aryl, -C(O)₂R6, -C(O)R8, -C(0)NR4R5, oder -C(O)R7, gegebenenfalls ein- oder mehrfach, gleich oder verschieden mit C₁-C₆-Alkyl, C₁-C₆-Haloalkyl, C₁-C₆-Alkoxy-C₁-C₆-alkyl, Halo-C₁-C₆-alkoxy-C₁-C₆-alkyl, C₂-C₆-Alkenyl, C₃-C₁₀-Cycloalkyl, C₃-C₁₀-Heterocycloalkyl, C₆-C₁₀-Aryl, -(CH₂)ₘ-C₆-C₁₀-Aryl, -(CH₂)ₙHeteroaryl, -C(O)R7, -C(O)₂R6, -NR4R5, -C(0)NR4R5, -S(O)R7, -S(O)₂R7, -S(O)₂NR4R5, R8, R9, -(CH₂)ₙC(O)NR4R5 oder -(CH₂)ₙC(O)₂R6, substituiert ist,
oder für
C₁-C₆-Alkyl, C₁-C₆-Haloalkyl, -(CH₂)ₙC(O)NR4R7, -(CH₂)ₘ-C₆-C₁₀-aryl oder -(CH₂)ₙheteroaryl steht,
wobei das besagte C₁-C₆-Alkyl, C₁-C₆-Haloalkyl, -(CH₂)ₙC(O)NR4R7, -(CH₂)ₘ-C₆-C₁₀-aryl oder -(CH₂)ₙheteroaryl
gegebenenfalls ein- oder mehrfach, gleich oder verschieden mit C₁-C₆-Alkyl, C₁-C₆-Haloalkyl, C₃-C₁₀-Cycloalkyl, C₃-C₁₀-Heterocycloalkyl, C₆-C₁₀-Aryl, -C(O)R7, -NR4R5, -S(O)R7, -S(O)₂R7, -S(O)₂NR4R5, R8 oder R9 substituiert ist.

8. **Verbindungen der allgemeinen Formel ( I ) gemäß Anspruch 1 mit einem der folgenden Namen:**
(*Z,E*/*Z*)-2-Cyan-2-[5-[[[2,3-dihydro-1-(1-methyl-4-piperidinyl)-1*H*-indol-6-yl]amino]methylen]-3-ethyl-4-oxo-2-thiazolidinyliden]-*N*-(2,2,2-trifluorethyl)acetamid
(*Z,E*/*Z*)-2-Cyan-*N*-(cyanmethyl)-2-[5-[[[2,3-dihydro-1-(1-methyl-4-piperidinyl)-1*H-*indol-6-yl]amino]methylen]-3-ethyl-4-oxo-2-thiazolidinyliden]acetamid
(*Z,E*/*Z*)-2-Cyan-2-[3-ethyl-5-[[[1-(1-methyl-4-piperidinyl)-1*H*-indol-6-yl]amino]methylen]-4-oxo-2-thiazolidinyliden]-*N*-(2,2,2-trifluorethyl)acetamid
(*Z,E*/*Z*)-2-Cyan-*N*-(cyanmethyl)-2-[3-ethyl-5-[[[1-(1-methyl-4-piperidinyl)-1*H*-indol-6-yl]amino]methylen]-4-oxo-2-thiazolidinyliden]acetamid
[*S*-(*Z,E*/*Z*)]-2-Cyan-2-[3-ethyl-5-[[[1-(1-methyl-4-piperidinyl)-1*H*-indol-6-yl]amino]methylen]-4-oxo-2-thiazolidinyliden]-*N*-[1-(hydroxymethyl)-2-methylpropyl]acetamid
(*Z,E*/*Z*)-2-Cyan-2-[3-ethyl-5-[[[1-(1-methyl-4-piperidinyl)-1*H*-indol-6-yl]amino]methylen]-4-oxo-2-thiazolidinyliden]-*N*-(2-hydroxy-1,1-dimethylethyl)acetamid
(*Z,E*/*Z*)-2-Cyan-2-[3-ethyl-5-[[[1-(2-methoxyethyl)-3-(1-piperidinylmethyl)-1*H*-indol-6-yl]amino]methylen]-4-oxo-2-thiazolidinyliden]-*N*-(2,2,2-trifluorethyl)acetamid
(*Z,E*/*Z*)-2-Cyan-*N*-(cyanmethyl)-2-[3-ethyl-5-[[[1-(2-methoxyethyl)-3-(1-piperidinylmethyl)-1*H*-indol-6-yl]amino]methylen]-4-oxo-2-thiazolidinyliden]acetamid
(*Z,E*/*Z*)-2-Cyan-2-[3-ethyl-5-[[[1-(2-methoxyethyl)-3-(4-morpholinylmethyl)-1*H*-indol-6-yl]amino]methylen]-4-oxo-2-thiazolidinyliden]-*N*-(2,2,2-trifluorethyl)acetamid
(*Z,E*/*Z*)-2-Cyan-*N*-(cyanmethyl)-2-[3-ethyl-5-[[[1-(2-methoxyethyl)-3-(4-morpholinylmethyl)-1*H*-indol-6-yl]amino]methylen]-4-oxo-2-thiazolidinyliden]acetamid
(*Z,E*/*Z*)-2-Cyan-2-[5-[[[3-[(dimethylamino)methyl]-1-(2-methoxyethyl)-1*H*-indol-6-yl]amino]methylen]-3-ethyl-4-oxo-2-thiazolidinyliden]-*N*-(2,2,2-trifluorethyl)acetamid
(*Z,E*/*Z*)-2-Cyan-*N*-(cyanmethyl)-2-[5-[[[3-[(dimethylamino)methyl]-1-(2-methoxyethyl)-1*H*-indol-6-yl]amino] methylen]-3-ethyl-4-oxo-2-thiazolidinyliden]acetamid
(*Z,E*/*Z*)-2-Cyan-2-[5-[[[2,3-dihydro-1-(1-methyl-4-piperidinyl)-1*H*-indol-5-yl]amino]methylen]-3-ethyl-4-oxo-2-thiazolidinyliden]-*N*-(2,2,2-trifluorethyl)acetamid
(*Z,E*/*Z*)-2-Cyan-2-[3-ethyl-5-[[[1-(1-methyl-4-piperidinyl)-1*H*-indol-5-yl]amino]methylen]-4-oxo-2-thiazolidinyliden]-*N*-(2,2,2-trifluorethyl)acetamid
(*Z,E*/*Z*)-2-Cyan-2-[3-ethyl-5-[[[1-[1-(1-methylethyl)-4-piperidinyl]-1*H*-indazol-6-yl]amino]methylen]-4-oxo-2-thiazolidinyliden]-*N*-(2,2,2-trifluorethyl)acetamid
(*Z,E*/*Z*)-2-Cyan-*N*-(cyanmethyl)-2-[3-ethyl-5-[[[1-[1-(1-methylethyl)-4-piperidinyl]-1*H-*indazol-6-yl]amino]methylen]-4-oxo-2-thiazolidinyliden]acetamid
(*Z,E*/*Z*)-2-Cyan-2-[3-ethyl-4-oxo-5-[[[1-(1-propyl-4-piperidinyl)-1*H*-indazol-6-yl]amino]methylen]-2-thiazolidinyliden]-*N*-(2,2,2-trifluorethyl)acetamid
(*Z,E*/*Z*)-2-Cyan-*N*-(cyanmethyl)-2-[3-ethyl-4-oxo-5-[[[1-(1-propyl-4-piperidinyl)-1*H-*indazol-6-yl]amino]methylen]-2-thiazolidinyliden]acetamid
(*Z,E*/*Z*)-2-Cyan-2-[3-ethyl-5-[[[2-(1-methyl-4-piperidinyl)-2*H*-indazol-6-yl]amino]methylen]-4-oxo-2-thiazolidinyliden]-*N*-(2,2,2-trifluorethyl)acetamid
(*Z,E*/*Z*)-2-Cyan-*N*-(cyanmethyl)-2-[3-ethyl-5-[[[2-(1-methyl-4-piperidinyl)-2*H*-indazol-6-yl]amino]methylen]-4-oxo-2-thiazolidinyliden]acetamid
(*Z,E*/*Z*)-2-Cyan-*N*-(cyanmethyl)-2-[3-ethyl-5-[[[1-(1-methyl-4-piperidinyl)-1*H*-indol-5-yl]amino]methylen]-4-oxo-2-thiazolidinyliden]acetamid
(*Z,E*/*Z*)-2-Cyan-2-[3-ethyl-5-[[[1-[1-(1-methylethyl)-4-piperidinyl]-1*H*-indol-5-yl]amino]methylen]-4-oxo-2-thiazolidinyliden]-*N*-(2,2,2-trifluorethyl)acetamid
(*Z,E*/*Z*)-2-Cyan-*N*-(cyanmethyl)-2-[3-ethyl-5-[[[1-[1-(1-methylethyl)-4-piperidinyl]-1*H-*indol-5-yl]amino]methylen]-4-oxo-2-thiazolidinyliden]acetamid
(*Z,E*/*Z*)-2-Cyan-2-[3-ethyl-5-[[[3-methyl-1-(1-methyl-4-piperidinyl)-1*H*-indol-5-yl]amino]methylen]-4-oxo-2-thiazolidinyliden]-*N*-(2,2,2-trifluorethyl)acetamid
(*Z,E*/*Z*)-2-Cyan-N-(cyanmethyl)-2-[3-ethyl-5-[[[3-methyl-1-(1-methyl-4-piperidinyl)-1*H*-indol-5-yl]amino]methylen]-4-oxo-2-thiazolidinyliden]acetamid
(*Z,E*/*Z*)-2-Cyan-2-[3-ethyl-5-[[[1-(1-ethyl-4-piperidinyl)-3-methyl-1*H*-indol-5-yl]amino]methylen]-4-oxo-2-thiazolidinyliden]-*N*-(2,2,2-trifluorethyl)acetamid
(*Z,E*/*Z*)-2-Cyan-*N*-(cyanmethyl)-2-[3-ethyl-5-[[[1-(1-ethyl-4-piperidinyl)-3-methyl-1*H-*indol-5-yl]amino]methylen]-4-oxo-2-thiazolidinyliden]acetamid
(*Z,E*/*Z*)-2-Cyan-2-[3-ethyl-5-[[[2-methyl-1-(1-methyl-4-piperidinyl)-1*H*-indol-6-yl]amino]methylen]-4-oxo-2-thiazolidinyliden]-*N*-(2,2,2-trifluorethyl)acetamid
(*Z,E*/*Z*)-2-Cyan-2-[3-ethyl-4-oxo-5-[[[1-(tetrahydro-2H-pyran-4-yl)-1*H*-indol-6-yl]amino]methylen]-2-thiazolidinyliden]-*N*-(2,2,2-trifluorethyl)acetamid
(*Z,E*/*Z*)-2-Cyan-2-[3-ethyl-4-oxo-5-[[[1-(1-propyl-4-piperidinyl)-1*H*-indol-6-yl]amino]methylen]-2-thiazolidinyliden]-*N*-(2,2,2-trifluorethyl)acetamid
(*Z,E*/*Z*)-2-Cyan-*N*-(cyanmethyl)-2-[3-ethyl-4-oxo-5-[[[1-(1-propyl-4-piperidinyl)-1*H-*indol-6-yl]amino]methylen]-2-thiazolidinyliden]acetamid
(*Z,E*/*Z*)-2-Cyan-2-[3-ethyl-5-[[[1-[1-(1-methylethyl)-4-piperidinyl]-1*H*-indol-6-yl]amino]methylen]-4-oxo-2-thiazolidinyliden]-*N*-(2,2,2-trifluorethyl)acetamid
(*Z,E*/*Z*)-2-Cyan-*N*-(cyanmethyl)-2-[3-ethyl-5-[[[1-[1-(1-methylethyl)-4-piperidinyl]-1*H-*indol-6-yl]amino]methylen]-4-oxo-2-thiazolidinyliden]acetamid
(*Z,E*/*Z*)-2-Cyan-2-[3-ethyl-5-[[[1-(1-ethyl-4-piperidinyl)-1*H*-indol-6-yl]amino]methylen]-4-oxo-2-thiazolidinyliden]-*N*-(2,2,2-trifluorethyl)acetamid
(*Z,E*/*Z*)-2-Cyan-*N*-(cyanmethyl)-2-[3-ethyl-5-[[[1-(1-ethyl-4-piperidinyl)-1*H*-indol-6-yl]amino]methylen]-4-oxo-2-thiazolidinyliden]acetamid
(*Z,E*/*Z*)-2-Cyan-2-[3-ethyl-5-[[[1-(1-methyl-3-piperidinyl)-1*H*-indol-6-yl]amino]methylen]-4-oxo-2-thiazolidinyliden]-*N*-(2,2,2-trifluorethyl)acetamid
(*Z,E*/*Z*)-2-Cyan-2-[3-ethyl-4-oxo-5-[[[1-[1-(2-methoxyethyl)-4-piperidinyl]-1*H*-indol-6-yl]amino]methylen]-2-thiazolidinyliden]-*N*-(2,2,2-trifluorethyl)acetamid
(*Z,E*/*Z*)-2-Cyan-*N*-(cyanmethyl)-2-[3-ethyl-4-oxo-5-[[[1-[1-(2-methoxyethyl)-4-piperidinyl]-1*H*-indol-6-yl]amino]methylen]-2-thiazolidinyliden]acetamid
(*Z,E*/*Z*)-4-[6-[[[2-[1-Cyan-2-oxo-2-[(2,2,2-trifluorethyl)amino]ethyliden]-3-ethyl-4-oxo-5-thiazolidinyliden]methyl]amino]-1*H*-indol-1-yl]-1-piperidinacetamid
(*Z,E*/*Z*)-Methyl 4-[6-[[[2-[1-cyan-2-oxo-2-[(2,2,2-trifluorethyl)amino]ethyliden]-3-ethyl-4-oxo-5-thiazolidinyliden]methyl]amino]-1*H*-indol-1-yl]-1-piperidinacetat
(*Z,E*/*Z*)-4-[6-[[[2-[1-Cyan-2-oxo-2-[(2,2,2-trifluorethyl)amino]ethyliden]-3-ethyl-4-oxo-5-thiazolidinyliden]methyl]amino]-1*H*-indol-1-yl]-1-piperidinessigsäure
(*Z,E*/*Z*)-4-[6-[[[2-[1-Cyan-2-oxo-2-[(2,2,2-trifluorethyl)amino]ethyliden]-3-ethyl-4-oxo-5-thiazolidinyliden]methyl]amino]-1*H*-indol-1-yl]-*N*-methyl-1-piperidinacetamid
(*Z,E*/*Z*)-4-[6-[[[2-[1-Cyan-2-oxo-2-[(2,2,2-trifluorethyl)amino]ethyliden]-3-ethyl-4-oxo-5-thiazolidinyliden]methyl]amino]-1*H*-indol-1-yl]-*N*,*N*-dimethyl-1-piperidinacetamid
(*Z,E*/*Z*)-Methyl6-[[[2-[1-cyan-2-oxo-2-[(2,2,2-trifluorethyl)amino]ethyliden]-3-ethyl-4-oxo-5-thiazolidinyliden]methyl]amino]-1*H*-indol-1-acetat
(*Z,E*/*Z*)-6-[[[2-[1-Cyan-2-oxo-2-[(2,2,2-trifluorethyl)amino]ethyliden]-3-ethyl-4-oxo-5-thiazolidinyliden]methyl]amino]-1*H*-indol-1-essigsäure
(*Z,E*/*Z*)-6-[[[2-[1-Cyan-2-oxo-2-[(2,2,2-trifluorethyl)amino]ethyliden]-3-ethyl-4-oxo-5-thiazolidinyliden]methyl]amino]-1*H*-indol-1-acetamid
(*Z,E*/*Z*)-6-[[[2-[1-Cyan-2-[(cyanmethyl)amino]-2-oxoethyliden]-3-ethyl-4-oxo-5-thiazolidinyliden]methyl]amino]-1*H*-indol-1-acetamid
(*Z,E*/*Z*)-2-Cyan-2-[3-ethyl-5-[[[1-[2-(4-methyl-1-piperazinyl)-2-oxoethyl]-1*H*-indol-6-yl]amino]methylen]-4-oxo-2-thiazolidinyliden]-*N*-(2,2,2-trifluorethyl)acetamid
(*Z,E*/*Z*)-2-Cyan-*N*-(cyanmethyl)-2-[3-ethyl-5-[[[1-[2-(4-methyl-1-piperazinyl)-2-oxoethyl]-1*H*-indol-6-yl]amino]methylen]-4-oxo-2-thiazolidinyliden]acetamid
(*Z,E*/*Z*)-2-Cyan-*N*-(cyanmethyl)-2-[3-ethyl-5-[[[1-ethyl-3-(1-methyl-4-piperidinyl)-1*H-*indol-5-yl]amino]methylen]-4-oxo-2-thiazolidinyliden]acetamid
(*Z,E*/*Z*)-2-Cyan-2-[3-ethyl-5-[[[1-ethyl-3-(1-methyl-4-piperidinyl)-1*H*-indol-5-yl]amino]methylen]-4-oxo-2-thiazolidinyliden]-*N*-(2,2,2-trifluorethyl)acetamid
(*Z,E*/*Z*)-2-Cyan-*N*-ethyl-2-[3-ethyl-5-[[[1-ethyl-3-(1-methyl-4-piperidinyl)-1*H*-indol-5-yl]amino]methylen]-4-oxo-2-thiazolidinyliden]acetamid
(*Z,E*/*Z*)-2-Cyan-*N*-(cyanmethyl)-2-[3-ethyl-5-[[[1-ethyl-3-(1-ethyl-4-piperidinyl)-1*H-*indol-5-yl]amino]methylen]-4-oxo-2-thiazolidinyliden]acetamid
(*Z,E*/*Z*)-2-Cyan-2-[3-ethyl-5-[[[1-ethyl-3-(1-ethyl-4-piperidinyl)-1*H*-indol-5-yl]amino]methylen]-4-oxo-2-thiazolidinyliden]-*N*-(2,2,2-trifluorethyl)acetamid
(*Z,E*/*Z*)-2-Cyan-N-ethyl-2-[3-ethyl-5-[[[1-ethyl-3-(1-ethyl-4-piperidinyl)-1*H*-indol-5-yl]amino]methylen]-4-oxo-2-thiazolidinyliden]acetamid
(*Z,E*/*Z*)-2-Cyan-*N*-(cyanmethyl)-2-[3-ethyl-5-[[[1-ethyl-3-(1-propyl-4-piperidinyl)-1*H-*indol-5-yl]amino]methylen]-4-oxo-2-thiazolidinyliden]acetamid
(*Z,E*/*Z*)-2-Cyan-2-[3-ethyl-5-[[[1-ethyl-3-(1-propyl-4-piperidinyl)-1*H*-indol-5-yl]amino]methylen]-4-oxo-2-thiazolidinyliden]-*N*-(2,2,2-trifluorethyl)acetamid
(*Z,E*/*Z*)-2-Cyan-*N*-ethyl-2-[3-ethyl-5-[[[1-ethyl-3-(1-propyl-4-piperidinyl)-1*H*-indol-5-yl]amino]methylen]-4-oxo-2-thiazolidinyliden]acetamid
(*Z,E*/*Z*)-2-Cyan-*N*-(cyanmethyl)-2-[3-ethyl-5-[[[1-ethyl-3-[1-(2-methylpropyl)-4-piperidinyl]-1H-indol-5-yl]amino]methylen]-4-oxo-2-thiazolidinyliden]acetamid
(*Z,E*/*Z*)-2-Cyan-2-[3-ethyl-5-[[[1-ethyl-3-[1-(2-methylpropyl)-4-piperidinyl]-1*H*-indol-5-yl]amino]methylen]-4-oxo-2-thiazolidinyliden]-*N*-(2,2,2-trifluorethyl)acetamid
(*Z,E*/*Z*)-2-Cyan-*N*-ethyl-2-[3-ethyl-5-[[[1-ethyl-3-[1-(2-methylpropyl)-4-piperidinyl]-1*H*-indol-5-yl]amino]methylen]-4-oxo-2-thiazolidinyliden]acetamid
(*Z,E*/*Z*)-2-Cyan-*N*-(cyanmethyl)-2-[3-ethyl-5-[[[3-(1-methyl-4-piperidinyl)-1-propyl-1*H-*indol-5-yl]amino]methylen]-4-oxo-2-thiazolidinyliden]acetamid
(*Z,E*/*Z*)-2-Cyan-2-[3-ethyl-5-[[[3-(1-methyl-4-piperidinyl)-1-propyl-1*H*-indol-5-yl]amino]methylen]-4-oxo-2-thiazolidinyliden]-*N*-(2,2,2-trifluorethyl)acetamid
(*Z,E*/*Z*)-2-Cyan-*N*-ethyl-2-[3-ethyl-5-[[[3-(1-methyl-4-piperidinyl)-1-propyl-1*H*-indol-5-yl]amino]methylen]-4-oxo-2-thiazolidinyliden]acetamid
(*Z,E*/*Z*)-2-Cyan-*N*-(cyanmethyl)-2-[3-ethyl-5-[[[3-(1-ethyl-4-piperidinyl)-1-propyl-1*H-*indol-5-yl]amino]methylen]-4-oxo-2-thiazolidinyliden]acetamid
(*Z,E*/*Z*)-2-Cyan-2-[3-ethyl-5-[[[3-(1-ethyl-4-piperidinyl)-1-propyl-1*H*-indol-5-yl]amino]methylen]-4-oxo-2-thiazolidinyliden]-N-(2,2,2-trifluorethyl)acetamid
(*Z,E*/*Z*)-2-Cyan-*N*-ethyl-2-[3-ethyl-5-[[[3-(1-ethyl-4-piperidinyl)-1-propyl-1*H*-indol-5-yl]amino]methylen]-4-oxo-2-thiazolidinyliden]acetamid
(*Z,E*/*Z*)-2-Cyan-*N*-(cyanmethyl)-2-[3-ethyl-5-[[[1-(1-methyl-4-piperidinyl)-1*H*-indazol-6-yl]amino]methylen]-4-oxo-2-thiazolidinyliden]acetamid
(*Z,E*/*Z*)-2-Cyan-2-[5-[[[1-(1-cyclopentyl-4-piperidinyl)-1*H*-indazol-6-yl]amino]methylen]-3-ethyl-4-oxo-2-thiazolidinyliden]-*N*-(2,2,2-trifluorethyl)acetamid
(*Z,E*/*Z*)-2-Cyan-*N*-(cyanmethyl)-2-[5-[[[1-(1-cyclopentyl-4-piperidinyl)-1*H*-indazol-6-yl]amino]methylen]-3-ethyl-4-oxo-2-thiazolidinyliden]acetamid
(*Z,E*/*Z*)-2-Cyan-2-[5-[[[1-(1-cyclopentyl-4-piperidinyl)-1*H*-indazol-6-yl]amino]methylen]-3-ethyl-4-oxo-2-thiazolidinyliden]-*N*-ethylacetamid
(*Z,E*/*Z*)-2-Cyan-2-[3-ethyl-4-oxo-5-[[[1-(1-methyl-4-piperidinyl)-1*H*-indazol-5-yl]amino]methylen]-2-thiazolidinyliden]-*N*-(2,2,2-trifluorethyl)acetamid
(*Z,E*/*Z*)-2-Cyan-*N*-(cyanmethyl)-2-[3-ethyl-5-[[[1-(1-methyl-4-piperidinyl)-1*H*-indazol-5-yl]amino]methylen]-4-oxo-2-thiazolidinyliden]acetamid
(*Z,E*/*Z*)-2-Cyan-*N*-ethyl-2-[3-ethyl-5-[[[1-(1-methyl-4-piperidinyl)-1*H*-indazol-5-yl]amino]methylen]-4-oxo-2-thiazolidinyliden]acetamid
(*Z,E*/*Z*)-2-Cyan-2-[3-ethyl-5-[[[2-[1-(1-methylethyl)-4-piperidinyl]-2*H*-indazol-6-yl]amino]methylen]-4-oxo-2-thiazolidinyliden]-*N*-(2,2,2-trifluorethyl)acetamid
(*Z,E*/*Z*)-2-Cyan-*N*-(cyanmethyl)-2-[3-ethyl-5-[[[2-[1-(1-methylethyl)-4-piperidinyl]-2*H-*indazol-6-yl]amino]methylen]-4-oxo-2-thiazolidinyliden]acetamid
(*Z,E*/*Z*)-2-Cyan-*N*-(cyanmethyl)-2-[3-ethyl-5-[[[1-methyl-3-(1-methyl-4-piperidinyl)-1 H-indol-5-yl]amino]methylen]-4-oxo-2-thiazolidinyliden]acetamid
(*Z,E*/*Z*)-2-Cyan-*N*-(cyanmethyl)-2-[5-[[[3-(1-cyclopropyl-4-piperidinyl)-1-methyl-1*H-*indol-5-yl]amino] methylen]-3-ethyl-4-oxo-2-thiazolidinyliden]acetamid
(*Z,E*/*Z*)-2-Cyan-*N*-(cyanmethyl)-2-[3-ethyl-5-[[[1-methyl-3-[1-(2-methylpropyl)-4-piperidinyl]-1*H*-indol-5-yl]amino]methylen]-4-oxo-2-thiazolidinyliden]acetamid
(*Z,E*/*Z*)-2-Cyan-*N*-(cyanmethyl)-2-[3-ethyl-5-[[[3-(1-ethyl-4-piperidinyl)-1-methyl-1*H-*indol-5-yl]amino]methylen]-4-oxo-2-thiazolidinyliden]acetamid
(*Z,E*/*Z*)-2-Cyan-2-[3-ethyl-5-[[[1-methyl-3-(1-methyl-4-piperidinyl)-1*H*-indol-5-yl]amino]methylen]-4-oxo-2-thiazolidinyliden]-*N*-(2,2,2-trifluorethyl)acetamid
(*Z,E*/*Z*)-2-Cyan-2-[3-ethyl-5-[[[3-(1-ethyl-4-piperidinyl)-1-methyl-1*H*-indol-5-yl]amino]methylen]-4-oxo-2-thiazolidinyliden]-*N*-(2,2,2-trifluorethyl)acetamid
(*Z,E*/*Z*)-2-[5-[[[3-(1-Acetyl-4-piperidinyl)-1-methyl-1*H*-indol-5-yl]amino]methylen]-3-ethyl-4-oxo-2-thiazolidinyliden]-2-cyan-*N*-(cyanmethyl)acetamid
(*Z,E*/*Z*)-2-Cyan-*N*-(cyanmethyl)-2-[3-ethyl-5-[[[1-methyl-3-[1-(1-methylethyl)-4-piperidinyl]-1*H*-indol-5-yl]amino]methylen]-4-oxo-2- thiazolidinyliden]acetamid
(*Z,E*/*Z*)-2-Cyan-2-[3-ethyl-5-[[[1-methyl-3-[1-(1-methylethyl)-4-piperidinyl]-1*H*-indol-5-yl]amino]methylen]-4-oxo-2-thiazolidinyliden]-*N*-(2,2,2-trifluorethyl)acetamid
(*Z,E*/*Z*)-2-Cyan-2-[5-[[[3-(1-cyclopropyl-4-piperidinyl)-1-methyl-1*H*-indol-5-yl]amino]methylen]-3-ethyl-4-oxo-2-thiazolidinyliden]-*N*-(2,2,2-trifluorethyl)acetamid
(*Z,E*/*Z*)-2-Cyan-2-[3-ethyl-5-[[[1-methyl-3-[1-(2-methylpropyl)-4-piperidinyl]-1*H*-indol-5-yl]amino]methylen]-4-oxo-2-thiazolidinyliden]-*N*-(2,2,2-trifluorethyl)acetamid
(*Z,E*/*Z*)-2-Cyan-*N*-ethyl-2-[3-ethyl-5-[[[1-methyl-3-(1-methyl-4-piperidinyl)-1*H*-indol-5-yl]amino]methylen]-4-oxo-2-thiazolidinyliden]acetamid
(*Z,E*/*Z*)-2-Cyan-*N*-ethyl-2-[3-ethyl-5-[[[3-(1-ethyl-4-piperidinyl)-1-methyl-1*H*-indol-5-yl]amino]methylen]-4-oxo-2-thiazolidinyliden]acetamid
(*Z,E*/*Z*)-2-[5-[[[3-(1-Acetyl-4-piperidinyl)-1-methyl-1*H*-indol-5-yl]amino]methylen]-3-ethyl-4-oxo-2-thiazolidinyliden]-2-cyan-*N*-(2,2,2-trifluorethyl)acetamid
(*Z,E*/*Z*)-2-Cyan-2-[3-ethyl-5-[[[1-(2-methoxyethyl)-3-[(4-methyl-1-piperazinyl)carbonyl]-1*H*-indol-5-yl]amino]methylen]-4-oxo-2-thiazolidinyliden]-*N-*(2,2,2-trifluorethyl)acetamid
(*Z,E*/*Z*)-2-Cyan-*N*-(cyanmethyl)-2-[3-ethyl-5-[[[1-(2-methoxyethyl)-3-[(4-methyl-1-piperazinyl)carbonyl]-1*H*-indol-5-yl]amino]methylen]-4-oxo-2-thiazolidinyliden]acetamid
(*Z,E*/*Z*)-2-Cyan-*N*-(cyanmethyl)-2-[3-ethyl-5-[[[1-methyl-3-(1-methyl-4-piperidinyl)-1*H*-indol-6-yl]amino]methylen]-4-oxo-2-thiazolidinyliden]acetamid
(*Z,E*/*Z*)-2-[5-[[[3-Chlor-1-(2-methoxyethyl)-1*H*-indol-6-yl]amino]methylen]-3-ethyl-4-oxo-2-thiazolidinyliden]-2-cyan-*N*-(cyanmethyl)acetamid
(*Z,E*/*Z*)-2-Cyan-*N*-(cyanmethyl)-2-[3-ethyl-5-[[[1-(2-methoxyethyl)-3-(1-pyrrolidinylmethyl)-1*H*-indol-6-yl]amino]methylen]-4-oxo-2-thiazolidinyliden]acetamid
(*Z,E*/*Z*)-2-Cyan-2-[3-ethyl-5-[[[1-methyl-3-(1-methyl-4-piperidinyl)-1*H*-indol-6-yl]amino]methylen]-4-oxo-2-thiazolidinyliden]-*N*-(2,2,2-trifluorethyl)acetamid
(*Z,E*/*Z*)-2-Cyan-2-[3-ethyl-5-[[[1-(2-methoxyethyl)-3-(1-pyrrolidinylmethyl)-1*H*-indol-6-yl]amino]methylen]-4-oxo-2-thiazolidinyliden]-*N*-(2,2,2-trifluorethyl)acetamid
(*Z,E*/*Z*)-2-Cyan-*N*-(cyanmethyl)-2-[3-ethyl-5-[[[1-(2-methoxyethyl)-3-[(4-methyl-1-piperazinyl)methyl]-1*H*-indol-6-yl]amino]methylen]-4-oxo-2-thiazolidinyliden]acetamid
(*Z,E*/*Z*)-2-Cyan-*N*-(cyanmethyl)-2-[3-ethyl-5-[[[1-methyl-3-[1-(2-methylpropyl)-4-piperidinyl]-1*H*-indol-6-yl]amino]methylen]-4-oxo-2-thiazolidinyliden]acetamid
(*Z,E*/*Z*)-2-Cyan-2-[3-ethyl-5-[[[1-methyl-3-[1-(2-methylpropyl)-4-piperidinyl]-1*H*-indol-6-yl]amino]methylen]-4-oxo-2-thiazolidinyliden]-*N*-(2,2,2-trifluorethyl)acetamid
(*Z,E*/*Z*)-2-Cyan-2-[3-ethyl-5-[[[1-(2-methoxyethyl)-3-[(4-methyl-1-piperazinyl)methyl]-1*H*-indol-6-yl]amino]methylen]-4-oxo-2-thiazolidinyliden]-*N*-(2,2,2-trifluorethyl)acetamid
(*Z,E*/*Z*)-2-Cyan-2-[5-[[[1-(1-cyclopentyl-4-piperidinyl)-1*H*-indol-6-yl]amino]methylen]-3-ethyl-4-oxo-2-thiazolidinyliden]-*N*-(2,2,2-trifluorethyl)acetamid
(*Z,E*/*Z*)-2-Cyan-*N*-(cyanmethyl)-2-[5-[[[1-(1-cyclopentyl-4-piperidinyl)-1*H*-indol-6-yl]amino]methylen]-3-ethyl-4-oxo-2-thiazolidinyliden]acetamid
(*Z,E*/*Z*)-2-Cyan-2-[3-ethyl-5-[[[1-(1-methyl-4-piperidinyl)-1*H*-benzimidazol-6-yl]amino]methylen]-4-oxo-2-thiazolidinyliden]-*N*-(2,2,2-trifluorethyl)acetamid
(*Z,E*/*Z*)-2-Cyan-*N*-(cyanmethyl)-2-[3-ethyl-5-[[[1-(1-methyl-4-piperidinyl)-1*H-*benzimidazol-6-yl]amino]methylen]-4-oxo-2-thiazolidinyliden]acetamid
(*Z,E*/*Z*)-2-Cyan-2-[5-[[[1-(1-cyclopentyl-4-piperidinyl)-1*H*-benzimidazol-6-yl]amino]methylen]-3-ethyl-4-oxo-2-thiazolidinyliden]-*N*-(2,2,2-trifluorethyl)acetamid
(*Z,F*/*Z*)-2-[5-[[[3-Chlor-1-(1-methyl-4-piperidinyl)-1*H*-indol-6-yl]amino]methylen]-3-ethyl-4-oxo-2-thiazolidinyliden]-2-cyan-*N*-(2,2,2-trifluorethyl)acetamid
(*Z,E*/*Z*)-2-[5-[[[3-Chlor-1-(1-methyl-4-piperidinyl)-1*H*-indol-6-yl]amino]methyten]-3-ethyl-4-oxo-2-thiazolidinyliden]-2-cyan-*N*-(cyanmethyl)acetamid
(*Z,E*/*Z*)-2-[5-[[[3-Acetyl-1-(1-methyl-4-piperidinyl)-1*H*-indol-6-yl]amino]methylen]-3-ethyl-4-oxo-2-thiazolidinyliden]-2-cyan-*N*-(2,2,2-trifluorethyl)acetamid
(*Z,E*/*Z*)-2-[5-[[[3-Acetyl-1-(1-methyl-4-piperidinyl)-1*H*-indol-6-yl]amino]methylen]-3-ethyl-4-oxo-2-thiazolidinyliden]-2-cyan-*N*-(cyanmethyl)acetamid
(*Z,E*/*Z*)-2-Cyan-2-[3-ethyl-5-[[[1-(2-methoxyethyl)-3-[(4-methyl-1-piperazinyl)carbonyl]-1*H*-indol-6-yl]amino]methylen]-4-oxo-2-thiazolidinyliden]-*N-*(2,2,2-trifluorethyl)acetamid
(*Z,E*/*Z*)-2-Cyan-*N*-(cyanmethyl)-2-[3-ethyl-5-[[[1-(2-methoxyethyl)-3-[(4-methyl-1-piperazinyl)carbonyl]-1*H*-indol-6-yl]amino]methylen]-4-oxo-2-thiazolidinyliden]acetamid
[*trans*-(*Z,E*/*Z*)]-2-Cyan-2-[5-[[[1-[4-(dimethylamino)cyclohexyl]-1*H*-indol-6-yl]amino]methylen]-3-ethyl-4-oxo-2-thiazolidinyliden]-*N*-(2,2,2-trifluorethyl)acetamid
[*trans*-(*Z,E*/*Z*)]-2-Cyan-*N*-(cyanmethyl)-2-[5-[[[1-[4-(dimethylamino)cyclohexyl]-1*H-*indol-6-yl]amino]methylen]-3-ethyl-4-oxo-2-thiazolidinyliden]acetamid
(*Z,E*/*Z*)-2-Cyan-*N*-(cyanmethyl)-2-[3-ethyl-4-oxo-5-[[[1-(1,2,2,6,6-pentamethyl-4-piperidinyl)-1*H*-indol-6-yl]amino]methylen]-2-thiazolidinyliden]acetamid
(*Z,E*/*Z*)-2-Cyan-2-[3-ethyl-4-oxo-5-[[[1-(1,2,2,6,6-pentamethyl-4-piperidinyl)-1*H-*indol-6-yl]amino]methylen]-2-thiazolidinyliden]-*N*-(2,2,2-trifluorethyl)acetamid
[*trans*-(*Z,E*/*Z*)]-2-[5-[[[3-Chlor-1-[4-(dimethylamino)cyclohexyl]-1*H*-indol-6-yl]amino]methylen]-3-ethyl-4-oxo-2-thiazolidinyliden]-2-cyan-*N*-(2,2,2-trifluorethyl)acetamid
[*trans*-(*Z,E*/*Z*)]-2-[5-[[[3-Chlor-1-[4-(dimethylamino)cyclohexyl]-1*H*-indol-6-yl]amino]methylen]-3-ethyl-4-oxo-2-thiazolidinyliden]-2-cyan-*N-*(cyanmethyl)acetamid
(*Z,E*/*Z*)-2-Cyan-2-[5-[[[2,3-dihydro-1-(1-methyl-4-piperidinyl)-2-oxo-1*H*-indol-6-yl]amino]methylen]-3-ethyl-4-oxo-2-thiazolidinyliden]-*N*-(2,2,2-trifluorethyl)acetamid
(*Z,E*/*Z*)-2-Cyan-*N*-(cyanmethyl)-2-[5-[[[2,3-dihydro-1-(1-methyl-4-piperidinyl)-2-oxo-1*H*-indol-6-yl]amino]methylen]-3-ethyl-4-oxo-2-thiazolidinyliden]acetamid
(*Z,E*/*Z*)-2-Cyan-*N*-(cyanmethyl)-2-[3-ethyl-5-[[[2-(4-methyl-1-piperazinyl)-5-benzoxazolyl]amino]methylen]-4-oxo-2-thiazolidinyliden]acetamid
(*Z,E*/*Z*)-2-Cyan-*N*-ethyl-2-[3-ethyl-5-[[[2-(4-methyl-1-piperazinyl)-5-benzoxazolyl]amino]methylen]-4-oxo-2-thiazolidinyliden]acetamid
(*Z,E*/*Z*)-2-Cyan-2-[3-ethyl-5-[[[2-(4-methyl-1-piperazinyl)-5-benzoxazolyl]amino]methylen]-4-oxo-2-thiazolidinyliden]-*N*-(2,2,2-trifluorethyl)acetamid
(*Z,E*/*Z*)-2-Cyan-2-[3-ethyl-5-[[[2-(4-methyl-1-piperazinyl)-5-benzoxazolyl]amino]methylen]-4-oxo-2-thiazolidinyliden]-*N*-(2-propinyl)acetamid
(*Z,E*/*Z*)-2-Cyan-2-[3-ethyl-5-[[[1-(1-methyl-4-piperidinyl)-1*H*-indol-6-yl]amino]methylen]-4-oxo-2-thiazolidinyliden]-*N*-(2-propinyl)acetamid
(*Z,E*/*Z*)-2-Cyan-2-[3-ethyl-5-[[[1-(1-methyl-4-piperidinyl)-1*H*-indol-6-yl]amino]methylen]-4-oxo-2-thiazolidinyliden]-*N*-(2-fluorethyl)acetamid
(*Z,E*/*Z*)-2-Cyan-*N*-(2,2-difluorethyl)-2-[3-ethyl-5-[[[1-(1-methyl-4-piperidinyl)-1*H-*indol-6-yl]amino]methylen]-4-oxo-2-thiazolidinyliden]acetamid
(*Z,E*/*Z*)-2-Cyan-*N*-ethyl-2-[3-ethyl-5-[[[1-(1-methyl-4-piperidinyl)-1*H*-indol-6-yl]amino]methylen]-4-oxo-2-thiazolidinyliden]acetamid
(*Z,E*/*Z*)-2-Cyan-2-[3-ethyl-5-[[[1-(1-methyl-4-piperidinyl)-1*H*-indol-6-yl]amino]methylen]-4-oxo-2-thiazolidinyliden]-*N*-[2,2,2-trifluor-1-(2-furanyl)ethyl]acetamid
(*Z,E*/*Z*)-2-Cyan-2-[3-ethyl-5-[[[1-(1-methyl-4-piperidinyl)-1*H*-indol-6-yl]amino]methylen]-4-oxo-2-thiazolidinyliden]-*N*-(2,2,3,3,3-pentafluorpropyl)acetamid
[*endo*-(*Z,E*/*Z*)]-2-Cyan-*N*-(cyanmethyl)-2-[3-ethyl-5-[[[1-(8-methyl-8-azabicyclo[3.2.1]oct-3-yl)-1*H*-indol-6-yl]amino]methylen]-4-oxo-2-thiazolidinyliden]acetamid
[*trans*-(*Z,E*/*Z*)]-2-Cyan-2-[3-ethyl-5-[[[1-(8-methyl-8-azabicyclo[3.2.1]oct-3-yl)-1*H-*indol-6-yl]amino]methylen]-4-oxo-2-thiazolidinyliden]-*N*-(2,2,2-trifluorethyl)acetamid
(*Z,E*/*Z*)-2-Cyan-2-[3-ethyl-5-[[[3-fluor-1-(1-methyl-4-piperidinyl)-1*H*-indol-6-yl]amino]methylen]-4-oxo-2-thiazolidinyliden]-*N*-(2,2,2-trifluorethyl)acetamid
(*Z,E*/*Z*)-2-Cyan-2-[5-[[[1-(2,2-dimethyl-1-oxopropyl)-3-(1-methyl-4-piperidinyl)-1*H-*indol-5-yl]amino]methylen]-3-ethyl-4-oxo-2-thiazolidinyliden]-*N*-(2,2,2-trifluorethyl)acetamid
(*Z,E*/*Z*)-2-Cyan-2-[5-[[[3-(2,2-dimethyl-1-oxopropyl)-1-(1-methyl-4-piperidinyl)-1*H-*indol-6-yl]amino]methylen]-3-ethyl-4-oxo-2-thiazolidinyliden]-*N*-(2,2,2-trifluorethyl)acetamid
(*Z,E*/*Z*)-2-Cyan-*N*-(cyanmethyl)-2-[5-[[[3-(2,2-dimethyl-1-oxopropyl)-1-(1-methyl-4-piperidinyl)-1*H*-indol-6-yl]amino]methylen]-3-ethyl-4-oxo-2-thiazolidinyliden]acetamid
(*Z,E*/*Z*)-6-[[[2-[1-Cyan-2-oxo-2-[(2,2,2-trifluorethyl)amino]ethyliden]-3-ethyl-4-oxo-5-thiazolidinyliden]methyl]amino]-1-(1-methyl-4-piperidinyl)-1*H*-indol-3-carboxamid
(*Z,E*/*Z*)-6-[[[2-[1-Cyan-2-[(cyanmethyl)amino]-2-oxoethyliden]-3-ethyl-4-oxo-5-thiazolidinyliden]methyl]amino]-1-(1-methyl-4-piperidinyl)-1*H*-indol-3-carboxamid
(*Z,E*/*Z*)-6-[[(E)-[2-[1-Cyan-2-oxo-2-[(2,2,3,3,3-pentafluorpropyl)amino]ethyliden]-3-ethyl-4-oxo-5-thiazolidinyliden]methyl]amino]-1-(1-methyl-4-piperidinyl)-1*H*-indol-3-carboxamid
(*Z,E*/*Z*)-2-Cyan-2-[5-[[[3-cyan-1-(1-methyl-4-piperidinyl)-1*H-*-indol-6-yl]amino]methylen]-3-ethyl-4-oxo-2-thiazolidinyliden]-*N-*-(2,2,2-trifluorethyl)acetamid
(*Z,E*/*Z*)-2-Cyan-*N*-(cyanmethyl)-2-[5-[[[3-cyan-1-(1-methyl-4-piperidinyl)-1*H*-indol-6-yl]amino]methylen]-3-ethyl-4-oxo-2-thiazolidinyliden]acetamid
[*trans*-(*Z,E*/*Z*)]-2-Cyan-2-[5-[[[1-[4-(dimethylamino)cyclohexyl]-1*H*-indol-6-yl]amino]methylen]-3-ethyl-4-oxo-2-thiazolidinyliden]-*N*-(2,2,3,3,3-pentafluorpropyl)acetamid
[*cis*-(*Z,E*/*Z*)]-2-Cyan-2-[5-[[[1-[4-(dimethylamino)cyclohexyl]-1*H*-indol-6-yl]amino]methylen]-3-ethyl-4-oxo-2-thiazolidinyliden]-*N*-(2,2,2-trifluorethyl)acetamid
[*cis*-(*Z,E*/*Z*)]-2-Cyan-*N*-(cyanmethyl)-2-[5-[[[1-[4-(dimethylamino)cyclohexyl]-1*H*-indol-6-yl]amino]methylen]-3-ethyl-4-oxo-2-thiazolidinyliden]acetamid
[1α(*Z,E*/*Z*),2α]-2-Cyan-2-[5-[[[1-[2-(dimethylamino)cyclohexyl]-1*H*-indol-6-yl]amino]methylen]-3-ethyl-4-oxo-2-thiazolidinyliden]-*N*-(2,2,2-trifluorethyl)acetamid
[1α(*Z,E*/*Z*),2α]-2-Cyan-*N*-(cyanmethyl)-2-[5-[[[1-[2-(dimethylamino)cyclohexyl]-1*H-*indol-6-yl]amino]methylen]-3-ethyl-4-oxo-2-thiazolidinyliden]acetamid

9. **Verbindungen der allgemeinen Formel ( I ) gemäß Anspruch 1 oder 7 mit einem der folgenden Namen:**
(*Z,E*/*Z*)-2-Cyan-2-[3-ethyl-5-[[[1-methyl-3-(1-piperidinylmethyl)-1*H*-indol-6-yl]amino]methylen]-4-oxo-2-thiazolidinyliden]-*N*-(2,2,2-trifluorethyl)acetamid
(*Z,E*/*Z*)-2-Cyan-*N*-(cyanmethyl)-2-[3-ethyl-5-[[[1-methyl-3-(1-piperidinylmethyl)-1*H-*indol-6-yl]amino]methylen]-4-oxo-2-thiazolidinyliden]acetamid
(*Z,E*/*Z*)-2-Cyan-2-[3-ethyl-5-[[[1-methyl-3-(4-morpholinylmethyl)-1*H*-indol-6-yl]amino]methylen]-4-oxo-2-thiazolidinyliden]-*N*-(2,2,2-trifluorethyl)acetamid
(*Z,E*/*Z*)-2-Cyan-*N*-(cyanmethyl)-2-[3-ethyl-5-[[[1-methyl-3-(4-morpholinylmethyl)-1*H-*indol-6-yl]amino]methylen]-4-oxo-2-thiazolidinyliden]acetamid
(*Z,E*/*Z*)-2-Cyan-2-[3-ethyl-5-[[1-methyl-3-[(4-methyl-1-piperazinyl)methyl]-1*H*-indol-6-yl]amino]methylen]-4-oxo-2-thiazolidinyliden]-*N*-(2,2,2-trifluorethyl)acetamid
(*Z,E*/*Z*)-2-Cyan-*N*-(cyanmethyl)-2-[3-ethyl-5-[[1-methyl-3-[(4-methyl-1-piperazinyl)methyl]-1*H*-indol-6-yl]amino]methylen]-4-oxo-2-thiazolidinyliden]acetamid
(*Z,E*/*Z*)-2-Cyan-2-[5-[[[3-[(dimethylamino)methyl]-1-methyl-1*H*-indol-6-yl]amino]methylen]-3-ethyl-4-oxo-2-thiazolidinyliden]-*N*-(2,2,2-trifluorethyl)acetamid
(*Z,E*/*Z*)-2-Cyan-*N*-(cyanmethyl)-2-[5-[[[3-[(dimethylamino)methyl]-1-methyl-1*H*-indol-6-yl]amino]methylen]-3-ethyl-4-oxo-2-thiazolidinyliden]acetamid
(*Z,E*/*Z*)-2-Cyan-2-[3-ethyl-5-[[(1-ethyl-2,3-dihydro-1*H*-indol-5-yl)amino]methylen]-4-oxo-2-thiazolidinyliden]-*N*-(2,2,2-trifluorethyl)acetamid
(*Z,E*/*Z*)-2-Cyan-*N*-(cyanmethyl)-2-[3-ethyl-5-[[(1-ethyl-2,3-dihydro-1*H*-indol-5-yl)amino]methylen]-4-oxo-2-thiazolidinyliden]acetamid
(*Z,E*/*Z*)-2-Cyan-2-[3-ethyl-5-[[[1-methyl-3-(1-pyrrolidinylmethyl)-1*H*-indol-5-yl]amino]methylen]-4-oxo-2-thiazolidinyliden]-*N*-(2,2,2-trifluorethyl)acetamid
(*Z,E*/*Z*)-2-Cyan-*N*-(cyanmethyl)-2-[3-ethyl-5-[[[1-methyl-3-(1-pyrrolidinylmethyl)-1*H-*indol-5-yl]amino]methylen]-4-oxo-2-thiazolidinyliden]acetamid
(*Z,E*/*Z*)-2-Cyan-2-[3-ethyl-5-[[[1-methyl-3-(4-morpholinylmethyl)-1*H*-indol-5-yl]amino]methylen]-4-oxo-2-thiazolidinyliden]-*N*-(2,2,2-trifluorethyl)acetamid
(*Z,E*/*Z*)-2-Cyan-*N*-(cyanmethyl)-2-[3-ethyl-5-[[[1-methyl-3-(4-morpholinylmethyl)-1*H-*indol-5-yl]amino]methylen]-4-oxo-2-thiazolidinyliden]acetamid
(*Z,E*/*Z*)-2-Cyan-*N*-(cyanmethyl)-2-[3-ethyl-5-[[[1-methyl-3-[(4-methyl-1-piperazinyl)methyl]-1*H*-indol-5-yl]amino]methylen]-4-oxo-2-thiazolidinyliden]acetamid
(*Z,E*/*Z*)-2-Cyan-2-[3-ethyl-5-[[[3-[(hexahydro-1*H*-azepin-1-yl)methyl]-1-methyl-1*H-*indol-5-yl]amino]methylen]-4-oxo-2-thiazolidinyliden]-*N*-(2,2,2-trifluorethyl)acetamid
(*Z,E*/*Z*)-2-Cyan-*N*-(cyanmethyl)-2-[3-ethyl-5-[[[3-[(hexahydro-1*H*-azepin-1-yl)methyl]-1-methyl-1*H*-indol-5-yl]amino]methylen]-4-oxo-2-thiazolidinyliden]acetamid
(*Z,E*/*Z*)-2-Cyan-2-[5-[[[3-[(dimethylamino)methyl]-1-methyl-1*H*-indol-5-yl]amino]methylen]-3-ethyl-4-oxo-2-thiazolidinyliden]-*N*-(2,2,2-trifluorethyl)acetamid
(*Z,E*/*Z*)-2-Cyan-*N*-(cyanmethyl)-2-[3-ethyl-4-oxo-5-[[[3-(1-piperidinylmethyl)-1-propyl-1*H*-indol-5-yl]amino]methylen]-2-thiazolidinyliden]acetamid
(*Z,E*/*Z*)-2-Cyan-2-[3-ethyl-5-[[[3-(4-morpholinylmethyl)-1-propyl-1*H*-indol-5-yl]amino]methylen]-4-oxo-2-thiazolidinyliden]-*N*-(2,2,2-trifluorethyl)acetamid
(*Z,E*/*Z*)-2-Cyan-2-[3-ethyl-5-[[[1-methyl-3-[2-(1-pyrrolidinyl)ethyl]-1*H*-indol-5-yl]amino]methylen]-4-oxo-2-thiazolidinyliden]-*N*-(2,2,2-trifluorethyl)acetamid
(*Z,E*/*Z*)-2-Cyan-2-[3-ethyl-5-[[[1-methyl-3-[2-(1-piperidinyl)ethyl]-1*H*-indol-5-yl]amino]methylen]-4-oxo-2-thiazolidinyliden]-*N*-(2,2,2-trifluorethyl)acetamid
(*Z,E*/*Z*)-2-Cyan-2-[3-ethyl-5-[[[1-methyl-3-[2-(4-morpholinyl)ethyl]-1*H*-indol-5-yl]amino]methylen]-4-oxo-2-thiazolidinyliden]-*N*-(2,2,2-trifluorethyl)acetamid
(*Z,E*/*Z*)-2-Cyan-2-[3-ethyl-5-[[[1-methyl-3-[2-(4-methyl-1-piperazinyl)ethyl]-1*H*-indol-5-yl]amino]methylen]-4-oxo-2-thiazolidinyliden]-*N*-(2,2,2-trifluorethyl)acetamid
(*Z,E*/*Z*)-2-Cyan-2-[5-[[[3-[2-(dimethylamino)ethyl]-1-methyl-1*H*-indol-5-yl]amino]methylen]-3-ethyl-4-oxo-2-thiazolidinyliden]-*N*-(2,2,2-trifluorethyl)acetamid
(*Z,E*/*Z*)-2-Cyan-2-[5-[[[3-[2-(diethylamino)ethyl]-1-methyl-1*H*-indol-5-yl]amino]methylen]-3-ethyl-4-oxo-2-thiazolidinyliden]-*N*-(2,2,2-trifluorethyl)acetamid
(*Z,E*/*Z*)-2-Cyan-2-[5-[[[1,2-dimethyl-3-(4-morpholinylmethyl)-1*H*-indol-5-yl]amino]methylen]-3-ethyl-4-oxo-2-thiazolidinyliden]-*N*-(2,2,2-trifluorethyl)acetamid
(*Z,E*/*Z*)-2-Cyan-*N*-(cyanmethyl)-2-[5-[[[1,2-dimethyl-3-(4-morpholinylmethyl)-1*H-*indol-5-yl]amino]methylen]-3-ethyl-4-oxo-2-thiazolidinyliden]acetamid
(*Z,E*/*Z*)-2-Cyan-2-[3-ethyl-5-[[[1-[(1-methyl-3-pyrrolidinyl)methyl]-1*H*-indol-6-yl]amino]methylen]-4-oxo-2-thiazolidinyliden]-*N*-(2,2,2-trifluorethyl)acetamid
(*Z,E*/*Z*)-2-Cyan-2-[3-ethyl-5-[[[1-[(1-ethyl-2-pyrrolidinyl)methyl]-1*H*-indol-6-yl]amino]methylen]-4-oxo-2-thiazolidinyliden]-*N*-(2,2,2-trifluorethyl)acetamid
(*Z,E*/*Z*)-2-Cyan-2-[3-ethyl-5-[[[1-[(1-methyl-4-piperidinyl)methyl]-1*H*-indol-6-yl]amino]methylen]-4-oxo-2-thiazolidinyliden]-*N*-(2,2,2-trifluorethyl)acetamid
(*Z,E*/*Z*)-2-Cyan-2-[3-ethyl-5-[[[2-methyl-1-[(1-methyl-4-piperidinyl)methyl]-1*H*-indol-6-yl]amino]methylen]-4-oxo-2-thiazolidinyliden]-*N*-(2,2,2-trifluorethyl)acetamid
(*Z,E*/*Z*)-6-[[[2-[1-Cyan-2-oxo-2-[(2,2,2-trifluorethyl)amino]ethyliden]-3-ethyl-4-oxo-5-thiazolidinyliden]methyl]amino]-*N*-methyl-1*H*-indol-1-acetamid
(*Z,E*/*Z*)-6-[[[2-[1-Cyan-2-[(cyanmethyl)amino]-2-oxoethyliden]-3-ethyl-4-oxo-5-thiazolidinyliden]methyl]amino]-*N*-methyl-1*H*-indol-1-acetamid
(*Z,E*/*Z*)-6-[[[2-[1-Cyan-2-oxo-2-[(2,2,2-trifluorethyl)amino]ethyliden]-3-ethyl-4-oxo-5-thiazolidinyliden]methyl]amino]-*N,N*-dimethyl-1*H*-indol-1-acetamid
(*Z,E*/*Z*)-6-[[[2-[1-Cyan-2-[(cyanmethyl)amino]-2-oxoethyliden]-3-ethyl-4-oxo-5-thiazolidinyliden]methyl]amino]-*N,N*-dimethyl-1*H*-indol-1-acetamid
(*Z,E*/*Z*)-2-Cyan-N-(cyanmethyl)-2-[3-ethyl-5-[[(1-methyl-1*H*-indol-5-yl)amino]methylen]-4-oxo-2-thiazolidinyliden]acetamid
(*Z,E*/*Z*)-2-Cyan-*N*-(cyanmethyl)-2-[3-ethyl-4-oxo-5-[[[1-[2-(1-pyrrolidinyl)ethyl]-1*H-*indol-6-yl]amino]methylen]-2-thiazolidinyliden]acetamid
(*Z,E*/*Z*)-2-Cyan-2-[3-ethyl-4-oxo-5-[[[1-[2-(1-pyrrolidinyl)ethyl]-1*H*-indol-6-yl]amino]methylen]-2-thiazolidinyliden]-*N*-(2,2,2-trifluorethyl)acetamid
(*Z,E*/*Z*)-2-Cyan-*N*-(cyanmethyl)-2-[3-ethyl-5-[[[1-[3-(4-methyl-1-piperazinyl)propyl]-1*H*-indol-6-yl]amino]methylen]-4-oxo-2-thiazolidinyliden]acetamid
(*Z,E*/*Z*)-2-[5-[[[3-Chlor-1-[2-(1-pyrrolidinyl)ethyl]-1*H*-indol-6-yl]amino]methylen]-3-ethyl-4-oxo-2-thiazolidinyliden]-2-cyan-N-(2,2,2-trifluorethyl)acetamid
(*Z,E*/*Z*)-2-[5-[[[3-Chlor-1-[2-(1-pyrrolidinyl)ethyl]-1*H*-indol-6-yl]amino]methylen]-3-ethyl-4-oxo-2-thiazolidinyliden]-2-cyan-*N*-(cyanmethyl)acetamid
(*Z,E*/*Z*)-2-Cyan-2-[3-ethyl-5-[[[1-[3-(4-methyl-1-piperazinyl)propyl]-1*H*-indol-6-yl]amino]methylen]-4-oxo-2-thiazolidinyliden]-*N*-(2,2,2-trifluorethyl)acetamid
(*Z,E*/*Z*)-2-Cyan-2-[3-ethyl-4-oxo-5-[[[1-[2-(1-pyrrolidinyl)ethyl]-1*H*-benzimidazol-6-yl]amino]methylen]-2-thiazolidinyliden]-*N*-(2,2,2-trifluorethyl)acetamid
(*Z,E*/*Z*)-2-Cyan-*N*-(cyanmethyl)-2-[3-ethyl-4-oxo-5-[[[1-[2-(1-pyrrolidinyl)ethyl]-1*H-*benzimidazol-6-yl]amino]methylen]-2-thiazolidinyliden]acetamid
(*Z,E*/*Z*)-2-Cyan-2-[3-ethyl-5-[[[3-[(4-methyl-1-piperazinyl)methy]-1-[2-(1-pyrrolidinyl)ethyl]-1*H*-indol-6-yl]amino]methylen]-4-oxo-2-thiazolidinyliden]-*N*-(2,2,2-trifluorethyl)acetamid
und
(*Z,E*/*Z*)-2-Cyan-*N*-(cyanmethyl)-2-[3-ethyl-5-[[[3-[(4-methyl-1-piperazinyl)methyl]-1-[2-(1-pyrrolidinyl)ethyl]-1*H*-indol-6-yl]amino]methylen]-4-oxo-2-thiazolidinyliden]acetamid.

10. Verfahren zur Herstellung von Verbindungen der allgemeinen Formel ( I ) entsprechend der Ansprüche 1 bis 9, bei dem ein Intermediat der allgemeinen Formel ( II ): 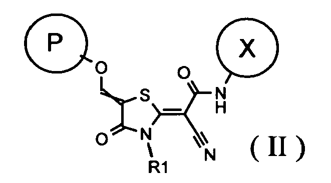 in welcher R1 und X die gemäß einem der Ansprüche 1 bis 9, angegebene Bedeutung haben und P einen C₁-C₆ Alkylrest bedeutet, in einem polaren Lösungsmittel (zum Beispiel Ethanol) mit einem Amin der Formel ( IV ) reagiert, 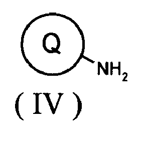 in welcher Q die gemäß einem der Ansprüche 1 bis 9, angegebene Bedeutung hat, und dabei eine Verbindung der Formel ( I ) entsteht, 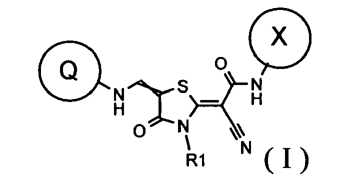 in welcher Q, R1 und X die gemäß einem der Ansprüche 1 bis 9, angegebene Bedeutung haben.

11. Ein Verfahren zur Herstellung von Verbindungen der allgemeinen Formel ( I ) entsprechend der Ansprüche 1 bis 9, bei der ein Intermediat der allgemeinen Formel ( III ): 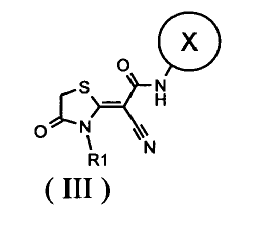 in welcher R1 und X die gemäß einem der Ansprüche 1 bis 9, angegebene Bedeutung haben mit einem Amin der Formel ( IV ), 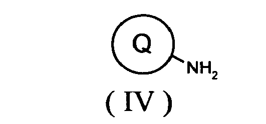 in welcher Q die gemäß einem der Ansprüche 1 bis 9, angegebene Bedeutung hat in einem Ameisensäureorthoester mit drei gleichen oder verschiedenen gegebenenenfalls verbrückten oder mit Halogen substituierten Alkoxy- oder Aryloxyresten und gegebenenfalls einem polaren Lösungsmittel erhitzt werden, und dabei eine Verbindung der Formel ( I ) entsteht, 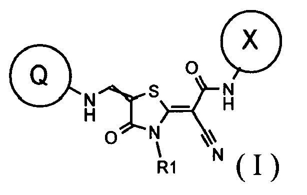 in welcher Q, R1 und X die gemäß einem der Ansprüche 1 bis 9, angegebene Bedeutung haben.

12. Ein Verfahren zur Herstellung von Verbindungen der allgemeinen Formel ( I ) entsprechend der Ansprüche 1 bis 9, bei der ein Intermediat der allgemeinen Formel ( VII ): 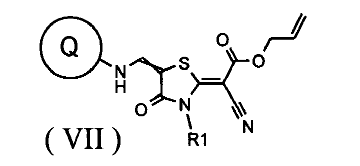 in welcher Q, und R1 die gemäß einem der Ansprüche 1 bis 9 angegebene Bedeutung haben mit einem Allylakzeptor (zum Beispiel Dimethylbarbitursäure) und einem Katalysator (zum Beispiel Tetrakis(triphenylphosphin)palladium(0) ) in einem aprotischen Lösungsmittel umgesetzt und nach abgeschlossener erster Teilreaktion mit einem Kupplungsreagenz zum (Beispiel TBTU), einer Base und mit einem Amin der Formel (VI) 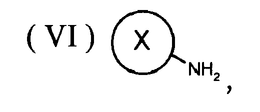 in welcher X die gemäß einem der Ansprüche 1 bis 9 angegebene Bedeutung hat kuppelt, und dabei eine Verbindung der Formel ( I ) entsteht, 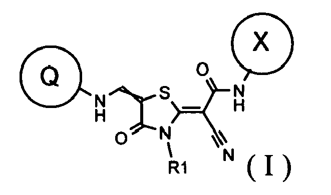 in der Q, R1 und X die gemäß einem der Ansprüche 1 bis 9 angegebene Bedeutung haben.

13. Arzneimittel, die mindestens eine Verbindung gemäß einem der Ansprüche 1 bis 9 enthalten.

14. Verbindungen gemäß einem der Ansprüche 1 bis 9 oder Arzneimittel gemäß Anspruch 13 mit geeigneten Formulierungs- und Trägerstoffen.

15. Verwendung der Verbindungen der allgemeinen Formel ( I ), gemäß einem der Ansprüche 1 bis 9, zur Herstellung eines Arzneimittels zu der Behandlung einer Krankheit.

16. Verwendung der Verbindungen der allgemeinen Formel ( I ), gemäß dem Anspruch 15, in dem die Krankheit durch Inhibitoren von Polo-like Kinasen behandelt werden kann, wie zum Beispiel Krebs, wie solide Tumoren und Leukämie, Autoimmunerkrankungen wie Psoriasis, Alopezie, und Multiple Sklerose, Chemotherapeutika-induzierte Alopezie und Mukositis, kardiovaskulare Erkrankungen, wie Stenosen, Arteriosklerosen und Restenosen, infektiöse Erkrankungen, wie zum Beispiel durch unizellulare Parasiten, wie Trypanosoma, Toxoplasma oder Plasmodium, oder durch Pilze hervorgerufen, nephrologische Erkrankungen, wie zum Beispiel Glomerulonephritis, chronische neurodegenerative Erkrankungen, wie Huntington's Erkrankung, amyotropisch laterale Sklerose, Parkinsonsche Erkrankung, AIDS induzierte Dementia und Alzheimersche Erkrankung, akute neurodegenerative Erkrankungen, wie Ischämien des Gehirns und Neurotraumata, virale Infektionen, wie zum Beispiel Cytomegalus-Infektionen, Herpes, Hepatitis B und C, und HIV.

17. Verbindungen der allgemeinen Formel ( II ): 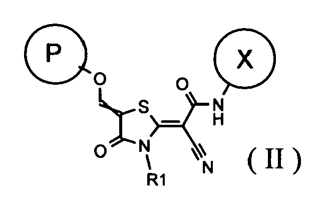 in der R1 und X die in der allgemeinen Formel ( I ), gemäß einem der Ansprüche 1 bis 9, angegebene Bedeutung haben und P einen C₁-C₆ Alkylrest bedeutet, sowie deren Solvate, Hydrate, Diastereomere, Enantiomere und Salze als Zwischenprodukte zur Herstellung von Verbindungen der allgemeinen Formel ( I ).

18. Verbindungen der allgemeinen Formel ( VII ): 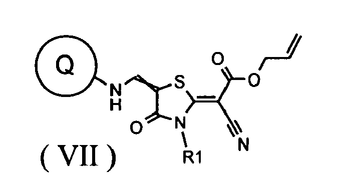 in der R1 und Q die in der allgemeinen Formel ( I ), gemäß einem der Ansprüche 1 bis 9, angegebene Bedeutung haben, sowie deren Solvate, Hydrate, Diastereomere, Enantiomere und Salze als Zwischenprodukte zur Herstellung von Verbindungen der allgemeinen Formel ( I ).

19. Verwendung der Verbindungen der allgemeinen Formeln ( II ) gemäß Anspruch 17 als Zwischenprodukte zur Herstellung von Verbindungen der allgemeinen Formel ( I ) gemäß einer der Ansprüche 1 bis 9.

20. Verwendung der Verbindungen der allgemeinen Formeln ( VII ) gemäß Anspruch 18 als Zwischenprodukte zur Herstellung von Verbindungen der allgemeinen Formel ( I ) gemäß einer der Ansprüche 1 bis 9.
